(19) **Europäisches Patentamt / European Patent Office / Office européen des brevets**

(11) **EP 3 544 607 B1**

(12) **EUROPEAN PATENT SPECIFICATION**

(45) Date of publication and mention of the grant of the patent:
**31.12.2025  Bulletin 2026/01**

(21) Application number: **17873940.5**

(22) Date of filing: **22.11.2017**

(51) International Patent Classification (IPC):
*A61K 31/7088* (2006.01)   *A61K 39/00* (2006.01)
*A61K 39/395* (2006.01)   *A61K 45/06* (2006.01)
*C12N 7/00* (2006.01)   *A61P 35/02* (2006.01)
*A61P 37/04* (2006.01)

(52) Cooperative Patent Classification (CPC):
(C-Sets available)
**A61K 45/06; A61K 31/7088; A61K 31/713;
A61K 39/0011; A61K 39/001188; A61K 39/001191;
A61P 35/00; A61P 35/02; A61P 37/04; C12N 7/00;**
A61K 2039/55544; A61K 2039/57; A61K 2039/585;
C12N 2710/10043; C12N 2710/10071;       (Cont.)

(86) International application number:
**PCT/US2017/063133**

(87) International publication number:
**WO 2018/098362 (31.05.2018 Gazette 2018/22)**

(54) **CHIMPANZEE ADENOVIRAL DELIVERY OF NEOANTIGENS**

SCHIMPANSEN ADENOVIRALE FREISETZUNG VON NEOANTIGENEN

ADMINISTRATION ADÉNOVIRALE CHIMPANZÉE DE NÉO-ANTIGÈNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Validation States:
**MA**

(30) Priority: **23.11.2016   US 201662425996 P
16.12.2016   US 201662435266 P
08.05.2017   US 201762503196 P
21.06.2017   US 201762523212 P**

(43) Date of publication of application:
**02.10.2019   Bulletin 2019/40**

(60) Divisional application:
**25211455.8**

(73) Proprietor: **Seattle Project Corp.
Dover, DE 19901 (US)**

(72) Inventors:
• **BLAIR, Wade
Emeryville, CA 94608 (US)**
• **BULIK-SULLIVAN, Brendan
Emeryville, CA 94608 (US)**
• **BUSBY, Jennifer
Emeryville, CA 94608 (US)**
• **DERTI, Adnan
Emeryville, CA 94608 (US)**
• **GITLIN, Leonid
Emeryville, CA 94608 (US)**
• **GROTENBREG, Gijsbert
Emeryville, CA 94608 (US)**
• **JOOSS, Karin
Emeryville, CA 94608 (US)**
• **SCALLAN, Ciaran Daniel
Emeryville, CA 94608 (US)**
• **YELENSKY,Roman
Emeryville, CA 94608 (US)**

(74) Representative: **Elkington and Fife LLP
Prospect House
8 Pembroke Road
Sevenoaks, Kent TN13 1XR (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
WO-A1-2015/085233    WO-A1-2016/100975
WO-A1-2018/208856    WO-A2-2014/168874
US-A1- 2009 093 050    US-A1- 2010 286 070
US-A1- 2015 125 465    US-A1- 2015 125 477
US-B2- 9 402 888

- NICOLA BORTHWICK ET AL: "Vaccine-elicited Human T Cells Recognizing Conserved Protein Regions Inhibit HIV-1", MOLECULAR THERAPY, vol. 22, no. 2, 31 October 2013 (2013-10-31), pages 464 - 475, XP055128052, ISSN: 1525-0016, DOI: 10.1038/mt.2013.248
- CASEY AGER ET AL: "31st Annual Meeting and Associated Programs of the Society for Immunotherapy of Cancer (SITC 2016): part two", ILLIAJOURNAL FOR IMMUNOTHERAPY OF C, BIOMED CENTRAL LTD, LONDON, UK, vol. 4, no. 1, 16 November 2016 (2016-11-16), pages 107 - 221, XP021241441, DOI: 10.1186/ S40425-016-0173-6
- GEORGE M WARIMWE ET AL: "Immunogenicity and efficacy of a chimpanzee adenovirus-vectored Rift Valley Fever vaccine in mice", VIROLOGY JOURNAL, vol. 10, no. 1, 1 January 2013 (2013-01-01), GB, pages 349, XP055534311, ISSN: 1743-422X, DOI: 10.1186/1743-422X-10-349
- FEDERICA CAPPUCCINI ET AL: "Immunogenicity and efficacy of the novel cancer vaccine based on simian adenovirus and MVA vectors alone and in combination with PD-1 mAb in a mouse model of prostate cancer", CANCER IMMUNOLOGY, IMMUNOTHERAPY, NIH AUTHOR MANUSCRIPT, vol. 65, no. 6, 1 June 2016 (2016-06-01), Berlin/Heidelberg, pages 701 - 713, XP055570556, ISSN: 0340-7004, DOI: 10.1007/s00262-016-1831-8
- LUIGI AURISICCHIO ET AL: "Immunogenicity and Therapeutic Efficacy of a Dual-Component Genetic Cancer Vaccine Cotargeting Carcinoembryonic Antigen and HER2/ neu in Preclinical Models", HUMAN GENE THERAPY, vol. 25, no. 2, 1 February 2014 (2014-02-01), GB, pages 121 - 131, XP055643329, ISSN: 1043-0342, DOI: 10.1089/hum.2013.103
- SUSAN J MORRIS ET AL: "Simian adenoviruses as vaccine vectors", FUTURE VIROLOGY, vol. 11, no. 9, 1 September 2016 (2016-09-01), UK, pages 649 - 659, XP055571430, ISSN: 1746-0794, DOI: 10.2217/fvl-2016-0070
- LETOURNEAU SVEN ET AL: "Design and Pre-Clinical Evaluation of a Universal HIV-1 Vaccine", PLOS ONE,, vol. 2, no. 10, 3 October 2007 (2007-10-03), pages E984 - 1, XP002599133, ISSN: 1932-6203, DOI: 10.1371/ JOURNAL.PONE.0000984
- S. COLLOCA ET AL: "Vaccine Vectors Derived from a Large Collection of Simian Adenoviruses Induce Potent Cellular Immunity Across Multiple Species", SCIENCE TRANSLATIONAL MEDICINE, vol. 4, no. 115, 4 January 2012 (2012-01-04), US, pages 115ra2 - 115ra2, XP055698922, ISSN: 1946-6234, DOI: 10.1126/ scitranslmed.3002925
- LEVY ET AL: "A melanoma multiepitope polypeptide induces specific CD8+ T-cell response", CELLULAR IMMUNOLOGY, ACADEMIC PRESS, SAN DIEGO, CA, US, vol. 250, no. 1-2, 13 February 2008 (2008-02-13), pages 24 - 30, XP022559220, ISSN: 0008-8749
- VELDERS M P ET AL: "DEFINED FLANKING SPACERS AND ENHANCED PROTEOLYSIS IS ESSENTIAL FOR ERADICATION OF ESTABLISHED TUMORS BY AN EPITOPE STRING DNA VACCINE", THE JOURNAL OF IMMUNOLOGY, AMERICAN ASSOCIATION OF IMMUNOLOGISTS, US, vol. 166, no. 9, 1 May 2001 (2001-05-01), pages 5366 - 5673, XP002974628, ISSN: 0022-1767
- TATSIS N ET AL: "Chimpanzee-origin adenovirus vectors as vaccine carriers", GENE THERAPY, NATURE PUBLISHING GROUP, LONDON, GB, vol. 13, no. 5, 1 March 2006 (2006-03-01), pages 421 - 429, XP002527576, ISSN: 0969-7128, DOI: 10.1038/SJ.GT.3302675

(52) Cooperative Patent Classification (CPC): (Cont.)
C12N 2710/14034; C12N 2710/16134;
C12N 2710/16234

C-Sets
A61K 31/7088, A61K 2300/00;
A61K 31/713, A61K 2300/00

**Description**

**BACKGROUND**

Therapeutic vaccines based on tumor-specific neoantigens hold great promise as a next-generation of personalized cancer immunotherapy. [1-3] Cancers with a high mutational burden, such as non-small cell lung cancer (NSCLC) and melanoma, are particularly attractive targets of such therapy given the relatively greater likelihood of neoantigen generation. [4,5] Early evidence shows that neoantigen-based vaccination can elicit T-cell responses[6] and that neoantigen targeted cell-therapy can cause tumor regression under certain circumstances in selected patients.[7]

US 9402888 B2 describes compositions comprising an adenoviral vector with a first expression cassette encoding a cancer antigen fused within or to a protein that inhibits an immunoinhibitory pathway operatively linked to an expression control sequence; and an adenoviral vector with a second expression cassette comprising a second nucleic acid sequence encoding fibroblast activation protein (FAP) operatively linked to an expression control sequence. In one example, the chimpanzee-derived adenoviral vector AdC68 is prepared, comprising melanoma antigen derived immunogenic epitopes from mutated Braf, Trp-1, Trp-2, and gp100, including CD4+ and CD8+ T cell epitopes, with components separated by conventional linker sequences.

One question for neoantigen vaccine design is which of the many coding mutations present in subject tumors can generate the "best" therapeutic neoantigens, e.g., antigens that can elicit anti-tumor immunity and cause tumor regression.

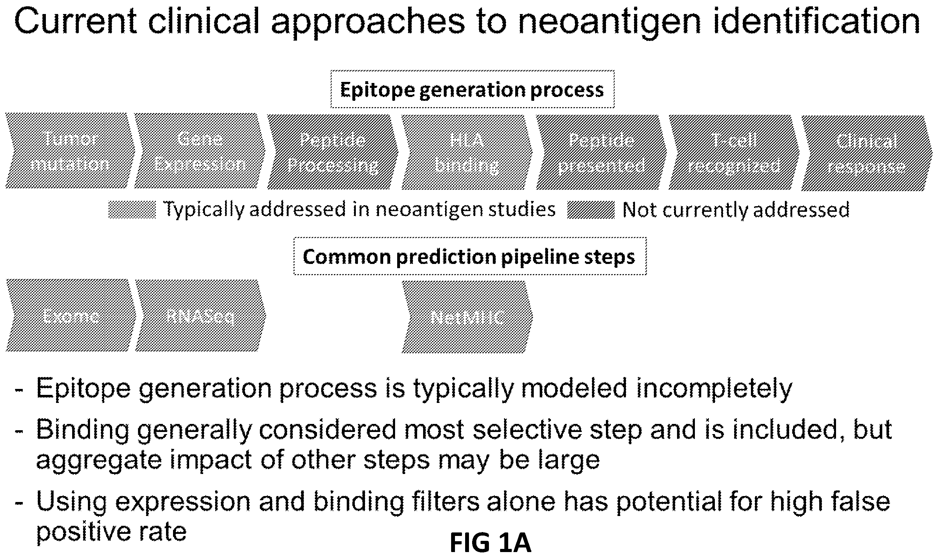

Initial methods have been proposed incorporating mutation-based analysis using next-generation sequencing, RNA gene expression, and prediction of MHC binding affinity of candidate neoantigen peptides [8]. However, these proposed methods can fail to model the entirety of the epitope generation process, which contains many steps (e.g., TAP transport, proteasomal cleavage, and/or TCR recognition) in addition to gene expression and MHC binding[9]. Consequently, existing methods are likely to suffer from reduced low positive predictive value (PPV). (Figure 1A)

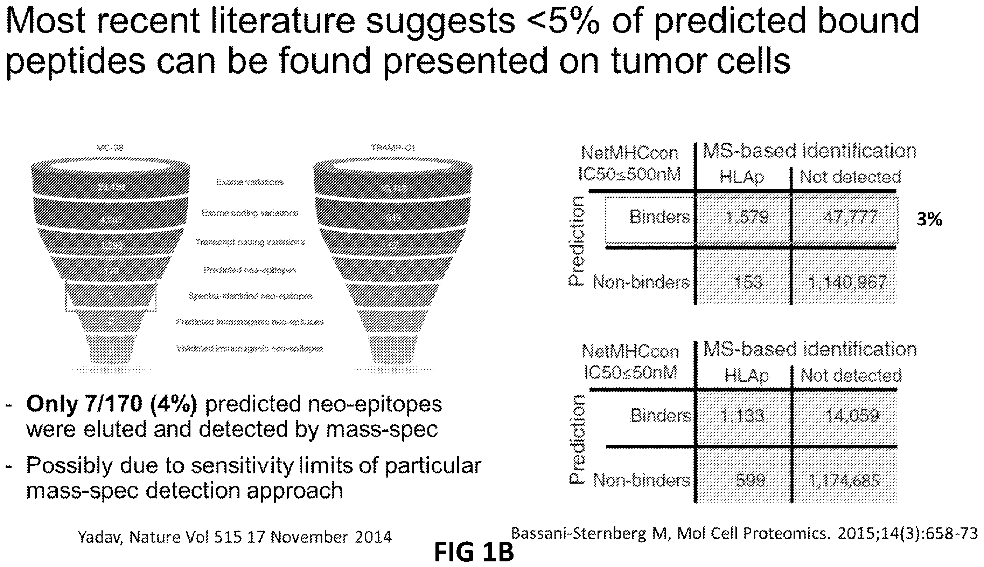

Indeed, analyses of peptides presented by tumor cells performed by multiple groups have shown that <5% of peptides that are predicted to be presented using gene expression and MHC binding affinity can be found on the tumor surface MHC[10,11] (Figure 1B). This low correlation between binding prediction and MHC presentation was further reinforced by recent observations of the lack of predictive accuracy improvement of binding-restricted neoantigens for checkpoint inhibitor response over the number of mutations alone.[12]

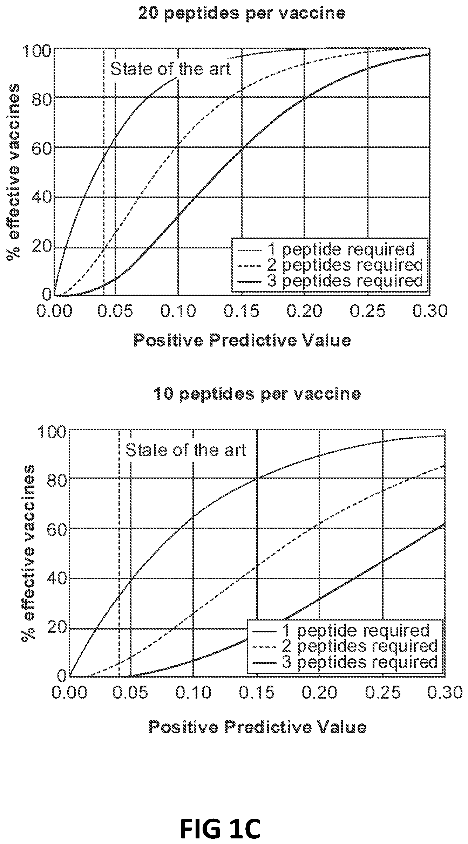

This low positive predictive value (PPV) of existing methods for predicting presentation presents a problem for neoantigen-based vaccine design. If vaccines are designed using predictions with a low PPV, most patients are unlikely to receive a therapeutic neoantigen and fewer still are likely to receive more than one (even assuming all presented peptides are immunogenic). Thus, neoantigen vaccination with current methods is unlikely to succeed in a substantial number of subjects having tumors. (Figure 1C)

Additionally, previous approaches generated candidate neoantigens using only *cis*-acting mutations, and largely neglected to consider additional sources of neo-ORFs, including mutations in splicing factors, which occur in multiple tumor types and lead to aberrant splicing of many genes[13], and mutations that create or remove protease cleavage sites.

Finally, standard approaches to tumor genome and transcriptome analysis can miss somatic mutations that give rise to candidate neoantigens due to suboptimal conditions in library construction, exome and transcriptome capture, sequencing, or data analysis. Likewise, standard tumor analysis approaches can inadvertently promote sequence artifacts or germline polymorphisms as neoantigens, leading to inefficient use of vaccine capacity or auto-immunity risk, respectively.

In addition to the challenges of current neoantigen prediction methods certain challenges also exist with the available vector systems that can be used for neoantigen delivery in humans, many of which are derived from humans. For example, many humans have pre-existing immunity to human viruses as a result of previous natural exposure, and this immunity can be a major obstacle to the use of recombinant human viruses for neoantigen delivery for cancer treatment.

**SUMMARY**

The invention is defined in the appended claims.

A first aspect of the invention is a chimpanzee adenovirus vector comprising a neoantigen cassette, the neoantigen cassette comprising: (1) a plurality of neoantigen-encoding nucleic acid sequences derived from a tumor present within a subject, the plurality comprising: at least two tumor-specific and subject-specific MHC class I neoantigen-encoding nucleic acid sequences each comprising: a. a MHC class I epitope encoding nucleic acid sequence with at least one alteration that makes the encoded peptide sequence distinct from the corresponding peptide sequence encoded by a wild-type nucleic acid sequence, b. a 5' linker sequence, wherein the 5' linker sequence is a native 5' nucleic acid sequence of the MHC I epitope, and wherein the 5' linker sequence encodes a peptide that is at least 2 amino acids in length, and c. a 3' linker sequence, wherein the 3' linker sequence is a native 3' nucleic acid sequence of the MHC I epitope, and wherein the 3' linker sequence encodes a peptide that is at least 2 amino acids in length, wherein at least one neoantigen-encoding nucleic acid sequence in the plurality is linked to a distinct neoantigen-encoding nucleic acid sequence in the plurality by its linker sequence; (2) at least one promoter sequence operably linked to at least one sequence of the plurality, (3) at least one MHC class II antigen-encoding nucleic acid sequence; (4) optionally, at least one GPGPG linker sequence (SEQ ID NO:56); and (5) optionally, at least one polyadenylation sequence..

In some aspects, the vector has an ordered sequence of each element of the neoantigen cassette as described in a formula, from 5' to 3', comprising:

$$P_a\text{-}(L5_b\text{-}N_c\text{-}L3_d)_X\text{-}(G5_e\text{-}U_f)_Y\text{-}G3_g\text{-}A_h$$

wherein P comprises the at least one promoter sequence operably linked to at least one sequence of the plurality, where $a = 1$, N comprises one of the MHC class I epitope encoding nucleic acid sequence with at least one alteration that makes the encoded peptide sequence distinct from the corresponding peptide sequence encoded by the wild-type nucleic acid sequence, where $c = 1$, L5 comprises the 5' linker sequence, where $b = 1$, L3 comprises the 3' linker sequence, where $d = 1$, G5 comprises a GPGPG-encoding linker sequence (SEQ ID NO: 56), where $e = 0$ or 1, G3 comprises a GPGPG-encoding linker sequences (SEQ ID NO: 56), where $g = 0$ or 1, U comprises one of the at least one MHC class II antigen-encoding nucleic acid sequence, where $f = 1$, A comprises at least one polyadenylation sequence, where $h = 0$ or 1, $X = 2$ to 400, where for each X the corresponding Nc is a distinct MHC class I epitope encoding nucleic acid sequence, and $Y = 1\text{-}2$, where for each Y the corresponding Uf is a distinct MHC class II antigen-encoding nucleic acid sequence. In a particular aspect, $b = 1$, $d = 1$, $e = 1$, $g = 1$, $h = 1$, $X = 20$, $Y = 2$, P is a CMV promoter sequence, each N encodes a MHC class I epitope 7-15 amino acids in length, L5 is the native 5' nucleic acid sequence of the MHC I epitope, wherein the 5' linker sequence encodes a peptide that is at least 5 amino acids in length, L3 is the native 3' nucleic acid sequence of the MHC I epitope, wherein the 3' linker sequence encodes a peptide that is at least 5 amino acids in length, U is each of a PADRE class II sequence and a Tetanus toxoid MHC class II sequence, the chimpanzee adenovirus vector comprises a modified ChAdV68 sequence comprising the sequence of SEQ ID NO: 1 having an E1 deletion from nucleotide 577 to nucleotide 3403 and an E3 deletion from nucleotide 27,125 to nucleotide 31,825 and the neoantigen cassette is inserted within the E1 deletion, and each of the MHC class I neoantigen-encoding nucleic acid sequences encodes a polypeptide that is 25 amino acids in length.

In some aspects, at least 1, 2, or optionally 3 neoantigen-encoding nucleic acid sequences in the plurality encode polypeptide sequences or portions thereof that is presented by MHC class I on the tumor cell surface.

In some aspects, at least one sequence in the plurality is linked, operably or directly, to a separate or contiguous sequence that enhances the expression, stability, cell trafficking, processing and presentation, and/or immunogenicity of the plurality. In some aspects, the separate or contiguous sequence comprises at least one of: a ubiquitin sequence, a ubiquitin sequence modified to increase proteasome targeting (e.g., the ubiquitin sequence contains a Gly to Ala substitution at position 76), an immunoglobulin signal sequence (e.g., IgK), a major histocompatibility class I sequence, lysosomal-associated membrane protein (LAMP)-1, human dendritic cell lysosomal-associated membrane protein, and a major histocompatibility class II sequence; optionally wherein the ubiquitin sequence modified to increase proteasome targeting is A76.

In some aspects, at least one of the neoantigen-encoding nucleic acid sequences in the plurality encodes a polypeptide sequence or portion thereof that has increased binding affinity to its corresponding MHC allele relative to the translated, corresponding wild-type nucleic acid sequence. In some aspects, at least one of the neoantigen-encoding nucleic acid sequences in the plurality encodes a polypeptide sequence or portion thereof that has increased binding stability to its corresponding MHC allele relative to the translated, corresponding wild-type, parental nucleic acid sequence. In some aspects, at least one of the neoantigen-encoding nucleic acid sequences in the plurality encodes a polypeptide sequence or portion thereof that has an increased likelihood of presentation on its corresponding MHC allele relative to the translated, corresponding wild-type, parental nucleic acid sequence.

In some aspects, at least one alteration comprises a point mutation, a frameshift mutation, a non-frameshift mutation, a deletion mutation, an insertion mutation, a splice variant, a genomic rearrangement, or a proteasome-generated spliced antigen.

In some aspects, the tumor is selected from the group consisting of: lung cancer, melanoma, breast cancer, ovarian cancer, prostate cancer, kidney cancer, gastric cancer, colon cancer, testicular cancer, head and neck cancer, pancreatic cancer, brain cancer, B-cell lymphoma, acute myelogenous leukemia, chronic myelogenous leukemia, chronic lymphocytic leukemia, T cell lymphocytic leukemia, non-small cell lung cancer, and small cell lung cancer.

In some aspects, expression of each sequence in the plurality is driven by the at least one promoter.

In some aspects, the plurality comprises at least 2, 3, 4, 5, 6, 7, 8, 9, or 10 nucleic acid sequences. In some aspects, the plurality comprises at least 11, 12, 13, 14, 15, 16, 17, 18, 19, 20 or up to 400 nucleic acid sequences. In some aspects, the plurality comprises at least 2-400 nucleic acid sequences and wherein at least two of the neoantigen-encoding nucleic acid sequences in the plurality encode polypeptide sequences or portions thereof that are presented by MHC I on the tumor cell surface. In some aspects, the plurality comprises at least 2-400 nucleic acid sequences and wherein, when administered to the subject and translated, at least one of the neoantigens are presented on antigen presenting cells resulting in an immune response targeting at least one of the neoantigens on the tumor cell surface. In some aspects, the plurality comprises at least 2-400 MHC class I and/or class II neoantigen-encoding nucleic acid sequences, wherein, when administered to the subject and translated, at least one of the MHC class I or class II neoantigens are presented on antigen presenting cells resulting in an immune response targeting at least one of the neoantigens on the tumor cell surface, and optionally wherein the expression of each of the at least 2-400 MHC class I or class II neoantigen-encoding nucleic acid sequences is driven by the at least one promoter.

In some aspects, each MHC class I neoantigen-encoding nucleic acid sequence encodes a polypeptide sequence between 8 and 35 amino acids in length, optionally 9-17, 9-25, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids in length.

In some aspects, the at least one MHC class II antigen-encoding nucleic acid sequence comprises at least one MHC class II neoantigen-encoding nucleic acid sequence that comprises at least one alteration that makes the encoded peptide sequence distinct from the corresponding peptide sequence encoded by a wild-type nucleic acid sequence. In some aspects, the at least one MHC class II antigen-encoding nucleic acid sequence is 12-20, 12, 13, 14, 15, 16, 17, 18, 19, 20, or 20-40 amino acids in length. In some aspects, the at least one MHC class II antigen-encoding nucleic acid sequence comprises at least one universal MHC class II antigen-encoding nucleic acid sequence, optionally wherein the at least one universal sequence comprises at least one of Tetanus toxoid and PADRE.

In some aspects, the at least one promoter sequence is inducible. In some aspects, the at least one promoter sequence is non-inducible. In some aspects, the at least one promoter sequence is a CMV, SV40, EF-1, RSV, PGK, or EBV promoter sequence.

In some aspects, the neoantigen cassette further comprises at least one poly-adenylation (polyA) sequence operably linked to at least one of the sequences in the plurality, optionally wherein the poly A sequence is located 3' of the at least one sequence in the plurality. In some aspects, the polyA sequence comprises an SV40 poly A sequence. In some aspects, the neoantigen cassette further comprises at least one of: an intron sequence, a woodchuck hepatitis virus posttranscriptional regulatory element (WPRE) sequence, an internal ribosome entry sequence (IRES) sequence, or a sequence in the 5' or 3' non-coding region known to enhance the nuclear export, stability, or translation efficiency of mRNA that is operably linked to at least one of the sequences in the plurality. In some aspects, the neoantigen cassette further comprises a reporter gene, including but not limited to, green fluorescent protein (GFP), a GFP variant, secreted alkaline phosphatase, luciferase, or a luciferase variant.

In some aspects, the vector further comprises one or more nucleic acid sequences encoding at least one immune modulator.

In some aspects, the immune modulator is an anti-CTLA4 antibody or an antigen-binding fragment thereof, an anti-PD-1 antibody or an antigen-binding fragment thereof, an anti-PD-L1 antibody or an antigen-binding fragment thereof, an anti-4-1BB antibody or an antigen-binding fragment thereof, or an anti-OX-40 antibody or an antigen-binding fragment thereof. In some aspects, the antibody or antigen-binding fragment thereof is a Fab fragment, a Fab' fragment, a single chain Fv (scFv), a single domain antibody (sdAb) either as single specific or multiple specificities linked together (e.g., camelid antibody domains), or full-length single-chain antibody (e.g., full-length IgG with heavy and light chains linked by a flexible linker). In some aspects, the heavy and light chain sequences of the antibody are a contiguous sequence separated by either a self-cleaving sequence such as 2A or IRES; or the heavy and light chain sequences of the antibody are linked by a flexible linker such as consecutive glycine residues.

In some aspects, the immune modulator is a cytokine. In some aspects, the cytokine is at least one of IL-2, IL-7, IL-12, IL-15, or IL-21 or variants thereof of each.

In some aspects, the vector is a chimpanzee adenovirus C68 vector. In some aspects, the vector comprises the sequence set forth in SEQ ID NO: 1. In some aspects, vector comprises the sequence set forth in SEQ ID NO: 1, except that the sequence is fully deleted or functionally deleted in at least one gene selected from the group consisting of the chimpanzee adenovirus E1A, E1B, E2A, E2B, E3, E4, L1, L2, L3, L4, and L5 genes of the sequence set forth in SEQ ID NO: 1, optionally wherein the sequence is fully deleted or functionally deleted in: (1) E1A and E1B; (2) E1A, E1B, and E3; or (3) E1A, E1B, E3, and E4 of the sequence set forth in SEQ ID NO: 1. In some aspects, the vector comprises a gene or regulatory sequence obtained from the sequence of SEQ ID NO: 1, optionally wherein the gene is selected from the group consisting of the chimpanzee adenovirus inverted terminal repeat (ITR), E1A, E1B, E2A, E2B, E3, E4, L1, L2, L3, L4, and L5 genes of the sequence set forth in SEQ ID NO: 1.

In some aspects, the neoantigen cassette is inserted in the vector at a deleted chimpanzee adenovirus region that allows incorporation of the neoantigen cassette, wherein the deleted chimpanzee adenovirus region may be an E1 region or a E3 region.

In some aspects, the vector is generated from one of a first generation, a second generation, or a helper-dependent adenoviral vector.

In some aspects, the adenovirus vector the vector comprises one or more deletions between base pair number 577 and 3403 or between base pair 456 and 3014, and optionally wherein the vector further comprises one or more deletions between base pair 27,125 and 31,825 or between base pair 27,816 and 31,333 of the sequence set forth in SEQ ID NO: 1. In some aspects, the adenovirus vector further comprises one or more deletions between base pair number 3957 and 10346, base pair number 21787 and 23370, and base pair number 33486 and 36193 of the sequence set forth in SEQ ID NO: 1.

In some aspects, the at least two MHC class I neoantigen-encoding nucleic acid sequences are selected by performing the steps of: obtaining at least one of exome, transcriptome, or whole genome tumor nucleotide sequencing data from the tumor, wherein the tumor nucleotide sequencing data is used to obtain data representing peptide sequences of each of a set of neoantigens; inputting the peptide sequence of each neoantigen into a presentation model to generate a set of numerical likelihoods that each of the neoantigens is presented by one or more of the MHC alleles on the tumor cell surface of the tumor, the set of numerical likelihoods having been identified at least based on received mass spectrometry data; and selecting a subset of the set of neoantigens based on the set of numerical likelihoods to generate a set of selected neoantigens which are used to generate the at least two MHC class I neoantigen-encoding nucleic acid sequences.

In some aspects, each of the MHC class I epitope encoding nucleic acid sequences are selected by performing the steps of: obtaining at least one of exome, transcriptome, or whole genome tumor nucleotide sequencing data from the tumor, wherein the tumor nucleotide sequencing data is used to obtain data representing peptide sequences of each of a set of neoantigens; inputting the peptide sequence of each neoantigen into a presentation model to generate a set of numerical likelihoods that each of the neoantigens is presented by one or more of the MHC alleles on the tumor cell surface of the tumor, the set of numerical likelihoods having been identified at least based on received mass spectrometry data; and selecting a subset of the set of neoantigens based on the set of numerical likelihoods to generate a set of selected neoantigens which are used to generate the at least two MHC class I neoantigen-encoding nucleic acid sequences.

In some aspects, a number of the set of selected neoantigens is 2-20.

In some aspects, the presentation model represents dependence between: presence of a pair of a particular one of the MHC alleles and a particular amino acid at a particular position of a peptide sequence; and likelihood of presentation on the tumor cell surface, by the particular one of the MHC alleles of the pair, of such a peptide sequence comprising the particular amino acid at the particular position.

In some aspects, selecting the set of selected neoantigens comprises selecting neoantigens that have an increased likelihood of being presented on the tumor cell surface relative to unselected neoantigens based on the presentation model. In some aspects, selecting the set of selected neoantigens comprises selecting neoantigens that have an increased likelihood of being capable of inducing a tumor-specific immune response in the subject relative to unselected neoantigens based on the presentation model. In some aspects, selecting the set of selected neoantigens comprises selecting neoantigens that have an increased likelihood of being capable of being presented to naive T cells by professional antigen presenting cells (APCs) relative to unselected neoantigens based on the presentation model, optionally wherein the APC is a dendritic cell (DC). In some aspects, selecting the set of selected neoantigens comprises selecting neoantigens that have a decreased likelihood of being subject to inhibition via central or peripheral tolerance relative to unselected neoantigens based on the presentation model. In some aspects, selecting the set of selected neoantigens comprises selecting neoantigens that have a decreased likelihood of being capable of inducing an autoimmune response to normal tissue in the subject relative to unselected neoantigens based on the presentation model. In some aspects, exome or transcriptome nucleotide sequencing data is obtained by performing sequencing on the tumor tissue. In some aspects, the sequencing is next generation sequencing (NGS) or any massively parallel sequencing approach.

In some aspects, the neoantigen cassette comprises junctional epitope sequences formed by adjacent sequences in the neoantigen cassette. In some aspects, the at least one or each junctional epitope sequence has an affinity of greater than 500 nM for MHC. In some aspects, each junctional epitope sequence is non-self. In some aspects, the neoantigen cassette does not encode a non-therapeutic MHC class I or class II epitope nucleic acid sequence comprising a translated, wild-type nucleic acid sequence, wherein the non-therapeutic epitope is predicted to be displayed on an MHC allele of the subject. In some aspects, the non-therapeutic predicted MHC class I or class II epitope sequence is a junctional epitope sequence formed by adjacent sequences in the neoantigen cassette. In some aspects, the prediction in based on presentation likelihoods generated by inputting sequences of the non-therapeutic epitopes into a presentation model. In some aspects, an order of the plurality of antigen-encoding nucleic acid sequences in the neoantigen cassette is determined by a series of steps comprising: 1. generating a set of candidate neoantigen cassette sequences corresponding to different orders of the plurality of antigen-encoding nucleic acid sequences; 2. determining, for each candidate neoantigen cassette sequence, a presentation score based on presentation of non-therapeutic epitopes in the candidate neoantigen cassette sequence; and 3. selecting a candidate cassette sequence associated with a presentation score below a predetermined threshold as the neoantigen cassette sequence for a neoantigen vaccine.

Also disclosed herein is a pharmaceutical composition comprising a ChAd-based vector as defined in the claims and a pharmaceutically acceptable carrier. In some aspects, the composition further comprises an adjuvant. In some aspects, the composition further comprises an immune modulator. In some aspects, immune modulator is an anti-CTLA4 antibody or an antigen-binding fragment thereof, an anti-PD-1 antibody or an antigen-binding fragment thereof, an anti-PD-L1 antibody or an antigen-binding fragment thereof, an anti-4-1BB antibody or an antigen-binding fragment thereof, or an anti-OX-40 antibody or an antigen-binding fragment thereof.

Also disclosed herein are the vectors or pharmaceutical compositions as defined in the claims for use in a method for treating a subject with cancer, the method comprising administering to the subject a vector disclosed herein or a pharmaceutical composition disclosed herein. In some aspects, the vector or composition is administered intramuscularly (IM), intradermally (ID), or subcutaneously (SC). In some aspects, the method further comprises administering to the subject an immune modulator, optionally wherein the immune modulator is administered before, concurrently with, or after administration of the vector or pharmaceutical composition. In some aspects, the immune modulator is an anti-CTLA4 antibody or an antigen-binding fragment thereof, an anti-PD-1 antibody or an antigen-binding fragment thereof, an anti-PD-L1 antibody or an antigen-binding fragment thereof, an anti-4-1BB antibody or an antigen-binding fragment thereof, or an anti-OX-40 antibody or an antigen-binding fragment thereof. In some aspects, the immune modulator is administered intravenously (IV), intramuscularly (IM), intradermally (ID), or subcutaneously (SC). In some aspects, wherein the subcutaneous administration is near the site of the vector or composition administration or in close proximity to one or more vector or composition draining lymph nodes.

In some aspects, the method further comprises administering to the subject a second vaccine composition. In some aspects, the second vaccine composition is administered prior to the administration of the vector or the pharmaceutical composition of any of the above vectors or compositions. In some aspects, the second vaccine composition is administered subsequent to the administration of the vector or the pharmaceutical composition of any of the above vectors or compositions. In some aspects, the second vaccine composition is the same as the vector or the pharmaceutical composition of any of the above vectors or compositions. In some aspects, the second vaccine composition is different from the vector or the pharmaceutical composition of any of the above vectors or compositions. In some aspects, the second vaccine composition comprises a self-replicating RNA (srRNA) vector encoding a plurality of neoantigen-encoding nucleic acid sequences. In some aspects, the plurality of neoantigen-encoding nucleic acid sequences encoded by the srRNA vector is the same as the plurality of neoantigen-encoding nucleic acid sequences of any of the above vector claims.

Also disclosed herein is a method of manufacturing a vector as defined in the claims, the method comprising: obtaining a plasmid sequence comprising the at least one promoter sequence and the neoantigen cassette; transfecting the plasmid sequence into one or more host cells; and isolating the vector from the one or more host cells.

In some aspects, isolating comprises: lysing the host cell to obtain a cell lysate comprising the vector; and purifying the vector from the cell lysate and optionally also from media used to culture the host cell.

In some aspects, the plasmid sequence is generated using one of the following; DNA recombination or bacterial recombination or full genome DNA synthesis or full genome DNA synthesis with amplification of synthesized DNA in bacterial cells. In some aspects, the one or more host cells are at least one of CHO, HEK293 or variants thereof, 911, HeLa, A549, LP-293, PER.C6, and AE1-2a cells. In some aspects, purifying the vector from the cell lysate involves one or more of chromatographic separation, centrifugation, virus precipitation, and filtration.

**BRIEF DESCRIPTION OF THE SEVERAL VIEWS OF THE DRAWINGS**

These and other features, aspects, and advantages of the present invention will become better understood with regard to the following description, and accompanying drawings, where:

Figure (FIG.) 1A shows current clinical approaches to neoantigen identification.
FIG. 1B shows that <5% of predicted bound peptides are presented on tumor cells.
FIG. 1C shows the impact of the neoantigen prediction specificity problem.

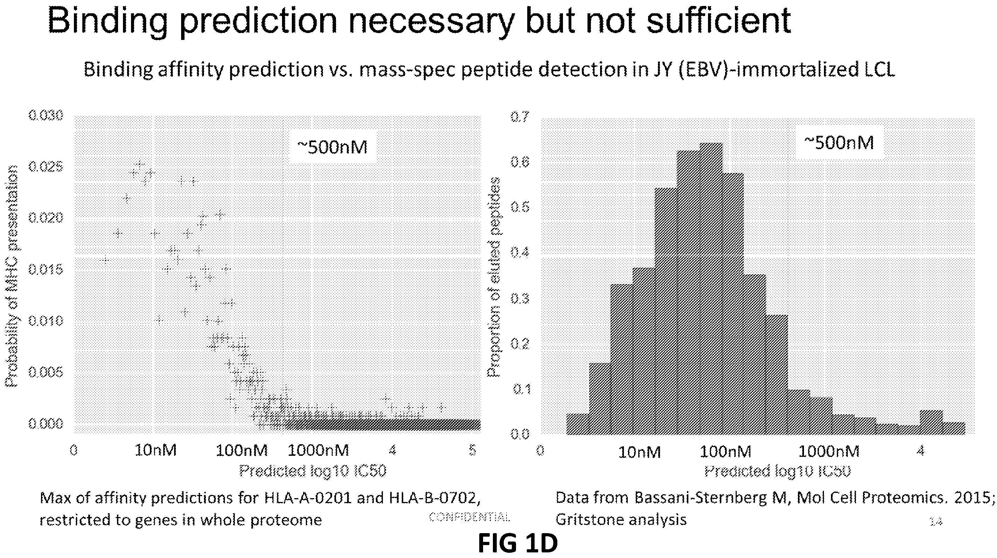

FIG. 1D shows that binding prediction is not sufficient for neoantigen identification.

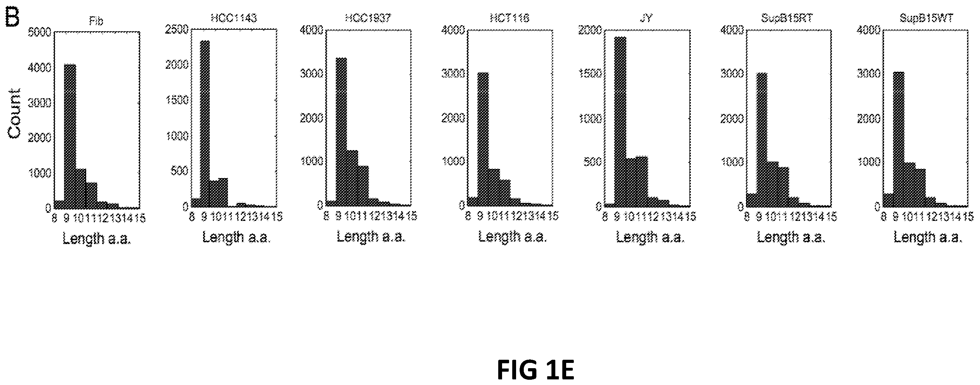

FIG. 1E shows probability of MHC-I presentation as a function of peptide length.

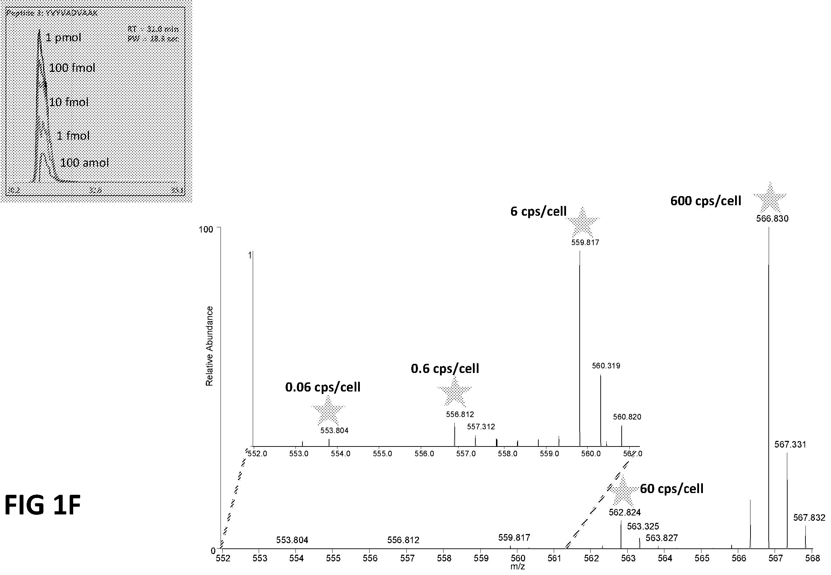

FIG. 1F shows an example peptide spectrum generated from Promega's dynamic range standard. Figure discloses SEQ ID NO: 59.

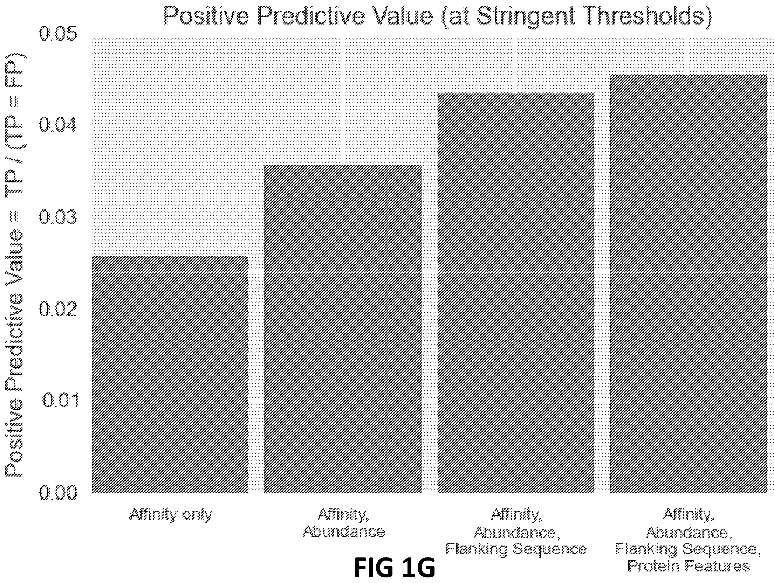

FIG. 1G shows how the addition of features increases the model positive predictive value.

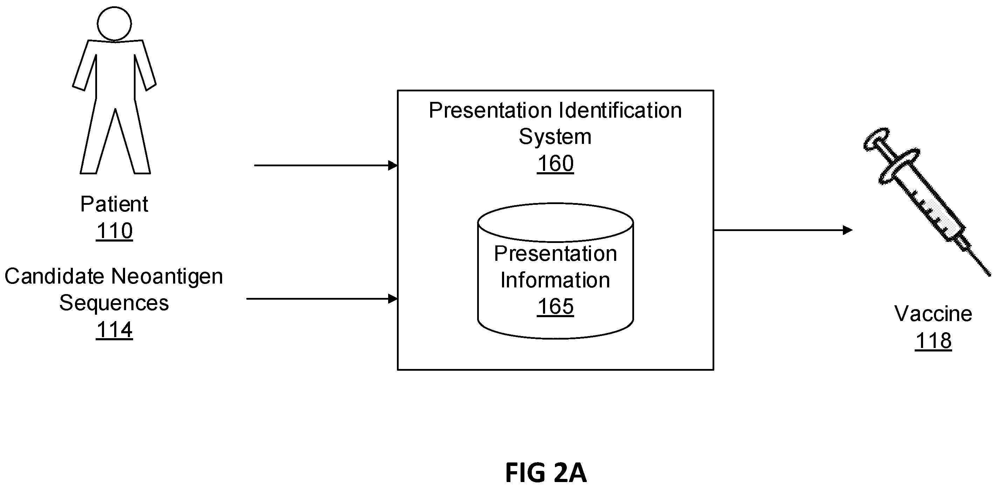

FIG. 2A is an overview of an environment for identifying likelihoods of peptide presentation in patients, in accordance with an embodiment.

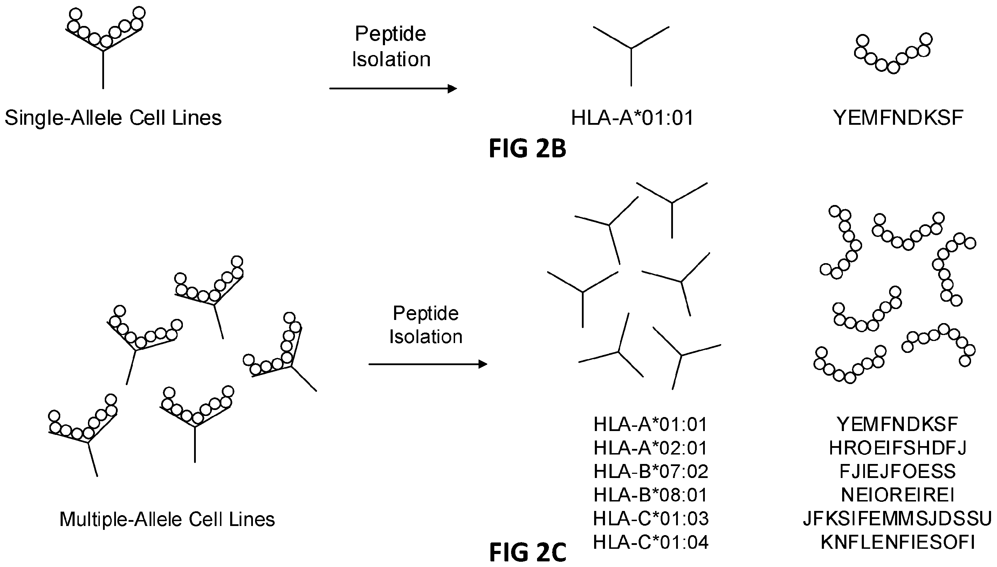

FIG. 2B and FIG. 2C illustrate a method of obtaining presentation information, in accordance with an embodiment. FIG. 2B discloses SEQ ID NO: 61. FIG. 2C discloses SEQ ID NOS 61-66, respectively, in order of appearance.

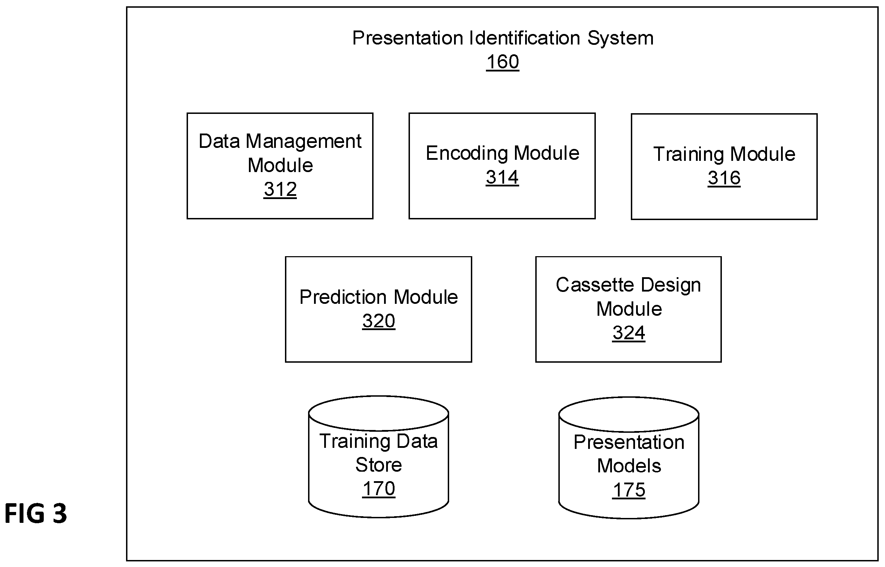

FIG. 3 is a high-level block diagram illustrating the computer logic components of the presentation identification system, according to one embodiment.

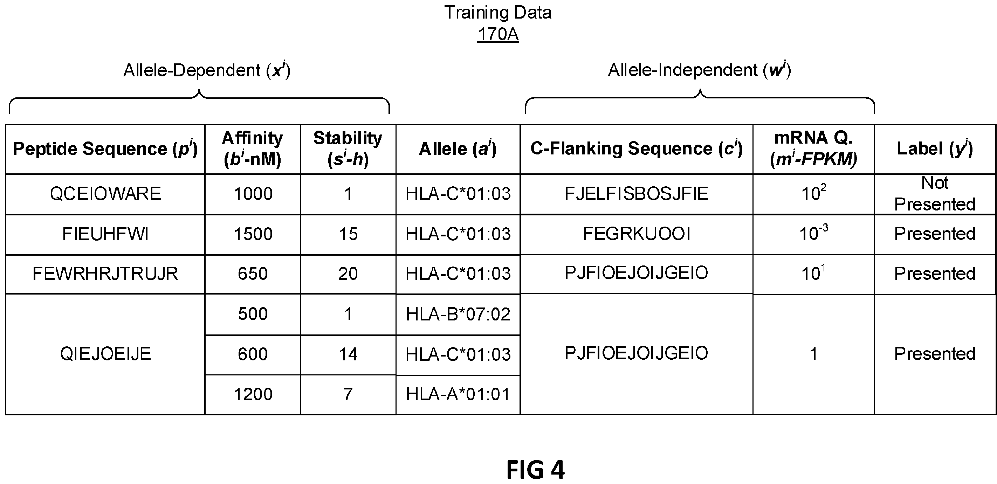

FIG. 4 illustrates an example set of training data, according to one embodiment. Figure discloses Peptide Sequences as SEQ ID NOS 68-71 and C-Flanking Sequences as SEQ ID NOS 72 and 154-155, respectively, in order of appearance.

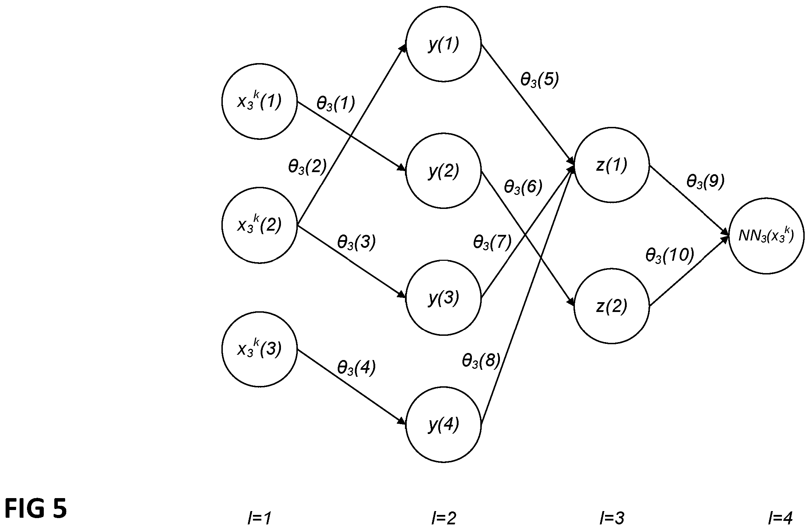

**FIG. 5** illustrates an example network model in association with an MHC allele.

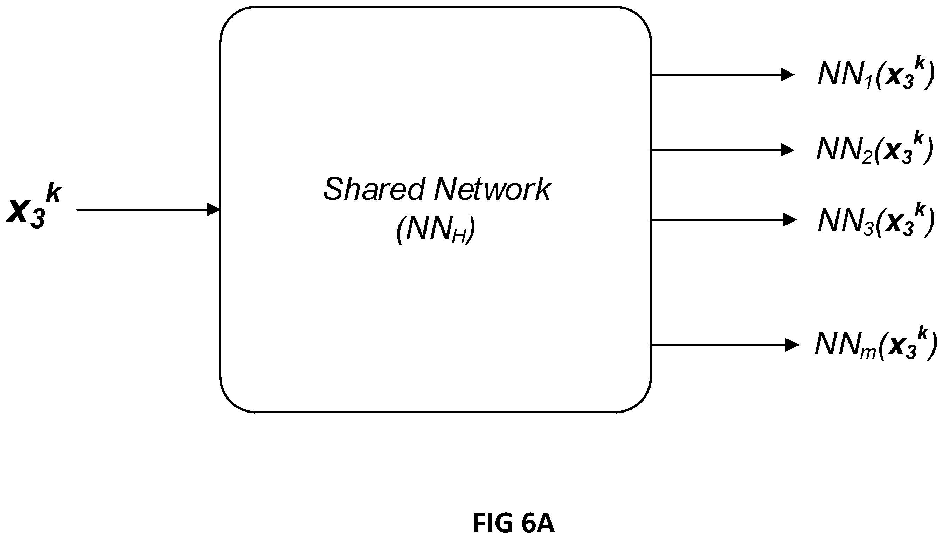

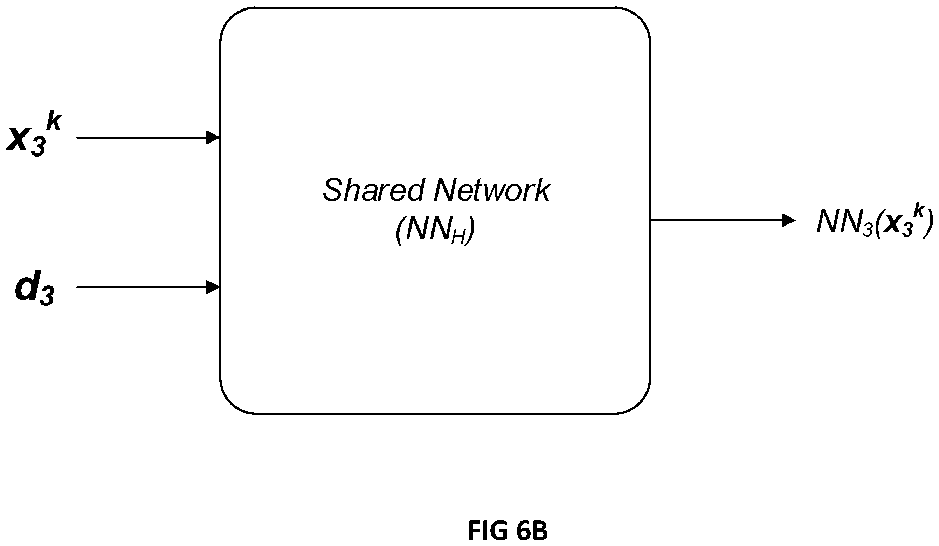

**FIG. 6** illustrates an example network model shared by MHC alleles.

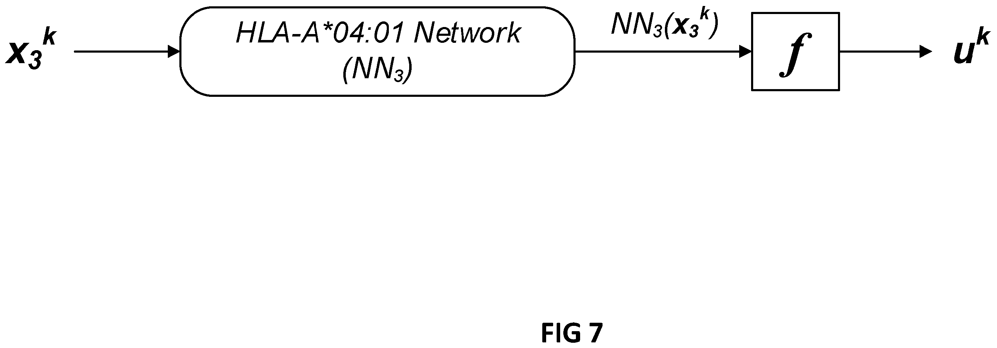

**FIG. 7** illustrates generating a presentation likelihood for a peptide in association with an MHC allele using an example network model.

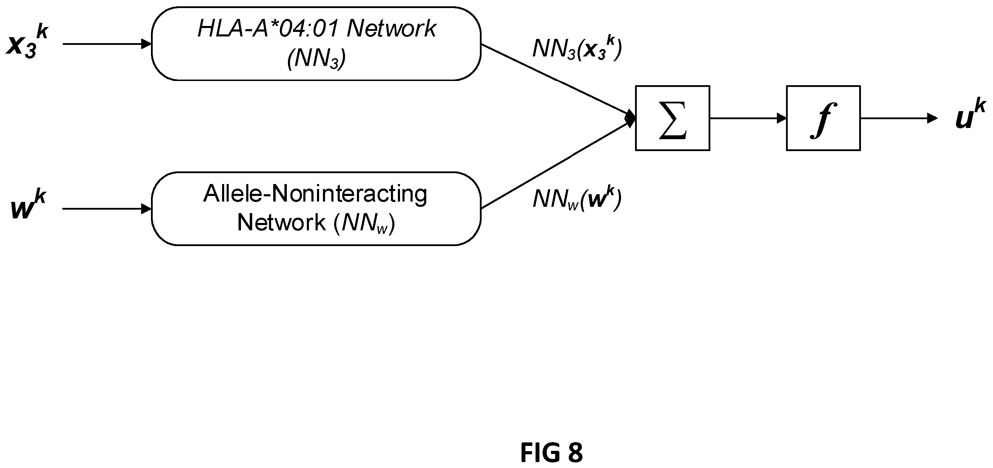

**FIG. 8** illustrates generating a presentation likelihood for a peptide in association with a MHC allele using example network models.

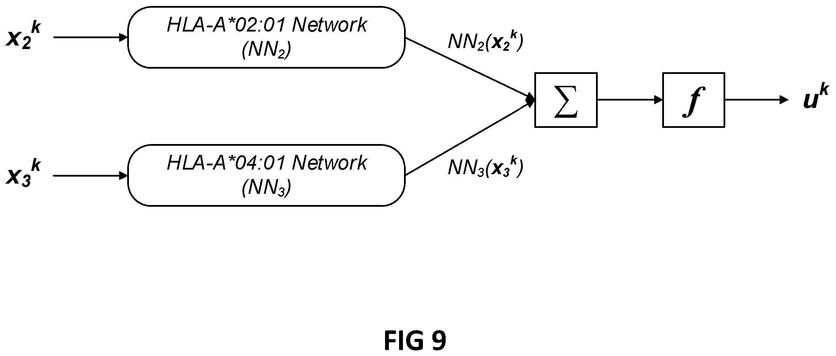

**FIG. 9** illustrates generating a presentation likelihood for a peptide in association with MHC alleles using example network models.

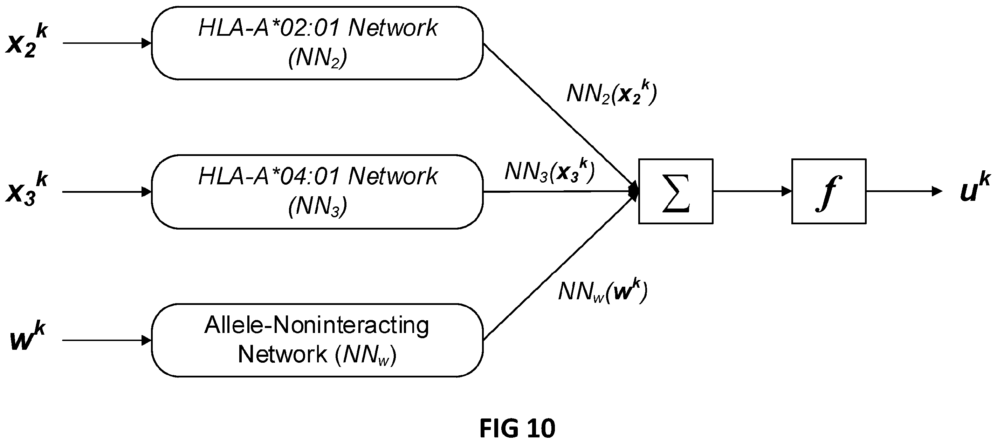

**FIG. 10** illustrates generating a presentation likelihood for a peptide in association with MHC alleles using example network models.

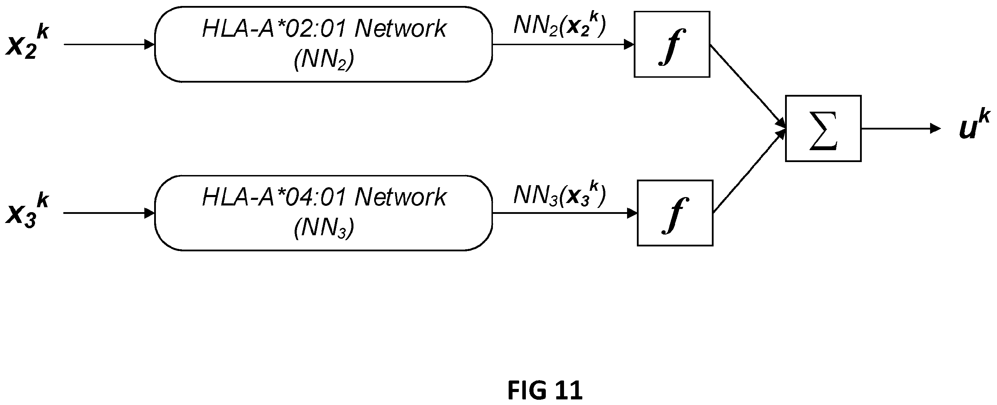

**FIG. 11** illustrates generating a presentation likelihood for a peptide in association with MHC alleles using example network models.

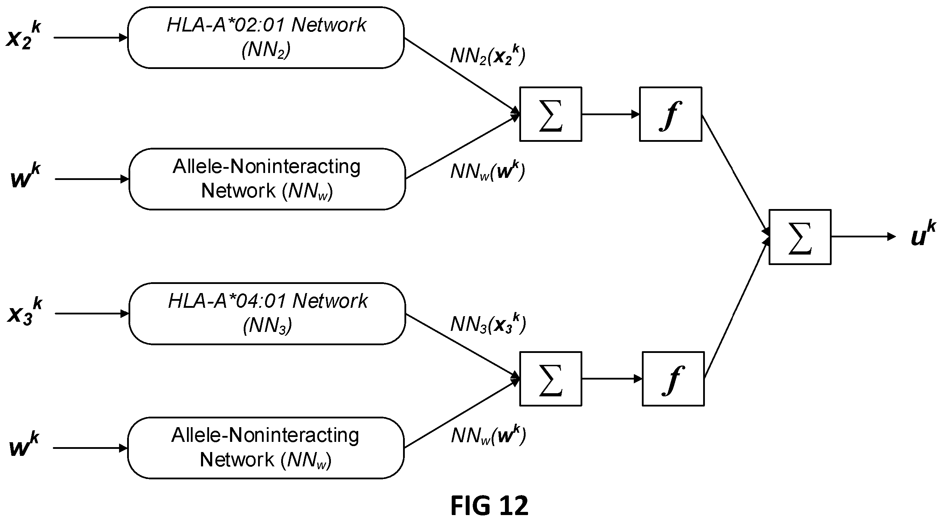

**FIG. 12** illustrates generating a presentation likelihood for a peptide in association with MHC alleles using example network models.

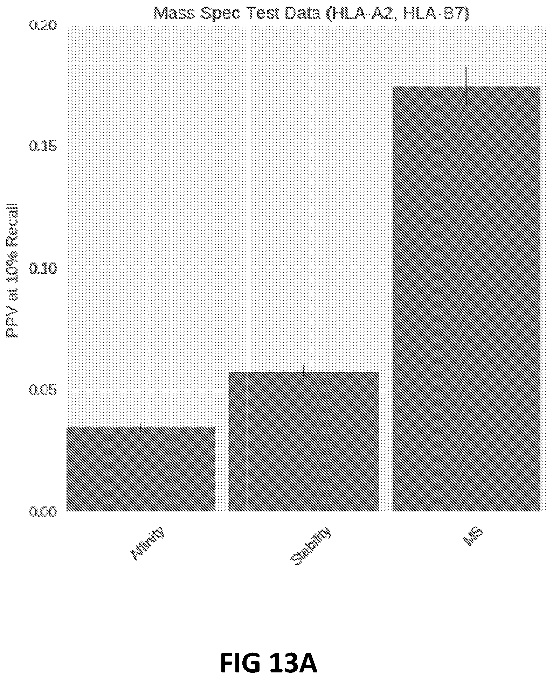

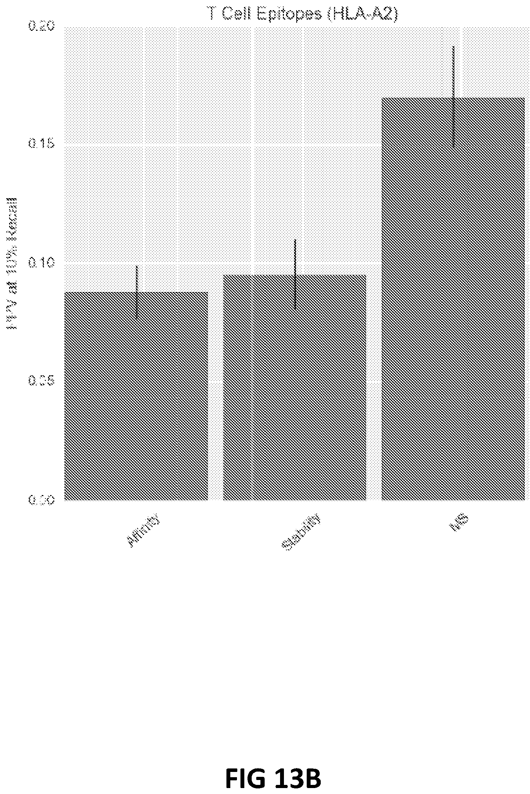

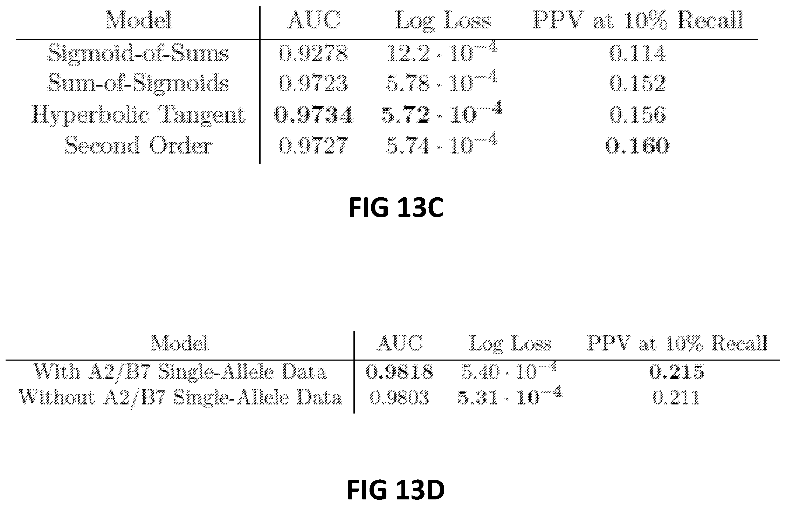

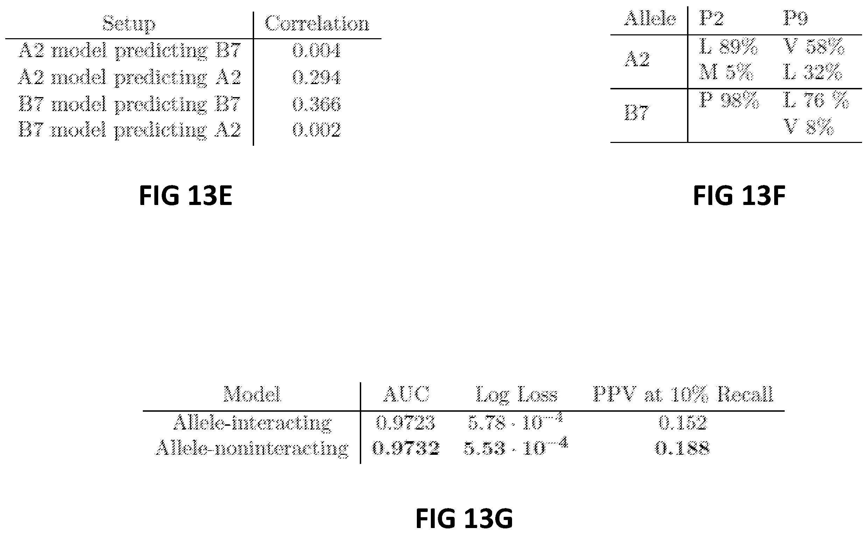

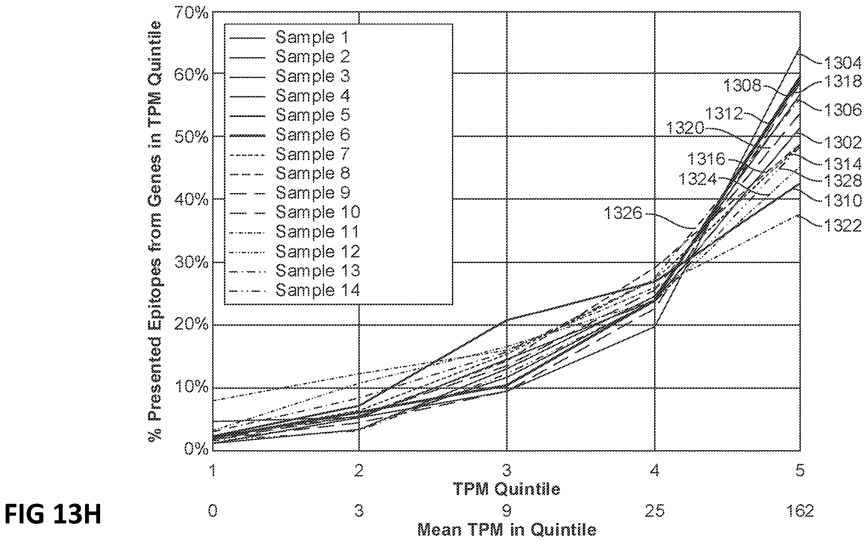

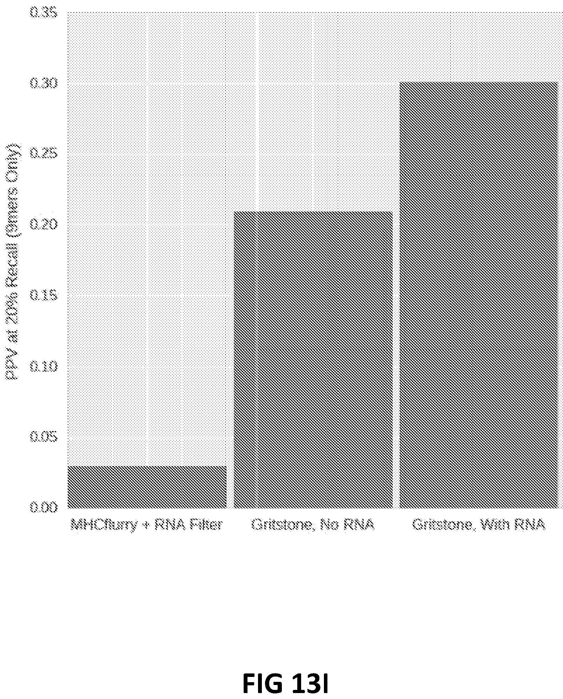

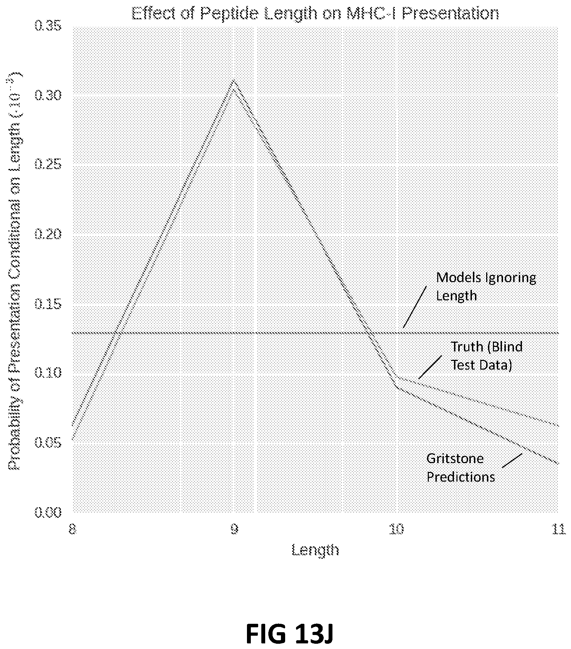

**FIG. 13** illustrates performance results of various example presentation models.

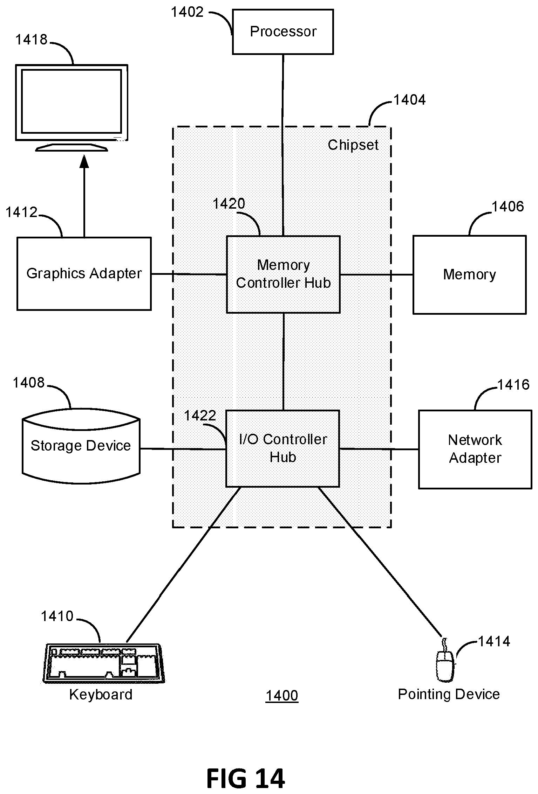

**FIG. 14** illustrates an example computer for implementing the entities shown in FIGS. 1 and 3.

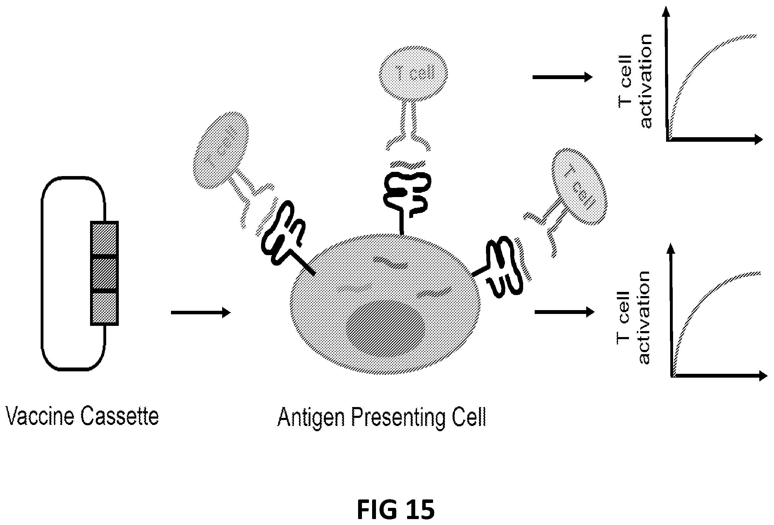

**FIG. 15** illustrates development of an *in vitro* T cell activation assay. Schematic of the assay in which the delivery of a vaccine cassette to antigen presenting cells, leads to expression, processing and MHC-restricted presentation of distinct peptide antigens. Reporter T cells engineered with T cell receptors that match the specific peptide-MHC combination become activated resulting in luciferase expression.

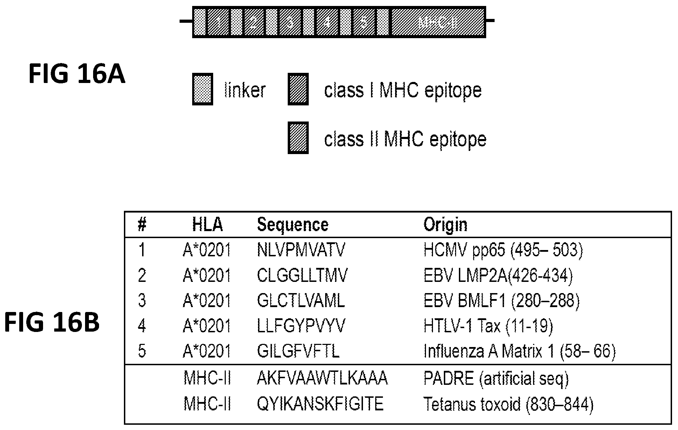

**FIG 16A** illustrates evaluation of linker sequences in short cassettes and shows five class I MHC restricted epitopes (epitopes 1 through 5) concatenated in the same position relative to each other followed by two universal class II MHC epitopes (MHC-II). Various iterations were generated using different linkers. In some cases the T cell epitopes are directly linked to each other. In others, the T cell epitopes are flanked on one or both sides by its natural sequence. In other iterations, the T cell epitopes are linked by the non-natural sequences AAY, RR, and DPP.

**FIG 16B** illustrates evaluation of linker sequences in short cassettes and shows sequence information on the T cell epitopes embedded in the short cassettes. Figure discloses SEQ ID NOS 128-129, 132, 131, 130, 49 and 156, respectively, in order of appearance.

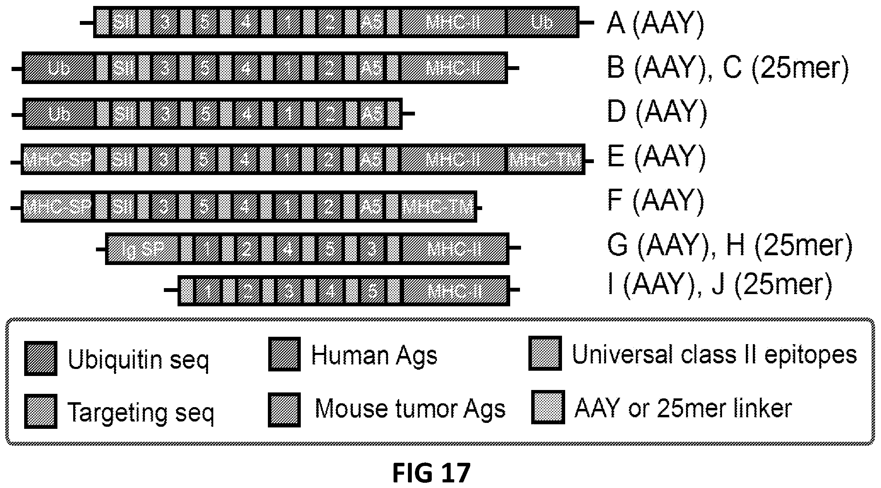

**FIG. 17** illustrates evaluation of cellular targeting sequences added to model vaccine cassettes. The targeting cassettes extend the short cassette designs with ubiquitin (Ub), signal peptides (SP) and/or transmembrane (TM) domains, feature next to the five marker human T cell epitopes (epitopes 1 through 5) also two mouse T cell epitopes SIINFEKL (SII) (SEQ ID NO: 57) and SPSYAYHQF (A5) (SEQ ID NO: 58), and use either the non natural linker AAY- or natural linkers flanking the T cell epitopes on both sides (25mer) .

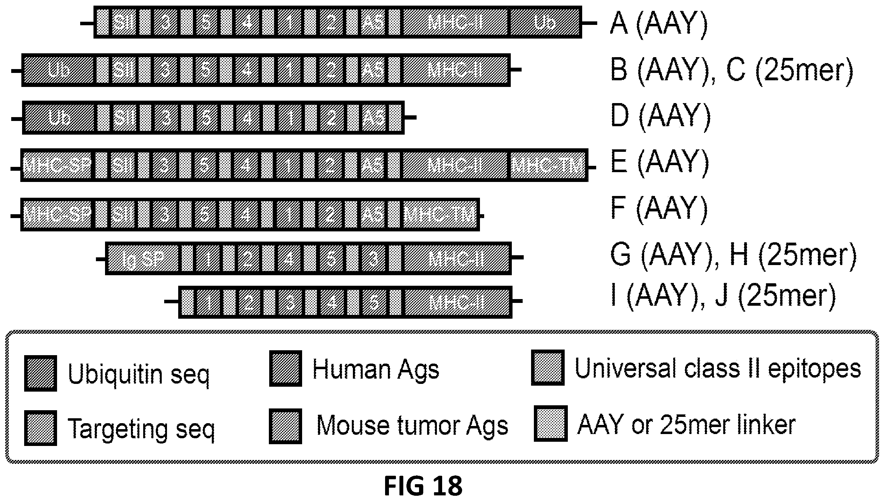

**FIG. 18** illustrates *in vivo* evaluation of linker sequences in short cassettes. A) Experimental design of the *in vivo* evaluation of vaccine cassettes using HLA-A2 transgenic mice.

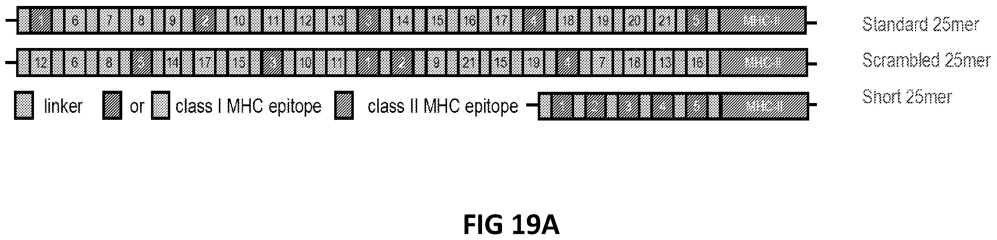

**FIG. 19A** illustrates *in vivo* evaluation of the impact of epitope position in long 21-mer cassettes and shows the design of long cassettes entails five marker class I epitopes (epitopes 1 through 5) contained in their 25-mer natural sequence (linker = natural flanking sequences), spaced with additional well-known T cell class I epitopes (epitopes 6 through 21) contained in their 25-mer natural sequence, and two universal class II epitopes (MHC-II0, with only the relative position of the class I epitopes varied.

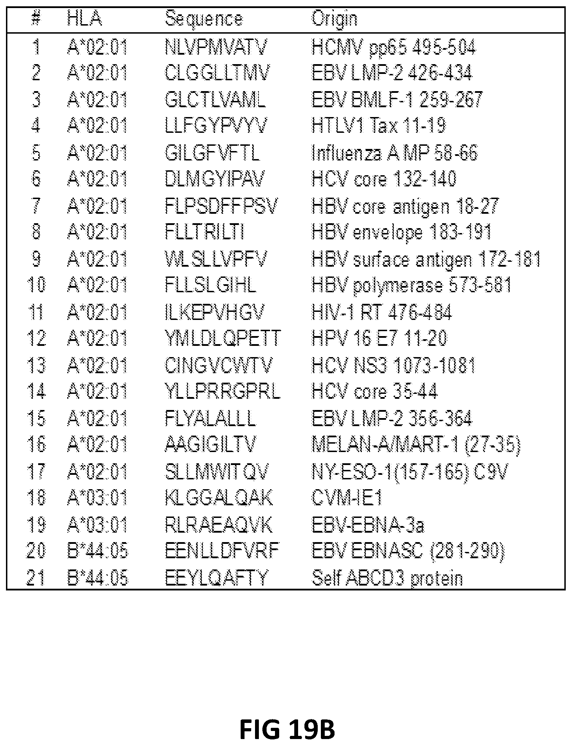

**FIG. 19B** illustrates *in vivo* evaluation of the impact of epitope position in long 21-mer cassettes and shows the sequence information on the T cell epitopes used. Figure discloses SEQ ID NOS 128-129, 132, 131, 130, 157-159, 133, 160-171, respectively, in order of appearance.

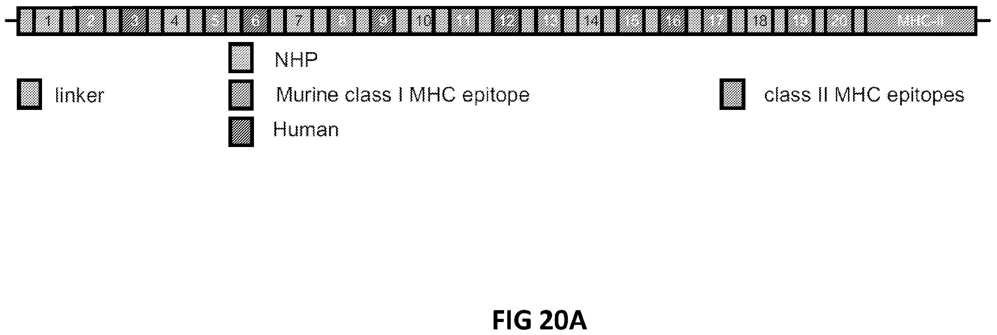

**FIG. 20A** illustrates final cassette design for preclinical IND-enabling studies and shows the design of the final cassettes comprises 20 MHC I epitopes contained in their 25-mer natural sequence (linker = natural flanking sequences), composed of 6 non-human primate (NHP) epitopes, 5 human epitopes, 9 murine epitopes, as well as 2 universal MHC class II epitopes.

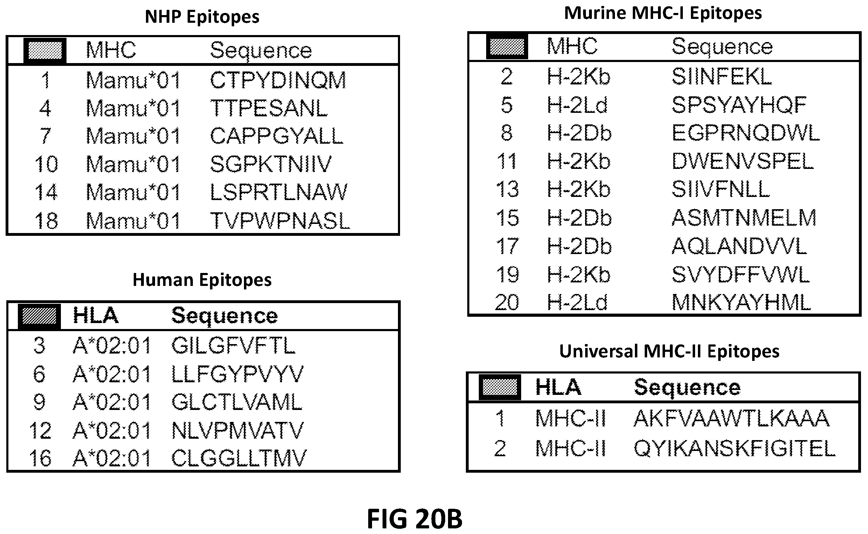

**FIG. 20B** illustrates final cassette design for preclinical IND-enabling studies and shows the sequence information for the T cell epitopes used that are presented on class I MHC of non-human primate (SEQ ID NOS 172-177, respectively, in order of appearance), mouse (SEQ ID NOS 57-58 and 178-184, respectively, in order of appearance) and human origin (SEQ ID NOS 130-132 and 128-129, respectively, in order of appearance), as well as sequences of 2 universal MHC class II epitopes PADRE and Tetanus toxoid (SEQ ID NOS 49 and 47, respectively, in order of appearance).

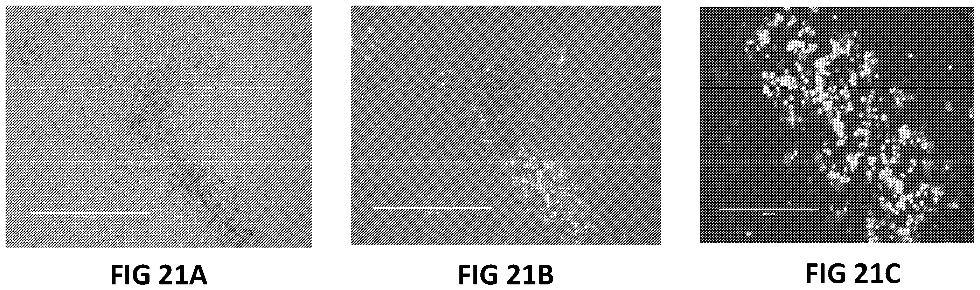

**FIG. 21A** illustrates ChAdV68.4WTnt.GFP virus production after transfection. HEK293A cells were transfected with ChAdV68.4WTnt.GFP DNA using the calcium phosphate protocol. Viral replication was observed 10 days after transfection and ChAdV68.4WTnt.GFP viral plaques were visualized using light microscopy (40x magnification).

**FIG. 21B** illustrates ChAdV68.4WTnt.GFP virus production after transfection. HEK293A cells were transfected with ChAdV68.4WTnt.GFP DNA using the calcium phosphate protocol. Viral replication was observed 10 days after transfection and ChAdV68.4WTnt.GFP viral plaques were visualized using fluorescent microscopy at 40x magnification.

**FIG. 21C** illustrates ChAdV68.4WTnt.GFP virus production after transfection. HEK293A cells were transfected with ChAdV68.4WTnt.GFP DNA using the calcium phosphate protocol. Viral replication was observed 10 days after transfection and ChAdV68.4WTnt.GFP viral plaques were visualized using fluorescent microscopy at 100x magnification.

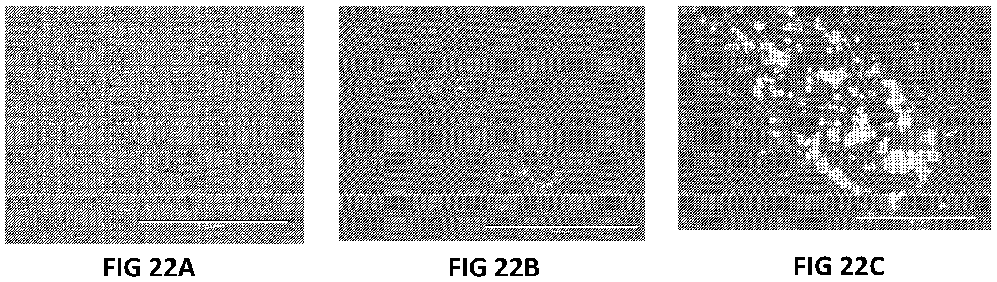

**FIG. 22A** illustrates ChAdV68.5WTnt.GFP virus production after transfection. HEK293A cells were transfected with ChAdV68.5WTnt.GFP DNA using the lipofectamine protocol. Viral replication (plaques) was observed 10 days after transfection. A lysate was made and used to reinfect a T25 flask of 293A cells. ChAdV68.5WTnt.GFP viral plaques were visualized and photographed 3 days later using light microscopy (40x magnification)

**FIG. 22B** illustrates ChAdV68.5WTnt.GFP virus production after transfection. HEK293A cells were transfected with ChAdV68.5WTnt.GFP DNA using the lipofectamine protocol. Viral replication (plaques) was observed 10 days after transfection. A lysate was made and used to reinfect a T25 flask of 293A cells. ChAdV68.5WTnt.GFP viral plaques were visualized and photographed 3 days later using fluorescent microscopy at 40x magnification.

**FIG. 22C** illustrates ChAdV68.5WTnt.GFP virus production after transfection. HEK293A cells were transfected with ChAdV68.5WTnt.GFP DNA using the lipofectamine protocol. Viral replication (plaques) was observed 10 days after transfection. A lysate was made and used to reinfect a T25 flask of 293A cells. ChAdV68.5WTnt.GFP viral plaques were visualized and photographed 3 days later using fluorescent microscopy at 100x magnification.

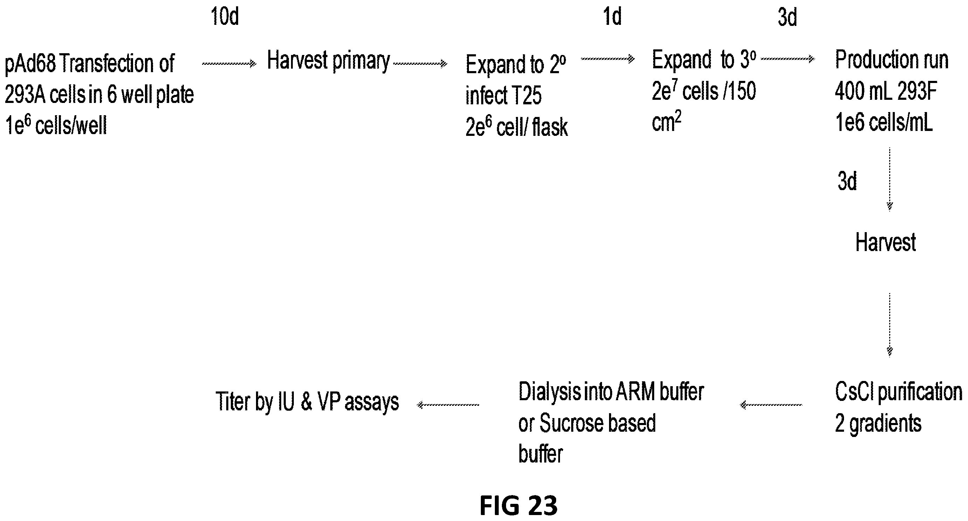

**FIG. 23** illustrates the viral particle production scheme.

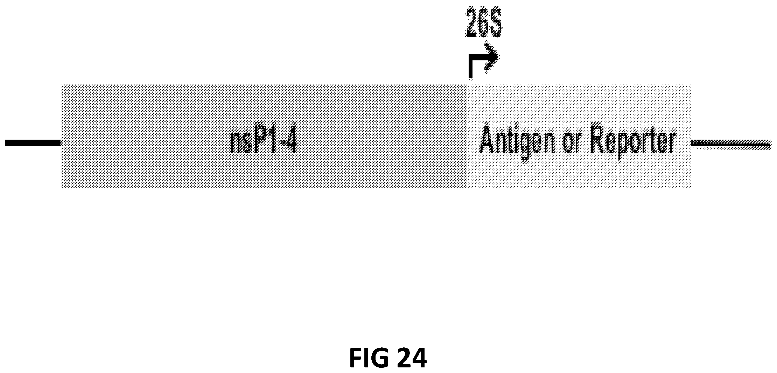

**FIG. 24** illustrates the alphavirus derived VEE self-replicating RNA (srRNA) vector.

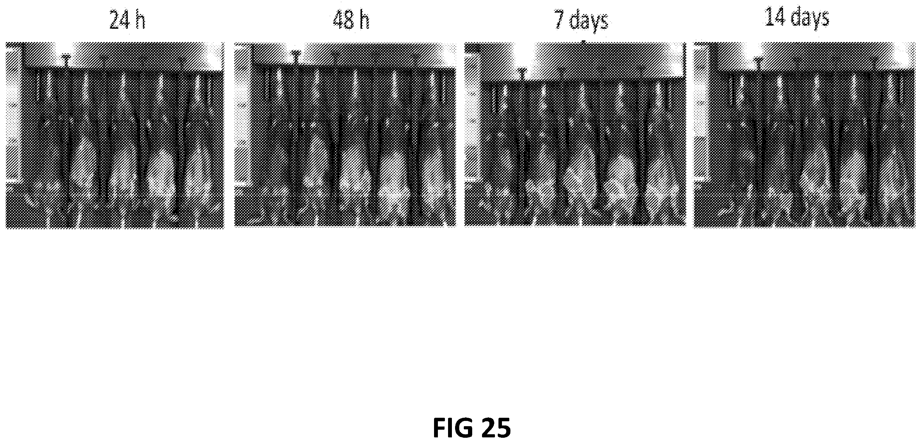

**FIG. 25** illustrates *in vivo* reporter expression after inoculation of C57BL/6J mice with VEE-Luciferase srRNA. Shown are representative images of luciferase signal following immunization of C57BL/6J mice with VEE-Luciferase srRNA (10 ug per mouse, bilateral intramuscular injection, MC3 encapsulated) at various timepoints.

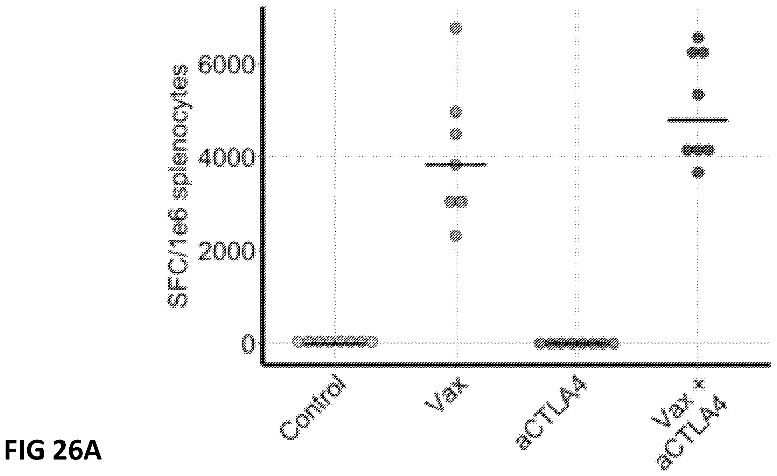

**FIG. 26A** illustrates T-cell responses measured 14 days after immunization with VEE srRNA formulated with MC3 LNP in B16-OVA tumor bearing mice. B16-OVA tumor bearing C57BL/6J mice were injected with 10 ug of VEE-Luciferase srRNA (control), VEE-UbAAY srRNA (Vax), VEE-Luciferase srRNA and anti-CTLA-4 (aCTLA-4) or VEE-UbAAY srRNA and anti-CTLA-4 (Vax + aCTLA-4). In addition, all mice were treated with anti-PD1 mAb starting at day 7. Each group consisted of 8 mice. Mice were sacrificed and spleens and lymph nodes were collected 14 days after immunization. SIINFEKL-specific T-cell responses ("SIINFEKL" disclosed as SEQ ID NO: 57) were assessed by IFN-gamma ELISPOT and are reported as spot-forming cells (SFC) per 106 splenocytes. Lines represent medians.

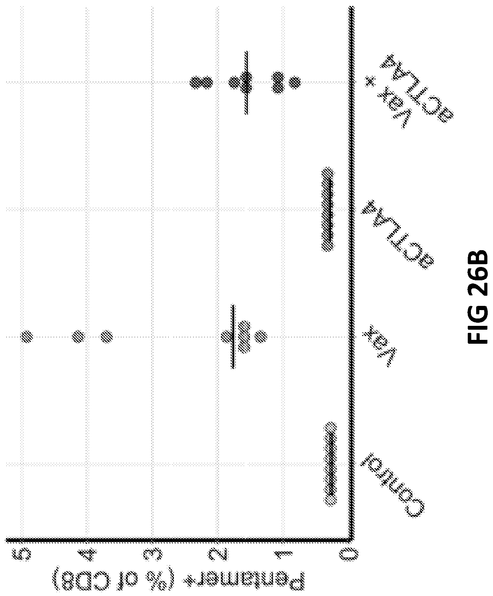

**FIG. 26B** illustrates T-cell responses measured 14 days after immunization with VEE srRNA formulated with MC3 LNP in B16-OVA tumor bearing mice. B16-OVA tumor bearing C57BL/6J mice were injected with 10 ug of VEE-Luciferase srRNA (control), VEE-UbAAY srRNA (Vax), VEE-Luciferase srRNA and anti-CTLA-4 (aCTLA-4) or VEE-UbAAY srRNA and anti-CTLA-4 (Vax + aCTLA-4). In addition, all mice were treated with anti-PD1 mAb starting at day 7. Each group consisted of 8 mice. Mice were sacrificed and spleens and lymph nodes were collected 14 days after immunization. SIINFEKL-specific T-cell responses ("SIINFEKL" disclosed as SEQ ID NO: 57) were assessed by MHCI-pentamer staining, reported as pentamer positive cells as a percent of CD8 positive cells. Lines represent medians.

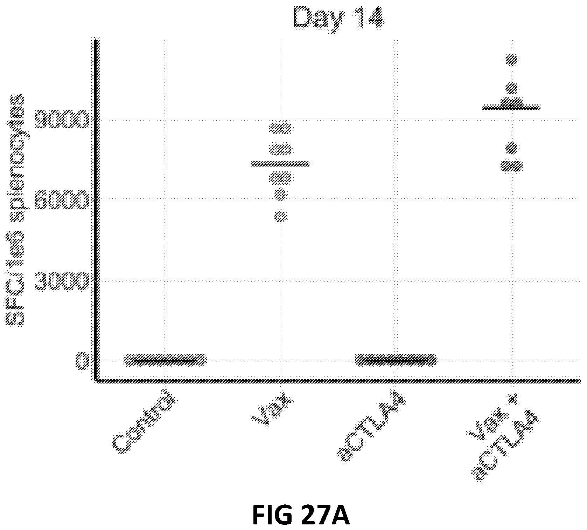

**FIG. 27A** illustrates antigen-specific T-cell responses following heterologous prime/boost in B16-OVA tumor bearing mice. B16-OVA tumor bearing C57BL/6J mice were injected with adenovirus expressing GFP (Ad5-GFP) and boosted with VEE-Luciferase srRNA formulated with MC3 LNP (Control) or Ad5-UbAAY and boosted with VEE-UbAAY srRNA (Vax). Both the Control and Vax groups were also treated with an IgG control mAb. A third group was treated with the Ad5-GFP prime/VEE-Luciferase srRNA boost in combination with anti-CTLA-4 (aCTLA-4), while the fourth group was treated with the Ad5-UbAAY prime/VEE-UbAAY boost in combination with anti-CTLA-4 (Vax + aCTLA-4). In addition, all mice were treated with anti-PD-1 mAb starting at day 21. T-cell responses were measured by IFN-gamma ELISPOT. Mice were sacrificed and spleens and lymph nodes collected at 14 days post immunization with adenovirus.

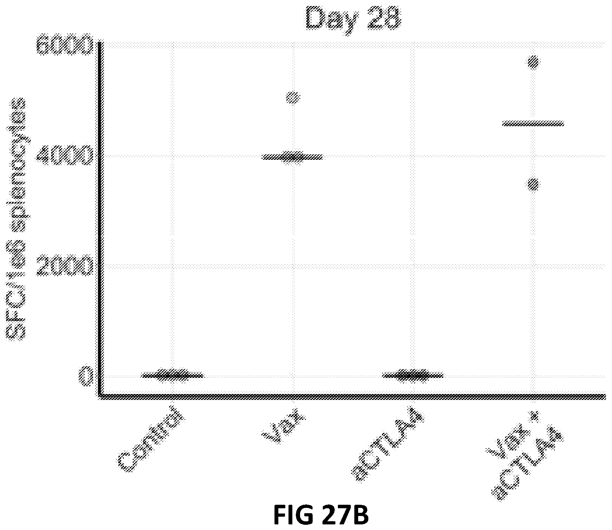

**FIG. 27B** illustrates antigen-specific T-cell responses following heterologous prime/boost in B16-OVA tumor bearing mice. B16-OVA tumor bearing C57BL/6J mice were injected with adenovirus expressing GFP (Ad5-GFP) and boosted with VEE-Luciferase srRNA formulated with MC3 LNP (Control) or Ad5-UbAAY and boosted with VEE-UbAAY srRNA (Vax). Both the Control and Vax groups were also treated with an IgG control mAb. A third group was treated with the Ad5-GFP prime/VEE-Luciferase srRNA boost in combination with anti-CTLA-4 (aCTLA-4), while the fourth group was treated with the Ad5-UbAAY prime/VEE-UbAAY boost in combination with anti-CTLA-4 (Vax + aCTLA-4). In addition, all mice were treated with anti-PD-1 mAb starting at day 21. T-cell responses were measured by IFN-gamma ELISPOT. Mice were sacrificed and spleens and lymph nodes collected at 14 days post immunization with adenovirus and 14 days post boost with srRNA (day 28 after prime).

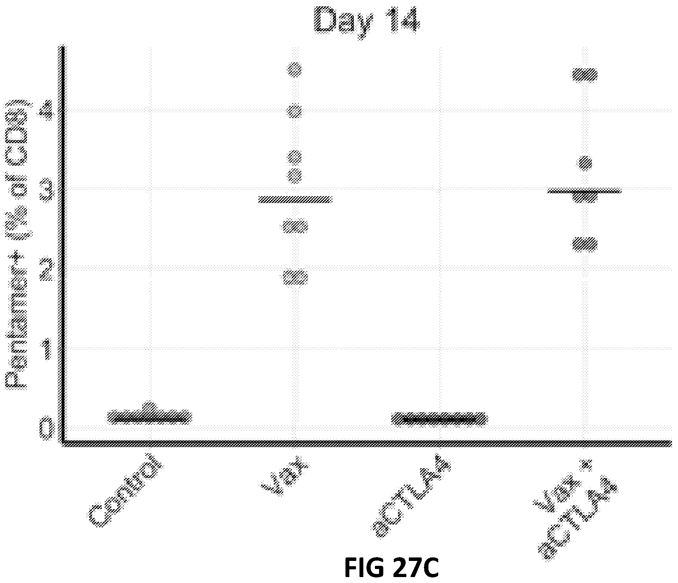

**FIG. 27C** illustrates antigen-specific T-cell responses following heterologous prime/boost in B16-OVA tumor bearing mice. B16-OVA tumor bearing C57BL/6J mice were injected with adenovirus expressing GFP (Ad5-GFP) and boosted with VEE-Luciferase srRNA formulated with MC3 LNP (Control) or Ad5-UbAAY and boosted with VEE-UbAAY srRNA (Vax). Both the Control and Vax groups were also treated with an IgG control mAb. A third group was treated with the Ad5-GFP prime/VEE-Luciferase srRNA boost in combination with anti-CTLA-4 (aCTLA-4), while the fourth group was treated with the Ad5-UbAAY prime/VEE-UbAAY boost in combination with anti-CTLA-4 (Vax + aCTLA-4). In addition, all mice were treated with anti-PD-1 mAb starting at day 21. T-cell responses were measured by MHC class I pentamer staining. Mice were sacrificed and spleens and lymph nodes collected at 14 days post immunization with adenovirus.

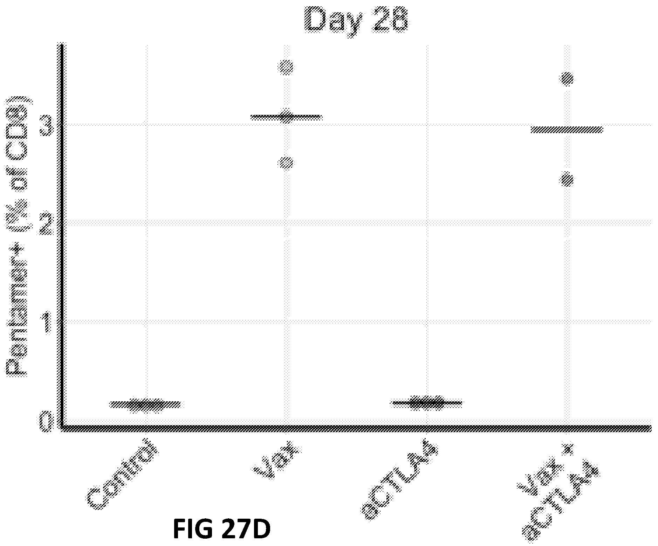

**FIG. 27D** illustrates antigen-specific T-cell responses following heterologous prime/boost in B16-OVA tumor bearing mice. B16-OVA tumor bearing C57BL/6J mice were injected with adenovirus expressing GFP (Ad5-GFP) and boosted with VEE-Luciferase srRNA formulated with MC3 LNP (Control) or Ad5-UbAAY and boosted with VEE-UbAAY srRNA (Vax). Both the Control and Vax groups were also treated with an IgG control mAb. A third group was treated with the Ad5-GFP prime/VEE-Luciferase srRNA boost in combination with anti-CTLA-4 (aCTLA-4), while the fourth group was treated with the Ad5-UbAAY prime/VEE-UbAAY boost in combination with anti-CTLA-4 (Vax + aCTLA-4). In addition, all mice were treated with anti-PD-1 mAb starting at day 21. T-cell responses were measured by MHC class I pentamer staining. Mice were sacrificed and spleens and lymph nodes collected at 14 days post immunization with adenovirus and 14 days post boost with srRNA (day 28 after prime).

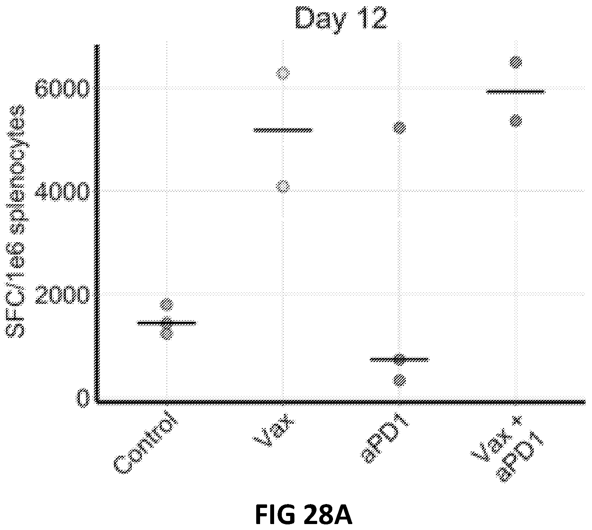

**FIG. 28A** illustrates antigen-specific T-cell responses following heterologous prime/boost in CT26 (Balb/c) tumor bearing mice. Mice were immunized with Ad5-GFP and boosted 15 days after the adenovirus prime with VEE-Luciferase srRNA formulated with MC3 LNP (Control) or primed with Ad5-UbAAY and boosted with VEE-UbAAY srRNA (Vax). Both the Control and Vax groups were also treated with an IgG control mAb. A separate group was administered the Ad5-GFP/VEE-Luciferase srRNA prime/boost in combination with anti-PD-1 (aPD1), while a fourth group received the Ad5-UbAAY/VEE-UbAAY srRNA prime/boost in combination with an anti-PD-1 mAb (Vax + aPD1). T-cell responses to the AH1 peptide were measured using IFN-gamma ELISPOT. Mice were sacrificed and spleens and lymph nodes collected at 12 days post immunization with adenovirus.

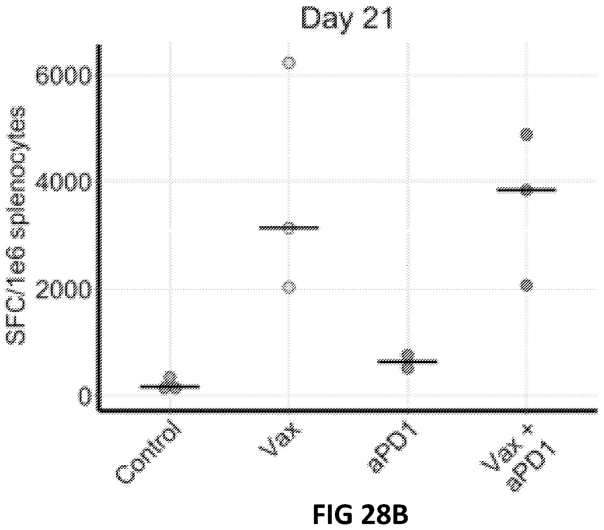

**FIG. 28B** illustrates antigen-specific T-cell responses following heterologous prime/boost in CT26 (Balb/c) tumor bearing mice. Mice were immunized with Ad5-GFP and boosted 15 days after the adenovirus prime with VEE-Luciferase srRNA formulated with MC3 LNP (Control) or primed with Ad5-UbAAY and boosted with VEE-UbAAY srRNA (Vax). Both the Control and Vax groups were also treated with an IgG control mAb. A separate group was administered the Ad5-GFP/VEE-Luciferase srRNA prime/boost in combination with anti-PD-1 (aPD1), while a fourth group received the Ad5-UbAAY/VEE-UbAAY srRNA prime/boost in combination with an anti-PD-1 mAb (Vax + aPD1). T-cell responses to the AH1 peptide were measured using IFN-gamma ELISPOT. Mice were sacrificed and spleens and lymph nodes collected at 12 days post immunization with adenovirus and 6 days post boost with srRNA (day 21 after prime).

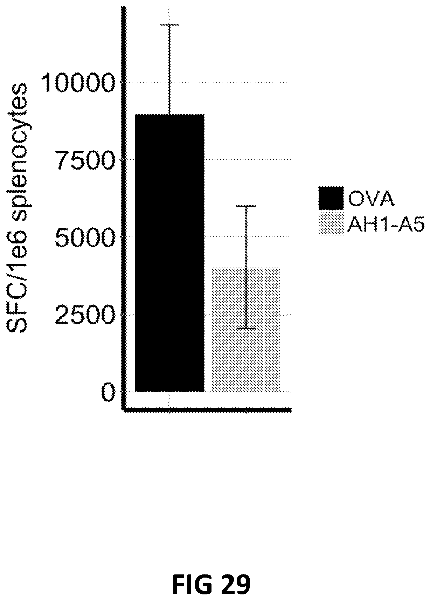

**FIG. 29** illustrates ChAdV68 eliciting T-Cell responses to mouse tumor antigens in mice. Mice were immunized with ChAdV68.5WTnt.MAG25mer, and T-cell responses to the MHC class I epitope SIINFEKL (OVA) (SEQ ID NO: 57) were measured in C57BL/6J female mice and the MHC class I epitope AH1-A5 measured in Balb/c mice. Mean spot forming cells (SFCs) per $10^6$ splenocytes measured in ELISpot assays presented. Error bars represent standard deviation.

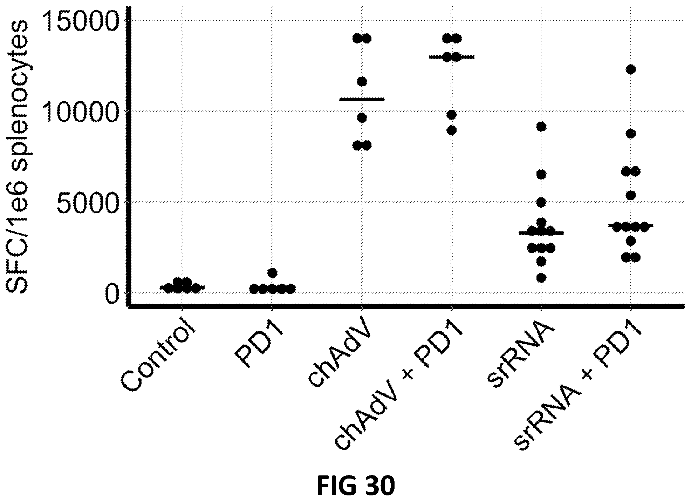

**FIG. 30** illustrates cellular immune responses in a CT26 tumor model following a single immunization with either ChAdV6, ChAdV + anti-PD-1, srRNA, srRNA + anti-PD-1, or anti-PD-1 alone. Antigen-specific IFN-gamma production was measured in splenocytes for 6 mice from each group using ELISpot. Results are presented as spot forming cells (SFC) per $10^6$ splenocytes. Median for each group indicated by horizontal line. P values determined using the Dunnett's multiple comparison test; *** P<0.0001, **P<0.001, *P<0.05. ChAdV= ChAdV68.5WTnt.MAG25mer; srRNA= VEE-MAG25mer srRNA.

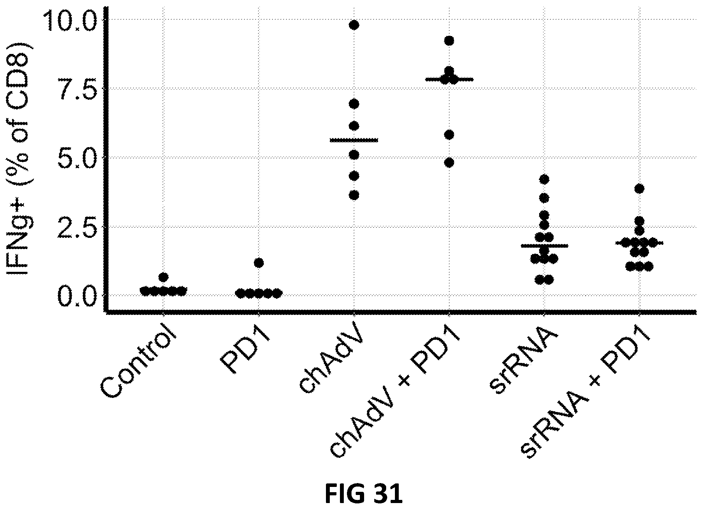

**FIG. 31** illustrates CD8 T-Cell responses in a CT26 tumor model following a single immunization with either ChAdV6, ChAdV + anti-PD-1, srRNA, srRNA + anti-PD-1, or anti-PD-1 alone. Antigen-specific IFN-gamma production in CD8 T cells measured using ICS and results presented as antigen-specific CD8 T cells as a percentage of total CD8 T cells. Median for each group indicated by horizontal line. P values determined using the Dunnett's multiple comparison test; *** P<0.0001, **P<0.001, *P<0.05. ChAdV= ChAdV68.5WTnt.MAG25mer; srRNA= VEE-MAG25mer srRNA.

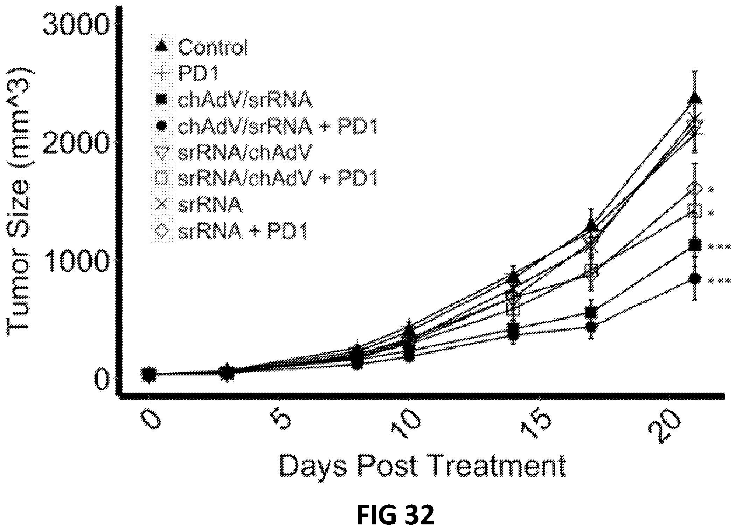

**FIG. 32** illustrates tumor growth in a CT26 tumor model following immunization with a ChAdV/srRNA heterologous prime/boost, a srRNA/ChAdV heterologous prime/boost, or a srRNA/srRNA homologous primer/boost. Also illustrated in a comparison of the prime/boost immunizations with or without administration of anti-PD1 during prime and boost. Tumor volumes measured twice per week and mean tumor volumes presented for the first 21 days of the study. 22-28 mice per group at study initiation. Error bars represent standard error of the mean (SEM). P values determined using the Dunnett's test; *** P<0.0001, **P<0.001, *P<0.05. ChAdV= ChAdV68.5WTnt.MAG25mer; srRNA= VEE-MAG25mer srRNA.

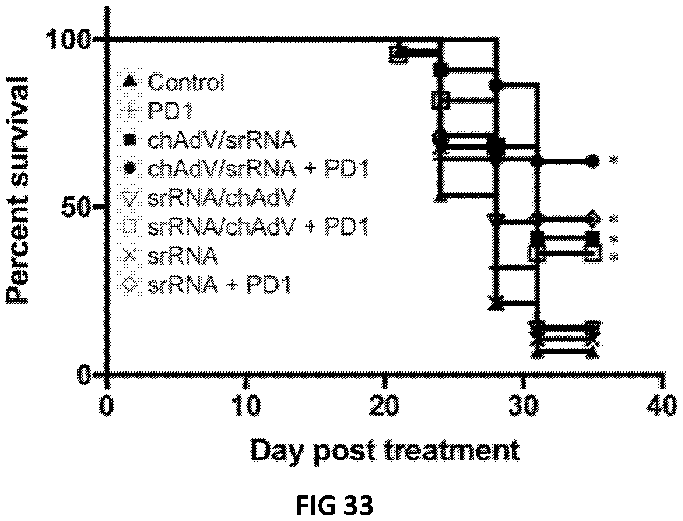

**FIG. 33** illustrates survival in a CT26 tumor model following immunization with a ChAdV/srRNA heterologous prime/boost, a srRNA/ChAdV heterologous prime/boost, or a srRNA/srRNA homologous primer/boost. Also illustrated in a comparison of the prime/boost immunizations with or without administration of anti-PD1 during prime and boost. P values determined using the log-rank test; *** P<0.0001, **P<0.001, *P<0.01. ChAdV= ChAdV68.5WTnt.MAG25mer; srRNA= VEE-MAG25mer srRNA.

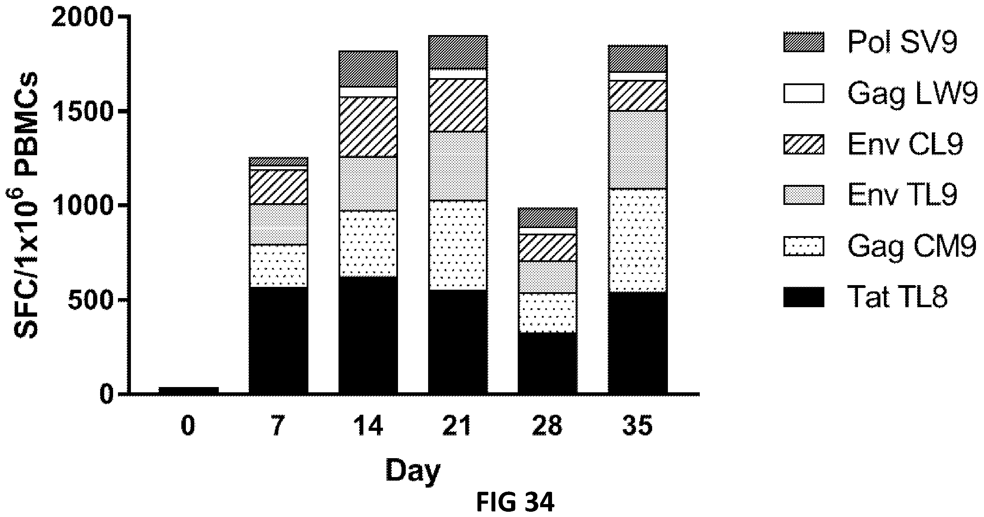

**FIG. 34** illustrates cellular immune responses in Indian rhesus macaques following a heterologous prime/boost immunization. Antigen-specific IFN-gamma production to six different mamu A01 restricted epitopes was measured in PBMCs for the ChAdV68.5WTnt.MAG25mer/VEE-MAG25mer srRNA heterologous prime/boost group (6 rhesus macaques) using ELISpot 7, 14, 21, 28 or 35 days after the intial prime immunization and 7 days after the first boost immunization. Results are presented as mean spot forming cells (SFC) per $10^6$ PBMCs for each epitope in a stacked bar graph format.

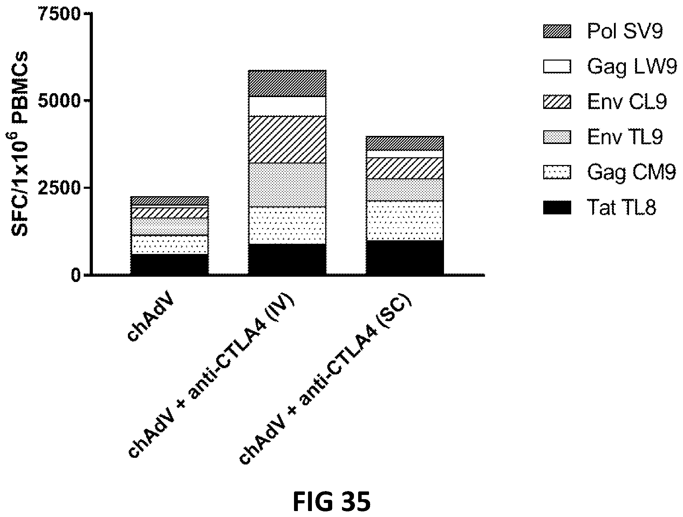

**FIG. 35** illustrates cellular immune responses in Indian rhesus macaques following a ChAdV immunization with or without anti-CTLA4. Antigen-specific IFN-gamma production to six different mamu A01 restricted epitopes was measured in PBMCs after immunization with ChAdV68.5WTnt.MAG25mer without or with the addition of anti-CTLA4 administered intravenously (IV) or locally (SC) (6 rhesus macaques per group) using ELISpot 14 after the initial immunization. Results are presented as mean spot forming cells (SFC) per $10^6$ PBMCs for each epitope in a stacked bar graph format.

## DETAILED DESCRIPTION

### I. Definitions

In general, terms used in the claims and the specification are intended to be construed as having the plain meaning understood by a person of ordinary skill in the art. Certain terms are defined below to provide additional clarity. In case of conflict between the plain meaning and the provided definitions, the provided definitions are to be used.

As used herein the term "antigen" is a substance that induces an immune response.

As used herein the term "neoantigen" is an antigen that has at least one alteration that makes it distinct from the corresponding wild-type antigen, e.g., via mutation in a tumor cell or post-translational modification specific to a tumor cell. A neoantigen can include a polypeptide sequence or a nucleotide sequence. A mutation can include a frameshift or nonframeshift indel, missense or nonsense substitution, splice site alteration, genomic rearrangement or gene fusion, or any genomic or expression alteration giving rise to a neoORF. A mutations can also include a splice variant. Post-translational modifications specific to a tumor cell can include aberrant phosphorylation. Post-translational modifications specific to a tumor cell can also include a proteasome-generated spliced antigen. *See* Liepe et al., A large fraction of HLA class I ligands are proteasome-generated spliced peptides; Science. 2016 Oct 21;354(6310):354-358.

As used herein the term "tumor neoantigen" is a neoantigen present in a subject's tumor cell or tissue but not in the subject's corresponding normal cell or tissue.

As used herein the term "neoantigen-based vaccine" is a vaccine construct based on one or more neoantigens, e.g., a plurality of neoantigens.

As used herein the term "candidate neoantigen" is a mutation or other aberration giving rise to a new sequence that may represent a neoantigen.

As used herein the term "coding region" is the portion(s) of a gene that encode protein.

As used herein the term "coding mutation" is a mutation occurring in a coding region.

As used herein the term "ORF" means open reading frame.

As used herein the term "NEO-ORF" is a tumor-specific ORF arising from a mutation or other aberration such as splicing.

As used herein the term "missense mutation" is a mutation causing a substitution from one amino acid to another.

As used herein the term "nonsense mutation" is a mutation causing a substitution from an amino acid to a stop codon.

As used herein the term "frameshift mutation" is a mutation causing a change in the frame of the protein.

As used herein the term "indel" is an insertion or deletion of one or more nucleic acids.

As used herein, the term percent "identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters. Alternatively, sequence similarity or dissimilarity can be established by the combined presence or absence of particular nucleotides, or, for translated sequences, amino acids at selected sequence positions (e.g., sequence motifs).

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information.

As used herein the term "non-stop or read-through" is a mutation causing the removal of the natural stop codon.

As used herein the term "epitope" is the specific portion of an antigen typically bound by an antibody or T cell receptor.

As used herein the term "immunogenic" is the ability to elicit an immune response, e.g., via T cells, B cells, or both.

As used herein the term "HLA binding affinity" "MHC binding affinity" means affinity of binding between a specific antigen and a specific MHC allele.

As used herein the term "bait" is a nucleic acid probe used to enrich a specific sequence of DNA or RNA from a sample.

As used herein the term "variant" is a difference between a subject's nucleic acids and the reference human genome used as a control.

As used herein the term "variant call" is an algorithmic determination of the presence of a variant, typically from sequencing.

As used herein the term "polymorphism" is a germline variant, i.e., a variant found in all DNA-bearing cells of an individual.

As used herein the term "somatic variant" is a variant arising in non-germline cells of an individual.

As used herein the term "allele" is a version of a gene or a version of a genetic sequence or a version of a protein.

As used herein the term "HLA type" is the complement of HLA gene alleles.

As used herein the term "nonsense-mediated decay" or "NMD" is a degradation of an mRNA by a cell due to a premature stop codon.

As used herein the term "truncal mutation" is a mutation originating early in the development of a tumor and present in a substantial portion of the tumor's cells.

As used herein the term "subclonal mutation" is a mutation originating later in the development of a tumor and present in only a subset of the tumor's cells.

As used herein the term "exome" is a subset of the genome that codes for proteins. An exome can be the collective exons of a genome.

As used herein the term "logistic regression" is a regression model for binary data from statistics where the logit of the probability that the dependent variable is equal to one is modeled as a linear function of the dependent variables.

As used herein the term "neural network" is a machine learning model for classification or regression consisting of multiple layers of linear transformations followed by element-wise nonlinearities typically trained via stochastic gradient descent and back-propagation.

As used herein the term "proteome" is the set of all proteins expressed and/or translated by a cell, group of cells, or individual.

As used herein the term "peptidome" is the set of all peptides presented by MHC-I or MHC-II on the cell surface. The peptidome may refer to a property of a cell or a collection of cells (e.g., the tumor peptidome, meaning the union of the peptidomes of all cells that comprise the tumor).

As used herein the term "ELISPOT" means Enzyme-linked immunosorbent spot assay - which is a common method for monitoring immune responses in humans and animals.

As used herein the term "dextramers" is a dextran-based peptide-MHC multimers used for antigen-specific T-cell staining in flow cytometry.

As used herein the term "tolerance or immune tolerance" is a state of immune non-responsiveness to one or more antigens, e.g. self-antigens.

As used herein the term "central tolerance" is a tolerance affected in the thymus, either by deleting self-reactive T-cell clones or by promoting self-reactive T-cell clones to differentiate into immunosuppressive regulatory T-cells (Tregs).

As used herein the term "peripheral tolerance" is a tolerance affected in the periphery by downregulating or anergizing self-reactive T-cells that survive central tolerance or promoting these T cells to differentiate into Tregs.

The term "sample" can include a single cell or multiple cells or fragments of cells or an aliquot of body fluid, taken from a subject, by means including venipuncture, excretion, ejaculation, massage, biopsy, needle aspirate, lavage sample, scraping, surgical incision, or intervention or other means known in the art.

The term "subject" encompasses a cell, tissue, or organism, human or non-human, whether in vivo, ex vivo, or in vitro, male or female. The term subject is inclusive of mammals including humans.

The term "mammal" encompasses both humans and non-humans and includes but is not limited to humans, non-human primates, canines, felines, murines, bovines, equines, and porcines.

The term "clinical factor" refers to a measure of a condition of a subject, e.g., disease activity or severity. "Clinical factor" encompasses all markers of a subject's health status, including non-sample markers, and/or other characteristics of a subject, such as, without limitation, age and gender. A clinical factor can be a score, a value, or a set of values that can be obtained from evaluation of a sample (or population of samples) from a subject or a subject under a determined condition. A clinical factor can also be predicted by markers and/or other parameters such as gene expression surrogates. Clinical factors can include tumor type, tumor sub-type, and smoking history.

The term "antigen-encoding nucleic acid sequences derived from a tumor" refers to nucleic acid sequences directly extracted from the tumor, e.g. via RT-PCR; or sequence data obtained by sequencing the tumor and then synthesizing the nucleic acid sequences using the sequencing data, e.g., via various synthetic or PCR-based methods known in the art.

The term "alphavirus" refers to members of the family *Togaviridae,* and are positive-sense single-stranded RNA viruses. Alphaviruses are typically classified as either Old World, such as Sindbis, Ross River, Mayaro, Chikungunya, and Semliki Forest viruses, or New World, such as eastern equine encephalitis, Aura, Fort Morgan, or Venezuelan equine encephalitis and its derivative strain TC-83. Alphaviruses are typically self-replicating RNA viruses.

The term "alphavirus backbone" refers to minimal sequence(s) of an alphavirus that allow for self-replication of the viral genome. Minimal sequences can include conserved sequences for nonstructural protein-mediated amplification, a nonstructural protein 1 (nsP1) gene, a nsP2 gene, a nsP3 gene, a nsP4 gene, and a poly A sequence, as well as sequences for expression of subgenomic viral RNA including a 26S promoter element.

The term "sequences for nonstructural protein-mediated amplification" includes alphavirus conserved sequence elements (CSE) well known to those in the art. CSEs include, but are not limited to, an alphavirus 5' UTR, a 51-nt CSE, a 24-nt CSE, or other 26S subgenomic promoter sequence, a 19-nt CSE, and an alphavirus 3' UTR.

The term "RNA polymerase" includes polymerases that catalyze the production of RNA polynucleotides from a DNA template. RNA polymerases include, but are not limited to, bacteriophage derived polymerases including T3, T7, and SP6.

The term "lipid" includes hydrophobic and/or amphiphilic molecules. Lipids can be cationic, anionic, or neutral. Lipids can be synthetic or naturally derived, and in some instances biodegradable. Lipids can include cholesterol, phospholipids, lipid conjugates including, but not limited to, polyethyleneglycol (PEG) conjugates (PEGylated lipids), waxes, oils, glycerides, fats, and fat-soluble vitamins. Lipids can also include dilinoleylmethyl- 4-dimethylaminobutyrate (MC3) and MC3-like molecules.

The term "lipid nanoparticle" or "LNP" includes vesicle like structures formed using a lipid containing membrane surrounding an aqueous interior, also referred to as liposomes. Lipid nanoparticles includes lipid-based compositions with a solid lipid core stabilized by a surfactant. The core lipids can be fatty acids, acylglycerols, waxes, and mixtures of these surfactants. Biological membrane lipids such as phospholipids, sphingomyelins, bile salts (sodium taurocholate), and sterols (cholesterol) can be utilized as stabilizers. Lipid nanoparticles can be formed using defined ratios of different lipid molecules, including, but not limited to, defined ratios of one or more cationic, anionic, or neutral lipids. Lipid nanoparticles can encapsulate molecules within an outer-membrane shell and subsequently can be contacted with target cells to deliver the encapsulated molecules to the host cell cytosol. Lipid nanoparticles can be modified or functionalized with non-lipid molecules, including on their surface. Lipid nanoparticles can be single-layered (unilamellar) or multi-layered (multilamellar). Lipid nanoparticles can be complexed with nucleic acid. Unilamellar lipid nanoparticles can be complexed with nucleic acid, wherein the nucleic acid is in the aqueous interior. Multilamellar lipid nanoparticles can be complexed with nucleic acid, wherein the nucleic acid is in the aqueous interior, or to form or sandwiched between

Abbreviations: MHC: major histocompatibility complex; HLA: human leukocyte antigen, or the human MHC gene locus; NGS: next-generation sequencing; PPV: positive predictive value; TSNA: tumor-specific neoantigen; FFPE: formalin-fixed, paraffin-embedded; NMD: nonsense-mediated decay; NSCLC: non-small-cell lung cancer; DC: dendritic cell.

It should be noted that, as used in the specification and the appended claims, the singular forms "a," "an," and "the" include plural referents unless the context clearly dictates otherwise.

II. **Methods** of Identifying **Neoantigens**

Described herein are methods for identifying neoantigens from a tumor of a subject that are likely to be presented on the cell surface of the tumor and/or are likely to be immunogenic, to select neoantigen-encoding nucleic acid sequences for the chimpanzee adenovirus vectors as claimed. As an example, one such method may comprise the steps of: obtaining at least one of exome, transcriptome or whole genome tumor nucleotide sequencing data from the tumor cell of the subject, wherein the tumor nucleotide sequencing data is used to obtain data representing peptide sequences of each of a set of neoantigens, and wherein the peptide sequence of each neoantigen comprises at least one alteration that makes it distinct from the corresponding wild-type peptide sequence; inputting the peptide sequence of each neoantigen into one or more presentation models to generate a set of numerical likelihoods that each of the neoantigens is presented by one or more MHC alleles on the tumor cell surface of the tumor cell of the subject or cells present in the tumor, the set of numerical likelihoods having been identified at least based on received mass spectrometry data; and selecting a subset of the set of neoantigens based on the set of numerical likelihoods to generate a set of selected neoantigens.

The presentation model can comprise a statistical regression or a machine learning (e.g., deep learning) model trained on a set of reference data (also referred to as a training data set) comprising a set of corresponding labels, wherein the set of reference data is obtained from each of a plurality of distinct subjects where optionally some subjects can have a tumor, and wherein the set of reference data comprises at least one of: data representing exome nucleotide sequences from tumor tissue, data representing exome nucleotide sequences from normal tissue, data representing transcriptome nucleotide sequences from tumor tissue, data representing proteome sequences from tumor tissue, and data representing MHC peptidome sequences from tumor tissue, and data representing MHC peptidome sequences from normal tissue. The reference data can further comprise mass spectrometry data, sequencing data, RNA sequencing data, and proteomics data for single-allele cell lines engineered to express a predetermined MHC allele that are subsequently exposed to synthetic protein, normal and tumor human cell lines, and fresh and frozen primary samples, and T cell assays (e.g., ELISPOT). In certain aspects, the set of reference data includes each form of reference data.

The presentation model can comprise a set of features derived at least in part from the set of reference data, and wherein the set of features comprises at least one of allele dependent-features and allele-independent features. In certain aspects each feature is included.

Dendritic cell presentation to naive T cell features can comprise at least one of: A feature described above. The dose and type of antigen in the vaccine. (e.g., peptide, mRNA, virus, etc.): (1) The route by which dendritic cells (DCs) take up the antigen type (e.g., endocytosis, micropinocytosis); and/or (2) The efficacy with which the antigen is taken up by DCs. The dose and type of adjuvant in the vaccine. The length of the vaccine antigen sequence. The number and sites of vaccine administration. Baseline patient immune functioning (e.g., as measured by history of recent infections, blood counts, etc). For RNA vaccines: (1) the turnover rate of the mRNA protein product in the dendritic cell; (2) the rate of translation of the mRNA after uptake by dendritic cells as measured in in vitro or in vivo experiments; and/or (3) the number or rounds of translation of the mRNA after uptake by dendritic cells as measured by in vivo or in vitro experiments. The presence of protease cleavage motifs in the peptide, optionally giving additional weight to proteases typically expressed in dendritic cells (as measured by RNA-seq or mass spectrometry). The level of expression of the proteasome and immunoproteasome in typical activated dendritic cells (which may be measured by RNA-seq, mass spectrometry, immunohistochemistry, or other standard techniques). The expression levels of the particular MHC allele in the individual in question (e.g., as measured by RNA-seq or mass spectrometry), optionally measured specifically in activated dendritic cells or other immune cells. The probability of peptide presentation by the particular MHC allele in other individuals who express the particular MHC allele, optionally measured specifically in activated dendritic cells or other immune cells. The probability of peptide presentation by MHC alleles in the same family of molecules (e.g., HLA-A, HLA-B, HLA-C, HLA-DQ, HLA-DR, HLA-DP) in other individuals, optionally measured specifically in activated dendritic cells or other immune cells.

Immune tolerance escape features can comprise at least one of: Direct measurement of the self-peptidome via protein mass spectrometry performed on one or several cell types. Estimation of the self-peptidome by taking the union of all k-mer (e.g. 5-25) substrings of self-proteins. Estimation of the self-peptidome using a model of presentation similar to the presentation model described above applied to all non-mutation self-proteins, optionally accounting for germline variants.

Ranking can be performed using the plurality of neoantigens provided by at least one model based at least in part on the numerical likelihoods. Following the ranking a selecting can be performed to select a subset of the ranked neoantigens according to a selection criteria. After selecting a subset of the ranked peptides can be provided as an output.

A number of the set of selected neoantigens may be 20.

The presentation model may represent dependence between presence of a pair of a particular one of the MHC alleles and a particular amino acid at a particular position of a peptide sequence; and likelihood of presentation on the tumor cell surface, by the particular one of the MHC alleles of the pair, of such a peptide sequence comprising the particular amino acid at the particular position.

A method described herein for selecting neoantigen-encoding nucleic acid sequences can also include applying the one or more presentation models to the peptide sequence of the corresponding neoantigen to generate a dependency score for each of the one or more MHC alleles indicating whether the MHC allele will present the corresponding neoantigen based on at least positions of amino acids of the peptide sequence of the corresponding neoantigen.

A method described herein for selecting neoantigen-encoding nucleic acid sequences can also include transforming the dependency scores to generate a corresponding per-allele likelihood for each MHC allele indicating a likelihood that the corresponding MHC allele will present the corresponding neoantigen; and combining the per-allele likelihoods to generate the numerical likelihood.

The step of transforming the dependency scores can model the presentation of the peptide sequence of the corresponding neoantigen as mutually exclusive.

A method described herein for selecting neoantigen-encoding nucleic acid sequences can also include transforming a combination of the dependency scores to generate the numerical likelihood.

The step of transforming the combination of the dependency scores can model the presentation of the peptide sequence of the corresponding neoantigen as interfering between MHC alleles.

The set of numerical likelihoods can be further identified by at least an allele noninteracting feature, and a method disclosed herein can also include applying an allele noninteracting one of the one or more presentation models to the allele noninteracting features to generate a dependency score for the allele noninteracting features indicating whether the peptide sequence of the corresponding neoantigen will be presented based on the allele noninteracting features.

A method described herein for selecting neoantigen-encoding nucleic acid sequences can also include combining the dependency score for each MHC allele in the one or more MHC alleles with the dependency score for the allele noninteracting feature; transforming the combined dependency scores for each MHC allele to generate a corresponding per-allele likelihood for the MHC allele indicating a likelihood that the corresponding MHC allele will present the corresponding neoantigen; and combining the per-allele likelihoods to generate the numerical likelihood.

A method described herein for selecting neoantigen-encoding nucleic acid sequences can also include transforming a combination of the dependency scores for each of the MHC alleles and the dependency score for the allele noninteracting features to generate the numerical likelihood.

A set of numerical parameters for the presentation model can be trained based on a training data set including at least a set of training peptide sequences identified as present in a plurality of samples and one or more MHC alleles associated with each training peptide sequence, wherein the training peptide sequences are identified through mass spectrometry on isolated peptides eluted from MHC alleles derived from the plurality of samples.

The samples can also include cell lines engineered to express a single MHC class I or class II allele.

The samples can also include cell lines engineered to express a plurality of MHC class I or class II alleles.

The samples can also include human cell lines obtained or derived from a plurality of patients.

The samples can also include fresh or frozen tumor samples obtained from a plurality of patients.

The samples can also include fresh or frozen tissue samples obtained from a plurality of patients.

The samples can also include peptides identified using T-cell assays.

The training data set can further include data associated with: peptide abundance of the set of training peptides present in the samples; peptide length of the set of training peptides in the samples.

The training data set may be generated by comparing the set of training peptide sequences via alignment to a database comprising a set of known protein sequences, wherein the set of training protein sequences are longer than and include the training peptide sequences.

The training data set may be generated based on performing or having performed nucleotide sequencing on a cell line to obtain at least one of exome, transcriptome, or whole genome sequencing data from the cell line, the sequencing data including at least one nucleotide sequence including an alteration.

The training data set may be generated based on obtaining at least one of exome, transcriptome, and whole genome normal nucleotide sequencing data from normal tissue samples.

The training data set may further include data associated with proteome sequences associated with the samples.

The training data set may further include data associated with MHC peptidome sequences associated with the samples.

The training data set may further include data associated with peptide-MHC binding affinity measurements for at least one of the isolated peptides.

The training data set may further include data associated with peptide-MHC binding stability measurements for at least one of the isolated peptides.

The training data set may further include data associated with transcriptomes associated with the samples.

The training data set may further include data associated with genomes associated with the samples.

The training peptide sequences may be of lengths within a range of k-mers where k is between 8-15, inclusive for MHC class I or 9-30 inclusive for MHC class II.

A method described herein for selecting neoantigen-encoding nucleic acid sequences can also include encoding the peptide sequence using a one-hot encoding scheme.

A method described herein for selecting neoantigen-encoding nucleic acid sequences can also include encoding the training peptide sequences using a left-padded one-hot encoding scheme.

A method described herein for selecting neoantigen-encoding nucleic acid sequences may also include selecting neoantigens that have an increased likelihood of being presented on the tumor cell surface relative to unselected neoantigens based on the presentation model.

A method described herein for selecting neoantigen-encoding nucleic acid sequences may also include selecting neoantigens that have an increased likelihood of being capable of inducing a tumor-specific immune response in the subject relative to unselected neoantigens based on the presentation model.

A method described herein for selecting neoantigen-encoding nucleic acid sequences may also include selecting neoantigens that have an increased likelihood of being capable of being presented to naive T cells by professional antigen presenting cells (APCs) relative to unselected neoantigens based on the presentation model, optionally wherein the APC is a dendritic cell (DC).

A method described herein for selecting neoantigen-encoding nucleic acid sequences may also include selecting neoantigens that have a decreased likelihood of being subject to inhibition via central or peripheral tolerance relative to unselected neoantigens based on the presentation model.

A method described herein for selecting neoantigen-encoding nucleic acid sequences may also include selecting neoantigens that have a decreased likelihood of being capable of inducing an autoimmune response to normal tissue in the subject relative to unselected neoantigens based on the presentation model.

The exome or transcriptome nucleotide sequencing data may be obtained by performing sequencing on the tumor tissue.

The sequencing may be next generation sequencing (NGS) or any massively parallel sequencing approach.

The set of numerical likelihoods may be further identified by at least MHC-allele interacting features comprising at least one of: the predicted affinity with which the MHC allele and the neoantigen encoded peptide bind; the predicted stability of the neoantigen encoded peptide-MHC complex; the sequence and length of the neoantigen encoded peptide; the probability of presentation of neoantigen encoded peptides with similar sequence in cells from other individuals expressing the particular MHC allele as assessed by mass-spectrometry proteomics or other means; the expression levels of the particular MHC allele in the subject in question (e.g. as measured by RNA-seq or mass spectrometry); the overall neoantigen encoded peptide-sequence-independent probability of presentation by the particular MHC allele in other distinct subjects who express the particular MHC allele; the overall neoantigen encoded peptide-sequence-independent probability of presentation by MHC alleles in the same family of molecules (e.g., HLA-A, HLA-B, HLA-C, HLA-DQ, HLA-DR, HLA-DP) in other distinct subjects.

The set of numerical likelihoods are further identified by at least MHC-allele noninteracting features comprising at least one of: the C- and N-terminal sequences flanking the neoantigen encoded peptide within its source protein sequence; the presence of protease cleavage motifs in the neoantigen encoded peptide, optionally weighted according to the expression of corresponding proteases in the tumor cells (as measured by RNA-seq or mass spectrometry); the turnover rate of the source protein as measured in the appropriate cell type; the length of the source protein, optionally considering the specific splice variants ("isoforms") most highly expressed in the tumor cells as measured by RNA-seq or proteome mass spectrometry, or as predicted from the annotation of germline or somatic splicing mutations detected in DNA or RNA sequence data; the level of expression of the proteasome, immunoproteasome, thymoproteasome, or other proteases in the tumor cells (which may be measured by RNA-seq, proteome mass spectrometry, or immunohistochemistry); the expression of the source gene of the neoantigen encoded peptide (e.g., as measured by RNA-seq or mass spectrometry); the typical tissue-specific expression of the source gene of the neoantigen encoded peptide during various stages of the cell cycle; a comprehensive catalog of features of the source protein and/or its domains as can be found in e.g. uniProt or PDB http://www.rcsb.org/pdb/home/home.do; features describing the properties of the domain of the source protein containing the peptide, for example: secondary or tertiary structure (e.g., alpha helix vs beta sheet); alternative splicing; the probability of presentation of peptides from the source protein of the neoantigen encoded peptide in question in other distinct subjects; the probability that the peptide will not be detected or over-represented by mass spectrometry due to technical biases; the expression of various gene modules/pathways as measured by RNASeq (which need not contain the source protein of the peptide) that are informative about the state of the tumor cells, stroma, or tumor-infiltrating lymphocytes (TILs); the copy number of the source gene of the neoantigen encoded peptide in the tumor cells; the probability that the peptide binds to the TAP or the measured or predicted binding affinity of the peptide to the TAP; the expression level of TAP in the tumor cells (which may be measured by RNA-seq, proteome mass spectrometry, immunohistochemistry); presence or absence of tumor mutations, including, but not limited to: driver mutations in known cancer driver genes such as EGFR, KRAS, ALK, RET, ROS 1, TP53, CDKN2A, CDKN2B, NTRK1, NTRK2, NTRK3, and in genes encoding the proteins involved in the antigen presentation machinery (e.g., B2M, HLA-A, HLA-B, HLA-C, TAP-1, TAP-2, TAPBP, CALR, CNX, ERP57, HLA-DM, HLA-DMA, HLA-DMB, HLA-DO, HLA-DOA, HLA-DOBHLA-DP, HLA-DPA1, HLA-DPB1, HLA-DQ, HLA-DQA1, HLA-DQA2, HLA-DQB1, HLA-DQB2, HLA-DR, HLA-DRA, HLA-DRB1, HLA-DRB3, HLA-DRB4, HLA-DRB5 or any of the genes coding for components of the proteasome or immunoproteasome). Peptides whose presentation relies on a component of the antigen-presentation machinery that is subject to loss-of-function mutation in the tumor have reduced probability of presentation; presence or absence of functional germline polymorphisms, including, but not limited to: in genes encoding the proteins involved in the antigen presentation machinery (e.g., B2M, HLA-A, HLA-B, HLA-C, TAP-1, TAP-2, TAPBP, CALR, CNX, ERP57, HLA-DM, HLA-DMA, HLA-DMB, HLA-DO, HLA-DOA, HLA-DOBHLA-DP, HLA-DPA1, HLA-DPB1, HLA-DQ, HLA-DQA1, HLA-DQA2, HLA-DQB1, HLA-DQB2, HLA-DR, HLA-DRA, HLA-DRB1, HLA-DRB3, HLA-DRB4, HLA-DRB5 or any of the genes coding for components of the proteasome or immunoproteasome); tumor type (e.g., NSCLC, melanoma); clinical tumor subtype (e.g., squamous lung cancer vs. non-squamous); smoking history; the typical expression of the source gene of the peptide in the relevant tumor type or clinical subtype, optionally stratified by driver mutation.

The at least one alteration may be a frameshift or nonframeshift indel, missense or nonsense substitution, splice site alteration, genomic rearrangement or gene fusion, or any genomic or expression alteration giving rise to a neoORF.

The tumor cell may be selected from the group consisting of: lung cancer, melanoma, breast cancer, ovarian cancer, prostate cancer, kidney cancer, gastric cancer, colon cancer, testicular cancer, head and neck cancer, pancreatic cancer, brain cancer, B-cell lymphoma, acute myelogenous leukemia, chronic myelogenous leukemia, chronic lymphocytic leukemia, and T cell lymphocytic leukemia, non-small cell lung cancer, and small cell lung cancer.

A method disclosed herein but not claimed may also include obtaining a tumor vaccine comprising the set of selected neoantigens or a subset thereof, optionally further comprising administering the tumor vaccine to the subject.

At least one of neoantigens in the set of selected neoantigens, when in polypeptide form, may include at least one of: a binding affinity with MHC with an IC50 value of less than 1000nM, for MHC Class 1 polypeptides a length of 8-15, 8, 9, 10, 11, 12, 13, 14, or 15 amino acids, presence of sequence motifs within or near the polypeptide in the parent protein sequence promoting proteasome cleavage, and presence of sequence motifs promoting TAP transport.

Also disclosed herein but not claimed are methods for generating a model for identifying one or more neoantigens that are likely to be presented on a tumor cell surface of a tumor cell, comprising the steps of: receiving mass spectrometry data comprising data associated with a plurality of isolated peptides eluted from major histocompatibility complex (MHC) derived from a plurality of samples; obtaining a training data set by at least identifying a set of training peptide sequences present in the samples and one or more MHCs associated with each training peptide sequence; training a set of numerical parameters of a presentation model using the training data set comprising the training peptide sequences, the presentation model providing a plurality of numerical likelihoods that peptide sequences from the tumor cell are presented by one or more MHC alleles on the tumor cell surface.

The presentation model may represent dependence between: presence of a particular amino acid at a particular position of a peptide sequence; and likelihood of presentation, by one of the MHC alleles on the tumor cell, of the peptide sequence containing the particular amino acid at the particular position.

The samples can also include cell lines engineered to express a single MHC class I or class II allele.

The samples can also include cell lines engineered to express a plurality of MHC class I or class II alleles.

The samples can also include human cell lines obtained or derived from a plurality of patients.

The samples can also include fresh or frozen tumor samples obtained from a plurality of patients.

The samples can also include peptides identified using T-cell assays.

The training data set may further include data associated with: peptide abundance of the set of training peptides present in the samples; peptide length of the set of training peptides in the samples.

A method disclosed herein but not claimed can also include obtaining a set of training protein sequences based on the training peptide sequences by comparing the set of training peptide sequences via alignment to a database comprising a set of known protein sequences, wherein the set of training protein sequences are longer than and include the training peptide sequences.

A method disclosed herein but not claimed can also include performing or having performed mass spectrometry on a cell line to obtain at least one of exome, transcriptome, or whole genome nucleotide sequencing data from the cell line, the nucelotide sequencing data including at least one protein sequence including a mutation.

A method disclosed herein but not claimed can also include: encoding the training peptide sequences using a one-hot encoding scheme.

A method disclosed herein but not claimed can also include obtaining at least one of exome, transcriptome, and whole genome normal nucleotide sequencing data from normal tissue samples; and training the set of parameters of the presentation model using the normal nucleotide sequencing data.

The training data set may further include data associated with proteome sequences associated with the samples.

The training data set may further include data associated with MHC peptidome sequences associated with the samples.

The training data set may further include data associated with peptide-MHC binding affinity measurements for at least one of the isolated peptides.

The training data set may further include data associated with peptide-MHC binding stability measurements for at least one of the isolated peptides.

The training data set may further include data associated with transcriptomes associated with the samples.

The training data set may further include data associated with genomes associated with the samples.

A method disclosed herein but not claimed may also include logistically regressing the set of parameters.

The training peptide sequences may be lengths within a range of k-mers where k is between 8-15, inclusive for MHC class I or 9-30, inclusive for MHC class II.

A method disclosed herein but not claimed may also include encoding the training peptide sequences using a left-padded one-hot encoding scheme.

A method disclosed herein but not claimed may also include determining values for the set of parameters using a deep learning algorithm.

Disclosed herein but not claimed are methods for identifying one or more neoantigens that are likely to be presented on a tumor cell surface of a tumor cell, comprising executing the steps of: receiving mass spectrometry data comprising data associated with a plurality of isolated peptides eluted from major histocompatibility complex (MHC) derived from a plurality of fresh or frozen tumor samples; obtaining a training data set by at least identifying a set of training peptide sequences present in the tumor samples and presented on one or more MHC alleles associated with each training peptide sequence; obtaining a set of training protein sequences based on the training peptide sequences; and training a set of numerical parameters of a presentation model using the training protein sequences and the training peptide sequences, the presentation model providing a plurality of numerical likelihoods that peptide sequences from the tumor cell are presented by one or more MHC alleles on the tumor cell surface.

The presentation model may represent dependence between: presence of a pair of a particular one of the MHC alleles and a particular amino acid at a particular position of a peptide sequence; and likelihood of presentation on the tumor cell surface, by the particular one of the MHC alleles of the pair, of such a peptide sequence comprising the particular amino acid at the particular position.

A method disclosed herein but not claimed can also include selecting a subset of neoantigens, wherein the subset of neoantigens is selected because each has an increased likelihood that it is presented on the cell surface of the tumor relative to one or more distinct tumor neoantigens.

A method disclosed herein but not claimed can also include selecting a subset of neoantigens, wherein the subset of neoantigens is selected because each has an increased likelihood that it is capable of inducing a tumor-specific immune response in the subject relative to one or more distinct tumor neoantigens.

A method disclosed herein but not claimed can also include selecting a subset of neoantigens, wherein the subset of neoantigens is selected because each has an increased likelihood that it is capable of being presented to naive T cells by professional antigen presenting cells (APCs) relative to one or more distinct tumor neoantigens, optionally wherein the APC is a dendritic cell (DC).

A method disclosed herein but not claimed can also include selecting a subset of neoantigens, wherein the subset of neoantigens is selected because each has a decreased likelihood that it is subject to inhibition via central or peripheral tolerance relative to one or more distinct tumor neoantigens.

A method disclosed herein but not claimed can also include selecting a subset of neoantigens, wherein the subset of neoantigens is selected because each has a decreased likelihood that it is capable of inducing an autoimmune response to normal tissue in the subject relative to one or more distinct tumor neoantigens.

A method disclosed herein but not claimed can also include selecting a subset of neoantigens, wherein the subset of neoantigens is selected because each has a decreased likelihood that it will be differentially post-translationally modified in tumor cells versus APCs, optionally wherein the APC is a dendritic cell (DC).

The practice of the methods herein will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

## III. Identification of Tumor Specific Mutations in Neoantigens

Also disclosed herein but not claimed are methods for the identification of certain mutations (e.g., the variants or alleles that are present in cancer cells). In particular, these mutations can be present in the genome, transcriptome, proteome, or exome of cancer cells of a subject having cancer but not in normal tissue from the subject.

Genetic mutations in tumors can be considered useful for the immunological targeting of tumors if they lead to changes in the amino acid sequence of a protein exclusively in the tumor. Useful mutations include: (1) non-synonymous mutations leading to different amino acids in the protein; (2) read-through mutations in which a stop codon is modified or deleted, leading to translation of a longer protein with a novel tumor-specific sequence at the C-terminus; (3) splice site mutations that lead to the inclusion of an intron in the mature mRNA and thus a unique tumor-specific protein sequence; (4) chromosomal rearrangements that give rise to a chimeric protein with tumor-specific sequences at the junction of 2 proteins (i.e., gene fusion); (5) frameshift mutations or deletions that lead to a new open reading frame with a novel tumor-specific protein sequence. Mutations can also include one or more of nonframeshift indel, missense or nonsense substitution, splice site alteration, genomic rearrangement or gene fusion, or any genomic or expression alteration giving rise to a neoORF.

Peptides with mutations or mutated polypeptides arising from for example, splice-site, frameshift, readthrough, or gene fusion mutations in tumor cells can be identified by sequencing DNA, RNA or protein in tumor versus normal cells.

Also mutations can include previously identified tumor specific mutations. Known tumor mutations can be found at the Catalogue of Somatic Mutations in Cancer (COSMIC) database.

A variety of methods are available for detecting the presence of a particular mutation or allele in an individual's DNA or RNA. Advancements in this field have provided accurate, easy, and inexpensive large-scale SNP genotyping. For example, several techniques have been described including dynamic allele-specific hybridization (DASH), microplate array diagonal gel electrophoresis (MADGE), pyrosequencing, oligonucleotide-specific ligation, the TaqMan system as well as various DNA "chip" technologies such as the Affymetrix SNP chips. These methods utilize amplification of a target genetic region, typically by PCR. Still other methods, based on the generation of small signal molecules by invasive cleavage followed by mass spectrometry or immobilized padlock probes and rolling-circle amplification. Several of the methods known in the art for detecting specific mutations are summarized below.

PCR based detection means can include multiplex amplification of a plurality of markers simultaneously. For example, it is well known in the art to select PCR primers to generate PCR products that do not overlap in size and can be analyzed simultaneously. Alternatively, it is possible to amplify different markers with primers that are differentially labeled and thus can each be differentially detected. Of course, hybridization based detection means allow the differential detection of multiple PCR products in a sample. Other techniques are known in the art to allow multiplex analyses of a plurality of markers.

Several methods have been developed to facilitate analysis of single nucleotide polymorphisms in genomic DNA or cellular RNA. For example, a single base polymorphism can be detected by using a specialized exonuclease-resistant nucleotide, as disclosed, e.g., in Mundy, C. R. (U.S. Pat. No. 4,656,127). According to the method, a primer complementary to the allelic sequence immediately 3' to the polymorphic site is permitted to hybridize to a target molecule obtained from a particular animal or human. If the polymorphic site on the target molecule contains a nucleotide that is complementary to the particular exonuclease-resistant nucleotide derivative present, then that derivative will be incorporated onto the end of the hybridized primer. Such incorporation renders the primer resistant to exonuclease, and thereby permits its detection. Since the identity of the exonuclease-resistant derivative of the sample is known, a finding that the primer has become resistant to exonucleases reveals that the nucleotide(s) present in the polymorphic site of the target molecule is complementary to that of the nucleotide derivative used in the reaction. This method has the advantage that it does not require the determination of large amounts of extraneous sequence data.

A solution-based method can be used for determining the identity of a nucleotide of a polymorphic site. Cohen, D. et al. (French Patent 2,650,840; PCT Appln. No. WO91/02087). As in the Mundy method of U.S. Pat. No. 4,656,127, a primer is employed that is complementary to allelic sequences immediately 3' to a polymorphic site. The method determines the identity of the nucleotide of that site using labeled dideoxynucleotide derivatives, which, if complementary to the nucleotide of the polymorphic site will become incorporated onto the terminus of the primer.

An alternative method, known as Genetic Bit Analysis or GBA is described by Goelet, P. et al. (PCT Appln. No. 92/15712). The method of Goelet, P. et al. uses mixtures of labeled terminators and a primer that is complementary to the sequence 3' to a polymorphic site. The labeled terminator that is incorporated is thus determined by, and complementary to, the nucleotide present in the polymorphic site of the target molecule being evaluated. In contrast to the method of Cohen et al. (French Patent 2,650,840; PCT Appln. No. WO91/02087) the method of Goelet, P. et al. can be a heterogeneous phase assay, in which the primer or the target molecule is immobilized to a solid phase.

Several primer-guided nucleotide incorporation procedures for assaying polymorphic sites in DNA have been described (Komher, J. S. et al., Nucl. Acids. Res. 17:7779-7784 (1989); Sokolov, B. P., Nucl. Acids Res. 18:3671 (1990); Syvanen, A.-C., et al., Genomics 8:684-692 (1990); Kuppuswamy, M. N. et al., Proc. Natl. Acad. Sci. (U.S.A.) 88:1143-1147 (1991); Prezant, T. R. et al., Hum. Mutat. 1:159-164 (1992); Ugozzoli, L. et al., GATA 9:107-112 (1992); Nyren, P. et al., Anal. Biochem. 208:171-175 (1993)). These methods differ from GBA in that they utilize incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvanen, A.-C., et al., Amer. J. Hum. Genet. 52:46-59 (1993)).

A number of initiatives obtain sequence information directly from millions of individual molecules of DNA or RNA in parallel. Real-time single molecule sequencing-by-synthesis technologies rely on the detection of fluorescent nucleotides as they are incorporated into a nascent strand of DNA that is complementary to the template being sequenced. In one method, oligonucleotides 30-50 bases in length are covalently anchored at the 5' end to glass cover slips. These anchored strands perform two functions. First, they act as capture sites for the target template strands if the templates are configured with capture tails complementary to the surface-bound oligonucleotides. They also act as primers for the template directed primer extension that forms the basis of the sequence reading. The capture primers function as a fixed position site for sequence determination using multiple cycles of synthesis, detection, and chemical cleavage of the dye-linker to remove the dye. Each cycle consists of adding the polymerase/labeled nucleotide mixture, rinsing, imaging and cleavage of dye. In an alternative method, polymerase is modified with a fluorescent donor molecule and immobilized on a glass slide, while each nucleotide is color-coded with an acceptor fluorescent moiety attached to a gamma-phosphate. The system detects the interaction between a fluorescently-tagged polymerase and a fluorescently modified nucleotide as the nucleotide becomes incorporated into the de novo chain. Other sequencing-by-synthesis technologies also exist.

Any suitable sequencing-by-synthesis platform can be used to identify mutations. As described above, four major sequencing-by-synthesis platforms are currently available: the Genome Sequencers from Roche/454 Life Sciences, the 1G Analyzer from Illumina/Solexa, the SOLiD system from Applied BioSystems, and the Heliscope system from Helicos Biosciences. Sequencing-by-synthesis platforms have also been described by Pacific BioSciences and VisiGen Biotechnologies. In some embodiments, a plurality of nucleic acid molecules being sequenced is bound to a support (e.g., solid support). To immobilize the nucleic acid on a support, a capture sequence/universal priming site can be added at the 3' and/or 5' end of the template. The nucleic acids can be bound to the support by hybridizing the capture sequence to a complementary sequence covalently attached to the support. The capture sequence (also referred to as a universal capture sequence) is a nucleic acid sequence complementary to a sequence attached to a support that may dually serve as a universal primer.

As an alternative to a capture sequence, a member of a coupling pair (such as, e.g., antibody/antigen, receptor/ligand, or the avidin-biotin pair as described in, e.g., US Patent Application No. 2006/0252077) can be linked to each fragment to be captured on a surface coated with a respective second member of that coupling pair.

Subsequent to the capture, the sequence can be analyzed, for example, by single molecule detection/sequencing, e.g., as described in the Examples and in U.S. Pat. No. 7,283,337, including template-dependent sequencing-by-synthesis. In sequencing-by-synthesis, the surface-bound molecule is exposed to a plurality of labeled nucleotide triphosphates in the presence of polymerase. The sequence of the template is determined by the order of labeled nucleotides incorporated into the 3' end of the growing chain. This can be done in real time or can be done in a step-and-repeat mode. For real-time analysis, different optical labels to each nucleotide can be incorporated and multiple lasers can be utilized for stimulation of incorporated nucleotides.

Sequencing can also include other massively parallel sequencing or next generation sequencing (NGS) techniques and platforms. Additional examples of massively parallel sequencing techniques and platforms are the Illumina HiSeq or MiSeq, Thermo PGM or Proton, the Pac Bio RS II or Sequel, Qiagen's Gene Reader, and the Oxford Nanopore MinION. Additional similar current massively parallel sequencing technologies can be used, as well as future generations of these technologies.

Any cell type or tissue can be utilized to obtain nucleic acid samples for use in methods described herein. For example, a DNA or RNA sample can be obtained from a tumor or a bodily fluid, e.g., blood, obtained by known techniques (e.g. venipuncture) or saliva. Alternatively, nucleic acid tests can be performed on dry samples (e.g. hair or skin). In addition, a sample can be obtained for sequencing from a tumor and another sample can be obtained from normal tissue for sequencing where the normal tissue is of the same tissue type as the tumor. A sample can be obtained for sequencing from a tumor and another sample can be obtained from normal tissue for sequencing where the normal tissue is of a distinct tissue type relative to the tumor.

Tumors can include one or more of lung cancer, melanoma, breast cancer, ovarian cancer, prostate cancer, kidney cancer, gastric cancer, colon cancer, testicular cancer, head and neck cancer, pancreatic cancer, brain cancer, B-cell lymphoma, acute myelogenous leukemia, chronic myelogenous leukemia, chronic lymphocytic leukemia, and T cell lymphocytic leukemia, non-small cell lung cancer, and small cell lung cancer.

Alternatively, protein mass spectrometry can be used to identify or validate the presence of mutated peptides bound to MHC proteins on tumor cells. Peptides can be acid-eluted from tumor cells or from HLA molecules that are immunoprecipitated from tumor, and then identified using mass spectrometry.

## IV. Neoantigens

The invention relates to chimpanzee adenovirus vectors comprising a plurality of neoantigen-encoding nucleic acid sequences, as defined in the claims. Also described herein (not claimed) are isolated peptides that comprise tumor specific mutations identified by the methods disclosed herein, peptides that comprise known tumor specific mutations, and mutant polypeptides or fragments thereof identified by methods disclosed herein. Neoantigen peptides can be described in the context of their coding sequence where a neoantigen includes the nucleotide sequence (e.g., DNA or RNA) that codes for the related polypeptide sequence.

One or more polypeptides encoded by a neoantigen nucleotide sequence can comprise at least one of: a binding affinity with MHC with an IC50 value of less than 1000nM, for MHC Class 1 peptides a length of 8-15, 8, 9, 10, 11, 12, 13, 14, or 15 amino acids, presence of sequence motifs within or near the peptide promoting proteasome cleavage, and presence or sequence motifs promoting TAP transport.

One or more neoantigens can be presented on the surface of a tumor.

One or more neoantigens can be is immunogenic in a subject having a tumor, e.g., capable of eliciting a T cell response or a B cell response in the subject.

One or more neoantigens that induce an autoimmune response in a subject can be excluded from consideration in the context of vaccine generation for a subject having a tumor.

The size of at least one neoantigenic peptide molecule can comprise, but is not limited to, about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, about 31, about 32, about 33, about 34, about 35, about 36, about 37, about 38, about 39, about 40, about 41, about 42, about 43, about 44, about 45, about 46, about 47, about 48, about 49, about 50, about 60, about 70, about 80, about 90, about 100, about 110, about 120 or greater amino molecule residues, and any range derivable therein. In specific embodiments the neoantigenic peptide molecules are equal to or less than 50 amino acids.

Neoantigenic peptides and polypeptides can be: for MHC Class I 15 residues or less in length and usually consist of between about 8 and about 11 residues, particularly 9 or 10 residues; for MHC Class II, 15-24 residues.

If desirable, a longer peptide can be designed in several ways. In one case, when presentation likelihoods of peptides on HLA alleles are predicted or known, a longer peptide could consist of either: (1) individual presented peptides with an extensions of 2-5 amino acids toward the N- and C-terminus of each corresponding gene product; (2) a concatenation of some or all of the presented peptides with extended sequences for each. In another case, when sequencing reveals a long (>10 residues) neoepitope sequence present in the tumor (e.g. due to a frameshift, read-through or intron inclusion that leads to a novel peptide sequence), a longer peptide would consist of: (3) the entire stretch of novel tumor-specific amino acids--thus bypassing the need for computational or in vitro test-based selection of the strongest HLA-presented shorter peptide. In both cases, use of a longer peptide allows endogenous processing by patient cells and may lead to more effective antigen presentation and induction of T cell responses.

Neoantigenic peptides and polypeptides (not claimed) can be presented on an HLA protein. In some aspects (not claimed) neoantigenic peptides and polypeptides are presented on an HLA protein with greater affinity than a wild-type peptide. In some aspects, a neoantigenic peptide or polypeptide can have an IC50 of at least less than 5000 nM, at least less than 1000 nM, at least less than 500 nM, at least less than 250 nM, at least less than 200 nM, at least less than 150 nM, at least less than 100 nM, at least less than 50 nM or less.

In some aspects (not claimed), neoantigenic peptides and polypeptides do not induce an autoimmune response and/or invoke immunological tolerance when administered to a subject.

The invention relates to chimpanzee adenovirus vectors comprising a plurality of neoantigen-encoding nucleic acid sequences, as defined in the claims. Also described (not claimed) are compositions comprising at least two or more neoantigenic peptides. In some embodiments (not claimed) the composition contains at least two distinct peptides. At least two distinct peptides can be derived from the same polypeptide. By distinct polypeptides is meant that the peptide vary by length, amino acid sequence, or both. The peptides are derived from any polypeptide known to or have been found to contain a tumor specific mutation. Suitable polypeptides from which the neoantigenic peptides can be derived can be found for example in the COSMIC database. COSMIC curates comprehensive information on somatic mutations in human cancer. The peptide contains the tumor specific mutation, which may be a driver mutation for a particular cancer type.

Neoantigenic peptides and polypeptides having a desired activity or property can be modified to provide certain desired attributes, e.g., improved pharmacological characteristics, while increasing or at least retaining substantially all of the biological activity of the unmodified peptide to bind the desired MHC molecule and activate the appropriate T cell. For instance, neoantigenic peptide and polypeptides can be subject to various changes, such as substitutions, either conservative or non-conservative, where such changes might provide for certain advantages in their use, such as improved MHC binding, stability or presentation. By conservative substitutions is meant replacing an amino acid residue with another which is biologically and/or chemically similar, e.g., one hydrophobic residue for another, or one polar residue for another. The substitutions include combinations such as Gly, Ala; Val, Ile, Leu, Met; Asp, Glu; Asn, Gln; Ser, Thr; Lys, Arg; and Phe, Tyr. The effect of single amino acid substitutions may also be probed using D-amino acids. Such modifications can be made using well known peptide synthesis procedures, as described in e.g., Merrifield, Science 232:341-347 (1986), Barany & Merrifield, The Peptides, Gross & Meienhofer, eds. (N.Y., Academic Press), pp. 1-284 (1979); and Stewart & Young, Solid Phase Peptide Synthesis, (Rockford, Ill., Pierce), 2d Ed. (1984).

Proteins or peptides can be made by any technique known to those of skill in the art, including the expression of proteins, polypeptides or peptides through standard molecular biological techniques, the isolation of proteins or peptides from natural sources, or the chemical synthesis of proteins or peptides. The nucleotide and protein, polypeptide and peptide sequences corresponding to various genes have been previously disclosed, and can be found at computerized databases known to those of ordinary skill in the art. One such database is the National Center for Biotechnology Information's Genbank and GenPept databases located at the National Institutes of Health website. The coding regions for known genes can be amplified and/or expressed using the techniques disclosed herein or as would be known to those of ordinary skill in the art. Alternatively, various commercial preparations of proteins, polypeptides and peptides are known to those of skill in the art.

## V. Vaccine Compositions

Also disclosed herein (not claimed) is an immunogenic composition, e.g., a vaccine composition, capable of raising a specific immune response, e.g., a tumor-specific immune response. Vaccine compositions typically comprise a plurality of neoantigens, e.g., selected using a method described herein. Vaccine compositions can also be referred to as vaccines. The invention relates to chimpanzee adenovirus vectors as defined in the claims. Other vaccine compositions are also described below (not claimed).

A vaccine can contain between 1 and 100 or more nucleotide sequences, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94,95, 96, 97, 98, 99, 100 or more different nucleotide sequences, 6, 7, 8, 9, 10 11, 12, 13, or 14 different nucleotide sequences, or 12, 13 or 14 different nucleotide sequences. A vaccine can contain between 1 and 30 neoantigen sequences, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94,95, 96, 97, 98, 99, 100 or more different neoantigen sequences, 6, 7, 8, 9, 10 11, 12, 13, or 14 different neoantigen sequences, or 12, 13 or 14 different neoantigen sequences.

In one embodiment, nucleotide sequences encoding different peptides and/or polypeptides are selected so that the peptides and/or polypeptides capable of associating with different MHC molecules, such as different MHC class I molecule. In some aspects, one vaccine composition comprises coding sequence for peptides and/or polypeptides capable of associating with the most frequently occurring MHC class I molecules. Hence, vaccine compositions can comprise different fragments capable of associating with at least 2 preferred, at least 3 preferred, or at least 4 preferred MHC class I molecules.

The vaccine composition can be capable of raising a specific cytotoxic T-cells response and/or a specific helper T-cell response.

A carrier (or excipient) can be present. The function of a carrier can for example be to increase the molecular weight of in particular mutant to increase activity or immunogenicity, to confer stability, to increase the biological activity, or to increase serum half-life. Furthermore, a carrier can aid presenting peptides to T-cells. A carrier can be any suitable carrier known to the person skilled in the art, for example a protein or an antigen presenting cell. A carrier protein could be but is not limited to keyhole limpet hemocyanin, serum proteins such as transferrin, bovine serum albumin, human serum albumin, thyroglobulin or ovalbumin, immunoglobulins, or hormones, such as insulin or palmitic acid. For immunization of humans, the carrier is generally a physiologically acceptable carrier acceptable to humans and safe. However, tetanus toxoid and/or diptheria toxoid are suitable carriers. Alternatively, the carrier can be dextrans for example sepharose.

Cytotoxic T-cells (CTLs) recognize an antigen in the form of a peptide bound to an MHC molecule rather than the intact foreign antigen itself. The MHC molecule itself is located at the cell surface of an antigen presenting cell. Thus, an activation of CTLs is possible if a trimeric complex of peptide antigen, MHC molecule, and APC is present. Correspondingly, it may enhance the immune response if not only the peptide is used for activation of CTLs, but if additionally APCs with the respective MHC molecule are added. Therefore, in some embodiments a vaccine composition additionally contains at least one antigen presenting cell.

## V.A. Neoantigen Cassette

The methods employed for the selection of one or more neoantigens, the cloning and construction of a "cassette" and its insertion into a viral vector are within the skill in the art given the teachings provided herein. By "neoantigen cassette" is meant the combination of a plurality of neoantigens, as defined in the claims, and the other regulatory elements necessary to transcribe the neoantigen(s) and express the transcribed product. A neoantigen or plurality of neoantigens can be operatively linked to regulatory components in a manner which permits transcription. Such components include conventional regulatory elements that can drive expression of the neoantigen(s) in a cell transfected with the viral vector. Thus the neoantigen cassette contains a selected promoter which is linked to the neoantigen(s) and located, with other, optional regulatory elements, within the selected viral sequences of the recombinant vector.

Useful promoters can be constitutive promoters or regulated (inducible) promoters, which will enable control of the amount of neoantigen(s) to be expressed. For example, a desirable promoter is that of the cytomegalovirus immediate early promoter/enhancer [see, e.g., Boshart et al, Cell, 41:521-530 (1985)]. Another desirable promoter includes the Rous sarcoma virus LTR promoter/enhancer. Still another promoter/enhancer sequence is the chicken cytoplasmic beta-actin promoter [T. A. Kost et al, Nucl. Acids Res., 11(23):8287 (1983)]. Other suitable or desirable promoters can be selected by one of skill in the art.

The neoantigen cassette can also include nucleic acid sequences heterologous to the viral vector sequences including sequences providing signals for efficient polyadenylation of the transcript (poly-A or pA) and introns with functional splice donor and acceptor sites. A common poly-A sequence which is employed in the exemplary vectors of this invention is that derived from the papovavirus SV-40. The poly-A sequence generally can be inserted in the cassette following the neoantigen-based sequences and before the viral vector sequences. A common intron sequence can also be derived from SV-40, and is referred to as the SV-40 T intron sequence. A neoantigen cassette can also contain such an intron, located between the promoter/enhancer sequence and the neoantigen(s). Selection of these and other common vector elements are conventional [see, e.g., Sambrook et al, "Molecular Cloning. A Laboratory Manual.", 2d edit., Cold Spring Harbor Laboratory, New York (1989) and references cited therein] and many such sequences are available from commercial and industrial sources as well as from Genbank.

A neoantigen cassette as defined in the claims encodes a plurality of neoantigens. For example, a given cassette can include 2-10, 2-20, 2-30, 10-20, 15-25, 15-20, 2, 3, 4, 5, 6, 7, 8, 9, 10, 11, 12, 13, 14, 15, 16, 17, 18, 19, 20, or more neoantigens. Neoantigens are linked to one another with linkers as defined in the claims. Neoantigens can be in any orientation relative to one another including N to C or C to N.

As above stated, the neoantigen cassette can be located in the site of any selected deletion in the viral vector, such as the site of the E1 gene region deletion or E3 gene region deletion, among others which may be selected.

## V.B. Immune Checkpoints

Chimpanzee adenovirus vectors as defined in the claims, such as C68 vectors described herein, can comprise a nucleic acid which encodes a plurality of neoantigens and the same or a separate vector can comprise a nucleic acid which encodes at least one immune modulator (e.g., an antibody such as an scFv) which binds to and blocks the activity of an immune checkpoint molecule. Vectors can comprise a neoantigen cassette and one or more nucleic acid molecules encoding a checkpoint inhibitor.

Illustrative immune checkpoint molecules that can be targeted for blocking or inhibition include, but are not limited to, CTLA-4, 4-1BB (CD137), 4-1BBL (CD137L), PDL1, PDL2, PD1, B7-H3, B7-H4, BTLA, HVEM, TIM3, GAL9, LAG3, TIM3, B7H3, B7H4, VISTA, KIR, 2B4 (belongs to the CD2 family of molecules and is expressed on all NK, $\gamma\delta$, and memory CD8+ ($\alpha\beta$) Tcells), CD160 (also referred to as BY55), and CGEN-15049. Immune checkpoint inhibitors include antibodies, or antigen binding fragments thereof, or other binding proteins, that bind to and block or inhibit the activity of one or more of CTLA-4, PDL1, PDL2, PD1, B7-H3, B7-H4, BTLA, HVEM, TIM3, GAL9, LAG3, TIM3, B7H3, B7H4, VISTA, KIR, 2B4, CD160, and CGEN-15049. Illustrative immune checkpoint inhibitors include Tremelimumab (CTLA-4 blocking antibody), anti-OX40, PD-L1 monoclonal Antibody (Anti-B7-H1; MEDI4736), ipilimumab, MK-3475 (PD-1 blocker), Nivolumamb (anti-PD1 antibody), CT-011 (anti-PD1 antibody), BY55 monoclonal antibody, AMP224 (anti-PDL1 antibody), BMS-936559 (anti-PDL1 antibody), MPLDL3280A (anti-PDL1 antibody), MSB0010718C (anti-PDL1 antibody) and Yervoy/ipilimumab (anti-CTLA-4 checkpoint inhibitor). Antibody-encoding sequences can be engineered into vectors such as C68 using ordinary skill in the art. An exemplary method is described in Fang et al., Stable antibody expression at therapeutic levels using the 2A peptide. Nat Biotechnol. 2005 May;23(5):584-90. Epub 2005 Apr 17.

## V.C. Additional Considerations for Vaccine Design and Manufacture

### V.C.1. Determination of a Set of Peptides that Cover All Tumor Subclones

Truncal peptides, meaning those presented by all or most tumor subclones, can be prioritized for inclusion into the vaccine.[53] Optionally, if there are no truncal peptides predicted to be presented and immunogenic with high probability, or if the number of truncal peptides predicted to be presented and immunogenic with high probability is small enough that additional non-truncal peptides can be included in the vaccine, then further peptides can be prioritized by estimating the number and identity of tumor subclones and choosing peptides so as to maximize the number of tumor subclones covered by the vaccine.[54]

### V.C.2. Neoantigen Prioritization

After all of the above above neoantigen filters are applied, more candidate neoantigens may still be available for vaccine inclusion than the vaccine technology can support. Additionally, uncertainty about various aspects of the neoantigen analysis may remain and tradeoffs may exist between different properties of candidate vaccine neoantigens. Thus, in place of predetermined filters at each step of the selection process, an integrated multidimensional model can be considered that places candidate neoantigens in a space with at least the following axes and optimizes selection using an integrative approach.

1. Risk of auto-immunity or tolerance (risk of germline) (lower risk of auto-immunity is typically preferred)
2. Probability of sequencing artifact (lower probability of artifact is typically preferred)

3. Probability of immunogenicity (higher probability of immunogenicity is typically preferred)

4. Probability of presentation (higher probability of presentation is typically preferred)

5. Gene expression (higher expression is typically preferred)

6. Coverage of HLA genes (larger number of HLA molecules involved in the presentation of a set of neoantigens may lower the probability that a tumor will escape immune attack via downregulation or mutation of HLA molecules)

### V.D. Alphavirus

### V.D.1. Alphavirus Biology

Alphaviruses are members of the family *Togaviridae,* and are positive-sense single stranded RNA viruses. Alphaviruses can also be referred to as self-replicating RNA or srRNA. Members are typically classified as either Old World, such as Sindbis, Ross River, Mayaro, Chikungunya, and Semliki Forest viruses, or New World, such as eastern equine encephalitis, Aura, Fort Morgan, or Venezuelan equine encephalitis virus and its derivative strain TC-83 (Strauss Microbrial Review 1994). A natural alphavirus genome is typically around 12kb in length, the first two-thirds of which contain genes encoding non-structural proteins (nsPs) that form RNA replication complexes for self-replication of the viral genome, and the last third of which contains a subgenomic expression cassette encoding structural proteins for virion production (Frolov RNA 2001).

A model lifecycle of an alphavirus involves several distinct steps (Strauss Microbrial Review 1994, Jose Future Microbiol 2009). Following virus attachment to a host cell, the virion fuses with membranes within endocytic compartments resulting in the eventual release of genomic RNA into the cytosol. The genomic RNA, which is in a plus-strand orientation and comprises a 5' methylguanylate cap and 3' poly A tail, is translated to produce non-structural proteins nsP1-4 that form the replication complex. Early in infection, the plus-strand is then replicated by the complex into a minus-stand template. In the current model, the replication complex is further processed as infection progresses, with the resulting processed complex switching to transcription of the minus-strand into both full-length positive-strand genomic RNA, as well as the 26S subgenomic positive-strand RNA containing the structural genes. Several conserved sequence elements (CSEs) of alphavirus have been identified to potentially play a role in the various RNA replication steps including; a complement of the 5' UTR in the replication of plus-strand RNAs from a minus-strand template, a 51-nt CSE in the replication of minus-strand synthesis from the genomic template, a 24-nt CSE in the junction region between the nsPs and the 26S RNA in the transcription of the subgenomic RNA from the minus-strand, and a 3' 19-nt CSE in minus-strand synthesis from the plus-strand template.

Following the replication of the various RNA species, virus particles are then typically assembled in the natural lifecycle of the virus. The 26S RNA is translated and the resulting proteins further processed to produce the structural proteins including capsid protein, glycoproteins E1 and E2, and two small polypeptides E3 and 6K (Strauss 1994). Encapsidation of viral RNA occurs, with capsid proteins normally specific for only genomic RNA being packaged, followed by virion assembly and budding at the membrane surface.

### V.D.2. Alphavirus as a delivery vector

The invention is directed to a chimpanzee adenoviral vector as defined in the claims. Alphaviruses are not claimed. Alphaviruses have previously been engineered for use as expression vector systems (Pushko 1997, Rheme 2004). Alphaviruses offer several advantages, particularly in a vaccine setting where heterologous antigen expression can be desired. Due to its ability to self-replicate in the host cytosol, alphavirus vectors are generally able to produce high copy numbers of the expression cassette within a cell resulting in a high level of heterologous antigen production. Additionally, the vectors are generally transient, resulting in improved biosafety as well as reduced induction of immunological tolerance to the vector. The public, in general, also lacks pre-existing immunity to alphavirus vectors as compared to other standard viral vectors, such as human adenovirus. Alphavirus based vectors also generally result in cytotoxic responses to infected cells. Cytotoxicity, to a certain degree, can be important in a vaccine setting to properly illicit an immune response to the heterologous antigen expressed. However, the degree of desired cytotoxicity can be a balancing act, and thus several attenuated alphaviruses have been developed, including the TC-83 strain of VEE. Thus, an example of a neoantigen expression vector described herein (not claimed) can utilize an alphavirus backbone that allows for a high level of neoantigen expression, elicits a robust immune response to neoantigen, does not elicit an immune response to the vector itself, and can be used in a safe manner. Furthermore, the neoantigen expression cassette can be designed to elicit different levels of an immune response through optimization of which alphavirus sequences the vector uses, including, but not limited to, sequences derived from VEE or its attenuated derivative TC-83.

Several expression vector design strategies (not claimed) have been engineered using alphavirus sequences (Pushko 1997). In one strategy (not claimed), a alphavirus vector design includes inserting a second copy of the 26S promoter sequence elements downstream of the structural protein genes, followed by a heterologous gene (Frolov 1993).

Thus, in addition to the natural non-structural and structural proteins, an additional subgenomic RNA is produced that expresses the heterologous protein. In this system, all the elements for production of infectious virions are present and, therefore, repeated rounds of infection of the expression vector in non-infected cells can occur.

Another expression vector design (not claimed) makes use of helper virus systems (Pushko 1997). In this strategy, the structural proteins are replaced by a heterologous gene. Thus, following self-replication of viral RNA mediated by still intact non-structural genes, the 26S subgenomic RNA provides for expression of the heterologous protein. Traditionally, additional vectors that expresses the structural proteins are then supplied *in trans,* such as by co-transfection of a cell line, to produce infectious virus. A system is described in detail in USPN 8,093,021. The helper vector system provides the benefit of limiting the possibility of forming infectious particles and, therefore, improves biosafety. In addition, the helper vector system reduces the total vector length, potentially improving the replication and expression efficiency. Thus, an example of a neoantigen expression vector described herein (not claimed) can utilize an alphavirus backbone wherein the structural proteins are replaced by a neoantigen cassette, the resulting vector both reducing biosafety concerns, while at the same time promoting efficient expression due to the reduction in overall expression vector size.

### V.D.3. Alphavirus production *in vitro*

Alphavirus delivery vectors (not claimed) are generally positive-sense RNA polynucleotides. A convenient technique well-known in the art for RNA production is *in vitro* transcription IVT. In this technique, a DNA template of the desired vector is first produced by techniques well-known to those in the art, including standard molecular biology techniques such as cloning, restriction digestion, ligation, gene synthesis, and polymerase chain reaction (PCR). The DNA template contains a RNA polymerase promoter at the 5' end of the sequence desired to be transcribed into RNA. Promoters include, but are not limited to, bacteriophage polymerase promoters such as T3, T7, or SP6. The DNA template is then incubated with the appropriate RNA polymerase enzyme, buffer agents, and nucleotides (NTPs). The resulting RNA polynucleotide can optionally be further modified including, but limited to, addition of a 5' cap structure such as 7-methylguanosine or a related structure, and optionally modifying the 3' end to include a polyadenylate (polyA) tail. The RNA can then be purified using techniques well-known in the field, such as phenol-chloroform extraction.

### V.D.4. Delivery via lipid nanoparticle

An important aspect to consider in vaccine vector design is immunity against the vector itself (Riley 2017). This may be in the form of preexisting immunity to the vector itself, such as with certain human adenovirus systems, or in the form of developing immunity to the vector following administration of the vaccine. The latter is an important consideration if multiple administrations of the same vaccine are performed, such as separate priming and boosting doses, or if the same vaccine vector system is to be used to deliver different neoantigen cassettes.

In the case of alphavirus vectors (not claimed), the standard delivery method is the previously discussed helper virus system that provides capsid, E1, and E2 proteins *in trans* to produce infectious viral particles. However, it is important to note that the E1 and E2 proteins are often major targets of neutralizing antibodies (Strauss 1994). Thus, the efficacy of using alphavirus vectors to deliver neoantigens of interest to target cells may be reduced if infectious particles are targeted by neutralizing antibodies.

Nucleic-acid vectors, such as expression vectors, exposed directly to serum can have several undesirable consequences, including degradation of the nucleic acid by serum nucleases or off-target stimulation of the immune system by the free nucleic acids. Therefore, encapsulation of an alphavirus vector (not claimed) can be used to avoid degradation, while also avoiding potential off-target affects. In certain examples (not claimed), an alphavirus vector is fully encapsulated within the delivery vehicle, such as within the aqueous interior of an LNP. Encapsulation of the alphavirus vector within an LNP can be carried out by techniques well-known to those skilled in the art, such as microfluidic mixing and droplet generation carried out on a microfluidic droplet generating device. Such devices include, but are not limited to, standard T-junction devices or flow-focusing devices. In an example (not claimed), the desired lipid formulation, such as MC3 or MC3-like containing compositions, is provided to the droplet generating device in parallel with the alphavirus delivery vector and other desired agents, such that the delivery vector and desired agents are fully encapsulated within the interior of the MC3 or MC3-like based LNP. In an example (not claimed), the droplet generating device can control the size range and size distribution of the LNPs produced. For example, the LNP can have a size ranging from 1 to 1000 nanometers in diameter, e.g., 1, 10, 50, 100, 500, or 1000 nanometers. Following droplet generation, the delivery vehicles encapsulating the expression vectors can be further treated or modified to prepare them for administration.

### V.E. Chimpanzee adenovirus (ChAd)

### V.E.1. Viral delivery with chimpanzee adenovirus

Vaccine compositions for delivery of one or more neoantigens (via a neoantigen cassette) are provided in the invention by providing adenovirus nucleotide sequences of chimpanzee origin as defined in the claims. A nucleotide sequence of a chimpanzee C68 adenovirus (also referred to herein as ChAdV68) can be used in a vaccine composition for neoantigen delivery *(See* SEQ ID NO: 1). Use of C68 adenovirus derived vectors is described in further detail in USPN 6,083,716.

In some embodiments, the chimpanzee adenovirus vector as defined in claim 1 is a recombinant adenovirus comprising the DNA sequence of a chimpanzee adenovirus such as C68 and a neoantigen cassette as defined in claim 1 operatively linked to regulatory sequences directing its expression. The recombinant virus is capable of infecting a mammalian, preferably a human, cell and capable of expressing the neoantigen cassette product in the cell. In this vector, the native chimpanzee E1 gene, and/or E3 gene, and/or E4 gene can be deleted. A neoantigen cassette as defined in claim 1 can be inserted into any of these sites of gene deletion. The neoantigen cassette is as defined in claim 1 and encodes a plurality of neoantigens against which a primed immune response is desired.

Still another aspect provides the recombinant chimpanzee adenovirus of the invention for use in a method for eliciting an immune response in a mammalian host to treat cancer. The method can comprise the step of administering to the host an effective amount of a recombinant chimpanzee adenovirus, such as C68, comprising a neoantigen cassette in accordance with the claimed invention that encodes one or more neoantigens from the tumor against which the immune response is targeted.

In some embodiments, a chimpanzee adenovirus vector as defined in claim 1 comprises a chimpanzee adenovirus DNA sequence obtained from SEQ ID NO: 1 and a neoantigen cassette as defined in claim 1 operatively linked to one or more regulatory sequences which direct expression of the cassette in a heterologous host cell, wherein the chimpanzee adenovirus DNA sequence comprises at least the cis-elements necessary for replication and virion encapsidation, the cis-elements flanking the neoantigen cassette and regulatory sequences. The chimpanzee adenovirus DNA sequence may comprise a gene selected from the group consisting of E1A, E1B, E2A, E2B, E3, E4, L1, L2, L3, L4 and L5 gene sequences of SEQ ID NO: 1. The vector can lack the E1A and/or E1B gene.

### V.E.2. E1-Expressing Complementation Cell Lines

To generate recombinant chimpanzee adenoviruses (Ad) deleted in any of the genes described herein, the function of the deleted gene region, if essential to the replication and infectivity of the virus, can be supplied to the recombinant virus by a helper virus or cell line, i.e., a complementation or packaging cell line. For example, to generate a replication-defective chimpanzee adenovirus vector, a cell line can be used which expresses the E1 gene products of the human or chimpanzee adenovirus; such a cell line can include HEK293 or variants thereof. The protocol for the generation of the cell lines expressing the chimpanzee E1 gene products (Examples 3 and 4 of USPN 6,083,716) can be followed to generate a cell line which expresses any selected chimpanzee adenovirus gene.

An AAV augmentation assay can be used to identify a chimpanzee adenovirus E1-expressing cell line. This assay is useful to identify E1 function in cell lines made by using the E1 genes of other uncharacterized adenoviruses, e.g., from other species. That assay is described in Example 4B of USPN 6,083,716.

A selected chimpanzee adenovirus gene, e.g., E1, can be under the transcriptional control of a promoter for expression in a selected parent cell line. Inducible or constitutive promoters can be employed for this purpose. Among inducible promoters are included the sheep metallothionine promoter, inducible by zinc, or the mouse mammary tumor virus (MMTV) promoter, inducible by a glucocorticoid, particularly, dexamethasone. Other inducible promoters, such as those identified in International patent application WO95/13392, can also be used in the production of packaging cell lines. Constitutive promoters in control of the expression of the chimpanzee adenovirus gene can be employed also.

A parent cell can be selected for the generation of a novel cell line expressing any desired C68 gene. Without limitation, such a parent cell line can be HeLa [ATCC Accession No. CCL 2], A549 [ATCC Accession No. CCL 185], KB [CCL 17], Detroit [e.g., Detroit 510, CCL 72] and WI-38 [CCL 75] cells. Other suitable parent cell lines can be obtained from other sources. Parent cell lines can include CHO, HEK293 or variants thereof, 911, HeLa, A549, LP-293, PER.C6, or AE1-2a.

An E1-expressing cell line can be useful in the generation of recombinant chimpanzee adenovirus E1 deleted vectors. Cell lines constructed using essentially the same procedures that express one or more other chimpanzee adenoviral gene products are useful in the generation of recombinant chimpanzee adenovirus vectors deleted in the genes that encode those products. Further, cell lines which express other human Ad E1 gene products are also useful in generating chimpanzee recombinant Ads.

### V.E.3. Recombinant Viral Particles as Vectors

The chimpanzee adenovirus vectors as defined in the claims comprise a chimpanzee adenovirus DNA sequence such as C68 and a neoantigen cassette with the features defined in claim 1 operatively linked to regulatory sequences which direct expression of the cassette. The C68 vector is capable of expressing the cassette in an infected mammalian cell. The C68 vector can be functionally deleted in one or more viral genes. Optional helper viruses and/or packaging cell lines can supply to the chimpanzee viral vector any necessary products of deleted adenoviral genes.

The term "functionally deleted" means that a sufficient amount of the gene region is removed or otherwise altered, e.g., by mutation or modification, so that the gene region is no longer capable of producing one or more functional products of gene expression. If desired, the entire gene region can be removed.

Modifications of the nucleic acid sequences forming the vectors disclosed herein, including sequence deletions, insertions, and other mutations may be generated using standard molecular biological techniques and are within the scope of this invention.

### V.E.4. Construction of The Viral Plasmid Vector

The chimpanzee adenovirus C68 vectors useful in this invention include recombinant, defective adenoviruses, that is, chimpanzee adenovirus sequences functionally deleted in the E1a or E1b genes, and optionally bearing other mutations, e.g., temperature-sensitive mutations or deletions in other genes. It is anticipated that these chimpanzee sequences are also useful in forming hybrid vectors from other adenovirus and/or adeno-associated virus sequences. Homologous adenovirus vectors prepared from human adenoviruses are described in the published literature [see, for example, Kozarsky I and II, cited above, and references cited therein, U.S. Pat. No. 5,240,846].

In the construction of useful chimpanzee adenovirus C68 vectors for delivery of a neoantigen cassette to a human (or other mammalian) cell, a range of adenovirus nucleic acid sequences can be employed in the vectors. A vector comprising minimal chimpanzee C68 adenovirus sequences can be used in conjunction with a helper virus to produce an infectious recombinant virus particle. The helper virus provides essential gene products required for viral infectivity and propagation of the minimal chimpanzee adenoviral vector. When only one or more selected deletions of chimpanzee adenovirus genes are made in an otherwise functional viral vector, the deleted gene products can be supplied in the viral vector production process by propagating the virus in a selected packaging cell line that provides the deleted gene functions in trans.

### V.E.5. Recombinant Minimal Adenovirus

A minimal chimpanzee Ad C68 virus is a viral particle containing just the adenovirus cis-elements necessary for replication and virion encapsidation. That is, the vector contains the cis-acting 5' and 3' inverted terminal repeat (ITR) sequences of the adenoviruses (which function as origins of replication) and the native 5' packaging/enhancer domains (that contain sequences necessary for packaging linear Ad genomes and enhancer elements for the E1 promoter). See, for example, the techniques described for preparation of a "minimal" human Ad vector in International Patent Application WO96/13597.

### V.E.6. Other Defective Adenoviruses

Recombinant, replication-deficient adenoviruses can also contain more than the minimal chimpanzee adenovirus sequences. These other Ad vectors can be characterized by deletions of various portions of gene regions of the virus, and infectious virus particles formed by the optional use of helper viruses and/or packaging cell lines.

As one example, suitable vectors may be formed by deleting all or a sufficient portion of the C68 adenoviral immediate early gene E1a and delayed early gene E1b, so as to eliminate their normal biological functions. Replication-defective E1-deleted viruses are capable of replicating and producing infectious virus when grown on a chimpanzee adenovirus-transformed, complementation cell line containing functional adenovirus E1a and E1b genes which provide the corresponding gene products in trans. Based on the homologies to known adenovirus sequences, it is anticipated that, as is true for the human recombinant E1-deleted adenoviruses of the art, the resulting recombinant chimpanzee adenovirus is capable of infecting many cell types and can express neoantigen(s), but cannot replicate in most cells that do not carry the chimpanzee E1 region DNA unless the cell is infected at a very high multiplicity of infection.

As another example, all or a portion of the C68 adenovirus delayed early gene E3 can be eliminated from the chimpanzee adenovirus sequence which forms a part of the recombinant virus.

Chimpanzee adenovirus C68 vectors can also be constructed having a deletion of the E4 gene. Still another vector can contain a deletion in the delayed early gene E2a.

Deletions can also be made in any of the late genes L1 through L5 of the chimpanzee C68 adenovirus genome.

Similarly, deletions in the intermediate genes IX and IVa2 can be useful for some purposes. Other deletions may be made in the other structural or non-structural adenovirus genes.

The above discussed deletions can be used individually, i.e., an adenovirus sequence can contain deletions of E1 only. Alternatively, deletions of entire genes or portions thereof effective to destroy or reduce their biological activity can be used in any combination. For example, in one exemplary vector, the adenovirus C68 sequence can have deletions of the E1 genes and the E4 gene, or of the E1, E2a and E3 genes, or of the E1 and E3 genes, or of E1, E2a and E4 genes, with or without deletion of E3, and so on. As discussed above, such deletions can be used in combination with other mutations, such as temperature-sensitive mutations, to achieve a desired result.

The cassette comprising neoantigen(s) be inserted optionally into any deleted region of the chimpanzee C68 Ad virus. Alternatively, the cassette can be inserted into an existing gene region to disrupt the function of that region, if desired.

### V.E.7. Helper Viruses

Depending upon the chimpanzee adenovirus gene content of the viral vectors employed to carry the neoantigen cassette, a helper adenovirus or non-replicating virus fragment can be used to provide sufficient chimpanzee adenovirus gene sequences to produce an infective recombinant viral particle containing the cassette.

Useful helper viruses contain selected adenovirus gene sequences not present in the adenovirus vector construct and/or not expressed by the packaging cell line in which the vector is transfected. A helper virus can be replication-defective and contain a variety of adenovirus genes in addition to the sequences described above. The helper virus can be used in combination with the E1-expressing cell lines described herein.

For C68, the "helper" virus can be a fragment formed by clipping the C terminal end of the C68 genome with SspI, which removes about 1300 bp from the left end of the virus. This clipped virus is then co-transfected into an E1-expressing cell line with the plasmid DNA, thereby forming the recombinant virus by homologous recombination with the C68 sequences in the plasmid.

Helper viruses can also be formed into poly-cation conjugates as described in Wu et al, J. Biol. Chem., 264:16985-16987 (1989); K. J. Fisher and J. M. Wilson, Biochem. J., 299:49 (Apr. 1, 1994). Helper virus can optionally contain a reporter gene. A number of such reporter genes are known to the art. The presence of a reporter gene on the helper virus which is different from the neoantigen cassette on the adenovirus vector allows both the Ad vector and the helper virus to be independently monitored. This second reporter is used to enable separation between the resulting recombinant virus and the helper virus upon purification.

### V.E.8. Assembly of Viral Particle and Infection of a Cell Line

The invention as claimed relates to chimpanzee adenovirus vectors. Other vectors are also described but not claimed. Assembly of the selected DNA sequences of the adenovirus, the neoantigen cassette, and other vector elements into various intermediate plasmids and shuttle vectors, and the use of the plasmids and vectors to produce a recombinant viral particle can all be achieved using conventional techniques. Such techniques include conventional cloning techniques of cDNA, in vitro recombination techniques (e.g., Gibson assembly), use of overlapping oligonucleotide sequences of the adenovirus genomes, polymerase chain reaction, and any suitable method which provides the desired nucleotide sequence. Standard transfection and co-transfection techniques are employed, e.g., CaPO4 precipitation techniques or liposome-mediated transfection methods such as lipofectamine. Other conventional methods employed include homologous recombination of the viral genomes, plaquing of viruses in agar overlay, methods of measuring signal generation, and the like.

For example, following the construction and assembly of the desired neoantigen cassette-containing chimpanzee adenoviral vector as claimed, the vector can be transfected in vitro in the presence of a helper virus into the packaging cell line. Homologous recombination occurs between the helper and the vector sequences, which permits the adenovirus-neoantigen sequences in the vector to be replicated and packaged into virion capsids, resulting in the recombinant viral vector particles.

The resulting recombinant chimpanzee C68 adenoviruses are useful in transferring a neoantigen cassette to a selected cell. In in vivo experiments with the recombinant virus grown in the packaging cell lines, the E1-deleted recombinant chimpanzee adenovirus demonstrates utility in transferring a cassette to a non-chimpanzee, preferably a human, cell.

### V.E.9. Use of the Recombinant Virus Vectors

The resulting recombinant chimpanzee C68 adenovirus containing the neoantigen cassette (produced by cooperation of the adenovirus vector and helper virus or adenoviral vector and packaging cell line, as described above) thus provides an efficient gene transfer vehicle which can deliver neoantigen(s) to a subject in vivo or ex vivo.

The above-described recombinant vectors are administered to humans according to published methods for gene therapy. A chimpanzee viral vector bearing a neoantigen cassette can be administered to a patient, preferably suspended in a biologically compatible solution or pharmaceutically acceptable delivery vehicle. A suitable vehicle includes sterile saline. Other aqueous and non-aqueous isotonic sterile injection solutions and aqueous and non-aqueous sterile suspensions known to be pharmaceutically acceptable carriers and well known to those of skill in the art may be employed for this purpose.

The chimpanzee adenoviral vectors are administered in sufficient amounts to transduce the human cells and to provide sufficient levels of neoantigen transfer and expression to provide a therapeutic benefit without undue adverse or with medically acceptable physiological effects, which can be determined by those skilled in the medical arts. Conventional and pharmaceutically acceptable routes of administration include, but are not limited to, direct delivery to the liver, intranasal, intravenous, intramuscular, subcutaneous, intradermal, oral and other parental routes of administration. Routes of administration may be combined, if desired.

Dosages of the chimpanzee adenoviral vector will depend primarily on factors such as the condition being treated, the age, weight and health of the patient, and may thus vary among patients. The dosage will be adjusted to balance the therapeutic benefit against any side effects and such dosages may vary depending upon the therapeutic application for which the recombinant vector is employed. The levels of expression of neoantigen(s) can be monitored to determine the frequency of dosage administration.

Recombinant, replication defective chimpanzee adenoviruses can be administered in a "pharmaceutically effective amount", that is, an amount of recombinant chimpanzee adenovirus that is effective in a route of administration to transfect the desired cells and provide sufficient levels of expression of the selected gene to provide a vaccinal benefit, i.e., some measurable level of protective immunity.

Conventional and pharmaceutically acceptable routes of administration include, but are not limited to, intranasal, intramuscular, intratracheal, subcutaneous, intradermal, rectal, oral and other parental routes of administration. Routes of administration may be combined, if desired, or adjusted depending upon the immunogen or the disease. For example, in prophylaxis of rabies, the subcutaneous, intratracheal and intranasal routes are preferred. The route of administration primarily will depend on the nature of the disease being treated.

The levels of immunity to neoantigen(s) can be monitored to determine the need, if any, for boosters. Following an assessment of antibody titers in the serum, for example, optional booster immunizations may be desired

## VI. Therapeutic and Manufacturing Methods

Also provided are chimpanzee adenovirus vectors and pharmaceutical compositions for use in a method of inducing a tumor specific immune response in a subject, vaccinating against a tumor, treating and or alleviating a symptom of cancer in a subject by administering to the subject a chimpanzee adenovirus vector as claimed comprising a plurality of neoantigen-encoding nucleic acid sequences such as a plurality of neoantigens identified using methods disclosed herein.

In some aspects, a subject has been diagnosed with cancer or is at risk of developing cancer. A subject can be a human, dog, cat, horse or any animal in which a tumor specific immune response is desired. A tumor can be any solid tumor such as breast, ovarian, prostate, lung, kidney, gastric, colon, testicular, head and neck, pancreas, brain, melanoma, and other tumors of tissue organs and hematological tumors, such as lymphomas and leukemias, including acute myelogenous leukemia, chronic myelogenous leukemia, chronic lymphocytic leukemia, T cell lymphocytic leukemia, and B cell lymphomas.

A chimpanzee adenovirus vector as defined in claim 1 can be administered in an amount sufficient to induce a CTL response.

A chimpanzee adenovirus vector as defined in claim 1 can be administered alone or in combination with other therapeutic agents. The therapeutic agent is for example, a chemotherapeutic agent, radiation, or immunotherapy. Any suitable therapeutic treatment for a particular cancer can be administered.

In addition, a subject can be further administered an anti-immunosuppressive/immunostimulatory agent such as a checkpoint inhibitor. For example, the subject can be further administered an anti-CTLA antibody or anti-PD-1 or anti-PD-L1. Blockade of CTLA-4 or PD-L1 by antibodies can enhance the immune response to cancerous cells in the patient. In particular, CTLA-4 blockade has been shown effective when following a vaccination protocol.

The optimum amount of each chimpanzee adenovirus vector as defined in claim 1 to be included in a vaccine composition and the optimum dosing regimen can be determined. For example, a chimpanzee adenovirus vector as defined in claim 1 can be prepared for intravenous (i.v.) injection, sub-cutaneous (s.c.) injection, intradermal (i.d.) injection, intraperitoneal (i.p.) injection, intramuscular (i.m.) injection. Methods of injection include s.c., i.d., i.p., i.m., and i.v. Methods of DNA or RNA injection include i.d., i.m., s.c., i.p. and i.v. Other methods of administration of the vaccine composition are known to those skilled in the art.

A vaccine can be compiled so that the selection, number and/or amount of neoantigens present in the chimpanzee adenovirus vector-comprising composition is/are tissue, cancer, and/or patient-specific. For instance, the exact selection of peptides can be guided by expression patterns of the parent proteins in a given tissue. The selection can be dependent on the specific type of cancer, the status of the disease, earlier treatment regimens, the immune status of the patient, and, of course, the HLA-haplotype of the patient. Furthermore, a vaccine can contain individualized components, according to personal needs of the particular patient. Examples include varying the selection of neoantigens according to the expression of the neoantigen in the particular patient or adjustments for secondary treatments following a first round or scheme of treatment.

For a chimpanzee adenovirus vector-comprising composition to be used as a vaccine for cancer, neoantigens with similar normal self-peptides that are expressed in high amounts in normal tissues can be avoided or be present in low amounts in a composition described herein. On the other hand, if it is known that the tumor of a patient expresses high amounts of a certain neoantigen, the respective pharmaceutical composition for treatment of this cancer can be present in high amounts and/or more than one neoantigen specific for this particularly neoantigen or pathway of this neoantigen can be included.

Compositions comprising a chimpanzee adenovirus vector as defined in claim 1 can be administered to an individual already suffering from cancer. In therapeutic applications, compositions are administered to a patient in an amount sufficient to elicit an effective CTL response to the tumor antigen and to cure or at least partially arrest symptoms and/or complications. An amount adequate to accomplish this is defined as "therapeutically effective dose." Amounts effective for this use will depend on, e.g., the composition, the manner of administration, the stage and severity of the disease being treated, the weight and general state of health of the patient, and the judgment of the prescribing physician. It should be kept in mind that compositions can generally be employed in serious disease states, that is, life-threatening or potentially life threatening situations, especially when the cancer has metastasized. In such cases, in view of the minimization of extraneous substances and the relative nontoxic nature of a neoantigen, it is possible and can be felt desirable by the treating physician to administer substantial excesses of these compositions.

For therapeutic use, administration can begin at the detection or surgical removal of tumors. This is followed by boosting doses until at least symptoms are substantially abated and for a period thereafter.

The pharmaceutical compositions (e.g., vaccine compositions) for therapeutic treatment are intended for parenteral, topical, nasal, oral or local administration. A pharmaceutical compositions can be administered parenterally, e.g., intravenously, subcutaneously, intradermally, or intramuscularly. The compositions can be administered at the site of surgical exiscion to induce a local immune response to the tumor. Disclosed herein are compositions for parenteral administration which comprise a solution of the chimpanzee adenovirus vector as defined in claim 1 comprising a plurality of neoantigen-encoding nucleic acid sequences as defined in the claims and vaccine compositions are dissolved or suspended in an acceptable carrier, e.g., an aqueous carrier. A variety of aqueous carriers can be used, e.g., water, buffered water, 0.9% saline, 0.3% glycine, hyaluronic acid and the like. These compositions can be sterilized by conventional, well known sterilization techniques, or can be sterile filtered. The resulting aqueous solutions can be packaged for use as is, or lyophilized, the lyophilized preparation being combined with a sterile solution prior to administration. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, etc.

The invention relates to chimpanzee adenovirus vectors as defined in claim 1. Neoantigens can also be administered via liposomes, which target them to a particular cells tissue, such as lymphoid tissue. Liposomes are also useful in increasing half-life. Liposomes include emulsions, foams, micelles, insoluble monolayers, liquid crystals, phospholipid dispersions, lamellar layers and the like. In these preparations the neoantigen to be delivered is incorporated as part of a liposome, alone or in conjunction with a molecule which binds to, e.g., a receptor prevalent among lymphoid cells, such as monoclonal antibodies which bind to the CD45 antigen, or with other therapeutic or immunogenic compositions. Thus, liposomes filled with a desired neoantigen can be directed to the site of lymphoid cells, where the liposomes then deliver the selected therapeutic/immunogenic compositions. Liposomes can be formed from standard vesicle-forming lipids, which generally include neutral and negatively charged phospholipids and a sterol, such as cholesterol. The selection of lipids is generally guided by consideration of, e.g., liposome size, acid lability and stability of the liposomes in the blood stream. A variety of methods are available for preparing liposomes, as described in, e.g., Szoka et al., Ann. Rev. Biophys. Bioeng. 9; 467 (1980), U.S. Pat. Nos. 4,235,871, 4,501,728, 4,501,728, 4,837,028, and 5,019,369.

For targeting to the immune cells, a ligand to be incorporated into the liposome can include, e.g., antibodies or fragments thereof specific for cell surface determinants of the desired immune system cells. A liposome suspension can be administered intravenously, locally, topically, etc. in a dose which varies according to, inter alia, the manner of administration, the peptide being delivered, and the stage of the disease being treated.

The invention relates to chimpanzee adenovirus vectors as defined in claim 1. For therapeutic or immunization purposes, nucleic acids encoding a peptide and optionally one or more of the peptides described herein can also be administered to the patient. A number of methods are conveniently used to deliver the nucleic acids to the patient. For instance, the nucleic acid can be delivered directly, as "naked DNA". This approach is described, for instance, in Wolff et al., Science 247: 1465-1468 (1990) as well as U.S. Pat. Nos. 5,580,859 and 5,589,466. The nucleic acids can also be administered using ballistic delivery as described, for instance, in U.S. Pat. No. 5,204,253. Particles comprised solely of DNA can be administered. Alternatively, DNA can be adhered to particles, such as gold particles. Approaches for delivering nucleic acid sequences can include viral vectors, mRNA vectors, and DNA vectors with or without electroporation.

The nucleic acids can also be delivered complexed to cationic compounds, such as cationic lipids. Lipid-mediated gene delivery methods are described, for instance, in 9618372WOAWO 96/18372; 9324640WOAWO 93/24640; Mannino & Gould-Fogerite, BioTechniques 6(7): 682-691 (1988); U.S. Pat. No. 5,279,833 Rose U.S. Pat. No. 5,279,833; 9106309WOAWO 91/06309; and Felgner et al., Proc. Natl. Acad. Sci. USA 84: 7413-7414 (1987).

## VII. Neoantigen Use and Administration

A vaccination protocol can be used to dose a subject with one or more neoantigens. A priming vaccine and a boosting vaccine can be used to dose the subject. The invention as claimed relates to the use of chimpanzee adenovirus vectors. Other vaccines are also described below. The priming vaccine can be based on C68 (e.g., the sequences shown in SEQ ID NO:1 or 2) or srRNA (e.g., the sequences shown in SEQ ID NO:3 or 4) and the boosting vaccine can be based on C68 (e.g., the sequences shown in SEQ ID NO:1 or 2) or srRNA (e.g., the sequences shown in SEQ ID NO:3 or 4). Each vector typically includes a cassette that includes neoantigens. Cassettes can include about 20 neoantigens, separated by spacers such as the natural sequence that normally surrounds each antigen or other non-natural spacer sequences such as AAY. Cassettes as defined in the claims also include MHCII antigens such a tetanus toxoid antigen and PADRE antigen, which can be considered universal class II antigens. Cassettes can also include a targeting sequence such as a ubiquitin targeting sequence. In addition, each vaccine dose can be administered to the subject in conjunction with (e.g., concurrently, before, or after) a checkpoint inhibitor (CPI). CPI's can include those that inhibit CTLA4, PD1, and/or PDL1 such as antibodies or antigen-binding portions thereof. Such antibodies can include tremelimumab or durvalumab.

A priming vaccine can be injected (e.g., intramuscularly) in a subject. Bilateral injections per dose can be used. For example, one or more injections of ChAdV68 (C68) can be used (e.g., total dose $1 \times 10^{12}$ viral particles); one or more injections of self-replicating RNA (srRNA) at low vaccine dose selected from the range 0.001 to 1 ug RNA, in particular 0.1 or 1 ug can be used; or one or more injections of srRNA at high vaccine dose selected from the range 1 to 100 ug RNA, in particular 10 or 100 ug can be used.

A vaccine boost (boosting vaccine) can be injected (e.g., intramuscularly) after prime vaccination. A boosting vaccine can be administered about every 1, 2, 3, 4, 5, 6, 7, 8, 9, or 10 weeks, e.g., every 4 weeks and/or 8 weeks after the prime. Bilateral injections per dose can be used. For example, one or more injections of ChAdV68 (C68) can be used (e.g., total dose $1 \times 10^{12}$ viral particles); one or more injections of self-replicating RNA (srRNA) at low vaccine dose selected from the range 0.001 to 1 ug RNA, in particular 0.1 or 1 ug can be used; or one or more injections of srRNA at high vaccine dose selected from the range 1 to 100 ug RNA, in particular 10 or 100 ug can be used.

Anti-CTLA-4 (e.g., tremelimumab) can also be administered to the subject. For example, anti-CTLA4 can be administered subcutaneously near the site of the intramuscular vaccine injection (ChAdV68 prime or srRNA low doses) to ensure drainage into the same lymph node. Tremelimumab is a selective human IgG2 mAb inhibitor of CTLA-4. Target Anti-CTLA-4 (tremelimumab) subcutaneous dose is typically 70-75 mg (in particular 75 mg) with a dose range of, e.g., 1-100 mg or 5-420 mg.

In certain instances an anti-PD-L1 antibody can be used such as durvalumab (MEDI 4736). Durvalumab is a selective, high affinity human IgG1 mAb that blocks PD-L1 binding to PD-1 and CD80. Durvalumab is generally administered at 20 mg/kg i.v. every 4 weeks.

Immune monitoring can be performed before, during, and/or after vaccine administration. Such monitoring can inform safety and efficacy, among other parameters.

To perform immune monitoring, PBMCs are commonly used. PBMCs can be isolated before prime vaccination, and after prime vaccination (e.g. 4 weeks and 8 weeks). PBMCs can be harvested just prior to boost vaccinations and after each boost vaccination (e.g. 4 weeks and 8 weeks).

T cell responses can be assessed as part of an immune monitoring protocol. T cell responses can be measured using one or more methods known in the art such as ELISpot, intracellular cytokine staining, cytokine secretion and cell surface capture, T cell proliferation, MHC multimer staining, or by cytotoxicity assay. T cell responses to epitopes encoded in vaccines can be monitored from PBMCs by measuring induction of cytokines, such as IFN-gamma, using an ELISpot assay. Specific CD4 or CD8 T cell responses to epitopes encoded in vaccines can be monitored from PBMCs by measuring induction of cytokines captured intracellularly or extracellularly, such as IFN-gamma, using flow cytometry. Specific CD4 or CD8 T cell responses to epitopes encoded in the vaccines can be monitored from PBMCs by measuring T cell populations expressing T cell receptors specific for epitope/MHC class I complexes using MHC multimer staining. Specific CD4 or CD8 T cell responses to epitopes encoded in the vaccines can be monitored from PBMCs by measuring the ex vivo expansion of T cell populations following 3H-thymidine, bromodeoxyuridine and carboxyfluoresceine-diacetate- succinimidylester (CFSE) incorporation. The antigen recognition capacity and lytic activity of PBMC-derived T cells that are specific for epitopes encoded in vaccines can be assessed functionally by chromium release assay or alternative colorimetric cytotoxicity assays.

## VIII. Neoantigen Identification

### VIII.A. Neoantigen Candidate Identification

Research methods for NGS analysis of tumor and normal exome and transcriptomes have been described and applied in the neoantigen identification space. [6,14,15] The example below considers certain optimizations for greater sensitivity and specificity for neoantigen identification in the clinical setting. These optimizations can be grouped into two areas, those related to laboratory processes and those related to the NGS data analysis.

### VIII.A.1. Laboratory process optimizations

The process improvements presented here address challenges in high-accuracy neoantigen discovery from clinical specimens with low tumor content and small volumes by extending concepts developed for reliable cancer driver gene assessment in targeted cancer panels[16] to the whole- exome and -transcriptome setting necessary for neoantigen identification. Specifically, these improvements include:

1. Targeting deep (>500x) unique average coverage across the tumor exome to detect mutations present at low mutant allele frequency due to either low tumor content or subclonal state.
2. Targeting uniform coverage across the tumor exome, with <5% of bases covered at <100x, so that the fewest possible neoantigens are missed, by, for instance:

   a. Employing DNA-based capture probes with individual probe QC[17]
   b. Including additional baits for poorly covered regions

3. Targeting uniform coverage across the normal exome, where <5% of bases are covered at <20x so that the fewest neoantigens possible remain unclassified for somatic/germline status (and thus not usable as TSNAs)
4. To minimize the total amount of sequencing required, sequence capture probes will be designed for coding regions of genes only, as non-coding RNA cannot give rise to neoantigens. Additional optimizations include:

   a. supplementary probes for HLA genes, which are GC-rich and poorly captured by standard exome sequencing[18]
   b. exclusion of genes predicted to generate few or no candidate neoantigens, due to factors such as insufficient expression, suboptimal digestion by the proteasome, or unusual sequence features.

5. Tumor RNA will likewise be sequenced at high depth (>100M reads) in order to enable variant detection, quantification of gene and splice-variant ("isoform") expression, and fusion detection. RNA from FFPE samples will be extracted using probe-based enrichment[19], with the same or similar probes used to capture exomes in DNA.

### VIII.A.2. NGS data analysis optimizations

Improvements in analysis methods address the suboptimal sensitivity and specificity of common research mutation calling approaches, and specifically consider customizations relevant for neoantigen identification in the clinical setting. These include:

1. Using the HG38 reference human genome or a later version for alignment, as it contains multiple MHC regions assemblies better reflective of population polymorphism, in contrast to previous genome releases.
2. Overcoming the limitations of single variant callers [20] by merging results from different programs[.5]

   a. Single-nucleotide variants and indels will be detected from tumor DNA, tumor RNA and normal DNA with a suite of tools including: programs based on comparisons of tumor and normal DNA, such as Strelka [21] and Mutect [22]; and programs that incorporate tumor DNA, tumor RNA and normal DNA, such as UNCeqR, which is particularly advantageous in low-purity samples [23].

b. Indels will be determined with programs that perform local re-assembly, such as Strelka and ABRA [24].
c. Structural rearrangements will be determined using dedicated tools such as Pindel [25] or Breakseq [26].

3. In order to detect and prevent sample swaps, variant calls from samples for the same patient will be compared at a chosen number of polymorphic sites.

4. Extensive filtering of artefactual calls will be performed, for instance, by:

a. Removal of variants found in normal DNA, potentially with relaxed detection parameters in cases of low coverage, and with a permissive proximity criterion in case of indels
b. Removal of variants due to low mapping quality or low base quality[27].
c. Removal of variants stemming from recurrent sequencing artifacts, even if not observed in the corresponding normal[27]. Examples include variants primarily detected on one strand.
d. Removal of variants detected in an unrelated set of controls[27]

5. Accurate HLA calling from normal exome using one of seq2HLA [28], ATHLATES [29] or Optitype and also combining exome and RNA sequencing data [28]. Additional potential optimizations include the adoption of a dedicated assay for HLA typing such as long-read DNA sequencing[30], or the adaptation of a method for joining RNA fragments to retain continuity [31].

6. Robust detection of neo-ORFs arising from tumor-specific splice variants will be performed by assembling transcripts from RNA-seq data using CLASS [32], Bayesembler [33], StringTie [34] or a similar program in its reference-guided mode (i.e., using known transcript structures rather than attempting to recreate transcripts in their entirety from each experiment). While Cufflinks [35] is commonly used for this purpose, it frequently produces implausibly large numbers of splice variants, many of them far shorter than the full-length gene, and can fail to recover simple positive controls. Coding sequences and nonsense-mediated decay potential will be determined with tools such as SpliceR[36] and MAMBA[37], with mutant sequences re-introduced. Gene expression will be determined with a tool such as Cufflinks[35] or Express (Roberts and Pachter, 2013). Wild-type and mutant-specific expression counts and/or relative levels will be determined with tools developed for these purposes, such as ASE[38] or HTSeq[39]. Potential filtering steps include:

a. Removal of candidate neo-ORFs deemed to be insufficiently expressed.
b. Removal of candidate neo-ORFs predicted to trigger non-sense mediated decay (NMD).

7. Candidate neoantigens observed only in RNA (e.g., neoORFs) that cannot directly be verified as tumor-specific will be categorized as likely tumor-specific according to additional parameters, for instance by considering:

a. Presence of supporting tumor DNA-only cis-acting frameshift or splice-site mutations
b. Presence of corroborating tumor DNA-only trans-acting mutation in a splicing factor. For instance, in three independently published experiments with R625-mutant SF3B1, the genes exhibiting the most differentially splicing were concordant even though one experiment examined uveal melanoma patients [40], the second a uveal melanoma cell line [41], and the third breast cancer patients [42].
c. For novel splicing isoforms, presence of corroborating "novel" splice-junction reads in the RNASeq data.
d. For novel re-arrangements, presence of corroborating juxta-exon reads in tumor DNA that are absent from normal DNA
e. Absence from gene expression compendium such as GTEx[43] (i.e. making germline origin less likely)

8. Complementing the reference genome alignment-based analysis by comparing assembled DNA tumor and normal reads (or k-mers from such reads) directly to avoid alignment and annotation based errors and artifacts. (e.g. for somatic variants arising near germline variants or repeat-context indels)

In samples with poly-adenylated RNA, the presence of viral and microbial RNA in the RNA-seq data will be assessed using RNA CoMPASS[44] or a similar method, toward the identification of additional factors that may predict patient response.

## VIII.B. Isolation and Detection of HLA Peptides

Isolation of HLA-peptide molecules was performed using classic immunoprecipitation (IP) methods after lysis and solubilization of the tissue sample (55-58). A clarified lysate was used for HLA specific IP.

Immunoprecipitation was performed using antibodies coupled to beads where the antibody is specific for HLA molecules. For a pan-Class I HLA immunoprecipitation, a pan-Class I CR antibody is used, for Class II HLA - DR, an HLA-DR antibody is used. Antibody is covalently attached to NHS-sepharose beads during overnight incubation. After covalent attachment, the beads were washed and aliquoted for IP. (59, 60)

The clarified tissue lysate is added to the antibody beads for the immunoprecipitation. After immunoprecipitation, the beads are removed from the lysate and the lysate stored for additional experiments, including additional IPs. The IP beads are washed to remove non-specific binding and the HLA/peptide complex is eluted from the beads using standard techniques. The protein components are removed from the peptides using a molecular weight spin column or C18 fractionation. The resultant peptides are taken to dryness by SpeedVac evaporation and in some instances are stored at -20C prior to MS analysis.

Dried peptides are reconstituted in an HPLC buffer suitable for reverse phase chromatography and loaded onto a C-18 microcapillary HPLC column for gradient elution in a Fusion Lumos mass spectrometer (Thermo). MS1 spectra of peptide mass/charge (m/z) were collected in the Orbitrap detector at high resolution followed by MS2 low resolution scans collected in the ion trap detector after HCD fragmentation of the selected ion. Additionally, MS2 spectra can be obtained using either CID or ETD fragmentation methods or any combination of the three techniques to attain greater amino acid coverage of the peptide. MS2 spectra can also be measured with high resolution mass accuracy in the Orbitrap detector.

MS2 spectra from each analysis are searched against a protein database using Comet (61, 62) and the peptide identification are scored using Percolator (63-65).

**VIII.B.1. MS limit of detection studies in support of comprehensive HLA peptide sequencing.**

Using the peptide YVYVADVAAK (SEQ ID NO: 59) it was determined what the limits of detection are using different amounts of peptide loaded onto the LC column. The amounts of peptide tested were 1 pmol, 100fmol, 10 fmol, 1 fmol, and 100amol. (Table 1) The results are shown in Figure 1F. These results indicate that the lowest limit of detection (LoD) is in the attomol range ($10^{-18}$), that the dynamic range spans five orders of magnitude, and that the signal to noise appears sufficient for sequencing at low femtomol ranges ($10^{-15}$)

**Table 1**

| Peptide m/z | Loaded on Column | Copies/Cell in 1e9cells |
|---|---|---|
| 566.830 | 1 pmol | 600 |
| 562.823 | 100 fmol | 60 |
| 559.816 | 10 fmol | 6 |
| 556.810 | 1 fmol | 0.6 |
| 553.802 | 100 amol | 0.06 |

**IX. Presentation Model**

**IX.A. System Overview**

FIG. 2A is an overview of an environment 100 for identifying likelihoods of peptide presentation in patients, in accordance with an embodiment. The environment 100 provides context in order to introduce a presentation identification system 160, itself including a presentation information store 165.

The presentation identification system 160 is one or computer models, embodied in a computing system as discussed below with respect to FIG. 14, that receives peptide sequences associated with a set of MHC alleles and determines likelihoods that the peptide sequences will be presented by one or more of the set of associated MHC alleles. This is useful in a variety of contexts. One specific use case for the presentation identification system 160 is that it is able to receive nucleotide sequences of candidate neoantigens associated with a set of MHC alleles from tumor cells of a patient 110 and determine likelihoods that the candidate neoantigens will be presented by one or more of the associated MHC alleles of the tumor and/or induce immunogenic responses in the immune system of the patient 110. Those candidate neoantigens with high likelihoods as determined by system 160 can be selected for inclusion in a vaccine 118, such an anti-tumor immune response can be elicited from the immune system of the patient 110 providing the tumor cells.

The presentation identification system 160 determines presentation likelihoods through one or more presentation models. Specifically, the presentation models generate likelihoods of whether given peptide sequences will be presented for a set of associated MHC alleles, and are generated based on presentation information stored in store 165. For example, the presentation models may generate likelihoods of whether a peptide sequence "YVYVADVAAK" (SEQ ID NO: 59) will be presented for the set of alleles HLA-A*02:01, HLA-B*07:02, HLA-B*08:03, HLA-C*01:04, HLA-A*06:03, HLA-B*01:04 on the cell surface of the sample. The presentation information 165 contains information on whether peptides bind to different types of MHC alleles such that those peptides are presented by MHC alleles, which in the models is determined depending on positions of amino acids in the peptide sequences. The presentation model can predict whether an unrecognized peptide sequence will be presented in association with an associated set of MHC alleles based on the presentation information 165.

## IX.B. Presentation Information

FIG. 2 illustrates a method of obtaining presentation information, in accordance with an embodiment. The presentation information 165 includes two general categories of information: allele-interacting information and allele-noninteracting information. Allele-interacting information includes information that influence presentation of peptide sequences that are dependent on the type of MHC allele. Allele-noninteracting information includes information that influence presentation of peptide sequences that are independent on the type of MHC allele.

## IX.B.1. Allele-interacting Information

Allele-interacting information primarily includes identified peptide sequences that are known to have been presented by one or more identified MHC molecules from humans, mice, etc. Notably, this may or may not include data obtained from tumor samples. The presented peptide sequences may be identified from cells that express a single MHC allele. In this case the presented peptide sequences are generally collected from single-allele cell lines that are engineered to express a predetermined MHC allele and that are subsequently exposed to synthetic protein. Peptides presented on the MHC allele are isolated by techniques such as acid-elution and identified through mass spectrometry. FIG. 2B shows an example of this, where the example peptide YEMFNDKS (SEQ ID NO: 60), presented on the predetermined MHC allele HLA-A*01:01, is isolated and identified through mass spectrometry. Since in this situation peptides are identified through cells engineered to express a single predetermined MHC protein, the direct association between a presented peptide and the MHC protein to which it was bound to is definitively known.

The presented peptide sequences may also be collected from cells that express multiple MHC alleles. Typically in humans, 6 different types of MHC molecules are expressed for a cell. Such presented peptide sequences may be identified from multiple-allele cell lines that are engineered to express multiple predetermined MHC alleles. Such presented peptide sequences may also be identified from tissue samples, either from normal tissue samples or tumor tissue samples. In this case particularly, the MHC molecules can be immunoprecipitated from normal or tumor tissue. Peptides presented on the multiple MHC alleles can similarly be isolated by techniques such as acid-elution and identified through mass spectrometry. FIG. 2C shows an example of this, where the six example peptides, YEMFNDKSF (SEQ ID NO: 61), HROEIFSHDFJ (SEQ ID NO: 62), FJIEJFOESS (SEQ ID NO: 63), NEIOREIREI (SEQ ID NO: 64), JFKSIFEMMSJDSSU (SEQ ID NO: 65), and KNFLENFIESOFI (SEQ ID NO: 66), are presented on identified MHC alleles HLA-A*01:01, HLA-A*02:01, HLA-B*07:02, HLA-B*08:01, HLA-C*01:03, and HLA-C*01:04 and are isolated and identified through mass spectrometry. In contrast to single-allele cell lines, the direct association between a presented peptide and the MHC protein to which it was bound to may be unknown since the bound peptides are isolated from the MHC molecules before being identified.

Allele-interacting information can also include mass spectrometry ion current which depends on both the concentration of peptide-MHC molecule complexes, and the ionization efficiency of peptides. The ionization efficiency varies from peptide to peptide in a sequence-dependent manner. Generally, ionization efficiency varies from peptide to peptide over approximately two orders of magnitude, while the concentration of peptide-MHC complexes varies over a larger range than that.

Allele-interacting information can also include measurements or predictions of binding affinity between a given MHC allele and a given peptide. One or more affinity models can generate such predictions. For example, going back to the example shown in FIG. 1D, presentation information 165 may include a binding affinity prediction of 1000nM between the peptide YEMFNDKSF (SEQ ID NO: 61) and the allele HLA-A*01:01. Few peptides with IC50 > 1000nm are presented by the MHC, and lower IC50 values increase the probability of presentation.

Allele-interacting information can also include measurements or predictions of stability of the MHC complex. One or more stability models that can generate such predictions. More stable peptide-MHC complexes (i.e., complexes with longer half-lives) are more likely to be presented at high copy number on tumor cells and on antigen-presenting cells that encounter vaccine antigen. For example, going back to the example shown in FIG. 2C, presentation information 165 may include a stability prediction of a half-life of 1h for the molecule HLA-A*01:01.

Allele-interacting information can also include the measured or predicted rate of the formation reaction for the peptide-MHC complex. Complexes that form at a higher rate are more likely to be presented on the cell surface at high concentration.

Allele-interacting information can also include the sequence and length of the peptide. MHC class I molecules typically prefer to present peptides with lengths between 8 and 15 peptides. 60-80% of presented peptides have length 9. Histograms of presented peptide lengths from several cell lines are shown in Figure 5.

Allele-interacting information can also include the presence of kinase sequence motifs on the neoantigen encoded peptide, and the absence or presence of specific post-translational modifications on the neoantigen encoded peptide. The presence of kinase motifs affects the probability of post-translational modification, which may enhance or interfere with MHC binding.

Allele-interacting information can also include the expression or activity levels of proteins involved in the process of post-translational modification, e.g., kinases (as measured or predicted from RNA seq, mass spectrometry, or other methods).

Allele-interacting information can also include the probability of presentation of peptides with similar sequence in cells from other individuals expressing the particular MHC allele as assessed by mass-spectrometry proteomics or other means.

Allele-interacting information can also include the expression levels of the particular MHC allele in the individual in question (e.g. as measured by RNA-seq or mass spectrometry). Peptides that bind most strongly to an MHC allele that is expressed at high levels are more likely to be presented than peptides that bind most strongly to an MHC allele that is expressed at a low level.

Allele-interacting information can also include the overall neoantigen encoded peptide-sequence-independent probability of presentation by the particular MHC allele in other individuals who express the particular MHC allele.

Allele-interacting information can also include the overall peptide-sequence-independent probability of presentation by MHC alleles in the same family of molecules (e.g., HLA-A, HLA-B, HLA-C, HLA-DQ, HLA-DR, HLA-DP) in other individuals. For example, HLA-C molecules are typically expressed at lower levels than HLA-A or HLA-B molecules, and consequently, presentation of a peptide by HLA-C is a priori less probable than presentation by HLA-A or HLA-B 11.

Allele-interacting information can also include the protein sequence of the particular MHC allele.

Any MHC allele-noninteracting information listed in the below section can also be modeled as an MHC allele-interacting information.

### IX.B.2. Allele-noninteracting Information

Allele-noninteracting information can include C-terminal sequences flanking the neoantigen encoded peptide within its source protein sequence. C-terminal flanking sequences may impact proteasomal processing of peptides. However, the C-terminal flanking sequence is cleaved from the peptide by the proteasome before the peptide is transported to the endoplasmic reticulum and encounters MHC alleles on the surfaces of cells. Consequently, MHC molecules receive no information about the C-terminal flanking sequence, and thus, the effect of the C-terminal flanking sequence cannot vary depending on MHC allele type. For example, going back to the example shown in FIG. 2C, presentation information 165 may include the C-terminal flanking sequence FOEIFNDKSLDKFJI (SEQ ID NO: 67) of the presented peptide FJIEJFOESS (SEQ ID NO: 63) identified from the source protein of the peptide.

Allele-noninteracting information can also include mRNA quantification measurements. For example, mRNA quantification data can be obtained for the same samples that provide the mass spectrometry training data. As later described in reference to FIG. 13H, RNA expression was identified to be a strong predictor of peptide presentation. In one embodiment, the mRNA quantification measurements are identified from software tool RSEM. Detailed implementation of the RSEM software tool can be found at Bo Li and Colin N. Dewey. RSEM: accurate transcript quantification from RNA-Seq data with or without a reference genome. BMC Bioinformatics, 12:323, August 2011. In one embodiment, the mRNA quantification is measured in units of fragments per kilobase of transcript per Million mapped reads (FPKM).

Allele-noninteracting information can also include the N-terminal sequences flanking the peptide within its source protein sequence.

Allele-noninteracting information can also include the presence of protease cleavage motifs in the peptide, optionally weighted according to the expression of corresponding proteases in the tumor cells (as measured by RNA-seq or mass spectrometry). Peptides that contain protease cleavage motifs are less likely to be presented, because they will be more readily degraded by proteases, and will therefore be less stable within the cell.

Allele-noninteracting information can also include the turnover rate of the source protein as measured in the appropriate cell type. Faster turnover rate (i.e., lower half-life) increases the probability of presentation; however, the predictive power of this feature is low if measured in a dissimilar cell type.

Allele-noninteracting information can also include the length of the source protein, optionally considering the specific splice variants ("isoforms") most highly expressed in the tumor cells as measured by RNA-seq or proteome mass spectrometry, or as predicted from the annotation of germline or somatic splicing mutations detected in DNA or RNA sequence data.

Allele-noninteracting information can also include the level of expression of the proteasome, immunoproteasome, thymoproteasome, or other proteases in the tumor cells (which may be measured by RNA-seq, proteome mass spectrometry, or immunohistochemistry). Different proteasomes have different cleavage site preferences. More weight will be given to the cleavage preferences of each type of proteasome in proportion to its expression level.

Allele-noninteracting information can also include the expression of the source gene of the peptide (e.g., as measured by RNA-seq or mass spectrometry). Possible optimizations include adjusting the measured expression to account for the presence of stromal cells and tumor-infiltrating lymphocytes within the tumor sample. Peptides from more highly expressed genes are more likely to be presented. Peptides from genes with undetectable levels of expression can be excluded from consideration.

Allele-noninteracting information can also include the probability that the source mRNA of the neoantigen encoded peptide will be subject to nonsense-mediated decay as predicted by a model of nonsense-mediated decay, for example, the model from Rivas et al, Science 2015.

Allele-noninteracting information can also include the typical tissue-specific expression of the source gene of the peptide during various stages of the cell cycle. Genes that are expressed at a low level overall (as measured by RNA-seq or mass spectrometry proteomics) but that are known to be expressed at a high level during specific stages of the cell cycle are likely to produce more presented peptides than genes that are stably expressed at very low levels.

Allele-noninteracting information can also include a comprehensive catalog of features of the source protein as given in e.g. uniProt or PDB http://www.rcsb.org/pdb/home/home.do. These features may include, among others: the secondary and tertiary structures of the protein, subcellular localization 11, Gene ontology (GO) terms. Specifically, this information may contain annotations that act at the level of the protein, e.g., 5' UTR length, and annotations that act at the level of specific residues, e.g., helix motif between residues 300 and 310. These features can also include turn motifs, sheet motifs, and disordered residues.

Allele-noninteracting information can also include features describing the properties of the domain of the source protein containing the peptide, for example: secondary or tertiary structure (e.g., alpha helix vs beta sheet); Alternative splicing.

Allele-noninteracting information can also include features describing the presence or absence of a presentation hotspot at the position of the peptide in the source protein of the peptide.

Allele-noninteracting information can also include the probability of presentation of peptides from the source protein of the peptide in question in other individuals (after adjusting for the expression level of the source protein in those individuals and the influence of the different HLA types of those individuals).

Allele-noninteracting information can also include the probability that the peptide will not be detected or over-represented by mass spectrometry due to technical biases.

The expression of various gene modules/pathways as measured by a gene expression assay such as RNASeq, microarray(s), targeted panel(s) such as Nanostring, or single/multi- gene representatives of gene modules measured by assays such as RT-PCR (which need not contain the source protein of the peptide) that are informative about the state of the tumor cells, stroma, or tumor-infiltrating lymphocytes (TILs).

Allele-noninteracting information can also include the copy number of the source gene of the peptide in the tumor cells. For example, peptides from genes that are subject to homozygous deletion in tumor cells can be assigned a probability of presentation of zero.

Allele-noninteracting information can also include the probability that the peptide binds to the TAP or the measured or predicted binding affinity of the peptide to the TAP. Peptides that are more likely to bind to the TAP, or peptides that bind the TAP with higher affinity are more likely to be presented.

Allele-noninteracting information can also include the expression level of TAP in the tumor cells (which may be measured by RNA-seq, proteome mass spectrometry, immunohistochemistry). Higher TAP expression levels increase the probability of presentation of all peptides.

Allele-noninteracting information can also include the presence or absence of tumor mutations, including, but not limited to:

i. Driver mutations in known cancer driver genes such as EGFR, KRAS, ALK, RET, ROS1, TP53, CDKN2A, CDKN2B, NTRK1, NTRK2, NTRK3

ii. In genes encoding the proteins involved in the antigen presentation machinery (e.g., B2M, HLA-A, HLA-B, HLA-C, TAP-1, TAP-2, TAPBP, CALR, CNX, ERP57, HLA-DM, HLA-DMA, HLA-DMB, HLA-DO, HLA-DOA, HLA-DOBHLA-DP, HLA-DPA1, HLA-DPB1, HLA-DQ, HLA-DQA1, HLA-DQA2, HLA-DQB1, HLA-DQB2, HLA-DR, HLA-DRA, HLA-DRB1, HLA-DRB3, HLA-DRB4, HLA-DRB5 or any of the genes coding for components of the proteasome or immunoproteasome). Peptides whose presentation relies on a component of the antigen-presentation machinery that is subject to loss-of-function mutation in the tumor have reduced probability of presentation.

Presence or absence of functional germline polymorphisms, including, but not limited to:

i.In genes encoding the proteins involved in the antigen presentation machinery (e.g., B2M, HLA-A, HLA-B, HLA-C, TAP-1, TAP-2, TAPBP, CALR, CNX, ERP57, HLA-DM, HLA-DMA, HLA-DMB, HLA-DO, HLA-DOA, HLA-DOBHLA-DP, HLA-DPA1, HLA-DPB1, HLA-DQ, HLA-DQA1, HLA-DQA2, HLA-DQB1, HLA-DQB2, HLA-DR, HLA-DRA, HLA-DRB1, HLA-DRB3, HLA-DRB4, HLA-DRB5 or any of the genes coding for components of the proteasome or immunoproteasome)

Allele-noninteracting information can also include tumor type (e.g., NSCLC, melanoma).

Allele-noninteracting information can also include known functionality of HLA alleles, as reflected by, for instance HLA allele suffixes. For example, the N suffix in the allele name HLA-A*24:09N indicates a null allele that is not expressed and is therefore unlikely to present epitopes; the full HLA allele suffix nomenclature is described at https://www.ebi.ac.uk/ipd/imgt/hla/nomenclature/suffixes.html.

Allele-noninteracting information can also include clinical tumor subtype (e.g., squamous lung cancer vs. non-squamous).

Allele-noninteracting information can also include smoking history.

Allele-noninteracting information can also include history of sunburn, sun exposure, or exposure to other mutagens.

Allele-noninteracting information can also include the typical expression of the source gene of the peptide in the relevant tumor type or clinical subtype, optionally stratified by driver mutation. Genes that are typically expressed at high levels in the relevant tumor type are more likely to be presented.

Allele-noninteracting information can also include the frequency of the mutation in all tumors, or in tumors of the same type, or in tumors from individuals with at least one shared MHC allele, or in tumors of the same type in individuals with at least one shared MHC allele.

In the case of a mutated tumor-specific peptide, the list of features used to predict a probability of presentation may also include the annotation of the mutation (e.g., missense, read-through, frameshift, fusion, etc.) or whether the mutation is predicted to result in nonsense-mediated decay (NMD). For example, peptides from protein segments that are not translated in tumor cells due to homozygous early-stop mutations can be assigned a probability of presentation of zero. NMD results in decreased mRNA translation, which decreases the probability of presentation.

### IX.C. Presentation Identification System

FIG. 3 is a high-level block diagram illustrating the computer logic components of the presentation identification system 160, according to one embodiment. In this example embodiment, the presentation identification system 160 includes a data management module 312, an encoding module 314, a training module 316, and a prediction module 320. The presentation identification system 160 is also comprised of a training data store 170 and a presentation models store 175. Some embodiments of the model management system 160 have different modules than those described here. Similarly, the functions can be distributed among the modules in a different manner than is described here.

### IX.C.1. Data Management Module

The data management module 312 generates sets of training data 170 from the presentation information 165. Each set of training data contains a plurality of data instances, in which each data instance $i$ contains a set of independent variables $z^i$ that include at least a presented or non-presented peptide sequence $p^i$, one or more associated MHC alleles $a^i$ associated with the peptide sequence $p^i$, and a dependent variable $y^i$ that represents information that the presentation identification system 160 is interested in predicting for new values of independent variables.

In one particular implementation referred throughout the remainder of the specification, the dependent variable $y^i$ is a binary label indicating whether peptide $p^i$ was presented by the one or more associated MHC alleles $a^i$. However, it is appreciated that in other implementations, the dependent variable $y^i$ can represent any other kind of information that the presentation identification system 160 is interested in predicting dependent on the independent variables $z^i$. For example, in another implementation, the dependent variable $y^i$ may also be a numerical value indicating the mass spectrometry ion current identified for the data instance.

The peptide sequence $p^i$ for data instance $i$ is a sequence of $k_i$ amino acids, in which $k_i$ may vary between data instances $i$ within a range. For example, that range may be 8-15 for MHC class I or 9-30 for MHC class II. In one specific implementation of system 160, all peptide sequences $p^i$ in a training data set may have the same length, e.g. 9. The number of amino acids in a peptide sequence may vary depending on the type of MHC alleles (e.g., MHC alleles in humans, etc.). The MHC alleles $a^i$ for data instance $i$ indicate which MHC alleles were present in association with the corresponding peptide sequence $p^i$.

The data management module 312 may also include additional allele-interacting variables, such as binding affinity $b^i$ and stability $s^i$ predictions in conjunction with the peptide sequences $p^i$ and associated MHC alleles $a^i$ contained in the training data 170. For example, the training data 170 may contain binding affinity predictions $b^i$ between a peptide $p^i$

and each of the associated MHC molecules indicated in $\boldsymbol{a^i}$. As another example, the training data 170 may contain stability predictions $\boldsymbol{s^i}$ for each of the MHC alleles indicated in $\boldsymbol{a^i}$.

The data management module 312 may also include allele-noninteracting variables $\boldsymbol{w^i}$, such as C-terminal flanking sequences and mRNA quantification measurements in conjunction with the peptide sequences $\boldsymbol{p^i}$.

The data management module 312 also identifies peptide sequences that are not presented by MHC alleles to generate the training data 170. Generally, this involves identifying the "longer" sequences of source protein that include presented peptide sequences prior to presentation. When the presentation information contains engineered cell lines, the data management module 312 identifies a series of peptide sequences in the synthetic protein to which the cells were exposed to that were not presented on MHC alleles of the cells. When the presentation information contains tissue samples, the data management module 312 identifies source proteins from which presented peptide sequences originated from, and identifies a series of peptide sequences in the source protein that were not presented on MHC alleles of the tissue sample cells.

The data management module 312 may also artificially generate peptides with random sequences of amino acids and identify the generated sequences as peptides not presented on MHC alleles. This can be accomplished by randomly generating peptide sequences allows the data management module 312 to easily generate large amounts of synthetic data for peptides not presented on MHC alleles. Since in reality, a small percentage of peptide sequences are presented by MHC alleles, the synthetically generated peptide sequences are highly likely not to have been presented by MHC alleles even if they were included in proteins processed by cells.

FIG. 4 illustrates an example set of training data 170A, according to one embodiment. Specifically, the first 3 data instances in the training data 170A indicate peptide presentation information from a single-allele cell line involving the allele HLA-C*01:03 and 3 peptide sequences QCEIOWARE (SEQ ID NO: 68), FIEUHFWI (SEQ ID NO: 69), and FEWRHRJTRUJR (SEQ ID NO: 70). The fourth data instance in the training data 170A indicates peptide information from a multiple-allele cell line involving the alleles HLA-B*07:02, HLA-C*01:03, HLA-A*01:01and a peptide sequence QIEJOEIJE (SEQ ID NO: 71). The first data instance indicates that peptide sequence QCEIOWARE (SEQ ID NO: 68) was not presented by the allele HLA-C*01:03. As discussed in the prior two paragraphs, the peptide sequence may be randomly generated by the data management module 312 or identified from source protein of presented peptides. The training data 170A also includes a binding affinity prediction of 1000nM and a stability prediction of a half-life of 1h for the peptide sequence-allele pair. The training data 170A also includes allele-noninteracting variables, such as the C-terminal flanking sequence of the peptide FJELFISBOSJFIE (SEQ ID NO: 72), and a mRNA quantification measurement of $10^2$ FPKM. The fourth data instance indicates that peptide sequence QIEJOEIJE (SEQ ID NO: 71) was presented by one of the alleles HLA-B*07:02, HLA-C*01:03, or HLA-A*01:01. The training data 170A also includes binding affinity predictions and stability predictions for each of the alleles, as well as the C-flanking sequence of the peptide and the mRNA quantification measurement for the peptide.

## IX.C.2. Encoding Module

The encoding module 314 encodes information contained in the training data 170 into a numerical representation that can be used to generate the one or more presentation models. In one implementation, the encoding module 314 one-hot encodes sequences (e.g., peptide sequences or C-terminal flanking sequences) over a predetermined 20-letter amino acid alphabet. Specifically, a peptide sequence $\boldsymbol{p^i}$ with $k_i$ amino acids is represented as a row vector of $20 \cdot k_i$ elements, where a single element among $p^i_{20 \cdot (j-1)+1}, p^i_{20 \cdot (j-1)+2}, \ldots, p'_{20 \cdot j}$ that corresponds to the alphabet of the amino acid at the $j$-$th$ position of the peptide sequence has a value of 1. Otherwise, the remaining elements have a value of 0. As an example, for a given alphabet {A, C, D, E, F, G, H, I, K, L, M, N, P, Q, R, S, T, V, W, Y}, the peptide sequence EAF of 3 amino acids for data instance i may be represented by the row vector of 60 elements $\boldsymbol{p^i}$=[0 0 0 1 0 0 0 0 0 0 0 0 0 0 0 0 0 0 0 0 1 0 0 0 0 0 0 0 0 0 0 0 0 0 0 0 0 0 0 0 0 0 0 0 0 0 1 0 0 0 0 0 0 0 0 0 0 0 0 0]. The C-terminal flanking sequence $\boldsymbol{c^i}$ can be similarly encoded as described above, as well as the protein sequence $\boldsymbol{d_h}$ for MHC alleles, and other sequence data in the presentation information.

When the training data 170 contains sequences of differing lengths of amino acids, the encoding module 314 may further encode the peptides into equal-length vectors by adding a PAD character to extend the predetermined alphabet. For example, this may be performed by left-padding the peptide sequences with the PAD character until the length of the peptide sequence reaches the peptide sequence with the greatest length in the training data 170. Thus, when the peptide sequence with the greatest length has $k_{max}$ amino acids, the encoding module 314 numerically represents each sequence as a row vector of $(20+1) \cdot k_{max}$ elements. As an example, for the extended alphabet {PAD, A, C, D, E, F, G, H, I, K, L, M, N, P, Q, R, S, T, V, W, Y} and a maximum amino acid length of $k_{max}$=5, the same example peptide sequence EAF of 3 amino acids may be represented by the row vector of 105 elements $\boldsymbol{p^i}$=[1 0 0 0 0 0 0 0 0 0 0 0 0 0 0 0 0 0 0 0 0 1 0 0 0 0 0 0 0 0 0 0 0 0 0 0 0 0 0 0 0 0 0 0 0 0 0 0 1 0 0 0 0 0 0 0 0 0 0 0 0 0 0 0 0 0 0 0 0 0 0 1 0 0 0 0 0 0 0 0 0 0 0 0 0 0 0 0 0 0 0 0 0 0 0 0 1 0 0 0 0 0 0 0 0]. The C-terminal flanking sequence $\boldsymbol{c^i}$ or other sequence data can be similarly encoded as described above. Thus, each independent variable or column in the peptide sequence $\boldsymbol{p^i}$ or $\boldsymbol{c^i}$ represents presence of a particular amino acid at a particular position of the sequence.

Although the above method of encoding sequence data was described in reference to sequences having amino acid sequences, the method can similarly be extended to other types of sequence data, such as DNA or RNA sequence data, and the like.

The encoding module 314 also encodes the one or more MHC alleles $a^i$ for data instance $i$ as a row vector of $m$ elements, in which each element $h=1, 2, ... , m$ corresponds to a unique identified MHC allele. The elements corresponding to the MHC alleles identified for the data instance $i$ have a value of 1. Otherwise, the remaining elements have a value of 0. As an example, the alleles HLA-B*07:02 and HLA-C*01:03 for a data instance $i$ corresponding to a multiple-allele cell line among $m=4$ unique identified MHC allele types {HLA-A*01:01, HLA-C*01:08, HLA-B*07:02, HLA-C*01:03} may be represented by the row vector of 4 elements $a^i=[0\ 0\ 1\ 1]$, in which $a_3^i=1$ and $a_4^i=1$. Although the example is described herein with 4 identified MHC allele types, the number of MHC allele types can be hundreds or thousands in practice. As previously discussed, each data instance $i$ typically contains at most 6 different MHC allele types in association with the peptide sequence $p_i$.

The encoding module 314 also encodes the label $y_i$ for each data instance $i$ as a binary variable having values from the set of {0, 1}, in which a value of 1 indicates that peptide $x^i$ was presented by one of the associated MHC alleles $a^i$, and a value of 0 indicates that peptide $x^i$ was not presented by any of the associated MHC alleles $a^i$. When the dependent variable $y_i$ represents the mass spectrometry ion current, the encoding module 314 may additionally scale the values using various functions, such as the log function having a range of $[-\infty, \infty]$ for ion current values between $[0, \infty]$.

The encoding module 314 may represent a pair of allele-interacting variables $x_h^i$ for peptide $p_i$ and an associated MHC allele $h$ as a row vector in which numerical representations of allele-interacting variables are concatenated one after the other. For example, the encoding module 314 may represent $x_h^i$ as a row vector equal to $[p^i]$, $[p^i\ b_h^i]$, $[p^i\ s_h^i]$, or $[p^i\ b_h^i\ s_h^i]$, where $b_h^i$ is the binding affinity prediction for peptide $p_i$ and associated MHC allele $h$, and similarly for $s_h^i$ for stability. Alternatively, one or more combination of allele-interacting variables may be stored individually (e.g., as individual vectors or matrices).

In one instance, the encoding module 314 represents binding affinity information by incorporating measured or predicted values for binding affinity in the allele-interacting variables $x_h^i$.

In one instance, the encoding module 314 represents binding stability information by incorporating measured or predicted values for binding stability in the allele-interacting variables $x_h^i$,

In one instance, the encoding module 314 represents binding on-rate information by incorporating measured or predicted values for binding on-rate in the allele-interacting variables $x_h^i$.

In one instance, the encoding module 314 represents peptide length as a vector

$$T_k=[\mathbb{1}(L_k=8)\ \mathbb{1}(L_k=9)\ \mathbb{1}(L_k=10)\ \mathbb{1}(L_k=11)\ \mathbb{1}(L_k=12)\ \mathbb{1}(L_k=13)\ \mathbb{1}(L_k=14)\ \mathbb{1}(L_k=15)]$$

where $\mathbb{1}$ is the indicator function, and $L_k$ denotes the length of peptide $p_k$. The vector $T_k$ can be included in the allele-interacting variables $x_h^i$.

In one instance, the encoding module 314 represents RNA expression information of MHC alleles by incorporating RNA-seq based expression levels of MHC alleles in the allele-interacting variables $x_h^i$.

Similarly, the encoding module 314 may represent the allele-noninteracting variables $w^i$ as a row vector in which numerical representations of allele-noninteracting variables are concatenated one after the other. For example, $w^i$ may be a row vector equal to $[c^i]$ or $[c^i\ m^i\ w^i]$ in which $w^i$ is a row vector representing any other allele-noninteracting variables in addition to the C-terminal flanking sequence of peptide $p^i$ and the mRNA quantification measurement $m^i$ associated with the peptide. Alternatively, one or more combination of allele-noninteracting variables may be stored individually (e.g., as individual vectors or matrices).

In one instance, the encoding module 314 represents turnover rate of source protein for a peptide sequence by incorporating the turnover rate or half-life in the allele-noninteracting variables $w^i$.

In one instance, the encoding module 314 represents length of source protein or isoform by incorporating the protein length in the allele-noninteracting variables $w^i$.

In one instance, the encoding module 314 represents activation of immunoproteasome by incorporating the mean expression of the immunoproteasome-specific proteasome subunits including the $\beta 1_i$, $\beta 2_i$, $\beta 5_i$ subunits in the allele-noninteracting variables $w^i$.

In one instance, the encoding module 314 represents the RNA-seq abundance of the source protein of the peptide or gene or transcript of a peptide (quantified in units of FPKM, TPM by techniques such as RSEM) can be incorporating the abundance of the source protein in the allele-noninteracting variables $w^i$.

In one instance, the encoding module 314 represents the probability that the transcript of origin of a peptide will undergo nonsense-mediated decay (NMD) as estimated by the model in, for example, Rivas et. al. Science, 2015 by incorporating this probability in the allele-noninteracting variables $w^i$.

In one instance, the encoding module 314 represents the activation status of a gene module or pathway assessed via RNA-seq by, for example, quantifying expression of the genes in the pathway in units of TPM using e.g., RSEM for each of the genes in the pathway then computing a summary statistics, e.g., the mean, across genes in the pathway. The mean can be incorporated in the allele-noninteracting variables $w^i$.

In one instance, the encoding module 314 represents the copy number of the source gene by incorporating the copy number in the allele-noninteracting variables $w^i$.

In one instance, the encoding module 314 represents the TAP binding affinity by including the measured or predicted TAP binding affinity (e.g., in nanomolar units) in the allele-noninteracting variables $w^i$.

In one instance, the encoding module 314 represents TAP expression levels by including TAP expression levels measured by RNA-seq (and quantified in units of TPM by e.g., RSEM) in the allele-noninteracting variables $w^i$.

In one instance, the encoding module 314 represents tumor mutations as a vector of indicator variables (i.e., $d^k = 1$ if peptide $p^k$ comes from a sample with a KRAS G12D mutation and 0 otherwise) in the allele-noninteracting variables $w^i$.

In one instance, the encoding module 314 represents germline polymorphisms in antigen presentation genes as a vector of indicator variables (i.e., $d^k = 1$ if peptide $p^k$ comes from a sample with a specic germline polymorphism in the TAP). These indicator variables can be included in the allele-noninteracting variables $w^i$.

In one instance, the encoding module 314 represents tumor type as a length-one one-hot encoded vector over the alphabet of tumor types (e.g., NSCLC, melanoma, colorectal cancer, etc). These one-hot-encoded variables can be included in the allele-noninteracting variables $w^i$.

In one instance, the encoding module 314 represents MHC allele suffixes by treating 4-digit HLA alleles with different suffixes. For example, HLA-A*24:09N is considered a different allele from HLA-A*24:09 for the purpose of the model. Alternatively, the probability of presentation by an N-suffixed MHC allele can be set to zero for all peptides, because HLA alleles ending in the N suffix are not expressed.

In one instance, the encoding module 314 represents tumor subtype as a length-one one-hot encoded vector over the alphabet of tumor subtypes (e.g., lung adenocarcinoma, lung squamous cell carcinoma, etc). These onehot-encoded variables can be included in the allele-noninteracting variables $w^i$.

In one instance, the encoding module 314 represents smoking history as a binary indicator variable ($d^k = 1$ if the patient has a smoking history, and 0 otherwise), that can be included in the allele-noninteracting variables $w^i$. Alternatively, smoking history can be encoded as a length-one one-hot-enocded variable over an alphabet of smoking severity. For example, smoking status can be rated on a 1-5 scale, where 1 indicates nonsmokers, and 5 indicates current heavy smokers. Because smoking history is primarily relevant to lung tumors, when training a model on multiple tumor types, this variable can also be defined to be equal to 1 if the patient has a history of smoking and the tumor type is lung tumors and zero otherwise.

In one instance, the encoding module 314 represents sunburn history as a binary indicator variable ($d^k = 1$ if the patient has a history of severe sunburn, and 0 otherwise), which can be included in the allele-noninteracting variables w^i. Because severe sunburn is primarily relevant to melanomas, when training a model on multiple tumor types, this variable can also be defined to be equal to 1 if the patient has a history of severe sunburn and the tumor type is melanoma and zero otherwise.

In one instance, the encoding module 314 represents distribution of expression levels of a particular gene or transcript for each gene or transcript in the human genome as summary statistics (e,g., mean, median) of distribution of expression levels by using reference databases such as TCGA. Specifically, for a peptide $p^k$ in a sample with tumor type melanoma, not only the measured gene or transcript expression level of the gene or transcript of origin of peptide $p^k$ in the allele-noninteracting variables $w^i$ be included, but also the mean and/or median gene or transcript expression of the gene or transcript of origin of peptide $p^k$ in melanomas as measured by TCGA.

In one instance, the encoding module 314 represents mutation type as a length-one one-hot-encoded variable over the alphabet of mutation types (e.g., missense, frameshift, NMD-inducing, etc). These onehot-encoded variables can be included in the allele-noninteracting variables $w^i$.

In one instance, the encoding module 314 represents protein-level features of protein as the value of the annotation (e.g., 5' UTR length) of the source protein in the allele-noninteracting variables $w^i$. In another instance, the encoding module 314 represents residue-level annotations of the source protein for peptide $p^k$ by including an indicator variable, that is equal to 1 if peptide $p^k$ overlaps with a helix motif and 0 otherwise, or that is equal to 1 if peptide $p^k$ is completely contained with within a helix motif in the allele-noninteracting variables $w^i$. In another instance, a feature representing proportion of residues in peptide $p^k$ that are contained within a helix motif annotation can be included in the allele-noninteracting variables $w^i$.

In one instance, the encoding module 314 represents type of proteins or isoforms in the human proteome as an indicator vector $o^k$ that has a length equal to the number of proteins or isoforms in the human proteome, and the corresponding element $o^k_i$ is 1 if peptide $p^k$ comes from protein $i$ and 0 otherwise.

The encoding module 314 may also represent the overall set of variables $z^i$ for peptide $p^i$ and an associated MHC allele $h$ as a row vector in which numerical representations of the allele-interacting variables $x^i$ and the allele-noninteracting variables $w^i$ are concatenated one after the other. For example, the encoding module 314 may represent $z_h^i$ as a row

vector equal to [$\boldsymbol{x_h}^i \boldsymbol{w^i}$] or [$\boldsymbol{w_i} \boldsymbol{x_h}^i$].

## X. Training Module

The training module 316 constructs one or more presentation models that generate likelihoods of whether peptide sequences will be presented by MHC alleles associated with the peptide sequences. Specifically, given a peptide sequence $\boldsymbol{p^k}$ and a set of MHC alleles $\boldsymbol{a^k}$ associated with the peptide sequence $\boldsymbol{p^k}$, each presentation model generates an estimate $u_k$ indicating a likelihood that the peptide sequence $\boldsymbol{p^k}$ will be presented by one or more of the associated MHC alleles $\boldsymbol{a^k}$.

### X.A. Overview

The training module 316 constructs the one more presentation models based on the training data sets stored in store 170 generated from the presentation information stored in 165. Generally, regardless of the specific type of presentation model, all of the presentation models capture the dependence between independent variables and dependent variables in the training data 170 such that a loss function is minimized. Specifically, the loss function $\ell(y_{i \in S}, u_{i \in S}, \theta)$ represents discrepancies between values of dependent variables $y_{i \in S}$ for one or more data instances S in the training data 170 and the estimated likelihoods $u_{i \in S}$ for the data instances S generated by the presentation model. In one particular implementation referred throughout the remainder of the specification, the loss function $(y_{i \in S}, u_{i \in S}, \theta)$ is the negative log likelihood function given by equation (1a) as follows:

$$\ell(y_{i \in S}, u_{i \in S}; \boldsymbol{\theta}) = \sum_{i \in S} (y_i \log u_i + (1 - y_i) \log(1 - u_i)). \qquad (1a)$$

However, in practice, another loss function may be used. For example, when predictions are made for the mass spectrometry ion current, the loss function is the mean squared loss given by equation 1b as follows:

$$\ell(y_{i \in S}, u_{i \in S}; \theta) = \sum_{i \in S} (\|y_i - u_i\|_2^2). \qquad (1b)$$

The presentation model may be a parametric model in which one or more parameters $\theta$ mathematically specify the dependence between the independent variables and dependent variables. Typically, various parameters of parametric-type presentation models that minimize the loss function $(y_{i \in S}, u_{i \in S}, \theta)$ are determined through gradient-based numerical optimization algorithms, such as batch gradient algorithms, stochastic gradient algorithms, and the like. Alternatively, the presentation model may be a non-parametric model in which the model structure is determined from the training data 170 and is not strictly based on a fixed set of parameters.

### X.B. Per-Allele Models

The training module 316 may construct the presentation models to predict presentation likelihoods of peptides on a per-allele basis. In this case, the training module 316 may train the presentation models based on data instances $S$ in the training data 170 generated from cells expressing single MHC alleles.

In one implementation, the training module 316 models the estimated presentation likelihood $u_k$ for peptide $\boldsymbol{p^k}$ for a specific allele $h$ by:

$$u_k^h = \Pr(\boldsymbol{p^k} \text{ presented; MHC allele } h) = f\left(g_h(x_h^k; \boldsymbol{\theta_h})\right), \qquad (2)$$

where peptide sequence $\boldsymbol{x_h}^k$ denotes the encoded allele-interacting variables for peptide $\boldsymbol{p^k}$ and corresponding MHC allele $h$, $f(\cdot)$ is any function, and is herein throughout is referred to as a transformation function for convenience of description. Further, $g_h(\cdot)$ is any function, is herein throughout referred to as a dependency function for convenience of description, and generates dependency scores for the allele-interacting variables $\boldsymbol{x_h}^k$ based on a set of parameters $\theta_h$ determined for MHC allele $h$. The values for the set of parameters $\theta_h$ for each MHC allele $h$ can be determined by minimizing the loss function with respect to $\theta_h$, where $i$ is each instance in the subset $S$ of training data 170 generated from cells expressing the single MHC allele $h$.

The output of the dependency function $g_h(\boldsymbol{x_h}^k; \theta_h)$ represents a dependency score for the MHC allele $h$ indicating whether the MHC allele $h$ will present the corresponding neoantigen based on at least the allele interacting features $x_h^k$, and in particular, based on positions of amino acids of the peptide sequence of peptide $p^k$. For example, the dependency score for the MHC allele $h$ may have a high value if the MHC allele $h$ is likely to present the peptide $p^k$, and may have a low value if presentation is not likely. The transformation function $f(\cdot)$ transforms the input, and more specifically, transforms the dependency score generated by $g_h(x_h^k; \theta_h)$ in this case, to an appropriate value to indicate the likelihood that the peptide $p^k$ will be presented by an MHC allele.

In one particular implementation referred throughout the remainder of the specification, $f(\cdot)$ is a function having the range within [0, 1] for an appropriate domain range. In one example, $f(\cdot)$ is the expit function given by:

$$f(z) = \frac{\exp(z)}{1 + \exp(z)}. \qquad (4)$$

As another example, $f(\cdot)$ can also be the hyperbolic tangent function given by:

$$f(z) = \tanh(z) \qquad (5)$$

when the values for the domain $z$ is equal to or greater than 0. Alternatively, when predictions are made for the mass spectrometry ion current that have values outside the range [0, 1], $f(\cdot)$ can be any function such as the identity function, the exponential function, the log function, and the like.

Thus, the per-allele likelihood that a peptide sequence $p^k$ will be presented by a MHC allele $h$ can be generated by applying the dependency function $g_h(\cdot)$ for the MHC allele h to the encoded version of the peptide sequence $p^k$ to generate the corresponding dependency score. The dependency score may be transformed by the transformation function $f(\cdot)$ to generate a per-allele likelihood that the peptide sequence $p^k$ will be presented by the MHC allele $h$.

### X.B.1 Dependency Functions for Allele Interacting Variables

In one particular implementation referred throughout the specification, the dependency function $g_h(\cdot)$ is an affine function given by:

$$g_h\left(x_h^i; \theta_h\right) = x_h^i \cdot \theta_h. \qquad (6)$$

that linearly combines each allele-interacting variable in $x_h^k$ with a corresponding parameter in the set of parameters $\theta_h$ determined for the associated MHC allele $h$.

In another particular implementation referred throughout the specification, the dependency function $g_h(\cdot)$ is a network function given by:

$$g_h\left(x_h^i; \theta_h\right) = NN_h(x_h^i; \theta_h). \qquad (7)$$

represented by a network model $NN_h(\cdot)$ having a series of nodes arranged in one or more layers. A node may be connected to other nodes through connections each having an associated parameter in the set of parameters $\theta_h$. A value at one particular node may be represented as a sum of the values of nodes connected to the particular node weighted by the associated parameter mapped by an activation function associated with the particular node. In contrast to the affine function, network models are advantageous because the presentation model can incorporate non-linearity and process data having different lengths of amino acid sequences. Specifically, through non-linear modeling, network models can capture interaction between amino acids at different positions in a peptide sequence and how this interaction affects peptide presentation.

In general, network models $NN_h(\cdot)$ may be structured as feed-forward networks, such as artificial neural networks (ANN), convolutional neural networks (CNN), deep neural networks (DNN), and/or recurrent networks, such as long short-term memory networks (LSTM), bi-directional recurrent networks, deep bi-directional recurrent networks, and the like.

In one instance referred throughout the remainder of the specification, each MHC allele in $h=1, 2, ... , m$ is associated with a separate network model, and $NN_h(\cdot)$ denotes the output(s) from a network model associated with MHC allele $h$.

FIG. 5 illustrates an example network model $NN_3(\cdot)$ in association with an arbitrary MHC allele $h=3$. As shown in FIG. 5, the network model $NN_3(\cdot)$ for MHC allele $h=3$ includes three input nodes at layer $l=1$, four nodes at layer $l=2$, two nodes at layer $l=3$, and one output node at layer $l=4$. The network model $NN_3(\cdot)$ is associated with a set of ten parameters $\theta_3$(1), $\theta_3$(2), ... , $\theta_3$(10). The network model $NN_3(\cdot)$ receives input values (individual data instances including encoded polypeptide sequence data and any other training data used) for three allele-interacting variables $x_3^k(1)$, $x_3^k(2)$, and $x_3^k(3)$ for MHC allele $h=3$ and outputs the value $NN_3(x_3^k)$.

In another instance, the identified MHC alleles $h=1, 2, ... , m$ are associated with a single network model $NN_H(\cdot)$, and $NN_h(\cdot)$ denotes one or more outputs of the single network model associated with MHC allele $h$. In such an instance, the set of parameters $\theta_h$ may correspond to a set of parameters for the single network model, and thus, the set of parameters $\theta_h$ may be shared by all MHC alleles.

FIG. 6A illustrates an example network model $NN_H(\cdot)$ shared by MHC alleles $h=1, 2, ... , m$. As shown in FIG. 6A, the network model $NN_H(\cdot)$ includes $m$ output nodes each corresponding to an MHC allele. The network model $NN_3(\cdot)$ receives the allele-interacting variables $x_3^k$ for MHC allele $h=3$ and outputs $m$ values including the value $NN_3(x_3^k)$ corresponding to the MHC allele $h=3$.

In yet another instance, the single network model $NN_H(\cdot)$ may be a network model that outputs a dependency score given the allele interacting variables $x_h^k$ and the encoded protein sequence $d_h$ of an MHC allele $h$. In such an instance, the set of parameters $\theta_h$ may again correspond to a set of parameters for the single network model, and thus, the set of parameters $\theta_h$ may be shared by all MHC alleles. Thus, in such an instance, $NN_H(\cdot)$ may denote the output of the single network model $NN_H(\cdot)$ given inputs $[x_h^k \, d_h]$ to the single network model. Such a network model is advantageous because peptide presentation probabilities for MHC alleles that were unknown in the training data can be predicted just by identification of their protein sequence.

FIG. 6B illustrates an example network model $NN_H(\cdot)$ shared by MHC alleles. As shown in FIG. 6B, the network model $NN_H(\cdot)$ receives the allele interacting variables and protein sequence of MHC allele $h=3$ as input, and outputs a dependency score $NN_3(x_3^k)$ corresponding to the MHC allele $h=3$.

In yet another instance, the dependency function $g_h(\cdot)$ can be expressed as:

$$g_h\left(x_h^k; \theta_h\right) = g'_h\left(x_h^k; \theta'_h\right) + \theta_h^0$$

where $g'_h(x_h^k; \theta'_h)$ is the affine function with a set of parameters $\theta'_h$, the network function, or the like, with a bias parameter $\theta_h^0$ in the set of parameters for allele interacting variables for the MHC allele that represents a baseline probability of presentation for the MHC allele $h$.

In another implementation, the bias parameter $\theta_h^0$ may be shared according to the gene family of the MHC allele $h$. That is, the bias parameter $\theta_h^0$ for MHC allele $h$ may be equal to $\theta_{gene(h)}^0$, where $gene(h)$ is the gene family of MHC allele $h$. For example, MHC alleles HLA-A*02:01, HLA-A*02:02, and HLA-A*02:03 may be assigned to the gene family of "HLA-A," and the bias parameter $\theta_h^0$ for each of these MHC alleles may be shared.

Returning to equation (2), as an example, the likelihood that peptide $p^k$ will be presented by MHC allele $h=3$, among $m=4$ different identified MHC alleles using the affine dependency function $g_h(\cdot)$, can be generated by:

$$u_k^3 = f\left(x_3^k \cdot \theta_3\right),$$

where $x_3^k$ are the identified allele-interacting variables for MHC allele $h=3$, and $\theta_3$ are the set of parameters determined for MHC allele $h=3$ through loss function minimization.

As another example, the likelihood that peptide $p^k$ will be presented by MHC allele $h=3$, among $m=4$ different identified MHC alleles using separate network transformation functions $g_h(\cdot)$, can be generated by:

$$u_k^3 = f\left(NN_3(x_3^k; \theta_3)\right),$$

where $x_3^k$ are the identified allele-interacting variables for MHC allele $h=3$, and $\theta_3$ are the set of parameters determined for the network model $NN_3(\cdot)$ associated with MHC allele $h=3$.

FIG. 7 illustrates generating a presentation likelihood for peptide $p^k$ in association with MHC allele $h=3$ using an example network model $NN_3(\cdot)$. As shown in FIG. 7, the network model $NN_3(\cdot)$ receives the allele-interacting variables $x_3^k$ for MHC allele $h=3$ and generates the output $NN_3(x_3^k)$. The output is mapped by function $f(\cdot)$ to generate the estimated presentation likelihood $u_k$.

### X.B.2. Per-Allele with Allele-Noninteracting Variables

In one implementation, the training module 316 incorporates allele-noninteracting variables and models the estimated presentation likelihood $u_k$ for peptide $p^k$ by:

$$u_k^h = \Pr\!\left(\boldsymbol{p^k}\text{ presented}\right) = f\left(g_w\!\left(\boldsymbol{w^k}; \boldsymbol{\theta_w}\right) + g_h\!\left(\boldsymbol{x_h^i}; \boldsymbol{\theta_h}\right)\right), \qquad (8)$$

where $\boldsymbol{w^k}$ denotes the encoded allele-noninteracting variables for peptide $\boldsymbol{p^k}$, $g_w(\cdot)$ is a function for the allele-noninteracting variables $\boldsymbol{w^k}$ based on a set of parameters $\theta_w$ determined for the allele-noninteracting variables. Specifically, the values for the set of parameters $\theta_h$ for each MHC allele $h$ and the set of parameters $\theta_w$ for allele-noninteracting variables can be determined by minimizing the loss function with respect to $\theta_h$ and $\theta_w$, where $i$ is each instance in the subset $S$ of training data 170 generated from cells expressing single MHC alleles.

The output of the dependency function $g_w(\boldsymbol{w^k}; \theta_w)$ represents a dependency score for the allele noninteracting variables indicating whether the peptide $\boldsymbol{p^k}$ will be presented by one or more MHC alleles based on the impact of allele noninteracting variables. For example, the dependency score for the allele noninteracting variables may have a high value if the peptide $\boldsymbol{p^k}$ is associated with a C-terminal flanking sequence that is known to positively impact presentation of the peptide $\boldsymbol{p^k}$, and may have a low value if the peptide $\boldsymbol{p^k}$ is associated with a C-terminal flanking sequence that is known to negatively impact presentation of the peptide $\boldsymbol{p^k}$.

According to equation (8), the per-allele likelihood that a peptide sequence $\boldsymbol{p^k}$ will be presented by a MHC allele $h$ can be generated by applying the function $g_h(\cdot)$ for the MHC allele $h$ to the encoded version of the peptide sequence $\boldsymbol{p^k}$ to generate the corresponding dependency score for allele interacting variables. The function $g_w(\cdot)$ for the allele noninteracting variables are also applied to the encoded version of the allele noninteracting variables to generate the dependency score for the allele noninteracting variables. Both scores are combined, and the combined score is transformed by the transformation function $f(\cdot)$ to generate a per-allele likelihood that the peptide sequence $\boldsymbol{p^k}$ will be presented by the MHC allele $h$.

Alternatively, the training module 316 may include allele-noninteracting variables $\boldsymbol{w^k}$ in the prediction by adding the allele-noninteracting variables $\boldsymbol{w^k}$ to the allele-interacting variables $\boldsymbol{x_h^k}$ in equation (2). Thus, the presentation likelihood can be given by:

$$u_k^h = \Pr\!\left(\boldsymbol{p^k}\text{ presented; allele }h\right) = f\left(g_h\!\left([\boldsymbol{x_h^k}\ \boldsymbol{w^k}]; \boldsymbol{\theta_h}\right)\right). \qquad (9)$$

### X.B.3 Dependency Functions for Allele-Noninteracting Variables

Similarly to the dependency function $g_h(\cdot)$ for allele-interacting variables, the dependency function $g_w(\cdot)$ for allele noninteracting variables may be an affine function or a network function in which a separate network model is associated with allele-noninteracting variables $\boldsymbol{w^k}$.

Specifically, the dependency function $g_w(\cdot)$ is an affine function given by:

$$g_w\!\left(\boldsymbol{w^k}; \boldsymbol{\theta_w}\right) = \boldsymbol{w^k} \cdot \boldsymbol{\theta_w}.$$

that linearly combines the allele-noninteracting variables in $\boldsymbol{w^k}$ with a corresponding parameter in the set of parameters $\theta_w$.

The dependency function $\boldsymbol{g_w(\cdot)}$ may also be a network function given by:

$$g_h\!\left(\boldsymbol{w^k}; \boldsymbol{\theta_w}\right) = NN_w(\boldsymbol{w^k}; \boldsymbol{\theta_w}).$$

represented by a network model $NN_w(\cdot)$ having an associated parameter in the set of parameters $\theta_w$.

In another instance, the dependency function $\boldsymbol{g_w(\cdot)}$ for the allele-noninteracting variables can be given by:

$$g_w\!\left(\boldsymbol{w^k}; \boldsymbol{\theta_w}\right) = g'_w\!\left(\boldsymbol{w^k}; \boldsymbol{\theta'_w}\right) + h(m^k; \theta_w^m), \qquad (10)$$

where $g'_w(\boldsymbol{w^k}; \theta'_w)$ is the affine function, the network function with the set of allele noninteracting parameters $\theta'_w$, or the like, $m^k$ is the mRNA quantification measurement for peptide $\boldsymbol{p^k}$, $h(\cdot)$ is a function transforming the quantification measurement, and $\theta_w^m$ is a parameter in the set of parameters for allele noninteracting variables that is combined with the mRNA quantification measurement to generate a dependency score for the mRNA quantification measurement. In one particular embodiment referred throughout the remainder of the specification, $h(\cdot)$ is the log function, however in practice $h(\cdot)$ may be any one of a variety of different functions.

In yet another instance, the dependency function the dependency function $\boldsymbol{g_w(\cdot)}$ for the allele-noninteracting variables can be given by:

$$g_w\left(\mathbf{w}^k; \boldsymbol{\theta}_w\right) = g'_w\left(\mathbf{w}^k; \boldsymbol{\theta}'_w\right) + \boldsymbol{\theta}_w^o \cdot \mathbf{o}^k, \tag{11}$$

where $g'_w(\mathbf{w}^k; \theta'_w)$ is the affine function, the network function with the set of allele noninteracting parameters $\theta'_w$, or the like, $\mathbf{o}^k$ is the indicator vector described above representing proteins and isoforms in the human proteome for peptide $\mathbf{p}^k$, and $\theta_w^o$ is a set of parameters in the set of parameters for allele noninteracting variables that is combined with the indicator vector. In one variation, when the dimensionality of $\mathbf{o}^k$ and the set of parameters $\theta_w^o$ are significantly high, a parameter regularization term, such as $\lambda \cdot ||\boldsymbol{\theta}_w^o||$, where $||\cdot||$ represents L1 norm, L2 norm, a combination, or the like, can be added to the loss function when determining the value of the parameters. The optimal value of the hyperparameter $\lambda$ can be determined through appropriate methods.

Returning to equation (8), as an example, the likelihood that peptide $\mathbf{p}^k$ will be presented by MHC allele $h=3$, among $m=4$ different identified MHC alleles using the affine transformation functions $g_h(\cdot)$, $g_w(\cdot)$, can be generated by:

$$u_k^3 = f\left(\mathbf{w}^k \cdot \boldsymbol{\theta}_w + \mathbf{x}_3^k \cdot \boldsymbol{\theta}_3\right),$$

where $\mathbf{w}^k$ are the identified allele-noninteracting variables for peptide $\mathbf{p}^k$, and $\theta_w$ are the set of parameters determined for the allele-noninteracting variables.

As another example, the likelihood that peptide $\mathbf{p}^k$ will be presented by MHC allele $h=3$, among $m=4$ different identified MHC alleles using the network transformation functions $g_h(\cdot)$, $g_w(\cdot)$, can be generated by:

$$u_k^3 = f\left(NN_w\left(\mathbf{w}^k; \boldsymbol{\theta}_w\right) + NN_3(\mathbf{x}_3^k; \boldsymbol{\theta}_3)\right)$$

where $\mathbf{w}^k$ are the identified allele-interacting variables for peptide $\mathbf{p}^k$, and $\theta_w$ are the set of parameters determined for allele-noninteracting variables.

FIG. 8 illustrates generating a presentation likelihood for peptide $\mathbf{p}^k$ in association with MHC allele $h=3$ using example network models $NN_3(\cdot)$ and $NN_w(\cdot)$. As shown in FIG. 8, the network model $NN_3(\cdot)$ receives the allele-interacting variables $\mathbf{x}_3^k$ for MHC allele $h=3$ and generates the output $NN_3(\mathbf{x}_3^k)$. The network model $NN_w(\cdot)$ receives the allele-noninteracting variables $\mathbf{w}^k$ for peptide $\mathbf{p}^k$ and generates the output $NN_w(\mathbf{w}^k)$. The outputs are combined and mapped by function $f(\cdot)$ to generate the estimated presentation likelihood $u_k$.

**X.C. Multiple-Allele Models**

The training module 316 may also construct the presentation models to predict presentation likelihoods of peptides in a multiple-allele setting where two or more MHC alleles are present. In this case, the training module 316 may train the presentation models based on data instances $S$ in the training data 170 generated from cells expressing single MHC alleles, cells expressing multiple MHC alleles, or a combination thereof.

**X.C.1. Example 1: Maximum of Per-Allele Models**

In one implementation, the training module 316 models the estimated presentation likelihood $u_k$ for peptide $\mathbf{p}^k$ in association with a set of multiple MHC alleles $H$ as a function of the presentation likelihoods $u_k^{h \in H}$ determined for each of the MHC alleles $h$ in the set $H$ determined based on cells expressing single-alleles, as described above in conjunction with equations (2)-(11). Specifically, the presentation likelihood $u_k$ can be any function of $u_k^{h \in H}$. In one implementation, as shown in equation (12), the function is the maximum function, and the presentation likelihood $u_k$ can be determined as the maximum of the presentation likelihoods for each MHC allele $h$ in the set $H$.

$$u_k = \Pr\left(\mathbf{p}^k \text{ presented; alleles } H\right) = \max\left(u_k^{h \in H}\right). \tag{12}$$

**X.C.2. Example 2.1: Function-of-Sums Models**

In one implementation, the training module 316 models the estimated presentation likelihood $u_k$ for peptide $\mathbf{p}^k$ by:

$$u_k = \Pr\left(\mathbf{p}^k \text{ presented}\right) = f\left(\sum_{h=1}^{m} a_h^k \cdot g_h\left(\mathbf{x}_h^k; \boldsymbol{\theta}_h\right)\right), \tag{13}$$

46

where elements $a_h{}^k$ are 1 for the multiple MHC alleles $H$ associated with peptide sequence $\boldsymbol{p}^k$ and $x_h{}^k$ denotes the encoded allele-interacting variables for peptide $\boldsymbol{p}^k$ and the corresponding MHC alleles. The values for the set of parameters $\theta_h$ for each MHC allele $h$ can be determined by minimizing the loss function with respect to $\theta_h$, where $i$ is each instance in the subset S of training data 170 generated from cells expressing single MHC alleles and/or cells expressing multiple MHC alleles. The dependency function $\boldsymbol{g_h}$ may be in the form of any of the dependency functions $\boldsymbol{g_h}$ introduced above in sections X.B.1.

According to equation (13), the presentation likelihood that a peptide sequence $\boldsymbol{p}^k$ will be presented by one or more MHC alleles $h$ can be generated by applying the dependency function $g_h(\cdot)$ to the encoded version of the peptide sequence $\boldsymbol{p}^k$ for each of the MHC alleles $H$ to generate the corresponding score for the allele interacting variables. The scores for each MHC allele $h$ are combined, and transformed by the transformation function $f(\cdot)$ to generate the presentation likelihood that peptide sequence $\boldsymbol{p}^k$ will be presented by the set of MHC alleles $H$.

The presentation model of equation (13) is different from the per-allele model of equation (2), in that the number of associated alleles for each peptide $\boldsymbol{p}^k$ can be greater than 1. In other words, more than one element in $a_h{}^k$ can have values of 1 for the multiple MHC alleles $H$ associated with peptide sequence $\boldsymbol{p}^k$.

As an example, the likelihood that peptide $\boldsymbol{p}^k$ will be presented by MHC alleles $h=2, h=3,$ among $m=4$ different identified MHC alleles using the affine transformation functions $g_h(\cdot)$, can be generated by:

$$u_k = f\left(x_2^k \cdot \boldsymbol{\theta_2} + x_3^k \cdot \boldsymbol{\theta_3}\right),$$

where $\boldsymbol{x_2}^k, \boldsymbol{x_3}^k$ are the identified allele-interacting variables for MHC alleles $h=2, h=3,$ and $\theta_2, \theta_3$ are the set of parameters determined for MHC alleles $h=2, h=3.$

As another example, the likelihood that peptide $\boldsymbol{p}^k$ will be presented by MHC alleles $h=2, h=3,$ among $m=4$ different identified MHC alleles using the network transformation functions $g_h(\cdot), g_w(\cdot)$, can be generated by:

$$\boldsymbol{u_k} = f\left(NN_2(\boldsymbol{x_2^k}; \boldsymbol{\theta_2}) + NN_3(\boldsymbol{x_3^k}; \boldsymbol{\theta_3})\right),$$

where $NN_2(\cdot), NN_3(\cdot)$ are the identified network models for MHC alleles $h=2, h=3,$ and $\theta_2, \theta_3$ are the set of parameters determined for MHC alleles $h=2, h=3.$

FIG. 9 illustrates generating a presentation likelihood for peptide $\boldsymbol{p}^k$ in association with MHC alleles $h=2, h=3$ using example network models $NN_2(\cdot)$ and $NN_3(\cdot)$. As shown in FIG. 9, the network model $NN_2(\cdot)$ receives the allele-interacting variables $x_2^k$ for MHC allele $h=2$ and generates the output $NN_2(x_2^k)$ and the network model $NN_3(\cdot)$ receives the allele-interacting variables $x_3^k$ for MHC allele $h=3$ and generates the output $NN_3(x_3^k)$. The outputs are combined and mapped by function $f(\cdot)$ to generate the estimated presentation likelihood $u_k$.

### X.C.3. Example 2.2: Function-of-Sums Models with Allele-Noninteracting Variables

In one implementation, the training module 316 incorporates allele-noninteracting variables and models the estimated presentation likelihood $u_k$ for peptide $\boldsymbol{p}^k$ by:

$$u_k = \Pr\left(\boldsymbol{p}^k \text{ presented}\right) = f\left(g_w\left(\boldsymbol{w}^k; \boldsymbol{\theta_w}\right) + \sum_{h=1}^{m} a_h^k \cdot g_h\left(\boldsymbol{x_h^k};\ \boldsymbol{\theta_h}\right)\right), \qquad (14)$$

where $\boldsymbol{w}^k$ denotes the encoded allele-noninteracting variables for peptide $\boldsymbol{p}^k$. Specifically, the values for the set of parameters $\theta_h$ for each MHC allele $h$ and the set of parameters $\theta_w$ for allele-noninteracting variables can be determined by minimizing the loss function with respect to $\theta_h$ and $\theta_w$, where $i$ is each instance in the subset S of training data 170 generated from cells expressing single MHC alleles and/or cells expressing multiple MHC alleles. The dependency function $\boldsymbol{g_w}$ may be in the form of any of the dependency functions $\boldsymbol{g_w}$ introduced above in sections X.B.3.

Thus, according to equation (14), the presentation likelihood that a peptide sequence $\boldsymbol{p}^k$ will be presented by one or more MHC alleles $H$ can be generated by applying the function $g_h(\cdot)$ to the encoded version of the peptide sequence $\boldsymbol{p}^k$ for each of the MHC alleles $H$ to generate the corresponding dependency score for allele interacting variables for each MHC allele $h$. The function $g_w(\cdot)$ for the allele noninteracting variables is also applied to the encoded version of the allele noninteracting variables to generate the dependency score for the allele noninteracting variables. The scores are combined, and the combined score is transformed by the transformation function $f(\cdot)$ to generate the presentation likelihood that peptide sequence $\boldsymbol{p}^k$ will be presented by the MHC alleles $H$.

In the presentation model of equation (14), the number of associated alleles for each peptide $\boldsymbol{p}^k$ can be greater than 1. In other words, more than one element in $a_h{}^k$ can have values of 1 for the multiple MHC alleles $H$ associated with peptide sequence $\boldsymbol{p^k}$.

As an example, the likelihood that peptide $\boldsymbol{p^k}$ will be presented by MHC alleles $h=2$, $h=3$, among $m=4$ different identified MHC alleles using the affine transformation functions $g_h(\cdot)$, $g_w(\cdot)$, can be generated by:

$$u_k = f\left(\boldsymbol{w^k} \cdot \boldsymbol{\theta}_w + x_2^k \cdot \boldsymbol{\theta}_2 + x_3^k \cdot \boldsymbol{\theta}_3\right),$$

where $\boldsymbol{w^k}$ are the identified allele-noninteracting variables for peptide $\boldsymbol{p^k}$, and $\theta_w$ are the set of parameters determined for the allele-noninteracting variables.

As another example, the likelihood that peptide $\boldsymbol{p^k}$ will be presented by MHC alleles $h=2$, $h=3$, among $m=4$ different identified MHC alleles using the network transformation functions $g_h(\cdot)$, $g_w(\cdot)$, can be generated by:

$$u_k = f\left(NN_w\left(\boldsymbol{w^k}; \boldsymbol{\theta}_w\right) + NN_2(x_2^k; \boldsymbol{\theta}_2) + NN_3(x_3^k; \boldsymbol{\theta}_3)\right)$$

where $\boldsymbol{w^k}$ are the identified allele-interacting variables for peptide $\boldsymbol{p^k}$, and $\theta_w$ are the set of parameters determined for allele-noninteracting variables.

FIG. 10 illustrates generating a presentation likelihood for peptide $\boldsymbol{p^k}$ in association with MHC alleles $h=2$, $h=3$ using example network models $NN_2(\cdot)$, $NN_3(\cdot)$, and $NN_w(\cdot)$. As shown in FIG. 10, the network model $NN_2(\cdot)$ receives the allele-interacting variables $\boldsymbol{x_2{}^k}$ for MHC allele $h=2$ and generates the output $NN_2(x_2{}^k)$. The network model $NN_3(\cdot)$ receives the allele-interacting variables $\boldsymbol{x_3{}^k}$ for MHC allele $h=3$ and generates the output $\boldsymbol{NN_3(x_3{}^k)}$. The network model $NN_w(\cdot)$ receives the allele-noninteracting variables $\boldsymbol{w^k}$ for peptide $\boldsymbol{p^k}$ and generates the output $\boldsymbol{NN_w(w^k)}$. The outputs are combined and mapped by function $f(\cdot)$ to generate the estimated presentation likelihood $u_k$.

Alternatively, the training module 316 may include allele-noninteracting variables $w^k$ in the prediction by adding the allele-noninteracting variables $\boldsymbol{w^k}$ to the allele-interacting variables $\boldsymbol{x_h{}^k}$ in equation (15). Thus, the presentation likelihood can be given by:

$$u_k = \Pr\left(\boldsymbol{p^k} \text{ presented}\right) = f\left(\sum_{h=1}^{m} a_h^k \cdot g_h\left([\boldsymbol{x_h^k}\, \boldsymbol{w^k}]; \boldsymbol{\theta}_h\right)\right). \tag{15}$$

### X.C.4. Example 3.1: Models Using Implicit Per-Allele Likelihoods

In another implementation, the training module 316 models the estimated presentation likelihood $u_k$ for peptide $\boldsymbol{p^k}$ by:

$$u_k = \Pr\left(\boldsymbol{p^k} \text{ presented}\right) = r\left(s\left(\boldsymbol{v} = \left[a_1^k \cdot u'^1_k(\boldsymbol{\theta}) \ldots a_m^k \cdot u'^m_k(\boldsymbol{\theta})\right]\right)\right), \tag{16}$$

where elements $a_h{}^k$ are 1 for the multiple MHC alleles $h \in H$ associated with peptide sequence $\boldsymbol{p^k}$, $u'_k{}^h$ is an implicit per-allele presentation likelihood for MHC allele $h$, vector $\boldsymbol{v}$ is a vector in which element $v_h$ corresponds to $a_h{}^k \cdot u'_k{}^h$, $s(\cdot)$ is a function mapping the elements of $\boldsymbol{v}$, and $r(\cdot)$ is a clipping function that clips the value of the input into a given range. As described below in more detail, $s(\cdot)$ may be the summation function or the second-order function, but it is appreciated that in other embodiments, $s(\cdot)$ can be any function such as the maximum function. The values for the set of parameters $\theta$ for the implicit per-allele likelihoods can be determined by minimizing the loss function with respect to $\theta$, where $i$ is each instance in the subset S of training data 170 generated from cells expressing single MHC alleles and/or cells expressing multiple MHC alleles.

The presentation likelihood in the presentation model of equation (17) is modeled as a function of implicit per-allele presentation likelihoods $u'_k{}^h$ that each correspond to the likelihood peptide $p^k$ will be presented by an individual MHC allele $h$. The implicit per-allele likelihood is distinct from the per-allele presentation likelihood of section X.B in that the parameters for implicit per-allele likelihoods can be learned from multiple allele settings, in which direct association between a presented peptide and the corresponding MHC allele is unknown, in addition to single-allele settings. Thus, in a multiple-allele setting, the presentation model can estimate not only whether peptide $\boldsymbol{p^k}$ will be presented by a set of MHC alleles $H$ as a whole, but can also provide individual likelihoods $u'_k{}^{h \in H}$ that indicate which MHC allele $h$ most likely presented peptide $\boldsymbol{p^k}$. An advantage of this is that the presentation model can generate the implicit likelihoods without training data for cells expressing single MHC alleles.

In one particular implementation referred throughout the remainder of the specification, $r(\cdot)$ is a function having the range [0, 1]. For example, $r(\cdot)$ may be the clip function:

$$r(z) = \min\left(\max(z, 0), 1\right),$$

where the minimum value between z and 1 is chosen as the presentation likelihood $u_k$. In another implementation, $r(\cdot)$ is the hyperbolic tangent function given by:

$$r(z) = \tanh(z)$$

when the values for the domain z is equal to or greater than 0.

### X.C.5. Example 3.2: Sum-of-Functions Model

In one particular implementation, $s(\cdot)$ is a summation function, and the presentation likelihood is given by summing the implicit per-allele presentation likelihoods:

$$u_k = \Pr\left(\boldsymbol{p^k} \text{ presented}\right) = r\left(\sum_{h=1}^{m} a_h^k \cdot u'_k^h(\boldsymbol{\theta})\right). \qquad (17)$$

In one implementation, the implicit per-allele presentation likelihood for MHC allele $h$ is generated by:

$$u'_k^h = f\left(g_h\left(\boldsymbol{x_h^k}; \boldsymbol{\theta_h}\right)\right), \qquad (18)$$

such that the presentation likelihood is estimated by:

$$u_k = \Pr\left(\boldsymbol{p^k} \text{ presented}\right) = r\left(\sum_{h=1}^{m} a_h^k \cdot f\left(g_h\left(\boldsymbol{x_h^k}; \boldsymbol{\theta_h}\right)\right)\right). \qquad (19)$$

According to equation (19), the presentation likelihood that a peptide sequence $\boldsymbol{p^k}$ will be presented by one or more MHC alleles $H$ can be generated by applying the function $g_h(\cdot)$ to the encoded version of the peptide sequence $\boldsymbol{p^k}$ for each of the MHC alleles $H$ to generate the corresponding dependency score for allele interacting variables. Each dependency score is first transformed by the function $f(\cdot)$ to generate implicit per-allele presentation likelihoods $u'_k^h$. The per-allele likelihoods $u'_k^h$ are combined, and the clipping function may be applied to the combined likelihoods to clip the values into a range [0, 1] to generate the presentation likelihood that peptide sequence $\boldsymbol{p^k}$ will be presented by the set of MHC alleles $H$. The dependency function $\boldsymbol{g_h}$ may be in the form of any of the dependency functions $\boldsymbol{g_h}$ introduced above in sections X.B.1.

As an example, the likelihood that peptide $\boldsymbol{p^k}$ will be presented by MHC alleles $h=2, h=3,$ among $m=4$ different identified MHC alleles using the affine transformation functions $g_h(\cdot)$, can be generated by:

$$u_k = r\left(f\left(\boldsymbol{x_2^k} \cdot \boldsymbol{\theta_2}\right) + f\left(\boldsymbol{x_3^k} \cdot \boldsymbol{\theta_3}\right)\right),$$

where $\boldsymbol{x_2^k}, \boldsymbol{x_3^k}$ are the identified allele-interacting variables for MHC alleles $h=2, h=3,$ and $\theta_2, \theta_3$ are the set of parameters determined for MHC alleles $h=2, h=3$.

As another example, the likelihood that peptide $\boldsymbol{p^k}$ will be presented by MHC alleles $h=2, h=3,$ among $m=4$ different identified MHC alleles using the network transformation functions $g_h(\cdot)$, $g_w(\cdot)$, can be generated by:

$$u_k = r\left(f\left(NN_2(\boldsymbol{x_2^k}; \boldsymbol{\theta_2})\right) + f\left(NN_3(\boldsymbol{x_3^k}; \boldsymbol{\theta_3})\right)\right),$$

where $NN_2(\cdot)$, $NN_3(\cdot)$ are the identified network models for MHC alleles $h=2, h=3,$ and $\theta_2, \theta_3$ are the set of parameters determined for MHC alleles $h=2, h=3$.

FIG. 11 illustrates generating a presentation likelihood for peptide $\boldsymbol{p^k}$ in association with MHC alleles $h=2, h=3$

using example network models $NN_2(\cdot)$ and $NN_3(\cdot)$. As shown in FIG. 9, the network model $NN_2(\cdot)$ receives the allele-interacting variables $x_2{}^k$ for MHC allele $h=2$ and generates the output $NN_2(x_2{}^k)$ and the network model $NN_3(\cdot)$ receives the allele-interacting variables $x_3{}^k$ for MHC allele $h=3$ and generates the output $NN_3(x_3{}^k)$. Each output is mapped by function $f(\cdot)$ and combined to generate the estimated presentation likelihood $u_k$.

In another implementation, when the predictions are made for the log of mass spectrometry ion currents, $r(\cdot)$ is the log function and $f(\cdot)$ is the exponential function.

### X.C.6. Example 3.3: Sum-of-Functions Models with Allele-noninteracting Variables

In one implementation, the implicit per-allele presentation likelihood for MHC allele $h$ is generated by:

$$u_k'{}^h = f\left(g_h\left(x_h^k; \boldsymbol{\theta_h}\right) + g_w\left(w^k; \boldsymbol{\theta_w}\right)\right), \qquad (20)$$

such that the presentation likelihood is generated by:

$$u_k = \Pr\left(p^k \text{ presented}\right) = r\left(\sum_{h=1}^{m} a_h^k \cdot f\left(g_w\left(w^k; \boldsymbol{\theta_w}\right) + g_h\left(x_h^k; \boldsymbol{\theta_h}\right)\right)\right), \qquad (21)$$

to incorporate the impact of allele noninteracting variables on peptide presentation.

According to equation (21), the presentation likelihood that a peptide sequence $p^k$ will be presented by one or more MHC alleles $H$ can be generated by applying the function $g_h(\cdot)$ to the encoded version of the peptide sequence $p^k$ for each of the MHC alleles $H$ to generate the corresponding dependency score for allele interacting variables for each MHC allele $h$. The function $g_w(\cdot)$ for the allele noninteracting variables is also applied to the encoded version of the allele noninteracting variables to generate the dependency score for the allele noninteracting variables. The score for the allele noninteracting variables are combined to each of the dependency scores for the allele interacting variables. Each of the combined scores are transformed by the function $f(\cdot)$ to generate the implicit per-allele presentation likelihoods. The implicit likelihoods are combined, and the clipping function may be applied to the combined outputs to clip the values into a range [0,1] to generate the presentation likelihood that peptide sequence $p^k$ will be presented by the MHC alleles $H$. The dependency function $g_w$ may be in the form of any of the dependency functions $g_w$ introduced above in sections X.B.3.

As an example, the likelihood that peptide $p^k$ will be presented by MHC alleles $h=2$, $h=3$, among $m=4$ different identified MHC alleles using the affine transformation functions $g_h(\cdot)$, $g_w(\cdot)$, can be generated by:

$$u_k = r\left(f\left(w^k \cdot \boldsymbol{\theta_w} + x_2^k \cdot \boldsymbol{\theta_2}\right) + f\left(w^k \cdot \boldsymbol{\theta_w} + x_3^k \cdot \boldsymbol{\theta_3}\right)\right),$$

where $w^k$ are the identified allele-noninteracting variables for peptide $p^k$, and $\theta_w$ are the set of parameters determined for the allele-noninteracting variables.

As another example, the likelihood that peptide $p^k$ will be presented by MHC alleles $h=2$, $h=3$, among $m=4$ different identified MHC alleles using the network transformation functions $g_h(\cdot)$, $g_w(\cdot)$, can be generated by:

$$u_k = r\left(f\left(NN_w\left(w^k; \boldsymbol{\theta_w}\right) + NN_2(x_2^k; \boldsymbol{\theta_2})\right) + f\left(NN_w\left(w^k; \boldsymbol{\theta_w}\right) + NN_3(x_3^k; \boldsymbol{\theta_3})\right)\right)$$

where $w^k$ are the identified allele-interacting variables for peptide $p^k$, and $\theta_w$ are the set of parameters determined for allele-noninteracting variables.

FIG. 12 illustrates generating a presentation likelihood for peptide $p^k$ in association with MHC alleles $h=2$, $h=3$ using example network models $NN_2(\cdot)$, $NN_3(\cdot)$, and $NN_w(\cdot)$. As shown in FIG. 12, the network model $NN_2(\cdot)$ receives the allele-interacting variables $x_2{}^k$ for MHC allele $h=2$ and generates the output $NN_2(x_2{}^k)$. The network model $NN_w(\cdot)$ receives the allele-noninteracting variables $w^k$ for peptide $p^k$ and generates the output $NN_w(w^k)$. The outputs are combined and mapped by function $f(\cdot)$. The network model $NN_3(\cdot)$ receives the allele-interacting variables $x_3{}^k$ for MHC allele $h=3$ and generates the output $NN_3(x_3{}^k)$, which is again combined with the output $NN_w(w^k)$ of the same network model $NN_w(\cdot)$ and mapped by function $f(\cdot)$. Both outputs are combined to generate the estimated presentation likelihood $u_k$.

In another implementation, the implicit per-allele presentation likelihood for MHC allele $h$ is generated by:

$$u_k'^{\,h} = f\left(g_h\left([\boldsymbol{x}_h^k\,\boldsymbol{w}^k];\boldsymbol{\theta_h}\right)\right). \tag{22}$$

such that the presentation likelihood is generated by:

$$u_k = \Pr\left(\boldsymbol{p}^k \text{ presented}\right) = r\left(\sum_{h=1}^{m} a_h^k \cdot f\left(g_h\left([\boldsymbol{x}_h^k\,\boldsymbol{w}^k];\boldsymbol{\theta_h}\right)\right)\right).$$

### X.C.7. Example 4: Second Order Models

In one implementation, , $s(\cdot)$ is a second-order function, and the estimated presentation likelihood $u_k$ for peptide $\boldsymbol{p}^k$ is given by:

$$u_k = \Pr\left(\boldsymbol{p}^k \text{ presented}\right) = \sum_{h=1}^{m} a_h^k \cdot u_k'^{\,h}(\boldsymbol{\theta}) - \sum_{h=1}^{m}\sum_{j<h} a_h^k \cdot a_j^k \cdot u_k'^{\,h}(\boldsymbol{\theta}) \cdot u_k'^{\,j}(\boldsymbol{\theta}) \tag{23}$$

where elements $u_k'^{\,h}$ are the implicit per-allele presentation likelihood for MHC allele $h$. The values for the set of parameters $\theta$ for the implicit per-allele likelihoods can be determined by minimizing the loss function with respect to $\theta$, where $i$ is each instance in the subset $S$ of training data 170 generated from cells expressing single MHC alleles and/or cells expressing multiple MHC alleles. The implicit per-allele presentation likelihoods may be in any form shown in equations (18), (20), and (22) described above.

In one aspect, the model of equation (23) may imply that there exists a possibility peptide $\boldsymbol{p}^k$ will be presented by two MHC alleles simultaneously, in which the presentation by two HLA alleles is statistically independent.

According to equation (23), the presentation likelihood that a peptide sequence $\boldsymbol{p}^k$ will be presented by one or more MHC alleles $H$ can be generated by combining the implicit per-allele presentation likelihoods and subtracting the likelihood that each pair of MHC alleles will simultaneously present the peptide $\boldsymbol{p}^k$ from the summation to generate the presentation likelihood that peptide sequence $\boldsymbol{p}^k$ will be presented by the MHC alleles $H$.

As an example, the likelihood that peptide $\boldsymbol{p}^k$ will be presented by HLA alleles $h=2, h=3,$ among $m=4$ different identified HLA alleles using the affine transformation functions $g_h(\cdot)$, can be generated by:

$$u_k = f\left(\boldsymbol{x}_2^k \cdot \boldsymbol{\theta}_2\right) + f\left(\boldsymbol{x}_3^k \cdot \boldsymbol{\theta}_3\right) - f\left(\boldsymbol{x}_2^k \cdot \boldsymbol{\theta}_2\right) \cdot f\left(\boldsymbol{x}_3^k \cdot \boldsymbol{\theta}_3\right),$$

where $\boldsymbol{x}_2^k, \boldsymbol{x}_3^k$ are the identified allele-interacting variables for HLA alleles $h=2, h=3,$ and $\theta_2, \theta_3$ are the set of parameters determined for HLA alleles $h=2, h=3$.

As another example, the likelihood that peptide $\boldsymbol{p}^k$ will be presented by HLA alleles $h=2, h=3,$ among $m=4$ different identified HLA alleles using the network transformation functions $g_h(\cdot)$, $g_w(\cdot)$, can be generated by:

$$u_k = f\left(NN_2(\boldsymbol{x}_2^k;\boldsymbol{\theta}_2)\right) + f\left(NN_3(\boldsymbol{x}_3^k;\boldsymbol{\theta}_3)\right) - f\left(NN_2(\boldsymbol{x}_2^k;\boldsymbol{\theta}_2)\right) \cdot f\left(NN_3(\boldsymbol{x}_3^k;\boldsymbol{\theta}_3)\right),$$

where $NN_2(\cdot)$, $NN_3(\cdot)$ are the identified network models for HLA alleles $h=2, h=3,$ and $\theta_2, \theta_3$ are the set of parameters determined for HLA alleles $h=2, h=3$.

### XI.A Example 5: Prediction Module

The prediction module 320 receives sequence data and selects candidate neoantigens in the sequence data using the presentation models. Specifically, the sequence data may be DNA sequences, RNA sequences, and/or protein sequences extracted from tumor tissue cells of patients. The prediction module 320 processes the sequence data into a plurality of peptide sequences $\boldsymbol{p}^k$ having 8-15 amino acids. For example, the prediction module 320 may process the given sequence "IEFROEIFJEF (SEQ ID NO: 73) into three peptide sequences having 9 amino acids "IEFROEIFJ (SEQ ID NO: 74)," "EFROEIFJE (SEQ ID NO: 75)," and "FROEIFJEF (SEQ ID NO: 76)." In one embodiment, the prediction module 320 may identify candidate neoantigens that are mutated peptide sequences by comparing sequence data extracted from normal tissue cells of a patient with the sequence data extracted from tumor tissue cells of the patient to identify portions containing one or more mutations.

The presentation module 320 applies one or more of the presentation models to the processed peptide sequences to estimate presentation likelihoods of the peptide sequences. Specifically, the prediction module 320 may select one or more candidate neoantigen peptide sequences that are likely to be presented on tumor HLA molecules by applying the presentation models to the candidate neoantigens. In one implementation, the presentation module 320 selects candidate neoantigen sequences that have estimated presentation likelihoods above a predetermined threshold. In another implementation, the presentation model selects the *N* candidate neoantigen sequences that have the highest estimated presentation likelihoods (where *N* is generally the maximum number of epitopes that can be delivered in a vaccine). A vaccine including the selected candidate neoantigens for a given patient can be injected into the patient to induce immune responses.

### XI.B. Example 6: Cassette Design Module

### XI.B.1 Overview

The cassette design module 324 generates a vaccine cassette sequence based on the *v* selected candidate peptides for injection into a patient. Specifically, for a set of selected peptides $p^k$, *k=1, 2, ... , v* for inclusion in a vaccine of capacity *v*, the cassette sequence is given by concatenation of a series of therapeutic epitope sequences $p'^k$, *k=1, 2, ... , v* that each include the sequence of a corresponding peptide $p^k$. The cassette design module 324 may concatenate the epitopes directly adjacent to one another. For example, a vaccine cassette **C** may be represented as:

$$\mathbf{C} = [\boldsymbol{p'}^{t_1} \ \boldsymbol{p'}^{t_2} \ \cdots \ \boldsymbol{p'}^{t_v}] \tag{24}$$

where $\boldsymbol{p'}^{ti}$ denotes the *i*-th epitope of the cassette. Thus, $t_i$ corresponds to an index *k=1, 2, ... , v* for the selected peptide at the *i*-th position of the cassette. In embodiments of the invention, the cassette design module 324 concatenates the epitopes with one or more linker sequences in between adjacent epitopes. For example, a vaccine cassette **C** may be represented as:

$$\mathbf{C} = [\boldsymbol{p'}^{t_1} \ \boldsymbol{l}_{(\mathrm{t_1,t_2})} \ \boldsymbol{p'}^{t_2} \ \boldsymbol{l}_{(\mathrm{t_2,t_3})} \cdots \boldsymbol{l}_{(\mathrm{t_{v-1},t_v})} \ \boldsymbol{p'}^{t_v}] \tag{25}$$

where $\boldsymbol{l}_{(ti,tj)}$ denotes a linker sequence placed between the *i*-th epitope $\boldsymbol{p'}^{ti}$ and the *j=i+1*-th epitope $\boldsymbol{p'}^{j=i+1}$ of the cassette. The cassette design module 324 determines which of the selected epitopes $\boldsymbol{p'}^k$, *k=1, 2, ... , v* are arranged at the different positions of the cassette, as well as any linker sequences placed between the epitopes. A cassette sequence **C** can be loaded as a vaccine based on any of the methods described in the present specification.

In one embodiment, the set of therapeutic epitopes may be generated based on the selected peptides determined by the prediction module 320 associated with presentation likelihoods above a predetermined threshold, where the presentation likelihoods are determined by the presentation models. However it is appreciated that in other embodiments, the set of therapeutic epitopes may be generated based on any one or more of a number of methods (alone or in combination), for example, based on binding affinity or predicted binding affinity to HLA class I or class II alleles of the patient, binding stability or predicted binding stability to HLA class I or class II alleles of the patient, random sampling, and the like.

In one embodiment, the therapeutic epitopes $\boldsymbol{p'}^k$ may correspond to the selected peptides $\boldsymbol{p}^k$ themselves. The therapeutic epitopes $\boldsymbol{p'}^k$ include C- and N-terminal flanking sequences in addition to the selected peptides. For example, an epitope $\boldsymbol{p'}^k$ included in the cassette may be represented as a sequence $[\boldsymbol{n}^k \ \boldsymbol{p}^k \ \boldsymbol{c}^k]$ where $\boldsymbol{c}^k$ is a C-terminal flanking sequence attached the C-terminus of the selected peptide $\boldsymbol{p}^k$, and $\boldsymbol{n}^k$ is an N-terminal flanking sequence attached to the N-terminus of the selected peptide $\boldsymbol{p}^k$. The N- and C-terminal flanking sequences are the native N- and C-terminal flanking sequences of the therapeutic vaccine epitope in the context of its source protein. In one instance referred throughout the remainder of the specification, the therapeutic epitope $\boldsymbol{p'}^k$ represents a fixed-length epitope. In another instance, the therapeutic epitope $\boldsymbol{p'}^k$ can represent a variable-length epitope, in which the length of the epitope can be varied depending on, for example, the length of the C or N-flanking sequence. For example, the C-terminal flanking sequence $c^k$ and the N-terminal flanking sequence $\boldsymbol{n}^k$ *can* each have varying lengths of 2-5 residues, resulting in 16 possible choices for the epitope $\boldsymbol{p'}^k$.

In one embodiment, the cassette design module 324 generates cassette sequences by taking into account presentation of junction epitopes that span the junction between a pair of therapeutic epitopes in the cassette. Junction epitopes are novel non-self but irrelevant epitope sequences that arise in the cassette due to the process of concatenating therapeutic epitopes and linker sequences in the cassette. The novel sequences of junction epitopes are different from the therapeutic epitopes of the cassette themselves. A junction epitope spanning epitopes $\boldsymbol{p'}^{ti}$ and $\boldsymbol{p'}^{tj}$ may include any epitope sequence that overlaps with both $\boldsymbol{p'}^{ti}$ or $\boldsymbol{p'}^{tj}$ that is different from the sequences of therapeutic epitopes $\boldsymbol{p'}^{ti}$ and $\boldsymbol{p'}^{tj}$

themselves. Specifically, each junction between epitope $p'^{ti}$ and an adjacent epitope $p'^{tj}$ of the cassette with or without an optional linker sequence $l^{(ti,tj)}$ may be associated with $n_{(ti,tj)}$ junction epitopes $e_n^{(ti,tj)}$, $n=1, 2, \ldots, n_{(ti,tj)}$. The junction epitopes may be sequences that at least partially overlap with both epitopes $p'^{ti}$ and $p'^{tj}$, or may be sequences that at least partially overlap with linker sequences placed between the epitopes $p'^{ti}$ and $p'^{tj}$. Junction epitopes may be presented by MHC class I, MHC class II, or both.

FIG. 13 shows two example cassette sequences, cassette 1 **(C$_1$)** and cassette 2 **(C$_2$)**. Each cassette has a vaccine capacity of $v=2$, and includes therapeutic epitopes $p'^{t1} = p^1$ = SINFEKL (SEQ ID NO: 185) and $p'^{t2} = p^2$ = LLLLLVVVV (SEQ ID NO: 77), and a linker sequence $l^{(t1,t2)}$ = AAY in between the two epitopes. Specifically, the sequence of cassette **C$_1$** is given by **[$p^1$ $l^{(tl,t2)}$ $p^2$]**, while the sequence of cassette **C$_2$** is given by **[$p^2$ $l^{(t1,t2)}$ $p^1$]**. Example junction epitopes $e_n^{(1,2)}$ of cassette **C$_1$** may be sequences such as EKLAAYLLL (SEQ ID NO: 78), KLAAYLLLLL (SEQ ID NO: 79), and FEKLAAYL (SEQ ID NO: 80) that span across both epitopes $p'^1$ and $p'^2$ in the cassette, and may be sequences such as AAYLLLLL (SEQ ID NO: 81) and YLLLLLVVV (SEQ ID NO: 82) that span across the linker sequence and a single selected epitope in the cassette. Similarly, example junction epitopes $e_m^{(2,1)}$ of cassette **C$_2$** may be sequences such as VVVVAAYSIN (SEQ ID NO: 83), VVVVAAY (SEQ ID NO: 84), and AYSINFEK (SEQ ID NO: 85). Although both cassettes involve the same set of sequences $p^1$, $l^{(c1,c2)}$, and $p^2$, the set of junction epitopes that are identified are different depending on the ordered sequence of the therapeutic epitopes within the cassette.

In one embodiment, the cassette design module 324 generates a cassette sequence that reduces the likelihood that junction epitopes are presented in the patient. Specifically, when the cassette is injected into the patient, junction epitopes have the potential to be presented by HLA class I or HLA class II alleles of the patient, and stimulate a CD8 or CD4 T-cell response, respectively. Such reactions are often times undesirable because T-cells reactive to the junction epitopes have no therapeutic benefit, and may diminish the immune response to the selected therapeutic epitopes in the cassette by antigenic competition.[76]

In one embodiment, the cassette design module 324 iterates through one or more candidate cassettes, and determines a cassette sequence for which a presentation score of junction epitopes associated with that cassette sequence is below a numerical threshold. The junction epitope presentation score is a quantity associated with presentation likelihoods of the junction epitopes in the cassette, and a higher value of the junction epitope presentation score indicates a higher likelihood that junction epitopes of the cassette will be presented by HLA class I or HLA class II or both.

In one embodiment, the cassette design module 324 may determine a cassette sequence associated with the lowest junction epitope presentation score among the candidate cassette sequences. In one instance, the presentation score for a given cassette sequence **C** is determined based on a set of distance metrics $d(e_n^{(ti,tj)}, n=1, 2, \ldots, n_{(ti,tj)}) = d_{(ti,tj)}$ each associated with a junction in the cassette **C**. Specifically, a distance metric $d_{(ti,tj)}$ specifies a likelihood that one or more of the junction epitopes spanning between the pair of adjacent therapeutic epitopes $p'^{ti}$ and $p'^{tj}$ will be presented. The junction epitope presentation score for cassette **C** can then be determined by applying a function (e.g., summation, statistical function) to the set of distance metrics for the cassette C. Mathematically, the presentation score is given by:

$$score = h(d_{(t_1,t_2)}, d_{(t_2,t_3)}, \ldots, d_{(t_{v-1},t_v)}) \qquad (26)$$

where $h(\cdot)$ is some function mapping the distance metrics of each junction to a score. In one particular instance referred throughout the remainder of the specification, the function $h(\cdot)$ is the summation across the distance metrics of the cassette.

The cassette design module 324 may iterate through one or more candidate cassette sequences, determine the junction epitope presentation score for the candidate cassettes, and identify an optimal cassette sequence associated with a junction epitope presentation score below the threshold. In one particular embodiment referred throughout the remainder of the specification, the distance metric $d(\cdot)$ for a given junction may be given by the sum of the presentation likelihoods or the expected number presented junction epitopes as determined by the presentation models described in sections VII and VIII of the specification. However, it is appreciated that in other embodiments, the distance metric may be derived from other factors alone or in combination with the models like the one exemplified above, where these other factors may include deriving the distance metric from any one or more of (alone or in combination): HLA binding affinity or stability measurements or predictions for HLA class I or HLA class II, and a presentation or immunogenicity model trained on HLA mass spectrometry or T-cell epitope data, for HLA class I or HLA class II. In one embodiment, the distance metric may combine information about HLA class I and HLA class II presentation. For example, the distance metric could be the number of junction epitopes predicted to bind any of the patient's HLA class I or HLA class II alleles with binding affinity below a threshold. In another example, the distance metric could be the expected number of epitopes predicted to be presented by any of the patient's HLA class I or HLA class II alleles.

The cassette design module 324 may further check the one or more candidate cassette sequences to identify if any of the junction epitopes in the candidate cassette sequences are self-epitopes for a given patient for whom the vaccine is being designed. To accomplish this, the cassette design module 324 checks the junction epitopes against a known database such as BLAST. In one embodiment, the cassette design module may be configured to design cassettes that avoid junction self-epitopes by setting the distance metric $d_{(ti,tj)}$ to a very large value (e.g., 100) for pairs of epitopes $t_i, t_j$ where contatenating epitope $t_i$ to the N-terminus of epitope $t_j$ results in the formation of a junction self-epitope.

Returning to the example in FIG. 13, the cassette design module 324 determines (for example) a distance metric $d_{(t1,t2)} = d_{(1,2)} = 0.39$ for the single junction $(t_1, t_2)$ in cassette $\mathbf{C_1}$ given by the summation of presentation likelihoods of all possible junction epitopes $e_n^{(t1,t2)} = e_n^{(1,2)}$ having lengths, for example, from 8 to 15 amino acids for MHC class I, or 9-30 amino acids for MHC class II. Since no other junctions are present in cassette $\mathbf{C_1}$, the junction epitope presentation score, which is a summation across the distance metrics for cassette $\mathbf{C_1}$, is also given by 0.39. The cassette design module 324 also determines a distance metric $d_{(t1,t2)} = d_{(2,1)} = 0.068$ for the single junction in cassette $\mathbf{C_2}$ given by the summation of presentation likelihoods of all possible junction epitopes $e_n^{(t1,t2)} = e_n^{(2,1)}$ having lengths from 8 to 15 for MHC class I, or 9-30 amino acids for MHC class II. In this example, the junction epitope presentation score for cassette $\mathbf{C_2}$ is also given by the distance metric of the single junction 0.068. The cassette design module 324 outputs the cassette sequence of $\mathbf{C_2}$ as the optimal cassette since the junction epitope presentation score is lower than the cassette sequence of $\mathbf{C_1}$.

In some cases, the cassette design module 324 can perform a brute force approach and iterates through all or most possible candidate cassette sequences to select the sequence with the smallest junction epitope presentation score. However, the number of such candidate cassettes can be prohibitively large as the capacity of the vaccine v increases. For example, for a vaccine capacity of $v=20$ epitopes, the cassette design module 324 has to iterate through $\sim 10^{18}$ possible candidate cassettes to determine the cassette with the lowest junction epitope presentation score. This determination may be computationally burdensome (in terms of computational processing resources required), and sometimes intractable, for the cassette design module 324 to complete within a reasonable amount of time to generate the vaccine for the patient. Moreover, accounting for the possible junction epitopes for each candidate cassette can be even more burdensome. Thus, the cassette design module 324 may select a cassette sequence based on ways of iterating through a number of candidate cassette sequences that are significantly smaller than the number of candidate cassette sequences for the brute force approach.

In one embodiment, the cassette design module 324 generates a subset of randomly or at least pseudo-randomly generated candidate cassettes, and selects the candidate cassette associated with a junction epitope presentation score below a predetermined threshold as the cassette sequence. Additionally, the cassette design module 324 may select the candidate cassette from the subset with the lowest junction epitope presentation score as the cassette sequence. For example, the cassette design module 324 may generate a subset of $\sim$1 million candidate cassettes for a set of $v=20$ selected epitopes, and select the candidate cassette with the smallest junction epitope presentation score. Although generating a subset of random cassette sequences and selecting a cassette sequence with a low junction epitope presentation score out of the subset may be sub-optimal relative to the brute force approach, it requires significantly less computational resources thereby making its implementation technically feasible. Further, performing the brute force method as opposed to this more efficient technique may only result in a minor or even negligible improvement in junction epitope presentation score, thus making it not worthwhile from a resource allocation perspective.

In another embodiment, the cassette design module 324 determines an improved cassette configuration by formulating the epitope sequence for the cassette as an asymmetric traveling salesman problem (TSP). Given a list of nodes and distances between each pair of nodes, the TSP determines a sequence of nodes associated with the shortest total distance to visit each node exactly once and return to the original node. For example, given cities A, B, and C with known distances between each other, the solution of the TSP generates a closed sequence of cities, for which the total distance traveled to visit each city exactly once is the smallest among possible routes. The asymmetric version of the TSP determines the optimal sequence of nodes when the distance between a pair of nodes are asymmetric. For example, the "distance" for traveling from node A to node B may be different from the "distance" for traveling from node B to node A.

The cassette design module 324 determines an improved cassette sequence by solving an asymmetric TSP, in which each node corresponds to a therapeutic epitope $\boldsymbol{p'^k}$. The distance from a node corresponding to epitope $\boldsymbol{p'^k}$ to another node corresponding to epitope $\boldsymbol{p'^m}$ is given by the junction epitope distance metric $d_{(k,m)}$, while the distance from the node corresponding to the epitope $\boldsymbol{p'^m}$ to the node corresponding to epitope $\boldsymbol{p'^k}$ is given by the distance metric $d_{(m,k)}$ that may be different from the distance metric $d_{(k,m)}$. By solving for an improved optimal cassette using an asymmetric TSP, the cassette design module 324 can find a cassette sequence that results in a reduced presentation score across the junctions between epitopes of the cassette. The solution of the asymmetric TSP indicates a sequence of therapeutic epitopes that correspond to the order in which the epitopes should be concatenated in a cassette to minimize the junction epitope presentation score across the junctions of the cassette. Specifically, given the set of therapeutic epitopes $k=1, 2, ..., v$, the cassette design module 324 determines the distance metrics $d_{(k,m)}$, $k, m = 1, 2, ..., v$ for each possible ordered pair of therapeutic epitopes in the cassette. In other words, for a given pair $k, m$ of epitopes, both the distance metric $d_{(k,m)}$ for concatenating therapeutic epitope $\boldsymbol{p'^m}$ after epitope $\boldsymbol{p'^k}$ and the distance metric $d_{(m,k)}$ for concatenating therapeutic epitope $\boldsymbol{p'^k}$ after epitope $\boldsymbol{p'^m}$ is determined, since these distance metrics may be different from each other.

In one embodiment, the cassette design module 324 solves the asymmetric TSP through an integer linear programming problem. Specifically, the cassette design module 324 generates a $(v+1)$ x $(v+1)$ path matrix **P** given by the following:

$$\mathbf{P} = \begin{bmatrix} 0 & \mathbf{0}^{1 \times v} \\ \mathbf{0}^{v \times 1} & \mathbf{D} \end{bmatrix} \tag{26}.$$

The v x v matrix **D** is an asymmetric distance matrix, where each element $D(k, m)$, $k=1, 2, ..., v$; $m=1, 2, ..., v$ corresponds to the distance metric for a junction from epitope $p'^k$ to epitope $p'^m$. Rows $k = 2, ..., v$ of **P** correspond to nodes of the original epitopes, while row 1 and column 1 corresponds to a "ghost node" that is at zero distance from all other nodes. The addition of the "ghost node" to the matrix encodes the notion that the vaccine cassette is linear rather than circular, so there is no junction between the first and last epitopes. In other words, the sequence is not circular, and the first epitope is not assumed to be concatenated after the last epitope in the sequence. Let $x_{km}$ denote a binary variable whose value is 1 if there is a directed path (i.e., an epitope-epitope junction in the cassette) where epitope $p'^k$ is concatenated to the N-terminus of epitope $p'^m$ and 0 otherwise. In addition, let $E$ denote the set of all $v$ therapeutic vaccine epitopes, and let $S \subset E$ denote a subset of epitopes. For any such subset $S$, let out($S$) denote the number of epitope-epitope junctions $x_{km} = 1$ where $k$ is an epitope in $S$ and $m$ is an epitope in $E\backslash S$. Given a known path matrix **P**, the cassette design module 324 finds a path matrix **X** that solves the following integer linear programming problem:

$$\min_x \sum_{k=1}^{v+1} \sum_{k \ne m, m=1}^{v+1} \mathrm{P}_{km} \cdot x_{km} \tag{27}$$

in which $P_{km}$ denotes element $P(k,m)$ of the path matrix **P,** subject to the following constraints:

$$\sum_{k=1}^{v+1} x_{km} = 1, \quad m = 1, 2, ..., v+1$$

$$\sum_{m=1}^{v+1} x_{km} = 1, \quad k = 1, 2, ..., v+1$$

$$x_{kk} = 0, \quad k = 1, 2, ..., v+1$$

$$\mathrm{out}(S) \ge 1, \quad S \subset E, 2 \le |S| \le |V|/2$$

The first two constraints guarantee that each epitope appears exactly once in the cassette. The last constraint ensures that the cassette is connected. In other words, the cassette encoded by $x$ is a connected linear protein sequence.
The solutions for $x_{km}$, $k,m = 1, 2, ..., v+1$ in the integer linear programming problem of equation (27) indicates the closed sequence of nodes and ghost nodes that can be used to infer one or more sequences of therapeutic epitopes for the cassette that lower the presentation score of junction epitopes. Specifically, a value of $x_{km} = 1$ indicates that a "path" exists from node $k$ to node $m$, or in other words, that therapeutic epitope $p'^m$ should be concatenated after therapeutic epitope $p'^k$ in the improved cassette sequence. A solution of $x_{km} = 0$ indicates that no such path exists, or in other words, that therapeutic epitope $p'^m$ should not be concatenated after therapeutic epitope $p'^k$ in the improved cassette sequence. Collectively, the values of $x_{km}$ in the integer programming problem of equation (27) represent a sequence of nodes and the ghost node, in which the path enters and exists each node exactly once. For example, the values of $x_{ghost,1}=1$, $x_{13}=1$, $x_{32}=1$, and $x_{2,ghost}=1$ (0 otherwise) may indicate a sequence $ghost \to 1 \to 3 \to 2 \to ghost$ of nodes and ghost nodes.
Once the sequence has been solved for, the ghost nodes are deleted from the sequence to generate a refined sequence with only the original nodes corresponding to therapeutic epitopes in the cassette. The refined sequence indicates the order in which selected epitopes should be concatenated in the cassette to improve the presentation score.
For example, continuing from the example in the previous paragraph, the ghost node may be deleted to generate a refined sequence $1 \to 3 \to 2$. The refined sequence indicates one possible way to concatenate epitopes in the cassette, namely $p^1 \to p^3 \to p^2$.
In one embodiment, when the therapeutic epitopes $p'^k$ are variable-length epitopes, the cassette design module 324 determines candidate distance metrics corresponding to different lengths of the therapeutic epitopes $p'^k$ and $p'^m$, and identifies the distance metric $d_{(k,m)}$ as the smallest candidate distance metric. For example, epitopes $p'^k=[n^k\ p^k\ c^k]$ and $p'^m=[n^m p^m\ c^m]$ may each include a corresponding N- and C-terminal flanking sequence that can vary from (in one embodiment) 2-5 amino acids. Thus, the junction between epitopes $p'^k$ and $p'^m$ is associated with 16 different sets of junction epitopes based on the 4 possible length values of $n^k$ and the 4 possible length values of $c^m$ that are placed in the junction. The cassette design module 324 may determine candidate distance metrics for each set of junction epitopes, and determine the distance metric $d_{(k,m)}$ as the smallest value. The cassette design module 324 can then construct the path matrix **P** and solve for the integer linear programming problem in equation (27) to determine the cassette sequence.

Compared to the random sampling approach, solving for the cassette sequence using the integer programming problem requires determination of $v$ x ($v$-1) distance metrics each corresponding to a pair of therapeutic epitopes in the vaccine. A cassette sequence determined through this approach can result in a sequence with significantly less presentation of junction epitopes while potentially requiring significantly less computational resources than the random sampling approach, especially when the number of generated candidate cassette sequences is large.

## XI.B.2. Comparison of Junction Epitope Presentation for Cassette Sequences Generated by Random Sampling vs. Asymmetric TSP

Two cassette sequences including $v$=20 therapeutic epitopes were generated by random sampling 1,000,000 permutations (cassette sequence $C_1$), and by solving the integer linear programming problem in equation (27) (cassette sequence $C_2$). The distance metrics, and thus, the presentation score was determined based on the presentation model described in equation (14), in which $f$ is the sigmoid function, $x_h^i$ is the sequence of peptide $p^i$, $g_h(\cdot)$ is the neural network function, **w** includes the flanking sequence, the log transcripts per kilobase million (TPM) of peptide $p^i$, the antigenicity of the protein of peptide $p^i$, and the sample ID of origin of peptide $p^i$, and $g_w(\cdot)$ of the flanking sequence and the log TPM are neural network functions, respectively. Each of the neural network functions for $g_h(\cdot)$ included one output node of a one-hidden-layer multilayer perceptron (MLP) with input dimensions 231 (11 residues x 21 characters per residue, including pad characters), width 256, rectified linear unit (ReLU) activations in the hidden layer, linear activations in the output layer, and one output node per HLA allele in the training data set. The neural network function for the flanking sequence was a one hidden-layer MLP with input dimension 210 (5 residues of N-terminal flanking sequence + 5 residues of C-terminal flanking sequence x 21 characters per residue, including the pad characters), width 32, ReLU activations in the hidden layer and linear activation in the output layer. The neural network function for the RNA log TPM was a one hidden layer MLP with input dimension 1, width 16, ReLU activations in the hidden layer and linear activation in the output layer. The presentation models were constructed for HLA alleles HLA-A*02:04, HLA-A*02:07, HLA-B*40:01, HLA-B*40:02, HLA-C*16:02, and HLA-C*16:04. The presentation score indicating the expected number of presented junction epitopes of the two cassette sequences were compared. Results showed that the presentation score for the cassette sequence generated by solving the equation of (27) was associated with a ~4 fold improvement over the presentation score for the cassette sequence generated by random sampling.

Specifically, the $v$=20 epitopes were given by:

$p'^1$ = YNYSYWISIFAHTMWYNIWHVQWNK (SEQ ID NO: 86)

$p'^2$ = IEALPYVFLQDQFELRLLKGEQGNN (SEQ ID NO: 87)

$p'^3$ = DSEETNTNYLHYCHFHWTWAQQTTV (SEQ ID NO: 88)

$p'^4$ = GMLSQYELKDCSLGFSWNDPAKYLR (SEQ ID NO: 89)

$p'^5$ =VRIDKFLMYVWYSAPFSAYPLYQDA (SEQ ID NO: 90)

$p'^6$ = CVHIYNNYPRMLGIPFSVMVSGFAM (SEQ ID NO: 91)

$p'^7$ = FTFKGNIWIEMAGQFERTWNYPLSL (SEQ ID NO: 92)

$p'^8$ = ANDDTPDFRKCYIEDHSFRFSQTMN (SEQ ID NO: 93)

$p'^9$=AAQYIACMVNRQMTIVYHLTRWGMK (SEQ ID NO: 94)

$p'^{10}$ = KYLKEFTQLLTFVDCYMWITFCGPD (SEQ ID NO: 95)

$p'^{11}$= AMHYRTDIHGYWIEYRQVDNQMWNT (SEQ ID NO: 96)

$p'^{12}$ = THVNEHQLEAVYRFHQVHCRFPYEN (SEQ ID NO: 97)

$p'^{13}$ = QTFSECLFFHCLKVWNNVKYAKSLK (SEQ ID NO: 98)

$p'^{14}$ = SFSSWHYKESHIALLMSPKKNHNNT (SEQ ID NO: 99)

$p'^{15}$ = ILDGIMSRWEKVCTRQTRYSYCQCA (SEQ ID NO: 100)

$p'^{16}$ = YRAAQMSKWPNKYFDFPEFMAYMPI (SEQ ID NO: 101)

$p'^{17}$= PRPGMPCQHHNTHGLNDRQAFDDFV (SEQ ID NO: 102)

$p'^{28}$ = HNIISDETEVWEQAPHITWVYMWCR (SEQ ID NO: 103)

$p'^{19}$ = AYSWPVVPMKWI PYPALCANHP PGT (SEQ ID NO: 104)

$p'^{20}$= HVMPHVAMNICNWYEFLYRISHIGR (SEQ ID NO: 105) .

In the first example, 1,000,000 different candidate cassette sequences were randomly generated with the 20 therapeutic epitopes. The presentation score was generated for each of the candidate cassette sequences. The candidate cassette sequence identified to have the lowest presentation score was:

$C_1$ = THVNEHQLEAVYRFHQVHCRFPYENAMHYQMWNTYRAAQMSKWPNKYFDFPEFMAYMPICVH
IYNNYPRMLGIPFSVMVSGFAMAYSWPVVPMKWIPYRALCANHPPGTANDDTPDFRKCYIEDHSFRFSQTMNI
EALPYVFLQDQFELRLLKGEQGNNDSEETNTNYLHYCHFHWTWAQQTTVILDGIMSRWEKVCTRQTRYSYCQC
AFTFKGNIWIEMAGQFERTWNYPLSLSFSSWHYKESHIALLMSPKKNHNNTQTFSECLFFHCLKVWNNVKYAK
SLKHVMPHVAMNICNWYEFLYRISHIGRHNIISDETEVWEQAPHITWVYMWCRVRIDKFLMYVWYSAPFSAYP
LYQDAKYLKEFTQLLTFVDCYMWITFCGPDAAQYIACMVNRQMTIVYHLTRWGMKYNYSYWISIFAHTMWYNI
WHVQWNKGMLSQYELKDCSLGFSWNDPAKYLRPRPGMPCQHHNTHGLNDRQAFDDFV (SEQ ID NO: 106
)

with a presentation score of 6.1 expected number of presented junction epitopes. The median presentation score of the 1,000,000 random sequences was 18.3. The experiment shows that the expected number of presented junction epitopes can be significantly reduced by identifying a cassette sequence among randomly sampled cassettes.

In the second example, a cassette sequence $C_2$ was identified by solving the integer linear programming problem in equation (27). Specifically, the distance metric of each potential junction between a pair of therapeutic epitopes was determined. The distance metrics were used to solve for the solution to the integer programming problem. The cassette sequence identified by this approach was:

$C_2$ = IEALPYVFLQDQFELRLLKGEQGNNILDGIMSRWEKVCTRQTRYSYCQCAHVMPHVAMNICN
WYEFLYRISHIGRTHVNEHQLEAVYRFHQVHCRFPYENFTFKGNIWIEMAGQFERTWNYPLSLAMHYQMWNTS
FSSWHYKESHIALLMSPKKNHNNTVRIDKFLMYVWYSAPFSAYPLYQDAQTFSECLFFHCLKVWNNVKYAKSL
KYRAAQMSKWPNKYFDFPEFMAYMPIAYSWPVVPMKWIPYRALCANHPPGTCVHIYNNYPRMLGIPFSVMVSG
FAMHNIISDETEVWEQAPHITWVYMWCRAAQYIACMVNRQMTIVYHLTRWGMKYNYSYWISIFAHTMWYNIWH
VQWNKGMLSQYELKDCSLGFSWNDPAKYLRKYLKEFTQLLTFVDCYMWITFCGPDANDDTPDFRKCYIEDHSF
RFSQTMNDSEETNTNYLHYCHFHWTWAQQTTVPRPGMPCQHHNTHGLNDRQAFDDFV (SEQ ID NO: 107
)

with a presentation score of 1.7. The presentation score of cassette sequence $C_2$ showed a ~4 fold improvement over the presentation score of cassette sequence $C_1$, and a~11 fold improvement over the median presentation score of the 1,000,000 randomly generated candidate cassettes. The run-time for generating cassette $C_1$ was 20 seconds on a single thread of a 2.30 GHz Intel Xeon E5-2650 CPU. The run-time for generating cassette $C_2$ was 1 second on a single thread of the same CPU. Thus in this example, the cassette sequence identified by solving the integer programming problem of equation (27) produces a ~4-fold better solution at 20-fold reduced computational cost.

The results show that the integer programming problem can potentially provide a cassette sequence with a lower number of presented junction epitopes than one identified from random sampling, potentially with less computation resources.

## XI.B.3. Comparison of Junction Epitope Presentation for Cassette Sequence Selection Generated by MHCflurry and the Presentation Model

In this example, cassette sequences including $v=20$ therapeutic epitopes were selecte d based off tumor/normal exome sequencing, tumor transcriptome sequencing and HLA typin g of a lung cancer sample were generated by random sampling 1,000,000 permutations, and b y solving the integer linear programming problem in equation (27). The distance metrics, and thus, the presentation score were determined based on the number of junction epitopes predict ed by MHCflurry, an HLA-peptide binding affinity predictor, to bind the patient's HLAs with affinity below a variety of thresholds (e.g., 50-1000nM, or higher, or lower). In this example, the 20 nonsynoymous somatic mutations chosen as therapeutic epitopes were selected from a mong the 98 somatic mutations identified in the tumor sample by ranking the mutations accor ding to the presentation model in Section XI.B above. However, it is appreciated that in other embodiments, the therapeutic epitopes may be selected based on other criteria; such as those based stability, or combinations of criteria such as presentation score, affinity, and so on. In a ddition, it is appreciated that the criteria used for prioritizing therapuetic epitopes for inclusio n in the vaccine need not be the same as the criteria used for determining the distance metric $D(k, m)$ used in the cassette design module 324.

The patient's HLA class I alleles were HLA-A*01:01, HLA-A*03:01, HLA-B*07:0 2, HLA-B*35:03, HLA-C*07:02, HLA-C*14:02.

Specifically in this example, the $v=20$ therapuetic epitopes were

SSTPYLYYGTSSVSYQFPMVPGGDR (SEQ ID NO: 108)
EMAGKIDLLRDSYIFQLFWREAAEP (SEQ ID NO: 109)
ALKQRTWQALAHKYNSQPSVSLRDF (SEQ ID NO: 110)
VSSHSSQATKDSAVGLKYSASTPVR (SEQ ID NO: 111)
KEAIDAWAPYLPEYIDHVISPGVTS (SEQ ID NO: 112)
SPVITAPPSSPVFDTSDIRKEPMNI (SEQ ID NO: 113)
PAEVAEQYSEKLVYMPHTFFIGDHA (SEQ ID NO: 114)
MADLDKLNIHSIIQRLLEVRGS (SEQ ID NO: 115)
AAAYNEKSGRITLLSLLFQKVFAQI (SEQ ID NO: 116)
KIEEVRDAMENEIRTQLRRQAAAHT (SEQ ID NO: 117)
DRGHYVLCDFGSTTNKFQNPQTEGV (SEQ ID NO: 118)
QVDNRKAEAEEAIKRLSYISQKVSD (SEQ ID NO: 119)
CLSDAGVRKMTAAVRVMKRGLENLT (SEQ ID NO: 120)
LPPRSLPSDPFSQVPASPQSQSSSQ (SEQ ID NO: 121)
ELVLEDLQDGDVKMGGSFRGAFSNS (SEQ ID NO: 122)
VTMDGVREEDLASFSLRKRWESEPH (SEQ ID NO: 123)
IVGVMFFERAFDEGADATYDHINEG (SEQ ID NO: 124)
TVTPTPTPTGTQSPTPTPITTTTTV (SEQ ID NO: 125)
QEEMPPRPCGGHTSSSLPKSHLEPS (SEQ ID NO: 126)
PNIQAVLLPKKTDSHHKAKGK (SEQ ID NO: 127)

Results from this example in the table below compare the number of junction epitopes predicted by MHCflurry to bind the patient's HLAs with affinity below the value in the threshold column (where nM stands for nanoMolar) as found via three example methods. For the first method, the optimal cassette found via the traveling salesman problem (ATSP) formulation described above with 1s run-time. For the second method, the optimal cassette as determined by taking the best cassette found after 1 million random samples. For the third method, the median number of junction epitopes was found in the 1 million random samples.

| Threshold (nM) | ATSP # Binding Junction Epitopes | Random Sampling # Binding Junction Epitopes | Median # Binding Junction Epitopes |
|---|---|---|---|
| 50 | 0 | 0 | 3 |
| 100 | 0 | 0 | 7 |
| 150 | 0 | 1 | 12 |
| 500 | 15 | 26 | 55 |
| 1000 | 68 | 91 | 131 |

The results of this example illustrate that any one of a number of criteria may be used to identify whether or not a given cassette design meets design requirements. Specifically, as demonstrated by prior examples, the selected cassette sequence out of many candidates may be specified by the cassette sequence having a lowest junction epitope presentation score, or at least such a score below an identified threshold. This example represents that another criteria, such as binding affinity, may be used to specify whether or not a given cassette design meets design requirements. For this criteria, a threshold binding affinity (e.g., 50-1000, or greater or lower) may be set specifying that the cassette design sequence should have fewer than some threshold number of junction epitopes above the threshold (e.g., 0), and any one of a number of methods may be used (e.g., methods one through three illustrated in the table) can be used to identify if a given candidate cassette sequence meets those requirements. These example methods further illustrate that depending on the method used, the thresholds may need to be set differently. Other criteria may be envisioned, such as those based stability, or combinations of criteria such as presentation score, affinity, and so on.

In another example, the same cassettes were generated using the same HLA type and 20 therapeutic epitopes from earlier in this section (XI.C), but instead of using distance metrics based off binding affinity prediction, the distance metric for epitopes m, k was the number of peptides spanning the m to k junction predicted to be presented by the patient's HLA class I alleles with probability of presentation above a series of thresholds (between probability of 0.005 and 0.5, or higher, or lower), where the probabilities of presentation were determined by the presentation model in Section XI.B above. This example further illustrates the breadth of criteria that may be considered in identifying whether a given candidate cassette sequence meets design requirements for use in the vaccine.

| Threshold (probability) | ATSP # Junction Epitopes | Random Sampling # Junction Epitopes | Median # Junction Epitopes |
|---|---|---|---|
| 0.005 | 58 | 79 | 118 |
| 0.01 | 39 | 59 | 93 |
| 0.05 | 7 | 33 | 47 |
| 0.1 | 5 | 14 | 35 |
| 0.2 | 1 | 8 | 25 |
| 0.5 | 0 | 2 | 14 |

The examples above have identified that the criteria for determining whether a candidate cassette sequence may vary by implementation. Each of these examples has illustrated that the count of the number of junction epitopes falling above or below the criteria may be a count used in determining whether the candidate cassette sequence meets that criteria. For example, if the criteria is number of epitopes meeting or exceeding a threshold binding affinity for HLA, whether the candidate cassette sequence has greater or fewer than that number may determine whether the candidate cassette sequence meets the criteria for use as the selected cassette for the vaccine. Similarly if the criteria is the number of junction epitopes exceeding a threshold presentation likelihood.

However, in other embodiments, calculations other than counting can be performed to determine whether a candidate cassette sequence meets the design criteria. For example, rather than the count of epitopes exceeding / falling below some threshold, it may instead be determined what proportion of junction epitopes exceed or fall below the threshold, for example whether the top X% of junction epitopes have a presentation likelihood above some threshold Y, or whether X% percent of junction epitopes have an HLA binding affinity less than or greater than Z nM. These are merely examples, generally the criteria may be based on any attribute of either individual junction epitopes, or statistics derived from aggregations of some or all of the junction epitopes. Here, X can generally be any number between 0 and 100% (e.g., 75% or less) and Y can be any value between 0 and 1, and Z can be any number suitable to the criteria in question. These values may be determined empirically, and depend on the models and criteria used, as well as the quality of the training data used.

As such, in certain aspects, junction epitopes with high probabilities of presentation can be removed; junction epitopes with low probabilities of presentation can be retained; junction epitopes that bind tightly, i.e., junction epitopes with binding affinity below 1000nM or 500nM or some other threshold can be removed; and/or junction epitopes that bind weakly, i.e., junction epitopes with binding affinity above 1000nM or 500nM or some other threshold can be retained.

Although the examples above have identified candidate sequences using an implementation of the presentation model described above, these principles apply equally to an implementation where the epitopes for arrangement in the cassette sequences are identified based on other types of models as well, such as those based on affinity, stability, and so on.

## XII. Example 7: Experimentation Results Showing Example Presentation Model Performance

The validity of the various presentation models described above were tested on test data T that were subsets of training data 170 that were not used to train the presentation models or a separate dataset from the training data 170 that have similar variables and data structures as the training data 170.

A relevant metric indicative of the performance of a presentation models is:

$$\text{Positive Predictive Value (PPV)} = P(y_{i \in T} = 1 \mid u_{i \in T} \geq t) = \frac{\sum_{i \in T} \mathbb{1}(y_i = 1, \ u_i \geq t)}{\sum_{i \in T} \mathbb{1}(u_i \geq t)}$$

that indicates the ratio of the number of peptide instances that were correctly predicted to be presented on associated HLA alleles to the number of peptide instances that were predicted to be presented on the HLA alleles. In one implementation, a peptide $p^i$ in the test data $T$ was predicted to be presented on one or more associated HLA alleles if the corresponding likelihood estimate $u_i$ is greater or equal to a given threshold value $t$. Another relevant metric indicative of the performance of presentation models is:

$$\text{Recall} = P(u_{i \in T} \geq t \,|\, y_{i \in T} = 1) = \frac{\sum_{i \in T} \mathbb{1}(y_i = 1,\, u_i \geq t)}{\sum_{i \in T} \mathbb{1}(y_i = 1)}$$

that indicates the ratio of the number of peptide instances that were correctly predicted to be presented on associated HLA alleles to the number of peptide instances that were known to be presented on the HLA alleles. Another relevant metric indicative of the performance of presentation models is the area-under-curve (AUC) of the receiver operating characteristic (ROC). The ROC plots the recall against the false positive rate (FPR), which is given by:

$$\text{FPR} = P(u_{i \in T} \geq t \,|\, y_{i \in T} = 0) = \frac{\sum_{i \in T} \mathbb{1}(y_i = 0,\, u_i \geq t)}{\sum_{i \in T} \mathbb{1}(y_i = 0)}.$$

## XII.A. Comparison of Presentation Model Performance on Mass Spectrometry Data Against State-of-the-Art Model

FIG. 13A compares performance results of an example presentation model, as presented herein, and state-of-the-art models for predicting peptide presentation on multiple-allele mass spectrometry data. Results showed that the example presentation model performed significantly better at predicting peptide presentation than state-of-the-art models based on affinity and stability predictions.

Specifically, the example presentation model shown in FIG. 13A as "MS" was the maximum of per-alleles presentation model shown in equation (12), using the affine dependency function $g_h(\cdot)$ and the expit function $f(\cdot)$. The example presentation model was trained based on a subset of the single-allele HLA-A*02:01 mass spectrometry data from the IEDB data set (data set "D1") (data can be found at http://www.iedb.org/doc/mhc_ligand_full.zip) and a subset of the single-allele HLA-B*07:02 mass spectrometry from the IEDB data set (data set "D2") (data can be found at http://www.iedb.org/doc/mhc_ligand_full.zip). All peptides from source protein that contain presented peptides in the test set were eliminated from the training data such that the example presentation model could not simply memorize the sequences of presented antigens.

The model shown in FIG. 13A as "Affinity" was a model similar to the current state-of-the-art model that predicts peptide presentation based on affinity predictions NETMHCpan. Implementation of NETMHCpan is provided in detail at http://www.cbs.dtu.dk/services/NetMHCpan/. The model shown in FIG. 13A as "Stability" was a model similar to the current state-of-the-art model that predicts peptide presentation based on stability predictions NETMHCstab. Implementation of NETMHCstab is provided in detail at http://www.cbs.dtu.dk/services/NetMHCstab-1.0/. The test data that is a subset of the multiple-allele JY cell line HLA-A*02:01 and HLA-B*07:02 mass spectrometry data from the Bassani-Stemberg data set (data set "D3") (data can be found at www.ebi.ac.uk/pride/archive/projects/PXD000394). The error bars (as indicated in solid lines) show 95% confidence intervals.

As shown in the results of FIG. 13A, the example presentation model trained on mass spectrometry data had a significantly higher PPV value at 10% recall rate relative to the state-of-the-art models that predict peptide presentation based on MHC binding affinity predictions or MHC binding stability predictions. Specifically, the example presentation model had approximately 14% higher PPV than the model based on affinity predictions, and had approximately 12% higher PPV than the model based on stability predictions.

These results demonstrate that the example presentation model had significantly better performance than the state-of-the-art models that predict peptide presentation based on MHC binding affinity or MHC binding stability predictions even though the example presentation model was not trained based on protein sequences that contained presented peptides.

## XII.B. Comparison of Presentation Model Performance on T-cell Epitope Data Against State-of-the-Art Models

FIG. 13B compares performance results of another example presentation model, as presented herein, and state-of-the-art models for predicting peptide presentation on T-cell epitope data. T-cell epitope data contains peptide sequences that were presented by MHC alleles on the cell surface, and recognized by T-cells. Results showed that even though the example presentation model is trained based on mass spectrometry data, the example presentation model performed significantly better at predicting T-cell epitopes than state-of-the-art models based on affinity and stability predictions. In other words, the results of FIG. 13B indicated that not only did the example presentation model perform better than state-of-the-art models at predicting peptide presentation on mass spectrometry test data, but the example presentation model also performed significantly better than state-of-the-art models at predicting epitopes that were actually recognized by T-cells. This is an indication that the variety of presentation models as presented herein can provide improved identification of antigens that are likely to induce immunogenic responses in the immune system.

Specifically, the example presentation model shown in FIG. 13B as "MS" was the per-allele presentation model shown in equation (2), using the affine transformation function $g_h(\cdot)$ and the expit function $f(\cdot)$ that was trained based on a subset of data set D1. All peptides from source protein that contain presented peptides in the test set were eliminated from the training data such that the presentation model could not simply memorize the sequences of presented antigens.

Each of the models were applied to the test data that is a subset of mass spectrometry data on HLA-A*02:01 T-cell epitope data (data set "D4") (data can be found at www.iedb.org/doc/tcell full v3.zip). The model shown in FIG. 13B as "Affiinity" was a model similar to the current state-of-the-art model that predicts peptide presentation based on affinity predictions NETMHCpan, and the model shown in FIG. 13B as "Stability" was a model similar to the current state-of-the-art model that predicts peptide presentation based on stability predictions NETMHCstab. The error bars (as indicated in solid lines) show 95% confidence intervals.

As shown in the results of FIG. 13A, the per-allele presentation model trained on mass spectrometry data had a significantly higher PPV value at 10% recall rate than the state-of-the-art models that predict peptide presentation based on MHC binding affinity or MHC binding stability predictions even though the presentation model was not trained based on protein sequences that contained presented peptides. Specifically, the per-allele presentation model had approximately 9% higher PPV than the model based on affinity predictions, and had approximately 8% higher PPV than the model based on stability predictions.

These results demonstrated that the example presentation model trained on mass spectrometry data performed significantly better than state-of-the-art models on predicting epitopes that were recognized by T-cells.

## XII.C. Comparison of Different Presentation Model Performances on Mass Spectrometry Data

FIG. 13C compares performance results for an example function-of-sums model (equation (13)), an example sum-of-functions model (equation (19)), and an example second order model (equation (23)) for predicting peptide presentation on multiple-allele mass spectrometry data. Results showed that the sum-of-functions model and second order model performed better than the function-of-sums model. This is because the function-of-sums model implies that alleles in a multiple-allele setting can interfere with each other for peptide presentation, when in reality, the presentation of peptides are effectively independent.

Specifically, the example presentation model labeled as "sigmoid-of-sums" in FIG. 13C was the function-of-sums model using a network dependency function $g_h(\cdot)$, the identity function $f(\cdot)$, and the expit function $r(\cdot)$. The example model labeled as "sum-of-sigmoids" was the sum-of-functions model in equation (19) with a network dependency function $g_h(\cdot)$, the expit function $f(\cdot)$, and the identity function $r(\cdot)$. The example model labeled as "hyperbolic tangent" was the sum-of-functions model in equation (19) with a network dependency function $g_h(\cdot)$, the expit function $f(\cdot)$, and the hyperbolic tangent function $r(\cdot)$. The example model labeled as "second order" was the second order model in equation (23) using an implicit per-allele presentation likelihood form shown in equation (18) with a network dependency function $g_h(\cdot)$ and the expit function $f(\cdot)$. Each model was trained based on a subset of data set D1, D2, and D3. The example presentation models were applied to a test data that is a random subset of data set D3 that did not overlap with the training data.

As shown in FIG. 13C, the first column refers to the AUC of the ROC when each presentation model was applied to the test set, the second column refers to the value of the negative log likelihood loss, and the third column refers to the PPV at 10% recall rate. As shown in FIG. 13C, the performance of presentation models "sum-of-sigmoids," "hyperbolic tangent," and "second order" were approximately tied at approximately 15-16% PPV at 10% recall, while the performance of the model "sigmoid-of-sums" was slightly lower at approximately 11%.

As discussed previously in section X.C.4., the results showed that the presentation models "sum-of-sigmoids," "hyperbolic tangent," and "second order" have high values of PPV compared to the "sigmoid-of-sums" model because the models correctly account for how peptides are presented independently by each MHC allele in a multiple-allele setting.

## XII.D. Comparison of Presentation Model Performance With and Without Training on Single-Allele Mass Spectrometry Data

FIG. 13D compares performance results for two example presentation models that are trained with and without single-allele mass spectrometry data on predicting peptide presentation for multiple-allele mass spectrometry data. The results indicated that example presentation models that are trained without single-allele data achieve comparable performance to that of example presentation models trained with single-allele data.

The example model "with A2/B7 single-allele data" was the "sum-of-sigmoids" presentation model in equation (19) with a network dependency function $g_h(\cdot)$, the expit function $f(\cdot)$, and the identity function $r(\cdot)$. The model was trained based on a subset of data set D3 and single-allele mass spectrometry data for a variety of MHC alleles from the IEDB database (data can be found at: http://www.iedb.org/doc/mhc_ligand_full.zip). The example model "without A2/B7 single-allele data" was the same model, but trained based on a subset of the multiple-allele D3 data set without single-allele mass spectrometry data for alleles HLA-A*02:01 and HLA-B*07:02, but with single-allele mass spectrometry data for other alleles. Within the multiple-allele training data, cell line HCC1937 expressed HLA-B*07:02 but not HLA-A*02:01, and cell line HCT116 expressed HLA-A*02:01 but not HLA-B*07:02. The example presentation models were applied to a test data that was a random subset of data set D3 and did not overlap with the training data.

The column "Correlation" refers to the correlation between the actual labels that indicate whether the peptide was presented on the corresponding allele in the test data, and the label for prediction. As shown in FIG. 13D, the predictions based on the implicit per-allele presentation likelihoods for MHC allele HLA-A*02:01 performed significantly better on single-allele test data for MHC allele HLA-A*02:01 rather than for MHC allele HLA-B*07:02. Similar results are shown for MHC allele HLA-B*07:02.

These results indicate that the implicit per-allele presentation likelihoods of the presentation model can correctly predict and distinguish binding motifs to individual MHC alleles, even though direct association between the peptides and each individual MHC allele was not known in the training data.

### XII.E. Comparison of Per-Allele Prediction Performance without Training on Single-Allele Mass Spectrometry Data

FIG. 13E shows performance for the "without A2/B7 single-allele data" and "with A2/B7 single-allele data" example models shown in FIG. 13D on single-allele mass spectrometry data for alleles HLA-A*02:01 and HLA-B*07:02 that were held out in the analysis shown in FIG. 13D. Results indicate that even through the example presentation model is trained without single-allele mass spectrometry data for these two alleles, the model is able to learn binding motifs for each MHC allele.

As shown in FIG. 13E, "A2 model predicting B7" indicates the performance of the model when peptide presentation is predicted for single-allele HLA-B*07:02 data based on the implicit per-allele presentation likelihood estimate for MHC allele HLA-A*02:01. Similarly, "A2 model predicting A2" indicates the performance of the model when peptide presentation is predicted for single-allele HLA-A*02:01 based on the implicit per-allele presentation likelihood estimate for MHC allele HLA-A*02:01. "B7 model predicting B7" indicates the performance of the model when peptide presentation is predicted for single-allele HLA-B*07:02 data based on the implicit per-allele presentation likelihood estimate for MHC allele HLA-B*07:02. "B7 model predicting A2" indicates the performance of the model when peptide presentation is predicted for single-allele HLA-A*02:01 based on the implicit per-allele presentation likelihood estimate for MHC allele HLA-B*07:02.

As shown in FIG. 13E, the predictive capacity of implicit per-allele likelihoods for an HLA allele is significantly higher for the intended allele, and significantly lower for the other HLA allele. Similarly to the results shown in FIG. 13D, the example presentation models correctly learned to differentiate peptide presentation of individual alleles HLA-A*02:01 and HLA-B*07:02, even though direct association between peptide presentation and these alleles were not present in the multiple-allele training data.

### XII.F. Frequently Ocurring Anchor Residues in Per-Allele Predictions Match Known Canonical Anchor Motifs

FIG. 13F shows the common anchor residues at positions 2 and 9 among nonamers predicted by the "without A2/B7 single-allele data" example model shown in FIG. 13D. The peptides were predicted to be presented if the estimated likelihood was above 5%. Results show that most common anchor residues in the peptides identified for presentation on the MHC alleles HLA-A*02:01 and HLA-B*07:02 matched previously known anchor motifs for these MHC alleles. This indicates that the example presentation models correctly learned peptide binding based on particular positions of amino acids of the peptide sequences, as expected.

As shown in FIG. 13F, amino acids L/M at position 2 and amino acids V/L at position 9 were known to be canonical anchor residue motifs (as shown in Table 4 of https://link.springer.com/article/10.1186/1745-7580-4-2) for HLA-A*02:01, and amino acid P at position 2 and amino acids L/V at position 9 were known to be canonical anchor residue motifs for HLA-B*07:02. The most common anchor residue motifs at positions 2 and 9 for peptides identified the model matched the known canonical anchor residue motifs for both HLA alleles.

### XII.G. Comparison of Presentation Model Performances With and Withtout Allele Noninteracting Variables

FIG. 13G compares performance results between an example presentation model that incorporated C- and N-terminal flanking sequences as allele-interacting variables, and an example presentation model that incorporated C- and N-terminal flanking sequences as allele-noninteracting variables. Results showed that incorporating C- and N-terminal flanking sequences as allele noninteracting variables significantly improved model performance. More specifically, it is valuable to identify appropriate features for peptide presentation that are common across different MHC alleles, and model them such that statistical strength for these allele-noninteracting variables are shared across MHC alleles to improve presentation model performance.

The example "allele-interacting" model was the sum-of-functions model using the form of implicit per-allele presentation likelihoods in equation (22) that incorporated C- and N-terminal flanking sequences as allele-interacting variables, with a network dependency function $g_h(\cdot)$ and the expit function $f(\cdot)$. The example "allele-noninteracting" model was the sum-of-functions model shown in equation (21) that incorporated C- and N-terminal flanking sequences as allele-noninteracting variables, with a network dependency function $g_h(\cdot)$ and the expit function $f(\cdot)$. The allele-noninteracting variables were modeled through a separate network dependency function $g_w(\cdot)$. Both models were trained on a subset of data set D3 and single-allele mass spectrometry data for a variety of MHC alleles from the IEDB database (data can be found at: http://www.iedb.org/doc/mhc_ligand_full.zip). Each of the presentation models was applied to a test data set that is a random subset of data set D3 that did not overlap with the training data.

As shown in FIG. 13G, incorporating C- and N-terminal flanking sequences in the example presentation model as allele-noninteracting variables achieved an approximately 3% improvement in PPV value relative to modeling them as allele-interacting variables. This is because, in general, the "allele-noninteracting" example presentation model was able to share statistical strength of allele-noninteracting variables across MHC alleles by modeling the effect with a separate network dependency function with very little addition in computing power.

## XII.H. Dependency Between Presented Peptides and mRNA Quantification

FIG. 13H illustrates the dependency between fraction of presented peptides for genes based on mRNA quantification for mass spectrometry data on tumor cells. Results show that there is a strong dependency between mRNA expression and peptide presentation.

Specifically, the horizontal axis in FIG.13G indicates mRNA expression in terms of transcripts per million (TPM) quartiles. The vertical axis in FIG. 13G indicates fraction of presented epitopes from genes in corresponding mRNA expression quartiles. Each solid line is a plot relating the two measurements from a tumor sample that is associated with corresponding mass spectrometry data and mRNA expression measurements. As shown in FIG. 13G, there is a strong positive correlation between mRNA expression, and the fraction of peptides in the corresponding gene. Specifically, peptides from genes in the top quartile of RNA expression are more than 20 times likely to be presented than the bottom quartile. Moreover, essentially 0 peptides are presented from genes that are not detected through RNA.

The results indicate that the performance of the presentation model can be greatly improved by incorporating mRNA quantification measurements, as these measurements are strongly predictive of peptide presentation.

## XII.I. Comparison of Presentation Model Performance With Incorporation of RNA Quantification Data

FIG. 13I shows performance of two example presentation models, one of which is trained based on mass spectrometry tumor cell data, another of which incorporates mRNA quantification data and mass spectrometry tumor cell data. As expected from FIG. 13H, results indicated that there is a significant improvement in performance by incorporating mRNA quantification measurements in the example presentation model, since the mRNA expression is a strong indicator of peptide presentation.

"MHCflurry + RNA filter" was a model similar to the current state-of-the-art model that predicts peptide presentation based on affinity predictions. It was implemented using MHCflurry along with a standard gene expression filter that removed all peptides from proteins with mRNA quantification measurements that were less than 3.2 FPKM. Implementation of MHCflurry is provided in detail at https://github.com/hammerlab/mhcflurry/, and at http://biorxiv.org/content/early/2016/05/22/054775. The "Example Model, no RNA" model was the "sum-of-sigmoids" example presentation model shown in equation (21) with the network dependency function $g_h(\cdot)$, the network dependency function $g_w(\cdot)$, and the expit function $f(\cdot)$. The "Example Model, no RNA" model incorporated C-terminal flanking sequences as allele-noninteracting variables through a network dependency function $g_w(\cdot)$.

The "Example Model, with RNA" model was the "sum-of-sigmoids" presentation model shown in equation (19) with network dependency function $g_h(\cdot)$, the network dependency function $g_w(\cdot)$ in equation (10) incorporating mRNA quantification data through a log function, and the expit function $f(\cdot)$. The "Example Model, with RNA" model incorporated C-terminal flanking sequences as allele-noninteracting variables through the network dependency functions $g_w(\cdot)$ and incorporated mRNA quantification measurements through the log function.

Each model was trained on a combination of the single-allele mass spectrometry data from the IEDB data set, 7 cell lines from the multiple-allele mass spectrometry data from the Bassani-Sternberg data set, and 20 mass spectrometry tumor samples. Each model was applied to a test set including 5,000 held-out proteins from 7 tumor samples that constituted 9,830 presented peptides from a total of 52,156,840 peptides.

As shown in the first two bar graphs of FIG. 13I, the "Example Model, no RNA" model has a PPV value at 20% Recall of 21%, while that of the state-of-the-art model is approximately 3%, This indicates an initial performance improvement of 18% in PPV value, even without the incorporation of mRNA quantification measurements. As shown in the third bar graph of FIG. 13I, the "Example Model, with RNA" model that incorporates mRNA quantification data into the presentation model shows a PPV value of approximately 30%, which is almost a 10% increase in performance compared to the example presentation model without mRNA quantification measurements.

Thus, results indicate that as expected from the findings in FIG. 13H, mRNA expression is indeed a strong predictor of peptide prediction, that allows significant improvement in the performance of a presentation model with very little addition of computational complexity.

### XII.J. Example of Parameters Determined for MHC Allele HLA-C*16:04

FIG. 13J compares probability of peptide presentation for different peptide lengths between results generated by the "Example Model, with RNA" presentation model described in reference to FIG. 13I, and predicted results by state-of-the-art models that do not account for peptide length when predicting peptide presentation. Results indicated that the "Example Model, with RNA" example presentation model from FIG. 13I captured variation in likelihoods across peptides of differing lengths.

The horizontal axis denoted samples of peptides with lengths 8, 9, 10, and 11. The vertical axis denoted the probability of peptide presentation conditioned on the lengths of the peptide. The plot "Actual Test Data Probability" showed the proportion of presented peptides according to the length of the peptide in a sample test data set. The presentation likelihood varied with the length of the peptide. For example, as shown in FIG. 13J, a 10mer peptide with canonical HLA-A2 L/V anchor motifs was approximately 3 times less likely to be presented than a 9mer with the same anchor residues. The plot "Models Ignoring Length" indicated predicted measurements if state-of-the-art models that ignore peptide length were to be applied to the same test data set for presentation prediction. These models may be NetMHC versions before version 4.0, NetMHCpan versions before version 3.0, and MHCflurry, that do not take into account variation in peptide presentation according to peptide length. As shown in FIG. 13J, the proportion of presented peptides would be constant across different values of peptide length, indicating that these models would fail to capture variation in peptide presentation according to length. The plot "Gritstone, with RNA" indicated measurements generated from the "Gritstone, with RNA" presentation model. As shown in FIG. 13J, the measurements generated by the "Gritstone, with RNA" model closely followed those shown in "Actual Test Data Probability" and correctly accounted for different degrees of peptide presentation for lengths 8, 9, 10, and 11.

Thus, the results showed that the example presentation models as presented herein generated improved predictions not only for 9mer peptides, but also for peptides of other lengths between 8-15, which account for up to 40% of the presented peptides in HLA class I alleles.

### XII.K. Example of Parameters Determined for MHC Allele HLA-C*16:04

The following shows a set of parameters determined for a variation of the per-allele presentation model (equation (2)) for MHC allele HLA-C*16:04 denoted by $h$:

$$u_k = \text{expit}\big(\text{relu}\big(x_h^k \cdot W_h^1 + b_h^1\big) \cdot W_h^2 + b_h^2\big),$$

where relu$(\cdot)$ is the rectified linear unit (RELU) function, and $W_h^1$, $b_h^1$, $W_h^2$, and $b_h^2$ are the set of parameters $\theta$ determined for the model. The allele interacting variables $x_h^k$ consist of peptide sequences. The dimensions of $W_h^1$ are (231 x 256), the dimensions of $b_h^1$ (1 x 256), the dimensions of $W_h^2$ are (256 x 1), and $b_h^2$ is a scalar. For demonstration purposes, values for $b_h^1$, $b_h^2$, $W_h^1$, and $W_h^2$ are described in detail in PCT publication WO2017106638.

### XIII. Example Computer

FIG. 14 illustrates an example computer 1400 for implementing the entities shown in FIGS. 1 and 3. The computer 1400 includes at least one processor 1402 coupled to a chipset 1404. The chipset 1404 includes a memory controller hub 1420 and an input/output (I/O) controller hub 1422. A memory 1406 and a graphics adapter 1412 are coupled to the memory controller hub 1420, and a display 1418 is coupled to the graphics adapter 1412. A storage device 1408, an input device 1414, and network adapter 1416 are coupled to the I/O controller hub 1422. Other embodiments of the computer 1400 have different architectures.

The storage device 1408 is a non-transitory computer-readable storage medium such as a hard drive, compact disk read-only memory (CD-ROM), DVD, or a solid-state memory device. The memory 1406 holds instructions and data used by the processor 1402. The input interface 1414 is a touch-screen interface, a mouse, track ball, or other type of pointing device, a keyboard, or some combination thereof, and is used to input data into the computer 1400. In some embodiments, the computer 1400 may be configured to receive input (e.g., commands) from the input interface 1414 via gestures from the user. The graphics adapter 1412 displays images and other information on the display 1418. The network adapter 1416 couples the computer 1400 to one or more computer networks.

The computer 1400 is adapted to execute computer program modules for providing functionality described herein. As used herein, the term "module" refers to computer program logic used to provide the specified functionality. Thus, a module can be implemented in hardware, firmware, and/or software. In one embodiment, program modules are stored on the storage device 1408, loaded into the memory 1406, and executed by the processor 1402.

The types of computers 1400 used by the entities of FIG. 1 can vary depending upon the embodiment and the processing power required by the entity. For example, the presentation identification system 160 can run in a single computer 1400 or multiple computers 1400 communicating with each other through a network such as in a server farm. The computers 1400 can lack some of the components described above, such as graphics adapters 1412, and displays 1418.

## XIV. Neoantigen Delivery Vector Example

The invention relates to chimpanzee adenovirus vectors comprising a plurality of neoantigen-encoding nucleic acid sequences, as defined in the claims. The examples are offered for illustrative purposes only, and are not intended to limit the scope of the present invention in any way. Efforts have been made to ensure accuracy with respect to numbers used (e.g., amounts, temperatures, etc.), but some experimental error and deviation should, of course, be allowed for.

The practice of the present invention will employ, unless otherwise indicated, conventional methods of protein chemistry, biochemistry, recombinant DNA techniques and pharmacology, within the skill of the art. Such techniques are explained fully in the literature. See, e.g., T.E. Creighton, Proteins: Structures and Molecular Properties (W.H. Freeman and Company, 1993); A.L. Lehninger, Biochemistry (Worth Publishers, Inc., current addition); Sambrook, et al., Molecular Cloning: A Laboratory Manual (2nd Edition, 1989); Methods In Enzymology (S. Colowick and N. Kaplan eds., Academic Press, Inc.); Remington's Pharmaceutical Sciences, 18th Edition (Easton, Pennsylvania: Mack Publishing Company, 1990); Carey and Sundberg Advanced Organic Chemistry 3rd Ed. (Plenum Press) Vols A and B(1992).

### XIV.A. Neoantigen Cassette Design

Through vaccination, multiple class I MHC restricted tumor-specific neoantigens (TSNAs) that stimulate the corresponding cellular immune response(s) can be delivered. In one example, a vaccine cassette was engineered to encode multiple epitopes as a single gene product where the epitopes were either embedded within their natural, surrounding peptide sequence or spaced by non-natural linker sequences. Several design parameters were identified that could potentially impact antigen processing and presentation and therefore the magnitude and breadth of the TSNA specific CD8 T cell responses. In the present example, several model cassettes were designed and constructed to evaluate: (1) whether robust T cell responses could be generated to multiple epitopes incorporated in a single expression cassette; (2) what makes an optimal linker placed between the TSNAs within the expression cassette- that leads to optimal processing and presentation of all epitopes; (3) if the relative position of the epitopes within the cassette impact T cell responses; (4) whether the number of epitopes within a cassette influences the magnitude or quality of the T cell responses to individual epitopes; (5) if the addition of cellular targeting sequences improves T cell responses.

Two readouts were developed to evaluate antigen presentation and T cell responses specific for marker epitopes within the model cassettes: (1) an *in vitro* cell-based screen which allowed assessment of antigen presentation as gauged by the activation of specially engineered reporter T cells (Aarnoudse et al., 2002; Nagai et al., 2012); and (2) an *in vivo* assay that used HLA-A2 transgenic mice (Vitiello et al., 1991) to assess post-vaccination immunogenicity of cassette-derived epitopes of human origin by their corresponding epitope-specific T cell responses (Cornet et al., 2006; Depla et al., 2008; Ishioka et al., 1999).

### XIV.B. Neoantigen Cassette Design Evaluation

### XIV.B.1. Methods and Materials

### TCR and cassette design and cloning

The selected TCRs recognize peptides NLVPMVATV (SEQ ID NO: 128) (PDB# 5D2N), CLGGLLTMV (SEQ ID NO: 129) (PDB#3REV), GILGFVFTL (SEQ ID NO: 130) (PDB#1OGA) LLFGYPVYV (SEQ ID NO: 131) (PDB#1AO7) when presented by A*0201. Transfer vectors were constructed that contain 2A peptide-linked TCR subunits (beta followed by alpha), the EMCV IRES, and 2A-linked CD8 subunits (beta followed by alpha and by the puromycin resistance gene). Open reading frame sequences were codon-optimized and synthesized by GeneArt.

## Cell line generation for in vitro epitope processing and presentation studies

Peptides were purchased from ProImmune or Genscript diluted to 10mg/mL with 10mM tris(2-carboxyethyl) phosphine (TCEP) in water/DMSO (2:8, v/v). Cell culture medium and supplements, unless otherwise noted, were from Gibco. Heat inactivated fetal bovine serum (FBShi) was from Seradigm. QUANTI-Luc Substrate, Zeocin, and Puromycin were from InvivoGen. Jurkat-Lucia NFAT Cells (InvivoGen) were maintained in RPMI 1640 supplemented with 10% FBShi, Sodium Pyruvate, and 100μg/mL Zeocin. Once transduced, these cells additionally received 0.3 μg/mL Puromycin. T2 cells (ATCC CRL-1992) were cultured in Iscove's Medium (IMDM) plus 20% FBShi. U-87 MG (ATCC HTB-14) cells were maintained in MEM Eagles Medium supplemented with 10% FBShi.

Jurkat-Lucia NFAT cells contain an NFAT-inducible Lucia reporter construct. The Lucia gene, when activated by the engagement of the T cell receptor (TCR), causes secretion of a coelenterazine-utilizing luciferase into the culture medium. This luciferase can be measured using the QUANTI-Luc luciferase detection reagent. Jurkat-Lucia cells were transduced with lentivirus to express antigen-specific TCRs. The HIV-derived lentivirus transfer vector was obtained from GeneCopoeia, and lentivirus support plasmids expressing VSV-G (pCMV-VsvG), Rev (pRSV-Rev) and Gag-pol (pCgpV) were obtained from Cell Design Labs.

Lentivirus was prepared by transfection of 50-80% confluent T75 flasks of HEK293 cells with Lipofectamine 2000 (Thermo Fisher), using 40 μl of lipofectamine and 20 μg of the DNA mixture (4:2: 1:1 by weight of the transfer plasmid:pCgpV:pRSV-Rev:pCMV-VsvG). 8-10 mL of the virus-containing media were concentrated using the Lenti-X system (Clontech), and the virus resuspended in 100-200 μl of fresh medium. This volume was used to overlay an equal volume of Jurkat-Lucia cells (5x10E4-1x10E6 cells were used in different experiments). Following culture in 0.3 μg/ml puromycin-containing medium, cells were sorted to obtain clonality. These Jurkat-Lucia TCR clones were tested for activity and selectivity using peptide loaded T2 cells.

## In vitro epitope processing and presentation assay

T2 cells are routinely used to examine antigen recognition by TCRs. T2 cells lack a peptide transporter for antigen processing (TAP deficient) and cannot load endogenous peptides in the endoplasmic reticulum for presentation on the MHC. However, the T2 cells can easily be loaded with exogenous peptides. The five marker peptides (NLVPMVATV (SEQ ID NO: 128), CLGGLLTMV (SEQ ID NO: 129), GLCTLVAML (SEQ ID NO: 132), LLFGYPVYV (SEQ ID NO: 131), GILGFVFTL (SEQ ID NO: 130)) and two irrelevant peptides (WLSLLVPFV (SEQ ID NO: 133), FLLTRICT (SEQ ID NO: 134)) were loaded onto T2 cells. Briefly, T2 cells were counted and diluted to 1x106 cells/mL with IMDM plus 1% FBShi. Peptides were added to result in 10 μg peptide/1x106 cells. Cells were then incubated at 37oC for 90 minutes. Cells were washed twice with IMDM plus 20% FBShi, diluted to 5x10E5 cells/mL and 100 μL plated into a 96-well Costar tissue culture plate. Jurkat-Lucia TCR clones were counted and diluted to 5x10E5 cells/mL in RPMI 1640 plus 10% FBShi and 100 μL added to the T2 cells. Plates were incubated overnight at 37°C, 5% CO2. Plates were then centrifuged at 400g for 3 minutes and 20 μL supernatant removed to a white flat bottom Greiner plate. QUANTI-Luc substrate was prepared according to instructions and 50 μL/well added. Luciferase expression was read on a Molecular Devices SpectraMax iE3x.

To test marker epitope presentation by the adenoviral cassettes, U-87 MG cells were used as surrogate antigen presenting cells (APCs) and were transduced with the adenoviral vectors. U-87 MG cells were harvested and plated in culture media as 5x10E5 cells/100 μl in a 96-well Costar tissue culture plate. Plates were incubated for approximately 2 hours at 37°C. Adenoviral cassettes were diluted with MEM plus 10% FBShi to an MOI of 100, 50, 10, 5, 1 and 0 and added to the U-87 MG cells as 5μl/well. Plates were again incubated for approximately 2 hours at 37°C. Jurkat-Lucia TCR clones were counted and diluted to 5x10E5 cells/mL in RPMI plus 10% FBShi and added to the U-87 MG cells as 100 μL/well. Plates were then incubated for approximately 24 hours at 37°C, 5% CO2. Plates were centrifuged at 400g for 3 minutes and 20 μL supernatant removed to a white flat bottom Greiner plate. QUANTI-Luc substrate was prepared according to instructions and 50 μL/well added. Luciferase expression was read on a Molecular Devices SpectraMax iE3x.

## Mouse strains for immunogenicity studies

Transgenic HLA-A2.1 (HLA-A2 Tg) mice were obtained from Taconic Labs, Inc. These mice carry a transgene consisting of a chimeric class I molecule comprised of the human HLA-A2.1 leader, α1, and α2 domains and the murine H2-Kb α3, transmembrane, and cytoplasmic domains (Vitiello et al., 1991). Mice used for these studies were the first generation offspring (F1) of wild type BALB/cAnNTac females and homozygous HLA-A2.1 Tg males on the C57Bl/6 background.

## Adenovirus vector (Ad5v) immunizations

HLA-A2 Tg mice were immunized with $1x10^{10}$ to $1x10^{6}$ viral particles of adenoviral vectors via bilateral intramuscular injection into the tibialis anterior. Immune responses were measured at 12 days post-immunization.

**Lymphocyte isolation**

Lymphocytes were isolated from freshly harvested spleens and lymph nodes of immunized mice. Tissues were dissociated in RPMI containing 10% fetal bovine serum with penicillin and streptomycin (complete RPMI) using the GentleMACS tissue dissociator according to the manufacturer's instructions.

**Ex vivo enzyme-linked immunospot (ELISPOT) analysis**

ELISPOT analysis was performed according to ELISPOT harmonization guidelines (Janetzki et al., 2015) with the mouse IFNg ELISpotPLUS kit (MABTECH). $1\times10^5$ splenocytes were incubated with 10uM of the indicated peptides for 16 hours in 96-well IFNg antibody coated plates. Spots were developed using alkaline phosphatase. The reaction was timed for 10 minutes and was quenched by running the plate under tap water. Spots were counted using an AID vSpot Reader Spectrum. For ELISPOT analysis, wells with saturation >50% were recorded as "too numerous to count". Samples with deviation of replicate wells > 10% were excluded from analysis. Spot counts were then corrected for well confluency using the formula: spot count + 2 x (spot count x %confluence /[100% - %confluence]). Negative background was corrected by subtraction of spot counts in the negative peptide stimulation wells from the antigen stimulated wells. Finally, wells labeled too numerous to count were set to the highest observed corrected value, rounded up to the nearest hundred.

**Ex vivo intracellular cytokine staining (ICS) and flow cytometry analysis**

Freshly isolated lymphocytes at a density of $2\text{-}5\times10^6$ cells/mL were incubated with 10uM of the indicated peptides for 2 hours. After two hours, brefeldin A was added to a concentration of 5ug/ml and cells were incubated with stimulant for an additional 4 hours. Following stimulation, viable cells were labeled with fixable viability dye eFluor780 according to manufacturer's protocol and stained with anti-CD8 APC (clone 53-6.7, BioLegend) at 1:400 dilution. Anti-IFNg PE (clone XMG1.2, BioLegend) was used at 1:100 for intracellular staining. Samples were collected on an Attune NxT Flow Cytometer (Thermo Scientific). Flow cytometry data was plotted and analyzed using FlowJo. To assess degree of antigen-specific response, both the percent IFNg+ of CD8+ cells and the total IFNg+ cell number/$1\times10^6$ live cells were calculated in response to each peptide stimulant.

**XIV.B.2. *In Vitro* Evaluation of Neoantigen Cassette Designs**

As an example of neoantigen cassette design evaluation, an in vitro cell-based assay was developed to assess whether selected human epitopes within model vaccine cassettes were being expressed, processed, and presented by antigen-presenting cells (Fig. 15). Upon recognition, Jurkat-Lucia reporter T cells that were engineered to express one of five TCRs specific for well-characterized peptide-HLA combinations become activated and translocate the nuclear factor of activated T cells (NFAT) into the nucleus which leads to transcriptional activation of a luciferase reporter gene. Antigenic stimulation of the individual reporter CD8 T cell lines was quantified by bioluminescence.

Individual Jurkat-Lucia reporter lines were modified by lentiviral transduction with an expression construct that includes an antigen-specific TCR beta and TCR alpha chain separated by a P2A ribosomal skip sequence to ensure equimolar amounts of translated product (Banu et al., 2014). The addition of a second CD8 beta-P2A-CD8 alpha element to the lentiviral construct provided expression of the CD8 co-receptor, which the parent reporter cell line lacks, as CD8 on the cell surface is crucial for the binding affinity to target pMHC molecules and enhances signaling through engagement of its cytoplasmic tail (Lyons et al., 2006; Yachi et al., 2006).

After lentiviral transduction, the Jurkat-Lucia reporters were expanded under puromycin selection, subjected to single cell fluorescence assisted cell sorting (FACS), and the monoclonal populations tested for luciferase expression. This yielded stably transduced reporter cell lines for specific peptide antigens 1, 2, 4, and 5 with functional cell responses. (Table 2).

**Table 2:** Development of an *in vitro* T cell activation assay. Peptide-specific T cell recognition as measured by induction of luciferase indicates effective processing and presentation of the vaccine cassette antigens.

| Epitope | Short Cassette Design AAY |
|---|---|
| 1 | 24.5 $\pm$ 0.5 |
| 2 | 11.3 $\pm$ 0.4 |

(continued)

| Epitope | Short Cassette Design |
|---|---|
| | AAY |
| 3* | n/a |
| 4 | 26.1 ± 3.1 |
| 5 | 46.3 ± 1.9 |
| * Reporter T cell for epitope 3 not yet generated | |

In another example, a series of short cassettes, all marker epitopes were incorporated in the same position (Fig. 16A) and only the linkers separating the HLA-A*0201 restricted epitopes (Fig. 16B) were varied. Reporter T cells were individually mixed with U-87 antigen-presenting cells (APCs) that were infected with adenoviral constructs expressing these short cassettes, and luciferase expression was measured relative to uninfected controls. All four antigens in the model cassettes were recognized by matching reporter T cells, demonstrating efficient processing and presentation of multiple antigens. The magnitude of T cell responses follow largely similar trends for the natural and AAY-linkers. The antigens released from the RR-linker based cassette show lower luciferase inductions (Table 3). The DPP-linker, designed to disrupt antigen processing, produced a vaccine cassette that led to poor epitope presentation (Table 3).

Table 3: Evaluation of linker sequences in short cassettes. Luciferase induction in the *in vitro* T cell activation assay indicated that, apart from the DPP-based cassette, all linkers facilitated efficient release of the cassette antigens. T cell epitope only (no linker) = 9AA, natural linker one side = 17AA, natural linker both sides = 25AA, non-natural linkers = AAY, RR, DPP

| Epitope | Short Cassette Designs | | | | | |
|---|---|---|---|---|---|---|
| | 9AA | 17AA | 25AA | AAY | RR | DPP |
| 1 | 33.6 ± 0.9 | 42.8 ± 2.1 | 42.3 ± 2.3 | 24.5 ± 0.5 | 21.7 ± 0.9 | 0.9 ± 0.1 |
| 2 | 12.0 ± 0.9 | 10.3 ± 0.6 | 14.6 ± 04 | 11.3 ± 0.4 | 8.5 ± 0.3 | **1.1** ± 0.2 |
| 3* | n/a | n/a | n/a | n/a | n/a | n/a |
| 4 | 26.6 ± 2.5 | 16.1 ± 0.6 | 16.6 ± 0.8 | 26.1 ± 3.1 | 12.5 ± 0.8 | 1.3 ± 0.2 |
| 5 | 29.7 ± 0.6 | 21.2 ± 0.7 | 24.3 ± 1.4 | 46.3 ± 1.9 | 19.7 ± 0.4 | 1.3 ± 0.1 |
| * Reporter T cell for epitope 3 not yet generated | | | | | | |

In another example, an additional series of short cassettes were constructed that, besides human and mouse epitopes, contained targeting sequences such as ubiquitin (Ub), MHC and Ig-kappa signal peptides (SP), and/or MHC transmembrane (TM) motifs positioned on either the N- or C-terminus of the cassette. (Fig. 17). When delivered to U-87 APCs by adenoviral vector, the reporter T cells again demonstrated efficient processing and presentation of multiple cassette-derived antigens. However, the magnitude of T cell responses were not significantly impacted by the various targeting features (Table 4).

Table 4: Evaluation of cellular targeting sequences added to model vaccine cassettes. Employing the *in vitro* T cell activation assay demonstrated that the four HLA-A*0201 restricted marker epitopes are liberated efficiently from the model cassettes and targeting sequences did not significantly improve T cell recognition and activation.

| Epitope | Short Cassette Designs | | | | | | | | | |
|---|---|---|---|---|---|---|---|---|---|---|
| | A | B | C | D | E | F | G | H | I | J |
| 1 | 32.5 ± 1.5 | 31.8 ± 0.8 | 29.1 ± 1.2 | 29.1 ± 1.1 | 28.4 ± 0.7 | 20.4 ± 0.5 | 35.0 ± 1.3 | 30.3 ± 2.0 | 22.5 ± 0.9 | 38.1 ± 1.6 |
| 2 | 6.1 ± 0.2 | 6.3 ± 0.2 | 7.6 ± 0.4 | 7.0 ± 0.5 | 5.9 ± 0.2 | 3.7 ± 0.2 | 7.6 ± 0.4 | 5.4 ± 0.3 | 6.2 ± 0.4 | 6.4 ± 0.3 |
| 3* | n/a | n/a | n/a | n/a | n/a | n/a | n/a | n/a | n/a | n/a |

(continued)

| Short Cassette Designs | | | | | | | | | | |
|---|---|---|---|---|---|---|---|---|---|---|
| Epitope | A | B | C | D | E | F | G | H | I | J |
| 4 | 12.3 ± 1.1 | 14.1 ± 0.7 | 12.2 ± 0.8 | 13.7 ± 1.0 | 11.7 ± 0.8 | 10.6 ± 0.4 | 11.0 ± 0.6 | 7.6 ± 0.6 | 16.1 ± 0.5 | 8.7 ± 0.5 |
| 5 | 44.4 ± 2.8 | 53.6 ± 1.6 | 49.9 ± 3.3 | 50.5 ± 2.8 | 41.7 ± 2.8 | 36.1 ± 1.1 | 46.5 ± 2.1 | 31.4 ± 0.6 | 75.4 ± 1.6 | 35.7 ± 2.2 |

| * Reporter T cell for epitope 3 not yet generated |
|---|

### XIV.B.3. *In Vivo* Evaluation of Neoantigen Cassette Designs

As another example of neoantigen cassette design evaluation, vaccine cassettes were designed to contain 5 well-characterized human class I MHC epitopes known to stimulate CD8 T cells in an HLA-A*02:01 restricted fashion (Fig. 16A, 17, 19A). For the evaluation of their in vivo immunogenicity, vaccine cassettes containing these marker epitopes were incorporated in adenoviral vectors and used to infect HLA-A2 transgenic mice (Fig. 18). This mouse model carries a transgene consisting partly of human HLA-A*0201 and mouse H2-Kb thus encoding a chimeric class I MHC molecule consisting of the human HLA-A2.1 leader, $\alpha$1 and $\alpha$2 domains ligated to the murine $\alpha$3, transmembrane and cytoplasmic H2-Kb domain (Vitiello et al., 1991). The chimeric molecule allows HLA-A*02:01-restricted antigen presentation whilst maintaining the species-matched interaction of the CD8 co-receptor with the $\alpha$3 domain on the MHC.

For the short cassettes, all marker epitopes generated a vigorous T cell response, as determined by IFN-gamma ELISPOT, that was approximately 10-50x stronger of what has been commonly reported (Cornet et al., 2006; Depla et al., 2008; Ishioka et al., 1999). Of all the linkers evaluated, the concatamer of 25mer sequences, each containing a minimal epitope flanked by their natural amino acids sequences as in the invention as claimed, generated the largest and broadest T cell response (Table 5). Intracellular cytokine staining (ICS) and flow cytometry analysis revealed that the antigen-specific T cell responses are derived from CD8 T cells.

**Table 5:** In vivo evaluation of linker sequences in short cassettes. ELISPOT data indicated that HLA-A2 transgenic mice, 17 days post-infectionwith 1e11 adenovirus viral particles,

| Short Cassette Designs | | | | | | |
|---|---|---|---|---|---|---|
| Epitope | 9AA | 17AA | 25AA | AAY | RR | DPP |
| 1 | 2020+/-583 | 2505+/-1281 | 6844+/-956 | 1489+/-762 | 1675+/-690 | 1781+/-774 |
| 2 | 4472+/-755 | 3792+/-1319 | 7629+/-996 | 3851+/-1748 | 4726+/-1715 | 5868+/-1427 |
| 3 | 5830+/-315 | 3629+/-862 | 7253+/-491 | 4813+/-1761 | 6779+/-1033 | 7328+/-1700 |
| 4 | 5536+/-375 | 2446+/-955 | 2961+/-1487 | 4230+/-1759 | 6518+/-909 | 7222+/-1824 |
| 5 | 8800+/-0 | 7943+/-821 | 8423+/-442 | 8312+/-696 | 8800+/-0 | 1836+/-328 |

In another example, a series of long vaccine cassettes was constructed and incorporated in adenoviral vectors that, next to the original 5 marker epitopes, contained an additional 16 HLA-A*02:01, A*03:01 and B*44:05 epitopes with known CD8 T cell reactivity (Fig. 19A, B). The size of these long cassettes closely mimicked the final clinical cassette design, and only the position of the epitopes relative to each other was varied. The CD8 T cell responses were comparable in magnitude and breadth for both long and short vaccine cassettes, demonstrating that (a) the addition of more epitopes did not impact the magnitude of immune response to the original set of epitopes, and (b) the position of an epitope in a cassette did not influence the ensuing T cell response to it (Table 6).

**Table 6:** In vivo evaluation of the impact of epitope position in long cassettes. ELISPOT data indicated that HLA-A2 transgenic mice, 17 days post-infection with 5e10 adenovirus viral particles, generated a T cell response comparable in magnitude for both long and short vaccine cassettes.

| Long Cassette Designs | | | |
|---|---|---|---|
| Epitope | Standard | Scrambled | Short |
| 1 | 863+/-1080 | 804+/-1113 | 1871+/-2859 |

(continued)

| Epitope | Long Cassette Designs | | |
| --- | --- | --- | --- |
| | Standard | Scrambled | Short |
| 2 | 6425+/-1594 | 28+/-62 | 5390+/-1357 |
| 3* | 23+/-30 | 36+/-18 | 0+/-48 |
| 4 | 2224+/-1074 | 2727+/-644 | 2637+/-1673 |
| 5 | 7952+/-297 | 8100+/-0 | 8100+/-0 |

* Suspected technical error caused an absence of a T cell response.

## XIV.B.4. Neoantigen Cassette Design for Immunogenicity and Toxicology Studies

In summary, the findings of the model cassette evaluations (Fig. 16-19, Tables 2-6) demonstrated that, for model vaccine cassettes, optimal immunogenicity was achieved when a "string of beads" approach was employed that encodes around 20 epitopes in the context of an adenovirus-based vector. The epitopes were best assembled by concatenating 25mer sequences, each embedding a minimal CD8 T cell epitope (e.g. 9 amino acid residues) that were flanked on both sides by its natural, surrounding peptide sequence (e.g. 8 amino acid residues on each side). As used herein, a "natural" or "native" flanking sequence refers to the N- and/or C-terminal flanking sequence of a given epitope in the naturally occurring context of that epitope within its source protein. For example, the HCMV pp65 MHC I epitope NLVPMVATV (SEQ ID NO: 128) is flanked on its 5' end by the native 5' sequence WQAGILAR (SEQ ID NO: 135) and on its 3' end by the native 3' sequence QGQNLKYQ (SEQ ID NO: 136), thus generating the WQAGILARNLVPMVATVQGQNLKYQ (SEQ ID NO: 137) 25mer peptide found within the HCMV pp65 source protein. The natural or native sequence can also refer to a nucleotide sequence that encodes an epitope flanked by native flanking sequence(s). Each 25mer sequence is directly connected to the following 25mer sequence. In instances where the minimal CD8 T cell epitope is greater than or less than 9 amino acids, the flanking peptide length can be adjusted such that the total length is still a 25mer peptide sequence. For example, a 10 amino acid CD8 T cell epitope can be flanked by an 8 amino acid sequence and a 7 amino acid. The concatamer was followed by two universal class II MHC epitopes that were included to stimulate CD4 T helper cells and improve overall *in vivo* immunogenicity of the vaccine cassette antigens. (Alexander et al., 1994; Panina-Bordignon et al., 1989) The class II epitopes were linked to the final class I epitope by a GPGPG amino acid linker (SEQ ID NO:56). The two class II epitopes were also linked to each other by a GPGPG amino acid linker (SEQ ID NO: 56), as a well as flanked on the C-terminus by a GPGPG amino acid linker (SEQ ID NO: 56). Neither the position nor the number of epitopes proved to substantially impact T cell recognition or response. Targeting sequences also did not appear to substantially impact the immunogenicity of cassette-derived antigens.

As a further example, based on the in vitro and in vivo data obtained with model cassettes (Fig. 16-19, Tables 2-6), a cassette design was generated that alternates well-characterized T cell epitopes known to be immunogenic in nonhuman primates (NHPs), mice and humans. The 20 epitopes, all embedded in their natural 25mer sequences, are followed by the two universal class II MHC epitopes that were present in all model cassettes evaluated (Fig. 20). This cassette design was used to study immunogenicity as well as pharmacology and toxicology studies in multiple species.

## XV. ChAd Neoantigen Cassette Delivery Vector

### XV.A. ChAd Neoantigen Cassette Delivery Vector Construction

In one example, Chimpanzee adenovirus (ChAd) was engineered to be a delivery vector for neoantigen cassettes. In a further example, a full-length ChAdV68 vector was synthesized based on AC_000011.1 (sequence 2 from Patent US 6083716) with E1 (nt 457 to 3014) and E3 (nt 27,816- 31,332) sequences deleted. Reporter genes under the control of the CMV promoter/enhancer were inserted in place of the deleted E1 sequences. Transfection of this clone into HEK293 cells did not yield infectious virus. To confirm the sequence of the wild-type C68 virus, isolate VR-594 was obtained from the ATCC, passaged, and then independently sequenced (SEQ ID NO:10). When comparing the AC_000011.1 sequence to the ATCC VR-594 sequence (SEQ ID NO:10) of wild-type ChAdV68 virus , 6 nucleotide differences were identified. In one example, a modified ChAdV68 vector was generated based on AC_000011.1, with the corresponding ATCC VR-594 nucleotides substituted at five positions (ChAdV68.5WTnt SEQ ID NO:1).

In another example, a modified ChAdV68 vector was generated based on AC_000011.1 with E1 (nt 577 to 3403) and E3 (nt 27,816- 31,332) sequences deleted and the corresponding ATCC VR-594 nucleotides substituted at four positions. A GFP reporter (ChAdV68.4WTnt.GFP; SEQ ID NO:11) or model neoantigen cassette (ChAdV68.4WTnt.MAG25mer; SEQ ID NO:12) under the control of the CMV promoter/enhancer was inserted in place of deleted E1 sequences.

In another example, a modified ChAdV68 vector was generated based on AC_000011.1 with E1 (nt 577 to 3403) and E3 (nt 27,125- 31,825) sequences deleted and the corresponding ATCC VR-594 nucleotides substituted at five positions. A GFP reporter (ChAdV68.5WTnt.GFP; SEQ ID NO:13) or model neoantigen cassette (ChAdV68.5WTnt.MAG25mer; SEQ ID NO:2) under the control of the CMV promoter/enhancer was inserted in place of deleted E1 sequences.

Full-Length ChAdVC68 sequence "ChAdV68.5WTnt" (SEQ ID NO:1); AC_000011.1 sequence with corresponding ATCC VR-594 nucleotides substituted at five positions.

```
CCATCTTCAATAATATACCTCAAACTTTTTGTGCGCGTTAATATGCAAATGAGGCGTTTGAATTTGGGGAGGAA
GGGCGGTGATTGGTCGAGGGATGAGCGACCGTTAGGGGCGGGGCGAGTGACGTTTTGATGACGTGGTTGCGAGG
AGGAGCCAGTTTGCAAGTTCTCGTGGGAAAAGTGACGTCAAACGAGGTGTGGTTTGAACACGGAAATACTCAAT
TTTCCCGCGCTCTCTGACAGGAAATGAGGTGTTTCTGGGCGGATGCAAGTGAAAACGGGCCATTTTCGCGCGAA
AACTGAATGAGGAAGTGAAAATCTGAGTAATTTCGCGTTTATGGCAGGGAGGAGTATTTGCCGAGGGCCGAGTA
GACTTTGACCGATTACGTGGGGGTTTCGATTACCGTGTTTTTCACCTAAATTTCCGCGTACGGTGTCAAAGTCC
GGTGTTTTTACGTAGGTGTCAGCTGATCGCCAGGGTATTTAAACCTGCGCTCTCCAGTCAAGAGGCCACTCTTG
AGTGCCAGCGAGAAGAGTTTTCTCCTCCGCGCCGCGAGTCAGATCTACACTTTGAAAGATGAGGCACCTGAGAG
ACCTGCCCGATGAGAAAATCATCATCGCTTCCGGGAACGAGATTCTGGAACTGGTGGTAAATGCCATGATGGGC
GACGACCCTCCGGAGCCCCCCACCCCATTTGAGACACCTTCGCTGCACGATTTGTATGATCTGGAGGTGGATGT
GCCCGAGGACGATCCCAATGAGGAGGCGGTAAATGATTTTTTTAGCGATGCCGCGCTGCTAGCTGCCGAGGAGG
CTTCGAGCTCTAGCTCAGACAGCGACTCTTCACTGCATACCCCTAGACCCGGCAGAGGTGAGAAAAAGATCCCC
GAGCTTAAAGGGGAAGAGATGGACTTGCGCTGCTATGAGGAATGCTTGCCCCCGAGCGATGATGAGGACGAGCA
GGCGATCCAGAACGCAGCGAGCCAGGGAGTGCAAGCCGCCAGCGAGAGCTTTGCGCTGGACTGCCCGCCTCTGC
CCGGACACGGCTGTAAGTCTTGTGAATTTCATCGCATGAATACTGGAGATAAAGCTGTGTTGTGTGCACTTTGC
TATATGAGAGCTTACAACCATTGTGTTTACAGTAAGTGTGATTAAGTTGAACTTTAGAGGGAGGCAGAGAGCAG
GGTGACTGGGCGATGACTGGTTTATTTATGTATATATGTTCTTTATATAGGTCCCGTCTCTGACGCAGATGATG
AGACCCCCACTACAAAGTCCACTTCGTCACCCCCAGAAATTGGCACATCTCCACCTGAGAATATTGTTAGACCA
GTTCCTGTTAGAGCCACTGGGAGGAGAGCAGCTGTGGAATGTTTGGATGACTTGCTACAGGGTGGGGTTGAACC
TTTGGACTTGTGTACCCGGAAACGCCCCAGGCACTAAGTGCCACACATGTGTGTTTACTTGAGGTGATGTCAGT
ATTTATAGGGTGTGGAGTGCAATAAAAAATGTGTTGACTTTAAGTGCGTGGTTTATGACTCAGGGGTGGGGACT
GTGAGTATATAAGCAGGTGCAGACCTGTGTGGTTAGCTCAGAGCGGCATGGAGATTTGGACGGTCTTGGAAGAC
TTTCACAAGACTAGACAGCTGCTAGAGAACGCCTCGAACGGAGTCTCTTACCTGTGGAGATTCTGCTTCGGTGG
CGACCTAGCTAGGCTAGTCTACAGGGCCAAACAGGATTATAGTGAACAATTTGAGGTTATTTTGAGAGAGTGTT
CTGGTCTTTTTGACGCTCTTAACTTGGGCCATCAGTCTCACTTTAACCAGAGGATTTCGAGAGCCCTTGATTTT
ACTACTCCTGGCAGAACCACTGCAGCAGTAGCCTTTTTTGCTTTTATTCTTGACAAATGGAGTCAAGAAACCCA
TTTCAGCAGGGATTACCAGCTGGATTTCTTAGCAGTAGCTTTGTGGAGAACATGGAAGTGCCAGCGCCTGAATG
CAATCTCCGGCTACTTGCCGGTACAGCCGCTAGACACTCTGAGGATCCTGAATCTCCAGGAGAGTCCCAGGGCA
CGCCAACGTCGCCAGCAGCAGCAGCAGGAGGAGGATCAAGAAGAGAACCCGAGAGCCGGCCTGGACCCTCCGGC
GGAGGAGGAGGAGTAGCTGACCTGTTTCCTGAACTGCGCCGGGTGCTGACTAGGTCTTCGAGTGGTCGGGAGAG
GGGGATTAAGCGGGAGAGGCATGATGAGACTAATCACAGAACTGAACTGACTGTGGGTCTGATGAGTCGCAAGC
GCCCAGAAACAGTGTGGTGGCATGAGGTGCAGTCGACTGGCACAGATGAGGTGTCGGTGATGCATGAGAGGTTT
TCTCTAGAACAAGTCAAGACTTGTTGGTTAGAGCCTGAGGATGATTGGGAGGTAGCCATCAGGAATTATGCCAA
```

```
GCTGGCTCTGAGGCCAGACAAGAAGTACAAGATTACTAAGCTGATAAATATCAGAAATGCCTGCTACATCTCAG
GGAATGGGGCTGAAGTGGAGATCTGTCTCCAGGAAAGGGTGGCTTTCAGATGCTGCATGATGAATATGTACCCG
GGAGTGGTGGGCATGGATGGGGTTACCTTTATGAACATGAGGTTCAGGGGAGATGGGTATAATGGCACGGTCTT
TATGGCCAATACCAAGCTGACAGTCCATGGCTGCTCCTTCTTTGGGTTTAATAACACCTGCATCGAGGCCTGGG
GTCAGGTCGGTGTGAGGGGCTGCAGTTTTTCAGCCAACTGGATGGGGGTCGTGGGCAGGACCAAGAGTATGCTG
TCCGTGAAGAAATGCTTGTTTGAGAGGTGCCACCTGGGGGTGATGAGCGAGGGCGAAGCCAGAATCCGCCACTG
CGCCTCTACCGAGACGGGCTGCTTTGTGCTGTGCAAGGGCAATGCTAAGATCAAGCATAATATGATCTGTGGAG
CCTCGGACGAGCGCGGCTACCAGATGCTGACCTGCGCCGGCGGGAACAGCCATATGCTGGCCACCGTACATGTG
GCTTCCCATGCTCGCAAGCCCTGGCCCGAGTTCGAGCACAATGTCATGACCAGGTGCAATATGCATCTGGGGGTC
CCGCCGAGGCATGTTCATGCCCTACCAGTGCAACCTGAATTATGTGAAGGTGCTGCTGGAGCCCGATGCCATGT
CCAGAGTGAGCCTGACGGGGGTGTTTGACATGAATGTGGAGGTGTGGAAGATTCTGAGATATGATGAATCCAAG
ACCAGGTGCCGAGCCTGCGAGTGCGGAGGGAAGCATGCCAGGTTCCAGCCCGTGTGTGTGGATGTGACGGAGGA
CCTGCGACCCGATCATTTGGTGTTGCCCTGCACCGGGACGGAGTTCGGTTCCAGCGGGGAAGAATCTGACTAGA
GTGAGTAGTGTTCTGGGGCGGGGGAGGACCTGCATGAGGGCCAGAATAACTGAAATCTGTGCTTTTCTGTGTGT
TGCAGCAGCATGAGCGGAAGCGGCTCCTTTGAGGGAGGGGTATTCAGCCCTTATCTGACGGGGCGTCTCCCCTC
CTGGGCGGGAGTGCGTCAGAATGTGATGGGATCCACGGTGGACGGCCGGCCCGTGCAGCCCGCGAACTCTTCAA
CCCTGACCTATGCAACCCTGAGCTCTTCGTCGTTGGACGCAGCTGCCGCCGCAGCTGCTGCATCTGCCGCCAGC
GCCGTGCGCGGAATGGCCATGGGCGCCGGCTACTACGGCACTCTGGTGGCCAACTCGAGTTCCACCAATAATCC
CGCCAGCCTGAACGAGGAGAAGCTGTTGCTGCTGATGGCCCAGCTCGAGGCCTTGACCCAGCGCCTGGGCGAGC
TGACCCAGCAGGTGGCTCAGCTGCAGGAGCAGACGCGGGCCGCGGTTGCCACGGTGAAATCCAAATAAAAAATG
AATCAATAAATAAACGGAGACGGTTGTTGATTTTAACACAGAGTCTGAATCTTTATTTGATTTTTCGCGCGCGG
TAGGCCCTGGACCACCGGTCTCGATCATTGAGCACCCGGTGGATCTTTTCCAGGACCCGGTAGAGGTGGGCTTG
GATGTTGAGGTACATGGGCATGAGCCCGTCCCGGGGGTGGAGGTAGCTCCATTGCAGGGCCTCGTGCTCGGGGG
TGGTGTTGTAAATCACCCAGTCATAGCAGGGGCGCAGGGCATGGTGTTGCACAATATCTTTGAGGAGGAGACTG
ATGGCCACGGGCAGCCCTTTGGTGTAGGTGTTTACAAATCTGTTGAGCTGGGAGGGATGCATGCGGGGGGGAGAT
GAGGTGCATCTTGGCCTGGATCTTGAGATTGGCGATGTTACCGCCCAGATCCCGCCTGGGGTTCATGTTGTGCA
GGACCACCAGCACGGTGTATCCGGTGCACTTGGGGAATTTATCATGCAACTTGGAAGGGAAGGCGTGAAAGAAT
TTGGCGACGCCTTTGTGCCCGCCCAGGTTTTCCATGCACTCATCCATGATGATGGCGATGGGCCCGTGGGCGGC
GGCCTGGGCAAAGACGTTTCGGGGGTCGGACACATCATAGTTGTGGTCCTGGGTGAGGTCATCATAGGCCATTT
TAATGAATTTGGGGCGGAGGGTGCCGGACTGGGGGACAAAGGTACCCTCGATCCCGGGGGCGTAGTTCCCCTCA
CAGATCTGCATCTCCCAGGCTTTGAGCTCGGAGGGGGGGATCATGTCCACCTGCGGGGCGATAAAGAACACGGT
TTCCGGGGCGGGGGAGATGAGCTGGGCCGAAAGCAAGTTCCGGAGCAGCTGGGACTTGCCGCAGCGGTGGGGC
CGTAGATGACCCCGATGACCGGCTGCAGGTGGTAGTTGAGGGAGAGACAGCTGCCGTCCTCCCGGAGGAGGGGG
GCCACCTCGTTCATCATCTCGCGCACGTGCATGTTCTCGCGCACCAGTTCCGCCAGGAGGCGCTCTCCCCCCAG
GGATAGGAGCTCCTGGAGCGAGGCGAAGTTTTTCAGCGGCTTGAGTCCGTCGGCCATGGGCATTTTGGAGAGGG
TTTGTTGCAAGAGTTCCAGGCGGTCCCAGAGCTCGGTGATGTGCTCTACGGCATCTCGATCCAGCAGACCTCCT
CGTTTCGCGGGTTGGGACGGCTGCGGGAGTAGGGCACCAGACGATGGGCGTCCAGCGCAGCCAGGGTCCGGTCC
TTCCAGGGTCGCAGCGTCCGCGTCAGGGTGGTCTCCGTCACGGTGAAGGGGTGCGCGCCGGGCTGGGCGCTTGC
GAGGGTGCGCTTCAGGCTCATCCGGCTGGTCGAAAACCGCTCCCGATCGGCGCCCTGCGCGTCGGCCAGGTAGC
AATTGACCATGAGTTCGTAGTTGAGCGCCTCGGCCGCGTGGCCTTTGGCGCGGAGCTTACCTTTGGAAGTCTGC
CCGCAGGCGGGACAGAGGAGGGACTTGAGGGCGTAGAGCTTGGGGGCGAGGAAGACGGACTCGGGGGCGTAGGC
GTCCGCGCCGCAGTGGGCGCAGACGGTCTCGCACTCCACGAGCCAGGTGAGGTCGGGCTGGTCGGGGTCAAAAA
CCAGTTTCCCGCCGTTCTTTTTGATGCGTTTCTTACCTTTGGTCTCCATGAGCTCGTGTCCCGCTGGGTGACA
AAGAGGCTGTCCGTGTCCCCGTAGACCGACTTTATGGGCCGGTCCTCGAGCGGTGTGCCGCGGTCCTCCTCGTA
GAGGAACCCCGCCCACTCCGAGACGAAAGCCCGGGTCCAGGCCAGCACGAAGGAGGCCACGTGGGACGGGTAGC
GGTCGTTGTCCACCAGCGGGTCCACCTTTTCCAGGGTATGCAAACACATGTCCCCCTCGTCCACATCCAGGAAG
GTGATTGGCTTGTAAGTGTAGGCCACGTGACCGGGGGGTCCCGGCCGGGGGGGGTATAAAAGGGTGCGGGTCCCTG
CTCGTCCTCACTGTCTTCCGGATCGCTGTCCAGGAGCGCCAGCTGTTGGGGTAGGTATTCCCTCTCGAAGGCGG
GCATGACCTCGGCACTCAGGTTGTCAGTTTCTAGAAACGAGGAGGATTTGATATTGACGGTGCCGGCGGAGATG
CCTTTCAAGAGCCCCTCGTCCATCTGGTCAGAAAAGACGATCTTTTTGTTGTCGAGCTTGGTGGCGAAGGAGCC
GTAGAGGGCGTTGGAGAGGAGCTTGGCGATGGAGCGCATGGTCTGGTTTTTTTCCTTGTCGGCGCGCTCCTTGG
CGGCGATGTTGAGCTGCACGTACTCGCGCGCCACGCACTTCCATTCGGGGAAGACGGTGGTCAGCTCGTCGGGC
ACGATTCTGACCTGCCAGCCCCGATTATGCAGGGTGATGAGGTCCACACTGGTGGCCACCTCGCCGCGCAGGGG
CTCATTAGTCCAGCAGAGGCGTCCGCCCTTGCGCGAGCAGAAGGGGGGCAGGGGGTCCAGCATGACCTCGTCGG
GGGGGTCGGCATCGATGGTGAAGATGCCGGGCAGGAGGTCGGGGTCAAAGTAGCTGATGGAAGTGGCCAGATCG
TCCAGGGCAGCTTGCCATTCGCGCACGGCCAGCGCGCGCTCGTAGGGACTGAGGGGCGTGCCCCAGGGCATGGG
ATGGGTAAGCGCGGAGGCGTACATGCCGCAGATGTCGTAGACGTAGAGGGGCTCCTCGAGGATGCCGATGTAGG
TGGGGTAGCAGCGCCCCCCGCGGATGCTGGCGCGCACGTAGTCATACAGCTCGTGCGAGGGGGCGAGGAGCCCC
GGGCCCAGGTTGGTGCGACTGGGCTTTTCGGCGCGGTAGACGATCTGGCGGAAAATGGCATGCGAGTTGGAGGA
GATGGTGGGCCTTTGGAAGATGTTGAAGTGGGCGTGGGGCAGTCCGACCGAGTCGCGGATGAAGTGGGCGTAGG
AGTCTTGCAGCTTGGCGACGAGCTCGGCGGTGACTAGGACGTCCAGAGCGCAGTAGTCGAGGGTCTCCTGGATG
ATGTCATACTTGAGCTGTCCCTTTTTGTTTCCACAGCTCGCGGTTGAGAAGGAACTCTTCGCGGTCCTTCCAGTA
```

(continued)

```
CTCTTCGAGGGGGAACCCGTCCTGATCTGCACGGTAAGAGCCTAGCATGTAGAACTGGTTGACGGCCTTGTAGG
CGCAGCAGCCCTTCTCCACGGGGAGGGCGTAGGCCTGGGCGGCCTTGCGCAGGGAGGTGTGCGTGAGGGCGAAA
GTGTCCCTGACCATGACCTTGAGGAACTGGTGCTTGAAGTCGATATCGTCGCAGCCCCCCTGCTCCCAGAGCTG
GAAGTCCGTGCGCTTCTTGTAGGCGGGGTTGGGCAAAGCGAAAGTAACATCGTTGAAGAGGATCTTGCCCGCGC
GGGGCATAAAGTTGCGAGTGATGCGGAAAGGTTGGGGCACCTCGGCCCGGTTGTTGATGACCTGGGCGGCGAGC
ACGATCTCGTCGAAGCCGTTGATGTTGTGGCCCACGATGTAGAGTTCCACGAATCGCGGACGGCCCTTGACGTG
GGGCAGTTTCTTGAGCTCCTCGTAGGTGAGCTCGTCGGGGTCGCTGAGCCCGTGCTGCTCGAGCGCCCAGTCGG
CGAGATGGGGGTTGGCGCGGAGGAAGGAAGTCCAGAGATCCACGGCCAGGGCGGTTTGCAGACGGTCCCGGTAC
TGACGGAACTGCTGCCCGACGGCCATTTTTTCGGGGGTGACGCAGTAGAAGGTGCGGGGGTCCCCGTGCCAGCG
ATCCCATTTGAGCTGGAGGGCGAGATCGAGGGCGAGCTCGACGAGCCGGTCGTCCCCGGAGAGTTTCATGACCA
GCATGAAGGGGACGAGCTGCTTGCCGAAGGACCCCATCCAGGTGTAGGTTTCCACATCGTAGGTGAGGAAGAGC
CTTTCGGTGCGAGGATGCGAGCCGATGGGGAAGAACTGGATCTCCTGCCACCAATTGGAGGAATGGCTGTTGAT
GTGATGGAAGTAGAAATGCCGACGGCGCGCCGAACACTCGTGCTTGTGTTTATACAAGCGGCCACAGTGCTCGC
AACGCTGCACGGGATGCACGTGCTGCACGAGCTGTACCTGAGTTCCTTTGACGAGGAATTTCAGTGGGAAGTGG
AGTCGTGGCGCCTGCATCTCGTGCTGTACTACGTCGTGGTGGTCGGCCTGGCCCTCTTCTGCCTCGATGGTGGT
CATGCTGACGAGCCCGCGCGGGAGGCAGGTCCAGACCTCGGCGCGAGCGGGTCGGAGAGCGAGGACGAGGGCGC
GCAGGCCGGAGCTGTCCAGGGTCCTGAGACGCTGCGGAGTCAGGTCAGTGGGCAGCGGCGGCGCGCGGTTGACT
TGCAGGAGTTTTTCCAGGGCGCGCGGGAGGTCCAGATGGTACTTGATCTCCACCGCGCCATTGGTGGCGACGTC
GATGGCTTGCAGGGTCCCGTGCCCCTGGGGTGTGACCACCGTCCCCCGTTTCTTCTTGGGCGGCTGGGGCGACG
GGGGCGGTGCCTCTTCCATGGTTAGAAGCGGCGGCGAGGACGCGCGCCGGGCGGCAGGGGCGGCTCGGGGCCCG
GAGGCAGGGGCGGCAGGGGCACGTCGGCGCCGCGCGGGTAGGTTCTGGTACTGCGCCCGGAGAAGACTGGCG
TGAGCGACGACGCGACGGTTGACGTCCTGGATCTGACGCCTCTGGGTGAAGGCCACGGGACCCGTGAGTTTGAA
CCTGAAAGAGAGTTCGACAGAATCAATCTCGGTATCGTTGACGGCGGCCTGCCGCAGGATCTCTTGCACGTCGC
CCGAGTTGTCCTGGTAGGCGATCTCGGTCATGAACTGCTCGATCTCCTCCTCTTGAAGGTCTCCGCGGCCGGCG
CGCTCCACGGTGGCCGCGAGGTCGTTGGAGATGCGGCCCATGAGCTGCGAGAAGGCGTTCATGCCCGCCTCGTT
CCAGACGCGGCTGTAGACCACGACGCCCTCGGGATCGCgGGCGCGCATGACCACCTGGGCGAGGTTGAGCTCCA
CGTGGCGCGTGAAGACCGCGTAGTTGCAGAGGCGCTGGTAGAGGTAGTTGAGCGTGGTGGCGATGTGCTCGGTG
ACGAAGAAATACATGATCCAGCGGCGGAGCGGCATCTCGCTGACGTCGCCCAGCGCCTCCAAACGTTCCATGGC
CTCGTAAAAGTCCACGGCGAAGTTGAAAAACTGGGAGTTGCGCGCCGAGACGGTCAACTCCTCCTCCAGAAGAC
GGATGAGCTCGGCGATGGTGGCGCGCACCTCGCGCTCGAAGGCCCCCGGGAGTTCCTCCACTTCCTCTTCTTCC
TCCTCCACTAACATCTCTTCTACTTCCTCCTCAGGCGGCAGTGGTGGCGGGGGAGGGGGCCTGCGTCGCCGGCG
GCGCACGGGCAGACGGTCGATGAAGCGCTCGATGGTCTCGCCGCGCCGGCGTCGCATGGTCTCGGTGACGGCGC
GCCCGTCCTCGCGGGGCCGCAGCGTGAAGACGCCGCCGCGCATCTCCAGGTGGCCGGGGGGGTCCCCGTTGGGC
AGGGAGAGGGCGCTGACGATGCATCTTATCAATTGCCCCGTAGGGACTCCGCGCAAGGACCTGAGCGTCTCGAG
ATCCACGGGATCTGAAAACCGCTGAACGAAGGCTTCGAGCCAGTCGCAGTCGCAAGGTAGGCTGAGCACGGTTT
CTTCTGGCGGGTCATGTTGGTTGGGAGCGGGGCGGGCGATGCTGCTGGTGATGAAGTTGAAATAGGCGGTTCTG
AGACGGCGGATGGTGGCGAGGAGCACCAGGTCTTTGGGCCCGGCTTGCTGGATGCGCAGACGGTCGGCCATGCC
CCAGGCGTGGTCCTGACACCTGGCCAGGTCCTTGTAGTAGTCCTGCATGAGCCGCTCCACGGGCACCTCCTCCT
CGCCCGCGCGGCCGTGCATGCGCGTGAGCCCGAAGCCGCGCTGGGGCTGGACGAGCGCCAGGTCGGCGACGACG
CGCTCGGCGAGGATGGCTTGCTGGATCTGGGTGAGGGTGGTCTGGAAGTCATCAAAGTCGACGAAGCGGTGGTA
GGCTCCGGTGTTGATGGTGTAGGAGCAGTTGGCCATGACGGACCAGTTGACGGTCTGGTGGCCCGGACGCACGA
GCTCGTGGTACTTGAGGCGCGAGTAGGCGCGCGTGTCGAAGATGTAGTCGTTGCAGGTGCGCACCAGGTACTGG
TAGCCGATGAGGAAGTGCGGCGGCGGCTGGCGGTAGAGCGGCCATCGCTCGGTGGCGGGGGCGCCGGGCGCGAG
GTCCTCGAGCATGGTGCGGTGGTAGCCGTAGATGTACCTGGACATCCAGGTGATGCCGGCGGCGGTGGTGGAGG
CGCGCGGGAACTCGCGGACGCGGTTCCAGATGTTGCGCAGCGGCAGGAAGTAGTTCATGGTGGGCACGGTCTGG
CCCGTGAGGCGCGCGCAGTCGTGGATGCTCTATACGGGCAAAAACGAAAGCGGTCAGCGGCTCGACTCCGTGGC
CTGGAGGCTAAGCGAACGGGTTGGGCTGCGCGTGTACCCCGGTTCGAATCTCGAATCAGGCTGGAGCCGCAGCT
AACGTGGTATTGGCACTCCCGTCTCGACCCAAGCCTGCACCAACCCTCCAGGATACGGAGGCGGGTCGTTTTGC
AACTTTTTTTTGGAGGCCGGATGAGACTAGTAAGCGCGGAAAGCGGCCGACCGCGATGGCTCGCTGCCGTAGTC
TGGAGAAGAATCGCCAGGGTTGCGTTGCGGTGTGCCCCGGTTCGAGGCCGGCCGGATTCCGCGGCTAACGAGGG
CGTGGCTGCCCCGTCGTTTCCAAGACCCCATAGCCAGCCGACTTCTCCAGTTACGGAGCGAGCCCCTCTTTTGT
TTTGTTTGTTTTTGCCAGATGCATCCCGTACTGCGGCAGATGCGCCCCCACCACCCTCCACCGCAACAACAGCC
CCCTCCACAGCCGGCGCTTCTGCCCCCGCCCCAGCAGCAACTTCCAGCCACGACCGCCGCGGCCGCCGTGAGCG
GGGCTGGACAGAGTTATGATCACCAGCTGGCCTTGGAAGAGGGCGAGGGGCTGGCGCGCCTGGGGGCGTCGTCG
CCGGAGCGGCACCCGCGCGTGCAGATGAAAAGGGACGCTCGCGAGGCCTACGTGCCCAAGCAGAACCTGTTCAG
AGACAGGAGCGGCGAGGAGCCCGAGGAGATGCGCGCGGCCCGGTTCCACGCGGGGCGGGAGCTGCGGCGCGGCC
TGGACCGAAAGAGGGTGCTGAGGGACGAGGATTTCGAGGCGGACGAGCTGACGGGGATCAGCCCCGCGCGCGCG
CACGTGGCCGCGGCCAACCTGGTCACGGCGTACGAGCAGACCGTGAAGGAGGAGAGCAACTTCCAAAAATCCTT
CAACAACCACGTGCGCACCCTGATCGCGCGCGAGGAGGTGACCCTGGGCCTGATGCACCTGTGGGACCTGCTGG
AGGCCATCGTGCAGAACCCCACCAGCAAGCCGCTGACGGCGCAGCTGTTCCTGGTGGTGCAGCATAGTCGGGAC
AACGAAGCGTTCAGGGAGGCGCTGCTGAATATCACCGAGCCCGAGGGCCGCTGGCTCCTGGACCTGGTGAACAT
TCTGCAGAGCATCGTGGTGCAGGAGCGCGGGCTGCCGCTGTCCGAGAAGCTGGCGGCCATCAACTTCTCGGTGC
```

(continued)

```
TGAGTTTGGGCAAGTACTACGCTAGGAAGATCTACAAGACCCCGTACGTGCCCATAGACAAGGAGGTGAAGATC
GACGGGTTTTACATGCGCATGACCCTGAAAGTGCTGACCCTGAGCGACGATCTGGGGGTGTACCGCAACGACAG
GATGCACCGTGCGGTGAGCGCCAGCAGGCGGCGCGAGCTGAGCGACCAGGAGCTGATGCATAGTCTGCAGCGGG
CCCTGACCGGGGCCGGGACCGAGGGGGAGAGCTACTTTGACATGGGCGCGGACCTGCACTGGCAGCCCAGCCGC
CGGGCCTTGGAGGCGGCGGCAGGACCCTACGTAGAAGAGGTGGACGATGAGGTGGACGAGGAGGGCGAGTACCT
GGAAGACTGATGGCGCGACCGTATTTTTGCTAGATGCAACAACAACAGCCACCTCCTGATCCCGCGATGCGGGC
GGCGCTGCAGAGCCAGCCGTCCGGCATTAACTCCTCGGACGATTGGACCCAGGCCATGCAACGCATCATGGCGC
TGACGACCCGCAACCCCGAAGCCTTTAGACAGCAGCCCCAGGCCAACCGGCTCTCGGCCATCCTGGAGGCCGTG
GTGCCCTCGCGCTCCAACCCCACGCACGAGAAGGTCCTGGCCATCGTGAACGCGCTGGTGGAGAACAAGGCCAT
CCGCGGCGACGAGGCCGGCCTGGTGTACAACGCGCTGCTGGAGCGCGTGGCCCGCTACAACAGCACCAACGTGC
AGACCAACCTGGACCGCATGGTGACCGACGTGCGCGAGGCCGTGGCCCAGCGCGAGCGGTTCCACCGCGAGTCC
AACCTGGGATCCATGGTGGCGCTGAACGCCTTCCTCAGCACCCAGCCCGCCAACGTGCCCCGGGGCCAGGAGGA
CTACACCAACTTCATCAGCGCCCTGCGCCTGATGGTGACCGAGGTGCCCCAGAGCGAGGTGTACCAGTCCGGGC
CGGACTACTTCTTCCAGACCAGTCGCCAGGGCTTGCAGACCGTGAACCTGAGCCAGGCTTTCAAGAACTTGCAG
GGCCTGTGGGGCGTGCAGGCCCCGGTCGGGGACCGCGCGACGGTGTCGAGCCTGCTGACGCCGAACTCGCGCCT
GCTGCTGCTGCTGGTGGCCCCCTTCACGGACAGCGGCAGCATCAACCGCAACTCGTACCTGGGCTACCTGATTA
ACCTGTACCGCGAGGCCATCGGCCAGGCGCACGTGGACGAGCAGACCTACCAGGAGATCACCCACGTGAGCCGC
GCCCTGGGCCAGGACGACCCGGGCAACCTGGAAGCCACCCTGAACTTTTTGCTGACCAACCGGTCGCAGAAGAT
CCCGCCCCAGTACGCGCTCAGCACCGAGGAGGAGCGCATCCTGCGTTACGTGCAGCAGAGCGTGGGCCTGTTCC
TGATGCAGGAGGGGGCCACCCCCAGCGCCGCGCTCGACATGACCGCGCGCAACATGGAGCCCAGCATGTACGCC
AGCAACCGCCCGTTCATCAATAAACTGATGGACTACTTGCATCGGGCGGCCGCCATGAACTCTGACTATTTCAC
CAACGCCATCCTGAATCCCCACTGGCTCCCGCCGCCGGGGTTCTACACGGGCGAGTACGACATGCCCGACCCCA
ATGACGGGTTCCTGTGGGACGATGTGGACAGCAGCGTGTTCTCCCCCGACCGGGTGCTAACGAGCGCCCCTTG
TGGAAGAAGGAAGGCAGCGACCGACGCCCGTCCTCGGCGCTGTCCGGCCGCGAGGGTGCTGCCGCGGCGGTGCC
CGAGGCCGCCAGTCCTTTCCCGAGCTTGCCCTTCTCGCTGAACAGTATCCGCAGCAGCGAGCTGGGCAGGATCA
CGCGCCCGCGCTTGCTGGGCGAAGAGGAGTACTTGAATGACTCGCTGTTGAGACCCGAGCGGGAGAAGAACTTC
CCCAATAACGGGATAGAAAGCCTGGTGGACAAGATGAGCCGCTGGAAGACGTATGCGCAGGAGCACAGGGACGA
TCCCCGGGCGTCGCAGGGGGCCACGAGCCGGGGCAGCGCCGCCCGTAAACGCCGGTGGCACGACAGGCAGCGGG
GACAGATGTGGGACGATGAGGACTCCGCCGACGACAGCAGCGTGTTGGACTTGGGTGGGAGTGGTAACCCGTTC
GCTCACCTGCGCCCCCGTATCGGGCGCATGATGTAAGAGAAACCGAAAATAAATGATACTCACCAAGGCCATGG
CGACCAGCGTGCGTTCGTTTCTTCTGTTGTTGTTGTATCTAGTATGATGAGGCGTGCGTACCCGGAGGGTCC
TCCTCCCTCGTACGAGAGCGTGATGCAGCAGGCGATGGCGGCGGCGGCGATGCAGCCCCCGCTGGAGGCTCCTT
ACGTGCCCCCGCGGTACCTGGCGCCTACGGAGGGGCGGAACAGCATTCGTTACTCGGAGCTGGCACCCTTGTAC
GATACCACCCGGTTGTACCTGGTGGACAACAAGTCGGCGGACATCGCCTCGCTGAACTACCAGAACGACCACAG
CAACTTCCTGACCACCGTGGTGCAGAACAATGACTTCACCCCCACGGAGGCCAGCACCCAGACCATCAACTTTG
ACGAGCGCTCGCGGTGGGGCGGCCAGCTGAAAACCATCATGCACACCAACATGCCCAACGTGAACGAGTTCATG
TACAGCAACAAGTTCAAGGCGCGGGTGATGGTCTCCCGCAAGACCCCCAATGGGGTGACAGTGACAGAGGATTA
TGATGGTAGTCAGGATGAGCTGAAGTATGAATGGGTGGAATTTGAGCTGCCCGAAGGCAACTTCTCGGTGACCA
TGACCATCGACCTGATGAACAACGCCATCATCGACAATTACTTGGCGGTGGGGCGGCAGAACGGGGTGCTGGAG
AGCGACATCGGCGTGAAGTTCGACACTAGGAACTTCAGGCTGGGCTGGGACCCCGTGACCGAGCTGGTCATGCC
CGGGGTGTACACCAACGAGGCTTTCCATCCCGATATTGTCTTGCTGCCCGGCTGCGGGGTGGACTTCACCGAGA
GCCGCCTCAGCAACCTGCTGGGCATTCGCAAGAGGCAGCCCTTCCAGGAAGGCTTCCAGATCATGTACGAGGAT
CTGGAGGGGGGCAACATCCCCGCGCTCCTGGATGTCGACGCCTATGAGAAAAGCAAGGAGGATGCAGCAGCTGA
AGCAACTGCAGCCGTAGCTACCGCCTCTACCGAGGTCAGGGGCGATAATTTTGCAAGCGCCGCAGCAGTGGCAG
CGGCCGAGGCGGCTGAAACCGAAAGTAAGATAGTCATTCAGCCGGTGGAGAAGGATAGCAAGAACAGGAGCTAC
AACGTACTACCGGACAAGATAAACACCGCCTACCGCAGCTGGTACCTAGCCTACAACTATGGCGACCCCGAGAA
GGGCGTGCGCTCCTGGACGCTGCTCACCACCTCGGACGTCACCTGCGGCGTGGAGCAAGTCTACTGGTCGCTGC
CCGACATGATGCAAGACCCGGTCACCTTCCGCTCCACGCGTCAAGTTAGCAACTACCCGGTGGTGGGCGCCGAG
CTCCTGCCCGTCTACTCCAAGAGCTTCTTCAACGAGCAGGCCGTCTACTCGCAGCAGCTGCGCGCCTTCACCTC
GCTTACGCACGTCTTCAACCGCTTCCCCGAGAACCAGATCCTCGTCCGCCCGCCCGCGCCCACCATTACCACCG
TCAGTGAAAACGTTCCTGCTCTCACAGATCACGGGACCCTGCCGCTGCGCAGCAGTATCCGGGGAGTCCAGCGC
GTGACCGTTACTGACGCCAGACGCCGCACCTGCCCCTACGTCTACAAGGCCCTGGGCATAGTCGCGCCGCGCGT
CCTCTCGAGCCGCACCTTCTAAATGTCCATTCTCATCTCGCCCAGTAATAACACCGGTTGGGGCCTGCGCGCGC
CCAGCAAGATGTACGGAGGCGCTCGCCAACGCTCCACGCAACACCCCGTGCGCGTGCGCGGGCACTTCCGCGCT
CCCTGGGGCGCCCTCAAGGGCCGCGTGCGGTCGCGCACCACCGTCGACGACGTGATCGACCAGGTGGTGGCCGA
CGCGCGCAACTACACCCCCGCCGCCGCGCCCGTCTCCACCGTGGACGCCGTCATCGACAGCGTGGTGGCcGACG
CGCGCCGGTACGCCCGCGCCAAGAGCCGGCGGCGGCGCATCGCCCGGCGGCACCGGAGCACCCCCGCCATGCGC
GCGGCGCGAGCCTTGCTGCGCAGGGCCAGGCGCACGGGACGCAGGGCCATGCTCAGGGCGGCCAGACGCGCGGC
TTCAGGCGCCAGCGCCGGCAGGACCCGGAGACGCGCGGCCACGGCGGCGGCAGCGGCCATCGCCAGCATGTCCC
GCCCGCGGCGAGGGAACGTGTACTGGGTGCGCGACGCCGCCACCGGTGTGCGCGTGCCCGTGCGCACCCGCCCC
CCTCGCACTTGAAGATGTTCACTTCGCGATGTTGATGTGTCCCAGCGGCGAGGAGGATGTCCAAGCGCAAATTC
AAGGAAGAGATGCTCCAGGTCATCGCGCCTGAGATCTACGGCCCTGCGGTGGTGAAGGAGGAAAGAAAGCCCCG
```

```
CAAAATCAAGCGGGTCAAAAAGGACAAAAAGGAAGAAGAAAGTGATGTGGACGGATTGGTGGAGTTTGTGCGCG
AGTTCGCCCCCCGGCGGCGCGTGCAGTGGCGCGGGCGGAAGGTGCAACCGGTGCTGAGACCCGGCACCACCGTG
GTCTTCACGCCCGGCGAGCGCTCCGGCACCGCTTCCAAGCGCTCCTACGACGAGGTGTACGGGGATGATGATAT
TCTGGAGCAGGCGGCCGAGCGCCTGGGCGAGTTTGCTTACGGCAAGCGCAGCCGTTCCGCACCGAAGGAAGAGG
CGGTGTCCATCCCGCTGGACCACGGCAACCCCACGCCGAGCCTCAAGCCCGTGACCTTGCAGCAGGTGCTGCCG
ACCGCGGCGCCGCGCCGGGGGTTCAAGCGCGAGGGCGAGGATCTGTACCCCACCATGCAGCTGATGGTGCCCAA
GCGCCAGAAGCTGGAAGACGTGCTGGAGACCATGAAGGTGGACCCGGACGTGCAGCCCGAGGTCAAGGTGCGGC
CCATCAAGCAGGTGGCCCCGGGCCTGGGCGTGCAGACCGTGGACATCAAGATTCCCACGGAGCCCATGGAAACG
CAGACCGAGCCCATGATCAAGCCCAGCACCAGCACCATGGAGGTGCAGACGGATCCCTGGATGCCATCGGCTCC
TAGTCGAAGACCCCGGCGCAAGTACGGCGCGGCCAGCCTGCTGATGCCCAACTACGCGCTGCATCCTTCCATCA
TCCCCACGCCGGGCTACCGCGGCACGCGCTTCTACCGCGGTCATACCAGCAGCCGCCGCCGCAAGACCACCACT
CGCCGCCGCCGTCGCCGCACCGCCGCTGCAACCACCCCTGCCGCCCTGGTGCGGAGAGTGTACCGCCGCGGCCG
CGCACCTCTGACCCTGCCGCGCGCGCGCTACCACCCGAGCATCGCCATTTAAACTTTCGCCtGCTTTGCAGATC
AATGGCCCTCACATGCCGCCTTCGCGTTCCCATTACGGGCTACCGAGGAAGAAAACCGCGCCGTAGAAGGCTGG
CGGGGAACGGGATGCGTCGCCACCACCACCGGCGGCGGCGCGCCATCAGCAAGCGGTTGGGGGGGAGGCTTCCTG
CCCGCGCTGATCCCCATCATCGCCGCGGCGATCGGGGCGATCCCCGGCATTGCTTCCGTGGCGGTGCAGGCCTC
TCAGCGCCACTGAGACACACTTGGAAACATCTTGTAATAAACCaATGGACTCTGACGCTCCTGGTCCTGTGATG
TGTTTTCGTAGACAGATGGAAGACATCAATTTTTCGTCCCTGGCTCCGCGACACGGCACGCGGCCGTTCATGGG
CACCTGGAGCGACATCGGCACCAGCCAACTGAACGGGGGCGCCTTCAATTGGAGCAGTCTCTGGAGCGGGCTTA
AGAATTTCGGGTCCACGCTTAAAACCTATGGCAGCAAGGCGTGGAACAGCACCACAGGGCAGGCGCTGAGGGAT
AAGCTGAAAGAGCAGAACTTCCAGCAGAAGGTGGTCGATGGGCTCGCCTCGGGCATCAACGGGGTGGTGGACCT
GGCCAACCAGGCCGTGCAGCGGCAGATCAACAGCCGCCTGGACCCGGTGCCGCCCGCCGGCTCCGTGGAGATGC
CGCAGGTGGAGGAGGAGCTGCCTCCCCTGGACAAGCGGGGCGAGAAGCGACCCCGCCCCGATGCGGAGGAGACG
CTGCTGACGCACACGGACGAGCCGCCCCCGTACGAGGAGGCGGTGAAACTGGGTCTGCCCACCACGCGGCCCAT
CGCGCCCCTGGCCACCGGGGTGCTGAAACCCGAAAAGCCCGCGACCCTGGACTTGCCTCCTCCCCAGCCTTCCC
GCCCCTCTACAGTGGCTAAGCCCCTGCCGCCGGTGGCCGTGGCCCGCGCGCGACCCGGGGGGCACCGCCCGCCCT
CATGCGAACTGGCAGAGCACTCTGAACAGCATCGTGGGTCTGGGAGTGCAGAGTGTGAAGCGCCGCCGCTGCTA
TTAAACCTACCGTAGCGCTTAACTTGCTTGTCTGTGTGTGTATGTATTATGTCGCCGCCGCCGCTGTCCACCAG
AAGGAGGAGTGAAGAGGCGCGTCGCCGAGTTGCAAGATGGCCACCCCATCGATGCTGCCCCAGTGGGCGTACAT
GCACATCGCCGGACAGGACGCTTCGGAGTACCTGAGTCCGGGTCTGGTGCAGTTTGCCCGCGCCACAGACACCT
ACTTCAGTCTGGGGAACAAGTTTAGGAACCCCACGGTGGCGCCCACGCACGATGTGACCACCGACCGCAGCCAG
CGGCTGACGCTGCGCTTCGTGCCCGTGGACCGCGAGGACAACACCTACTCGTACAAAGTGCGCTACACGCTGGC
CGTGGGCGACAACCGCGTGCTGGACATGGCCAGCACCTACTTTGACATCCGCGGCGTGCTGGATCGGGGCCCTA
GCTTCAAACCCTACTCCGGCACCGCCTACAACAGTCTGGCCCCCAAGGGAGCACCCAACACTTGTCAGTGGACA
TATAAAGCCGATGGTGAAACTGCCACAGAAAAAACCTATACATATGGAAATGCACCCGTGCAGGGCATTAACAT
CACAAAAGATGGTATTCAACTTGGAACTGACACCGATGATCAGCCAATCTACGCAGATAAAACCTATCAGCCTG
AACCTCAAGTGGGTGATGCTGAATGGCATGACATCACTGGTACTGATGAAAAGTATGGAGGCAGAGCTCTTAAG
CCTGATACCAAAATGAAGCCTTGTTATGGTTCTTTTGCCAAGCCTACTAATAAAGAAGGAGGTCAGGCAAATGT
GAAAACAGGAACAGGCACTACTAAAGAATATGACATAGACATGGCTTTCTTTGACAACAGAAGTGCGGCTGCTG
CTGGCCTAGCTCCAGAAATTGTTTTGTATACTGAAAATGTGGATTTGGAAACTCCAGATACCCATATTGTATAC
AAAGCAGGCACAGATGACAGCAGCTCTTCTATTAATTTGGGTCAGCAAGCCATGCCCAACAGACCTAACTACAT
TGGTTTCAGAGACAACTTTATCGGGCTCATGTACTACAACAGCACTGGCAATATGGGGGTGCTGGCCGGTCAGG
CTTCTCAGCTGAATGCTGTGGTTGACTTGCAAGACAGAAACACCGAGCTGTCCTACCAGCTCTTGCTTGACTCT
CTGGGTGACAGAACCCGGTATTTCAGTATGTGGAATCAGGCGGTGGACAGCTATGATCCTGATGTGCGCATTAT
TGAAAATCATGGTGTGGAGGATGAACTTCCCAACTATTGTTTCCCTCTGGATGCTGTTGGCAGAACAGATACTT
ATCAGGGAATTAAGGCTAATGGAACTGATCAAACCACATGGACCAAAGATGACAGTGTCAATGATGCTAATGAG
ATAGGCAAGGGTAATCCATTCGCCATGGAAATCAACATCCAAGCCAACCTGTGGAGGAACTTCCTCTACGCCAA
CGTGGCCCTGTACCTGCCCGACTCTTACAAGTACACGCCGGCCAATGTTACCCTGCCCACCAACACCAACACCT
ACGATTACATGAACGGCCGGGTGGTGGCGCCCTCGCTGGTGGACTCCTACATCAACATCGGGGCGCGCTGGTCG
CTGGATCCCATGGACAACGTGAACCCCTTCAACCACCACCGCAATGCGGGGCTGCGCTACCGCTCCATGCTCCT
GGGCAACGGGCGCTACGTGCCCTTCCACATCCAGGTGCCCCAGAAATTTTTCGCCATCAAGAGCCTCCTGCTCC
TGCCCGGGTCCTACACCTACGAGTGGAACTTCCGCAAGGACGTCAACATGATCCTGCAGAGCTCCCTCGGCAAC
GACCTGCGCACGGACGGGGCCTCCATCTCCTTCACCAGCATCAACCTCTACGCCACCTTCTTCCCCATGGCGCA
CAACACGGCCTCCACGCTCGAGGCCATGCTGCGCAACGACACCAACGACCAGTCCTTCAACGACTACCTCTCGG
CGGCCAACATGCTCTACCCCATCCCGGCCAACGCCACCAACGTGCCCATCTCCATCCCCTCGCGCAACTGGGCC
GCCTTCCGCGGCTGGTCCTTCACGCGTCTCAAGACCAAGGAGACGCCCTCGCTGGGCTCCGGGTTCGACCCCTA
CTTCGTCTACTCGGGCTCCATCCCCTACCTCGACGGCACCTTCTACCTCAACCACACCTTCAAGAAGGTCTCCA
TCACCTTCGACTCCTCCGTCAGCTGGCCCGGCAACGACCGGCTCCTGACGCCCAACGAGTTCGAAATCAAGCGC
ACCGTCGACGGCGAGGGCTACAACGTGGCCCAGTGCAACATGACCAAGGACTGGTTCCTGGTCCAGATGCTGGC
CCACTACAACATCGGCTACCAGGGCTTCTACGTGCCCGAGGGCTACAAGGACCGCATGTACTCCTTCTTCCGCA
ACTTCCAGCCCATGAGCCGCCAGGTGGTGGACGAGGTCAACTACAAGGACTACCAGGCCGTCACCCTGGCCTAC
CAGCACAACAACTCGGGCTTCGTCGGCTACCTCGCGCCCACCATGCGCCAGGGCCAGCCCTACCCCGCCAACTA
```

(continued)

```
CCCCTACCCGCTCATCGGCAAGAGCGCCGTCACCAGCGTCACCCAGAAAAAGTTCCTCTGCGACAGGGTCATGT
GGCGCATCCCCTTCTCCAGCAACTTCATGTCCATGGGCGCGCTCACCGACCTCGGCCAGAACATGCTCTATGCC
AACTCCGCCCACGCGCTAGACATGAATTTCGAAGTCGACCCCATGGATGAGTCCACCCTTCTCTATGTTGTCTT
CGAAGTCTTCGACGTCGTCCGAGTGCACCAGCCCCACCGCGGCGTCATCGAGGCCGTCTACCTGCGCACCCCCT
TCTCGGCCGGTAACGCCACCACCTAAGCTCTTGCTTCTTGCAAGCCATGGCCGCGGGCTCCGGCGAGCAGGAGC
TCAGGGCCATCATCCGCGACCTGGGCTGCGGGCCCTACTTCCTGGGCACCTTCGATAAGCGCTTCCCGGGATTC
ATGGCCCCGCACAAGCTGGCCTGCGCCATCGTCAACACGGCCGGCCGCGAGACCGGGGGCGAGCACTGGCTGGC
CTTCGCCTGGAACCCGCGCTCGAACACCTGCTACCTCTTCGACCCCTTCGGGTTCTCGGACGAGCGCCTCAAGC
AGATCTACCAGTTCGAGTACGAGGGCCTGCTGCGCCGCAGCGCCCTGGCCACCGAGGACCGCTGCGTCACCCTG
GAAAAGTCCACCCAGACCGTGCAGGGTCCGCGCTCGGCCGCCTGCGGGCTCTTCTGCTGCATGTTCCTGCACGC
CTTCGTGCACTGGCCCGACCGCCCCCATGGACAAGAACCCCACCATGAACTTGCTGACGGGGGTGCCCAACGGCA
TGCTCCAGTCGCCCCAGGTGGAACCCACCCTGCGCCGCAACCAGGAGGCGCTCTACCGCTTCCTCAACTCCCAC
TCCGCCTACTTTCGCTCCCACCGCGCGCGCATCGAGAAGGCCACCGCCTTCGACCGCATGAATCAAGACATGTA
AACCGTGTGTGTATGTTAAATGTCTTTAATAAACAGCACTTTCATGTTACACATGCATCTGAGATGATTTATTT
AGAAATCGAAAGGGTTCTGCCGGGTCTCGGCATGGCCCGCGGGCAGGGACACGTTGCGGAACTGGTACTTGGCC
AGCCACTTGAACTCGGGGATCAGCAGTTTGGGCAGCGGGGTGTCGGGGAAGGAGTCGGTCCACAGCTTCCGCGT
CAGTTGCAGGGCGCCCAGCAGGTCGGGCGCGGAGATCTTGAAATCGCAGTTGGGACCCGCGTTCTGCGCGCGGG
AGTTGCGGTACACGGGGTTGCAGCACTGGAACACCATCAGGGCCGGGTGCTTCACGCTCGCCAGCACCGTCGCG
TCGGTGATGCTCTCCACGTCGAGGTCCTCGGCGTTGGCCATCCCGAAGGGGGTCATCTTGCAGGTCTGCCTTCC
CATGGTGGGCACGCACCCGGGCTTGTGGTTGCAATCGCAGTGCAGGGGGATCAGCATCATCTGGGCCTGGTCGG
CGTTCATCCCCGGGTACATGGCCTTCATGAAAGCCTCCAATTGCCTGAACGCCTGCTGGGCCTTGGCTCCCTCG
GTGAAGAAGACCCCGCAGGACTTGCTAGAGAACTGGTTGGTGGCGCACCCGGCGTCGTGCACGCAGCAGCGCGC
GTCGTTGTTGGCCAGCTGCACCACGCTGCGCCCCCAGCGGTTCTGGGTGATCTTGGCCCGGTCGGGGTTCTCCT
TCAGCGCGCGCTGCCCGTTCTCGCTCGCCACATCCATCTCGATCATGTGCTCCTTCTGGATCATGGTGGTCCCG
TGCAGGCACCGCAGCTTGCCCTCGGCCTCGGTGCACCCGTGCAGCCACAGCGCGCACCCGGTGCACTCCCAGTT
CTTGTGGGCGATCTGGGAATGCGCGTGCACGAAGCCCTGCAGGAAGCGGCCCATCATGGTGGTCAGGGTCTTGT
TGCTAGTGAAGGTCAGCGGAATGCCGCGGTGCTCCTCGTTGATGTACAGGTGGCAGATGCGGCGGTACACCTCG
CCCTGCTCGGGCATCAGCTGGAAGTTGGCTTTCAGGTCGGTCTCCACGCGGTAGCGGTCCATCAGCATAGTCAT
GATTTCCATACCCTTCTCCCAGGCCGAGACGATGGGCAGGCTCATAGGGTTCTTCACCATCATCTTAGCGCTAG
CAGCCGCGGCCAGGGGGTCGCTCTCGTCCAGGGTCTCAAAGCTCCGCTTGCCGTCCTTCTCGGTGATCCGCACC
GGGGGGTAGCTGAAGCCCACGCCGCCAGCTCCTCCTCGGCCTGTCTTTCGTCCTCGCTGTCCTGGCTGACGTC
CTGCAGGACCACATGCTTGGTCTTGCGGGGTTTCTTCTTGGGCGGCAGCGGCGGCGGAGATGTTGGAGATGGCG
AGGGGGAGCGCGAGTTCTCGCTCACCACTACTATCTCTTCCTCTTCTTGGTCCGAGGCCACGCGGCGGTAGGTA
TGTCTCTTCGGGGGGCAGAGGCGGAGGCGACGGGCTCTCGCCGCCGCGACTTGGCGGATGGCTGGCAGAGCCCCT
TCCGCGTTCGGGGGTGCGCTCCCGGCGGCGCTCTGACTGACTTCCTCCGCGGCCGGCCATTGTGTTCTCCTAGG
GAGGAACAACAAGCATGGAGACTCAGCCATCGCCAACCTCGCCATCTGCCCCCACCGCCGACGAGAAGCAGCAG
CAGCAGAATGAAAGCTTAACCGCCCCGCCGCCCAGCCCCGCCACCTCCGACGCGGCCGTCCCAGACATGCAAGA
GATGGAGGAATCCATCGAGATTGACCTGGGCTATGTGACGCCCGCGGAGCACGAGGAGGAGCTGGCAGTGCGCT
TTTCACAAGAAGAGATACACCAAGAACAGCCAGAGCAGGAAGCAGAGAATGAGCAGAGTCAGGCTGGGCTCGAG
CATGACGGCGACTACCTCCACCTGAGCGGGGGGGAGGACGCGCTCATCAAGCATCTGGCCCGGCAGGCCACCAT
CGTCAAGGATGCGCTGCTCGACCGCACCGAGGTGCCCCTCAGCGTGGAGGAGCTCAGCCGCGCCTACGAGTTGA
ACCTCTTCTCGCCGCGCGTGCCCCCCAAGCGCCAGCCCAATGGCACCTGCGAGCCCAACCCGCGCCTCAACTTC
TACCCGGTCTTCGCGGTGCCCGAGGCCCTGGCCACCTACCACATCTTTTTCAAGAACCAAAAGATCCCCGTCTC
CTGCCGCGCCAACCGCACCCGCGCCGACGCCCTTTTCAACCTGGGTCCCGGCGCCCGCCTACCTGATATCGCCT
CCTTGGAAGAGGTTCCCAAGATCTTCGAGGGTCTGGGCAGCGACGAGACTCGGGCCGCGAACGCTCTGCAAGGA
GAAGGAGGAGAGCATGAGCACCACAGCGCCCTGGTCGAGTTGGAAGGCGACAACGCGCGGCTGGCGGTGCTCAA
ACGCACGGTCGAGCTGACCCATTTCGCCTACCCGGCTCTGAACCTGCCCCCCAAAGTCATGAGCGCGGTCATGG
ACCAGGTGCTCATCAAGCGCGCGTCGCCCATCTCCGAGGACGAGGGCATGCAAGACTCCGAGGAGGGCAAGCCC
GTGGTCAGCGACGAGCAGCTGGCCCGGTGGCTGGGTCCTAATGCTAGTCCCCAGAGTTTGGAAGAGCGGCGCAA
ACTCATGATGGCCGTGGTCCTGGTGACCGTGGAGCTGGAGTGCCTGCGCCGCTTCTTCGCCGACGCGGAGACCC
TGCGCAAGGTCGAGGAGAACCTGCACTACCTCTTCAGGCACGGGTTCGTGCGCCAGGCCTGCAAGATCTCCAAC
GTGGAGCTGACCAACCTGGTCTCCTACATGGGCATCTTGCACGAGAACCGCCTGGGGCAGAACGTGCTGCACAC
CACCCTGCGCGGGGAGGCCCGGCGCGACTACATCCGCGACTGCGTCTACCTCTACCTCTGCCACACCTGGCAGA
CGGGCATGGGCGTGTGGCAGCAGTGTCTGGAGGAGCAGAACCTGAAAGAGCTCTGCAAGCTCCTGCAGAAGAAC
CTCAAGGGTCTGTGGACCGGGTTCGACGAGCGCACCACCGCCTCGGACCTGGCCGACCTCATTTTCCCCGAGCG
CCTCAGGCTGACGCTGCGCAACGGCCTGCCCGACTTTATGAGCCAAAGCATGTTGCAAAACTTTCGCTCTTTCA
TCCTCGAACGCTCCGGAATCCTGCCCGCCACCTGCTCCGCGCTGCCCTCGGACTTCGTGCCGCTGACCTTCCGC
GAGTGCCCCCCGCCGCTGTGGAGCCACTGCTACCTGCTGCGCCTGGCCAACTACCTGGCCTACCACTCGGACGT
GATCGAGGACGTCAGCGGCGAGGGCCTGCTCGAGTGCCACTGCCGCTGCAACCTCTGCACGCCGCACCGCTCCC
TGGCCTGCAACCCCCAGCTGCTGAGCGAGACCCAGATCATCGGCACCTTCGAGTTGCAAGGGCCCAGCGAAGGC
GAGGGTTCAGCCGCCAAGGGGGGTCTGAAACTCACCCCGGGGCTGTGGACCTCGGCCTACTTGCGCAAGTTCGT
GCCCGAGGACTACCATCCCTTCGAGATCAGGTTCTACGAGGACCAATCCCATCCGCCCAAGGCCGAGCTGTCGG
```

```
CCTGCGTCATCACCCAGGGGGCGATCCTGGCCCAATTGCAAGCCATCCAGAAATCCCGCCAAGAATTCTTGCTG
AAAAAGGGGCCGCGGGGTCTACCTCGACCCCCAGACCGGTGAGGAGCTCAACCCCGGCTTCCCCCAGGATGCCCC
GAGGAAACAAGAAGCTGAAAGTGGAGCTGCCGCCCGTGGAGGATTTGGAGGAAGACTGGGAGAACAGCAGTCAG
GCAGAGGAGGAGGAGATGGAGGAAGACTGGGACAGCACTCAGGCAGAGGAGGACAGCCTGCAAGACAGTCTGGA
GGAAGACGAGGAGGAGGCAGAGGAGGAGGTGGAAGAAGCAGCCGCCGCCAGACCGTCGTCCTCGGCGGGGGAGA
AAGCAAGCAGCACGGATACCATCTCCGCTCCGGGTCGGGGTCCCGCTCGACCACACAGTAGATGGGACGAGACC
GGACGATTCCCGAACCCCACCACCCAGACCGGTAAGAAGGAGCGGCAGGGATACAAGTCCTGGCGGGGGCACAA
AAACGCCATCGTCTCCTGCTTGCAGGCCTGCGGGGGCAACATCTCCTTCACCCGGCGCTACCTGCTCTTCCACC
GCGGGGTGAACTTTCCCCGCAACATCTTGCATTACTACCGTCACCTCCACAGCCCCTACTACTTCCAAGAAGAG
GCAGCAGCAGCAGAAAAAGACCAGCAGAAACCAGCAGCTAGAAAATCCACAGCGGCGGCAGCAGGTGGACTGA
GGATCGCGGCGAACGAGCCGGCGCAAACCCGGGAGCTGAGGAACCGGATCTTTCCCACCCTCTATGCCATCTTC
CAGCAGAGTCGGGGGCAGGAGCAGGAACTGAAAGTCAAGAACCGTTCTCTGCGCTCGCTCACCCGCAGTTGTCT
GTATCACAAGAGCGAAGACCAACTTCAGCGCACTCTCGAGGACGCCGAGGCTCTCTTCAACAAGTACTGCGCGC
TCACTCTTAAAGAGTAGCCCGCGCCCGCCCAGTCGCAGAAAAAGGCGGGAATTACGTCACCTGTGCCCTTCGCC
CTAGCCGCCTCCACCCATCATCATGAGCAAAGAGATTCCCACGCCTTACATGTGGAGCTACCAGCCCCAGATGG
GCCTGGCCGCCGGTGCCGCCCAGGACTACTCCACCCGCATGAATTGGCTCAGCGCCGGGCCCGCGATGATCTCA
CGGGTGAATGACATCCGCGCCCACCGAAACCAGATACTCCTAGAACAGTCAGCGCTCACCGCCACGCCCCGCAA
TCACCTCAATCCGCGTAATTGGCCCGCCGCCCTGGTGTACCAGGAAATTCCCCAGCCCACGACCGTACTACTTC
CGCGAGACGCCCAGGCCGAAGTCCAGCTGACTAACTCAGGTGTCCAGCTGGCGGGCGGCGCCACCCTGTGTCGT
CACCGCCCCGCTCAGGGTATAAAGCGGCTGGTGATCCGGGGCAGAGGCACACAGCTCAACGACGAGGTGGTGAG
CTCTTCGCTGGGTCTGCGACCTGACGGAGTCTTCCAACTCGCCGGATCGGGGAGATCTTCCTTCACGCCTCGTC
AGGCCGTCCTGACTTTGGAGAGTTCGTCCTCGCAGCCCCGCTCGGGTGGCATCGGCACTCTCCAGTTCGTGGAG
GAGTTCACTCCCTCGGTCTACTTCAACCCCTTCTCCGGCTCCCCGGCCACTACCCGGACGAGTTCATCCCGAA
CTTCGACGCCATCAGCGAGTCGGTGGACGGCTACGATTGAATGTCCCATGGTGGCGCAGCTGACCTAGCTCGGC
TTCGACACCTGGACCACTGCCGCCGCTTCCGCTGCTTCGCTCGGGATCTCGCCGAGTTTGCCTACTTTGAGCTG
CCCGAGGAGCACCCTCAGGGCCCGGCCCACGGAGTGCGGATCGTCGTCGAAGGGGGCCTCGACTCCCACCTGCT
TCGGATCTTCAGCCAGCGTCCGATCCTGGTCGAGCGCGAGCAAGGACAGACCCTTCTGACTCTGTACTGCATCT
GCAACCACCCCGGCCTGCATGAAAGTCTTTGTTGTCTGCTGTGTACTGAGTATAATAAAAGCTGAGATCAGCGA
CTACTCCGGACTTCCGTGTGTTCCTGAATCCATCAACCAGTCTTTGTTCTTCACCGGGAACGAGACCGAGCTCC
AGCTCCAGTGTAAGCCCCACAAGAAGTACCTCACCTGGCTGTTCCAGGGCTCCCGATCGCCGTTGTCAACCAC
TGCGACAACGACGGAGTCCTGCTGAGCGGCCCTGCCAACCTTACTTTTTCCACCCGCAGAAGCAAGCTCCAGCT
CTTCCAACCCTTCCTCCCCGGGACCTATCAGTGCGTCTCGGGACCCTGCCATCACACCTTCCACCTGATCCCGA
ATACCACAGCGTCGCTCCCCGCTACTAACAACCAAACTAACCTCCACCAACGCCACCGTCGCGACCTTTCTGAA
TCTAATACTACCACCCACACCGGAGGTGAGCTCCGAGGTCAACCAACCTCTGGGATTTACTACGGCCCCTGGGA
GGTGGTTGGGTTAATAGCGCTAGGCCTAGTTGCGGGTGGGCTTTTGGTTCTCTGCTACCTATACCTCCCTTGCT
GTTCGTACTTAGTGGTGCTGTGTTGCTGGTTTAAGAAATGGGGAAGATCACCCTAGTGAGCTGCGGTGCGCTGG
TGGCGGTGTTGCTTTCGATTGTGGGACTGGGCGGTGCGGCTGTAGTGAAGGAGAAGGCCGATCCCTGCTTGCAT
TTCAATCCCAACAAATGCCAGCTGAGTTTTCAGCCGATGGCAATCGGTGCGCGGTACTGATCAAGTGCGGATG
GGAATGCGAGAACGTGAGAATCGAGTACAATAACAAGACTCGGAACAATACTCTCGCGTCCGTGTGGCAGCCCG
GGGACCCCGAGTGGTACACCGTCTCTGTCCCCGGTGCTGACGGCTCCCCGCGCACCGTGAATAATACTTTCATT
TTTGCGCACATGTGCGACACGGTCATGTGGATGAGCAAGCAGTACGATATGTGGCCCCCCACGAAGGAGAACAT
CGTGGTCTTCTCCATCGCTTACAGCCTGTGCACGGCGCTAATCACCGCTATCGTGTGCCTGAGCATTCACATGC
TCATCGCTATTCGCCCCAGAAATAATGCCGAAAAGAAAAACAGCCATAACGTTTTTTTTCACACCTTTTTCAG
ACCATGGCCTCTGTTAAATTTTTGCTTTTATTTGCCAGTCTCATTGCCGTCATTCATGGAATGAGTAATGAGAA
AATTACTATTTACACTGGCACTAATCACACATTGAAAGGTCCAGAAAAAGCCACAGAAGTTTCATGGTATTGTT
ATTTTAATGAATCAGATGTATCTACTGAACTCTGTGGAAACAATAACAAAAAAAATGAGAGCATTACTCTCATC
AAGTTTCAATGTGGATCTGACTTAACCCTAATTAACATCACTAGAGACTATGTAGGTATGTATTATGGAACTAC
AGCAGGCATTTCGGACATGGAATTTTATCAAGTTTCTGTGTCTGAACCCACCACGCCTAGAATGACCACAACCA
CAAAAACTACACCTGTTACCACTATGCAGCTCACTACCAATAACATTTTTGCCATGCGTCAAATGGTCAACAAT
AGCACTCAACCCACCCCACCCAGTGAGGAAATTCCCAAATCCATGATTGGCATTATTGTTGCTGTAGTGGTGTG
CATGTTGATCATCGCCTTGTGCATGGTGTACTATGCCTTCTGCTACAGAAAGCACAGACTGAACGACAAGCTGG
AACACTTACTAAGTGTTGAATTTTAATTTTTTAGAACCATGAAGATCCTAGGCCTTTTAATTTTTTCTATCATT
ACCTCTGCTCTATGCAATTCTGACAATGAGGACGTTACTGTCGTTGTCGGATCAAATTATACACTGAAAGGTCC
AGCGAAGGGTATGCTTTCGTGGTATTGCTATTTTGGATCTGACACTACAGAAACTGAATTATGCAATCTTAAGA
ATGGCAAAATTCAAAATTCTAAAATTAACAATTATATATGCAATGGTACTGATCTGATACTCCTCAATATCACG
AAATCATATGCTGGCAGTTACACCTGCCCTGGAGATGATGCTGACAGTATGATTTTTTACAAAGTAACTGTTGT
TGATCCCACTACTCCACCTCCACCCACCACAACTACTCACACCACACACACAGATCAAACCGCAGCAGAGGAGG
CAGCAAAGTTAGCCTTGCAGGTCCAAGACAGTTCATTTGTTGGCATTACCCCTACACCTGATCAGCGGTGTCCG
GGGCTGCTAGTCAGCGGCATTGTCGGTGTGCTTTCGGGATTAGCAGTCATAATCATCTGCATGTTCATTTTTGC
TTGCTGCTATAGAAGGCTTTACCGACAAAAATCAGACCCACTGCTGAACCTCTATGTTTAATTTTTTCCAGAGT
CATGAAGGCAGTTAGCGCTCTAGTTTTTTGTTCTTTGATTGGCATTGTTTTTTGCAATCCTATTCCTAAAGTTA
GCTTTATTAAAGATGTGAATGTTACTGAGGGGGGCAATGTGACACTGGTAGGTGTAGAGGGTGCTGAAAACACC
```

(continued)

```
ACCTGGACAAAATACCACCTCAATGGGTGGAAAGATATTTGCAATTGGAGTGTATTAGTTTATACATGTGAGGG
AGTTAATCTTACCATTGTCAATGCCACCTCAGCTCAAAATGGTAGAATTCAAGGACAAAGTGTCAGTGTATCTA
ATGGGTATTTTACCCAACATACTTTTATCTATGACGTTAAAGTCATACCACTGCCTACGCCTAGCCCACCTAGC
ACTACCACACAGACAACCCACACTACACAGACAACCACATACAGTACATTAAATCAGCCTACCACCACTACAGC
AGCAGAGGTTGCCAGCTCGTCTGGGGTCCGAGTGGCATTTTTGATGTGGGCCCCATCTAGCAGTCCCACTGCTA
GTACCAATGAGCAGACTACTGAATTTTTGTCCACTGTCGAGAGCCACACCACAGCTACCTCCAGTGCCTTCTCT
AGCACCGCCAATCTCTCCTCGCTTTCCTCTACACCAATCAGTCCCGCTACTACTCCTAGCCCCGCTCCTCTTCC
CACTCCCCTGAAGCAAACAGACGGCGGCATGCAATGGCAGATCACCCTGCTCATTGTGATCGGGTTGGTCATCC
TGGCCGTGTTGCTCTACTACATCTTCTGCCGCCGCATTCCCAACGCGCACCGCAAGCCGGTCTACAAGCCCATC
ATTGTCGGGCAGCCGGAGCCGCTTCAGGTGGAAGGGGGTCTAAGGAATCTTCTCTTCTCTTTTACAGTATGGTG
ATTGAACTATGATTCCTAGACAATTCTTGATCACTATTCTTATCTGCCTCCTCCAAGTCTGTGCCACCCTCGCT
CTGGTGGCCAACGCCAGTCCAGACTGTATTGGGCCCTTCGCCTCCTACGTGCTCTTTGCCTTCACCACCTGCAT
CTGCTGCTGTAGCATAGTCTGCCTGCTTATCACCTTCTTCCAGTTCATTGACTGGATCTTTGTGCGCATCGCCT
ACCTGCGCCACCACCCCAGTACCGCGACCAGCGAGTGGCGCGGCTGCTCAGGCTCCTCTGATAAGCATGCGGG
CTCTGCTACTTCTCGCGCTTCTGCTGTTAGTGCTCCCCCGTCCCGTCGACCCCCGGTCCCCCACCCAGTCCCCC
GAGGAGGTCCGCAAATGCAAATTCCAAGAACCCTGGAAATTCCTCAAATGCTACCGCCAAAAATCAGACATGCA
TCCCAGCTGGATCATGATCATTGGGATCGTGAACATTCTGGCCTGCACCCTCATCTCCTTTGTGATTTACCCCT
GCTTTGACTTTGGTTGGAACTCGCCAGAGGCGCTCTATCTCCCGCCTGAACCTGACACACCACCACAGCAACCT
CAGGCACACGCACTACCACCACTACAGCCTAGGCCACAATACATGCCCATATTAGACTATGAGGCCGAGCCACA
GCGACCCATGCTCCCCGCTATTAGTTACTTCAATCTAACCGGCGGAGATGACTGACCCACTGGCCAACAACAAC
GTCAACGACCTTCTCCTGGACATGGACGGCCGCGCCTCGGAGCAGCGACTCGCCCAACTTCGCATTCGCCAGCA
GCAGGAGAGAGCCGTCAAGGAGCTGCAGGATGCGGTGGCCATCCACCAGTGCAAGAGAGGCATCTTCTGCCTGG
TGAAACAGGCCAAGATCTCCTACGAGGTCACTCCAAACGACCATCGCCTCTCCTACGAGCTCCTGCAGCAGCGC
CAGAAGTTCACCTGCCTGGTCGGAGTCAACCCCATCGTCATCACCCAGCAGTCTGGCGATACCAAGGGGTGCAT
CCACTGCTCCTGCGACTCCCCCGACTGCGTCCACACTCTGATCAAGACCCTCTGCGGCCTCCGCGACCTCCTCC
CCATGAACTAATCACCCCCTTATCCAGTGAAATAAAGATCATATTGATGATGATTTTACAGAAATAAAAAATAA
TCATTTGATTTGAAATAAAGATACAATCATATTGATGATTTGAGTTTAACAAAAAAATAAAGAATCACTTACTT
GAAATCTGATACCAGGTCTCTGTCCATGTTTTCTGCCAACACCACTTCACTCCCCTCTTCCCAGCTCTGGTACT
GCAGGCCCCGGCGGGCTGCAAACTTCCTCCACACGCTGAAGGGGATGTCAAATTCCTCCTGTCCCTCAATCTTC
ATTTTATCTTCTATCAGATGTCCAAAAAGCGCGTCCGGGTGGATGATGACTTCGACCCCGTCTACCCCTACGAT
GCAGACAACGCACCGACCGTGCCCTTCATCAACCCCCCCTTCGTCTCTTCAGATGGATTCCAAGAGAAGCCCCT
GGGGGGTGTTGTCCCTGCGACTGGCCGACCCCGTCACCACCAAGAACGGGGAAATCACCCTCAAGCTGGGAGAGG
GGGTGGACCTCGATTCCTCGGGAAAACTCATCTCCAACACGGCCACCAAGGCCGCCGCCCCTCTCAGTTTTTCC
AACAACACCATTTCCCTTAACATGGATCACCCCTTTTACACTAAAGATGGAAAATTATCCTTACAAGTTTCTCC
ACCATTAAATATACTGAGAACAAGCATTCTAAACACACTAGCTTTAGGTTTTGGATCAGGTTTAGGACTCCGTG
GCTCTGCCTTGGCAGTACAGTTAGTCTCTCCACTTACATTTGATACTGATGGAAACATAAAGCTTACCTTAGAC
AGAGGTTTGCATGTTACAACAGGAGATGCAATTGAAAGCAACATAAGCTGGGCTAAAGGTTTAAAATTTGAAGA
TGGAGCCATAGCAACCAACATTGGAAATGGGTTAGAGTTTGGAAGCAGTAGTACAGAAACAGGTGTTGATGATG
CTTACCCAATCCAAGTTAAACTTGGATCTGGCCTTAGCTTTGACAGTACAGGAGCCATAATGGCTGGTAACAAA
GAAGACGATAAACTCACTTTGTGGACAACACCTGATCCATCACCAAACTGTCAAATACTCGCAGAAAATGATGC
AAAACTAACACTTTGCTTGACTAAATGTGGTAGTCAAATACTGGCCACTGTGTCAGTCTTAGTTGTAGGAAGTG
GAAACCTAAACCCCATTACTGGCACCGTAAGCAGTGCTCAGGTGTTTCTACGTTTTGATGCAAACGGTGTTCTT
TTAACAGAACATTCTACACTAAAAAAATACTGGGGGTATAGGCAGGGAGATAGCATAGATGGCACTCCATATAC
CAATGCTGTAGGATTCATGCCCAATTTAAAAGCTTATCCAAAGTCACAAAGTTCTACTACTAAAAATAATATAG
TAGGGCAAGTATACATGAATGGAGATGTTTCAAAACCTATGCTTCTCACTATAACCCTCAATGGTACTGATGAC
AGCAACAGTACATATTCAATGTCATTTTCATACACCTGGACTAATGGAAGCTATGTTGGAGCAACATTTGGGGC
TAACTCTTATACCTTCTCATACATCGCCCAAGAATGAACACTGTATCCCACCCTGCATGCCAACCCTTCCCACC
CCACTCTGTGGAACAAACTCTGAAACACAAAATAAAATAAAGTTCAAGTGTTTTATTGATTCAACAGTTTTACA
GGATTCGAGCAGTTATTTTTCCTCCACCCTCCCAGGACATGGAATACACCACCCTCTCCCCCCGCACAGCCTTG
AACATCTGAATGCCATTGGTGATGGACATGCTTTTGGTCTCCACGTTCCACACAGTTTCAGAGCGAGCCAGTCT
CGGGTCGGTCAGGGAGATGAAACCCTCCGGGCACTCCCGCATCTGCACCTCACAGCTCAACAGCTGAGGATTGT
CCTCGGTGGTCGGGATCACGGTTATCTGGAAGAAGCAGAAGAGCGGCGGTGGGAATCATAGTCCGCGAACGGGA
TCGGCCGGTGGTGTCGCATCAGGCCCCGCAGCAGTCGCTGCCGCCGCCGCTCCGTCAAGCTGCTGCTCAGGGGG
TCCGGGTCCAGGGACTCCCTCAGCATGATGCCCACGGCCCTCAGCATCAGTCGTCTGGTGCGGCGGGCGCAGCA
GCGCATGCGGATCTCGCTCAGGTCGCTGCAGTACGTGCAACACAGAACCACCAGGTTGTTCAACAGTCCATAGT
TCAACACGCTCCAGCCGAAACTCATCGCGGGAAGGATGCTACCCACGTGGCCGTCGTACCAGATCCTCAGGTAA
ATCAAGTGGTGCCCCCTCCAGAACACGCTGCCCACGTACATGATCTCCTTGGGCATGTGGCGGTTCACCACCTC
CCGGTACCACATCACCCTCTGGTTGAACATGCAGCCCCGGATGATCCTGCGGAACCACAGGGCCAGCACCGCCC
CGCCCGCCATGCAGCGAAGAGACCCCGGGTCCCGGCAATGGCAATGGAGGACCCACCGCTCGTACCCGTGGATC
ATCTGGGAGCTGAACAAGTCTATGTTGGCACAGCACAGGCATATGCTCATGCATCTCTTCAGCACTCTCAACTC
CTCGGGGGTCAAAACCATATCCCAGGGCACGGGGAACTCTTGCAGGACAGCGAACCCCGCAGAACAGGGCAATC
CTCGCACAGAACTTACATTGTGCATGGACAGGGTATCGCAATCAGGCAGCACCGGGTGATCCTCCACCAGAGAA
```

```
GCGCGGGTCTCGGTCTCCTCACAGCGTGGTAAGGGGGCCGGCCGATACGGGTGATGGCGGGACGCGGCTGATCG
TGTTCGCGACCGTGTCATGATGCAGTTGCTTTCGGACATTTTCGTACTTGCTGTAGCAGAACCTGGTCCGGGCG
CTGCACACCGATCGCCGGCGGCGGTCTCGGCGCTTGGAACGCTCGGTGTTGAAATTGTAAAACAGCCACTCTCT
CAGACCGTGCAGCAGATCTAGGGCCTCAGGAGTGATGAAGATCCCATCATGCCTGATGGCTCTGATCACATCGA
CCACCGTGGAATGGGCCAGACCCAGCCAGATGATGCAATTTTGTTGGGTTTCGGTGACGGCGGGGGAGGGAAGA
ACAGGAAGAACCATGATTAACTTTTAATCCAAACGGTCTCGGAGTACTTCAAAATGAAGATCGCGGAGATGGCA
CCTCTCGCCCCCGCTGTGTTGGTGGAAAATAACAGCCAGGTCAAAGGTGATACGGTTCTCGAGATGTTCCACGG
TGGCTTCCAGCAAAGCCTCCACGCGCACATCCAGAAACAAGACAATAGCGAAAGCGGGAGGGTTCTCTAATTCC
TCAATCATCATGTTACACTCCTGCACCATCCCCAGATAATTTTCATTTTTCCAGCCTTGAATGATTCGAACTAG
TTCcTGAGGTAAATCCAAGCCAGCCATGATAAAGAGCTCGCGCAGAGCGCCCTCCACCGGCATTCTTAAGCACA
CCCTCATAATTCCAAGATATTCTGCTCCTGGTTCACCTGCAGCAGATTGACAAGCGGAATATCAAAATCTCTGC
CGCGATCCCTGAGCTCCTCCCTCAGCAATAACTGTAAGTACTCTTTCATATCCTCTCCGAAATTTTTAGCCATA
GGACCACCAGGAATAAGATTAGGGCAAGCCACAGTACAGATAAACCGAAGTCCTCCCCAGTGAGCATTGCCAAA
TGCAAGACTGCTATAAGCATGCTGGCTAGACCCGGTGATATCTTCCAGATAACTGGACAGAAAATCGCCCAGGC
AATTTTTAAGAAAATCAACAAAAGAAAAATCCTCCAGGTGGACGTTTAGAGCCTCGGGAACAACGATGAAGTAA
ATGCAAGCGGTGCGTTCCAGCATGGTTAGTTAGCTGATCTGTAGAAAAAACAAAAATGAACATTAAACCATGCT
AGCCTGGCGAACAGGTGGGTAAATCGTTCTCTCCAGCACCAGGCAGGCCACGGGGTCTCCGGCGCGACCCTCGT
AAAAATTGTCGCTATGATTGAAAACCATCACAGAGAGACGTTCCCGGTGGCCGGCGTGAATGATTCGACAAGAT
GAATACACCCCCGGAACATTGGCGTCCGCGAGTGAAAAAAAGCGCCCGAGGAAGCAATAAGGCACTACAATGCT
CAGTCTCAAGTCCAGCAAAGCGATGCCATGCGGATGAAGCACAAAATTCTCAGGTGCGTACAAAATGTAATTAC
TCCCCTCCTGCACAGGCAGCAAAGCCCCCGATCCCTCCAGGTACACATACAAAGCCTCAGCGTCCATAGCTTAC
CGAGCAGCAGCACACAACAGGCGCAAGAGTCAGAGAAAGGCTGAGCTCTAACCTGTCCACCCGCTCTCTGCTCA
ATATATAGCCCAGATCTACACTGACGTAAAGGCCAAAGTCTAAAAATACCCGCCAAATAATCACACACGCCCAG
CACACGCCCAGAAACCGGTGACACACTCAAAAAAATACGCGCACTTCCTCAAACGCCCAAAACTGCCGTCATTT
CCGGGTTCCCACGCTACGTCATCAAAACACGACTTTCAAATTCCGTCGACCGTTAAAAACGTCACCCGCCCCGC
CCCTAACGGTCGCCCGTCTCTCAGCCAATCAGCGCCCCGCATCCCCAAATTCAAACACCTCATTTGCATATTAA
CGCGCACAAAAAGTTTGAGGTATATTATTGATGATGG
```

ATCC VR-594 C68 (SEQ ID NO:10); Indepentdently sequenced; Full-Length C68

(continued)

```
CCATCTTCAATAATATACCTCAAACTTTTTGTGCGCGTTAATATGCAAATGAGGCGTTTGAATTTGGGGAGGAA
GGGCGGTGATTGGTCGAGGGATGAGCGACCGTTAGGGGCGGGGCGAGTGACGTTTTGATGACGTGGTTGCGAGG
AGGAGCCAGTTTGCAAGTTCTCGTGGGAAAAGTGACGTCAAACGAGGTGTGGTTTGAACACGGAAATACTCAAT
TTTCCCGCGCTCTCTGACAGGAAATGAGGTGTTTCTGGGCGGATGCAAGTGAAAACGGGCCATTTTCGCGCGAA
AACTGAATGAGGAAGTGAAAATCTGAGTAATTTCGCGTTTATGGCAGGGAGGAGTATTTGCCGAGGGCCGAGTA
GACTTTGACCGATTACGTGGGGGTTTCGATTACCGTGTTTTTCACCTAAATTTCCGCGTACGGTGTCAAAGTCC
GGTGTTTTTACGTAGGTGTCAGCTGATCGCCAGGGTATTTAAACCTGCGCTCTCCAGTCAAGAGGCCACTCTTG
AGTGCCAGCGAGAAGAGTTTTCTCCTCCGCGCCGCGAGTCAGATCTACACTTTGAAAGATGAGGCACCTGAGAG
ACCTGCCCGATGAGAAAATCATCATCGCTTCCGGGAACGAGATTCTGGAACTGGTGGTAAATGCCATGATGGGC
GACGACCCTCCGGAGCCCCCCACCCCATTTGAGACACCTTCGCTGCACGATTTGTATGATCTGGAGGTGGATGT
GCCCGAGGACGATCCCAATGAGGAGGCGGTAAATGATTTTTTTAGCGATGCCGCGCTGCTAGCTGCCGAGGAGG
CTTCGAGCTCTAGCTCAGACAGCGACTCTTCACTGCATACCCCTAGACCCGGCAGAGGTGAGAAAAAGATCCCC
GAGCTTAAAGGGGAAGAGATGGACTTGCGCTGCTATGAGGAATGCTTGCCCCCGAGCGATGATGAGGACGAGCA
GGCGATCCAGAACGCAGCGAGCCAGGGAGTGCAAGCCGCCAGCGAGAGCTTTGCGCTGGACTGCCCGCCTCTGC
CCGGACACGGCTGTAAGTCTTGTGAATTTCATCGCATGAATACTGGAGATAAAGCTGTGTTGTGTGCACTTTGC
TATATGAGAGCTTACAACCATTGTGTTTACAGTAAGTGTGATTAAGTTGAACTTTAGAGGGAGGCAGAGAGCAG
GGTGACTGGGCGATGACTGGTTTATTTATGTATATATGTTCTTTATATAGGTCCCGTCTCTGACGCAGATGATG
AGACCCCCACTACAAAGTCCACTTCGTCACCCCCAGAAATTGGCACATCTCCACCTGAGAATATTGTTAGACCA
GTTCCTGTTAGAGCCACTGGGAGGAGAGCAGCTGTGGAATGTTTGGATGACTTGCTACAGGGTGGGGTTGAACC
TTTGGACTTGTGTACCCGGAAACGCCCCAGGCACTAAGTGCCACACATGTGTGTTTACTTGAGGTGATGTCAGT
ATTTATAGGGTGTGGAGTGCAATAAAAAATGTGTTGACTTTAAGTGCGTGGTTTATGACTCAGGGGTGGGGACT
GTGAGTATATAAGCAGGTGCAGACCTGTGTGGTTAGCTCAGAGCGGCATGGAGATTTGGACGGTCTTGGAAGAC
TTTCACAAGACTAGACAGCTGCTAGAGAACGCCTCGAACGGAGTCTCTTACCTGTGGAGATTCTGCTTCGGTGG
CGACCTAGCTAGGCTAGTCTACAGGGCCAAACAGGATTATAGTGAACAATTTGAGGTTATTTTGAGAGAGTGTT
CTGGTCTTTTTGACGCTCTTAACTTGGGCCATCAGTCTCACTTTAACCAGAGGATTTCGAGAGCCCTTGATTTT
ACTACTCCTGGCAGAACCACTGCAGCAGTAGCCTTTTTTGCTTTTATTCTTGACAAATGGAGTCAAGAAACCCA
TTTCAGCAGGGATTACCAGCTGGATTTCTTAGCAGTAGCTTTGTGGAGAACATGGAAGTGCCAGCGCCTGAATG
CAATCTCCGGCTACTTGCCGGTACAGCCGCTAGACACTCTGAGGATCCTGAATCTCCAGGAGAGTCCCAGGGCA
CGCCAACGTCGCCAGCAGCAGCAGCAGGAGGAGGATCAAGAAGAGAACCCGAGAGCCGGCCTGGACCCTCCGGC
GGAGGAGGAGGAGTAGCTGACCTGTTTCCTGAACTGCGCCGGGTGCTGACTAGGTCTTCGAGTGGTCGGGAGAG
GGGGATTAAGCGGGAGAGGCATGATGAGACTAATCACAGAACTGAACTGACTGTGGGTCTGATGAGTCGCAAGC
GCCCAGAAACAGTGTGGTGGCATGAGGTGCAGTCGACTGGCACAGATGAGGTGTCGGTGATGCATGAGAGGTTT
TCTCTAGAACAAGTCAAGACTTGTTGGTTAGAGCCTGAGGATGATTGGGAGGTAGCCATCAGGAATTATGCCAA
GCTGGCTCTGAGGCCAGACAAGAAGTACAAGATTACTAAGCTGATAAATATCAGAAATGCCTGCTACATCTCAG
```

(continued)

```
GGAATGGGGCTGAAGTGGAGATCTGTCTCCAGGAAAGGGTGGCTTTCAGATGCTGCATGATGAATATGTACCCG
GGAGTGGTGGGCATGGATGGGGTTACCTTTATGAACATGAGGTTCAGGGGAGATGGGTATAATGGCACGGTCTT
TATGGCCAATACCAAGCTGACAGTCCATGGCTGCTCCTTCTTTGGGTTTAATAACACCTGCATCGAGGCCTGGG
GTCAGGTCGGTGTGAGGGGCTGCAGTTTTTCAGCCAACTGGATGGGGGTCGTGGGCAGGACCAAGAGTATGCTG
TCCGTGAAGAAATGCTTGTTTGAGAGGTGCCACCTGGGGGTGATGAGCGAGGGCGAAGCCAGAATCCGCCACTG
CGCCTCTACCGAGACGGGCTGCTTTGTGCTGTGCAAGGGCAATGCTAAGATCAAGCATAATATGATCTGTGGAG
CCTCGGACGAGCGCGGCTACCAGATGCTGACCTGCGCCGGCGGGAACAGCCATATGCTGGCCACCGTACATGTG
GCTTCCCATGCTCGCAAGCCCTGGCCCGAGTTCGAGCACAATGTCATGACCAGGTGCAATATGCATCTGGGGTC
CCGCCGAGGCATGTTCATGCCCTACCAGTGCAACCTGAATTATGTGAAGGTGCTGCTGGAGCCCGATGCCATGT
CCAGAGTGAGCCTGACGGGGGTGTTTGACATGAATGTGGAGGTGTGGAAGATTCTGAGATATGATGAATCCAAG
ACCAGGTGCCGAGCCTGCGAGTGCGGAGGGAAGCATGCCAGGTTCCAGCCCGTGTGTGTGGATGTGACGGAGGA
CCTGCGACCCGATCATTTGGTGTTGCCCTGCACCGGGACGGAGTTCGGTTCCAGCGGGGAAGAATCTGACTAGA
GTGAGTAGTGTTCTGGGGCGGGGGAGGACCTGCATGAGGGCCAGAATAACTGAAATCTGTGCTTTTCTGTGTGT
TGCAGCAGCATGAGCGGAAGCGGCTCCTTTGAGGGAGGGGTATTCAGCCCTTATCTGACGGGGCGTCTCCCCTC
CTGGGCGGGAGTGCGTCAGAATGTGATGGGATCCACGGTGGACGGCCGGCCCGTGCAGCCCGCGAACTCTTCAA
CCCTGACCTATGCAACCCTGAGCTCTTCGTCGTTGGACGCAGCTGCCGCCGCAGCTGCTGCATCTGCCGCCAGC
GCCGTGCGCGGAATGGCCATGGGCGCCGGCTACTACGGCACTCTGGTGGCCAACTCGAGTTCCACCAATAATCC
CGCCAGCCTGAACGAGGAGAAGCTGTTGCTGCTGATGGCCCAGCTCGAGGCCTTGACCCAGCGCCTGGGCGAGC
TGACCCAGCAGGTGGCTCAGCTGCAGGAGCAGACGCGGGCCGCGGTTGCCACGGTGAAATCCAAATAAAAAATG
AATCAATAAATAAACGGAGACGGTTGTTGATTTTAACACAGAGTCTGAATCTTTATTTGATTTTTCGCGCGCGG
TAGGCCCTGGACCACCGGTCTCGATCATTGAGCACCCGGTGGATCTTTTCCAGGACCCGGTAGAGGTGGGCTTG
GATGTTGAGGTACATGGGCATGAGCCCGTCCCGGGGGTGGAGGTAGCTCCATTGCAGGGCCTCGTGCTCGGGGG
TGGTGTTGTAAATCACCCAGTCATAGCAGGGGCGCAGGGCATGGTGTTGCACAATATCTTTGAGGAGGAGACTG
ATGGCCACGGGCAGCCCTTTGGTGTAGGTGTTTACAAATCTGTTGAGCTGGGAGGGATGCATGCGGGGGGGAGAT
GAGGTGCATCTTGGCCTGGATCTTGAGATTGGCGATGTTACCGCCCAGATCCCGCCTGGGGTTCATGTTGTGCA
GGACCACCAGCACGGTGTATCCGGTGCACTTGGGGAATTTATCATGCAACTTGGAAGGGAAGGCGTGAAAGAAT
TTGGCGACGCCTTTGTGCCCGCCCAGGTTTTCCATGCACTCATCCATGATGATGGCGATGGGCCCGTGGGCGGC
GGCCTGGGCAAAGACGTTTCGGGGGTCGGACACATCATAGTTGTGGTCCTGGGTGAGGTCATCATAGGCCATTT
TAATGAATTTGGGGCGGAGGGTGCCGGACTGGGGGACAAAGGTACCCTCGATCCCGGGGGCGTAGTTCCCCTCA
CAGATCTGCATCTCCCAGGCTTTGAGCTCGGAGGGGGGGATCATGTCCACCTGCGGGGCGATAAAGAACACGGT
TTCCGGGGCGGGGGAGATGAGCTGGGCCGAAAGCAAGTTCCGGAGCAGCTGGGACTTGCCGCAGCGGTGGGGC
CGTAGATGACCCCGATGACCGGCTGCAGGTGGTAGTTGAGGGAGAGACAGCTGCCGTCCTCCCGGAGGAGGGGG
GCCACCTCGTTCATCATCTCGCGCACGTGCATGTTCTCGCGCACCAGTTCCGCCAGGAGGCGCTCTCCCCCCAG
GGATAGGAGCTCCTGGAGCGAGGCGAAGTTTTTCAGCGGCTTGAGTCCGTCGGCCATGGGCATTTTGGAGAGGG
TTTGTTGCAAGAGTTCCAGGCGGTCCCAGAGCTCGGTGATGTGCTCTACGGCATCTCGATCCAGCAGACCTCCT
CGTTTCGCGGGTTGGGACGGCTGCGGGAGTAGGGCACCAGACGATGGGCGTCCAGCGCAGCCAGGGTCCGGTCC
TTCCAGGGTCGCAGCGTCCGCGTCAGGGTGGTCTCCGTCACGGTGAAGGGGTGCGCGCCGGGCTGGGCGCTTGC
GAGGGTGCGCTTCAGGCTCATCCGGCTGGTCGAAAACCGCTCCCGATCGGCGCCCTGCGCGTCGGCCAGGTAGC
AATTGACCATGAGTTCGTAGTTGAGCGCCTCGGCCGCGTGGCCTTTGGCGCGGAGCTTACCTTTGGAAGTCTGC
CCGCAGGCGGGACAGAGGAGGGACTTGAGGGCGTAGAGCTTGGGGGCGAGGAAGACGGACTCGGGGGCGTAGGC
GTCCGCGCCGCAGTGGGCGCAGACGGTCTCGCACTCCACGAGCCAGGTGAGGTCGGGCTGGTCGGGGTCAAAAA
CCAGTTTCCCGCCGTTCTTTTTGATGCGTTTCTTACCTTTGGTCTCCATGAGCTCGTGTCCCGCTGGGTGACA
AAGAGGCTGTCCGTGTCCCCGTAGACCGACTTTATGGGCCGGTCCTCGAGCGGTGTGCCGCGGTCCTCCTCGTA
GAGGAACCCCGCCCACTCCGAGACGAAAGCCCGGGTCCAGGCCAGCACGAAGGAGGCCACGTGGGACGGGTAGC
GGTCGTTGTCCACCAGCGGGTCCACCTTTTCCAGGGTATGCAAACACATGTCCCCCTCGTCCACATCCAGGAAG
GTGATTGGCTTGTAAGTGTAGGCCACGTGACCGGGGGGTCCCGGCCGGGGGGGGTATAAAAGGGTGCGGGTCCCTG
CTCGTCCTCACTGTCTTCCGGATCGCTGTCCAGGAGCGCCAGCTGTTGGGGTAGGTATTCCCTCTCGAAGGCGG
GCATGACCTCGGCACTCAGGTTGTCAGTTTCTAGAAACGAGGAGGATTTGATATTGACGGTGCCGGCGGAGATG
CCTTTCAAGAGCCCCTCGTCCATCTGGTCAGAAAAGACGATCTTTTTGTTGTCGAGCTTGGTGGCGAAGGAGCC
GTAGAGGGCGTTGGAGAGGAGCTTGGCGATGGAGCGCATGGTCTGGTTTTTTTCCTTGTCGGCGCGCTCCTTGG
CGGCGATGTTGAGCTGCACGTACTCGCGCGCCACGCACTTCCATTCGGGGAAGACGGTGGTCAGCTCGTCGGGC
ACGATTCTGACCTGCCAGCCCCGATTATGCAGGGTGATGAGGTCCACACTGGTGGCCACCTCGCCGCGCAGGGG
CTCATTAGTCCAGCAGAGGCGTCCGCCCTTGCGCGAGCAGAAGGGGGGCAGGGGGTCCAGCATGACCTCGTCGG
GGGGGGTCGGCATCGATGGTGAAGATGCCGGGCAGGAGGTCGGGGTCAAAGTAGCTGATGGAAGTGGCCAGATCG
TCCAGGGCAGCTTGCCATTCGCGCACGGCCAGCGCGCGCTCGTAGGGACTGAGGGGCGTGCCCCAGGGCATGGG
ATGGGTAAGCGCGGAGGCGTACATGCCGCAGATGTCGTAGACGTAGAGGGGCTCCTCGAGGATGCCGATGTAGG
TGGGGTAGCAGCGCCCCCCGCGGATGCTGGCGCGCACGTAGTCATACAGCTCGTGCGAGGGGGCGAGGAGCCCC
GGGCCCAGGTTGGTGCGACTGGGCTTTTCGGCGCGGTAGACGATCTGGCGGAAAATGGCATGCGAGTTGGAGGA
GATGGTGGGCCTTTGGAAGATGTTGAAGTGGGCGTGGGGCAGTCCGACCGAGTCGCGGATGAAGTGGGCGTAGG
AGTCTTGCAGCTTGGCGACGAGCTCGGCGGTGACTAGGACGTCCAGAGCGCAGTAGTCGAGGGTCTCCTGGATG
ATGTCATACTTGAGCTGTCCCTTTTTGTTTCCACAGCTCGCGGTTGAGAAGGAACTCTTCGCGGTCCTTCCAGTA
CTCTTCGAGGGGGAACCCGTCCTGATCTGCACGGTAAGAGCCTAGCATGTAGAACTGGTTGACGGCCTTGTAGG
```

```
CGCAGCAGCCCTTCTCCACGGGGAGGGCGTAGGCCTGGGCGGCCTTGCGCAGGGAGGTGTGCGTGAGGGCGAAA
GTGTCCCTGACCATGACCTTGAGGAACTGGTGCTTGAAGTCGATATCGTCGCAGCCCCCCTGCTCCCAGAGCTG
GAAGTCCGTGCGCTTCTTGTAGGCGGGGTTGGGCAAAGCGAAAGTAACATCGTTGAAGAGGATCTTGCCCGCGC
GGGGCATAAAGTTGCGAGTGATGCGGAAAGGTTGGGGCACCTCGGCCCGGTTGTTGATGACCTGGGCGGCGAGC
ACGATCTCGTCGAAGCCGTTGATGTTGTGGCCCACGATGTAGAGTTCCACGAATCGCGGACGGCCCTTGACGTG
GGGCAGTTTCTTGAGCTCCTCGTAGGTGAGCTCGTCGGGGTCGCTGAGCCCGTGCTGCTCGAGCGCCCAGTCGG
CGAGATGGGGGTTGGCGCGGAGGAAGGAAGTCCAGAGATCCACGGCCAGGGCGGTTTGCAGACGGTCCCGGTAC
TGACGGAACTGCTGCCCGACGGCCATTTTTTCGGGGGTGACGCAGTAGAAGGTGCGGGGGTCCCCGTGCCAGCG
ATCCCATTTGAGCTGGAGGGCGAGATCGAGGGCGAGCTCGACGAGCCGGTCGTCCCCGGAGAGTTTCATGACCA
GCATGAAGGGGACGAGCTGCTTGCCGAAGGACCCCATCCAGGTGTAGGTTTCCACATCGTAGGTGAGGAAGAGC
CTTTCGGTGCGAGGATGCGAGCCGATGGGGAAGAACTGGATCTCCTGCCACCAATTGGAGGAATGGCTGTTGAT
GTGATGGAAGTAGAAATGCCGACGGCGCGCCGAACACTCGTGCTTGTGTTTATACAAGCGGCCACAGTGCTCGC
AACGCTGCACGGGATGCACGTGCTGCACGAGCTGTACCTGAGTTCCTTTGACGAGGAATTTCAGTGGGAAGTGG
AGTCGTGGCGCCTGCATCTCGTGCTGTACTACGTCGTGGTGGTCGGCCTGGCCCTCTTCTGCCTCGATGGTGGT
CATGCTGACGAGCCCGCGCGGGAGGCAGGTCCAGACCTCGGCGCGAGCGGGTCGGAGAGCGAGGACGAGGGCGC
GCAGGCCGGAGCTGTCCAGGGTCCTGAGACGCTGCGGAGTCAGGTCAGTGGGCAGCGGCGGCGCGCGGTTGACT
TGCAGGAGTTTTTTCCAGGGCGCGCGGGAGGTCCAGATGGTACTTGATCTCCACCGCGCCATTGGTGGCGACGTC
GATGGCTTGCAGGGTCCCGTGCCCCTGGGGTGTGACCACCGTCCCCCGTTTCTTCTTGGGCGGCTGGGGCGACG
GGGGCGGTGCCTCTTCCATGGTTAGAAGCGGCGGCGAGGACGCGCGCCGGGCGGCAGGGGCGGCTCGGGGCCCG
GAGGCAGGGGCGGCAGGGGCACGTCGGCGCCGCGCGCGGGTAGGTTCTGGTACTGCGCCCGGAGAAGACTGGCG
TGAGCGACGACGCGACGGTTGACGTCCTGGATCTGACGCCTCTGGGTGAAGGCCACGGGACCCGTGAGTTTGAA
CCTGAAAGAGAGTTCGACAGAATCAATCTCGGTATCGTTGACGGCGGCCTGCCGCAGGATCTCTTGCACGTCGC
CCGAGTTGTCCTGGTAGGCGATCTCGGTCATGAACTGCTCGATCTCCTCCTCTTGAAGGTCTCCGCGGCCGGCG
CGCTCCACGGTGGCCGCGAGGTCGTTGGAGATGCGGCCCATGAGCTGCGAGAAGGCGTTCATGCCCGCCTCGTT
CCAGACGCGGCTGTAGACCACGACGCCCTCGGGATCGCgGGCGCGCATGACCACCTGGGCGAGGTTGAGCTCCA
CGTGGCGCGTGAAGACCGCGTAGTTGCAGAGGCGCTGGTAGAGGTAGTTGAGCGTGGTGGCGATGTGCTCGGTG
ACGAAGAAATACATGATCCAGCGGCGGAGCGGCATCTCGCTGACGTCGCCCAGCGCCTCCAAACGTTCCATGGC
CTCGTAAAAGTCCACGGCGAAGTTGAAAAACTGGGAGTTGCGCGCCGAGACGGTCAACTCCTCCTCCAGAAGAC
GGATGAGCTCGGCGATGGTGGCGCGCACCTCGCGCTCGAAGGCCCCCGGGAGTTCCTCCACTTCCTCTTCTTCC
TCCTCCACTAACATCTCTTCTACTTCCTCCTCAGGCGGCAGTGGTGGCGGGGGAGGGGGCCTGCGTCGCCGGCG
GCGCACGGGCAGACGGTCGATGAAGCGCTCGATGGTCTCGCCGCGCCGGCGTCGCATGGTCTCGGTGACGGCGC
GCCCGTCCTCGCGGGGCCGCAGCGTGAAGACGCCGCCGCGCATCTCCAGGTGGCCGGGGGGGTCCCCGTTGGGC
AGGGAGAGGGCGCTGACGATGCATCTTATCAATTGCCCCGTAGGGACTCCGCGCAAGGACCTGAGCGTCTCGAG
ATCCACGGGATCTGAAAACCGCTGAACGAAGGCTTCGAGCCAGTCGCAGTCGCAAGGTAGGCTGAGCACGGTTT
CTTCTGGCGGGTCATGTTGGTTGGGAGCGGGGCGGGCGATGCTGCTGGTGATGAAGTTGAAATAGGCGGTTCTG
AGACGGCGGATGGTGGCGAGGAGCACCAGGTCTTTGGGCCCGGCTTGCTGGATGCGCAGACGGTCGGCCATGCC
CCAGGCGTGGTCCTGACACCTGGCCAGGTCCTTGTAGTAGTCCTGCATGAGCCGCTCCACGGGCACCTCCTCCT
CGCCCGCGCGGCCGTGCATGCGCGTGAGCCCGAAGCCGCGCTGGGGCTGGACGAGCGCCAGGTCGGCGACGACG
CGCTCGGCGAGGATGGCTTGCTGGATCTGGGTGAGGGTGGTCTGGAAGTCATCAAAGTCGACGAAGCGGTGGTA
GGCTCCGGTGTTGATGGTGTAGGAGCAGTTGGCCATGACGGACCAGTTGACGGTCTGGTGGCCCGGACGCACGA
GCTCGTGGTACTTGAGGCGCGAGTAGGCGCGCGTGTCGAAGATGTAGTCGTTGCAGGTGCGCACCAGGTACTGG
TAGCCGATGAGGAAGTGCGGCGGCGGCTGGCGGTAGAGCGGCCATCGCTCGGTGGCGGGGGCGCCGGGCGCGAG
GTCCTCGAGCATGGTGCGGTGGTAGCCGTAGATGTACCTGGACATCCAGGTGATGCCGGCGGCGGTGGTGGAGG
CGCGCGGGAACTCGCGGACGCGGTTCCAGATGTTGCGCAGCGGCAGGAAGTAGTTCATGGTGGGCACGGTCTGG
CCCGTGAGGCGCGCGCAGTCGTGGATGCTCTATACGGGCAAAAACGAAAGCGGTCAGCGGCTCGACTCCGTGGC
CTGGAGGCTAAGCGAACGGGTTGGGCTGCGCGTGTACCCCGGTTCGAATCTCGAATCAGGCTGGAGCCGCAGCT
AACGTGGTATTGGCACTCCCGTCTCGACCCAAGCCTGCACCAACCCTCCAGGATACGGAGGCGGGTCGTTTTGC
AACTTTTTTTTGGAGGCCGGATGAGACTAGTAAGCGCGGAAAGCGGCCGACCGCGATGGCTCGCTGCCGTAGTC
TGGAGAAGAATCGCCAGGGTTGCGTTGCGGTGTGCCCCGGTTCGAGGCCGGCCGGATTCCGCGGCTAACGAGGG
CGTGGCTGCCCCGTCGTTTCCAAGACCCCATAGCCAGCCGACTTCTCCAGTTACGGAGCGAGCCCCTCTTTTGT
TTTGTTTGTTTTTGCCAGATGCATCCCGTACTGCGGCAGATGCGCCCCCACCACCCTCCACCGCAACAACAGCC
CCCTCCACAGCCGGCGCTTCTGCCCCCGCCCCAGCAGCAACTTCCAGCCACGACCGCCGCGGCCGCCGTGAGCG
GGGCTGGACAGAGTTATGATCACCAGCTGGCCTTGGAAGAGGGCGAGGGGCTGGCGCGCCTGGGGGCGTCGTCG
CCGGAGCGGCACCCGCGCGTGCAGATGAAAAGGGACGCTCGCGAGGCCTACGTGCCCAAGCAGAACCTGTTCAG
AGACAGGAGCGGCGAGGAGCCCGAGGAGATGCGCGCGGCCCGGTTCCACGCGGGGCGGGAGCTGCGGCGCGGCC
TGGACCGAAAGAGGGTGCTGAGGGACGAGGATTTCGAGGCGGACGAGCTGACGGGGATCAGCCCCGCGCGCGCG
CACGTGGCCGCGGCCAACCTGGTCACGGCGTACGAGCAGACCGTGAAGGAGGAGAGCAACTTCCAAAAATCCTT
CAACAACCACGTGCGCACCCTGATCGCGCGCGAGGAGGTGACCCTGGGCCTGATGCACCTGTGGGACCTGCTGG
AGGCCATCGTGCAGAACCCCACCAGCAAGCCGCTGACGGCGCAGCTGTTCCTGGTGGTGCAGCATAGTCGGGAC
AACGAAGCGTTCAGGGAGGCGCTGCTGAATATCACCGAGCCCGAGGGCCGCTGGCTCCTGGACCTGGTGAACAT
TCTGCAGAGCATCGTGGTGCAGGAGCGCGGGCTGCCGCTGTCCGAGAAGCTGGCGGCCATCAACTTCTCGGTGC
TGAGTTTGGGCAAGTACTACGCTAGGAAGATCTACAAGACCCCGTACGTGCCCATAGACAAGGAGGTGAAGATC
```

```
GACGGGTTTTACATGCGCATGACCCTGAAAGTGCTGACCCTGAGCGACGATCTGGGGGTGTACCGCAACGACAG
GATGCACCGTGCGGTGAGCGCCAGCAGGCGGCGCGAGCTGAGCGACCAGGAGCTGATGCATAGTCTGCAGCGGG
CCCTGACCGGGGCCGGGACCGAGGGGGAGAGCTACTTTGACATGGGCGCGGACCTGCACTGGCAGCCCAGCCGC
CGGGCCTTGGAGGCGGCGGCAGGACCCTACGTAGAAGAGGTGGACGATGAGGTGGACGAGGAGGGCGAGTACCT
GGAAGACTGATGGCGCGACCGTATTTTTGCTAGATGCAACAACAACAGCCACCTCCTGATCCCGCGATGCGGGC
GGCGCTGCAGAGCCAGCCGTCCGGCATTAACTCCTCGGACGATTGGACCCAGGCCATGCAACGCATCATGGCGC
TGACGACCCGCAACCCCGAAGCCTTTAGACAGCAGCCCCAGGCCAACCGGCTCTCGGCCATCCTGGAGGCCGTG
GTGCCCTCGCGCTCCAACCCCACGCACGAGAAGGTCCTGGCCATCGTGAACGCGCTGGTGGAGAACAAGGCCAT
CCGCGGCGACGAGGCCGGCCTGGTGTACAACGCGCTGCTGGAGCGCGTGGCCCGCTACAACAGCACCAACGTGC
AGACCAACCTGGACCGCATGGTGACCGACGTGCGCGAGGCCGTGGCCCAGCGCGAGCGGTTCCACCGCGAGTCC
AACCTGGGATCCATGGTGGCGCTGAACGCCTTCCTCAGCACCCAGCCCGCCAACGTGCCCCGGGGCCAGGAGGA
CTACACCAACTTCATCAGCGCCCTGCGCCTGATGGTGACCGAGGTGCCCCAGAGCGAGGTGTACCAGTCCGGGC
CGGACTACTTCTTCCAGACCAGTCGCCAGGGCTTGCAGACCGTGAACCTGAGCCAGGCTTTCAAGAACTTGCAG
GGCCTGTGGGGCGTGCAGGCCCCGGTCGGGGACCGCGCGACGGTGTCGAGCCTGCTGACGCCGAACTCGCGCCT
GCTGCTGCTGCTGGTGGCCCCCTTCACGGACAGCGGCAGCATCAACCGCAACTCGTACCTGGGCTACCTGATTA
ACCTGTACCGCGAGGCCATCGGCCAGGCGCACGTGGACGAGCAGACCTACCAGGAGATCACCCACGTGAGCCGC
GCCCTGGGCCAGGACGACCCGGGCAACCTGGAAGCCACCCTGAACTTTTTGCTGACCAACCGGTCGCAGAAGAT
CCCGCCCCAGTACGCGCTCAGCACCGAGGAGGAGCGCATCCTGCGTTACGTGCAGCAGAGCGTGGGCCTGTTCC
TGATGCAGGAGGGGGCCACCCCCAGCGCCGCGCTCGACATGACCGCGCGCAACATGGAGCCCAGCATGTACGCC
AGCAACCGCCCGTTCATCAATAAACTGATGGACTACTTGCATCGGGCGGCCGCCATGAACTCTGACTATTTCAC
CAACGCCATCCTGAATCCCCACTGGCTCCCGCCGCCGGGGTTCTACACGGGCGAGTACGACATGCCCGACCCCA
ATGACGGGTTCCTGTGGGACGATGTGGACAGCAGCGTGTTCTCCCCCCGACCGGGTGCTAACGAGCGCCCCTTG
TGGAAGAAGGAAGGCAGCGACCGACGCCCGTCCTCGGCGCTGTCCGGCCGCGAGGGTGCTGCCGCGGCGGTGCC
CGAGGCCGCCAGTCCTTTCCCGAGCTTGCCCTTCTCGCTGAACAGTATCCGCAGCAGCGAGCTGGGCAGGATCA
CGCGCCCGCGCTTGCTGGGCGAAGAGGAGTACTTGAATGACTCGCTGTTGAGACCCGAGCGGGAGAAGAACTTC
CCCAATAACGGGATAGAAAGCCTGGTGGACAAGATGAGCCGCTGGAAGACGTATGCGCAGGAGCACAGGGACGA
TCCCCGGGCGTCGCAGGGGGCCACGAGCCGGGGCAGCGCCGCCCGTAAACGCCGGTGGCACGACAGGCAGCGGG
GACAGATGTGGGACGATGAGGACTCCGCCGACGACAGCAGCGTGTTGGACTTGGGTGGGAGTGGTAACCCGTTC
GCTCACCTGCGCCCCCGTATCGGGCGCATGATGTAAGAGAAACCGAAAATAAATGATACTCACCAAGGCCATGG
CGACCAGCGTGCGTTCGTTTCTTCTCTGTTGTTGTTGTATCTAGTATGATGAGGCGTGCGTACCCGGAGGGTCC
TCCTCCCTCGTACGAGAGCGTGATGCAGCAGGCGATGGCGGCGGCGGCGATGCAGCCCCCGCTGGAGGCTCCTT
ACGTGCCCCGCGGTACCTGGCGCCTACGGAGGGGCGGAACAGCATTCGTTACTCGGAGCTGGCACCCTTGTAC
GATACCACCCGGTTGTACCTGGTGGACAACAAGTCGGCGGACATCGCCTCGCTGAACTACCAGAACGACCACAG
CAACTTCCTGACCACCGTGGTGCAGAACAATGACTTCACCCCCACGGAGGCCAGCACCCAGACCATCAACTTTG
ACGAGCGCTCGCGGTGGGGCGGCCAGCTGAAAACCATCATGCACACCAACATGCCCAACGTGAACGAGTTCATG
TACAGCAACAAGTTCAAGGCGCGGGTGATGGTCTCCCGCAAGACCCCCAATGGGGTGACAGTGACAGAGGATTA
TGATGGTAGTCAGGATGAGCTGAAGTATGAATGGGTGGAATTTGAGCTGCCCGAAGGCAACTTCTCGGTGACCA
TGACCATCGACCTGATGAACAACGCCATCATCGACAATTACTTGGCGGTGGGGCGGCAGAACGGGGTGCTGGAG
AGCGACATCGGCGTGAAGTTCGACACTAGGAACTTCAGGCTGGGCTGGGACCCCGTGACCGAGCTGGTCATGCC
CGGGGGTGTACACCAACGAGGCTTTCCATCCCGATATTGTCTTGCTGCCCGGCTGCGGGGTGGACTTCACCGAGA
GCCGCCTCAGCAACCTGCTGGGCATTCGCAAGAGGCAGCCCTTCCAGGAAGGCTTCCAGATCATGTACGAGGAT
CTGGAGGGGGGCAACATCCCCGCGCTCCTGGATGTCGACGCCTATGAGAAAAGCAAGGAGGATGCAGCAGCTGA
AGCAACTGCAGCCGTAGCTACCGCCTCTACCGAGGTCAGGGGCGATAATTTTGCAAGCGCCGCAGCAGTGGCAG
CGGCCGAGGCGGCTGAAACCGAAAGTAAGATAGTCATTCAGCCGGTGGAGAAGGATAGCAAGAACAGGAGCTAC
AACGTACTACCGGACAAGATAAACACCGCCTACCGCAGCTGGTACCTAGCCTACAACTATGGCGACCCCGAGAA
GGGCGTGCGCTCCTGGACGCTGCTCACCACCTCGGACGTCACCTGCGGCGTGGAGCAAGTCTACTGGTCGCTGC
CCGACATGATGCAAGACCCGGTCACCTTCCGCTCCACGCGTCAAGTTAGCAACTACCCGGTGGTGGGCGCCGAG
CTCCTGCCCGTCTACTCCAAGAGCTTCTTCAACGAGCAGGCCGTCTACTCGCAGCAGCTGCGCGCCTTCACCTC
GCTTACGCACGTCTTCAACCGCTTCCCCGAGAACCAGATCCTCGTCCGCCCGCCCGCGCCCACCATTACCACCG
TCAGTGAAAACGTTCCTGCTCTCACAGATCACGGGACCCTGCCGCTGCGCAGCAGTATCCGGGGAGTCCAGCGC
GTGACCGTTACTGACGCCAGACGCCGCACCTGCCCCTACGTCTACAAGGCCCTGGGCATAGTCGCGCCGCGCGT
CCTCTCGAGCCGCACCTTCTAAATGTCCATTCTCATCTCGCCCAGTAATAACACCGGTTGGGGCCTGCGCGCGC
CCAGCAAGATGTACGGAGGCGCTCGCCAACGCTCCACGCAACACCCCGTGCGCGTGCGCGGGCACTTCCGCGCT
CCCTGGGGCGCCCTCAAGGGCCGCGTGCGGTCGCGCACCACCGTCGACGACGTGATCGACCAGGTGGTGGCCGA
CGCGCGCAACTACACCCCCGCCGCCGCGCCCGTCTCCACCGTGGACGCCGTCATCGACAGCGTGGTGGCcGACG
CGCGCCGGTACGCCCGCGCCAAGAGCCGGCGGCGGCGCATCGCCCGGCGGCACCGGAGCACCCCCGCCATGCGC
GCGGCGCGAGCCTTGCTGCGCAGGGCCAGGCGCACGGGACGCAGGGCCATGCTCAGGGCGGCCAGACGCGCGGC
TTCAGGCGCCAGCGCCGGCAGGACCCGGAGACGCGCGGCCACGGCGGCGGCAGCGGCCATCGCCAGCATGTCCC
GCCCGCGGCGAGGGAACGTGTACTGGGTGCGCGACGCCGCCACCGGTGTGCGCGTGCCCGTGCGCACCCGCCCC
CCTCGCACTTGAAGATGTTCACTTCGCGATGTTGATGTGTCCCAGCGGCGAGGAGGATGTCCAAGCGCAAATTC
AAGGAAGAGATGCTCCAGGTCATCGCGCCTGAGATCTACGGCCCTGCGGTGGTGAAGGAGGAAAGAAAGCCCCG
CAAAATCAAGCGGGTCAAAAAGGACAAAAAGGAAGAAGAAAGTGATGTGGACGGATTGGTGGAGTTTGTGCGCG
```

```
AGTTCGCCCCCCGGCGGCGCGTGCAGTGGCGCGGGCGGAAGGTGCAACCGGTGCTGAGACCCGGCACCACCGTG
GTCTTCACGCCCGGCGAGCGCTCCGGCACCGCTTCCAAGCGCTCCTACGACGAGGTGTACGGGGATGATGATAT
TCTGGAGCAGGCGGCCGAGCGCCTGGGCGAGTTTGCTTACGGCAAGCGCAGCCGTTCCGCACCGAAGGAAGAGG
CGGTGTCCATCCCGCTGGACCACGGCAACCCCACGCCGAGCCTCAAGCCCGTGACCTTGCAGCAGGTGCTGCCG
ACCGCGGCGCCGCGCCGGGGGTTCAAGCGCGAGGGCGAGGATCTGTACCCCACCATGCAGCTGATGGTGCCCAA
GCGCCAGAAGCTGGAAGACGTGCTGGAGACCATGAAGGTGGACCCGGACGTGCAGCCCGAGGTCAAGGTGCGGC
CCATCAAGCAGGTGGCCCCGGGCCTGGGCGTGCAGACCGTGGACATCAAGATTCCCACGGAGCCCATGGAAACG
CAGACCGAGCCCATGATCAAGCCCAGCACCAGCACCATGGAGGTGCAGACGGATCCCTGGATGCCATCGGCTCC
TAGTCGAAGACCCCGGCGCAAGTACGGCGCGGCCAGCCTGCTGATGCCCAACTACGCGCTGCATCCTTCCATCA
TCCCCACGCCGGGCTACCGCGGCACGCGCTTCTACGCGGTCATACCAGCAGCCGCCGCCGCAAGACCACCACT
CGCCGCCGCCGTCGCCGCACCGCCGCTGCAACCACCCCTGCCGCCCTGGTGCGGAGAGTGTACCGCCGCGGCCG
CGCACCTCTGACCCTGCCGCGCGCGCGCTACCACCCGAGCATCGCCATTTAAACTTTCGCCtGCTTTGCAGATC
AATGGCCCTCACATGCCGCCTTCGCGTTCCCATTACGGGCTACCGAGGAAGAAAACCGCGCCGTAGAAGGCTGG
CGGGGAACGGGATGCGTCGCCACCACCACCGGCGGCGGCGCGCCATCAGCAAGCGGTTGGGGGGAGGCTTCCTG
CCCGCGCTGATCCCCATCATCGCCGCGGCGATCGGGGCGATCCCCGGCATTGCTTCCGTGGCGGTGCAGGCCTC
TCAGCGCCACTGAGACACACTTGGAAACATCTTGTAATAAACCaATGGACTCTGACGCTCCTGGTCCTGTGATG
TGTTTTCGTAGACAGATGGAAGACATCAATTTTTCGTCCCTGGCTCCGCGACACGGCACGCGGCCGTTCATGGG
CACCTGGAGCGACATCGGCACCAGCCAACTGAACGGGGGCGCCTTCAATTGGAGCAGTCTCTGGAGCGGGCTTA
AGAATTTCGGGTCCACGCTTAAAAACCTATGGCAGCAAGGCGTGGAACAGCACCACAGGGCAGGCGCTGAGGGAT
AAGCTGAAAGAGCAGAACTTCCAGCAGAAGGTGGTCGATGGGCTCGCCTCGGGCATCAACGGGGTGGTGGACCT
GGCCAACCAGGCCGTGCAGCGGCAGATCAACAGCCGCCTGGACCCGGTGCCGCCCGCCGGCTCCGTGGAGATGC
CGCAGGTGGAGGAGGAGCTGCCTCCCCTGGACAAGCGGGGCGAGAAGCGACCCCGCCCCGATGCGGAGGAGACG
CTGCTGACGCACACGGACGAGCCGCCCCCGTACGAGGAGGCGGTGAAACTGGGTCTGCCCACCACGCGGCCCAT
CGCGCCCCTGGCCACCGGGGTGCTGAAACCCGAAAAGCCCGCGACCCTGGACTTGCCTCCTCCCCAGCCTTCCC
GCCCCTCTACAGTGGCTAAGCCCCTGCCGCCGGTGGCCGTGGCCCGCGCGCGACCCGGGGGGCACCGCCCGCCCT
CATGCGAACTGGCAGAGCACTCTGAACAGCATCGTGGGTCTGGGAGTGCAGAGTGTGAAGCGCCGCCGCTGCTA
TTAAACCTACCGTAGCGCTTAACTTGCTTGTCTGTGTGTGTATGTATTATGTCGCCGCCGCCGCTGTCCACCAG
AAGGAGGAGTGAAGAGGCGCGTCGCCGAGTTGCAAGATGGCCACCCCATCGATGCTGCCCCAGTGGGCGTACAT
GCACATCGCCGGACAGGACGCTTCGGAGTACCTGAGTCCGGGTCTGGTGCAGTTTGCCCGCGCCACAGACACCT
ACTTCAGTCTGGGGAACAAGTTTAGGAACCCCACGGTGGCGCCCACGCACGATGTGACCACCGACCGCAGCCAG
CGGCTGACGCTGCGCTTCGTGCCCGTGGACCGCGAGGACAACACCTACTCGTACAAAGTGCGCTACACGCTGGC
CGTGGGCGACAACCGCGTGCTGGACATGGCCAGCACCTACTTTGACATCCGCGGCGTGCTGGATCGGGGCCCTA
GCTTCAAACCCTACTCCGGCACCGCCTACAACAGTCTGGCCCCCAAGGGAGCACCCAACACTTGTCAGTGGACA
TATAAAGCCGATGGTGAAACTGCCACAGAAAAAACCTATACATATGGAAATGCACCCGTGCAGGGCATTAACAT
CACAAAAGATGGTATTCAACTTGGAACTGACACCGATGATCAGCCAATCTACGCAGATAAAACCTATCAGCCTG
AACCTCAAGTGGGTGATGCTGAATGGCATGACATCACTGGTACTGATGAAAAGTATGGAGGCAGAGCTCTTAAG
CCTGATACCAAAATGAAGCCTTGTTATGGTTCTTTTGCCAAGCCTACTAATAAAGAAGGAGGTCAGGCAAATGT
GAAAACAGGAACAGGCACTACTAAAGAATATGACATAGACATGGCTTTCTTTGACAACAGAAGTGCGGCTGCTG
CTGGCCTAGCTCCAGAAATTGTTTTGTATACTGAAAATGTGGATTTGGAAACTCCAGATACCCATATTGTATAC
AAAGCAGGCACAGATGACAGCAGCTCTTCTATTAATTTGGGTCAGCAAGCCATGCCCAACAGACCTAACTACAT
TGGTTTCAGAGACAACTTTATCGGGCTCATGTACTACAACAGCACTGGCAATATGGGGGTGCTGGCCGGTCAGG
CTTCTCAGCTGAATGCTGTGGTTGACTTGCAAGACAGAAACACCGAGCTGTCCTACCAGCTCTTGCTTGACTCT
CTGGGTGACAGAACCCGGTATTTCAGTATGTGGAATCAGGCGGTGGACAGCTATGATCCTGATGTGCGCATTAT
TGAAAATCATGGTGTGGAGGATGAACTTCCCAACTATTGTTTCCCTCTGGATGCTGTTGGCAGAACAGATACTT
ATCAGGGAATTAAGGCTAATGGAACTGATCAAACCACATGGACCAAAGATGACAGTGTCAATGATGCTAATGAG
ATAGGCAAGGGTAATCCATTCGCCATGGAAATCAACATCCAAGCCAACCTGTGGAGGAACTTCCTCTACGCCAA
CGTGGCCCTGTACCTGCCCGACTCTTACAAGTACACGCCGGCCAATGTTACCCTGCCCACCAACACCAACACCT
ACGATTACATGAACGGCCGGGTGGTGGCGCCCTCGCTGGTGGACTCCTACATCAACATCGGGGCGCGCTGGTCG
CTGGATCCCATGGACAACGTGAACCCCTTCAACCACCACCGCAATGCGGGGCTGCGCTACCGCTCCATGCTCCT
GGGCAACGGGCGCTACGTGCCCTTCCACATCCAGGTGCCCCAGAAATTTTTCGCCATCAAGAGCCTCCTGCTCC
TGCCCGGGTCCTACACCTACGAGTGGAACTTCCGCAAGGACGTCAACATGATCCTGCAGAGCTCCCTCGGCAAC
GACCTGCGCACGGACGGGGCCTCCATCTCCTTCACCAGCATCAACCTCTACGCCACCTTCTTCCCCATGGCGCA
CAACACGGCCTCCACGCTCGAGGCCATGCTGCGCAACGACACCAACGACCAGTCCTTCAACGACTACCTCTCGG
CGGCCAACATGCTCTACCCCATCCCGGCCAACGCCACCAACGTGCCCATCTCCATCCCCTCGCGCAACTGGGCC
GCCTTCCGCGGCTGGTCCTTCACGCGTCTCAAGACCAAGGAGACGCCCTCGCTGGGCTCCGGGTTCGACCCCTA
CTTCGTCTACTCGGGCTCCATCCCCTACCTCGACGGCACCTTCTACCTCAACCACACCTTCAAGAAGGTCTCCA
TCACCTTCGACTCCTCCGTCAGCTGGCCCGGCAACGACCGGCTCCTGACGCCCAACGAGTTCGAAATCAAGCGC
ACCGTCGACGGCGAGGGCTACAACGTGGCCCAGTGCAACATGACCAAGGACTGGTTCCTGGTCCAGATGCTGGC
CCACTACAACATCGGCTACCAGGGCTTCTACGTGCCCGAGGGCTACAAGGACCGCATGTACTCCTTCTTCCGCA
ACTTCCAGCCCATGAGCCGCCAGGTGGTGGACGAGGTCAACTACAAGGACTACCAGGCCGTCACCCTGGCCTAC
CAGCACAACAACTCGGGCTTCGTCGGCTACCTCGCGCCCACCATGCGCCAGGGCCAGCCCTACCCCGCCAACTA
CCCCTACCCGCTCATCGGCAAGAGCGCCGTCACCAGCGTCACCCAGAAAAAGTTCCTCTGCGACAGGGTCATGT
```

```
GGCGCATCCCCTTCTCCAGCAACTTCATGTCCATGGGCGCGCTCACCGACCTCGGCCAGAACATGCTCTATGCC
AACTCCGCCCACGCGCTAGACATGAATTTCGAAGTCGACCCCATGGATGAGTCCACCCTTCTCTATGTTGTCTT
CGAAGTCTTCGACGTCGTCCGAGTGCACCAGCCCCACCGCGGCGTCATCGAGGCCGTCTACCTGCGCACCCCCT
TCTCGGCCGGTAACGCCACCACCTAAGCTCTTGCTTCTTGCAAGCCATGGCCGCGGGCTCCGGCGAGCAGGAGC
TCAGGGCCATCATCCGCGACCTGGGCTGCGGGCCCTACTTCCTGGGCACCTTCGATAAGCGCTTCCCGGGATTC
ATGGCCCCGCACAAGCTGGCCTGCGCCATCGTCAACACGGCCGGCCGCGAGACCGGGGGCGAGCACTGGCTGGC
CTTCGCCTGGAACCCGCGCTCGAACACCTGCTACCTCTTCGACCCCTTCGGGTTCTCGGACGAGCGCCTCAAGC
AGATCTACCAGTTCGAGTACGAGGGCCTGCTGCGCCGCAGCGCCCTGGCCACCGAGGACCGCTGCGTCACCCTG
GAAAAGTCCACCCAGACCGTGCAGGGTCCGCGCTCGGCCGCCTGCGGGCTCTTCTGCTGCATGTTCCTGCACGC
CTTCGTGCACTGGCCCGACCGCCCCATGGACAAGAACCCCACCATGAACTTGCTGACGGGGGTGCCCAACGGCA
TGCTCCAGTCGCCCCAGGTGGAACCCACCCTGCGCCGCAACCAGGAGGCGCTCTACCGCTTCCTCAACTCCCAC
TCCGCCTACTTTCGCTCCCACCGCGCGCGCATCGAGAAGGCCACCGCCTTCGACCGCATGAATCAAGACATGTA
AACCGTGTGTGTATGTTAAATGTCTTTAATAAACAGCACTTTCATGTTACACATGCATCTGAGATGATTTATTT
AGAAATCGAAAGGGTTCTGCCGGGTCTCGGCATGGCCCGCGGGCAGGGACACGTTGCGGAACTGGTACTTGGCC
AGCCACTTGAACTCGGGGATCAGCAGTTTGGGCAGCGGGGTGTCGGGGAAGGAGTCGGTCCACAGCTTCCGCGT
CAGTTGCAGGGCGCCCAGCAGGTCGGGCGCGGAGATCTTGAAATCGCAGTTGGGACCCGCGTTCTGCGCGCGGG
AGTTGCGGTACACGGGGTTGCAGCACTGGAACACCATCAGGGCCGGGTGCTTCACGCTCGCCAGCACCGTCGCG
TCGGTGATGCTCTCCACGTCGAGGTCCTCGGCGTTGGCCATCCCGAAGGGGGGTCATCTTGCAGGTCTGCCTTCC
CATGGTGGGCACGCACCCGGGCTTGTGGTTGCAATCGCAGTGCAGGGGGATCAGCATCATCTGGGCCTGGTCGG
CGTTCATCCCGGGTACATGGCCTTCATGAAAGCCTCCAATTGCCTGAACGCCTGCTGGGCCTTGGCTCCCTCG
GTGAAGAAGACCCCGCAGGACTTGCTAGAGAACTGGTTGGTGGCGCACCCGGCGTCGTGCACGCAGCAGCGCGC
GTCGTTGTTGGCCAGCTGCACCACGCTGCGCCCCCAGCGGTTCTGGGTGATCTTGGCCCGGTCGGGGTTCTCCT
TCAGCGCGCGCTGCCCGTTCTCGCTCGCCACATCCATCTCGATCATGTGCTCCTTCTGGATCATGGTGGTCCCG
TGCAGGCACCGCAGCTTGCCCTCGGCCTCGGTGCACCGTGCAGCCACAGCGCGCACCCGGTGCACTCCAGTT
CTTGTGGGCGATCTGGGAATGCGCGTGCACGAAGCCCTGCAGGAAGCGGCCCATCATGGTGGTCAGGGTCTTGT
TGCTAGTGAAGGTCAGCGGAATGCCGCGGTGCTCCTCGTTGATGTACAGGTGGCAGATGCGGCGGTACACCTCG
CCCTGCTCGGGCATCAGCTGGAAGTTGGCTTTCAGGTCGGTCTCCACGCGGTAGCGGTCCATCAGCATAGTCAT
GATTTCCATACCCTTCTCCCAGGCCGAGACGATGGGCAGGCTCATAGGGTTCTTCACCATCATCTTAGCGCTAG
CAGCCGCGGCCAGGGGGTCGCTCTCGTCCAGGGTCTCAAAGCTCCGCTTGCCGTCCTTCTCGGTGATCCGCACC
GGGGGGTAGCTGAAGCCCACGCCGCCAGCTCCTCCTCGGCCTGTCTTTCGTCCTCGCTGTCCTGGCTGACGTC
CTGCAGGACCACATGCTTGGTCTTGCGGGGTTTCTTCTTGGGCGGCAGCGGCGGCGGAGATGTTGGAGATGGCG
AGGGGGAGCGCGAGTTCTCGCTCACCACTACTATCTCTTCCTCTTCTTGGTCCGAGGCCACGCGGCGGTAGGTA
TGTCTCTTCGGGGGCAGAGGCGGAGGCGACGGGCTCTCGCCGCCGCGACTTGGCGGATGGCTGGCAGAGCCCCT
TCCGCGTTCGGGGGTGCGCTCCCGGCGGCGCTCTGACTGACTTCCTCCGCGGCCGGCCATTGTGTTCTCCTAGG
GAGGAACAACAAGCATGGAGACTCAGCCATCGCCAACCTCGCCATCTGCCCCCACCGCCGACGAGAAGCAGCAG
CAGCAGAATGAAAGCTTAACCGCCCCGCCGCCCAGCCCCGCCACCTCCGACGCGGCCGTCCCAGACATGCAAGA
GATGGAGGAATCCATCGAGATTGACCTGGGCTATGTGACGCCCGCGGAGCACGAGGAGGAGCTGGCAGTGCGCT
TTTCACAAGAAGAGATACACCAAGAACAGCCAGAGCAGGAAGCAGAGAATGAGCAGAGTCAGGCTGGGCTCGAG
CATGACGGCGACTACCTCCACCTGAGCGGGGGGGGAGGACGCGCTCATCAAGCATCTGGCCCGGCAGGCCACCAT
CGTCAAGGATGCGCTGCTCGACCGCACCGAGGTGCCCCTCAGCGTGGAGGAGCTCAGCCGCGCCTACGAGTTGA
ACCTCTTCTCGCCGCGCGTGCCCCCCAAGCGCCAGCCCAATGGCACCTGCGAGCCCAACCCGCGCCTCAACTTC
TACCCGGTCTTCGCGGTGCCCGAGGCCCTGGCCACCTACCACATCTTTTTCAAGAACCAAAAGATCCCCGTCTC
CTGCCGCGCCAACCGCACCCGCGCCGACGCCCTTTTCAACCTGGGTCCCGGCGCCCGCCTACCTGATATCGCCT
CCTTGGAAGAGGTTCCCAAGATCTTCGAGGGTCTGGGCAGCGACGAGACTCGGGCCGCGAACGCTCTGCAAGGA
GAAGGAGGAGAGCATGAGCACCACAGCGCCCTGGTCGAGTTGGAAGGCGACAACGCGCGGCTGGCGGTGCTCAA
ACGCACGGTCGAGCTGACCCATTTCGCCTACCCGGCTCTGAACCTGCCCCCCAAAGTCATGAGCGCGGTCATGG
ACCAGGTGCTCATCAAGCGCGCGTCGCCCATCTCCGAGGACGAGGGCATGCAAGACTCCGAGGAGGGCAAGCCC
GTGGTCAGCGACGAGCAGCTGGCCCGGTGGCTGGGTCCTAATGCTAGTCCCCAGAGTTTGGAAGAGCGGCGCAA
ACTCATGATGGCCGTGGTCCTGGTGACCGTGGAGCTGGAGTGCCTGCGCCGCTTCTTCGCCGACGCGGAGACCC
TGCGCAAGGTCGAGGAGAACCTGCACTACCTCTTCAGGCACGGGTTCGTGCGCCAGGCCTGCAAGATCTCCAAC
GTGGAGCTGACCAACCTGGTCTCCTACATGGGCATCTTGCACGAGAACCGCCTGGGGCAGAACGTGCTGCACAC
CACCCTGCGCGGGGAGGCCCGGCGCGACTACATCCGCGACTGCGTCTACCTCTACCTCTGCCACACCTGGCAGA
CGGGCATGGGCGTGTGGCAGCAGTGTCTGGAGGAGCAGAACCTGAAAGAGCTCTGCAAGCTCCTGCAGAAGAAC
CTCAAGGGTCTGTGGACCGGGTTCGACGAGCGCACCACCGCCTCGGACCTGGCCGACCTCATTTTCCCCGAGCG
CCTCAGGCTGACGCTGCGCAACGGCCTGCCCGACTTTATGAGCCAAAGCATGTTGCAAAACTTTCGCTCTTTCA
TCCTCGAACGCTCCGGAATCCTGCCCGCCACCTGCTCCGCGCTGCCCTCGGACTTCGTGCCGCTGACCTTCCGC
GAGTGCCCCCCGCCGCTGTGGAGCCACTGCTACCTGCTGCGCCTGGCCAACTACCTGGCCTACCACTCGGACGT
GATCGAGGACGTCAGCGGCGAGGGCCTGCTCGAGTGCCACTGCCGCTGCAACCTCTGCACGCCGCACCGCTCCC
TGGCCTGCAACCCCCAGCTGCTGAGCGAGACCCAGATCATCGGCACCTTCGAGTTGCAAGGGCCCAGCGAAGGC
GAGGGTTCAGCCGCCAAGGGGGGTCTGAAACTCACCCCGGGGCTGTGGACCTCGGCCTACTTGCGCAAGTTCGT
GCCCGAGGACTACCATCCCTTCGAGATCAGGTTCTACGAGGACCAATCCCATCCGCCCAAGGCCGAGCTGTCGG
CCTGCGTCATCACCCAGGGGGCGATCCTGGCCCAATTGCAAGCCATCCAGAAATCCCGCCAAGAATTCTTGCTG
```

```
AAAAAGGGCCGCGGGGTCTACCTCGACCCCCAGACCGGTGAGGAGCTCAACCCCGGCTTCCCCCAGGATGCCCC
GAGGAAACAAGAAGCTGAAAGTGGAGCTGCCGCCCGTGGAGGATTTGGAGGAAGACTGGGAGAACAGCAGTCAG
GCAGAGGAGGAGGAGATGGAGGAAGACTGGGACAGCACTCAGGCAGAGGAGGACAGCCTGCAAGACAGTCTGGA
GGAAGACGAGGAGGAGGCAGAGGAGGAGGTGGAAGAAGCAGCCGCCGCCAGACCGTCGTCCTCGGCGGGGGAGA
AAGCAAGCAGCACGGATACCATCTCCGCTCCGGGTCGGGGTCCCGCTCGACCACACAGTAGATGGGACGAGACC
GGACGATTCCCGAACCCCACCACCCAGACCGGTAAGAAGGAGCGGCAGGGATACAAGTCCTGGCGGGGGCACAA
AAACGCCATCGTCTCCTGCTTGCAGGCCTGCGGGGGCAACATCTCCTTCACCCGGCGCTACCTGCTCTTCCACC
GCGGGGTGAACTTTCCCCGCAACATCTTGCATTACTACCGTCACCTCCACAGCCCCTACTACTTCCAAGAAGAG
GCAGCAGCAGCAGAAAAAGACCAGCAGAAACCAGCAGCTAGAAAATCCACAGCGGCGGCAGCAGGTGGACTGA
GGATCGCGGCGAACGAGCCGGCGCAAACCCGGGAGCTGAGGAACCGGATCTTTCCCACCCTCTATGCCATCTTC
CAGCAGAGTCGGGGGCAGGAGCAGGAACTGAAAGTCAAGAACCGTTCTCTGCGCTCGCTCACCCGCAGTTGTCT
GTATCACAAGAGCGAAGACCAACTTCAGCGCACTCTCGAGGACGCCGAGGCTCTCTTCAACAAGTACTGCGCGC
TCACTCTTAAAGAGTAGCCCGCGCCCGCCCAGTCGCAGAAAAAGGCGGGAATTACGTCACCTGTGCCCTTCGCC
CTAGCCGCCTCCACCCATCATCATGAGCAAAGAGATTCCCACGCCTTACATGTGGAGCTACCAGCCCCAGATGG
GCCTGGCCGCCGGTGCCGCCCAGGACTACTCCACCCGCATGAATTGGCTCAGCGCCGGGCCCGCGATGATCTCA
CGGGTGAATGACATCCGCGCCCACCGAAACCAGATACTCCTAGAACAGTCAGCGCTCACCGCCACGCCCCGCAA
TCACCTCAATCCGCGTAATTGGCCCGCCGCCCTGGTGTACCAGGAAATTCCCCAGCCCACGACCGTACTACTTC
CGCGAGACGCCCAGGCCGAAGTCCAGCTGACTAACTCAGGTGTCCAGCTGGCGGGCGGCGCCACCCTGTGTCGT
CACCGCCCCGCTCAGGGTATAAAGCGGCTGGTGATCCGGGGCAGAGGCACACAGCTCAACGACGAGGTGGTGAG
CTCTTCGCTGGGTCTGCGACCTGACGGAGTCTTCCAACTCGCCGGATCGGGGAGATCTTCCTTCACGCCTCGTC
AGGCCGTCCTGACTTTGGAGAGTTCGTCCTCGCAGCCCCGCTCGGGTGGCATCGGCACTCTCCAGTTCGTGGAG
GAGTTCACTCCCTCGGTCTACTTCAACCCCTTCTCCGGCTCCCCGGCCACTACCCGGACGAGTTCATCCCGAA
CTTCGACGCCATCAGCGAGTCGGTGGACGGCTACGATTGAATGTCCCATGGTGGCGCAGCTGACCTAGCTCGGC
TTCGACACCTGGACCACTGCCGCCGCTTCCGCTGCTTCGCTCGGGATCTCGCCGAGTTTGCCTACTTTGAGCTG
CCCGAGGAGCACCCTCAGGGCCCGGCCCACGGAGTGCGGATCGTCGTCGAAGGGGGCCTCGACTCCCACCTGCT
TCGGATCTTCAGCCAGCGTCCGATCCTGGTCGAGCGCGAGCAAGGACAGACCCTTCTGACTCTGTACTGCATCT
GCAACCACCCCGGCCTGCATGAAAGTCTTTGTTGTCTGCTGTGTACTGAGTATAATAAAAGCTGAGATCAGCGA
CTACTCCGGACTTCCGTGTGTTCCTGAATCCATCAACCAGTCTTTGTTCTTCACCGGGAACGAGACCGAGCTCC
AGCTCCAGTGTAAGCCCCACAAGAAGTACCTCACCTGGCTGTTCCAGGGCTCCCCGATCGCCGTTGTCAACCAC
TGCGACAACGACGGAGTCCTGCTGAGCGGCCCTGCCAACCTTACTTTTTCCACCCGCAGAAGCAAGCTCCAGCT
CTTCCAACCCTTCCTCCCCGGGACCTATCAGTGCGTCTCGGGACCCTGCCATCACACCTTCCACCTGATCCCGA
ATACCACAGCGTCGCTCCCCGCTACTAACAACCAAACTAACCTCCACCAACGCCACCGTCGCGACCTTTCTGAA
TCTAATACTACCACCCACACCGGAGGTGAGCTCCGAGGTCAACCAACCTCTGGGATTTACTACGGCCCCTGGGA
GGTGGTTGGGTTAATAGCGCTAGGCCTAGTTGCGGGTGGGCTTTTGGTTCTCTGCTACCTATACCTCCCTTGCT
GTTCGTACTTAGTGGTGCTGTGTTGCTGGTTTAAGAAATGGGGAAGATCACCCTAGTGAGCTGCGGTGCGCTGG
TGGCGGTGTTGCTTTCGATTGTGGGACTGGGCGGTGCGGCTGTAGTGAAGGAGAAGGCCGATCCCTGCTTGCAT
TTCAATCCCAACAAATGCCAGCTGAGTTTTCAGCCGATGGCAATCGGTGCGCGGTACTGATCAAGTGCGGATG
GGAATGCGAGAACGTGAGAATCGAGTACAATAACAAGACTCGGAACAATACTCTCGCGTCCGTGTGGCAGCCCG
GGGACCCCGAGTGGTACACCGTCTCTGTCCCCGGTGCTGACGGCTCCCCGCGCACCGTGAATAATACTTTCATT
TTTGCGCACATGTGCGACACGGTCATGTGGATGAGCAAGCAGTACGATATGTGGCCCCCCACGAAGGAGAACAT
CGTGGTCTTCTCCATCGCTTACAGCCTGTGCACGGCGCTAATCACCGCTATCGTGTGCCTGAGCATTCACATGC
TCATCGCTATTCGCCCCAGAAATAATGCCGAAAAAGAAAACAGCCATAACGTTTTTTTTCACACCTTTTTCAG
ACCATGGCCTCTGTTAAATTTTTGCTTTTATTTGCCAGTCTCATTGCCGTCATTCATGGAATGAGTAATGAGAA
AATTACTATTTACACTGGCACTAATCACACATTGAAAGGTCCAGAAAAAGCCACAGAAGTTTCATGGTATTGTT
ATTTTAATGAATCAGATGTATCTACTGAACTCTGTGGAAACAATAACAAAAAAAATGAGAGCATTACTCTCATC
AAGTTTCAATGTGGATCTGACTTAACCCTAATTAACATCACTAGAGACTATGTAGGTATGTATTATGGAACTAC
AGCAGGCATTTCGGACATGGAATTTTATCAAGTTTCTGTGTCTGAACCCACCACGCCTAGAATGACCACAACCA
CAAAAACTACACCTGTTACCACTATGCAGCTCACTACCAATAACATTTTTGCCATGCGTCAAATGGTCAACAAT
AGCACTCAACCCACCCCACCCAGTGAGGAAATTCCCAAATCCATGATTGGCATTATTGTTGCTGTAGTGGTGTG
CATGTTGATCATCGCCTTGTGCATGGTGTACTATGCCTTCTGCTACAGAAAGCACAGACTGAACGACAAGCTGG
AACACTTACTAAGTGTTGAATTTTAATTTTTTAGAACCATGAAGATCCTAGGCCTTTTAATTTTTTCTATCATT
ACCTCTGCTCTATGCAATTCTGACAATGAGGACGTTACTGTCGTTGTCGGATCAAATTATACACTGAAAGGTCC
AGCGAAGGGTATGCTTTCGTGGTATTGCTATTTTGGATCTGACACTACAGAAACTGAATTATGCAATCTTAAGA
ATGGCAAAATTCAAAATTCTAAAATTAACAATTATATATGCAATGGTACTGATCTGATACTCCTCAATATCACG
AAATCATATGCTGGCAGTTACACCTGCCCTGGAGATGATGCTGACAGTATGATTTTTTACAAAGTAACTGTTGT
TGATCCCACTACTCCACCTCCACCCACCACAACTACTCACACCACACACAGATCAAACCGCAGCAGAGGAGG
CAGCAAAGTTAGCCTTGCAGGTCCAAGACAGTTCATTTGTTGGCATTACCCCTACACCTGATCAGCGGTGTCCG
GGGCTGCTAGTCAGCGGCATTGTCGGTGTGCTTTCGGGATTAGCAGTCATAATCATCTGCATGTTCATTTTTGC
TTGCTGCTATAGAAGGCTTTACCGACAAAAATCAGACCCACTGCTGAACCTCTATGTTTAATTTTTTCCAGAGT
CATGAAGGCAGTTAGCGCTCTAGTTTTTTGTTCTTTGATTGGCATTGTTTTTTGCAATCCTATTCCTAAAGTTA
GCTTTATTAAAGATGTGAATGTTACTGAGGGGGGCAATGTGACACTGGTAGGTGTAGAGGGTGCTGAAAACACC
ACCTGGACAAAATACCACCTCAATGGGTGGAAAGATATTTGCAATTGGAGTGTATTAGTTTATACATGTGAGGG
```

```
AGTTAATCTTACCATTGTCAATGCCACCTCAGCTCAAAATGGTAGAATTCAAGGACAAAGTGTCAGTGTATCTA
ATGGGTATTTTACCCAACATACTTTTATCTATGACGTTAAAGTCATACCACTGCCTACGCCTAGCCCACCTAGC
ACTACCACACAGACAACCCACACTACACAGACAACCACATACAGTACATTAAATCAGCCTACCACCACTACAGC
AGCAGAGGTTGCCAGCTCGTCTGGGGTCCGAGTGGCATTTTTGATGTtGGCCCCATCTAGCAGTCCCACTGCTA
GTACCAATGAGCAGACTACTGAATTTTTGTCCACTGTCGAGAGCCACACCACAGCTACCTCCAGTGCCTTCTCT
AGCACCGCCAATCTCTCCTCGCTTTCCTCTACACCAATCAGTCCCGCTACTACTCCTAGCCCCGCTCCTCTTCC
CACTCCCCTGAAGCAAACAGACGGCGGCATGCAATGGCAGATCACCCTGCTCATTGTGATCGGGTTGGTCATCC
TGGCCGTGTTGCTCTACTACATCTTCTGCCGCCGCATTCCCAACGCGCACCGCAAGCCGGTCTACAAGCCCATC
ATTGTCGGGCAGCCGGAGCCGCTTCAGGTGGAAGGGGGTCTAAGGAATCTTCTCTTCTCTTTTACAGTATGGTG
ATTGAACTATGATTCCTAGACAATTCTTGATCACTATTCTTATCTGCCTCCTCCAAGTCTGTGCCACCCTCGCT
CTGGTGGCCAACGCCAGTCCAGACTGTATTGGGCCCTTCGCCTCCTACGTGCTCTTTGCCTTCACCACCTGCAT
CTGCTGCTGTAGCATAGTCTGCCTGCTTATCACCTTCTTCCAGTTCATTGACTGGATCTTTGTGCGCATCGCCT
ACCTGCGCCACCACCCCAGTACCGCGACCAGCGAGTGGCGCGGCTGCTCAGGCTCCTCTGATAAGCATGCGGG
CTCTGCTACTTCTCGCGCTTCTGCTGTTAGTGCTCCCCGTCCCGTCGACCCCCGGTCCCCCACCCAGTCCCCC
GAGGAGGTCCGCAAATGCAAATTCCAAGAACCCTGGAAATTCCTCAAATGCTACCGCCAAAAATCAGACATGCA
TCCCAGCTGGATCATGATCATTGGGATCGTGAACATTCTGGCCTGCACCCTCATCTCCTTTGTGATTTACCCCT
GCTTTGACTTTGGTTGGAACTCGCCAGAGGCGCTCTATCTCCCGCCTGAACCTGACACACCACCACAGCAACCT
CAGGCACACGCACTACCACCACTACAGCCTAGGCCACAATACATGCCCATATTAGACTATGAGGCCGAGCCACA
GCGACCCATGCTCCCCGCTATTAGTTACTTCAATCTAACCGGCGGAGATGACTGACCCACTGGCCAACAACAAC
GTCAACGACCTTCTCCTGGACATGGACGGCCGCGCCTCGGAGCAGCGACTCGCCCAACTTCGCATTCGCCAGCA
GCAGGAGAGAGCCGTCAAGGAGCTGCAGGATGCGGTGGCCATCCACCAGTGCAAGAGAGGCATCTTCTGCCTGG
TGAAACAGGCCAAGATCTCCTACGAGGTCACTCCAAACGACCATCGCCTCTCCTACGAGCTCCTGCAGCAGCGC
CAGAAGTTCACCTGCCTGGTCGGAGTCAACCCCATCGTCATCACCCAGCAGTCTGGCGATACCAAGGGGTGCAT
CCACTGCTCCTGCGACTCCCCCGACTGCGTCCACACTCTGATCAAGACCCTCTGCGGCCTCCGCGACCTCCTCC
CCATGAACTAATCACCCCCTTATCCAGTGAAATAAAGATCATATTGATGATGATTTTACAGAAATAAAAAATAA
TCATTTGATTTGAAATAAAGATACAATCATATTGATGATTTGAGTTTAACAAAAAAATAAAGAATCACTTACTT
GAAATCTGATACCAGGTCTCTGTCCATGTTTTCTGCCAACACCACTTCACTCCCCTCTTCCCAGCTCTGGTACT
GCAGGCCCCGGCGGGCTGCAAACTTCCTCCACACGCTGAAGGGGATGTCAAATTCCTCCTGTCCCTCAATCTTC
ATTTTATCTTCTATCAGATGTCCAAAAAGCGCGTCCGGGTGGATGATGACTTCGACCCCGTCTACCCCTACGAT
GCAGACAACGCACCGACCGTGCCCTTCATCAACCCCCCCTTCGTCTCTTCAGATGGATTCCAAGAGAAGCCCCT
GGGGGGTGTTGTCCCTGCGACTGGCCGACCCCGTCACCACCAAGAACGGGGAAATCACCCTCAAGCTGGGAGAGG
GGGTGGACCTCGATTCCTCGGGAAAACTCATCTCCAACACGGCCACCAAGGCCGCCGCCCCTCTCAGTTTTTCC
AACAACACCATTTCCCTTAACATGGATCACCCCTTTTACACTAAAGATGGAAAATTATCCTTACAAGTTTCTCC
ACCATTAAATATACTGAGAACAAGCATTCTAAACACACTAGCTTTAGGTTTTGGATCAGGTTTAGGACTCCGTG
GCTCTGCCTTGGCAGTACAGTTAGTCTCTCCACTTACATTTGATACTGATGGAAACATAAAGCTTACCTTAGAC
AGAGGTTTGCATGTTACAACAGGAGATGCAATTGAAAGCAACATAAGCTGGGCTAAAGGTTTAAAATTTGAAGA
TGGAGCCATAGCAACCAACATTGGAAATGGGTTAGAGTTTGGAAGCAGTAGTACAGAAACAGGTGTTGATGATG
CTTACCCAATCCAAGTTAAACTTGGATCTGGCCTTAGCTTTGACAGTACAGGAGCCATAATGGCTGGTAACAAA
GAAGACGATAAACTCACTTTGTGGACAACACCTGATCCATCACCAAACTGTCAAATACTCGCAGAAAATGATGC
AAAACTAACACTTTGCTTGACTAAATGTGGTAGTCAAATACTGGCCACTGTGTCAGTCTTAGTTGTAGGAAGTG
GAAACCTAAACCCCATTACTGGCACCGTAAGCAGTGCTCAGGTGTTTCTACGTTTTGATGCAAACGGTGTTCTT
TTAACAGAACATTCTACACTAAAAAAATACTGGGGGTATAGGCAGGGAGATAGCATAGATGGCACTCCATATAC
CAATGCTGTAGGATTCATGCCCAATTTAAAAGCTTATCCAAAGTCACAAAGTTCTACTACTAAAAATAATATAG
TAGGGCAAGTATACATGAATGGAGATGTTTCAAAACCTATGCTTCTCACTATAACCCTCAATGGTACTGATGAC
AGCAACAGTACATATTCAATGTCATTTTCATACACCTGGACTAATGGAAGCTATGTTGGAGCAACATTTGGGGC
TAACTCTTATACCTTCTCATACATCGCCCAAGAATGAACACTGTATCCCACCCTGCATGCCAACCCTTCCCACC
CCACTCTGTGGAACAAACTCTGAAACACAAAATAAATAAAGTTCAAGTGTTTTATTGATTCAACAGTTTTACA
GGATTCGAGCAGTTATTTTTCCTCCACCCTCCCAGGACATGGAATACACCACCCTCTCCCCCCGCACAGCCTTG
AACATCTGAATGCCATTGGTGATGGACATGCTTTTGGTCTCCACGTTCCACACAGTTTCAGAGCGAGCCAGTCT
CGGGTCGGTCAGGGAGATGAAACCCTCCGGGCACTCCCGCATCTGCACCTCACAGCTCAACAGCTGAGGATTGT
CCTCGGTGGTCGGGATCACGGTTATCTGGAAGAAGCAGAAGAGCGGCGGTGGGAATCATAGTCCGCGAACGGGA
TCGGCCGGTGGTGTCGCATCAGGCCCCGCAGCAGTCGCTGCCGCCGCCGCTCCGTCAAGCTGCTGCTCAGGGGG
TCCGGGTCCAGGGACTCCCTCAGCATGATGCCCACGGCCCTCAGCATCAGTCGTCTGGTGCGGCGGGCGCAGCA
GCGCATGCGGATCTCGCTCAGGTCGCTGCAGTACGTGCAACACAGAACCACCAGGTTGTTCAACAGTCCATAGT
TCAACACGCTCCAGCCGAAACTCATCGCGGGAAGGATGCTACCCACGTGGCCGTCGTACCAGATCCTCAGGTAA
ATCAAGTGGTGCCCCCTCCAGAACACGCTGCCCACGTACATGATCTCCTTGGGCATGTGGCGGTTCACCACCTC
CCGGTACCACATCACCCTCTGGTTGAACATGCAGCCCCGGATGATCCTGCGGAACCACAGGGCCAGCACCGCCC
CGCCCGCCATGCAGCGAAGAGACCCCGGGTCCCGGCAATGGCAATGGAGGACCCACCGCTCGTACCCGTGGATC
ATCTGGGAGCTGAACAAGTCTATGTTGGCACAGCACAGGCATATGCTCATGCATCTCTTCAGCACTCTCAACTC
CTCGGGGGTCAAAACCATATCCCAGGGCACGGGGAACTCTTGCAGGACAGCGAACCCCGCAGAACAGGGCAATC
CTCGCACAGAACTTACATTGTGCATGGACAGGGTATCGCAATCAGGCAGCACCGGGTGATCCTCCACCAGAGAA
GCGCGGGTCTCGGTCTCCTCACAGCGTGGTAAGGGGGCCGGCCGATACGGGTGATGGCGGGACGCGGCTGATCG
```

```
TGTTCGCGACCGTGTCATGATGCAGTTGCTTTCGGACATTTTCGTACTTGCTGTAGCAGAACCTGGTCCGGGCG
CTGCACACCGATCGCCGGCGGCGGTCTCGGCGCTTGGAACGCTCGGTGTTGAAATTGTAAAACAGCCACTCTCT
CAGACCGTGCAGCAGATCTAGGGCCTCAGGAGTGATGAAGATCCCATCATGCCTGATGGCTCTGATCACATCGA
CCACCGTGGAATGGGCCAGACCCAGCCAGATGATGCAATTTTGTTGGGTTTCGGTGACGGCGGGGGAGGGAAGA
ACAGGAAGAACCATGATTAACTTTTAATCCAAACGGTCTCGGAGTACTTCAAAATGAAGATCGCGGAGATGGCA
CCTCTCGCCCCCGCTGTGTTGGTGGAAAATAACAGCCAGGTCAAAGGTGATACGGTTCTCGAGATGTTCCACGG
TGGCTTCCAGCAAAGCCTCCACGCGCACATCCAGAAACAAGACAATAGCGAAAGCGGGAGGGTTCTCTAATTCC
TCAATCATCATGTTACACTCCTGCACCATCCCCAGATAATTTTCATTTTTCCAGCCTTGAATGATTCGAACTAG
TTCcTGAGGTAAATCCAAGCCAGCCATGATAAAGAGCTCGCGCAGAGCGCCCTCCACCGGCATTCTTAAGCACA
CCCTCATAATTCCAAGATATTCTGCTCCTGGTTCACCTGCAGCAGATTGACAAGCGGAATATCAAAATCTCTGC
CGCGATCCCTGAGCTCCTCCCTCAGCAATAACTGTAAGTACTCTTTCATATCCTCTCCGAAATTTTTAGCCATA
GGACCACCAGGAATAAGATTAGGGCAAGCCACAGTACAGATAAACCGAAGTCCTCCCCAGTGAGCATTGCCAAA
TGCAAGACTGCTATAAGCATGCTGGCTAGACCCGGTGATATCTTCCAGATAACTGGACAGAAAATCGCCCAGGC
AATTTTTAAGAAAATCAACAAAAGAAAAATCCTCCAGGTGGACGTTTAGAGCCTCGGGAACAACGATGAAGTAA
ATGCAAGCGGTGCGTTCCAGCATGGTTAGTTAGCTGATCTGTAGAAAAAACAAAAATGAACATTAAACCATGCT
AGCCTGGCGAACAGGTGGGTAAATCGTTCTCTCCAGCACCAGGCAGGCCACGGGGTCTCCGGCGCGACCCTCGT
AAAAATTGTCGCTATGATTGAAAACCATCACAGAGAGACGTTCCCGGTGGCCGGCGTGAATGATTCGACAAGAT
GAATACACCCCCGGAACATTGGCGTCCGCGAGTGAAAAAAAGCGCCCGAGGAAGCAATAAGGCACTACAATGCT
CAGTCTCAAGTCCAGCAAAGCGATGCCATGCGGATGAAGCACAAAATTCTCAGGTGCGTACAAAATGTAATTAC
TCCCCTCCTGCACAGGCAGCAAAGCCCCCGATCCCTCCAGGTACACATACAAAGCCTCAGCGTCCATAGCTTAC
CGAGCAGCAGCACACAACAGGCGCAAGAGTCAGAGAAAGGCTGAGCTCTAACCTGTCCACCCGCTCTCTGCTCA
ATATATAGCCCAGATCTACACTGACGTAAAGGCCAAAGTCTAAAAATACCCGCCAAATAATCACACACGCCCAG
CACACGCCCAGAAACCGGTGACACACTCAAAAAAATACGCGCACTTCCTCAAACGCCCAAAACTGCCGTCATTT
CCGGGTTCCCACGCTACGTCATCAAAACACGACTTTCAAATTCCGTCGACCGTTAAAAACGTCACCCGCCCCGC
CCCTAACGGTCGCCCGTCTCTCAGCCAATCAGCGCCCCGCATCCCCAAATTCAAACACCTCATTTGCATATTAA
CGCGCACAAAAGTTTGAGGTATATTATTGATGATGG
```

ChAdV68.4WTnt.GFP (SEQ ID NO:11); AC_000011.1 with E1 (nt 577 to 3403) and E3 (nt 27,816- 31,332) sequences deleted; corresponding ATCC VR-594 nucleotides substituted at four positions; GFP reporter under the control of the CMV promoter/enhancer inserted in place of deleted E1

```
CCATCTTCAATAATATACCTCAAACTTTTTGTGCGCGTTAATATGCAAATGAGGCGTTTGAATTTGGGGAGGAA
GGGCGGTGATTGGTCGAGGGATGAGCGACCGTTAGGGGCGGGGCGAGTGACGTTTTGATGACGTGGTTGCGAGG
AGGAGCCAGTTTGCAAGTTCTCGTGGGAAAAGTGACGTCAAACGAGGTGTGGTTTGAACACGGAAATACTCAAT
TTTCCCGCGCTCTCTGACAGGAAATGAGGTGTTTCTGGGCGGATGCAAGTGAAAACGGGCCATTTTCGCGCGAA
AACTGAATGAGGAAGTGAAAATCTGAGTAATTTCGCGTTTATGGCAGGGAGGAGTATTTGCCGAGGGCCGAGTA
GACTTTGACCGATTACGTGGGGGTTTCGATTACCGTGTTTTTCACCTAAATTTCCGCGTACGGTGTCAAAGTCC
GGTGTTTTTACGTAGGTGTCAGCTGATCGCCAGGGTATTTAAACCTGCGCTCTCCAGTCAAGAGGCCACTCTTG
AGTGCCAGCGAGAAGAGTTTTCTCCTCCGCGCCGCGAGTCAGATCTACACTTTGAAAGTAGGGATAACAGGGTA
ATGgacattgattattgactagttGttaaTAGTAATCAATTACGGGGTCATTAGTTCATAGCCCATATATGGAGT
TCCGCGTTACATAACTTACGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTGGAGTATTTACGGTAAAC
TGCCCACTTGGCAGTACATCAAGTGTATCATATGCCAAGTCCGCCCCCTATTGACGTCAATGACGGTAAATGGC
CCGCCTGGCATTATGCCCAGTACATGACCTTACGGGACTTTCCTACTTGGCAGTACATCTACGTATTAGTCATC
GCTATTACCATGgTGATGCGGTTTTGGCAGTACACCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCAAAATGTCGTA
ATAACCCCGCCCCGTTGACGCAAATGGGCGGTAGGCGTGTACGGTGGGAGGTCTATATAAGCAGAgcTCGTTTA
GTGAACCGTCAGATCGCCTGGAACGCCATCCACGCTGTTTTGACCTCCATAGAAGACAGCGATCGCGccaccAT
GGTGAGCAAGGGCGAGGAGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCCTGAAGTTCATCTGCACC
ACCGGCAAGCTGCCCGTGCCCTGGCCCACCCTCGTGACCACCCTGACCTACGGCGTGCAGTGCTTCAGCCGCTA
CCCCGACCACATGAAGCAGCACGACTTCTTCAAGTCCGCCATGCCCGAAGGCTACGTCCAGGAGCGCACCATCT
TCTTCAAGGACGACGGCAACTACAAGACCCGCGCCGAGGTGAAGTTCGAGGGCGACACCCTGGTGAACCGCATC
GAGCTGAAGGGCATCGACTTCAAGGAGGACGGCAACATCCTGGGGCACAAGCTGGAGTACAACTACAACAGCCA
CAACGTCTATATCATGGCCGACAAGCAGAAGAACGGCATCAAGGTGAACTTCAAGATCCGCCACAACATCGAGG
ACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGA
GTTCGTGACCGCCGCCGGGATCACTCTCGGCATGGACGAGCTtTACAAGTAGtgaGTTTAAACTCCCATTTAAA
TGTGAGGGTTAATGCTTCGAGCAGACATGATAAGATACATTGATGAGTTTGGACAAACCACAACTAGAATGCAG
TGAAAAAAATGCTTTATTTGTGAAATTTGTGATGCTATTGCTTTATTTGTAACCATTATAAGCTGCAATAAACA
AGTTAACAACAACAATTGCATTCATTTTATGTTTCAGGTTCAGGGGGAGATGTGGGAGGTTTTTTAAAGCAAGT
AAAACCTCTACAAATGTGGTAAAATAACTATAACGGTCCTAAGGTAGCGAGTGAGTAGTGTTCTGGGGCGGGGG
AGGACCTGCATGAGGGCCAGAATAACTGAAATCTGTGCTTTTCTGTGTGTTGCAGCAGCATGAGCGGAAGCGGC
```

(continued)

```
TCCTTTGAGGGAGGGGTATTCAGCCCTTATCTGACGGGGCGTCTCCCCTCCTGGGCGGGAGTGCGTCAGAATGT
GATGGGATCCACGGTGGACGGCCGGCCCGTGCAGCCCGCGAACTCTTCAACCCTGACCTATGCAACCCTGAGCT
CTTCGTCGTTGGACGCAGCTGCCGCCGCAGCTGCTGCATCTGCCGCCAGCGCCGTGCGCGGAATGGCCATGGGC
GCCGGCTACTACGGCACTCTGGTGGCCAACTCGAGTTCCACCAATAATCCCGCCAGCCTGAACGAGGAGAAGCT
GTTGCTGCTGATGGCCCAGCTCGAGGCCTTGACCCAGCGCCTGGGCGAGCTGACCCAGCAGGTGGCTCAGCTGC
AGGAGCAGACGCGGGCCGCGGTTGCCACGGTGAAATCCAAATAAAAAATGAATCAATAAATAAACGGAGACGGT
TGTTGATTTTAACACAGAGTCTGAATCTTTATTTGATTTTTCGCGCGCGGTAGGCCCTGGACCACCGGTCTCGA
TCATTGAGCACCGGTGGATCTTTTCCAGGACCCGGTAGAGGTGGGCTTGGATGTTGAGGTACATGGGCATGAG
CCCGTCCCGGGGGTGGAGGTAGCTCCATTGCAGGGCCTCGTGCTCGGGGGTGGTGTTGTAAATCACCCAGTCAT
AGCAGGGGCGCAGGGCATGGTGTTGCACAATATCTTTGAGGAGGAGACTGATGGCCACGGGCAGCCCTTTGGTG
TAGGTGTTTACAAATCTGTTGAGCTGGGAGGGATGCATGCGGGGGGAGATGAGGTGCATCTTGGCCTGGATCTT
GAGATTGGCGATGTTACCGCCCAGATCCCGCCTGGGGTTCATGTTGTGCAGGACCACCAGCACGGTGTATCCGG
TGCACTTGGGGAATTTATCATGCAACTTGGAAGGGAAGGCGTGAAAGAATTTGGCGACGCCTTTGTGCCCGCCC
AGGTTTTCCATGCACTCATCCATGATGATGGCGATGGGCCCGTGGGCGGCGGCCTGGGCAAAGACGTTTCGGGG
GTCGGACACATCATAGTTGTGGTCCTGGGTGAGGTCATCATAGGCCATTTTAATGAATTTGGGGCGGAGGGTGC
CGGACTGGGGGACAAAGGTACCCTCGATCCCGGGGGCGTAGTTCCCCTCACAGATCTGCATCTCCCAGGCTTTG
AGCTCGGAGGGGGGGATCATGTCCACCTGCGGGGCGATAAAGAACACGGTTTCCGGGGCGGGGGAGATGAGCTG
GGCCGAAAGCAAGTTCCGGAGCAGCTGGGACTTGCCGCAGCCGGTGGGGCCGTAGATGACCCCGATGACCGGCT
GCAGGTGGTAGTTGAGGGAGAGACAGCTGCCGTCCTCCCGGAGGAGGGGGGGCCACCTCGTTCATCATCTCGCGC
ACGTGCATGTTCTCGCGCACCAGTTCCGCCAGGAGGCGCTCTCCCCCCAGGGATAGGAGCTCCTGGAGCGAGGC
GAAGTTTTTCAGCGGCTTGAGTCCGTCGGCCATGGGCATTTTGGAGAGGGTTTGTTGCAAGAGTTCCAGGCGGT
CCCAGAGCTCGGTGATGTGCTCTACGGCATCTCGATCCAGCAGACCTCCTCGTTTCGCGGGTTGGGACGGCTGC
GGGAGTAGGGCACCAGACGATGGGCGTCCAGCGCAGCCAGGGTCCGGTCCTTCCAGGGTCGCAGCGTCCGCGTC
AGGGTGGTCTCCGTCACGGTGAAGGGGTGCGCGCCGGGCTGGGCGCTTGCGAGGGTGCGCTTCAGGCTCATCCG
GCTGGTCGAAAACCGCTCCCGATCGGCGCCCTGCGCGTCGGCCAGGTAGCAATTGACCATGAGTTCGTAGTTGA
GCGCCTCGGCCGCGTGGCCTTTGGCGCGGAGCTTACCTTTGGAAGTCTGCCCGCAGGCGGGACAGAGGAGGGAC
TTGAGGGCGTAGAGCTTGGGGGCGAGGAAGACGGACTCGGGGGCGTAGGCGTCCGCGCCGCAGTGGGCGCAGAC
GGTCTCGCACTCCACGAGCCAGGTGAGGTCGGGCTGGTCGGGGTCAAAAACCAGTTTCCCGCCGTTCTTTTTGA
TGCGTTTCTTACCTTTGGTCTCCATGAGCTCGTGTCCCCGCTGGGTGACAAAGAGGCTGTCCGTGTCCCCGTAG
ACCGACTTTATGGGCCGGTCCTCGAGCGGTGTGCCGCGGTCCTCCTCGTAGAGGAACCCCGCCCACTCCGAGAC
GAAAGCCCGGGTCCAGGCCAGCACGAAGGAGGCCACGTGGGACGGGTAGCGGTCGTTGTCCACCAGCGGGTCCA
CCTTTTCCAGGGTATGCAAACACATGTCCCCCTCGTCCACATCCAGGAAGGTGATTGGCTTGTAAGTGTAGGCC
ACGTGACCGGGGGTCCCGGCCGGGGGGGGTATAAAAGGGTGCGGGTCCCTGCTCGTCCTCACTGTCTTCCGGATC
GCTGTCCAGGAGCGCCAGCTGTTGGGGTAGGTATTCCCTCTCGAAGGCGGGCATGACCTCGGCACTCAGGTTGT
CAGTTTCTAGAAACGAGGAGGATTTGATATTGACGGTGCCGGCGGAGATGCCTTTCAAGAGCCCCTCGTCCATC
TGGTCAGAAAAGACGATCTTTTTGTTGTCGAGCTTGGTGGCGAAGGAGCCGTAGAGGGCGTTGGAGAGGAGCTT
GGCGATGGAGCGCATGGTCTGGTTTTTTTCCTTGTCGGCGCGCTCCTTGGCGGCGATGTTGAGCTGCACGTACT
CGCGCGCCACGCACTTCCATTCGGGGAAGACGGTGGTCAGCTCGTCGGGCACGATTCTGACCTGCCAGCCCCGA
TTATGCAGGGTGATGAGGTCCACACTGGTGGCCACCTCGCCGCGCAGGGGCTCATTAGTCCAGCAGAGGCGTCC
GCCCTTGCGCGAGCAGAAGGGGGGCAGGGGGTCCAGCATGACCTCGTCGGGGGGGTCGGCATCGATGGTGAAGA
TGCCGGGCAGGAGGTCGGGGTCAAAGTAGCTGATGGAAGTGGCCAGATCGTCCAGGCAGCTTGCCATTCGCGC
ACGGCCAGCGCGCtCTCGTAGGGACTGAGGGGCGTGCCCCAGGGCATGGGATGGGTAAGCGCGGAGGCGTACAT
GCCGCAGATGTCGTAGACGTAGAGGGGCTCCTCGAGGATGCCGATGTAGGTGGGGTAGCAGCGCCCCCCGCGGA
TGCTGGCGCGCACGTAGTCATACAGCTCGTGCGAGGGGCGAGGAGCCCCGGGCCCAGGTTGGTGCGACTGGGC
TTTTCGGCGCGGTAGACGATCTGGCGGAAAATGGCATGCGAGTTGGAGGAGATGGTGGGCCTTTGGAAGATGTT
GAAGTGGGCGTGGGGCAGTCCGACCGAGTCGCGGATGAAGTGGGCGTAGGAGTCTTGCAGCTTGGCGACGAGCT
CGGCGGTGACTAGGACGTCCAGAGCGCAGTAGTCGAGGGTCTCCTGGATGATGTCATACTTGAGCTGTCCCTTT
TGTTTCCACAGCTCGCGGTTGAGAAGGAACTCTTCGCGGTCCTTCCAGTACTCTTCGAGGGGGAACCCGTCCTG
ATCTGCACGGTAAGAGCCTAGCATGTAGAACTGGTTGACGGCCTTGTAGGCGCAGCAGCCCTTCTCCACGGGGA
GGGCGTAGGCCTGGGCGGCCTTGCGCAGGGAGGTGTGCGTGAGGGCGAAAGTGTCCCTGACCATGACCTTGAGG
AACTGGTGCTTGAAGTCGATATCGTCGCAGCCCCCTGCTCCCAGAGCTGGAAGTCCGTGCGCTTCTTGTAGGC
GGGGTTGGGCAAAGCGAAAGTAACATCGTTGAAGAGGATCTTGCCCGCGCGGGGCATAAAGTTGCGAGTGATGC
GGAAAGGTTGGGGCACCTCGGCCCGGTTGTTGATGACCTGGGCGGCGAGCACGATCTCGTCGAAGCCGTTGATG
TTGTGGCCCACGATGTAGAGTTCCACGAATCGCGGACGGCCCTTGACGTGGGGCAGTTTCTTGAGCTCCTCGTA
GGTGAGCTCGTCGGGGTCGCTGAGCCCGTGCTGCTCGAGCGCCCAGTCGGCGAGATGGGGGTTGGCGCGGAGGA
AGGAAGTCCAGAGATCCACGGCCAGGGCGGTTTGCAGACGGTCCCGGTACTGACGGAACTGCTGCCCGACGGCC
ATTTTTTCGGGGGTGACGCAGTAGAAGGTGCGGGGGTCCCCGTGCCAGCGATCCCATTTGAGCTGGAGGGCGAG
ATCGAGGGCGAGCTCGACGAGCCGGTCGTCCCCGGAGAGTTTCATGACCAGCATGAAGGGGACGAGCTGCTTGC
CGAAGGACCCCATCCAGGTGTAGGTTTCCACATCGTAGGTGAGGAAGAGCCTTTCGGTGCGAGGATGCGAGCCG
ATGGGGAAGAACTGGATCTCCTGCCACCAATTGGAGGAATGGCTGTTGATGTGATGGAAGTAGAAATGCCGACG
GCGCGCCGAACACTCGTGCTTGTGTTTATACAAGCGGCCACAGTGCTCGCAACGCTGCACGGGATGCACGTGCT
GCACGAGCTGTACCTGAGTTCCTTTGACGAGGAATTTCAGTGGGAAGTGGAGTCGTGGCGCCTGCATCTCGTGC
```

```
TGTACTACGTCGTGGTGGTCGGCCTGGCCCTCTTCTGCCTCGATGGTGGTCATGCTGACGAGCCCGCGCGGGAG
GCAGGTCCAGACCTCGGCGCGAGCGGGTCGGAGAGCGAGGACGAGGGCGCGCAGGCCGGAGCTGTCCAGGGTCC
TGAGACGCTGCGGAGTCAGGTCAGTGGGCAGCGGCGGCGCGCGGTTGACTTGCAGGAGTTTTTCCAGGGCGCGC
GGGAGGTCCAGATGGTACTTGATCTCCACCGCGCCATTGGTGGCGACGTCGATGGCTTGCAGGGTCCCGTGCCC
CTGGGGTGTGACCACCGTCCCCCGTTTCTTCTTGGGCGGCTGGGGCGACGGGGCGGTGCCTCTTCCATGGTTA
GAAGCGGCGGCGAGGACGCGCGCCGGGCGGCAGGGGCGGCTCGGGGCCCGGAGGCAGGGCGGCAGGGGCACGT
CGGCGCCGCGCGCGGGTAGGTTCTGGTACTGCGCCCGGAGAAGACTGGCGTGAGCGACGACGCGACGGTTGACG
TCCTGGATCTGACGCCTCTGGGTGAAGGCCACGGGACCCGTGAGTTTGAACCTGAAAGAGAGTTCGACAGAATC
AATCTCGGTATCGTTGACGGCGGCCTGCCGCAGGATCTCTTGCACGTCGCCCGAGTTGTCCTGGTAGGCGATCT
CGGTCATGAACTGCTCGATCTCCTCCTCTTGAAGGTCTCCGCGGCCGGCGCGCTCCACGGTGGCCGCGAGGTCG
TTGGAGATGCGGCCCATGAGCTGCGAGAAGGCGTTCATGCCCGCCTCGTTCCAGACGCGGCTGTAGACCACGAC
GCCCTCGGGATCGCgGGCGCGCATGACCACCTGGGCGAGGTTGAGCTCCACGTGGCGCGTGAAGACCGCGTAGT
TGCAGAGGCGCTGGTAGAGGTAGTTGAGCGTGGTGGCGATGTGCTCGGTGACGAAGAAATACATGATCCAGCGG
CGGAGCGGCATCTCGCTGACGTCGCCCAGCGCCTCCAAACGTTCCATGGCCTCGTAAAAGTCCACGGCGAAGTT
GAAAAACTGGGAGTTGCGCGCCGAGACGGTCAACTCCTCCTCCAGAAGACGGATGAGCTCGGCGATGGTGGCGC
GCACCTCGCGCTCGAAGGCCCCGGGAGTTCCTCCACTTCCTCTTCTTCCTCCTCCACTAACATCTCTTCTACT
TCCTCCTCAGGCGGCAGTGGTGGCGGGGGAGGGGGCCTGCGTCGCCGGCGGCGCACGGGCAGACGGTCGATGAA
GCGCTCGATGGTCTCGCCGCGCCGGCGTCGCATGGTCTCGGTGACGGCGCGCCCGTCCTCGCGGGGCCGCAGCG
TGAAGACGCCGCCGCGCATCTCCAGGTGGCCGGGGGGGTCCCCGTTGGGCAGGGAGAGGGCGCTGACGATGCAT
CTTATCAATTGCCCCGTAGGGACTCCGCGCAAGGACCTGAGCGTCTCGAGATCCACGGGATCTGAAAACCGCTG
AACGAAGGCTTCGAGCCAGTCGCAGTCGCAAGGTAGGCTGAGCACGGTTTCTTCTGGCGGGTCATGTTGGTTGG
GAGCGGGGCGGGCGATGCTGCTGGTGATGAAGTTGAAATAGGCGGTTCTGAGACGGCGGATGGTGGCGAGGAGC
ACCAGGTCTTTGGGCCCGGCTTGCTGGATGCGCAGACGGTCGGCCATGCCCCAGGCGTGGTCCTGACACCTGGC
CAGGTCCTTGTAGTAGTCCTGCATGAGCCGCTCCACGGGCACCTCCTCCTCGCCCGCGCGGCCGTGCATGCGCG
TGAGCCCGAAGCCGCGCTGGGGCTGGACGAGCGCCAGGTCGGCGACGACGCGCTCGGCGAGGATGGCTTGCTGG
ATCTGGGTGAGGGTGGTCTGGAAGTCATCAAAGTCGACGAAGCGGTGGTAGGCTCCGGTGTTGATGGTGTAGGA
GCAGTTGGCCATGACGGACCAGTTGACGGTCTGGTGGCCCGGACGCACGAGCTCGTGGTACTTGAGGCGCGAGT
AGGCGCGCGTGTCGAAGATGTAGTCGTTGCAGGTGCGCACCAGGTACTGGTAGCCGATGAGGAAGTGCGGCGGC
GGCTGGCGGTAGAGCGGCCATCGCTCGGTGGCGGGGGCGCCGGGCGCGAGGTCCTCGAGCATGGTGCGGTGGTA
GCCGTAGATGTACCTGGACATCCAGGTGATGCCGGCGGCGGTGGTGGAGGCGCGCGGGAACTCGCGGACGCGGT
TCCAGATGTTGCGCAGCGGCAGGAAGTAGTTCATGGTGGGCACGGTCTGGCCCGTGAGGCGCGCGCAGTCGTGG
ATGCTCTATACGGGCAAAAACGAAAGCGGTCAGCGGCTCGACTCCGTGGCCTGGAGGCTAAGCGAACGGGTTGG
GCTGCGCGTGTACCCCGGTTCGAATCTCGAATCAGGCTGGAGCCGCAGCTAACGTGGTATTGGCACTCCCGTCT
CGACCCAAGCCTGCACCAACCCTCCAGGATACGGAGGCGGGTCGTTTTGCAACTTTTTTTTGGAGGCCGGATGA
GACTAGTAAGCGCGGAAAGCGGCCGACCGCGATGGCTCGCTGCCGTAGTCTGGAGAAGAATCGCCAGGGTTGCG
TTGCGGTGTGCCCCGGTTCGAGGCCGGCCGGATTCCGCGGCTAACGAGGGCGTGGCTGCCCCGTCGTTTCCAAG
ACCCCATAGCCAGCCGACTTCTCCAGTTACGGAGCGAGCCCCTCTTTTGTTTTGTTTGTTTTTGCCAGATGCAT
CCCGTACTGCGGCAGATGCGCCCCCACCACCCTCCACCGCAACAACAGCCCCCTCCACAGCCGGCGCTTCTGCC
CCCGCCCCAGCAGCAACTTCCAGCCACGACCGCCGCGGCCGCCGTGAGCGGGGCTGGACAGAGTTATGATCACC
AGCTGGCCTTGGAAGAGGGCGAGGGGCTGGCGCGCCTGGGGGCGTCGTCGCCGGAGCGGCACCCGCGCGTGCAG
ATGAAAAGGGACGCTCGCGAGGCCTACGTGCCCAAGCAGAACCTGTTCAGAGACAGGAGCGGCGAGGAGCCCGA
GGAGATGCGCGCGGCCCGGTTCCACGCGGGGCGGGAGCTGCGGCGCGGCCTGGACCGAAAGAGGGTGCTGAGGG
ACGAGGATTTCGAGGCGGACGAGCTGACGGGGATCAGCCCCGCGCGCGCGCACGTGGCCGCGGCCAACCTGGTC
ACGGCGTACGAGCAGACCGTGAAGGAGGAGAGCAACTTCCAAAAATCCTTCAACAACCACGTGCGCACCCTGAT
CGCGCGCGAGGAGGTGACCCTGGGCCTGATGCACCTGTGGGACCTGCTGGAGGCCATCGTGCAGAACCCCACCA
GCAAGCCGCTGACGGCGCAGCTGTTCCTGGTGGTGCAGCATAGTCGGGACAACGAAGCGTTCAGGGAGGCGCTG
CTGAATATCACCGAGCCCGAGGGCCGCTGGCTCCTGGACCTGGTGAACATTCTGCAGAGCATCGTGGTGCAGGA
GCGCGGGCTGCCGCTGTCCGAGAAGCTGGCGGCCATCAACTTCTCGGTGCTGAGTTTGGGCAAGTACTACGCTA
GGAAGATCTACAAGACCCCGTACGTGCCCATAGACAAGGAGGTGAAGATCGACGGGTTTTACATGCGCATGACC
CTGAAAGTGCTGACCCTGAGCGACGATCTGGGGGTGTACCGCAACGACAGGATGCACCGTGCGGTGAGCGCCAG
CAGGCGGCGCGAGCTGAGCGACCAGGAGCTGATGCATAGTCTGCAGCGGGCCCTGACCGGGGCCGGGACCGAGG
GGGAGAGCTACTTTGACATGGGCGCGGACCTGCACTGGCAGCCCAGCCGCCGGGCCTTGGAGGCGGCGGCAGGA
CCCTACGTAGAAGAGGTGGACGATGAGGTGGACGAGGAGGGCGAGTACCTGGAAGACTGATGGCGCGACCGTAT
TTTTGCTAGATGCAACAACAACAGCCACCTCCTGATCCCGCGATGCGGGCGGCGCTGCAGAGCCAGCCGTCCGG
CATTAACTCCTCGGACGATTGGACCCAGGCCATGCAACGCATCATGGCGCTGACGACCCGCAACCCCGAAGCCT
TTAGACAGCAGCCCCAGGCCAACCGGCTCTCGGCCATCCTGGAGGCCGTGGTGCCCTCGCGCTCCAACCCCACG
CACGAGAAGGTCCTGGCCATCGTGAACGCGCTGGTGGAGAACAAGGCCATCCGCGGCGACGAGGCCGGCCTGGT
GTACAACGCGCTGCTGGAGCGCGTGGCCCGCTACAACAGCACCAACGTGCAGACCAACCTGGACCGCATGGTGA
CCGACGTGCGCGAGGCCGTGGCCCAGCGCGAGCGGTTCCACCGCGAGTCCAACCTGGGATCCATGGTGGCGCTG
AACGCCTTCCTCAGCACCCAGCCCGCCAACGTGCCCCGGGGCCAGGAGGACTACACCAACTTCATCAGCGCCCT
GCGCCTGATGGTGACCGAGGTGCCCCAGAGCGAGGTGTACCAGTCCGGGCCGGACTACTTCTTCCAGACCAGTC
GCCAGGGCTTGCAGACCGTGAACCTGAGCCAGGCTTTTCAAGAACTTGCAGGGCCTGTGGGGCGTGCAGGCCCCG
```

```
GTCGGGGACCGCGCGACGGTGTCGAGCCTGCTGACGCCGAACTCGCGCCTGCTGCTGCTGCTGGTGGCCCCCTT
CACGGACAGCGGCAGCATCAACCGCAACTCGTACCTGGGCTACCTGATTAACCTGTACCGCGAGGCCATCGGCC
AGGCGCACGTGGACGAGCAGACCTACCAGGAGATCACCCACGTGAGCCGCGCCCTGGGCCAGGACGACCGGGC
AACCTGGAAGCCACCCTGAACTTTTTGCTGACCAACCGGTCGCAGAAGATCCCGCCCCAGTACGCGCTCAGCAC
CGAGGAGGAGCGCATCCTGCGTTACGTGCAGCAGAGCGTGGGCCTGTTCCTGATGCAGGAGGGGGGCCACCCCCA
GCGCCGCGCTCGACATGACCGCGCGCAACATGGAGCCCAGCATGTACGCCAGCAACCGCCCGTTCATCAATAAA
CTGATGGACTACTTGCATCGGGCGGCCGCCATGAACTCTGACTATTTCACCAACGCCATCCTGAATCCCCACTG
GCTCCCGCCGCCGGGGTTCTACACGGGCGAGTACGACATGCCCGACCCCAATGACGGGTTCCTGTGGGACGATG
TGGACAGCAGCGTGTTCTCCCCCCGACCGGGTGCTAACGAGCGCCCCTTGTGGAAGAAGGAAGGCAGCGACCGA
CGCCCGTCCTCGGCGCTGTCCGGCCGCGAGGGTGCTGCCGCGGCGGTGCCCGAGGCCGCCAGTCCTTTCCCGAG
CTTGCCCTTCTCGCTGAACAGTATCCGCAGCAGCGAGCTGGGCAGGATCACGCGCCCGCGCTTGCTGGGCGAAG
AGGAGTACTTGAATGACTCGCTGTTGAGACCCGAGCGGGAGAAGAACTTCCCCAATAACGGGATAGAAAGCCTG
GTGGACAAGATGAGCCGCTGGAAGACGTATGCGCAGGAGCACAGGGACGATCCCCGGGCGTCGCAGGGGGCCAC
GAGCCGGGGCAGCGCCGCCCGTAAACGCCGGTGGCACGACAGGCAGCGGGGACAGATGTGGGACGATGAGGACT
CCGCCGACGACAGCAGCGTGTTGGACTTGGGTGGGAGTGGTAACCCGTTCGCTCACCTGCGCCCCCGTATCGGG
CGCATGATGTAAGAGAAACCGAAAATAAATGATACTCACCAAGGCCATGGCGACCAGCGTGCGTTCGTTTCTTC
TCTGTTGTTGTTGTATCTAGTATGATGAGGCGTGCGTACCGGAGGGTCCTCCTCCCTCGTACGAGAGCGTGAT
GCAGCAGGCGATGGCGGCGGCGGCGATGCAGCCCCGCTGGAGGCTCCTTACGTGCCCCCGCGGTACCTGGCGC
CTACGGAGGGGCGGAACAGCATTCGTTACTCGGAGCTGGCACCCTTGTACGATACCACCGGTTGTACCTGGTG
GACAACAAGTCGGCGGACATCGCCTCGCTGAACTACCAGAACGACCACAGCAACTTCCTGACCACCGTGGTGCA
GAACAATGACTTCACCCCCACGGAGGCCAGCACCCAGACCATCAACTTTGACGAGCGCTCGCGGTGGGGCGGCC
AGCTGAAAACCATCATGCACACCAACATGCCCAACGTGAACGAGTTCATGTACAGCAACAAGTTCAAGGCGCGG
GTGATGGTCTCCCGCAAGACCCCCAATGGGGTGACAGTGACAGAGGATTATGATGGTAGTCAGGATGAGCTGAA
GTATGAATGGGTGGAATTTGAGCTGCCCGAAGGCAACTTCTCGGTGACCATGACCATCGACCTGATGAACAACG
CCATCATCGACAATTACTTGGCGGTGGGGCGGCAGAACGGGGTGCTGGAGAGCGACATCGGCGTGAAGTTCGAC
ACTAGGAACTTCAGGCTGGGCTGGGACCCCGTGACCGAGCTGGTCATGCCCGGGGTGTACACCAACGAGGCTTT
CCATCCCGATATTGTCTTGCTGCCCGGCTGCGGGGTGGACTTCACCGAGAGCCGCCTCAGCAACCTGCTGGGCA
TTCGCAAGAGGCAGCCCTTCCAGGAAGGCTTCCAGATCATGTACGAGGATCTGGAGGGGGGGCAACATCCCCGCG
CTCCTGGATGTCGACGCCTATGAGAAAAGCAAGGAGGATGCAGCAGCTGAAGCAACTGCAGCCGTAGCTACCGC
CTCTACCGAGGTCAGGGGCGATAATTTTGCAAGCGCCGCAGCAGTGGCAGCGGCCGAGGCGGCTGAAACCGAAA
GTAAGATAGTCATTCAGCCGGTGGAGAAGGATAGCAAGAACAGGAGCTACAACGTACTACCGGACAAGATAAAC
ACCGCCTACCGCAGCTGGTACCTAGCCTACAACTATGGCGACCCCGAGAAGGGCGTGCGCTCCTGGACGCTGCT
CACCACCTCGGACGTCACCTGCGGCGTGGAGCAAGTCTACTGGTCGCTGCCCGACATGATGCAAGACCCGGTCA
CCTTCCGCTCCACGCGTCAAGTTAGCAACTACCCGGTGGTGGGCGCCGAGCTCCTGCCCGTCTACTCCAAGAGC
TTCTTCAACGAGCAGGCCGTCTACTCGCAGCAGCTGCGCGCCTTCACCTCGCTTACGCACGTCTTCAACCGCTT
CCCCGAGAACCAGATCCTCGTCCGCCCGCCCGCGCCCACCATTACCACCGTCAGTGAAAACGTTCCTGCTCTCA
CAGATCACGGGACCCTGCCGCTGCGCAGCAGTATCCGGGGAGTCCAGCGCGTGACCGTTACTGACGCCAGACGC
CGCCACCTGCCCCTACGTCTACAAGGCCCTGGGCATAGTCGCGCCGCGCGTCCTCTCGAGCCGCACCTTCTAAAT
GTCCATTCTCATCTCGCCCAGTAATAACACCGGTTGGGGCCTGCGCGCGCCCAGCAAGATGTACGGAGGCGCTC
GCCAACGCTCCACGCAACACCCCGTGCGCGTGCGCGGGCACTTCCGCGCTCCCTGGGGCGCCCTCAAGGGCCGC
GTGCGGTCGCGCACCACCGTCGACGACGTGATCGACCAGGTGGTGGCCGACGCGCGCAACTACACCCCCGCCGC
CGCGCCCGTCTCCACCGTGGACGCCGTCATCGACAGCGTGGTGGCcGACGCGCGCCGGTACGCCCGCGCCAAGA
GCCGGCGGCGGCGCATCGCCCGGCGGCACCGGAGCACCCCCGCCATGCGCGCGGCGCGAGCCTTGCTGCGCAGG
GCCAGGCGCACGGGACGCAGGGCCATGCTCAGGGCGGCCAGACGCGCGGCTTCAGGCGCCAGCGCCGGCAGGAC
CCGGAGACGCGCGGCCACGGCGGCGGCAGCGGCCATCGCCAGCATGTCCCGCCCGCGGCGAGGGAACGTGTACT
GGGTGCGCGACGCCGCCACCGGTGTGCGCGTGCCCGTGCGCACCCGCCCCCCTCGCACTTGAAGATGTTCACTT
CGCGATGTTGATGTGTCCCAGCGGCGAGGAGGATGTCCAAGCGCAAATTCAAGGAAGAGATGCTCCAGGTCATC
GCGCCTGAGATCTACGGCCCTGCGGTGGTGAAGGAGGAAAGAAAGCCCGCAAAATCAAGCGGGTCAAAAAGGA
CAAAAAGGAAGAAGAAAGTGATGTGGACGGATTGGTGGAGTTTGTGCGCGAGTTCGCCCCCCGGCGGCGCGTGC
AGTGGCGCGGGCGGAAGGTGCAACCGGTGCTGAGACCCGGCACCACCGTGGTCTTCACGCCCGGCGAGCGCTCC
GGCACCGCTTCCAAGCGCTCCTACGACGAGGTGTACGGGGATGATGATATTCTGGAGCAGGCGGCCGAGCGCCT
GGGCGAGTTTGCTTACGGCAAGCGCAGCCGTTCCGCACCGAAGGAAGAGGCGGTGTCCATCCCGCTGGACCACG
GCAACCCCACGCCGAGCCTCAAGCCCGTGACCTTGCAGCAGGTGCTGCCGACCGCGGCGCCGCGCCGGGGGTTC
AAGCGCGAGGGCGAGGATCTGTACCCCACCATGCAGCTGATGGTGCCCAAGCGCCAGAAGCTGGAAGACGTGCT
GGAGACCATGAAGGTGGACCCGGACGTGCAGCCCGAGGTCAAGGTGCGGCCCATCAAGCAGGTGGCCCCGGGCC
TGGGCGTGCAGACCGTGGACATCAAGATTCCCACGGAGCCCATGGAAACGCAGACCGAGCCCATGATCAAGCCC
AGCACCAGCACCATGGAGGTGCAGACGGATCCCTGGATGCCATCGGCTCCTAGTCGAAGACCCCGGCGCAAGTA
CGGCGCGGCCAGCCTGCTGATGCCCAACTACGCGCTGCATCCTTCCATCATCCCCACGCCGGGCTACCGCGGCA
CGCGCTTCTACCGCGGTCATACCAGCAGCCGCCGCCGCAAGACCACCACTCGCCGCCGCCGTCGCCGCACCGCC
GCTGCAACCACCCCTGCCGCCCTGGTGCGGAGAGTGTACCGCCGCGGCCGCGCACCTCTGACCCTGCCGCGCGC
GCGCTACCACCCGAGCATCGCCATTTAAACTTTCGCCtGCTTTGCAGATCAATGGCCCTCACATGCCGCCTTCG
CGTTCCCATTACGGGCTACCGAGGAAGAAAACCGCGCCGTAGAAGGCTGGCGGGGAACGGGATGCGTCGCCACC
```

```
ACCACCGGCGGCGGCGCGCCATCAGCAAGCGGTTGGGGGGGAGGCTTCCTGCCCGCGCTGATCCCCATCATCGCC
GCGGCGATCGGGGCGATCCCCGGCATTGCTTCCGTGGCGGTGCAGGCCTCTCAGCGCCACTGAGACACACTTGG
AAACATCTTGTAATAAACCaATGGACTCTGACGCTCCTGGTCCTGTGATGTGTTTTCGTAGACAGATGGAAGAC
ATCAATTTTTCGTCCCTGGCTCCGCGACACGGCACGCGGCCGTTCATGGGCACCTGGAGCGACATCGGCACCAG
CCAACTGAACGGGGGCGCCTTCAATTGGAGCAGTCTCTGGAGCGGGCTTAAGAATTTCGGGTCCACGCTTAAAA
CCTATGGCAGCAAGGCGTGGAACAGCACCACAGGGCAGGCGCTGAGGGATAAGCTGAAAGAGCAGAACTTCCAG
CAGAAGGTGGTCGATGGGCTCGCCTCGGGCATCAACGGGGTGGTGGACCTGGCCAACCAGGCCGTGCAGCGGCA
GATCAACAGCCGCCTGGACCCGGTGCCGCCCGCCGGCTCCGTGGAGATGCCGCAGGTGGAGGAGGAGCTGCCTC
CCCTGGACAAGCGGGGCGAGAAGCGACCCCGCCCCGATGCGGAGGAGACGCTGCTGACGCACACGGACGAGCCG
CCCCCGTACGAGGAGGCGGTGAAACTGGGTCTGCCCACCACGCGGCCCATCGCGCCCCTGGCCACCGGGGTGCT
GAAACCCGAAAAGCCCGCGACCCTGGACTTGCCTCCTCCCCAGCCTTCCCGCCCCTCTACAGTGGCTAAGCCCC
TGCCGCCGGTGGCCGTGGCCCGCGCGCGACCCGGGGGCACCGCCCGCCCTCATGCGAACTGGCAGAGCACTCTG
AACAGCATCGTGGGTCTGGGAGTGCAGAGTGTGAAGCGCCGCCGCTGCTATTAAACCTACCGTAGCGCTTAACT
TGCTTGTCTGTGTGTGTATGTATTATGTCGCCGCCGCCGCTGTCCACCAGAAGGAGGAGTGAAGAGGCGCGTCG
CCGAGTTGCAAGATGGCCACCCCATCGATGCTGCCCCAGTGGGCGTACATGCACATCGCCGGACAGGACGCTTC
GGAGTACCTGAGTCCGGGTCTGGTGCAGTTTGCCCGCGCCACAGACACCTACTTCAGTCTGGGGAACAAGTTTA
GGAACCCCACGGTGGCGCCCACGCACGATGTGACCACCGACCGCAGCCAGCGGCTGACGCTGCGCTTCGTGCCC
GTGGACCGCGAGGACAACACCTACTCGTACAAAGTGCGCTACACGCTGGCCGTGGGCGACAACCGCGTGCTGGA
CATGGCCAGCACCTACTTTGACATCCGCGGCGTGCTGGATCGGGGCCCTAGCTTCAAACCCTACTCCGGCACCG
CCTACAACAGTCTGGCCCCCAAGGGAGCACCCAACACTTGTCAGTGGACATATAAAGCCGATGGTGAAACTGCC
ACAGAAAAAACCTATACATATGGAAATGCACCCGTGCAGGGCATTAACATCACAAAGATGGTATTCAACTTGG
AACTGACACCGATGATCAGCCAATCTACGCAGATAAAACCTATCAGCCTGAACCTCAAGTGGGTGATGCTGAAT
GGCATGACATCACTGGTACTGATGAAAAGTATGGAGGCAGAGCTCTTAAGCCTGATACCAAAATGAAGCCTTGT
TATGGTTCTTTTGCCAAGCCTACTAATAAAGAAGGAGGTCAGGCAAATGTGAAAACAGGAACAGGCACTACTAA
AGAATATGACATAGACATGGCTTTCTTTGACAACAGAAGTGCGGCTGCTGCTGGCCTAGCTCCAGAAATTGTTT
TGTATACTGAAAATGTGGATTTGGAAACTCCAGATACCCATATTGTATACAAAGCAGGCACAGATGACAGCAGC
TCTTCTATTAATTTGGGTCAGCAAGCCATGCCCAACAGACCTAACTACATTGGTTTCAGAGACAACTTTATCGG
GCTCATGTACTACAACAGCACTGGCAATATGGGGGTGCTGGCCGGTCAGGCTTCTCAGCTGAATGCTGTGGTTG
ACTTGCAAGACAGAAACACCGAGCTGTCCTACCAGCTCTTGCTTGACTCTCTGGGTGACAGAACCCGGTATTTC
AGTATGTGGAATCAGGCGGTGGACAGCTATGATCCTGATGTGCGCATTATTGAAAATCATGGTGTGGAGGATGA
ACTTCCCAACTATTGTTTCCCTCTGGATGCTGTTGGCAGAACAGATACTTATCAGGGAATTAAGGCTAATGGAA
CTGATCAAACCACATGGACCAAAGATGACAGTGTCAATGATGCTAATGAGATAGGCAAGGGTAATCCATTCGCC
ATGGAAATCAACATCCAAGCCAACCTGTGGAGGAACTTCCTCTACGCCAACGTGGCCCTGTACCTGCCCGACTC
TTACAAGTACACGCCGGCCAATGTTACCCTGCCCACCAACACCAACACCTACGATTACATGAACGGCCGGGTGG
TGGCGCCCTCGCTGGTGGACTCCTACATCAACATCGGGGCGCGCTGGTCGCTGGATCCCATGGACAACGTGAAC
CCCTTCAACCACCACCGCAATGCGGGGCTGCGCTACCGCTCCATGCTCCTGGGCAACGGGCGCTACGTGCCCTT
CCACATCCAGGTGCCCCAGAAATTTTTCGCCATCAAGAGCCTCCTGCTCCTGCCCGGGTCCTACACCTACGAGT
GGAACTTCCGCAAGGACGTCAACATGATCCTGCAGAGCTCCCTCGGCAACGACCTGCGCACGGACGGGGCCTCC
ATCTCCTTCACCAGCATCAACCTCTACGCCACCTTCTTCCCCATGGCGCACAACACGGCCTCCACGCTCGAGGC
CATGCTGCGCAACGACACCAACGACCAGTCCTTCAACGACTACCTCTCGGCGGCCAACATGCTCTACCCCATCC
CGGCCAACGCCACCAACGTGCCCATCTCCATCCCCTCGCGCAACTGGGCCGCCTTCCGCGGCTGGTCCTTCACG
CGTCTCAAGACCAAGGAGACGCCCTCGCTGGGCTCCGGGTTCGACCCCTACTTCGTCTACTCGGGCTCCATCCC
CTACCTCGACGGCACCTTCTACCTCAACCACACCTTCAAGAAGGTCTCCATCACCTTCGACTCCTCCGTCAGCT
GGCCCGGCAACGACCGGCTCCTGACGCCCAACGAGTTCGAAATCAAGCGCACCGTCGACGGCGAGGGCTACAAC
GTGGCCCAGTGCAACATGACCAAGGACTGGTTCCTGGTCCAGATGCTGGCCCACTACAACATCGGCTACCAGGG
CTTCTACGTGCCCGAGGGCTACAAGGACCGCATGTACTCCTTCTTCCGCAACTTCCAGCCCATGAGCCGCCAGG
TGGTGGACGAGGTCAACTACAAGGACTACCAGGCCGTCACCCTGGCCTACCAGCACAACAACTCGGGCTTCGTC
GGCTACCTCGCGCCCACCATGCGCCAGGGCCAGCCCTACCCCGCCAACTACCCCTACCCGCTCATCGGCAAGAG
CGCCGTCACCAGCGTCACCCAGAAAAAGTTCCTCTGCGACAGGGTCATGTGGCGCATCCCCTTCTCCAGCAACT
TCATGTCCATGGGCGCGCTCACCGACCTCGGCCAGAACATGCTCTATGCCAACTCCGCCCACGCGCTAGACATG
AATTTCGAAGTCGACCCCATGGATGAGTCCACCCTTCTCTATGTTGTCTTCGAAGTCTTCGACGTCGTCCGAGT
GCACCAGCCCCACCGCGGCGTCATCGAGGCCGTCTACCTGCGCACCCCCTTCTCGGCCGGTAACGCCACCACCT
AAGCTCTTGCTTCTTGCAAGCCATGGCCGCGGGCTCCGGCGAGCAGGAGCTCAGGGCCATCATCCGCGACCTGG
GCTGCGGGCCCTACTTCCTGGGCACCTTCGATAAGCGCTTCCCGGGATTCATGGCCCCGCACAAGCTGGCCTGC
GCCATCGTCAACACGGCCGGCCGCGAGACCGGGGGCGAGCACTGGCTGGCCTTCGCCTGGAACCCGCGCTCGAA
CACCTGCTACCTCTTCGACCCCTTCGGGTTCTCGGACGAGCGCCTCAAGCAGATCTACCAGTTCGAGTACGAGG
GCCTGCTGCGCCGCAGCGCCCTGGCCACCGAGGACCGCTGCGTCACCCTGGAAAAGTCCACCCAGACCGTGCAG
GGTCCGCGCTCGGCCGCCTGCGGGCTCTTCTGCTGCATGTTCCTGCACGCCTTCGTGCACTGGCCCGACCGCCC
CATGGACAAGAACCCCACCATGAACTTGCTGACGGGGGTGCCCAACGGCATGCTCCAGTCGCCCCAGGTGGAAC
CCACCCTGCGCCGCAACCAGGAGGCGCTCTACCGCTTCCTCAACTCCCACTCCGCCTACTTTCGCTCCCACCGC
GCGCGCATCGAGAAGGCCACCGCCTTCGACCGCATGAATCAAGACATGTAAACCGTGTGTGTATGTTAAATGTC
TTTAATAAACAGCACTTTCATGTTACACATGCATCTGAGATGATTTATTTAGAAATCGAAAGGGTTCTGCCGGG
```

```
TCTCGGCATGGCCCGCGGGCAGGGACACGTTGCGGAACTGGTACTTGGCCAGCCACTTGAACTCGGGGATCAGC
AGTTTGGGCAGCGGGGTGTCGGGGAAGGAGTCGGTCCACAGCTTCCGCGTCAGTTGCAGGGCGCCCAGCAGGTC
GGGCGCGGAGATCTTGAAATCGCAGTTGGGACCCGCGTTCTGCGCGCGGGAGTTGCGGTACACGGGGTTGCAGC
ACTGGAACACCATCAGGGCCGGGTGCTTCACGCTCGCCAGCACCGTCGCGTCGGTGATGCTCTCCACGTCGAGG
TCCTCGGCGTTGGCCATCCCGAAGGGGGTCATCTTGCAGGTCTGCCTTCCCATGGTGGGCACGCACCGGGCTT
GTGGTTGCAATCGCAGTGCAGGGGGATCAGCATCATCTGGGCCTGGTCGGCGTTCATCCCCGGGTACATGGCCT
TCATGAAAGCCTCCAATTGCCTGAACGCCTGCTGGGCCTTGGCTCCCTCGGTGAAGAAGACCCCGCAGGACTTG
CTAGAGAACTGGTTGGTGGCGCACCGGCGTCGTGCACGCAGCAGCGCGCGTCGTTGTTGGCCAGCTGCACCAC
GCTGCGCCCCAGCGGTTCTGGGTGATCTTGGCCCGGTCGGGGTTCTCCTTCAGCGCGCGCTGCCCGTTCTCGC
TCGCCACATCCATCTCGATCATGTGCTCCTTCTGGATCATGGTGGTCCCGTGCAGGCACCGCAGCTTGCCCTCG
GCCTCGGTGCACCCGTGCAGCCACAGCGCGCACCCGGTGCACTCCCAGTTCTTGTGGGCGATCTGGGAATGCGC
GTGCACGAAGCCCTGCAGGAAGCGGCCCATCATGGTGGTCAGGGTCTTGTTGCTAGTGAAGGTCAGCGGAATGC
CGCGGTGCTCCTCGTTGATGTACAGGTGGCAGATGCGGCGGTACACCTCGCCCTGCTCGGGCATCAGCTGGAAG
TTGGCTTTCAGGTCGGTCTCCACGCGGTAGCGGTCCATCAGCATAGTCATGATTTCCATACCCTTCTCCCAGGC
CGAGACGATGGGCAGGCTCATAGGGTTCTTCACCATCATCTTAGCGCTAGCAGCCGCGGCCAGGGGGTCGCTCT
CGTCCAGGGTCTCAAAGCTCCGCTTGCCGTCCTTCTCGGTGATCCGCACCGGGGGGGTAGCTGAAGCCCACGGCC
GCCAGCTCCTCCTCGGCCTGTCTTTCGTCCTCGCTGTCCTGGCTGACGTCCTGCAGGACCACATGCTTGGTCTT
GCGGGGTTTCTTCTTGGGCGGCAGCGGCGGCGGAGATGTTGGAGATGGCGAGGGGGAGCGCGAGTTCTCGCTCA
CCACTACTATCTCTTCCTCTTCTTGGTCCGAGGCCACGCGGCGGTAGGTATGTCTCTTCGGGGGCAGAGGCGGA
GGCGACGGGCTCTCGCCGCCGCGACTTGGCGGATGGCTGGCAGAGCCCCTTCCGCGTTCGGGGGTGCGCTCCCG
GCGGCGCTCTGACTGACTTCCTCCGCGGCCGGCCATTGTGTTCTCCTAGGGAGGAACAACAAGCATGGAGACTC
AGCCATCGCCAACCTCGCCATCTGCCCCCACCGCCGACGAGAAGCAGCAGCAGCAGAATGAAAGCTTAACCGCC
CCGCCGCCCAGCCCCGCCACCTCCGACGCGGCCGTCCCAGACATGCAAGAGATGGAGGAATCCATCGAGATTGA
CCTGGGCTATGTGACGCCCGCGGAGCACGAGGAGGAGCTGGCAGTGCGCTTTTCACAAGAAGAGATACACCAAG
AACAGCCAGAGCAGGAAGCAGAGAATGAGCAGAGTCAGGCTGGGCTCGAGCATGACGGCGACTACCTCCACCTG
AGCGGGGGGGAGGACGCGCTCATCAAGCATCTGGCCCGGCAGGCCACCATCGTCAAGGATGCGCTGCTCGACCG
CACCGAGGTGCCCCTCAGCGTGGAGGAGCTCAGCCGCGCCTACGAGTTGAACCTCTTCTCGCCGCGCGTGCCCC
CCAAGCGCCAGCCCAATGGCACCTGCGAGCCCAACCCGCGCCTCAACTTCTACCCGGTCTTCGCGGTGCCCGAG
GCCCTGGCCACCTACCACATCTTTTTCAAGAACCAAAAGATCCCCGTCTCCTGCCGCGCCAACCGCACCCGCGC
CGACGCCCTTTTCAACCTGGGTCCCGGCGCCCGCCTACCTGATATCGCCTCCTTGGAAGAGGTTCCCAAGATCT
TCGAGGGTCTGGGCAGCGACGAGACTCGGGCCGCGAACGCTCTGCAAGGAGAAGGAGGAGAGCATGAGCACCAC
AGCGCCCTGGTCGAGTTGGAAGGCGACAACGCGCGGCTGGCGGTGCTCAAACGCACGGTCGAGCTGACCCATTT
CGCCTACCCGGCTCTGAACCTGCCCCCCAAAGTCATGAGCGCGGTCATGGACCAGGTGCTCATCAAGCGCGCGT
CGCCCATCTCCGAGGACGAGGGCATGCAAGACTCCGAGGAGGGCAAGCCCGTGGTCAGCGACGAGCAGCTGGCC
CGGTGGCTGGGTCCTAATGCTAGTCCCCAGAGTTTGGAAGAGCGGCGCAAACTCATGATGGCCGTGGTCCTGGT
GACCGTGGAGCTGGAGTGCCTGCGCCGCTTCTTCGCCGACGCGGAGACCCTGCGCAAGGTCGAGGAGAACCTGC
ACTACCTCTTCAGGCACGGGTTCGTGCGCCAGGCCTGCAAGATCTCCAACGTGGAGCTGACCAACCTGGTCTCC
TACATGGGCATCTTGCACGAGAACCGCCTGGGGCAGAACGTGCTGCACACCACCCTGCGCGGGGAGGCCCGGCG
CGACTACATCCGCGACTGCGTCTACCTCTACCTCTGCCACACCTGGCAGACGGGCATGGGCGTGTGGCAGCAGT
GTCTGGAGGAGCAGAACCTGAAAGAGCTCTGCAAGCTCCTGCAGAAGAACCTCAAGGGTCTGTGGACCGGGTTC
GACGAGCGCACCACCGCCTCGGACCTGGCCGACCTCATTTTCCCCGAGCGCCTCAGGCTGACGCTGCGCAACGG
CCTGCCCGACTTTATGAGCCAAAGCATGTTGCAAAACTTTCGCTCTTTCATCCTCGAACGCTCCGGAATCCTGC
CCGCCACCTGCTCCGCGCTGCCCTCGGACTTCGTGCCGCTGACCTTCCGCGAGTGCCCCCCGCCGCTGTGGAGC
CACTGCTACCTGCTGCGCCTGGCCAACTACCTGGCCTACCACTCGGACGTGATCGAGGACGTCAGCGGCGAGGG
CCTGCTCGAGTGCCACTGCCGCTGCAACCTCTGCACGCCGCACCGCTCCCTGGCCTGCAACCCCCAGCTGCTGA
GCGAGACCCAGATCATCGGCACCTTCGAGTTGCAAGGGCCCAGCGAAGGCGAGGGTTCAGCCGCCAAGGGGGGT
CTGAAACTCACCCCGGGGCTGTGGACCTCGGCCTACTTGCGCAAGTTCGTGCCCGAGGACTACCATCCCTTCGA
GATCAGGTTCTACGAGGACCAATCCCATCCGCCCAAGGCCGAGCTGTCGGCCTGCGTCATCACCCAGGGGGCGA
TCCTGGCCCAATTGCAAGCCATCCAGAAATCCCGCCAAGAATTCTTGCTGAAAAAGGGCCGCGGGGTCTACCTC
GACCCCCAGACCGGTGAGGAGCTCAACCCCGGCTTCCCCCAGGATGCCCCGAGGAAACAAGAAGCTGAAAGTGG
AGCTGCCGCCCGTGGAGGATTTGGAGGAAGACTGGGAGAACAGCAGTCAGGCAGAGGAGGAGGAGATGGAGGAA
GACTGGGACAGCACTCAGGCAGAGGAGGACAGCCTGCAAGACAGTCTGGAGGAAGACGAGGAGGAGGCAGAGGA
GGAGGTGGAAGAAGCAGCCGCCGCCAGACCGTCGTCCTCGGCGGGGGAGAAAGCAAGCAGCACGGATACCATCT
CCGCTCCGGGTCGGGGTCCCGCTCGACCACACAGTAGATGGGACGAGACCGGACGATTCCCGAACCCCACCACC
CAGACCGGTAAGAAGGAGCGGCAGGGATACAAGTCCTGGCGGGGGCACAAAAACGCCATCGTCTCCTGCTTGCA
GGCCTGCGGGGGCAACATCTCCTTCACCCGGCGCTACCTGCTCTTCCACCGCGGGGTGAACTTTCCCGCAACA
TCTTGCATTACTACCGTCACCTCCACAGCCCCTACTACTTCCAAGAAGAGGCAGCAGCAGCAGAAAAAGACCAG
CAGAAAACCAGCAGCTAGAAAATCCACAGCGGCGGCAGCAGGTGGACTGAGGATCGCGGCGAACGAGCCGGCGC
AAACCCGGGAGCTGAGGAACCGGATCTTTCCCACCCTCTATGCCATCTTCCAGCAGAGTCGGGGGCAGGAGCAG
GAACTGAAAGTCAAGAACCGTTCTCTGCGCTCGCTCACCCGCAGTTGTCTGTATCACAAGAGCGAAGACCAACT
TCAGCGCACTCTCGAGGACGCCGAGGCTCTCTTCAACAAGTACTGCGCGCTCACTCTTAAAGAGTAGCCCGCGC
CCGCCCAGTCGCAGAAAAAGGCGGGAATTACGTCACCTGTGCCCTTCGCCCTAGCCGCCTCCACCCATCATCAT
```

```
GAGCAAAGAGATTCCCACGCCTTACATGTGGAGCTACCAGCCCCAGATGGGCCTGGCCGCCGGTGCCGCCCAGG
ACTACTCCACCCGCATGAATTGGCTCAGCGCCGGGCCCGCGATGATCTCACGGGTGAATGACATCCGCGCCCAC
CGAAACCAGATACTCCTAGAACAGTCAGCGCTCACCGCCACGCCCCGCAATCACCTCAATCCGCGTAATTGGCC
CGCCGCCCTGGTGTACCAGGAAATTCCCCAGCCCACGACCGTACTACTTCCGCGAGACGCCCAGGCCGAAGTCC
AGCTGACTAACTCAGGTGTCCAGCTGGCGGGCGGCGCCACCCTGTGTCGTCACCGCCCCGCTCAGGGTATAAAG
CGGCTGGTGATCCGGGGCAGAGGCACACAGCTCAACGACGAGGTGGTGAGCTCTTCGCTGGGTCTGCGACCTGA
CGGAGTCTTCCAACTCGCCGGATCGGGGAGATCTTCCTTCACGCCTCGTCAGGCCGTCCTGACTTTGGAGAGTT
CGTCCTCGCAGCCCCGCTCGGGTGGCATCGGCACTCTCCAGTTCGTGGAGGAGTTCACTCCCTCGGTCTACTTC
AACCCCTTCTCCGGCTCCCCGGCCACTACCCGGACGAGTTCATCCCGAACTTCGACGCCATCAGCGAGTCGGT
GGACGGCTACGATTGAATGTCCCATGGTGGCGCAGCTGACCTAGCTCGGCTTCGACACCTGGACCACTGCCGCC
GCTTCCGCTGCTTCGCTCGGGATCTCGCCGAGTTTGCCTACTTTGAGCTGCCCGAGGAGCACCCTCAGGGCCCG
GCCCACGGAGTGCGGATCGTCGTCGAAGGGGGGCCTCGACTCCCACCTGCTTCGGATCTTCAGCCAGCGTCCGAT
CCTGGTCGAGCGCGAGCAAGGACAGACCCTTCTGACTCTGTACTGCATCTGCAACCACCCCGGCCTGCATGAAA
GTCTTTGTTGTCTGCTGTGTACTGAGTATAATAAAAGCTGAGATCAGCGACTACTCCGGACTTCCGTGTGTTCC
TGAATCCATCAACCAGTCTTTGTTCTTCACCGGGAACGAGACCGAGCTCCAGCTCCAGTGTAAGCCCCACAAGA
AGTACCTCACCTGGCTGTTCCAGGGCTCCCCGATCGCCGTTGTCAACCACTGCGACAACGACGGAGTCCTGCTG
AGCGGCCCTGCCAACCTTACTTTTTCCACCCGCAGAAGCAAGCTCCAGCTCTTCCAACCCTTCCTCCCCGGGAC
CTATCAGTGCGTCTCGGGACCCTGCCATCACACCTTCCACCTGATCCCGAATACCACAGCGTCGCTCCCCGCTA
CTAACAACCAAACTAACCTCCACCAACGCCACCGTCGCGACGGCCACAATACATGCCCATATTAGACTATGAGG
CCGAGCCACAGCGACCCATGCTCCCCGCTATTAGTTACTTCAATCTAACCGGCGGAGATGACTGACCCACTGGC
CAACAACAACGTCAACGACCTTCTCCTGGACATGGACGGCCGCGCCTCGGAGCAGCGACTCGCCCAACTTCGCA
TTCGCCAGCAGCAGGAGAGAGCCGTCAAGGAGCTGCAGGATGCGGTGGCCATCCACCAGTGCAAGAGAGGCATC
TTCTGCCTGGTGAAACAGGCCAAGATCTCCTACGAGGTCACTCCAAACGACCATCGCCTCTCCTACGAGCTCCT
GCAGCAGCGCCAGAAGTTCACCTGCCTGGTCGGAGTCAACCCCATCGTCATCACCCAGCAGTCTGGCGATACCA
AGGGGTGCATCCACTGCTCCTGCGACTCCCCCGACTGCGTCCACACTCTGATCAAGACCCTCTGCGGCCTCCGC
GACCTCCTCCCCATGAACTAATCACCCCCTTATCCAGTGAAATAAAGATCATATTGATGATGATTTTACAGAAA
TAAAAAATAATCATTTGATTTGAAATAAAGATACAATCATATTGATGATTTGAGTTTAACAAAAAAATAAAGAA
TCACTTACTTGAAATCTGATACCAGGTCTCTGTCCATGTTTTCTGCCAACACCACTTCACTCCCCTCTTCCCAG
CTCTGGTACTGCAGGCCCCGGCGGGCTGCAAACTTCCTCCACACGCTGAAGGGGATGTCAAATTCCTCCTGTCC
CTCAATCTTCATTTTATCTTCTATCAGATGTCCAAAAAGCGCGTCCGGGTGGATGATGACTTCGACCCCGTCTA
CCCCTACGATGCAGACAACGCACCGACCGTGCCCTTCATCAACCCCCCCCTTCGTCTCTTCAGATGGATTCCAAG
AGAAGCCCCTGGGGGTGTTGTCCCTGCGACTGGCCGACCCCGTCACCACCAAGAACGGGGAAATCACCCTCAAG
CTGGGAGAGGGGGTGGACCTCGATTCCTCGGGAAAACTCATCTCCAACACGGCCACCAAGGCCGCCGCCCCTCT
CAGTTTTTCCAACAACACCATTTCCCTTAACATGGATCACCCCTTTTACACTAAAGATGGAAAATTATCCTTAC
AAGTTTCTCCACCATTAAATATACTGAGAACAAGCATTCTAAACACACTAGCTTTAGGTTTTGGATCAGGTTTA
GGACTCCGTGGCTCTGCCTTGGCAGTACAGTTAGTCTCTCCACTTACATTTGATACTGATGGAAACATAAAGCT
TACCTTAGACAGAGGTTTGCATGTTACAACAGGAGATGCAATTGAAAGCAACATAAGCTGGGCTAAAGGTTTAA
AATTTGAAGATGGAGCCATAGCAACCAACATTGGAAATGGGTTAGAGTTTGGAAGCAGTAGTACAGAAACAGGT
GTTGATGATGCTTACCCAATCCAAGTTAAACTTGGATCTGGCCTTAGCTTTGACAGTACAGGAGCCATAATGGC
TGGTAACAAAGAAGACGATAAACTCACTTTGTGGACAACACCTGATCCATCACCAAACTGTCAAATACTCGCAG
AAAATGATGCAAAACTAACACTTTGCTTGACTAAATGTGGTAGTCAAATACTGGCCACTGTGTCAGTCTTAGTT
GTAGGAAGTGGAAACCTAAACCCCATTACTGGCACCGTAAGCAGTGCTCAGGTGTTTCTACGTTTTGATGCAAA
CGGTGTTCTTTTAACAGAACATTCTACACTAAAAAAATACTGGGGGTATAGGCAGGGAGATAGCATAGATGGCA
CTCCATATACCAATGCTGTAGGATTCATGCCCAATTTAAAAGCTTATCCAAAGTCACAAAGTTCTACTACTAAA
AATAATATAGTAGGGCAAGTATACATGAATGGAGATGTTTCAAAACCTATGCTTCTCACTATAACCCTCAATGG
TACTGATGACAGCAACAGTACATATTCAATGTCATTTTCATACACCTGGACTAATGGAAGCTATGTTGGAGCAA
CATTTGGGGCTAACTCTTATACCTTCTCATACATCGCCCAAGAATGAACACTGTATCCCACCCTGCATGCCAAC
CCTTCCCACCCCACTCTGTGGAACAAACTCTGAAACACAAAATAAAATAAAGTTCAAGTGTTTTATTGATTCAA
CAGTTTTACAGGATTCGAGCAGTTATTTTTCCTCCACCCTCCCAGGACATGGAATACACCACCCTCTCCCCCCG
CACAGCCTTGAACATCTGAATGCCATTGGTGATGGACATGCTTTTGGTCTCCACGTTCCACACAGTTTCAGAGC
GAGCCAGTCTCGGGTCGGTCAGGGAGATGAAACCCTCCGGGCACTCCCGCATCTGCACCTCACAGCTCAACAGC
TGAGGATTGTCCTCGGTGGTCGGGATCACGGTTATCTGGAAGAAGCAGAAGAGCGGCGGTGGGAATCATAGTCC
GCGAACGGGATCGGCCGGTGGTGTCGCATCAGGCCCCGCAGCAGTCGCTGCCGCCGCCGCTCCGTCAAGCTGCT
GCTCAGGGGGTCCGGGTCCAGGGACTCCCTCAGCATGATGCCCACGGCCCTCAGCATCAGTCGTCTGGTGCGGC
GGGCGCAGCAGCGCATGCGGATCTCGCTCAGGTCGCTGCAGTACGTGCAACACAGAACCACCAGGTTGTTCAAC
AGTCCATAGTTCAACACGCTCCAGCCGAAACTCATCGCGGGAAGGATGCTACCCACGTGGCCGTCGTACCAGAT
CCTCAGGTAAATCAAGTGGTGCCCCCTCCAGAACACGCTGCCCACGTACATGATCTCCTTGGGCATGTGGCGGT
TCACCACCTCCCGGTACCACATCACCCTCTGGTTGAACATGCAGCCCCGGATGATCCTGCGGAACCACAGGGCC
AGCACCGCCCCGCCCGCCATGCAGCGAAGAGACCCCGGGTCCCGGCAATGGCAATGGAGGACCCACCGCTCGTA
CCCGTGGATCATCTGGGAGCTGAACAAGTCTATGTTGGCACAGCACAGGCATATGCTCATGCATCTCTTCAGCA
CTCTCAACTCCTCGGGGGGTCAAAACCATATCCCAGGGCACGGGGAACTCTTGCAGGACAGCGAACCCCGCAGAA
CAGGGCAATCCTCGCACAGAACTTACATTGTGCATGGACAGGGTATCGCAATCAGGCAGCACCGGGTGATCCTC
```

(continued)

```
CACCAGAGAAGCGCGGGTCTCGGTCTCCTCACAGCGTGGTAAGGGGGCCGGCCGATACGGGTGATGGCGGGACG
CGGCTGATCGTGTTCGCGACCGTGTCATGATGCAGTTGCTTTCGGACATTTTCGTACTTGCTGTAGCAGAACCT
GGTCCGGGCGCTGCACACCGATCGCCGGCGGCGGTCTCGGCGCTTGGAACGCTCGGTGTTGAAATTGTAAAACA
GCCACTCTCTCAGACCGTGCAGCAGATCTAGGGCCTCAGGAGTGATGAAGATCCCATCATGCCTGATGGCTCTG
ATCACATCGACCACCGTGGAATGGGCCAGACCCAGCCAGATGATGCAATTTTGTTGGGTTTCGGTGACGGCGGG
GGAGGGAAGAACAGGAAGAACCATGATTAACTTTTAATCCAAACGGTCTCGGAGTACTTCAAAATGAAGATCGC
GGAGATGGCACCTCTCGCCCCCGCTGTGTTGGTGGAAAATAACAGCCAGGTCAAAGGTGATACGGTTCTCGAGA
TGTTCCACGGTGGCTTCCAGCAAAGCCTCCACGCGCACATCCAGAAACAAGACAATAGCGAAAGCGGGAGGGTT
CTCTAATTCCTCAATCATCATGTTACACTCCTGCACCATCCCCAGATAATTTTCATTTTTCCAGCCTTGAATGA
TTCGAACTAGTTCGTGAGGTAAATCCAAGCCAGCCATGATAAAGAGCTCGCGCAGAGCGCCCTCCACCGGCATT
CTTAAGCACACCCTCATAATTCCAAGATATTCTGCTCCTGGTTCACCTGCAGCAGATTGACAAGCGGAATATCA
AAATCTCTGCCGCGATCCCTGAGCTCCTCCCTCAGCAATAACTGTAAGTACTCTTTCATATCCTCTCCGAAATT
TTTAGCCATAGGACCACCAGGAATAAGATTAGGGCAAGCCACAGTACAGATAAACCGAAGTCCTCCCCAGTGAG
CATTGCCAAATGCAAGACTGCTATAAGCATGCTGGCTAGACCCGGTGATATCTTCCAGATAACTGGACAGAAAA
TCGCCCAGGCAATTTTTAAGAAAATCAACAAAAGAAAAATCCTCCAGGTGGACGTTTAGAGCCTCGGGAACAAC
GATGAAGTAAATGCAAGCGGTGCGTTCCAGCATGGTTAGTTAGCTGATCTGTAGAAAAAACAAAAATGAACATT
AAACCATGCTAGCCTGGCGAACAGGTGGGTAAATCGTTCTCTCCAGCACCAGGCAGGCCACGGGGTCTCCGGCG
CGACCCTCGTAAAAATTGTCGCTATGATTGAAAACCATCACAGAGAGACGTTCCCGGTGGCCGGCGTGAATGAT
TCGACAAGATGAATACACCCCCGGAACATTGGCGTCCGCGAGTGAAAAAAAGCGCCCGAGGAAGCAATAAGGCA
CTACAATGCTCAGTCTCAAGTCCAGCAAAGCGATGCCATGCGGATGAAGCACAAAATTCTCAGGTGCGTACAAA
ATGTAATTACTCCCCTCCTGCACAGGCAGCAAAGCCCCCGATCCCTCCAGGTACACATACAAAGCCTCAGCGTC
CATAGCTTACCGAGCAGCAGCACACAACAGGCGCAAGAGTCAGAGAAAGGCTGAGCTCTAACCTGTCCACCCGC
TCTCTGCTCAATATATAGCCCAGATCTACACTGACGTAAAGGCCAAAGTCTAAAAATACCCGCCAAATAATCAC
ACACGCCCAGCACACGCCCAGAAACCGGTGACACACTCAAAAAAATACGCGCACTTCCTCAAACGCCCAAAACT
GCCGTCATTTCCGGGTTCCCACGCTACGTCATCAAAAACACGACTTTCAAATTCCGTCGACCGTTAAAAACGTCA
CCCGCCCCGCCCCTAACGGTCGCCCGTCTCTCAGCCAATCAGCGCCCCGCATCCCCAAATTCAAACACCTCATT
TGCATATTAACGCGCACAAAAAGTTTGAGGTATATTATTGATGATGG
```

ChAdV68.4WTnt.MAG25mer (SEQ ID NO:12); AC_000011.1 with E1 (nt 577 to 3403) and E3 (nt 27,816- 31,332) sequences deleted; corresponding ATCC VR-594 nucleotides substituted at four positions; model neoantigen cassette under the control of the CMV promoter/enhancer inserted in place of deleted E1

```
CCATCTTCAATAATATACCTCAAACTTTTTGTGCGCGTTAATATGCAAATGAGGCGTTTGAATTTGGGGAGGAA
GGGCGGTGATTGGTCGAGGGATGAGCGACCGTTAGGGGCGGGGCGAGTGACGTTTTGATGACGTGGTTGCGAGG
AGGAGCCAGTTTGCAAGTTCTCGTGGGAAAAGTGACGTCAAACGAGGTGTGGTTTGAACACGGAAATACTCAAT
TTTCCCGCGCTCTCTGACAGGAAATGAGGTGTTTCTGGGCGGATGCAAGTGAAAACGGGCCATTTTCGCGCGAA
AACTGAATGAGGAAGTGAAAATCTGAGTAATTTCGCGTTTATGGCAGGGAGGAGTATTTGCCGAGGGCCGAGTA
GACTTTGACCGATTACGTGGGGGTTTCGATTACCGTGTTTTTCACCTAAATTTCCGCGTACGGTGTCAAAGTCC
GGTGTTTTTACGTAGGTGTCAGCTGATCGCCAGGGTATTTAAACCTGCGCTCTCCAGTCAAGAGGCCACTCTTG
AGTGCCAGCGAGAAGAGTTTTCTCCTCCGCGCCGCGAGTCAGATCTACACTTTGAAAGTAGGGATAACAGGGTA
ATgacattgattattgactagttGttaaTAGTAATCAATTACGGGGTCATTAGTTCATAGCCCATATATGGAGT
TCCGCGTTACATAACTTACGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTGGAGTATTTACGGTAAAC
TGCCCACTTGGCAGTACATCAAGTGTATCATATGCCAAGTCCGCCCCCTATTGACGTCAATGACGGTAAATGGC
CCGCCTGGCATTATGCCCAGTACATGACCTTACGGGACTTTCCTACTTGGCAGTACATCTACGTATTAGTCATC
GCTATTACCATGgTGATGCGGTTTTGGCAGTACACCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCAAAATGTCGTA
ATAACCCCGCCCCGTTGACGCAAATGGGCGGTAGGCGTGTACGGTGGGAGGTCTATATAAGCAGAgcTCGTTTA
GTGAACCGTCAGATCGCCTGGAACGCCATCCACGCTGTTTTGACCTCCATAGAAGACAGCGATCGCGccaccAT
GGCCGGGATGTTCCAGGCACTGTCCGAAGGCTGCACACCTATGATATTAACCAGATGCTGAATGTCCTGGGAG
ACCACCAGGTCTCTGGCCTGGAGCAGCTGGAGAGCATCATCAACTTCGAGAAGCTGACCGAGTGGACAAGCTCC
AATGTGATGCCTATCCTGTCCCCACTGACCAAGGGCATCCTGGGCTTCGTGTTTACCCTGACAGTGCCTTCTGA
GCGGGGCCTGTCTTGCATCAGCGAGGCAGACGCAACCACACCAGAGTCCGCCAATCTGGGCGAGGAGATCCTGT
CTCAGCTGTACCTGTGGCCCCGGGTGACATATCACTCCCCTTCTTACGCCTATCACCAGTTCGAGCGGAGAGCC
AAGTACAAGAGACACTTCCCAGGCTTTGGCCAGTCTCTGCTGTTCGGCTACCCCGTGTACGTGTTCGGCGATTG
CGTGCAGGGCGACTGGATGCCATCCGGTTTAGATACTGCGCACCACCTGGATATGCACTGCTGAGGTGTAACG
ACACCAATTATTCCGCCCTGCTGGCAGTGGGCGCCCTGGAGGGCCCTCGCAATCAGGATTGGCTGGGCGTGCCA
AGGCAGCTGGTGACACGCATGCAGGCCATCCAGAACGCAGGCCTGTGCACCCTGGTGGCAATGCTGGAGGAGAC
AATCTTCTGGCTGCAGGCCTTTCTGATGGCCCTGACCGACAGCGGCCCCAAGACAAACATCATCGTGGATTCCC
AGTACGTGATGGGCATCTCCAAGCCTTCTTTCCAGGAGTTTGTGGACTGGGAGAACGTGAGCCCAGAGCTGAAT
TCCACCGATCAGCCATTCTGGCAGGCAGGAATCCTGGCAAGGAACCTGGTGCCTATGGTGGCCACAGTGCAGGG
CCAGAATCTGAAGTACCAGGGCCAGAGCCTGGTCATCAGCGCCTCCATCATCGTGTTTAACCTGCTGGAGCTGG
```

(continued)

```
AGGGCGACTATCGGGACGATGGCAACGTGTGGGTGCACACCCCACTGAGCCCCAGAACACTGAACGCCTGGGTG
AAGGCCGTGGAGGAGAAGAAGGGCATCCCAGTGCACCTGGAGCTGGCCTCCATGACCAATATGGAGCTGATGTC
TAGCATCGTGCACCAGCAGGTGAGGACATACGGACCCGTGTTCATGTGCCTGGGAGGCCTGCTGACCATGGTGG
CAGGAGCCGTGTGGCTGACAGTGCGGGTGCTGGAGCTGTTCAGAGCCGCCCAGCTGGCCAACGATGTGGTGCTG
CAGATCATGGAGCTGTGCGGAGCAGCCTTTCGCCAGGTGTGCCACACCACAGTGCCATGGCCCAATGCCTCCCT
GACCCCCAAGTGGAACAATGAGACAACACAGCCTCAGATCGCCAACTGTAGCGTGTACGACTTCTTCGTGTGGC
TGCACTACTATAGCGTGAGGGATACCCTGTGGCCCCGCGTGACATACCACATGAATAAGTACGCCTATCACATG
CTGGAGAGGCGCGCCAAGTATAAGAGAGGCCCTGGCCCAGGCGCAAAGTTTGTGGCAGCATGGACCCTGAAGGC
CGCCGCCGGCCCCGGCCCCGGCCAGTATATCAAGGCTAACAGTAAGTTCATTGGAATCACAGAGCTGGGACCCG
GACCTGGATAATGAGTTTAAACTCCCATTTAAATGTGAGGGTTAATGCTTCGAGCAGACATGATAAGATACATT
GATGAGTTTGGACAAACCACAACTAGAATGCAGTGAAAAAAATGCTTTATTTGTGAAATTTGTGATGCTATTGC
TTTATTTGTAACCATTATAAGCTGCAATAAACAAGTTAACAACAACAATTGCATTCATTTTATGTTTCAGGTTC
AGGGGGAGATGTGGGAGGTTTTTTAAAGCAAGTAAAACCTCTACAAATGTGGTAAAATAACTATAACGGTCCTA
AGGTAGCGAGTGAGTAGTGTTCTGGGGCGGGGGAGGACCTGCATGAGGGCCAGAATAACTGAAATCTGTGCTTT
TCTGTGTGTTGCAGCAGCATGAGCGGAAGCGGCTCCTTTGAGGGAGGGGTATTCAGCCCTTATCTGACGGGGCG
TCTCCCCTCCTGGGCGGGAGTGCGTCAGAATGTGATGGGATCCACGGTGGACGGCCGGCCCGTGCAGCCCGCGA
ACTCTTCAACCCTGACCTATGCAACCCTGAGCTCTTCGTCGTTGGACGCAGCTGCCGCCGCAGCTGCTGCATCT
GCCGCCAGCGCCGTGCGCGGAATGGCCATGGGCGCCGGCTACTACGGCACTCTGGTGGCCAACTCGAGTTCCAC
CAATAATCCCGCCAGCCTGAACGAGGAGAAGCTGTTGCTGCTGATGGCCCAGCTCGAGGCCTTGACCCAGCGCC
TGGGCGAGCTGACCCAGCAGGTGGCTCAGCTGCAGGAGCAGACGCGGGCCGCGGTTGCCACGGTGAAATCCAAA
TAAAAAATGAATCAATAAATAAACGGAGACGGTTGTTGATTTTAACACAGAGTCTGAATCTTTATTTGATTTTT
CGCGCGCGGTAGGCCCTGGACCACCGGTCTCGATCATTGAGCACCCGGTGGATCTTTTCCAGGACCCGGTAGAG
GTGGGCTTGGATGTTGAGGTACATGGGCATGAGCCCGTCCCGGGGGTGGAGGTAGCTCCATTGCAGGGCCTCGT
GCTCGGGGGTGGTGTTGTAAATCACCCAGTCATAGCAGGGGCGCAGGGCATGGTGTTGCACAATATCTTTGAGG
AGGAGACTGATGGCCACGGGCAGCCCTTTGGTGTAGGTGTTTACAAATCTGTTGAGCTGGGAGGGATGCATGCG
GGGGGAGATGAGGTGCATCTTGGCCTGGATCTTGAGATTGGCGATGTTACCGCCCAGATCCCGCCTGGGGTTCA
TGTTGTGCAGGACCACCAGCACGGTGTATCCGGTGCACTTGGGGAATTTATCATGCAACTTGGAAGGGAAGGCG
TGAAAGAATTTGGCGACGCCTTTGTGCCCGCCCAGGTTTTCCATGCACTCATCCATGATGATGGCGATGGGCCC
GTGGGCGGCGGCCTGGGCAAAGACGTTTCGGGGGTCGGACACATCATAGTTGTGGTCCTGGGTGAGGTCATCAT
AGGCCATTTTAATGAATTTGGGGCGGAGGGTGCCGGACTGGGGGACAAAGGTACCTCGATCCCGGGGGCGTAG
TTCCCCTCACAGATCTGCATCTCCCAGGCTTTGAGCTCGGAGGGGGGGATCATGTCCACCTGCGGGGCGATAAA
GAACACGGTTTCCGGGGCGGGGGAGATGAGCTGGGCCGAAAGCAAGTTCCGGAGCAGCTGGGACTTGCCGCAGC
CGGTGGGGCCGTAGATGACCCCGATGACCGGCTGCAGGTGGTAGTTGAGGGAGAGACAGCTGCCGTCCTCCCGG
AGGAGGGGGGCCACCTCGTTCATCATCTCGCGCACGTGCATGTTCTCGCGCACCAGTTCCGCCAGGAGGCGCTC
TCCCCCCAGGGATAGGAGCTCCTGGAGCGAGGCGAAGTTTTTCAGCGGCTTGAGTCCGTCGGCCATGGGCATTT
TGGAGAGGGTTTGTTGCAAGAGTTCCAGGCGGTCCCAGAGCTCGGTGATGTGCTCTACGGCATCTCGATCCAGC
AGACCTCCTCGTTTCGCGGGTTGGGACGGCTGCGGGAGTAGGGCACCAGACGATGGGCGTCCAGCGCAGCCAGG
GTCCGGTCCTTCCAGGGTCGCAGCGTCCGCGTCAGGGTGGTCTCCGTCACGGTGAAGGGGTGCGCGCCGGGCTG
GGCGCTTGCGAGGGTGCGCTTCAGGCTCATCCGGCTGGTCGAAAACCGCTCCCGATCGGCGCCCTGCGCGTCGG
CCAGGTAGCAATTGACCATGAGTTCGTAGTTGAGCGCCTCGGCCGCGTGGCCTTTGGCGCGGAGCTTACCTTTG
GAAGTCTGCCCGCAGGCGGGACAGAGGAGGGACTTGAGGGCGTAGAGCTTGGGGGCGAGGAAGACGGACTCGGG
GGCGTAGGCGTCCGCGCCGCAGTGGGCGCAGACGGTCTCGCACTCCACGAGCCAGGTGAGGTCGGGCTGGTCGG
GGTCAAAAACCAGTTTCCCGCCGTTCTTTTTGATGCGTTTCTTACCTTTGGTCTCCATGAGCTCGTGTCCCCGC
TGGGTGACAAAGAGGCTGTCCGTGTCCCCGTAGACCGACTTTATGGGCCGGTCCTCGAGCGGTGTGCCGCGGTC
CTCCTCGTAGAGGAACCCCGCCCACTCCGAGACGAAAGCCCGGGTCCAGGCCAGCACGAAGGAGGCCACGTGGG
ACGGGTAGCGGTCGTTGTCCACCAGCGGGTCCACCTTTTCCAGGGTATGCAAACACATGTCCCCCTCGTCCACA
TCCAGGAAGGTGATTGGCTTGTAAGTGTAGGCCACGTGACCGGGGGTCCCGGCCGGGGGGGTATAAAAGGGTGC
GGGTCCCTGCTCGTCCTCACTGTCTTCCGGATCGCTGTCCAGGAGCGCCAGCTGTTGGGGTAGGTATTCCCTCT
CGAAGGCGGGCATGACCTCGGCACTCAGGTTGTCAGTTTCTAGAAACGAGGAGGATTTGATATTGACGGTGCCG
GCGGAGATGCCTTTCAAGAGCCCCTCGTCCATCTGGTCAGAAAAGACGATCTTTTTGTTGTCGAGCTTGGTGGC
GAAGGAGCCGTAGAGGGCGTTGGAGAGGAGCTTGGCGATGGAGCGCATGGTCTGGTTTTTTTCCTTGTCGGCGC
GCTCCTTGGCGGCGATGTTGAGCTGCACGTACTCGCGCGCCACGCACTTCCATTCGGGGAAGACGGTGGTCAGC
TCGTCGGGCACGATTCTGACCTGCCAGCCCCGATTATGCAGGGTGATGAGGTCCACACTGGTGGCCACCTCGCC
GCGCAGGGGCTCATTAGTCCAGCAGAGGCGTCCGCCCTTGCGCGAGCAGAAGGGGGGCAGGGGGTCCAGCATGA
CCTCGTCGGGGGGGTCGGCATCGATGGTGAAGATGCCGGGCAGGAGGTCGGGGTCAAAGTAGCTGATGGAAGTG
GCCAGATCGTCCAGGGCAGCTTGCCATTCGCGCACGGCCAGCGCGCtCTCGTAGGGACTGAGGGGCGTGCCCCA
GGGCATGGGATGGGTAAGCGCGGAGGCGTACATGCCGCAGATGTCGTAGACGTAGAGGGGCTCCTCGAGGATGC
CGATGTAGGTGGGGTAGCAGCGCCCCCCGCGGATGCTGGCGCGCACGTAGTCATACAGCTCGTGCGAGGGGGCG
AGGAGCCCCGGGCCCAGGTTGGTGCGACTGGGCTTTTCGGCGCGGTAGACGATCTGGCGGAAAATGGCATGCGA
GTTGGAGGAGATGGTGGGCCTTTGGAAGATGTTGAAGTGGGCGTGGGGCAGTCCGACCGAGTCGCGGATGAAGT
GGGCGTAGGAGTCTTGCAGCTTGGCGACGAGCTCGGCGGTGACTAGGACGTCCAGAGCGCAGTAGTCGAGGGTC
TCCTGGATGATGTCATACTTGAGCTGTCCCTTTTTGTTTCCACAGCTCGCGGTTGAGAAGGAACTCTTCGCGGTC
```

(continued)

```
CTTCCAGTACTCTTCGAGGGGGAACCCGTCCTGATCTGCACGGTAAGAGCCTAGCATGTAGAACTGGTTGACGG
CCTTGTAGGCGCAGCAGCCCTTCTCCACGGGGAGGGCGTAGGCCTGGGCGGCCTTGCGCAGGGAGGTGTGCGTG
AGGGCGAAAGTGTCCCTGACCATGACCTTGAGGAACTGGTGCTTGAAGTCGATATCGTCGCAGCCCCCCTGCTC
CCAGAGCTGGAAGTCCGTGCGCTTCTTGTAGGCGGGGTTGGGCAAAGCGAAAGTAACATCGTTGAAGAGGATCT
TGCCCGCGCGGGGCATAAAGTTGCGAGTGATGCGGAAAGGTTGGGGCACCTCGGCCCGGTTGTTGATGACCTGG
GCGGCGAGCACGATCTCGTCGAAGCCGTTGATGTTGTGGCCCACGATGTAGAGTTCCACGAATCGCGGACGGCC
CTTGACGTGGGGCAGTTTCTTGAGCTCCTCGTAGGTGAGCTCGTCGGGGTCGCTGAGCCCGTGCTGCTCGAGCG
CCCAGTCGGCGAGATGGGGGTTGGCGCGGAGGAAGGAAGTCCAGAGATCCACGGCCAGGGCGGTTTGCAGACGG
TCCCGGTACTGACGGAACTGCTGCCCGACGGCCATTTTTTCGGGGGTGACGCAGTAGAAGGTGCGGGGGTCCCC
GTGCCAGCGATCCCATTTGAGCTGGAGGGCGAGATCGAGGGCGAGCTCGACGAGCCGGTCGTCCCCGGAGAGTT
TCATGACCAGCATGAAGGGGACGAGCTGCTTGCCGAAGGACCCCATCCAGGTGTAGGTTTCCACATCGTAGGTG
AGGAAGAGCCTTTCGGTGCGAGGATGCGAGCCGATGGGGAAGAACTGGATCTCCTGCCACCAATTGGAGGAATG
GCTGTTGATGTGATGGAAGTAGAAATGCCGACGGCGCGCCGAACACTCGTGCTTGTGTTTATACAAGCGGCCAC
AGTGCTCGCAACGCTGCACGGGATGCACGTGCTGCACGAGCTGTACCTGAGTTCCTTTGACGAGGAATTTCAGT
GGGAAGTGGAGTCGTGGCGCCTGCATCTCGTGCTGTACTACGTCGTGGTGGTCGGCCTGGCCCTCTTCTGCCTC
GATGGTGGTCATGCTGACGAGCCCGCGCGGGAGGCAGGTCCAGACCTCGGCGCGAGCGGGTCGGAGAGCGAGGA
CGAGGGCGCGCAGGCCGGAGCTGTCCAGGGTCCTGAGACGCTGCGGAGTCAGGTCAGTGGGCAGCGGCGGCGCG
CGGTTGACTTGCAGGAGTTTTTTCCAGGGCGCGCGGGAGGTCCAGATGGTACTTGATCTCCACCGCGCCATTGGT
GGCGACGTCGATGGCTTGCAGGGTCCCGTGCCCCTGGGGTGTGACCACCGTCCCCGTTTCTTCTTGGGCGGCT
GGGGCGACGGGGGCGGTGCCTCTTCCATGGTTAGAAGCGGCGGCGAGGACGCGCGCCGGGCGGCAGGGGCGGCT
CGGGGCCCGGAGGCAGGGGCGGCAGGGGCACGTCGGCGCCGCGCGCGGGTAGGTTCTGGTACTGCGCCCGGAGA
AGACTGGCGTGAGCGACGACGCGACGGTTGACGTCCTGGATCTGACGCCTCTGGGTGAAGGCCACGGGACCCGT
GAGTTTGAACCTGAAAGAGAGTTCGACAGAATCAATCTCGGTATCGTTGACGGCGGCCTGCCGCAGGATCTCTT
GCACGTCGCCCGAGTTGTCCTGGTAGGCGATCTCGGTCATGAACTGCTCGATCTCCTCCTCTTGAAGGTCTCCG
CGGCCGGCGCGCTCCACGGTGGCCGCGAGGTCGTTGGAGATGCGGCCCATGAGCTGCGAGAAGGCGTTCATGCC
CGCCTCGTTCCAGACGCGGCTGTAGACCACGACGCCCTCGGGATCGCgGGCGCGCATGACCACCTGGGCGAGGT
TGAGCTCCACGTGGCGCGTGAAGACCGCGTAGTTGCAGAGGCGCTGGTAGAGGTAGTTGAGCGTGGTGGCGATG
TGCTCGGTGACGAAGAAATACATGATCCAGCGGCGGAGCGGCATCTCGCTGACGTCGCCCAGCGCCTCCAAACG
TTCCATGGCCTCGTAAAAGTCCACGGCGAAGTTGAAAAACTGGGAGTTGCGCGCCGAGACGGTCAACTCCTCCT
CCAGAAGACGGATGAGCTCGGCGATGGTGGCGCGCACCTCGCGCTCGAAGGCCCCCGGGAGTTCCTCCACTTCC
TCTTCTTCCTCCTCCACTAACATCTCTTCTACTTCCTCCTCAGGCGGCAGTGGTGGCGGGGGAGGGGGCCTGCG
TCGCCGGCGGCGCACGGGCAGACGGTCGATGAAGCGCTCGATGGTCTCGCCGCGCCGGCGTCGCATGGTCTCGG
TGACGGCGCGCCCGTCCTCGCGGGGCCGCAGCGTGAAGACGCCGCCGCGCATCTCCAGGTGGCCGGGGGGGTCC
CCGTTGGGCAGGGAGAGGGCGCTGACGATGCATCTTATCAATTGCCCCGTAGGGACTCCGCGCAAGGACCTGAG
CGTCTCGAGATCCACGGGATCTGAAAACCGCTGAACGAAGGCTTCGAGCCAGTCGCAGTCGCAAGGTAGGCTGA
GCACGGTTTCTTCTGGCGGGTCATGTTGGTTGGGAGCGGGGCGGGCGATGCTGCTGGTGATGAAGTTGAAATAG
GCGGTTCTGAGACGGCGGATGGTGGCGAGGAGCACCAGGTCTTTGGGCCCGGCTTGCTGGATGCGCAGACGGTC
GGCCATGCCCCAGGCGTGGTCCTGACACCTGGCAGGTCCTTGTAGTAGTCCTGCATGAGCCGCTCCACGGGCA
CCTCCTCCTCGCCCGCGCGGCCGTGCATGCGCGTGAGCCCGAAGCCGCGCTGGGGCTGGACGAGCGCCAGGTCG
GCGACGACGCGCTCGGCGAGGATGGCTTGCTGGATCTGGGTGAGGGTGGTCTGGAAGTCATCAAAGTCGACGAA
GCGGTGGTAGGCTCCGGTGTTGATGGTGTAGGAGCAGTTGGCCATGACGGACCAGTTGACGGTCTGGTGGCCCG
GACGCACGAGCTCGTGGTACTTGAGGCGCGAGTAGGCGCGCGTGTCGAAGATGTAGTCGTTGCAGGTGCGCACC
AGGTACTGGTAGCCGATGAGGAAGTGCGGCGGCGGCTGGCGGTAGAGCGGCCATCGCTCGGTGGCGGGGGCGCC
GGGCGCGAGGTCCTCGAGCATGGTGCGGTGGTAGCCGTAGATGTACCTGGACATCCAGGTGATGCCGGCGGCGG
TGGTGGAGGCGCGCGGGAACTCGCGGACGCGGTTCCAGATGTTGCGCAGCGGCAGGAAGTAGTTCATGGTGGGC
ACGGTCTGGCCCGTGAGGCGCGCGCAGTCGTGGATGCTCTATACGGGCAAAAACGAAAGCGGTCAGCGGCTCGA
CTCCGTGGCCTGGAGGCTAAGCGAACGGGTTGGGCTGCGCGTGTACCCCGGTTCGAATCTCGAATCAGGCTGGA
GCCGCAGCTAACGTGGTATTGGCACTCCCGTCTCGACCCAAGCCTGCACCAACCCTCCAGGATACGGAGGCGGG
TCGTTTTGCAACTTTTTTTTGGAGGCCGGATGAGACTAGTAAGCGCGGAAAGCGGCCGACCGCGATGGCTCGCT
GCCGTAGTCTGGAGAAGAATCGCCAGGGTTGCGTTGCGGTGTGCCCCGGTTCGAGGCCGGCCGGATTCCGCGGC
TAACGAGGGCGTGGCTGCCCCGTCGTTTCCAAGACCCATAGCCAGCCGACTTCTCCAGTTACGGAGCGAGCCC
CTCTTTTGTTTTGTTTGTTTTTGCCAGATGCATCCCGTACTGCGGCAGATGCGCCCCACCACCCTCCACCGCA
ACAACAGCCCCCTCCACAGCCGGCGCTTCTGCCCCCGCCCCAGCAGCAACTTCCAGCCACGACCGCCGCGGCCG
CCGTGAGCGGGGCTGGACAGAGTTATGATCACCAGCTGGCCTTGGAAGAGGGCGAGGGGCTGGCGCGCCTGGGG
GCGTCGTCGCCGGAGCGGCACCCGCGCGTGCAGATGAAAAGGGACGCTCGCGAGGCCTACGTGCCCAAGCAGAA
CCTGTTCAGAGACAGGAGCGGCGAGGAGCCCGAGGAGATGCGCGCGGCCCGGTTCCACGCGGGGCGGGAGCTGC
GGCGCGGCCTGGACCGAAAGAGGGTGCTGAGGGACGAGGATTTCGAGGCGGACGAGCTGACGGGGATCAGCCCC
GCGCGCGCGCACGTGGCCGCGGCCAACCTGGTCACGGCGTACGAGCAGACCGTGAAGGAGGAGAGCAACTTCCA
AAAATCCTTCAACAACCACGTGCGCACCCTGATCGCGCGCGAGGAGGTGACCCTGGGCCTGATGCACCTGTGGG
ACCTGCTGGAGGCCATCGTGCAGAACCCCACCAGCAAGCCGCTGACGGCGCAGCTGTTCCTGGTGGTGCAGCAT
AGTCGGGACAACGAAGCGTTCAGGGAGGCGCTGCTGAATATCACCGAGCCCGAGGGCCGCTGGCTCCTGGACCT
GGTGAACATTCTGCAGAGCATCGTGGTGCAGGAGCGCGGGCTGCCGCTGTCCGAGAAGCTGGCGGCCATCAACT
```

```
TCTCGGTGCTGAGTTTGGGCAAGTACTACGCTAGGAAGATCTACAAGACCCCGTACGTGCCCATAGACAAGGAG
GTGAAGATCGACGGGTTTTACATGCGCATGACCCTGAAAGTGCTGACCCTGAGCGACGATCTGGGGGTGTACCG
CAACGACAGGATGCACCGTGCGGTGAGCGCCAGCAGGCGGCGCGAGCTGAGCGACCAGGAGCTGATGCATAGTC
TGCAGCGGGCCCTGACCGGGGCCGGGACCGAGGGGGAGAGCTACTTTGACATGGGCGCGGACCTGCACTGGCAG
CCCAGCCGCCGGGCCTTGGAGGCGGCGGCAGGACCCTACGTAGAAGAGGTGGACGATGAGGTGGACGAGGAGGG
CGAGTACCTGGAAGACTGATGGCGCGACCGTATTTTTGCTAGATGCAACAACAACAGCCACCTCCTGATCCCGC
GATGCGGGCGGCGCTGCAGAGCCAGCCGTCCGGCATTAACTCCTCGGACGATTGGACCCAGGCCATGCAACGCA
TCATGGCGCTGACGACCCGCAACCCCGAAGCCTTTAGACAGCAGCCCCAGGCCAACCGGCTCTCGGCCATCCTG
GAGGCCGTGGTGCCCTCGCGCTCCAACCCCACGCACGAGAAGGTCCTGGCCATCGTGAACGCGCTGGTGGAGAA
CAAGGCCATCCGCGGCGACGAGGCCGGCCTGGTGTACAACGCGCTGCTGGAGCGCGTGGCCCGCTACAACAGCA
CCAACGTGCAGACCAACCTGGACCGCATGGTGACCGACGTGCGCGAGGCCGTGGCCCAGCGCGAGCGGTTCCAC
CGCGAGTCCAACCTGGGATCCATGGTGGCGCTGAACGCCTTCCTCAGCACCCAGCCCGCCAACGTGCCCCGGGG
CCAGGAGGACTACACCAACTTCATCAGCGCCCTGCGCCTGATGGTGACCGAGGTGCCCCAGAGCGAGGTGTACC
AGTCCGGGCCGGACTACTTCTTCCAGACCAGTCGCCAGGGCTTGCAGACCGTGAACCTGAGCCAGGCTTTCAAG
AACTTGCAGGGCCTGTGGGGCGTGCAGGCCCCGGTCGGGGACCGCGCGACGGTGTCGAGCCTGCTGACGCCGAA
CTCGCGCCTGCTGCTGCTGCTGGTGGCCCCCTTCACGGACAGCGGCAGCATCAACCGCAACTCGTACCTGGGCT
ACCTGATTAACCTGTACCGCGAGGCCATCGGCCAGGCGCACGTGGACGAGCAGACCTACCAGGAGATCACCCAC
GTGAGCCGCGCCCTGGGCCAGGACGACCCGGGCAACCTGGAAGCCACCCTGAACTTTTTGCTGACCAACCGGTC
GCAGAAGATCCCCGCCCCAGTACGCGCTCAGCACCGAGGAGGAGCGCATCCTGCGTTACGTGCAGCAGAGCGTGG
GCCTGTTCCTGATGCAGGAGGGGGCCCACCCCCAGCGCCGCGCTCGACATGACCGCGCGCAACATGGAGCCCAGC
ATGTACGCCAGCAACCGCCCGTTCATCAATAAACTGATGGACTACTTGCATCGGGCGGCCGCCATGAACTCTGA
CTATTTCACCAACGCCATCCTGAATCCCCACTGGCTCCCGCCGCCGGGGTTCTACACGGGCGAGTACGACATGC
CCGACCCCAATGACGGGTTCCTGTGGGACGATGTGGACAGCAGCGTGTTCTCCCCCGACCGGGTGCTAACGAG
CGCCCCTTGTGGAAGAAGGAAGGCAGCGACCGACGCCCGTCCTCGGCGCTGTCCGGCCGCGAGGGTGCTGCCGC
GGCGGTGCCCGAGGCCGCCAGTCCTTTCCCGAGCTTGCCCTTCTCGCTGAACAGTATCCGCAGCAGCGAGCTGG
GCAGGATCACGCGCCCGCGCTTGCTGGGCGAAGAGGAGTACTTGAATGACTCGCTGTTGAGACCCGAGCGGGAG
AAGAACTTCCCCAATAACGGGATAGAAAGCCTGGTTGGACAAGATGAGCCGCTGGAAGACGTATGCGCAGGAGCA
CAGGGACGATCCCCGGGCGTCGCAGGGGGCCCACGAGCCGGGGCAGCGCCGCCCGTAAACGCCGGTGGCACGACA
GGCAGCGGGGACAGATGTGGGACGATGAGGACTCCGCCGACGACAGCAGCGTGTTGGACTTGGGTGGGAGTGGT
AACCCGTTCGCTCACCTGCGCCCCCGTATCGGGCGCATGATGTAAGAGAAACCGAAAATAAATGATACTCACCA
AGGCCATGGCGACCAGCGTGCGTTCGTTTCTTCTCTGTTGTTGTTGTATCTAGTATGATGAGGCGTGCGTACCC
GGAGGGTCCTCCTCCCTCGTACGAGAGCGTGATGCAGCAGGCGATGGCGGCGGCGGCGATGCAGCCCCCGCTGG
AGGCTCCTTACGTGCCCCCGCGGTACCTGGCGCCTACGGAGGGGCGGAACAGCATTCGTTACTCGGAGCTGGCA
CCCTTGTACGATACCACCCGGTTGTACCTGGTGGACAACAAGTCGGCGGACATCGCCTCGCTGAACTACCAGAA
CGACCACAGCAACTTCCTGACCACCGTGGTGCAGAACAATGACTTCACCCCCACGGAGGCCAGCACCCAGACCA
TCAACTTTGACGAGCGCTCGCGGTGGGGCGGCCAGCTGAAAACCATCATGCACACCAACATGCCCAACGTGAAC
GAGTTCATGTACAGCAACAAGTTCAAGGCGCGGGTGATGGTCTCCCGCAAGACCCCCAATGGGGTGACAGTGAC
AGAGGATTATGATGGTAGTCAGGATGAGCTGAAGTATGAATGGGTGGAATTTGAGCTGCCCGAAGGCAACTTCT
CGGTGACCATGACCATCGACCTGATGAACAACGCCATCATCGACAATTACTTGGCGGTGGGGCGGCAGAACGGG
GTGCTGGAGAGCGACATCGGCGTGAAGTTCGACACTAGGAACTTCAGGCTGGGCTGGGACCCCGTGACCGAGCT
GGTCATGCCCGGGGTGTACACCAACGAGGCTTTCCATCCCGATATTGTCTTGCTGCCCGGCTGCGGGGTGGACT
TCACCGAGAGCCGCCTCAGCAACCTGCTGGGCATTCGCAAGAGGCAGCCCTTCCAGGAAGGCTTCCAGATCATG
TACGAGGATCTGGAGGGGGGCAACATCCCCGCGCTCCTGGATGTCGACGCCTATGAGAAAAGCAAGGAGGATGC
AGCAGCTGAAGCAACTGCAGCCGTAGCTACCGCCTCTACCGAGGTCAGGGGCGATAATTTTGCAAGCGCCGCAG
CAGTGGCAGCGGCCGAGGCGGCTGAAACCGAAAGTAAGATAGTCATTCAGCCGGTGGAGAAGGATAGCAAGAAC
AGGAGCTACAACGTACTACCGGACAAGATAAACACCGCCTACCGCAGCTGGTACCTAGCCTACAACTATGGCGA
CCCCGAGAAGGGCGTGCGCTCCTGGACGCTGCTCACCACCTCGGACGTCACCTGCGGCGTGGAGCAAGTCTACT
GGTCGCTGCCCGACATGATGCAAGACCCGGTCACCTTCCGCTCCACGCGTCAAGTTAGCAACTACCCGGTGGTG
GGCGCCGAGCTCCTGCCCGTCTACTCCAAGAGCTTCTTCAACGAGCAGGCCGTCTACTCGCAGCAGCTGCGCGC
CTTCACCTCGCTTACGCACGTCTTCAACCGCTTCCCCGAGAACCAGATCCTCGTCCGCCCGCCCGCGCCCACCA
TTACCACCGTCAGTGAAAACGTTCCTGCTCTCACAGATCACGGGACCCTGCCGCTGCGCAGCAGTATCCGGGGA
GTCCAGCGCGTGACCGTTACTGACGCCAGACGCCGCACCTGCCCCTACGTCTACAAGGCCCTGGGCATAGTCGC
GCCGCGCGTCCTCTCGAGCCGCACCTTCTAAATGTCCATTCTCATCTCGCCCAGTAATAACACCGGTTGGGGCC
TGCGCGCGCCCAGCAAGATGTACGGAGGCGCTCGCCAACGCTCCACGCAACACCCCGTGCGCGTGCGCGGGCAC
TTCCGCGCTCCCTGGGGCGCCCTCAAGGGCCGCGTGCGGTCGCGCACCACCGTCGACGACGTGATCGACCAGGT
GGTGGCCGACGCGCGCAACTACACCCCCGCCGCCGCGCCGTCTCCACCGTGGACGCCGTCATCGACAGCGTGG
TGGCcGACGCGCGCCGGTACGCCCGCGCCAAGAGCCGGCGGCGGCGCATCGCCCGGCGGCACCGGAGCACCCCC
GCCATGCGCGCGGCGCGAGCCTTGCTGCGCAGGGCCAGGCGCACGGGACGCAGGGCCATGCTCAGGGCGGCCAG
ACGCGCGGCTTCAGGCGCCAGCGCCGGCAGGACCCGGAGACGCGCGGCCACGGCGGCGGCAGCGGCCATCGCCA
GCATGTCCCGCCCGCGGCGAGGGAACGTGTACTGGGTGCGCGACGCCGCCACCGGTGTGCGCGTGCCCGTGCGC
ACCCGCCCCCCTCGCACTTGAAGATGTTCACTTCGCGATGTTGATGTGTCCCAGCGGCGAGGAGGATGTCCAAG
CGCAAATTCAAGGAAGAGATGCTCCAGGTCATCGCGCCTGAGATCTACGGCCCTGCGGTGGTGAAGGAGGAAAG
```

```
AAAGCCCCGCAAAATCAAGCGGGTCAAAAAGGACAAAAAGGAAGAAGAAAGTGATGTGGACGGATTGGTGGAGT
TTGTGCGCGAGTTCGCCCCCGGCGGCGCGTGCAGTGGCGCGGGCGGAAGGTGCAACCGGTGCTGAGACCCGGC
ACCACCGTGGTCTTCACGCCCGGCGAGCGCTCCGGCACCGCTTCCAAGCGCTCCTACGACGAGGTGTACGGGGA
TGATGATATTCTGGAGCAGGCGGCCGAGCGCCTGGGCGAGTTTGCTTACGGCAAGCGCAGCCGTTCCGCACCGA
AGGAAGAGGCGGTGTCCATCCCGCTGGACCACGGCAACCCCACGCCGAGCCTCAAGCCCGTGACCTTGCAGCAG
GTGCTGCCGACCGCGGCGCCGCGCCGGGGGTTCAAGCGCGAGGGCGAGGATCTGTACCCCACCATGCAGCTGAT
GGTGCCCAAGCGCCAGAAGCTGGAAGACGTGCTGGAGACCATGAAGGTGGACCCGGACGTGCAGCCCGAGGTCA
AGGTGCGGCCCATCAAGCAGGTGGCCCCGGGCCTGGGCGTGCAGACCGTGGACATCAAGATTCCCACGGAGCCC
ATGGAAACGCAGACCGAGCCCATGATCAAGCCCAGCACCAGCACCATGGAGGTGCAGACGGATCCCTGGATGCC
ATCGGCTCCTAGTCGAAGACCCCGGCGCAAGTACGGCGCGGCCAGCCTGCTGATGCCCAACTACGCGCTGCATC
CTTCCATCATCCCCACGCCGGGCTACCGCGGCACGCGCTTCTACCGCGGTCATACCAGCAGCCGCCGCCGCAAG
ACCACCACTCGCCGCCGCCGTCGCCGCACCGCCGCTGCAACCACCCCTGCCGCCCTGGTGCGGAGAGTGTACCG
CCGCGGCCGCGCACCTCTGACCCTGCCGCGCGCGCGCTACCACCCGAGCATCGCCATTTAAACTTTCGCCtGCT
TTGCAGATCAATGGCCCTCACATGCCGCCTTCGCGTTCCCATTACGGGCTACCGAGGAAGAAAACCGCGCCGTA
GAAGGCTGGCGGGGAACGGGATGCGTCGCCACCACCACCGGCGGCGGCGCGCCATCAGCAAGCGGTTGGGGGGA
GGCTTCCTGCCCGCGCTGATCCCCATCATCGCCGCGGCGATCGGGGGCGATCCCCGGCATTGCTTCCGTGGCGGT
GCAGGCCTCTCAGCGCCACTGAGACACACTTGGAAACATCTTGTAATAAACCaATGGACTCTGACGCTCCTGGT
CCTGTGATGTGTTTTCGTAGACAGATGGAAGACATCAATTTTTCGTCCCTGGCTCCGCGACACGGCACGCGGCC
GTTCATGGGCACCTGGAGCGACATCGGCACCAGCCAACTGAACGGGGGCGCCTTCAATTGGAGCAGTCTCTGGA
GCGGGCTTAAGAATTTCGGGTCCACGCTTAAAACCTATGGCAGCAAGGCGTGGAACAGCACCACAGGGCAGGCG
CTGAGGGATAAGCTGAAAGAGCAGAACTTCCAGCAGAAGGTGGTCGATGGGCTCGCCTCGGGCATCAACGGGGT
GGTGGACCTGGCCAACCAGGCCGTGCAGCGGCAGATCAACAGCCGCCTGGACCCGGTGCCGCCCGCCGGCTCCG
TGGAGATGCCGCAGGTGGAGGAGGAGCTGCCTCCCCTGGACAAGCGGGGCGAGAAGCGACCCCGCCCCGATGCG
GAGGAGACGCTGCTGACGCACACGGACGAGCCGCCCCCGTACGAGGAGGCGGTGAAACTGGGTCTGCCCACCAC
GCGGCCCATCGCGCCCCTGGCCACCGGGGTGCTGAAACCCGAAAAGCCCGCGACCCTGGACTTGCCTCCTCCCC
AGCCTTCCCGCCCCTCTACAGTGGCTAAGCCCCTGCCGCCGGTGGCCGTGGCCCGCGCGCGACCCGGGGGCACC
GCCCGCCCTCATGCGAACTGGCAGAGCACTCTGAACAGCATCGTGGGTCTGGGAGTGCAGAGTGTGAAGCGCCG
CCGCTGCTATTAAACCTACCGTAGCGCTTAACTTGCTTGTCTGTGTGTGTATGTATTATGTCGCCGCCGCCGCT
GTCCACCAGAAGGAGGAGTGAAGAGGCGCGTCGCCGAGTTGCAAGATGGCCACCCCATCGATGCTGCCCCAGTG
GGCGTACATGCACATCGCCGGACAGGACGCTTCGGAGTACCTGAGTCCGGGTCTGGTGCAGTTTGCCCGCGCCA
CAGACACCTACTTCAGTCTGGGGAACAAGTTTAGGAACCCCACGGTGGCGCCCACGCACGATGTGACCACCGAC
CGCAGCCAGCGGCTGACGCTGCGCTTCGTGCCCGTGGACCGCGAGGACAACACCTACTCGTACAAAGTGCGCTA
CACGCTGGCCGTGGGCGACAACCGCGTGCTGGACATGGCCAGCACCTACTTTGACATCCGCGGCGTGCTGGATC
GGGGCCCTAGCTTCAAACCCTACTCCGGCACCGCCTACAACAGTCTGGCCCCCAAGGGAGCACCCAACACTTGT
CAGTGGACATATAAAGCCGATGGTGAAACTGCCACAGAAAAAACCTATACATATGGAAATGCACCCGTGCAGGG
CATTAACATCACAAAAGATGGTATTCAACTTGGAACTGACACCGATGATCAGCCAATCTACGCAGATAAAACCT
ATCAGCCTGAACCTCAAGTGGGTGATGCTGAATGGCATGACATCACTGGTACTGATGAAAAGTATGGAGGCAGA
GCTCTTAAGCCTGATACCAAAATGAAGCCTTGTTATGGTTCTTTTGCCAAGCCTACTAATAAAGAAGGAGGTCA
GGCAAATGTGAAAACAGGAACAGGCACTACTAAAGAATATGACATAGACATGGCTTTCTTTGACAACAGAAGTG
CGGCTGCTGCTGGCCTAGCTCCAGAAATTGTTTTGTATACTGAAAATGTGGATTTGGAAACTCCAGATACCCAT
ATTGTATACAAAGCAGGCACAGATGACAGCAGCTCTTCTATTAATTTGGGTCAGCAAGCCATGCCCAACAGACC
TAACTACATTGGTTTCAGAGACAACTTTATCGGGCTCATGTACTACAACAGCACTGGCAATATGGGGGTGCTGG
CCGGTCAGGCTTCTCAGCTGAATGCTGTGGTTGACTTGCAAGACAGAAACACCGAGCTGTCCTACCAGCTCTTG
CTTGACTCTCTGGGTGACAGAACCCGGTATTTCAGTATGTGGAATCAGGCGGTGGACAGCTATGATCCTGATGT
GCGCATTATTGAAAATCATGGTGTGGAGGATGAACTTCCCAACTATTGTTTCCCTCTGGATGCTGTTGGCAGAA
CAGATACTTATCAGGGAATTAAGGCTAATGGAACTGATCAAACACATGGACCAAAGATGACAGTGTCAATGAT
GCTAATGAGATAGGCAAGGGTAATCCATTCGCCATGGAAATCAACATCCAAGCCAACCTGTGGAGGAACTTCCT
CTACGCCAACGTGGCCCTGTACCTGCCCGACTCTTACAAGTACACGCCGGCCAATGTTACCCTGCCCACCAACA
CCAACACCTACGATTACATGAACGGCCGGGTGGTGGCGCCCTCGCTGGTGGACTCCTACATCAACATCGGGGCG
CGCTGGTCGCTGGATCCCATGGACAACGTGAACCCCTTCAACCACCACCGCAATGCGGGGCTGCGCTACCGCTC
CATGCTCCTGGGCAACGGGCGCTACGTGCCCTTCCACATCCAGGTGCCCCAGAAATTTTTTCGCCATCAAGAGCC
TCCTGCTCCTGCCCGGGTCCTACACCTACGAGTGGAACTTCCGCAAGGACGTCAACATGATCCTGCAGAGCTCC
CTCGGCAACGACCTGCGCACGGACGGGGCCTCCATCTCCTTCACCAGCATCAACCTCTACGCCACCTTCTTCCC
CATGGCGCACAACACGGCCTCCACGCTCGAGGCCATGCTGCGCAACGACACCAACGACCAGTCCTTCAACGACT
ACCTCTCGGCGGCCAACATGCTCTACCCCATCCCGGCCAACGCCACCAACGTGCCCATCTCCATCCCCTCGCGC
AACTGGGCCGCCTTCCGCGGCTGGTCCTTCACGCGTCTCAAGACCAAGGAGACGCCCTCGCTGGGCTCCGGGTT
CGACCCCTACTTCGTCTACTCGGGCTCCATCCCCTACCTCGACGGCACCTTCTACCTCAACCACACCTTCAAGA
AGGTCTCCATCACCTTCGACTCCTCCGTCAGCTGGCCCGGCAACGACCGGCTCCTGACGCCCAACGAGTTCGAA
ATCAAGCGCACCGTCGACGGCGAGGGCTACAACGTGGCCCAGTGCAACATGACCAAGGACTGGTTCCTGGTCCA
GATGCTGGCCCACTACAACATCGGCTACCAGGGCTTCTACGTGCCCGAGGGCTACAAGGACCGCATGTACTCCT
TCTTCCGCAACTTCCAGCCCATGAGCCGCCAGGTGGTGGACGAGGTCAACTACAAGGACTACCAGGCCGTCACC
CTGGCCTACCAGCACAACAACTCGGGCTTCGTCGGCTACCTCGCGCCCACCATGCGCCAGGGCCAGCCCTACCC
```

```
CGCCAACTACCCCTACCCGCTCATCGGCAAGAGCGCCGTCACCCAGCGTCACCCAGAAAAAGTTCCTCTGCGACA
GGGTCATGTGGCGCATCCCCTTCTCCAGCAACTTCATGTCCATGGGCGCGCTCACCGACCTCGGCCAGAACATG
CTCTATGCCAACTCCGCCCACGCGCTAGACATGAATTTCGAAGTCGACCCCATGGATGAGTCCACCCTTCTCTA
TGTTGTCTTCGAAGTCTTCGACGTCGTCCGAGTGCACCAGCCCCACCGCGGCGTCATCGAGGCCGTCTACCTGC
GCACCCCCTTCTCGGCCGGTAACGCCACCACCTAAGCTCTTGCTTCTTGCAAGCCATGGCCGCGGGCTCCGGCG
AGCAGGAGCTCAGGGCCATCATCCGCGACCTGGGCTGCGGGCCCTACTTCCTGGGCACCTTCGATAAGCGCTTC
CCGGGATTCATGGCCCCGCACAAGCTGGCCTGCGCCATCGTCAACACGGCCGGCCGCGAGACCGGGGGGCGAGCA
CTGGCTGGCCTTCGCCTGGAACCCGCGCTCGAACACCTGCTACCTCTTCGACCCCTTCGGGTTCTCGGACGAGC
GCCTCAAGCAGATCTACCAGTTCGAGTACGAGGGCCTGCTGCGCCGCAGCGCCCTGGCCACCGAGGACCGCTGC
GTCACCCTGGAAAAGTCCACCCAGACCGTGCAGGGTCCGCGCTCGGCCGCCTGCGGGCTCTTCTGCTGCATGTT
CCTGCACGCCTTCGTGCACTGGCCCGACCGCCCCATGGACAAGAACCCCACCATGAACTTGCTGACGGGGGTGC
CCAACGGCATGCTCCAGTCGCCCCAGGTGGAACCCACCCTGCGCCGCAACCAGGAGGCGCTCTACCGCTTCCTC
AACTCCCACTCCGCCTACTTTCGCTCCCACCGCGCGCGCATCGAGAAGGCCACCGCCTTCGACCGCATGAATCA
AGACATGTAAACCGTGTGTGTATGTTAAATGTCTTTAATAAACAGCACTTTCATGTTACACATGCATCTGAGAT
GATTTATTTAGAAATCGAAAGGGTTCTGCCGGGTCTCGGCATGGCCCGCGGGCAGGGACACGTTGCGGAACTGG
TACTTGGCCAGCCACTTGAACTCGGGGATCAGCAGTTTGGGCAGCGGGGTGTCGGGGAAGGAGTCGGTCCACAG
CTTCCGCGTCAGTTGCAGGGCGCCCAGCAGGTCGGGCGCGGAGATCTTGAAATCGCAGTTGGGACCCGCGTTCT
GCGCGCGGGAGTTGCGGTACACGGGGTTGCAGCACTGGAACACCATCAGGGCCGGGTGCTTCACGCTCGCCAGC
ACCGTCGCGTCGGTGATGCTCTCCACGTCGAGGTCCTCGGCGTTGGCCATCCCGAAGGGGGTCATCTTGCAGGT
CTGCCTTCCCATGGTGGGCACGCACCCGGGCTTGTGGTTGCAATCGCAGTGCAGGGGGATCAGCATCATCTGGG
CCTGGTCGGCGTTCATCCCCGGGTACATGGCCTTCATGAAAGCCTCCAATTGCCTGAACGCCTGCTGGGCCTTG
GCTCCCTCGGTGAAGAAGACCCCGCAGGACTTGCTAGAGAACTGGTTGGTGGCGCACCGGCGTCGTGCACGCA
GCAGCGCGCGTCGTTGTTGGCCAGCTGCACCACGCTGCGCCCCCAGCGGTTCTGGGTGATCTTGGCCCGGTCGG
GGTTCTCCTTCAGCGCGCGCTGCCCGTTCTCGCTCGCCACATCCATCTCGATCATGTGCTCCTTCTGGATCATG
GTGGTCCCGTGCAGGCACCGCAGCTTGCCCTCGGCCTCGGTGCACCCGTGCAGCCACAGCGCGCACCCGGTGCA
CTCCCAGTTCTTGTGGGCGATCTGGGAATGCGCGTGCACGAAGCCCTGCAGGAAGCGGCCCATCATGGTGGTCA
GGGTCTTGTTGCTAGTGAAGGTCAGCGGAATGCCGCGGTGCTCCTCGTTGATGTACAGGTGGCAGATGCGGCGG
TACACCTCGCCCTGCTCGGGCATCAGCTGGAAGTTGGCTTTCAGGTCGGTCTCCACGCGGTAGCGGTCCATCAG
CATAGTCATGATTTCCATACCCTTCTCCCAGGCCGAGACGATGGGCAGGCTCATAGGGTTCTTCACCATCATCT
TAGCGCTAGCAGCCGCGGCCAGGGGGTCGCTCTCGTCCAGGGTCTCAAAGCTCCGCTTGCCGTCCTTCTCGGTG
ATCCGCACCGGGGGGGTAGCTGAAGCCCACGGCCGCCAGCTCCTCCTCGGCCTGTCTTTCGTCCTCGCTGTCCTG
GCTGACGTCCTGCAGGACCACATGCTTGGTCTTGCGGGGTTTCTTCTTGGGCGGCAGCGGCGGCGGAGATGTTG
GAGATGGCGAGGGGGAGCGCGAGTTCTCGCTCACCACTACTATCTCTTCCTCTTCTTGGTCCGAGGCCACGCGG
CGGTAGGTATGTCTCTTCGGGGGCAGAGGCGGAGGCGACGGGCTCTCGCCGCCGCGACTTGGCGGATGGCTGGC
AGAGCCCCTTCCGCGTTCGGGGGTGCGCTCCCGGCGGCGCTCTGACTGACTTCCTCCGCGGCCGGCCATTGTGT
TCTCCTAGGGAGGAACAACAAGCATGGAGACTCAGCCATCGCCAACCTCGCCATCTGCCCCCACCGCCGACGAG
AAGCAGCAGCAGCAGAATGAAAGCTTAACCGCCCCGCCGCCCAGCCCCGCCACCTCCGACGCGGCCGTCCCAGA
CATGCAAGAGATGGAGGAATCCATCGAGATTGACCTGGGCTATGTGACGCCCGCGGAGCACGAGGAGGAGCTGG
CAGTGCGCTTTTCACAAGAAGAGATACACCAAGAACAGCCAGAGCAGGAAGCAGAGAATGAGCAGAGTCAGGCT
GGGCTCGAGCATGACGGCGACTACCTCCACCTGAGCGGGGGGGAGGACGCGCTCATCAAGCATCTGGCCCGGCA
GGCCACCATCGTCAAGGATGCGCTGCTCGACCGCACCGAGGTGCCCCTCAGCGTGGAGGAGCTCAGCCGCGCCT
ACGAGTTGAACCTCTTCTCGCCGCGCGTGCCCCCCAAGCGCCAGCCCAATGGCACCTGCGAGCCCAACCCGCGC
CTCAACTTCTACCCGGTCTTCGCGGTGCCCGAGGCCCTGGCCACCTACCACATCTTTTTCAAGAACCAAAAGAT
CCCCGTCTCCTGCCGCGCCAACCGCACCCGCGCCGACGCCCTTTTCAACCTGGGTCCCGGCGCCCGCCTACCTG
ATATCGCCTCCTTGGAAGAGGTTCCCAAGATCTTCGAGGGTCTGGGCAGCGACGAGACTCGGGCCGCGAACGCT
CTGCAAGGAGAAGGAGGAGAGCATGAGCACCACAGCGCCCTGGTCGAGTTGGAAGGCGACAACGCGCGGCTGGC
GGTGCTCAAACGCACGGTCGAGCTGACCCATTTCGCCTACCCGGCTCTGAACCTGCCCCCCAAAGTCATGAGCG
CGGTCATGGACCAGGTGCTCATCAAGCGCGCGTCGCCCATCTCCGAGGACGAGGGCATGCAAGACTCCGAGGAG
GGCAAGCCCGTGGTCAGCGACGAGCAGCTGGCCCGGTGGCTGGGTCCTAATGCTAGTCCCCAGAGTTTGGAAGA
GCGGCGCAAACTCATGATGGCCGTGGTCCTGGTGACCGTGGAGCTGGAGTGCCTGCGCCGCTTCTTCGCCGACG
CGGAGACCCTGCGCAAGGTCGAGGAGAACCTGCACTACCTCTTCAGGCACGGGTTCGTGCGCCAGGCCTGCAAG
ATCTCCAACGTGGAGCTGACCAACCTGGTCTCCTACATGGGCATCTTGCACGAGAACCGCCTGGGGCAGAACGT
GCTGCACACCACCCTGCGCGGGGAGGCCCGGCGCGACTACATCCGCGACTGCGTCTACCTCTACCTCTGCCACA
CCTGGCAGACGGGCATGGGCGTGTGGCAGCAGTGTCTGGAGGAGCAGAACCTGAAAGAGCTCTGCAAGCTCCTG
CAGAAGAACCTCAAGGGTCTGTGGACCGGGTTCGACGAGCGCACCACCGCCTCGGACCTGGCCGACCTCATTTT
CCCCGAGCGCCTCAGGCTGACGCTGCGCAACGGCCTGCCCGACTTTATGAGCCAAAGCATGTTGCAAAACTTTC
GCTCTTTCATCCTCGAACGCTCCGGAATCCTGCCCGCCACCTGCTCCGCGCTGCCCTCGGACTTCGTGCCGCTG
ACCTTCCGCGAGTGCCCCCCGCCGCTGTGGAGCCACTGCTACCTGCTGCGCCTGGCCAACTACCTGGCCTACCA
CTCGGACGTGATCGAGGACGTCAGCGGCGAGGGCCTGCTCGAGTGCCACTGCCGCTGCAACCTCTGCACGCCGC
ACCGCTCCCTGGCCTGCAACCCCCAGCTGCTGAGCGAGACCCAGATCATCGGCACCTTCGAGTTGCAAGGGCCC
AGCGAAGGCGAGGGTTCAGCCGCCAAGGGGGGGTCTGAAACTCACCCCGGGGCTGTGGACCTCGGCCTACTTGCG
CAAGTTCGTGCCCGAGGACTACCATCCCTTCGAGATCAGGTTCTACGAGGACCAATCCCATCCGCCCAAGGCCG
```

```
AGCTGTCGGCCTGCGTCATCACCCAGGGGGCGATCCTGGCCCAATTGCAAGCCATCCAGAAATCCCGCCAAGAA
TTCTTGCTGAAAAAGGGCCGCGGGGTCTACCTCGACCCCCAGACCGGTGAGGAGCTCAACCCCGGCTTCCCCCA
GGATGCCCCGAGGAAACAAGAAGCTGAAAGTGGAGCTGCCGCCCGTGGAGGATTTGGAGGAAGACTGGGAGAAC
AGCAGTCAGGCAGAGGAGGAGGAGATGGAGGAAGACTGGGACAGCACTCAGGCAGAGGAGGACAGCCTGCAAGA
CAGTCTGGAGGAAGACGAGGAGGAGGCAGAGGAGGAGGTGGAAGAAGCAGCCGCCGCCAGACCGTCGTCCTCGG
CGGGGGAGAAAGCAAGCAGCACGGATACCATCTCCGCTCCGGGTCGGGGTCCCGCTCGACCACACAGTAGATGG
GACGAGACCGGACGATTCCCGAACCCCACCACCCAGACCGGTAAGAAGGAGCGGCAGGGATACAAGTCCTGGCG
GGGGCACAAAAACGCCATCGTCTCCTGCTTGCAGGCCTGCGGGGGCAACATCTCCTTCACCCGGCGCTACCTGC
TCTTCCACCGCGGGGTGAACTTTCCCCGCAACATCTTGCATTACTACCGTCACCTCCACAGCCCCTACTACTTC
CAAGAAGAGGCAGCAGCAGCAGAAAAAGACCAGCAGAAAACCAGCAGCTAGAAAATCCACAGCGGCGGCAGCAG
GTGGACTGAGGATCGCGGCGAACGAGCCGGCGCAAACCCGGGAGCTGAGGAACCGGATCTTTCCCACCCTCTAT
GCCATCTTCCAGCAGAGTCGGGGGCAGGAGCAGGAACTGAAAGTCAAGAACCGTTCTCTGCGCTCGCTCACCCG
CAGTTGTCTGTATCACAAGAGCGAAGACCAACTTCAGCGCACTCTCGAGGACGCCGAGGCTCTCTTCAACAAGT
ACTGCGCGCTCACTCTTAAAGAGTAGCCCGCGCCCGCCCAGTCGCAGAAAAAGGCGGGAATTACGTCACCTGTG
CCCTTCGCCCTAGCCGCCTCCACCCATCATCATGAGCAAAGAGATTCCCACGCCTTACATGTGGAGCTACCAGC
CCCAGATGGGCCTGGCCGCCGGTGCCGCCCAGGACTACTCCACCCGCATGAATTGGCTCAGCGCCGGGCCCGCG
ATGATCTCACGGGTGAATGACATCCGCGCCCACCGAAACCAGATACTCCTAGAACAGTCAGCGCTCACCGCCAC
GCCCCGCAATCACCTCAATCCGCGTAATTGGCCCGCCGCCCTGGTGTACCAGGAAATTCCCCAGCCCACGACCG
TACTACTTCCGCGAGACGCCCAGGCCGAAGTCCAGCTGACTAACTCAGGTGTCCAGCTGGCGGGCGGCGCCACC
CTGTGTCGTCACCGCCCCGCTCAGGGTATAAAGCGGCTGGTGATCCGGGGCAGAGGCACACAGCTCAACGACGA
GGTGGTGAGCTCTTCGCTGGGTCTGCGACCTGACGGAGTCTTCCAACTCGCCGGATCGGGGAGATCTTCCTTCA
CGCCTCGTCAGGCCGTCCTGACTTTGGAGAGTTCGTCCTCGCAGCCCCGCTCGGGTGGCATCGGCACTCTCCAG
TTCGTGGAGGAGTTCACTCCCTCGGTCTACTTCAACCCCTTCTCCGGCTCCCCCGGCCACTACCCGGACGAGTT
CATCCCGAACTTCGACGCCATCAGCGAGTCGGTGGACGGCTACGATTGAATGTCCCATGGTGGCGCAGCTGACC
TAGCTCGGCTTCGACACCTGGACCACTGCCGCCGCTTCCGCTGCTTCGCTCGGGATCTCGCCGAGTTTGCCTAC
TTTGAGCTGCCCGAGGAGCACCCTCAGGGCCCGGCCCACGGAGTGCGGATCGTCGTCGAAGGGGGCCTCGACTC
CCACCTGCTTCGGATCTTCAGCCAGCGTCCGATCCTGGTCGAGCGCGAGCAAGGACAGACCCTTCTGACTCTGT
ACTGCATCTGCAACCACCCCGGCCTGCATGAAAGTCTTTGTTGTCTGCTGTGTACTGAGTATAATAAAAGCTGA
GATCAGCGACTACTCCGGACTTCCGTGTGTTCCTGAATCCATCAACCAGTCTTTGTTCTTCACCGGGAACGAGA
CCGAGCTCCAGCTCCAGTGTAAGCCCCACAAGAAGTACCTCACCTGGCTGTTCCAGGGCTCCCCGATCGCCGTT
GTCAACCACTGCGACAACGACGGAGTCCTGCTGAGCGGCCCTGCCAACCTTACTTTTTCCACCCGCAGAAGCAA
GCTCCAGCTCTTCCAACCCTTCCTCCCCGGGACCTATCAGTGCGTCTCGGGACCCTGCCATCACACCTTCCACC
TGATCCCGAATACCACAGCGTCGCTCCCCGCTACTAACAACCAAACTAACCTCCACCAACGCCACCGTCGCGAC
GGCCACAATACATGCCCATATTAGACTATGAGGCCGAGCCACAGCGACCCATGCTCCCCGCTATTAGTTACTTC
AATCTAACCGGCGGAGATGACTGACCCACTGGCCAACAACAACGTCAACGACCTTCTCCTGGACATGGACGGCC
GCGCCTCGGAGCAGCGACTCGCCCAACTTCGCATTCGCCAGCAGCAGGAGAGAGCCGTCAAGGAGCTGCAGGAT
GCGGTGGCCATCCACCAGTGCAAGAGAGGCATCTTCTGCCTGGTGAAACAGGCCAAGATCTCCTACGAGGTCAC
TCCAAACGACCATCGCCTCTCCTACGAGCTCCTGCAGCAGCGCCAGAAGTTCACCTGCCTGGTCGGAGTCAACC
CCATCGTCATCACCCAGCAGTCTGGCGATACCAAGGGGTGCATCCACTGCTCCTGCGACTCCCCCGACTGCGTC
CACACTCTGATCAAGACCCTCTGCGGCCTCCGCGACCTCCTCCCCATGAACTAATCACCCCCTTATCCAGTGAA
ATAAAGATCATATTGATGATGATTTTACAGAAATAAAAAATAATCATTTGATTTGAAATAAAGATACAATCATA
TTGATGATTTGAGTTTAACAAAAAAATAAAGAATCACTTACTTGAAATCTGATACCAGGTCTCTGTCCATGTTT
TCTGCCAACACCACTTCACTCCCCTCTTCCCAGCTCTGGTACTGCAGGCCCCGGCGGGCTGCAAACTTCCTCCA
CACGCTGAAGGGGATGTCAAATTCCTCCTGTCCCTCAATCTTCATTTTATCTTCTATCAGATGTCCAAAAAGCG
CGTCCGGGTGGATGATGACTTCGACCCCGTCTACCCCTACGATGCAGACAACGCACCGACCGTGCCCTTCATCA
ACCCCCCCTTCGTCTCTTCAGATGGATTCCAAGAGAAGCCCCTGGGGGTGTTGTCCCTGCGACTGGCCGACCCC
GTCACCACCAAGAACGGGGAAATCACCCTCAAGCTGGGAGAGGGGGTGGACCTCGATTCCTCGGGAAAACTCAT
CTCCAACACGGCCACCAAGGCCGCCGCCCCTCTCAGTTTTTCCAACAACACCATTTCCCTTAACATGGATCACC
CCTTTTACACTAAAGATGGAAAATTATCCTTACAAGTTTCTCCACCATTAAATATACTGAGAACAAGCATTCTA
AACACACTAGCTTTAGGTTTTGGATCAGGTTTAGGACTCCGTGGCTCTGCCTTGGCAGTACAGTTAGTCTCTCC
ACTTACATTTGATACTGATGGAAACATAAAGCTTACCTTAGACAGAGGTTTGCATGTTACAACAGGAGATGCAA
TTGAAAGCAACATAAGCTGGGCTAAAGGTTTAAAATTTGAAGATGGAGCCATAGCAACCAACATTGGAAATGGG
TTAGAGTTTGGAAGCAGTAGTACAGAAACAGGTGTTGATGATGCTTACCCAATCCAAGTTAAACTTGGATCTGG
CCTTAGCTTTGACAGTACAGGAGCCATAATGGCTGGTAACAAAGAAGACGATAAACTCACTTTGTGGACAACAC
CTGATCCATCACCAAACTGTCAAATACTCGCAGAAAATGATGCAAAACTAACACTTTGCTTGACTAAATGTGGT
AGTCAAATACTGGCCACTGTGTCAGTCTTAGTTGTAGGAAGTGGAAACCTAAACCCCATTACTGGCACCGTAAG
CAGTGCTCAGGTGTTTCTACGTTTTGATGCAAACGGTGTTCTTTTAACAGAACATTCTACACTAAAAAAATACT
GGGGGTATAGGCAGGGAGATAGCATAGATGGCACTCCATATACCAATGCTGTAGGATTCATGCCCAATTTAAAA
GCTTATCCAAAGTCACAAAGTTCTACTACTAAAATAATATAGTAGGGCAAGTATACATGAATGGAGATGTTTC
AAAACCTATGCTTCTCACTATAACCCTCAATGGTACTGATGACAGCAACAGTACATATTCAATGTCATTTTCAT
ACACCTGGACTAATGGAAGCTATGTTGGAGCAACATTTGGGGCTAACTCTTATACCTTCTCATACATCGCCCAA
GAATGAACACTGTATCCCACCCTGCATGCCAACCCTTCCCACCCCACTCTGTGGAACAAACTCTGAAACACAAA
```

```
ATAAAATAAAGTTCAAGTGTTTTATTGATTCAACAGTTTTACAGGATTCGAGCAGTTATTTTTCCTCCACCCTC
CCAGGACATGGAATACACCACCCTCTCCCCCCGCACAGCCTTGAACATCTGAATGCCATTGGTGATGGACATGC
TTTTGGTCTCCACGTTCCACACAGTTTCAGAGCGAGCCAGTCTCGGGTCGGTCAGGGAGATGAAACCCTCCGGG
CACTCCCGCATCTGCACCTCACAGCTCAACAGCTGAGGATTGTCCTCGGTGGTCGGGATCACGGTTATCTGGAA
GAAGCAGAAGAGCGGCGGTGGGAATCATAGTCCGCGAACGGGATCGGCCGGTGGTGTCGCATCAGGCCCCGCAG
CAGTCGCTGCCGCCGCTCCGTCAAGCTGCTGCTCAGGGGGTCCGGGTCCAGGGACTCCCTCAGCATGATGC
CCACGGCCCTCAGCATCAGTCGTCTGGTGCGGCGGGCGCAGCAGCGCATGCGGATCTCGCTCAGGTCGCTGCAG
TACGTGCAACACAGAACCACCAGGTTGTTCAACAGTCCATAGTTCAACACGCTCCAGCCGAAACTCATCGCGGG
AAGGATGCTACCCACGTGGCCGTCGTACCAGATCCTCAGGTAAATCAAGTGGTGCCCCCTCCAGAACACGCTGC
CCACGTACATGATCTCCTTGGGCATGTGGCGGTTCACCACCTCCCGGTACCACATCACCCTCTGGTTGAACATG
CAGCCCCGGATGATCCTGCGGAACCACAGGGCCAGCACCGCCCCGCCCGCCATGCAGCGAAGAGACCCCGGGTC
CCGGCAATGGCAATGGAGGACCCACCGCTCGTACCCGTGGATCATCTGGGAGCTGAACAAGTCTATGTTGGCAC
AGCACAGGCATATGCTCATGCATCTCTTCAGCACTCTCAACTCCTCGGGGGGTCAAAAACCATATCCCAGGGCACG
GGGAACTCTTGCAGGACAGCGAACCCCGCAGAACAGGGCAATCCTCGCACAGAACTTACATTGTGCATGGACAG
GGTATCGCAATCAGGCAGCACCGGGTGATCCTCCACCAGAGAAGCGCGGGTCTCGGTCTCCTCACAGCGTGGTA
AGGGGGCCGGCCGATACGGGTGATGGCGGGACGCGGCTGATCGTGTTCGCGACCGTGTCATGATGCAGTTGCTT
TCGGACATTTTCGTACTTGCTGTAGCAGAACCTGGTCCGGGCGCTGCACACCGATCGCCGGCGGCGGTCTCGGC
GCTTGGAACGCTCGGTGTTGAAATTGTAAAACAGCCACTCTCTCAGACCGTGCAGCAGATCTAGGGCCTCAGGA
GTGATGAAGATCCCATCATGCCTGATGGCTCTGATCACATCGACCACCGTGGAATGGGCCAGACCCAGCCAGAT
GATGCAATTTTGTTGGGTTTCGGTGACGGCGGGGGAGGGAAGAACAGGAAGAACCATGATTAACTTTTAATCCA
AACGGTCTCGGAGTACTTCAAAATGAAGATCGCGGAGATGGCACCTCTCGCCCCCGCTGTGTTGGTGGAAAATA
ACAGCCAGGTCAAAGGTGATACGGTTCTCGAGATGTTCCACGGTGGCTTCCAGCAAAGCCTCCACGCGCACATC
CAGAAACAAGACAATAGCGAAAGCGGGAGGGGTTCTCTAATTCCTCAATCATCATGTTACACTCCTGCACCATCC
CCAGATAATTTTCATTTTTCCAGCCTTGAATGATTCGAACTAGTTCGTGAGGTAAATCCAAGCCAGCCATGATA
AAGAGCTCGCGCAGAGCGCCCTCCACCGGCATTCTTAAGCACACCCTCATAATTCCAAGATATTCTGCTCCTGG
TTCACCTGCAGCAGATTGACAAGCGGAATATCAAAATCTCTGCCGCGATCCCTGAGCTCCTCCCTCAGCAATAA
CTGTAAGTACTCTTTCATATCCTCTCCGAAATTTTTAGCCATAGGACCACCAGGAATAAGATTAGGGCAAGCCA
CAGTACAGATAAACCGAAGTCCTCCCCAGTGAGCATTGCCAAATGCAAGACTGCTATAAGCATGCTGGCTAGAC
CCGGTGATATCTTCCAGATAACTGGACAGAAAATCGCCCAGGCAATTTTTAAGAAAATCAACAAAAGAAAAATC
CTCCAGGTGGACGTTTAGAGCCTCGGGAACAACGATGAAGTAAATGCAAGCGGTGCGTTCCAGCATGGTTAGTT
AGCTGATCTGTAGAAAAAACAAAAATGAACATTAAACCATGCTAGCCTGGCGAACAGGTGGGTAAATCGTTCTC
TCCAGCACCAGGCAGGCCACGGGGTCTCCGGCGCGACCCTCGTAAAAATTGTCGCTATGATTGAAAACCATCAC
AGAGAGACGTTCCCGGTGGCCGGCGTGAATGATTCGACAAGATGAATACACCCCCGGAACATTGGCGTCCGCGA
GTGAAAAAAGCGCCCGAGGAAGCAATAAGGCACTACAATGCTCAGTCTCAAGTCCAGCAAAGCGATGCCATGC
GGATGAAGCACAAAATTCTCAGGTGCGTACAAAATGTAATTACTCCCCTCCTGCACAGGCAGCAAAGCCCCCGA
TCCCTCCAGGTACACATACAAAGCCTCAGCGTCCATAGCTTACCGAGCAGCAGCACACAACAGGCGCAAGAGTC
AGAGAAAGGCTGAGCTCTAACCTGTCCACCCGCTCTCTGCTCAATATATAGCCCAGATCTACACTGACGTAAAG
GCCAAAGTCTAAAAATACCCGCCAAATAATCACACACGCCCAGCACACGCCCAGAAACCGGTGACACACTCAAA
AAAATACGCGCACTTCCTCAAACGCCCAAAACTGCCGTCATTTCCGGGTTCCCACGCTACGTCATCAAAACACG
ACTTTCAAATTCCGTCGACCGTTAAAAACGTCACCCGCCCCGCCCCTAACGGTCGCCCGTCTCTCAGCCAATCA
GCGCCCCGCATCCCCAAATTCAAACACCTCATTTGCATATTAACGCGCACAAAAGTTTGAGGTATATTATTGA
TGATGG
```

| ChAdV68.5WTnt.GFP (SEQ ID NO:13); AC_000011.1 with E1 (nt 577 to 3403) and E3 (nt 27,125- 31,825) sequences deleted; corresponding ATCC VR-594 nucleotides substituted at five positions; GFP reporter under the control of the CMV promoter/enhancer inserted in place of deleted E1 |
| --- |

```
CCATCTTCAATAATATACCTCAAACTTTTTGTGCGCGTTAATATGCAAATGAGGCGTTTGAATTTGGGGAGGAA
GGGCGGTGATTGGTCGAGGGATGAGCGACCGTTAGGGGCGGGGCGAGTGACGTTTTGATGACGTGGTTGCGAGG
AGGAGCCAGTTTGCAAGTTCTCGTGGGAAAAGTGACGTCAAACGAGGTGTGGTTTGAACACGGAAATACTCAAT
TTTCCCGCGCTCTCTGACAGGAAATGAGGTGTTTCTGGGCGGATGCAAGTGAAAACGGGCCATTTTCGCGCGAA
AACTGAATGAGGAAGTGAAATCTGAGTAATTTCGCGTTTATGGCAGGGAGGAGTATTTGCCGAGGGCCGAGTA
GACTTTGACCGATTACGTGGGGGTTTCGATTACCGTGTTTTTCACCTAAATTTCCGCGTACGGTGTCAAAGTCC
GGTGTTTTTACGTAGGTGTCAGCTGATCGCCAGGGTATTTAAACCTGCGCTCTCCAGTCAAGAGGCCACTCTTG
AGTGCCAGCGAGAAGAGTTTTCTCCTCCGCCGCGAGTCAGATCTACACTTTGAAAGTAGGGATAACAGGGTA
ATgacattgattattgactagttGttaaTAGTAATCAATTACGGGGTCATTAGTTCATAGCCCATATATGGAGT
TCCGCGTTACATAACTTACGGTAAATGGCCCGCCTGGCTGACCGCCCAACGACCCCCGCCCATTGACGTCAATA
ATGACGTATGTTCCCATAGTAACGCCAATAGGGACTTTCCATTGACGTCAATGGGTGGAGTATTTACGGTAAAC
TGCCCACTTGGCAGTACATCAAGTGTATCATATGCCAAGTCCGCCCCCTATTGACGTCAATGACGGTAAATGGC
CCGCCTGGCATTATGCCCAGTACATGACCTTACGGGACTTTCCTACTTGGCAGTACATCTACGTATTAGTCATC
GCTATTACCATGgTGATGCGGTTTTGGCAGTACACCAATGGGCGTGGATAGCGGTTTGACTCACGGGGATTTCC
AAGTCTCCACCCCATTGACGTCAATGGGAGTTTGTTTTGGCACCAAAATCAACGGGACTTTCCAAAATGTCGTA
ATAACCCCGCCCCGTTGACGCAAATGGGCGGTAGGCGTGTACGGTGGGAGGTCTATATAAGCAGAgcTCGTTTA
```

```
GTGAACCGTCAGATCGCCTGGAACGCCATCCACGCTGTTTTGACCTCCATAGAAGACAGCGATCGCGccaccAT
GGTGAGCAAGGGCGAGGAGCTGTTCACCGGGGTGGTGCCCATCCTGGTCGAGCTGGACGGCGACGTAAACGGCC
ACAAGTTCAGCGTGTCCGGCGAGGGCGAGGGCGATGCCACCTACGGCAAGCTGACCCTGAAGTTCATCTGCACC
ACCGGCAAGCTGCCCGTGCCCTGGCCCACCCTCGTGACCACCCTGACCTACGGCGTGCAGTGCTTCAGCCGCTA
CCCCGACCACATGAAGCAGCACGACTTCTTCAAGTCCGCCATGCCCGAAGGCTACGTCCAGGAGCGCACCATCT
TCTTCAAGGACGACGGCAACTACAAGACCCGCGCCGAGGTGAAGTTCGAGGGCGACACCCTGGTGAACCGCATC
GAGCTGAAGGGCATCGACTTCAAGGAGGACGGCAACATCCTGGGGCACAAGCTGGAGTACAACTACAACAGCCA
CAACGTCTATATCATGGCCGACAAGCAGAAGAACGGCATCAAGGTGAACTTCAAGATCCGCCACAACATCGAGG
ACGGCAGCGTGCAGCTCGCCGACCACTACCAGCAGAACACCCCCATCGGCGACGGCCCCGTGCTGCTGCCCGAC
AACCACTACCTGAGCACCCAGTCCGCCCTGAGCAAAGACCCCAACGAGAAGCGCGATCACATGGTCCTGCTGGA
GTTCGTGACCGCCGCCGGGATCACTCTCGGCATGGACGAGCTtTACAAGTAGtgaGTTTAAACTCCCATTTAAA
TGTGAGGGTTAATGCTTCGAGCAGACATGATAAGATACATTGATGAGTTTGGACAAACCACAACTAGAATGCAG
TGAAAAAAATGCTTTATTTGTGAAATTTGTGATGCTATTGCTTTATTTGTAACCATTATAAGCTGCAATAAACA
AGTTAACAACAACAATTGCATTCATTTTATGTTTCAGGTTCAGGGGGAGATGTGGGAGGTTTTTTAAAGCAAGT
AAAACCTCTACAAATGTGGTAAAATAACTATAACGGTCCTAAGGTAGCGAGTGAGTAGTGTTCTGGGGCGGGGG
AGGACCTGCATGAGGGCCAGAATAACTGAAATCTGTGCTTTTCTGTGTGTTGCAGCAGCATGAGCGGAAGCGGC
TCCTTTGAGGGAGGGGTATTCAGCCCTTATCTGACGGGGCGTCTCCCTCCTGGGCGGGAGTGCGTCAGAATGT
GATGGGATCCACGGTGGACGGCCGGCCCGTGCAGCCCGCGAACTCTTCAACCCTGACCTATGCAACCCTGAGCT
CTTCGTCGTTGGACGCAGCTGCCGCCGCAGCTGCTGCATCTGCCGCCAGCGCCGTGCGCGGAATGGCCATGGGC
GCCGGCTACTACGGCACTCTGGTGGCCAACTCGAGTTCCACCAATAATCCCGCCAGCCTGAACGAGGAGAAGCT
GTTGCTGCTGATGGCCCAGCTCGAGGCCTTGACCCAGCGCCTGGGCGAGCTGACCCAGCAGGTGGCTCAGCTGC
AGGAGCAGACGCGGGCCGCGGTTGCCACGGTGAAATCCAAATAAAAAATGAATCAATAAATAAACGGAGACGGT
TGTTGATTTTAACACAGAGTCTGAATCTTTATTTGATTTTTCGCGCGCGGTAGGCCCTGGACCACCGGTCTCGA
TCATTGAGCACCCGGTGGATCTTTTCCAGGACCCGGTAGAGGTGGGCTTGGATGTTGAGGTACATGGGCATGAG
CCCGTCCCGGGGGTGGAGGTAGCTCCATTGCAGGGCCTCGTGCTCGGGGGTGGTGTTGTAAATCACCCAGTCAT
AGCAGGGGCGCAGGGCATGGTGTTGCACAATATCTTTGAGGAGGAGACTGATGGCCACGGGCAGCCCTTTGGTG
TAGGTGTTTACAAATCTGTTGAGCTGGGAGGGATGCATGCGGGGGGAGATGAGGTGCATCTTGGCCTGGATCTT
GAGATTGGCGATGTTACCGCCCAGATCCCGCCTGGGGTTCATGTTGTGCAGGACCACCAGCACGGTGTATCCGG
TGCACTTGGGGAATTTATCATGCAACTTGGAAGGGAAGGCGTGAAAGAATTTGGCGACGCCTTTGTGCCCGCCC
AGGTTTTCCATGCACTCATCCATGATGATGGCGATGGGCCCGTGGGCGGCGGCCTGGGCAAAGACGTTTCGGGG
GTCGGACACATCATAGTTGTGGTCCTGGGTGAGGTCATCATAGGCCATTTTAATGAATTTGGGGCGGAGGGTGC
CGGACTGGGGGACAAAGGTACCCTCGATCCCGGGGGCGTAGTTCCCCTCACAGATCTGCATCTCCCAGGCTTTG
AGCTCGGAGGGGGGGATCATGTCCACCTGCGGGGCGATAAAGAACACGGTTTCCGGGGCGGGGGAGATGAGCTG
GGCCGAAAGCAAGTTCCGGAGCAGCTGGGACTTGCCGCAGCCGGTGGGGCCGTAGATGACCCCGATGACCGGCT
GCAGGTGGTAGTTGAGGGAGAGACAGCTGCCGTCCTCCCGGAGGAGGGGGGCCACCTCGTTCATCATCTCGCGC
ACGTGCATGTTCTCGCGCACCAGTTCCGCCAGGAGGCGCTCTCCCCCCAGGGATAGGAGCTCCTGGAGCGAGGC
GAAGTTTTTCAGCGGCTTGAGTCCGTCGGCCATGGGCATTTTGGAGAGGGTTTGTTGCAAGAGTTCCAGGCGGT
CCCAGAGCTCGGTGATGTGCTCTACGGCATCTCGATCCAGCAGACCTCCTCGTTTCGCGGGTTGGGACGGCTGC
GGGAGTAGGGCACCAGACGATGGGCGTCCAGCGCAGCCAGGGTCCGGTCCTTCCAGGGTCGCAGCGTCCGCGTC
AGGGTGGTCTCCGTCACGGTGAAGGGGTGCGCGCCGGGCTGGGCGCTTGCGAGGGTGCGCTTCAGGCTCATCCG
GCTGGTCGAAAACCGCTCCCGATCGGCGCCCTGCGCGTCGGCCAGGTAGCAATTGACCATGAGTTCGTAGTTGA
GCGCCTCGGCCGCGTGGCCTTTGGCGCGGAGCTTACCTTTGGAAGTCTGCCCGCAGGCGGGACAGAGGAGGGAC
TTGAGGGCGTAGAGCTTGGGGGCGAGGAAGACGGACTCGGGGGCGTAGGCGTCCGCGCCGCAGTGGGCGCAGAC
GGTCTCGCACTCCACGAGCCAGGTGAGGTCGGGCTGGTCGGGGTCAAAAACCAGTTTCCCGCCGTTCTTTTTGA
TGCGTTTCTTACCTTTGGTCTCCATGAGCTCGTGTCCCCGCTGGGTGACAAAGAGGCTGTCCGTGTCCCCGTAG
ACCGACTTTATGGGCCGGTCCTCGAGCGGTGTGCCGCGGTCCTCCTCGTAGAGGAACCCCGCCCACTCCGAGAC
GAAAGCCCGGGTCCAGGCCAGCACGAAGGAGGCCACGTGGGACGGGTAGCGGTCGTTGTCCACCAGCGGGTCCA
CCTTTTCCAGGGTATGCAAACACATGTCCCCCTCGTCCACATCCAGGAAGGTGATTGGCTTGTAAGTGTAGGCC
ACGTGACCGGGGGTCCCGGCCGGGGGGGTATAAAAGGGTGCGGGTCCCTGCTCGTCCTCACTGTCTTCCGGATC
GCTGTCCAGGAGCGCCAGCTGTTGGGGTAGGTATTCCCTCTCGAAGGCGGGCATGACCTCGGCACTCAGGTTGT
CAGTTTCTAGAAACGAGGAGGATTTGATATTGACGGTGCCGGCGGAGATGCCTTTCAAGAGCCCCTCGTCCATC
TGGTCAGAAAAGACGATCTTTTTGTTGTCGAGCTTGGTGGCGAAGGAGCCGTAGAGGGCGTTGGAGAGGAGCTT
GGCGATGGAGCGCATGGTCTGGTTTTTTTCCTTGTCGGCGCGCTCCTTGGCGGCGATGTTGAGCTGCACGTACT
CGCGCGCCACGCACTTCCATTCGGGGAAGACGGTGGTCAGCTCGTCGGGCACGATTCTGACCTGCCAGCCCCGA
TTATGCAGGGTGATGAGGTCCACACTGGTGGCCACCTCGCCGCGCAGGGGCTCATTAGTCCAGCAGAGGCGTCC
GCCCTTGCGCGAGCAGAAGGGGGGCAGGGGGTCCAGCATGACCTCGTCGGGGGGGTCGGCATCGATGGTGAAGA
TGCCGGGCAGGAGGTCGGGGTCAAAGTAGCTGATGGAAGTGGCCAGATCGTCCAGGGCAGCTTGCCATTCGCGC
ACGGCCAGCGCGCGCTCGTAGGGACTGAGGGGCGTGCCCCAGGGCATGGGATGGGTAAGCGCGGAGGCGTACAT
GCCGCAGATGTCGTAGACGTAGAGGGGCTCCTCGAGGATGCCGATGTAGGTGGGGTAGCAGCGCCCCCGCGGA
TGCTGGCGCGCACGTAGTCATACAGCTCGTGCGAGGGGGCGAGGAGCCCCGGGCCCAGGTTGGTGCGACTGGGC
TTTTCGGCGCGGTAGACGATCTGGCGGAAAATGGCATGCGAGTTGGAGGAGATGGTGGGCCTTTGGAAGATGTT
GAAGTGGGCGTGGGGCAGTCCGACCGAGTCGCGGATGAAGTGGGCGTAGGAGTCTTGCAGCTTGGCGACGAGCT
```

```
CGGCGGTGACTAGGACGTCCAGAGCGCAGTAGTCGAGGGTCTCCTGGATGATGTCATACTTGAGCTGTCCCTTT
TGTTTCCACAGCTCGCGGTTGAGAAGGAACTCTTCGCGGTCCTTCCAGTACTCTTCGAGGGGGAACCCGTCCTG
ATCTGCACGGTAAGAGCCTAGCATGTAGAACTGGTTGACGGCCTTGTAGGCGCAGCAGCCCTTCTCCACGGGGA
GGGCGTAGGCCTGGGCGGCCTTGCGCAGGGAGGTGTGCGTGAGGGCGAAAGTGTCCCTGACCATGACCTTGAGG
AACTGGTGCTTGAAGTCGATATCGTCGCAGCCCCCCTGCTCCCAGAGCTGGAAGTCCGTGCGCTTCTTGTAGGC
GGGGTTGGGCAAAGCGAAAGTAACATCGTTGAAGAGGATCTTGCCCGCGCGGGGCATAAAGTTGCGAGTGATGC
GGAAAGGTTGGGGCACCTCGGCCCGGTTGTTGATGACCTGGGCGGCGAGCACGATCTCGTCGAAGCCGTTGATG
TTGTGGCCCACGATGTAGAGTTCCACGAATCGCGGACGGCCCTTGACGTGGGGCAGTTTCTTGAGCTCCTCGTA
GGTGAGCTCGTCGGGGTCGCTGAGCCCGTGCTGCTCGAGCGCCCAGTCGGCGAGATGGGGGTTGGCGCGGAGGA
AGGAAGTCCAGAGATCCACGGCCAGGGCGGTTTGCAGACGGTCCCGGTACTGACGGAACTGCTGCCCGACGGCC
ATTTTTTCGGGGGTGACGCAGTAGAAGGTGCGGGGGTCCCCGTGCCAGCGATCCCATTTGAGCTGGAGGGCGAG
ATCGAGGGCGAGCTCGACGAGCCGGTCGTCCCCGGAGAGTTTCATGACCAGCATGAAGGGGACGAGCTGCTTGC
CGAAGGACCCCATCCAGGTGTAGGTTTCCACATCGTAGGTGAGGAAGAGCCTTTCGGTGCGAGGATGCGAGCCG
ATGGGGAAGAACTGGATCTCCTGCCACCAATTGGAGGAATGGCTGTTGATGTGATGGAAGTAGAAATGCCGACG
GCGCGCCGAACACTCGTGCTTGTGTTTATACAAGCGGCCACAGTGCTCGCAACGCTGCACGGGATGCACGTGCT
GCACGAGCTGTACCTGAGTTCCTTTGACGAGGAATTTCAGTGGGAAGTGGAGTCGTGGCGCCTGCATCTCGTGC
TGTACTACGTCGTGGTGGTCGGCCTGGCCCTCTTCTGCCTCGATGGTGGTCATGCTGACGAGCCCGCGCGGGAG
GCAGGTCCAGACCTCGGCGCGAGCGGGTCGGAGAGCGAGGACGAGGGCGCGCAGGCCGGAGCTGTCCAGGGTCC
TGAGACGCTGCGGAGTCAGGTCAGTGGGCAGCGGCGGCGCGCGGTTGACTTGCAGGAGTTTTTCCAGGGCGCGC
GGGAGGTCCAGATGGTACTTGATCTCCACCGCGCCATTGGTGGCGACGTCGATGGCTTGCAGGGTCCCGTGCCC
CTGGGGTGTGACCACCGTCCCCGTTTCTTCTTGGGCGGCTGGGGCGACGGGGGCGGTGCCTCTTCCATGGTTA
GAAGCGGCGGCGAGGACGCGCGCCGGGCGGCAGGGGCGGCTCGGGGCCCGGAGGCAGGGCGGCAGGGGCACGT
CGGCGCCGCGCGCGGGTAGGTTCTGGTACTGCGCCCGGAGAAGACTGGCGTGAGCGACGACGCGACGGTTGACG
TCCTGGATCTGACGCCTCTGGGTGAAGGCCACGGGACCCGTGAGTTTGAACCTGAAAGAGAGTTCGACAGAATC
AATCTCGGTATCGTTGACGGCGGCCTGCCGCAGGATCTCTTGCACGTCGCCCGAGTTGTCCTGGTAGGCGATCT
CGGTCATGAACTGCTCGATCTCCTCCTCTTGAAGGTCTCCGCGGCCGGCGCGCTCCACGGTGGCCGCGAGGTCG
TTGGAGATGCGGCCCATGAGCTGCGAGAAGGCGTTCATGCCCGCCTCGTTCCAGACGCGGCTGTAGACCACGAC
GCCCTCGGGATCGCgGGCGCGCATGACCACCTGGGCGAGGTTGAGCTCCACGTGGCGCGTGAAGACCGCGTAGT
TGCAGAGGCGCTGGTAGAGGTAGTTGAGCGTGGTGGCGATGTGCTCGGTGACGAAGAAATACATGATCCAGCGG
CGGAGCGGCATCTCGCTGACGTCGCCCAGCGCCTCCAAACGTTCCATGGCCTCGTAAAAGTCCACGGCGAAGTT
GAAAAACTGGGAGTTGCGCGCCGAGACGGTCAACTCCTCCTCCAGAAGACGGATGAGCTCGGCGATGGTGGCGC
GCACCTCGCGCTCGAAGGCCCCCGGGAGTTCCTCCACTTCCTCTTCTTCCTCCTCCACTAACATCTCTTCTACT
TCCTCCTCAGGCGGCAGTGGTGGCGGGGGAGGGGGCCTGCGTCGCCGGCGGCGCACGGGCAGACGGTCGATGAA
GCGCTCGATGGTCTCGCCGCGCCGGCGTCGCATGGTCTCGGTGACGGCGCGCCCGTCCTCGCGGGGCCGCAGCG
TGAAGACGCCGCCGCGCATCTCCAGGTGGCCGGGGGGGTCCCCGTTGGGCAGGGAGAGGGCGCTGACGATGCAT
CTTATCAATTGCCCCGTAGGGACTCCGCGCAAGGACCTGAGCGTCTCGAGATCCACGGGATCTGAAAACCGCTG
AACGAAGGCTTCGAGCCAGTCGCAGTCGCAAGGTAGGCTGAGCACGGTTTCTTCTGGCGGGTCATGTTGGTTGG
GAGCGGGGCGGGCGATGCTGCTGGTGATGAAGTTGAAATAGGCGGTTCTGAGACGGCGGATGGTGGCGAGGAGC
ACCAGGTCTTTGGGCCCGGCTTGCTGGATGCGCAGACGGTCGGCCATGCCCCAGGCGTGGTCCTGACACCTGGC
CAGGTCCTTGTAGTAGTCCTGCATGAGCCGCTCCACGGGCACCTCCTCCTCGCCCGCGCGGCCGTGCATGCGCG
TGAGCCCGAAGCCGCGCTGGGGCTGGACGAGCGCCAGGTCGGCGACGACGCGCTCGGCGAGGATGGCTTGCTGG
ATCTGGGTGAGGGTGGTCTGGAAGTCATCAAAGTCGACGAAGCGGTGGTAGGCTCCGGTGTTGATGGTGTAGGA
GCAGTTGGCCATGACGGACCAGTTGACGGTCTGGTGGCCCGGACGCACGAGCTCGTGGTACTTGAGGCGCGAGT
AGGCGCGCGTGTCGAAGATGTAGTCGTTGCAGGTGCGCACCAGGTACTGGTAGCCGATGAGGAAGTGCGGCGGC
GGCTGGCGGTAGAGCGGCCATCGCTCGGTGGCGGGGGCGCCGGGCGCGAGGTCCTCGAGCATGGTGCGGTGGTA
GCCGTAGATGTACCTGGACATCCAGGTGATGCCGGCGGCGGTGGTGGAGGCGCGCGGGAACTCGCGGACGCGGT
TCCAGATGTTGCGCAGCGGCAGGAAGTAGTTCATGGTGGGCACGGTCTGGCCCGTGAGGCGCGCGCAGTCGTGG
ATGCTCTATACGGGCAAAAACGAAAGCGGTCAGCGGCTCGACTCCGTGGCCTGGAGGCTAAGCGAACGGGTTGG
GCTGCGCGTGTACCCCGGTTCGAATCTCGAATCAGGCTGGAGCCGCAGCTAACGTGGTATTGGCACTCCCGTCT
CGACCCAAGCCTGCACCAACCCTCCAGGATACGGAGGCGGGTCGTTTTGCAACTTTTTTTTGGAGGCCGGATGA
GACTAGTAAGCGCGGAAAGCGGCCGACCGCGATGGCTCGCTGCCGTAGTCTGGAGAAGAATCGCCAGGGTTGCG
TTGCGGTGTGCCCCGGTTCGAGGCCGGCCGGATTCGCGGCTAACGAGGGCGTGGCTGCCCCGTCGTTTCCAAG
ACCCCATAGCCAGCCGACTTCTCCAGTTACGGAGCGAGCCCCTCTTTTGTTTTGTTTGTTTTTGCCAGATGCAT
CCCGTACTGCGGCAGATGCGCCCCCACCACCCTCCACCGCAACAACAGCCCCCTCCACAGCCGGCGCTTCTGCC
CCCGCCCCAGCAGCAACTTCCAGCCACGACCGCCGCGGCCGCCGTGAGCGGGGCTGGACAGAGTTATGATCACC
AGCTGGCCTTGGAAGAGGGCGAGGGGCTGGCGCGCCTGGGGGCGTCGTCGCCGGAGCGGCACCCGCGCGTGCAG
ATGAAAAGGGACGCTCGCGAGGCCTACGTGCCCAAGCAGAACCTGTTCAGAGACAGGAGCGGCGAGGAGCCCGA
GGAGATGCGCGCGGCCCGGTTCCACGCGGGGCGGGAGCTGCGGCGCGGCCTGGACCGAAAGAGGGTGCTGAGGG
ACGAGGATTTCGAGGCGGACGAGCTGACGGGGATCAGCCCCGCGCGCGCGCACGTGGCCGCGGCCAACCTGGTC
ACGGCGTACGAGCAGACCGTGAAGGAGGAGAGCAACTTCCAAAAATCCTTCAACAACCACGTGCGCACCCTGAT
CGCGCGCGAGGAGGTGACCCTGGGCCTGATGCACCTGTGGGACCTGCTGGAGGCCATCGTGCAGAACCCCACCA
GCAAGCCGCTGACGGCGCAGCTGTTCCTGGTGGTGCAGCATAGTCGGGACAACGAAGCGTTCAGGGAGGCGCTG
```

```
CTGAATATCACCGAGCCCGAGGGCCGCTGGCTCCTGGACCTGGTGAACATTCTGCAGAGCATCGTGGTGCAGGA
GCGCGGGCTGCCGCTGTCCGAGAAGCTGGCGGCCATCAACTTCTCGGTGCTGAGTTTGGGCAAGTACTACGCTA
GGAAGATCTACAAGACCCCGTACGTGCCCATAGACAAGGAGGTGAAGATCGACGGGTTTTACATGCGCATGACC
CTGAAAGTGCTGACCCTGAGCGACGATCTGGGGGTGTACCGCAACGACAGGATGCACCGTGCGGTGAGCGCCAG
CAGGCGGCGCGAGCTGAGCGACCAGGAGCTGATGCATAGTCTGCAGCGGGCCCTGACCGGGGCCGGGACCGAGG
GGGAGAGCTACTTTGACATGGGCGCGGACCTGCACTGGCAGCCCAGCCGCCGGGCCTTGGAGGCGGCGGCAGGA
CCCTACGTAGAAGAGGTGGACGATGAGGTGGACGAGGAGGGCGAGTACCTGGAAGACTGATGGCGCGACCGTAT
TTTTGCTAGATGCAACAACAACAGCCACCTCCTGATCCCGCGATGCGGGCGGCGCTGCAGAGCCAGCCGTCCGG
CATTAACTCCTCGGACGATTGGACCCAGGCCATGCAACGCATCATGGCGCTGACGACCCGCAACCCCGAAGCCT
TTAGACAGCAGCCCCAGGCCAACCGGCTCTCGGCCATCCTGGAGGCCGTGGTGCCCTCGCGCTCCAACCCCACG
CACGAGAAGGTCCTGGCCATCGTGAACGCGCTGGTGGAGAACAAGGCCATCCGCGGCGACGAGGCCGGCCTGGT
GTACAACGCGCTGCTGGAGCGCGTGGCCCGCTACAACAGCACCAACGTGCAGACCAACCTGGACCGCATGGTGA
CCGACGTGCGCGAGGCCGTGGCCCAGCGCGAGCGGTTCCACCGCGAGTCCAACCTGGGATCCATGGTGGCGCTG
AACGCCTTCCTCAGCACCCAGCCCGCCAACGTGCCCCGGGGCCAGGAGGACTACACCAACTTCATCAGCGCCCT
GCGCCTGATGGTGACCGAGGTGCCCCAGAGCGAGGTGTACCAGTCCGGGCCGGACTACTTCTTCCAGACCAGTC
GCCAGGGCTTGCAGACCGTGAACCTGAGCCAGGCTTTCAAGAACTTGCAGGGCCTGTGGGGCGTGCAGGCCCCG
GTCGGGGACCGCGCGACGGTGTCGAGCCTGCTGACGCCGAACTCGCGCCTGCTGCTGCTGCTGGTGGCCCCCTT
CACGGACAGCGGCAGCATCAACCGCAACTCGTACCTGGGCTACCTGATTAACCTGTACCGCGAGGCCATCGGCC
AGGCGCACGTGGACGAGCAGACCTACCAGGAGATCACCCACGTGAGCCGCGCCCTGGGCCAGGACGACCCGGGC
AACCTGGAAGCCACCCTGAACTTTTTGCTGACCAACCGGTCGCAGAAGATCCCGCCCCAGTACGCGCTCAGCAC
CGAGGAGGAGCGCATCCTGCGTTACGTGCAGCAGAGCGTGGGCCTGTTCCTGATGCAGGAGGGGGCCACCCCCA
GCGCCGCGCTCGACATGACCGCGCGCAACATGGAGCCCAGCATGTACGCCAGCAACCGCCCGTTCATCAATAAA
CTGATGGACTACTTGCATCGGGCGGCCGCCATGAACTCTGACTATTTCACCAACGCCATCCTGAATCCCCACTG
GCTCCCGCCGCCGGGGTTCTACACGGGCGAGTACGACATGCCCGACCCCAATGACGGGTTCCTGTGGGACGATG
TGGACAGCAGCGTGTTCTCCCCCCGACCGGGTGCTAACGAGCGCCCCTTGTGGAAGAAGGAAGGCAGCGACCGA
CGCCCGTCCTCGGCGCTGTCCGGCCGCGAGGGTGCTGCCGCGGCGGTGCCCGAGGCCGCCAGTCCTTTCCCGAG
CTTGCCCTTCTCGCTGAACAGTATCCGCAGCAGCGAGCTGGGCAGGATCACGCGCCCGCGCTTGCTGGGCGAAG
AGGAGTACTTGAATGACTCGCTGTTGAGACCCGAGCGGGAGAAGAACTTCCCCAATAACGGGATAGAAAGCCTG
GTGGACAAGATGAGCCGCTGGAAGACGTATGCGCAGGAGCACAGGGACGATCCCCGGGCGTCGCAGGGGGCCAC
GAGCCGGGGCAGCGCCGCCCGTAAACGCCGGTGGCACGACAGGCAGCGGGGACAGATGTGGGACGATGAGGACT
CCGCCGACGACAGCAGCGTGTTGGACTTGGGTGGGAGTGGTAACCCGTTCGCTCACCTGCGCCCCCGTATCGGG
CGCATGATGTAAGAGAAACCGAAAATAAATGATACTCACCAAGGCCATGGCGACCAGCGTGCGTTCGTTTCTTC
TCTGTTGTTGTTGTATCTAGTATGATGAGGCGTGCGTACCCGGAGGGTCCTCCTCCCTCGTACGAGAGCGTGAT
GCAGCAGGCGATGGCGGCGGCGGCGATGCAGCCCCCGCTGGAGGCTCCTTACGTGCCCCCGCGGTACCTGGCGC
CTACGGAGGGGCGGAACAGCATTCGTTACTCGGAGCTGGCACCCTTGTACGATACCACCCGGTTGTACCTGGTG
GACAACAAGTCGGCGGACATCGCCTCGCTGAACTACCAGAACGACCACAGCAACTTCCTGACCACCGTGGTGCA
GAACAATGACTTCACCCCCACGGAGGCCAGCACCCAGACCATCAACTTTGACGAGCGCTCGCGGTGGGGCGGCC
AGCTGAAAACCATCATGCACACCAACATGCCCAACGTGAACGAGTTCATGTACAGCAACAAGTTCAAGGCGCGG
GTGATGGTCTCCCGCAAGACCCCCAATGGGGTGACAGTGACAGAGGATTATGATGGTAGTCAGGATGAGCTGAA
GTATGAATGGGTGGAATTTGAGCTGCCCGAAGGCAACTTCTCGGTGACCATGACCATCGACCTGATGAACAACG
CCATCATCGACAATTACTTGGCGGTGGGGCGGCAGAACGGGGTGCTGGAGAGCGACATCGGCGTGAAGTTCGAC
ACTAGGAACTTCAGGCTGGGCTGGGACCCCGTGACCGAGCTGGTCATGCCCGGGGTGTACACCAACGAGGCTTT
CCATCCCGATATTGTCTTGCTGCCCGGCTGCGGGGTGGACTTCACCGAGAGCCGCCTCAGCAACCTGCTGGGCA
TTCGCAAGAGGCAGCCCTTCCAGGAAGGCTTCCAGATCATGTACGAGGATCTGGAGGGGGGCAACATCCCCGCG
CTCCTGGATGTCGACGCCTATGAGAAAAGCAAGGAGGATGCAGCAGCTGAAGCAACTGCAGCCGTAGCTACCGC
CTCTACCGAGGTCAGGGGCGATAATTTTGCAAGCGCCGCAGCAGTGGCAGCGGCCGAGGCGGCTGAAACCGAAA
GTAAGATAGTCATTCAGCCGGTGGAGAAGGATAGCAAGAACAGGAGCTACAACGTACTACCGGACAAGATAAAC
ACCGCCTACCGCAGCTGGTACCTAGCCTACAACTATGGCGACCCCGAGAAGGGCGTGCGCTCCTGGACGCTGCT
CACCCACCTCGGACGTCACCTGCGGCGTGGAGCAAGTCTACTGGTCGCTGCCCGACATGATGCAAGACCCGGTCA
CCTTCCGCTCCACGCGTCAAGTTAGCAACTACCCGGTGGTGGGCGCCGAGCTCCTGCCCGTCTACTCCAAGAGC
TTCTTCAACGAGCAGGCCGTCTACTCGCAGCAGCTGCGCGCCTTCACCTCGCTTACGCACGTCTTCAACCGCTT
CCCCGAGAACCAGATCCTCGTCCGCCCGCCCGCGCCCACCATTACCACCGTCAGTGAAAACGTTCCTGCTCTCA
CAGATCACGGGACCCTGCCGCTGCGCAGCAGTATCCGGGGAGTCCAGCGCGTGACCGTTACTGACGCCAGACGC
CGCACCTGCCCCTACGTCTACAAGGCCCTGGGCATAGTCGCGCCGCGCGTCCTCTCGAGCCGCACCTTCTAAAT
GTCCATTCTCATCTCGCCCAGTAATAACACCGGTTGGGGCCTGCGCGCGCCCAGCAAGATGTACGGAGGCGCTC
GCCAACGCTCCACGCAACACCCCGTGCGCGTGCGCGGGCACTTCCGCGCTCCCTGGGGCGCCCTCAAGGGCCGC
GTGCGGTCGCGCACCACCGTCGACGACGTGATCGACCAGGTGGTGGCCGACGCGCGCAACTACACCCCCGCCGC
CGCGCCCGTCTCCACCGTGGACGCCGTCATCGACAGCGTGGTGGCCGACGCGCGCCGGTACGCCCGCGCCAAGA
GCCGGCGGCGGCGCATCGCCCGGCGGCACCGGAGCACCCCCGCCATGCGCGCGGCGCGAGCCTTGCTGCGCAGG
GCCAGGCGCACGGGACGCAGGGCCATGCTCAGGGCGGCCAGACGCGCGGCTTCAGGCGCCAGCGCCGGCAGGAC
CCGGAGACGCGCGGCCACGGCGGCGGCAGCGGCCATCGCCAGCATGTCCCGCCCGCGGCGAGGGAACGTGTACT
GGGTGCGCGACGCCGCCACCGGTGTGCGCGTGCCCGTGCGCACCCGCCCCCCTCGCACTTGAAGATGTTCACTT
```

```
CGCGATGTTGATGTGTCCCAGCGGCGAGGAGGATGTCCAAGCGCAAATTCAAGGAAGAGATGCTCCAGGTCATC
GCGCCTGAGATCTACGGCCCTGCGGTGGTGAAGGAGGAAAGAAAGCCCCGCAAAATCAAGCGGGTCAAAAAGGA
CAAAAAGGAAGAAGAAAGTGATGTGGACGGATTGGTGGAGTTTGTGCGCGAGTTCGCCCCCCGGCGGCGCGTGC
AGTGGCGCGGGCGGAAGGTGCAACCGGTGCTGAGACCCGGCACCACCGTGGTCTTCACGCCCGGCGAGCGCTCC
GGCACCGCTTCCAAGCGCTCCTACGACGAGGTGTACGGGGATGATGATATTCTGGAGCAGGCGGCCGAGCGCCT
GGGCGAGTTTGCTTACGGCAAGCGCAGCCGTTCCGCACCGAAGGAAGAGGCGGTGTCCATCCCGCTGGACCACG
GCAACCCCACGCCGAGCCTCAAGCCCGTGACCTTGCAGCAGGTGCTGCCGACCGCGGCGCCGCGCCGGGGGTTC
AAGCGCGAGGGCGAGGATCTGTACCCCACCATGCAGCTGATGGTGCCCAAGCGCCAGAAGCTGGAAGACGTGCT
GGAGACCATGAAGGTGGACCCGGACGTGCAGCCCGAGGTCAAGGTGCGGCCCATCAAGCAGGTGGCCCCGGGCC
TGGGCGTGCAGACCGTGGACATCAAGATTCCCACGGAGCCCATGGAAACGCAGACCGAGCCCATGATCAAGCCC
AGCACCAGCACCATGGAGGTGCAGACGGATCCCTGGATGCCATCGGCTCCTAGTCGAAGACCCCGGCGCAAGTA
CGGCGCGGCCAGCCTGCTGATGCCCAACTACGCGCTGCATCCTTCCATCATCCCCACGCCGGGCTACCGCGGCA
CGCGCTTCTACCGCGGTCATACCAGCAGCCGCCGCCGCAAGACCACCACTCGCCGCCGCCGTCGCCGCACCGCC
GCTGCAACCACCCCTGCCGCCCTGGTGCGGAGAGTGTACCGCCGCGGCCGCGCACCTCTGACCCTGCCGCGCGC
GCGCTACCACCCGAGCATCGCCATTTAAACTTTCGCCtGCTTTGCAGATCAATGGCCCTCACATGCCGCCTTCG
CGTTCCCATTACGGGCTACCGAGGAAGAAAACCGCGCCGTAGAAGGCTGGCGGGGAACGGGATGCGTCGCCACC
ACCACCGGCGGCGGCGCGCCATCAGCAAGCGGTTGGGGGGGAGGCTTCCTGCCCGCGCTGATCCCCATCATCGCC
GCGGCGATCGGGGCGATCCCCGGCATTGCTTCCGTGGCGGTGCAGGCCTCTCAGCGCCACTGAGACACACTTGG
AAACATCTTGTAATAAACCaATGGACTCTGACGCTCCTGGTCCTGTGATGTGTTTTCGTAGACAGATGGAAGAC
ATCAATTTTTCGTCCCTGGCTCCGCGACACGGCACGCGGCCGTTCATGGGCACCTGGAGCGACATCGGCACCAG
CCAACTGAACGGGGGCGCCTTCAATTGGAGCAGTCTCTGGAGCGGGCTTAAGAATTTCGGGTCCACGCTTAAAA
CCTATGGCAGCAAGGCGTGGAACAGCACCACAGGGCAGGCGCTGAGGGATAAGCTGAAAGAGCAGAACTTCCAG
CAGAAGGTGGTCGATGGGCTCGCCTCGGGCATCAACGGGGTGGTGGACCTGGCCAACCAGGCCGTGCAGCGGCA
GATCAACAGCCGCCTGGACCCGGTGCCGCCCGCCGGCTCCGTGGAGATGCCGCAGGTGGAGGAGGAGCTGCCTC
CCCTGGACAAGCGGGGCGAGAAGCGACCCCGCCCCGATGCGGAGGAGACGCTGCTGACGCACACGGACGAGCCG
CCCCCGTACGAGGAGGCGGTGAAACTGGGTCTGCCCACCACGCGGCCCATCGCGCCCCTGGCCACCGGGGTGCT
GAAACCCGAAAAGCCCGCGACCCTGGACTTGCCTCCTCCCCAGCCTTCCCGCCCCTCTACAGTGGCTAAGCCCC
TGCCGCCGGTGGCCGTGGCCCGCGCGCGACCCGGGGGCACCGCCCGCCCTCATGCGAACTGGCAGAGCACTCTG
AACAGCATCGTGGGTCTGGGAGTGCAGAGTGTGAAGCGCCGCCGCTGCTATTAAACCTACCGTAGCGCTTAACT
TGCTTGTCTGTGTGTGTATGTATTATGTCGCCGCCGCCGCTGTCCACCAGAAGGAGGAGTGAAGAGGCGCGTCG
CCGAGTTGCAAGATGGCCACCCCATCGATGCTGCCCCAGTGGGCGTACATGCACATCGCCGGACAGGACGCTTC
GGAGTACCTGAGTCCGGGTCTGGTGCAGTTTGCCCGCGCCACAGACACCTACTTCAGTCTGGGGAACAAGTTTA
GGAACCCCACGGTGGCGCCCACGCACGATGTGACCACCGACCGCAGCCAGCGGCTGACGCTGCGCTTCGTGCCC
GTGGACCGCGAGGACAACACCTACTCGTACAAAGTGCGCTACACGCTGGCCGTGGGCGACAACCGCGTGCTGGA
CATGGCCAGCACCTACTTTGACATCCGCGGCGTGCTGGATCGGGGCCCTAGCTTCAAACCCTACTCCGGCACCG
CCTACAACAGTCTGGCCCCCAAGGGAGCACCCAACACTTGTCAGTGGACATATAAAGCCGATGGTGAAACTGCC
ACAGAAAAAACCTATACATATGGAAATGCACCCGTGCAGGGCATTAACATCACAAAGATGGTATTCAACTTGG
AACTGACACCGATGATCAGCCAATCTACGCAGATAAAACCTATCAGCCTGAACCTCAAGTGGGTGATGCTGAAT
GGCATGACATCACTGGTACTGATGAAAAGTATGGAGGCAGAGCTCTTAAGCCTGATACCAAAATGAAGCCTTGT
TATGGTTCTTTTGCCAAGCCTACTAATAAAGAAGGAGGTCAGGCAAATGTGAAAACAGGAACAGGCACTACTAA
AGAATATGACATAGACATGGCTTTCTTTGACAACAGAAGTGCGGCTGCTGCTGGCCTAGCTCCAGAAATTGTTT
TGTATACTGAAAATGTGGATTTGGAAACTCCAGATACCCATATTGTATACAAAGCAGGCACAGATGACAGCAGC
TCTTCTATTAATTTGGGTCAGCAAGCCATGCCCAACAGACCTAACTACATTGGTTTCAGAGACAACTTTATCGG
GCTCATGTACTACAACAGCACTGGCAATATGGGGGTGCTGGCCGGTCAGGCTTCTCAGCTGAATGCTGTGGTTG
ACTTGCAAGACAGAAACACCGAGCTGTCCTACCAGCTCTTGCTTGACTCTCTGGGTGACAGAACCCGGTATTTC
AGTATGTGGAATCAGGCGGTGGACAGCTATGATCCTGATGTGCGCATTATTGAAAATCATGGTGTGGAGGATGA
ACTTCCCAACTATTGTTTCCCTCTGGATGCTGTTGGCAGAACAGATACTTATCAGGGAATTAAGGCTAATGGAA
CTGATCAAACCACATGGACCAAAGATGACAGTGTCAATGATGCTAATGAGATAGGCAAGGGTAATCCATTCGCC
ATGGAAATCAACATCCAAGCCAACCTGTGGAGGAACTTCCTCTACGCCAACGTGGCCCTGTACCTGCCCGACTC
TTACAAGTACACGCCGGCCAATGTTACCCTGCCCACCAACACCAACACCTACGATTACATGAACGGCCGGGTGG
TGGCGCCCTCGCTGGTGGACTCCTACATCAACATCGGGGCGCGCTGGTCGCTGGATCCCATGGACAACGTGAAC
CCCTTCAACCACCACCGCAATGCGGGGCTGCGCTACCGCTCCATGCTCCTGGGCAACGGGCGCTACGTGCCCTT
CCACATCCAGGTGCCCCAGAAATTTTTCGCCATCAAGAGCCTCCTGCTCCTGCCCGGGTCCTACACCTACGAGT
GGAACTTCCGCAAGGACGTCAACATGATCCTGCAGAGCTCCCTCGGCAACGACCTGCGCACGGACGGGGCCTCC
ATCTCCTTCACCAGCATCAACCTCTACGCCACCTTCTTCCCCATGGCGCACAACACGGCCTCCACGCTCGAGGC
CATGCTGCGCAACGACACCAACGACCAGTCCTTCAACGACTACCTCTCGGCGGCCAACATGCTCTACCCCATCC
CGGCCAACGCCACCAACGTGCCCATCTCCATCCCCTCGCGCAACTGGGCCGCCTTCCGCGGCTGGTCCTTCACG
CGTCTCAAGACCAAGGAGACGCCCTCGCTGGGCTCCGGGTTCGACCCCTACTTCGTCTACTCGGGCTCCATCCC
CTACCTCGACGGCACCTTCTACCTCAACCACACCTTCAAGAAGGTCTCCATCACCTTCGACTCCTCCGTCAGCT
GGCCCGGCAACGACCGGCTCCTGACGCCCAACGAGTTCGAAATCAAGCGCACCGTCGACGGCGAGGGCTACAAC
GTGGCCCAGTGCAACATGACCAAGGACTGGTTCCTGGTCCAGATGCTGGCCCACTACAACATCGGCTACCAGGG
CTTCTACGTGCCCGAGGGCTACAAGGACCGCATGTACTCCTTCTTCCGCAACTTCCAGCCCATGAGCCGCCAGG
```

```
TGGTGGACGAGGTCAACTACAAGGACTACCAGGCCGTCACCCTGGCCTACCAGCACAACAACTCGGGCTTCGTC
GGCTACCTCGCGCCCACCATGCGCCAGGGCCAGCCCTACCCCGCCAACTACCCCTACCCGCTCATCGGCAAGAG
CGCCGTCACCAGCGTCACCCAGAAAAAGTTCCTCTGCGACAGGGTCATGTGGCGCATCCCCTTCTCCAGCAACT
TCATGTCCATGGGCGCGCTCACCGACCTCGGCCAGAACATGCTCTATGCCAACTCCGCCCACGCGCTAGACATG
AATTTCGAAGTCGACCCCATGGATGAGTCCACCCTTCTCTATGTTGTCTTCGAAGTCTTCGACGTCGTCCGAGT
GCACCAGCCCCACCGCGGCGTCATCGAGGCCGTCTACCTGCGCACCCCCTTCTCGGCCGGTAACGCCACCACCT
AAGCTCTTGCTTCTTGCAAGCCATGGCCGCGGGCTCCGGCGAGCAGGAGCTCAGGGCCATCATCCGCGACCTGG
GCTGCGGGCCCTACTTCCTGGGCACCTTCGATAAGCGCTTCCCGGGATTCATGGCCCCGCACAAGCTGGCCTGC
GCCATCGTCAACACGGCCGGCCGCGAGACCGGGGGCGAGCACTGGCTGGCCTTCGCCTGGAACCCGCGCTCGAA
CACCTGCTACCTCTTCGACCCCTTCGGGTTCTCGGACGAGCGCCTCAAGCAGATCTACCAGTTCGAGTACGAGG
GCCTGCTGCGCCGCAGCGCCCTGGCCACCGAGGACCGCTGCGTCACCCTGGAAAAGTCCACCCAGACCGTGCAG
GGTCCGCGCTCGGCCGCCTGCGGGCTCTTCTGCTGCATGTTCCTGCACGCCTTCGTGCACTGGCCCGACCGCCC
CATGGACAAGAACCCCACCATGAACTTGCTGACGGGGGTGCCCAACGGCATGCTCCAGTCGCCCCAGGTGGAAC
CCACCCTGCGCCGCAACCAGGAGGCGCTCTACCGCTTCCTCAACTCCCACTCCGCCTACTTTCGCTCCCACCGC
GCGCGCATCGAGAAGGCCACCGCCTTCGACCGCATGAATCAAGACATGTAAACCGTGTGTGTATGTTAAATGTC
TTTAATAAACAGCACTTTCATGTTACACATGCATCTGAGATGATTTATTTAGAAATCGAAAGGGTTCTGCCGGG
TCTCGGCATGGCCCGCGGGCAGGGACACGTTGCGGAACTGGTACTTGGCCAGCCACTTGAACTCGGGGATCAGC
AGTTTGGGCAGCGGGGTGTCGGGGAAGGAGTCGGTCCACAGCTTCCGCGTCAGTTGCAGGGCGCCCAGCAGGTC
GGGCGCGGAGATCTTGAAATCGCAGTTGGGACCCGCGTTCTGCGCGCGGGAGTTGCGGTACACGGGGTTGCAGC
ACTGGAACACCATCAGGGCCGGGTGCTTCACGCTCGCCAGCACCGTCGCGTCGGTGATGCTCTCCACGTCGAGG
TCCTCGGCGTTGGCCATCCCGAAGGGGGTCATCTTGCAGGTCTGCCTTCCCATGGTGGGCACGCACCCGGGCTT
GTGGTTGCAATCGCAGTGCAGGGGGATCAGCATCATCTGGGCCTGGTCGGCGTTCATCCCCGGGTACATGGCCT
TCATGAAAGCCTCCAATTGCCTGAACGCCTGCTGGGCCTTGGCTCCCTCGGTGAAGAAGACCCCGCAGGACTTG
CTAGAGAACTGGTTGGTGGCGCACCCGGCGTCGTGCACGCAGCAGCGCGCGTCGTTGTTGGCCAGCTGCACCAC
GCTGCGCCCCAGCGGTTCTGGGTGATCTTGGCCCGGTCGGGGTTCTCCTTCAGCGCGCGCTGCCCGTTCTCGC
TCGCCACATCCATCTCGATCATGTGCTCCTTCTGGATCATGGTGGTCCCGTGCAGGCACCGCAGCTTGCCCTCG
GCCTCGGTGCACCCGTGCAGCCACAGCGCGCACCCGGTGCACTCCCAGTTCTTGTGGGCGATCTGGGAATGCGC
GTGCACGAAGCCCTGCAGGAAGCGGCCCATCATGGTGGTCAGGGTCTTGTTGCTAGTGAAGGTCAGCGGAATGC
CGCGGTGCTCCTCGTTGATGTACAGGTGGCAGATGCGGCGGTACACCTCGCCCTGCTCGGGCATCAGCTGGAAG
TTGGCTTTCAGGTCGGTCTCCACGCGGTAGCGGTCCATCAGCATAGTCATGATTTCATACCCTTCTCCCAGGC
CGAGACGATGGGCAGGCTCATAGGGTTCTTCACCATCATCTTAGCGCTAGCAGCCGCGGCCAGGGGGTCGCTCT
CGTCCAGGGTCTCAAAGCTCCGCTTGCCGTCCTTCTCGGTGATCCGCACCGGGGGGGTAGCTGAAGCCCACGGCC
GCCAGCTCCTCCTCGGCCTGTCTTTCGTCCTCGCTGTCCTGGCTGACGTCCTGCAGGACCACATGCTTGGTCTT
GCGGGGTTTCTTCTTGGGCGGCAGCGGCGGCGGAGATGTTGGAGATGGCGAGGGGGAGCGCGAGTTCTCGCTCA
CCACTACTATCTCTTCCTCTTCTTGGTCCGAGGCCACGCGGCGGTAGGTATGTCTCTTCGGGGGGCAGAGGCGGA
GGCGACGGGCTCTCGCCGCCGCGACTTGGCGGATGGCTGGCAGAGCCCCTTCCGCGTTCGGGGGGTGCGCTCCCG
GCGGCGCTCTGACTGACTTCCTCCGCGGCCGGCCATTGTGTTCTCCTAGGGAGGAACAACAAGCATGGAGACTC
AGCCATCGCCAACCTCGCCATCTGCCCCCACCGCCGACGAGAAGCAGCAGCAGCAGAATGAAAGCTTAACCGCC
CCGCCGCCCAGCCCCGCCACCTCCGACGCGGCCGTCCCAGACATGCAAGAGATGGAGGAATCCATCGAGATTGA
CCTGGGCTATGTGACGCCCGCGGAGCACGAGGAGGAGCTGGCAGTGCGCTTTTCACAAGAAGAGATACACCAAG
AACAGCCAGAGCAGGAAGCAGAGAATGAGCAGAGTCAGGCTGGGCTCGAGCATGACGGCGACTACCTCCACCTG
AGCGGGGGGGAGGACGCGCTCATCAAGCATCTGGCCCGGCAGGCCACCATCGTCAAGGATGCGCTGCTCGACCG
CACCGAGGTGCCCCTCAGCGTGGAGGAGCTCAGCCGCGCCTACGAGTTGAACCTCTTCTCGCCGCGCGTGCCCC
CCAAGCGCCAGCCCAATGGCACCTGCGAGCCCAACCCGCGCCTCAACTTCTACCCGGTCTTCGCGGTGCCCGAG
GCCCTGGCCACCTACCACATCTTTTTTCAAGAACCAAAAGATCCCCGTCTCCTGCCGCGCCAACCGCACCCGCGC
CGACGCCCTTTTCAACCTGGGTCCCGGCGCCCGCCTACCTGATATCGCCTCCTTGGAAGAGGTTCCCAAGATCT
TCGAGGGTCTGGGCAGCGACGAGACTCGGGCCGCGAACGCTCTGCAAGGAGAAGGAGGAGAGCATGAGCACCAC
AGCGCCCTGGTCGAGTTGGAAGGCGACAACGCGCGGCTGGCGGTGCTCAAACGCACGGTCGAGCTGACCCATTT
CGCCTACCCGGCTCTGAACCTGCCCCCCAAAGTCATGAGCGCGGTCATGGACCAGGTGCTCATCAAGCGCGCGT
CGCCCATCTCCGAGGACGAGGGCATGCAAGACTCCGAGGAGGGCAAGCCCGTGGTCAGCGACGAGCAGCTGGCC
CGGTGGCTGGGTCCTAATGCTAGTCCCCAGAGTTTGGAAGAGCGGCGCAAACTCATGATGGCCGTGGTCCTGGT
GACCGTGGAGCTGGAGTGCCTGCGCCGCTTCTTCGCCGACGCGGAGACCCTGCGCAAGGTCGAGGAGAACCTGC
ACTACCTCTTCAGGCACGGGTTCGTGCGCCAGGCCTGCAAGATCTCCAACGTGGAGCTGACCAACCTGGTCTCC
TACATGGGCATCTTGCACGAGAACCGCCTGGGGCAGAACGTGCTGCACACCACCCTGCGCGGGGAGGCCCGGCG
CGACTACATCCGCGACTGCGTCTACCTCTACCTCTGCCACACCTGGCAGACGGGCATGGGCGTGTGGCAGCAGT
GTCTGGAGGAGCAGAACCTGAAAGAGCTCTGCAAGCTCCTGCAGAAGAACCTCAAGGGTCTGTGGACCGGGTTC
GACGAGCGCACCACCGCCTCGGACCTGGCCGACCTCATTTTCCCCGAGCGCCTCAGGCTGACGCTGCGCAACGG
CCTGCCCGACTTTATGAGCCAAAGCATGTTGCAAAACTTTCGCTCTTTCATCCTCGAACGCTCCGGAATCCTGC
CCGCCACCTGCTCCGCGCTGCCCTCGGACTTCGTGCCGCTGACCTTCCGCGAGTGCCCCCCGCCGCTGTGGAGC
CACTGCTACCTGCTGCGCCTGGCCAACTACCTGGCCTACCACTCGGACGTGATCGAGGACGTCAGCGGCGAGGG
CCTGCTCGAGTGCCACTGCCGCTGCAACCTCTGCACGCCGCACCGCTCCCTGGCCTGCAACCCCCAGCTGCTGA
GCGAGACCCAGATCATCGGCACCTTCGAGTTGCAAGGGCCCAGCGAAGGCGAGGGTTCAGCCGCCAAGGGGGGT
```

```
CTGAAACTCACCCCGGGGCTGTGGACCTCGGCCTACTTGCGCAAGTTCGTGCCCGAGGACTACCATCCCTTCGA
GATCAGGTTCTACGAGGACCAATCCCATCCGCCCAAGGCCGAGCTGTCGGCCTGCGTCATCACCCAGGGGGCGA
TCCTGGCCCAATTGCAAGCCATCCAGAAATCCCGCCAAGAATTCTTGCTGAAAAAGGGCCGCGGGGTCTACCTC
GACCCCCAGACCGGTGAGGAGCTCAACCCCGGCTTCCCCCAGGATGCCCCGAGGAAACAAGAAGCTGAAAGTGG
AGCTGCCGCCCGTGGAGGATTTGGAGGAAGACTGGGAGAACAGCAGTCAGGCAGAGGAGGAGGAGATGGAGGAA
GACTGGGACAGCACTCAGGCAGAGGAGGACAGCCTGCAAGACAGTCTGGAGGAAGACGAGGAGGAGGCAGAGGA
GGAGGTGGAAGAAGCAGCCGCCGCCAGACCGTCGTCCTCGGCGGGGGAGAAAGCAAGCAGCACGGATACCATCT
CCGCTCCGGGTCGGGGTCCCGCTCGACCACACAGTAGATGGGACGAGACCGGACGATTCCCGAACCCCACCACC
CAGACCGGTAAGAAGGAGCGGCAGGGATACAAGTCCTGGCGGGGGGCACAAAAACGCCATCGTCTCCTGCTTGCA
GGCCTGCGGGGGCAACATCTCCTTCACCCGGCGCTACCTGCTCTTCCACCGCGGGGTGAACTTTCCCCGCAACA
TCTTGCATTACTACCGTCACCTCCACAGCCCCTACTACTTCCAAGAAGAGGCAGCAGCAGCAGAAAAAGACCAG
CAGAAAACCAGCAGCTAGAAAATCCACAGCGGCGGCAGCAGGTGGACTGAGGATCGCGGCGAACGAGCCGGCGC
AAACCCGGGAGCTGAGGAACCGGATCTTTCCCACCCTCTATGCCATCTTCCAGCAGAGTCGGGGGCAGGAGCAG
GAACTGAAAGTCAAGAACCGTTCTCTGCGCTCGCTCACCCGCAGTTGTCTGTATCACAAGAGCGAAGACCAACT
TCAGCGCACTCTCGAGGACGCCGAGGCTCTCTTCAACAAGTACTGCGCGCTCACTCTTAAAGAGTAGCCCGCGC
CCGCCCAGTCGCAGAAAAAGGCGGGAATTACGTCACCTGTGCCCTTCGCCCTAGCCGCCTCCACCCATCATCAT
GAGCAAAGAGATTCCCACGCCTTACATGTGGAGCTACCAGCCCCAGATGGGCCTGGCCGCCGGTGCCGCCCAGG
ACTACTCCACCCGCATGAATTGGCTCAGCGCCGGGCCCGCGATGATCTCACGGGTGAATGACATCCGCGCCCAC
CGAAACCAGATACTCCTAGAACAGTCAGCGCTCACCGCCACGCCCCGCAATCACCTCAATCCGCGTAATTGGCC
CGCCGCCCTGGTGTACCAGGAAATTCCCCAGCCCACGACCGTACTACTTCCGCGAGACGCCCAGGCCGAAGTCC
AGCTGACTAACTCAGGTGTCCAGCTGGCGGGCGGCGCCACCCTGTGTCGTCACCGCCCCGCTCAGGGTATAAAG
CGGCTGGTGATCCGGGGCAGAGGCACACAGCTCAACGACGAGGTGGTGAGCTCTTCGCTGGGTCTGCGACCTGA
CGGAGTCTTCCAACTCGCCGGATCGGGGAGATCTTCCTTCACGCCTCGTCAGGCCGTCCTGACTTTGGAGAGTT
CGTCCTCGCAGCCCCGCTCGGGTGGCATCGGCACTCTCCAGTTCGTGGAGGAGTTCACTCCCTCGGTCTACTTC
AACCCCTTCTCCGGCTCCCCCGGCCACTACCCGGACGAGTTCATCCCGAACTTCGACGCCATCAGCGAGTCGGT
GGACGGCTACGATTGAAACTAATCACCCCCTTATCCAGTGAAATAAAGATCATATTGATGATGATTTTACAGAA
ATAAAAAATAATCATTTGATTTGAAATAAAGATACAATCATATTGATGATTTGAGTTTAACAAAAAAATAAAGA
ATCACTTACTTGAAATCTGATACCAGGTCTCTGTCCATGTTTTCTGCCAACACCACTTCACTCCCCTCTTCCCA
GCTCTGGTACTGCAGGCCCCGGCGGGCTGCAAACTTCCTCCACACGCTGAAGGGGATGTCAAATTCCTCCTGTC
CCTCAATCTTCATTTTATCTTCTATCAGATGCCAAAAAGCGCGTCCGGGTGGATGATGACTTCGACCCCGTCT
ACCCCTACGATGCAGACAACGCACCGACCGTGCCCTTCATCAACCCCCCCTTCGTCTCTTCAGATGGATTCCAA
GAGAAGCCCCTGGGGGTGTTGTCCCTGCGACTGGCCGACCCCGTCACCACCAAGAACGGGGAAATCACCCTCAA
GCTGGGAGAGGGGTGGACCTCGATTCCTCGGGAAAACTCATCTCCAACACGGCCACCAAGGCCGCCGCCCCTC
TCAGTTTTTCCAACAACACCATTTCCCTTAACATGGATCACCCCTTTTACACTAAAGATGGAAAATTATCCTTA
CAAGTTTCTCCACCATTAAATATACTGAGAACAAGCATTCTAAACACACTAGCTTTAGGTTTTGGATCAGGTTT
AGGACTCCGTGGCTCTGCCTTGGCAGTACAGTTAGTCTCTCCACTTACATTTGATACTGATGGAAACATAAAGC
TTACCTTAGACAGAGGTTTGCATGTTACAACAGGAGATGCAATTGAAAGCAACATAAGCTGGGCTAAAGGTTTA
AAATTTGAAGATGGAGCCATAGCAACCAACATTGGAAATGGGTTAGAGTTTGGAAGCAGTAGTACAGAAACAGG
TGTTGATGATGCTTACCCAATCCAAGTTAAACTTGGATCTGGCCTTAGCTTTGACAGTACAGGAGCCATAATGG
CTGGTAACAAAGAAGACGATAAACTCACTTTGTGGACAACACCTGATCCATCACCAAACTGTCAAATACTCGCA
GAAAATGATGCAAAACTAACACTTTGCTTGACTAAATGTGGTAGTCAAATACTGGCCACTGTGTCAGTCTTAGT
TGTAGGAAGTGGAAACCTAAACCCCATTACTGGCACCGTAAGCAGTGCTCAGGTGTTTCTACGTTTTGATGCAA
ACGGTGTTCTTTTAACAGAACATTCTACACTAAAAAAATACTGGGGGTATAGGCAGGGAGATAGCATAGATGGC
ACTCCATATACCAATGCTGTAGGATTCATGCCCAATTTAAAAGCTTATCCAAAGTCACAAAGTTCTACTACTAA
AAATAATATAGTAGGGCAAGTATACATGAATGGAGATGTTTCAAAACCTATGCTTCTCACTATAACCCTCAATG
GTACTGATGACAGCAACAGTACATATTCAATGTCATTTTCATACACCTGGACTAATGGAAGCTATGTTGGAGCA
ACATTTGGGGCTAACTCTTATACCTTCTCATACATCGCCCAAGAATGAACACTGTATCCCACCCTGCATGCCAA
CCCTTCCCACCCCACTCTGTGGAACAAACTCTGAAACACAAAATAAAATAAAGTTCAAGTGTTTTATTGATTCA
ACAGTTTTACAGGATTCGAGCAGTTATTTTTCCTCCACCCTCCCAGGACATGGAATACACCACCCTCTCCCCCC
GCACAGCCTTGAACATCTGAATGCCATTGGTGATGGACATGCTTTTGGTCTCCACGTTCCACACAGTTTCAGAG
CGAGCCAGTCTCGGGTCGGTCAGGGAGATGAAACCCTCCGGGCACTCCCGCATCTGCACCTCACAGCTCAACAG
CTGAGGATTGTCCTCGGTGGTCGGGATCACGGTTATCTGGAAGAAGCAGAAGAGCGGCGGTGGGAATCATAGTC
CGCGAACGGGATCGGCCGGTGGTGTCGCATCAGGCCCCGCAGCAGTCGCTGCCGCCGCCGCTCCGTCAAGCTGC
TGCTCAGGGGGTCCGGGTCCAGGGACTCCCTCAGCATGATGCCCACGGCCCTCAGCATCAGTCGTCTGGTGCGG
CGGGCGCAGCAGCGCATGCGGATCTCGCTCAGGTCGCTGCAGTACGTGCAACACAGAACCACCAGGTTGTTCAA
CAGTCCATAGTTCAACACGCTCCAGCCGAAACTCATCGCGGGAAGGATGCTACCCACGTGGCCGTCGTACCAGA
TCCTCAGGTAAATCAAGTGGTGCCCCCTCCAGAACACGCTGCCCACGTACATGATCTCCTTGGGCATGTGGCGG
TTCACCACCTCCCGGTACCACATCACCCTCTGGTTGAACATGCAGCCCCGGATGATCCTGCGGAACCACAGGGC
CAGCACCGCCCCGCCCGCCATGCAGCGAAGAGACCCCGGGTCCCGGCAATGGCAATGGAGGACCCACCGCTCGT
ACCCGTGGATCATCTGGGAGCTGAACAAGTCTATGTTGGCACAGCACAGGCATATGCTCATGCATCTCTTCAGC
ACTCTCAACTCCTCGGGGGTCAAAACCATATCCCAGGGCACGGGGAACTCTTGCAGGACAGCGAACCCGCAGA
ACAGGGCAATCCTCGCACAGAACTTACATTGTGCATGGACAGGGTATCGCAATCAGGCAGCACCGGGTGATCCT
```

(continued)

```
CCACCAGAGAAGCGCGGGTCTCGGTCTCCTCACAGCGTGGTAAGGGGGCCGGCCGATACGGGTGATGGCGGGAC
GCGGCTGATCGTGTTCGCGACCGTGTCATGATGCAGTTGCTTTCGGACATTTTCGTACTTGCTGTAGCAGAACC
TGGTCCGGGCGCTGCACACCGATCGCCGGCGGCGGTCTCGGCGCTTGGAACGCTCGGTGTTGAAATTGTAAAAC
AGCCACTCTCTCAGACCGTGCAGCAGATCTAGGGCCTCAGGAGTGATGAAGATCCCATCATGCCTGATGGCTCT
GATCACATCGACCACCGTGGAATGGGCCAGACCCAGCCAGATGATGCAATTTTGTTGGGTTTCGGTGACGGCGG
GGGAGGGAAGAACAGGAAGAACCATGATTAACTTTTAATCCAAACGGTCTCGGAGTACTTCAAAATGAAGATCG
CGGAGATGGCACCTCTCGCCCCCGCTGTGTTGGTGGAAAATAACAGCCAGGTCAAAGGTGATACGGTTCTCGAG
ATGTTCCACGGTGGCTTCCAGCAAAGCCTCCACGCGCACATCCAGAAACAAGACAATAGCGAAAGCGGGAGGGT
TCTCTAATTCCTCAATCATCATGTTACACTCCTGCACCATCCCCAGATAATTTTCATTTTTCCAGCCTTGAATG
ATTCGAACTAGTTCcTGAGGTAAATCCAAGCCAGCCATGATAAAGAGCTCGCGCAGAGCGCCCTCCACCGGCAT
TCTTAAGCACACCCTCATAATTCCAAGATATTCTGCTCCTGGTTCACCTGCAGCAGATTGACAAGCGGAATATC
AAAATCTCTGCCGCGATCCCTGAGCTCCTCCCTCAGCAATAACTGTAAGTACTCTTTCATATCCTCTCCGAAAT
TTTTAGCCATAGGACCACCAGGAATAAGATTAGGGCAAGCCACAGTACAGATAAACCGAAGTCCTCCCCAGTGA
GCATTGCCAAATGCAAGACTGCTATAAGCATGCTGGCTAGACCCGGTGATATCTTCCAGATAACTGGACAGAAA
ATCGCCCAGGCAATTTTTAAGAAAATCAACAAAAGAAAAATCCTCCAGGTGGACGTTTAGAGCCTCGGGAACAA
CGATGAAGTAAATGCAAGCGGTGCGTTCCAGCATGGTTAGTTAGCTGATCTGTAGAAAAAACAAAAATGAACAT
TAAACCATGCTAGCCTGGCGAACAGGTGGGTAAATCGTTCTCTCCAGCACCAGGCAGGCCACGGGGTCTCCGGC
GCGACCCTCGTAAAAATTGTCGCTATGATTGAAAACCATCACAGAGAGACGTTCCCGGTGGCCGGCGTGAATGA
TTCGACAAGATGAATACACCCCCGGAACATTGGCGTCCGCGAGTGAAAAAAAGCGCCCGAGGAAGCAATAAGGC
ACTACAATGCTCAGTCTCAAGTCCAGCAAAGCGATGCCATGCGGATGAAGCACAAAATTCTCAGGTGCGTACAA
AATGTAATTACTCCCCTCCTGCACAGGCAGCAAAGCCCCCGATCCCTCCAGGTACACATACAAAGCCTCAGCGT
CCATAGCTTACCGAGCAGCAGCACACAACAGGCGCAAGAGTCAGAGAAAGGCTGAGCTCTAACCTGTCCACCCG
CTCTCTGCTCAATATATAGCCCAGATCTACACTGACGTAAAGGCCAAAGTCTAAAAATACCCGCCAAATAATCA
CACACGCCCAGCACACGCCCAGAAACCGGTGACACACTCAAAAAAAATACGCGCACTTCCTCAAACGCCCAAAAC
TGCCGTCATTTCCGGGTTCCCACGCTACGTCATCAAAACACGACTTTCAAATTCCGTCGACCGTTAAAAACGTC
ACCCGCCCCGCCCCTAACGGTCGCCCGTCTCTCAGCCAATCAGCGCCCCGCATCCCCAAATTCAAACACCTCAT
TTGCATATTAACGCGCACAAAAAGTTTGAGGTATATTATTGATGATGG
```

### XV.B. ChAd Neoantigen Cassette Delivery Testing

### XV.B.1 ChAd Vector Evaluation Methods and Materials

### Transfection of HEK293A cells using lipofectamine

DNA for the ChAdV68 constructs (ChAdV68.4WTnt.GFP, ChAdV68.5WTnt.GFP, ChAdV68.4WTnt.MAG25mer and ChAdV68.5WTnt.MAG25mer) was prepared and transfected into HEK293A cells using the following protocol.

10 ug of plasmid DNA was digested with PacI to liberate the viral genome. DNA was then purified using GeneJet DNA cleanup Micro columns (Thermo Fisher) according to manufacturer's instructions for long DNA fragments, and eluted in 20 ul of pre-heated water; columns were left at 37 degrees for 0.5-1 hours before the elution step.

HEK293A cells were introduced into 6-well plates at a cell density of $10^6$ cells/well 14-18 hours prior to transfection. Cells were overlaid with 1 ml of fresh medium (DMEM-10% hiFBS with pen/strep and glutamate) per well. 1-2 ug of purified DNA was used per well in a transfection with twice the ul volume (2-4 ul) of Lipofectamine2000, according to the manufacturer's protocol. 0.5 ml of OPTI-MEM medium containing the transfection mix was added to the 1 ml of normal growth medium in each well, and left on cells overnight

Transfected cell cultures were incubated at 37°C for at least 5-7 days. If viral plaques were not visible by day 7 post-transfection, cells were split 1:4 or 1:6, and incubated at 37°C to monitor for plaque development. Alternatively, transfected cells were harvested and subjected to 3 cycles of freezing and thawing and the cell lysates were used to infect HEK293A cells and the cells were incubated until virus plaques were observed.

### Transfection of ChAdV68 vectors into HEK293A cells using calcium phosphate and generation of the tertiary viral stock

DNA for the ChAdV68 constructs (ChAdV68.4WTnt.GFP, ChAdV68.5WTnt.GFP, ChAdV68.4WTnt.MAG25mer, ChAdV68.5WTnt.MAG25mer) was prepared and transfected into HEK293A cells using the following protocol.

HEK293A cells were seeded one day prior to the transfection at $10^6$ cells/ well of a 6 well plate in 5% BS/DMEM/ 1XP/S, 1XGlutamax. Two wells are needed per transfection. Two to four hours prior to transfection the media was changed to fresh media. The ChAdV68.4WTnt.GFP plasmid was linearized with PacI. The linearized DNA was then phenol chloroform extracted and precipitated using one tenth volume of 3M Sodium acetate pH 5.3 and two volumes of 100% ethanol. The precipitated DNA was pelleted by centrifugation at 12,000xg for 5 min before washing 1X with 70% ethanol. The pellet was air dried and resuspended in 50 $\mu$L of sterile water. The DNA concentration was determined using a NanoDrop™ (ThermoFisher) and the volume adjusted to 5 $\mu$g of DNA/50 $\mu$L.

169 $\mu$L of sterile water was added to a microfuge tube. 5 $\mu$L of 2M $CaCl_2$ was then added to the water and mixed gently by pipetting. 50 $\mu$L of DNA was added dropwise to the $CaCl_2$ water solution. Twenty six $\mu$L of 2M $CaCl_2$ was then added and mixed gently by pipetting twice with a micro-pipetor. This final solution should consist of 5 $\mu$g of DNA in 250 $\mu$L of 0.25M $CaCl_2$. A second tube was then prepared containing 250 $\mu$L of 2XHBS (Hepes buffered solution). Using a 2 mL sterile pipette attached to a Pipet-Aid air was slowly bubbled through the 2XHBS solution. At the same time the DNA solution in the 0.25M $CaCl_2$ solution was added in a dropwise fashion. Bubbling was continued for approximately 5 seconds after addition of the final DNA droplet. The solution was then incubated at room temperature for up to 20 minutes before adding to 293A cells. 250 $\mu$L of the DNA/Calcium phosphate solution was added dropwise to a monolayer of 293A cells that had been seeded one day prior at $10^6$ cells per well of a 6 well plate. The cells were returned to the incubator and incubated overnight The media was changed 24h later. After 72h the cells were split 1:6 into a 6 well plate. The monolayers were monitored daily by light microscopy for evidence of cytopathic effect (CPE). 7-10 days post transfection viral plaques were observed and the monolayer harvested by pipetting the media in the wells to lift the cells. The harvested cells and media were transferred to a 50 mL centrifuge tube followed by three rounds of freeze thawing (at -80 °C and 37 °C). The subsequent lysate, called the primary virus stock was clarified by centrifugation at full speed on a bench top centrifuge (4300Xg) and a proportion of the lysate 10-50%) used to infect 293A cells in a T25 flask. The infected cells were incubated for 48h before harvesting cells and media at complete CPE. The cells were once again harvested, freeze thawed and clarified before using this secondary viral stock to infect a T150 flask seeded at 1.5x $10^7$ cells per flask. Once complete CPE was achieved at 72h the media and cells were harvested and treated as with earlier viral stocks to generate a tertiary stock.

### Production in 293F cells

ChAdV68 virus production was performed in 293F cells grown in 293 FreeStyleT™ (ThermoFisher) media in an incubator at 8% $CO_2$. On the day of infection cells were diluted to $10^6$ cells per mL, with 98% viability and 400 mL were used per production run in 1L Shake flasks (Corning). 4 mL of the tertiary viral stock with a target MOI of >3.3 was used per infection. The cells were incubated for 48-72h until the viability was <70% as measured by Trypan blue. The infected cells were then harvested by centrifugation, full speed bench top centrifuge and washed in 1XPBS, re-centrifuged and then re-suspended in 20 mL of 10mM Tris pH7.4. The cell pellet was lysed by freeze thawing 3X and clarified by centrifugation at 4,300Xg for 5 minutes.

### Purification by CsCl centrifugation

Viral DNA was purified by CsCl centrifugation. Two discontinuous gradient runs were performed. The first to purify virus from cellular components and the second to further refine separation from cellular components and separate defective from infectious particles.

10 mL of 1.2 (26.8g CsCl dissolved in 92 mL of 10 mM Tris pH 8.0) CsCl was added to polyallomer tubes. Then 8 mL of 1.4 CsCl (53g CsCl dissolved in 87 mL of 10 mM Tris pH 8.0) was carefully added using a pipette delivering to the bottom of the tube. The clarified virus was carefully layered on top of the 1.2 layer. If needed more 10 mM Tris was added to balance the tubes. The tubes were then placed in a SW-32Ti rotor and centrifuged for 2h 30 min at $10^0$C. The tube was then removed to a laminar flow cabinet and the virus band pulled using an 18 guage needle and a 10 mL syringe. Care was taken not to remove contaminating host cell DNA and protein. The band was then diluted at least 2X with 10 mM Tris pH 8.0 and layered as before on a discontinuous gradient as described above. The run was performed as described before except that this time the run was performed overnight. The next day the band was pulled with care to avoid pulling any of the defective particle band. The virus was then dialyzed using a Slide-a-LyzerT™ Cassette (Pierce) against ARM buffer (20 mM Tris pH 8.0, 25 mM NaCl, 2.5% Glycerol). This was performed 3X, 1h per buffer exchange. The virus was then aliquoted for storage at -80°C.

### Viral assays

VP concentration was performed by using an OD 260 assay based on the extinction coefficient of 1.1x $10^{12}$ viral particles (VP) is equivalent to an Absorbance value of 1 at OD260 nm. Two dilutions (1:5 and 1:10) of adenovirus were made in a viral lysis buffer (0.1% SDS, 10 mM Tris pH 7.4, 1mM EDTA). OD was measured in duplicate at both dilutions and the VP concentration/ mL was measured by multiplying the OD260 value X dilution factor X 1.1x $10^{12}$VP.

An infectious unit (IU) titer was calculated by a limiting dilution assay of the viral stock. The virus was initially diluted 100X in DMEM/5% NS/1X PS and then subsequently diluted using 10-fold dilutions down to 1x $10^{-7}$. 100 $\mu$L of these dilutions were then added to 293A cells that were seeded at least an hour before at 3e5 cells/ well of a 24 well plate.

This was performed in duplicate. Plates were incubated for 48h in a CO2 (5%) incubator at 37 $^0$C. The cells were then washed with 1XPBS and were then fixed with 100% cold methanol (-20 °C). The plates were then incubated at -20 $^0$C for a minimum of 20 minutes. The wells were washed with 1XPBS then blocked in 1XPBS/0.1% BSA for 1 h at room temperature. A rabbit anti-Ad antibody (Abcam, Cambridge, MA) was added at 1:8,000 dilution in blocking buffer (0.25 ml per well) and incubated for 1 h at room temperature. The wells were washed 4X with 0.5 mL PBS per well. A HRP conjugated Goat anti-Rabbit antibody (Bethyl Labs, Montgomery Texas) diluted 1000X was added per well and incubated for 1h prior to a final round of washing. 5 PBS washes were performed and the plates were developed using DAB (Diaminobenzidine tetrahydrochloride) substrate in Tris buffered saline (0.67 mg/mL DAB in 50 mM Tris pH 7.5, 150 mM NaCl) with 0.01% $H_2O_2$. Wells were developed for 5 min prior to counting. Cells were counted under a 10X objective using a dilution that gave between 4-40 stained cells per field of view. The field of view that was used was a 0.32 mm$^2$ grid of which there are equivalent to 625 per field of view on a 24 well plate. The number of infectious viruses/ mL can be determined by the number of stained cells per grid multiplied by the number of grids per field of view multiplied by a dilution factor 10. Similarly, when working with GFP expressing cells florescent can be used rather than capsid staining to determine the number of GFP expressing virions per mL.

**Immunizations**

C57BL/6J female mice and Balb/c female mice were injected with 1x10$^8$ viral particles (VP) of ChAdV68.5WTnt.MAG25mer in 100 uL volume, bilateral intramuscular injection (50 uL per leg).

**Splenocyte dissociation**

Spleen and lymph nodes for each mouse were pooled in 3 mL of complete RPMI (RPMI, 10% FBS, penicillin/-streptomycin). Mechanical dissociation was performed using the gentleMACS Dissociator (Miltenyi Biotec), following manufacturer's protocol. Dissociated cells were filtered through a 40 micron filter and red blood cells were lysed with ACK lysis buffer (150mM $NH_4Cl$, 10mM $KHCO_3$, 0.1mM $Na_2EDTA$). Cells were filtered again through a 30 micron filter and then resuspended in complete RPMI. Cells were counted on the Attune NxT flow cytometer (Thermo Fisher) using propidium iodide staining to exclude dead and apoptotic cells. Cell were then adjusted to the appropriate concentration of live cells for subsequent analysis.

**Ex vivo enzyme-linked immunospot (ELISPOT) analysis**

ELISPOT analysis was performed according to ELISPOT harmonization guidelines {DOI: 10.1038/nprot.2015.068} with the mouse IFNg ELISpotPLUS kit (MABTECH). 5x10$^4$ splenocytes were incubated with 10uM of the indicated peptides for 16 hours in 96-well IFNg antibody coated plates. Spots were developed using alkaline phosphatase. The reaction was timed for 10 minutes and was terminated by running plate under tap water. Spots were counted using an AID vSpot Reader Spectrum. For ELISPOT analysis, wells with saturation >50% were recorded as "too numerous to count". Samples with deviation of replicate wells > 10% were excluded from analysis. Spot counts were then corrected for well confluency using the formula: spot count + 2 x (spot count x %confluence /[100% - %confluence]). Negative background was corrected by subtraction of spot counts in the negative peptide stimulation wells from the antigen stimulated wells. Finally, wells labeled too numerous to count were set to the highest observed corrected value, rounded up to the nearest hundred.

**XV.B.2. Production of ChAdV68 Viral Delivery Particles after DNA Transfection**

In one example, ChAdV68.4WTnt.GFP (Fig. 21) and ChAdV68.5WTnt.GFP (Fig. 22) DNA was transfected into HEK293A cells and virus replication (viral plaques) was observed 7-10 days after transfection. ChAdV68 viral plaques were visualized using light (Fig. 21 A and 22A) and fluorescent microscopy (Fig. 21 B-C and Fig. 22 B-C ). GFP denotes productive ChAdV68 viral delivery particle production.

**XV.B.3. ChAdV68 Viral Delivery Particles Expansion**

In one example, ChAdV68.4WTnt.GFP, ChAdV68.5WTnt.GFP, and ChAdV68.5WTnt.MAG25mer viruses were expanded in HEK293F cells and a purified virus stock produced 18 days after transfection (Fig 23). Viral particles were quantified in the purified ChAdV68 virus stocks and compared to adenovirus type 5 (Ad5) and ChAdVY25 (a closely related ChAdV; Dicks, 2012, PloS ONE 7, e40385) viral stocks produced using the same protocol. ChAdV68 viral titers were comparable to Ad5 and ChAdVY25 (Table 7).

**Table 7.** Adenoviral vector production in 293F suspension cells

| Construct | Average VP/cell+/- SD |
|---|---|
| Ad5-Vectors (Multiple vectors) | 2.96e4+/- 2.26e4 |
| Ad5-GFP | 3.89e4 |
| chAdY25-GFP | 1.75e3+/-6.03el |
| ChAdV68.4WTnt.GFP | 1.2e4 +/-6.5e3 |
| ChAdV 68. 5WTnt. GFP | 1.8e3 |
| ChAdV68.5WTnt.MAG25mer | 1.39e3+/-1.1e3 |
| *SD is only reported where multiple Production runs have been performed | |

### XV.B.4. Evaluation of Immunogenicity in Tumor Models

C68 vector expressing mouse tumor antigens were evaluated in mouse immunogenicity studies to demonstrate the C68 vector elicits T-cell responses. T-cell responses to the MHC class I epitope SIINFEKL (SEQ ID NO: 57) were measured in C57BL/6J female mice and the MHC class I epitope AH1-A5 (Slansky et al., 2000, Immunity13:529-538) measured in Balb/c mice. As shown in Fig 29, strong T-cell responses were measured after immunization of mice with ChAdV68.5WTnt.MAG25mer. Mean cellular immune responses of 8957 or 4019 spot forming cells (SFCs) per $10^6$ splenocytes were observed in ELISpot assays when C57BL/6J or Balb/c mice were immunized with ChAdV68.5WTnt.MAG25mer, respectively, 10 days after immunization.

### XVI. Alphavirus Neoantigen Cassette Delivery Vector (comparative)

### XVI.A. Alphavirus Delivery Vector Evaluation Materials and Methods In vitro transcription to generate RNA (comparative)

*For in vitro testing:* plasmid DNA was linearized by restriction digest with PmeI, column purified following manufacturer's protocol (GeneJet DNA cleanup kit, Thermo) and used as template. *In vitro* transcription was performed using the RiboMAX Large Scale RNA production System (Promega) with the $m^7G$ cap analog (Promega) according to manufacturer's protocol. mRNA was purified using the RNeasy kit (Qiagen) according to manufacturer's protocol.
*For in vivo studies*: RNA was generated and purified by TriLInk Biotechnologies and capped with Enzymatic Cap1.

### Transfection of RNA

HEK293A cells were seeded at 6e4 cells/well for 96 wells and 2e5 cells/well for 24 wells, ~16 hours prior to transfection. Cells were transfected with mRNA using MessengerMAX lipofectamine (Invitrogen) and following manufacturer's protocol. For 96-wells, 0.15 uL of lipofectamine and 10 ng of mRNA was used per well, and for 24-wells, 0.75 uL of lipofectamine and 150 ng of mRNA was used per well. A GFP expressing mRNA (TriLink Biotechnologies) was used as a transfection control.

### Luciferase assay

Luciferase reporter assay was performed in white-walled 96-well plates with each condition in triplicate using the ONE-Glo luciferase assay (Promega) following manufacturer's protocol. Luminescence was measured using the SpectraMax.

### qRT-PCR

Transfected cells were rinsed and replaced with fresh media 2 hours post transfection to remove any untransfected mRNA. Cells were then harvested at various timepoints in RLT plus lysis buffer (Qiagen), homogenized using a QiaShredder (Qiagen) and RNA was extracted using the RNeasy kit (Qiagen), all according to manufacturer's protocol. Total RNA was quantified using a Nanodrop (Thermo Scientific). qRT-PCR was performed using the Quantitect Probe One-Step RT-PCR kit (Qiagen) on the qTower[3] (Analytik Jena) according to manufacturer's protocol, using 20 ng of total RNA per reaction. Each sample was run in triplicate for each probe. Actin or GusB were used as reference genes. Custom primer/probes were generated by IDT (Table 8).

**Table 8.** qPCR primers/probes

| Target | | | SEQ ID NO: |
|---|---|---|---|
| Luci | Primer1 | GTGGTGTGCAGCGAGAATAG | 138 |
| | Primer2 | CGCTCGTTGTAGATGTCGTTAG | 139 |
| | Probe | /56-FAM/TTGCAGTTC/ZEN/TTCATGCCCGTGTTG/3IABkFQ/ | 140 |
| GusB | Primer1 | GTTTTTGATCCAGACCCAGATG | 141 |
| | Primer2 | GCCCATTATTCAGAGCGAGTA | 142 |
| | Probe | /56-FAM/TGCAGGGTT/ZEN/TCACCAGGATCCAC/3IABkFQ/ | 143 |
| ActB | Primer1 | CCTTGCACATGCCGGAG | 144 |
| | Primer2 | ACAGAGCCTCGCCTTTG | 145 |
| | Probe | /56-FAM/TCATCCATG/ZEN/GTGAGCTGGCGG/3IABkFQ/ | 146 |
| MAG-25mer | Primer1 | CTGAAAGCTCGGTTTGCTAATG | 147 |
| Set1 | Primer2 | CCATGCTGGAAGAGACAATCT | 148 |
| | Probe | /56-FAM/CGTTTCTGA/ZEN/TGGCGCTGACCGATA/3IABkFQ/ | 149 |
| MAG-25mer | Primer1 | TATGCCTATCCTGTCTCCTCTG | 150 |
| Set2 | Primer2 | GCTAATGCAGCTAAGTCCTCTC | 151 |
| | Probe | /56-FAM/TGTTTACCC/ZEN/TGACCGTGCCTTCTG/3IABkFQ/ | 152 |

**B16-OVA tumor model**

C57BL/6J mice were injected in the lower left abdominal flank with $10^5$ B16-OVA cells/animal. Tumors were allowed to grow for 3 days prior to immunization.

**CT26 tumor model**

Balb/c mice were injected in the lower left abdominal flank with $10^6$ CT26 cells/animal. Tumors were allowed to grow for 7 days prior to immunization.

**Immunizations**

For srRNA vaccine, mice were injected with 10 ug of RNA in 100 uL volume, bilateral intramuscular injection (50 uL per leg). For Ad5 vaccine, mice were injected with $5x10^{10}$ viral particles (VP) in 100 uL volume, bilateral intramuscular injection (50 uL per leg). Animals were injected with anti-CTLA-4 (clone 9D9, BioXcell), anti-PD-1 (clone RMP1-14, BioXcell) or anti-IgG (clone MPC-11, BioXcell), 250 ug dose, 2 times per week, via intraperitoneal injection.

**In vivo bioluminescent imaging**

At each timepoint mice were injected with 150 mg/kg luciferin substrate via intraperitoneal injection and bioluminescence was measured using the IVIS In vivo imaging system (PerkinElmer) 10-15 minutes after injection.

**Splenocyte dissociation**

Spleen and lymph nodes for each mouse were pooled in 3 mL of complete RPMI (RPMI, 10% FBS, penicillin/-streptomycin). Mechanical dissociation was performed using the gentleMACS Dissociator (Miltenyi Biotec), following manufacturer's protocol. Dissociated cells were filtered through a 40 micron filter and red blood cells were lysed with ACK lysis buffer (150mM $NH_4Cl$, 10mM $KHCO_3$, 0.1mM $Na_2EDTA$). Cells were filtered again through a 30 micron filter and then resuspended in complete RPMI. Cells were counted on the Attune NxT flow cytometer (Thermo Fisher) using propidium iodide staining to exclude dead and apoptotic cells. Cell were then adjusted to the appropriate concentration of live cells for subsequent analysis.

**Ex vivo enzyme-linked immunospot (ELISPOT) analysis**

ELISPOT analysis was performed according to ELISPOT harmonization guidelines {DOI: 10.1038/nprot.2015.068} with the mouse IFNg ELISpotPLUS kit (MABTECH). $5x10^4$ splenocytes were incubated with 10uM of the indicated peptides for 16 hours in 96-well IFNg antibody coated plates. Spots were developed using alkaline phosphatase. The reaction was timed for 10 minutes and was terminated by running plate under tap water. Spots were counted using an AID vSpot Reader Spectrum. For ELISPOT analysis, wells with saturation >50% were recorded as "too numerous to count". Samples with deviation of replicate wells > 10% were excluded from analysis. Spot counts were then corrected for well confluency using the formula: spot count + 2 x (spot count x %confluence /[100% - %confluence]). Negative background was corrected by subtraction of spot counts in the negative peptide stimulation wells from the antigen stimulated wells. Finally, wells labeled too numerous to count were set to the highest observed corrected value, rounded up to the nearest hundred.

**XVI.B. Alphavirus Vector (comparative)**

**XVI.B.1. Alphavirus Vector *in vitro* Evaluation (comparative)**

An RNA alphavirus backbone for the neoantigen expression system was generated from a Venezuelan Equine Encephalitis (VEE) (Kinney, 1986, Virology 152: 400-413) based self-replicating RNA (srRNA) vector. In one example, the sequences encoding the structural proteins of VEE located 3' of the 26S sub-genomic promoter were deleted (VEE sequences 7544 to 11,175 deleted; numbering based on Kinney *et al* 1986; SEQ ID NO:6) and replaced by antigen sequences (SEQ ID NO: 14 and SEQ ID NO:4) or a luciferase reporter (e.g., VEE-Luciferase, SEQ ID NO: 15) (Fig. 24). RNA was transcribed from the srRNA DNA vector *in vitro,* transfected into HEK293A cells and luciferase reporter expression was measured. In addition, an (non-replicating) mRNA encoding luciferase was transfected for comparison. An ~30,000-fold increase in srRNA reporter signal was observed for VEE-Luciferase srRNA when comparing the 23 hour measurement vs the 2 hour measurement (Table 9). In contrast, the mRNA reporter exhibited a < 10-fold increase in signal over the same time period (Table 9).

**Table 9.** Expression of luciferase from VEE self-replicating vector increases over time. HEK293A cells transfected with 10 ng of VEE-Luciferase srRNA or 10 ng of non-replicating luciferase mRNA (TriLink L-6307) per well in 96 wells. Luminescence was measured at various times post transfection. Luciferase expression is reported as relative luminescence units (RLU). Each data point is the mean +/- SD of 3 transfected wells.

| Construct | Timepoint (hr) | Mean RLU | Standard Dev (triplicate wells) |
|---|---|---|---|
| mRNA | 2 | 878.6666667 | 120.7904522 |
| mRNA | 5 | 1847.333333 | 978.515372 |
| mRNA | 9 | 4847 | 868.3271273 |
| mRNA | 23 | 8639.333333 | 751.6816702 |
| SRRNA | 2 | 27 | 15 |
| SRRNA | 5 | 4884.333333 | 2955.158935 |
| SRRNA | 9 | 182065.5 | 16030.81784 |
| SRRNA | 23 | 783658.3333 | 68985.05538 |

In another example, replication of the srRNA was confirmed directly by measuring RNA levels after transfection of either the luciferase encoding srRNA (VEE-Luciferase) or an srRNA encoding a multi-epitope cassette (VEE-MAG25mer) using quantitative reverse transcription polymerase chain reaction (qRT-PCR). An ~150-fold increase in RNA was observed for the VEE-luciferase srRNA (Table 10), while a 30-50-fold increase in RNA was observed for the VEE-MAG25mer srRNA (Table 11). These data confirm that the VEE srRNA vectors replicate when transfected into cells.

**Table** 10. Direct measurement of RNA replication in VEE-Luciferase srRNA transfected cells. HEK293A cells transfected with VEE-Luciferase srRNA (150 ng per well, 24-well) and RNA levels quantified by qRT-PCR at various times after transfection. Each measurement was normalized based on the Actin reference gene and fold-change relative to the 2 hour timepoint is presented.

| Timepoint (hr) | Luciferase Ct | Actin Ct | dCt | Ref dCt | ddCt | Relative Fold change |
|---|---|---|---|---|---|---|
| 2 | 20.51 | 18.14 | 2.38 | 2.38 | 0.00 | 1.00 |
| 4 | 20.09 | 18.39 | 1.70 | 2.38 | -0.67 | 1.59 |
| 6 | 15.50 | 18.19 | -2.69 | 2.38 | -5.07 | 33.51 |
| 8 | 13.51 | 18.36 | -4.85 | 2.38 | -7.22 | 149.43 |

**Table 11.** Direct measurement of RNA replication in VEE-MAG25mer srRNA transfected cells. HEK293 cells transfected with VEE-MAG25mer srRNA (150 ng per well, 24-well) and RNA levels quantified by qRT-PCR at various times after transfection. Each measurement was normalized based on the GusB reference gene and fold-change relative to the 2 hour timepoint is presented. Different lines on the graph represent 2 different qPCR primer/probe sets, both of which detect the epitope cassette region of the srRNA.

| Primer/ probe | Timepoint (hr) | Ct | GusB Ct | dCt | Ref dCt | ddCt | Relative Fold-Change |
|---|---|---|---|---|---|---|---|
| Set1 | 2 | 18.96 | 22.41 | -3.45 | -3.45 | 0.00 | 1.00 |
| Set1 | 4 | 17.46 | 22.27 | -4.81 | -3.45 | -1.37 | 2.58 |
| Set1 | 6 | 14.87 | 22.04 | -7.17 | -3.45 | -3.72 | 13.21 |
| Set1 | 8 | 14.16 | 22.19 | -8.02 | -3.45 | -4.58 | 23.86 |
| Set1 | 24 | 13.16 | 22.01 | -8.86 | -3.45 | -5.41 | 42.52 |
| Set1 | 36 | 13.53 | 22.63 | -9.10 | -3.45 | -5.66 | 50.45 |
| Set2 | 2 | 17.75 | 22.41 | -4.66 | -4.66 | 0.00 | 1.00 |
| Set2 | 4 | 16.66 | 22.27 | -5.61 | -4.66 | -0.94 | 1.92 |
| Set2 | 6 | 14.22 | 22.04 | -7.82 | -4.66 | -3.15 | 8.90 |
| Set2 | 8 | 13.18 | 22.19 | -9.01 | -4.66 | -4.35 | 20.35 |
| Set2 | 24 | 12.22 | 22.01 | -9.80 | -4.66 | -5.13 | 35.10 |
| Set2 | 36 | 13.08 | 22.63 | -9.55 | -4.66 | -4.89 | 29.58 |

### XVI.B.2. Alphavirus Vector *in vivo* Evaluation (comparative)

In another example, VEE-Luciferase reporter expression was evaluated in vivo. Mice were injected with 10 ug of VEE-Luciferase srRNA encapsulated in lipid nanoparticle (MC3) and imaged at 24 and 48 hours, and 7 and 14 days post injection to determine bioluminescent signal. Luciferase signal was detected at 24 hours post injection and increased over time and appeared to peak at 7 days after srRNA injection (Fig. 25).

### XVI.B.3. Alphavirus Vector Tumor Model Evaluation (comparative)

In one implementation, to determine if the VEE srRNA vector directs antigen-specific immune responses *in vivo,* a VEE srRNA vector was generated (VEE-UbAAY, SEQ ID NO: 14) that expresses 2 different MHC class I mouse tumor epitopes, SIINFEKL (SEQ ID NO: 57) and AH1-A5 (Slansky et al., 2000, Immunity 13:529-538). The SFL (SIINFEKL (SEQ ID NO: 57)) epitope is expressed by the B16-OVA melanoma cell line, and the AH1-A5 (SPSYAYHQF (SEQ ID NO: 58); Slansky et al., 2000, Immunity) epitope induces T cells targeting a related epitope (AH1/ SPSYVYHQF (SEQ ID NO: 153); Huang et al., 1996, Proc Natl Acad Sci USA 93:9730-9735) that is expressed by the CT26 colon carcinoma cell line. In one example, for *in vivo* studies, VEE-UbAAY srRNA was generated by *in vitro* transcription using T7 polymerase (TriLink Biotechnologies) and encapsulated in a lipid nanoparticle (MC3).

A strong antigen-specific T-cell response targeting SFL was observed two weeks after immunization of B16-OVA tumor bearing mice with MC3 formulated VEE-UbAAY srRNA. In one example, a median of 3835 spot forming cells (SFC) per $10^6$ splenocytes was measured after stimulation with the SFL peptide in ELISpot assays (Fig. 26A, Table 12) and 1.8% (median) of CD8 T-cells were SFL antigen-specific as measured by pentamer staining (Fig. 26B, Table 12). In another example, co-administration of an anti-CTLA-4 monoclonal antibody (mAb) with the VEE srRNA vaccine resulted in a moderate increase in overall T-cell responses with a median of 4794.5 SFCs per $10^6$ splenocytes measured in the ELISpot assay (Fig. 26A, Table 12).

**Table 12.** Results of ELISPOT and MHCI-pentamer staining assays 14 days post VEE srRNA immunization in B16-OVA tumor bearing C57BL/6J mice.

| Group | Mouse | SFC/1e6 splenocytes | Pentamer positive (% of CD8) | Group | Mouse | SFC/1e6 splenocytes | Pentamer positive (% of CD8) |
|---|---|---|---|---|---|---|---|
| Control | 1 | 47 | 0.22 | Vax | 1 | 6774 | 4.92 |
| | 2 | 80 | 0.32 | | 2 | 2323 | 1.34 |
| | 3 | 0 | 0.27 | | 3 | 2997 | 1.52 |
| | 4 | 0 | 0.29 | | 4 | 4492 | 1.86 |
| | 5 | 0 | 0.27 | | 5 | 4970 | 3.7 |
| | 6 | 0 | 0.25 | | 6 | | 4.13 |
| | 7 | 0 | 0.23 | | 7 | 3835 | 1.66 |
| | 8 | 87 | 0.25 | | 8 | 3119 | 1.64 |
| aCTLA4 | 1 | 0 | 0.24 | Vax + aCTLA4 | 1 | 6232 | 2.16 |
| | 2 | 0 | 0.26 | | 2 | 4242 | 0.82 |
| | 3 | 0 | 0.39 | | 3 | 5347 | 1.57 |
| | 4 | 0 | 0.28 | | 4 | 6568 | 2.33 |
| | 5 | 0 | 0.28 | | 5 | 6269 | *1.55* |
| | 6 | 0 | 0.28 | | 6 | 4056 | 1.74 |
| | 7 | 0 | 0.31 | | 7 | 4163 | 1.14 |
| | 8 | 6 | 0.26 | | 8 | 3667 | 1.01 |

*Note that results from mouse #6 in the Vax group were excluded from analysis due to high variability between triplicate wells.*

In another implementation, to mirror a clinical approach, a heterologous prime/boost in the B16-OVA and CT26 mouse tumor models was performed, where tumor bearing mice were immunized first with adenoviral vector expressing the same antigen cassette (Ad5-UbAAY), followed by a boost immunization with the VEE-UbAAY srRNA vaccine 14 days after the Ad5-UbAAY prime. In one example, an antigen-specific immune response was induced by the Ad5-UbAAY vaccine resulting in 7330 (median) SFCs per $10^6$ splenocytes measured in the ELISpot assay (Fig. 27A, Table 13) and 2.9% (median) of CD8 T-cells targeting the SFL antigen as measured by pentamer staining (Fig. 27C, Table 13). In another example, the T-cell response was maintained 2 weeks after the VEE-UbAAY srRNA boost in the B16-OVA model with 3960 (median) SFL-specific SFCs per $10^6$ splenocytes measured in the ELISpot assay (Fig. 27B, Table 13) and 3.1% (median) of CD8 T-cells targeting the SFL antigen as measured by pentamer staining (Fig. 27D, Table 13).

**Table 13.** Immune monitoring of B16-OVA mice following heterologous prime/boost with Ad5 vaccine prime and srRNA boost.

| Day 14 | | | | | | | |
|---|---|---|---|---|---|---|---|
| Group | Mouse | SFC/1e6 splenocytes | Pentamer positive (% of CD8) | Group | Mouse | SFC/1e6 splenocytes | Pentamer positive (% of CD8) |
| Control | 1 | 0 | 0.10 | Vax | 1 | 8514 | 1.87 |
| | 2 | 0 | 0.09 | | 2 | 7779 | 1.91 |
| | 3 | 0 | 0.11 | | 3 | 6177 | 3.17 |
| | 4 | 46 | 0.18 | | 4 | 7945 | 3.41 |
| | 5 | 0 | 0.11 | | 5 | 8821 | 4.51 |
| | 6 | 16 | 0.11 | | 6 | 6881 | 2.48 |
| | 7 | 0 | 0.24 | | 7 | 5365 | 2.57 |
| | 8 | 37 | 0.10 | | 8 | 6705 | 3.98 |
| aCTLA4 | 1 | 0 | 0.08 | Vax + aCTLA4 | 1 | 9416 | 2.35 |
| | 2 | 29 | 0.10 | | 2 | 7918 | 3.33 |
| | 3 | 0 | 0.09 | | 3 | 10153 | 4.50 |
| | 4 | 29 | 0.09 | | 4 | 7212 | 2.98 |
| | 5 | 0 | 0.10 | | 5 | 11203 | 4.38 |
| | 6 | 49 | 0.10 | | 6 | 9784 | 2.27 |
| | 7 | 0 | 0.10 | | 8 | 7267 | 2.87 |
| | 8 | 31 | 0.14 | | | | |
| Day 28 | | | | | | | |
| Group | Mouse | SFC/1e6 splenocytes | Pentamer positive (% of CD8) | Group | Mouse | SFC/1e6 splenocytes | Pentamer positive (% of CD8) |
| Control | 2 | 0 | 0.17 | Vax | 1 | 5033 | 2.61 |
| | 4 | 0 | 0.15 | | 2 | 3958 | 3.08 |
| | 6 | 20 | 0.17 | | 4 | 3960 | 3.58 |
| aCTLA4 | 1 | 7 | 0.23 | Vax + aCTLA4 | 4 | 3460 | 2.44 |
| | 2 | 0 | 0.18 | | 5 | 5670 | 3.46 |
| | 3 | 0 | 0.14 | | | | |

In another implementation, similar results were observed after an Ad5-UbAAY prime and VEE-UbAAY srRNA boost in the CT26 mouse model. In one example, an AH1 antigen-specific response was observed after the Ad5-UbAAY prime (day 14) with a mean of 5187 SFCs per $10^6$ splenocytes measured in the ELISpot assay (Fig. 28A, Table 14) and 3799 SFCs per $10^6$ splenocytes measured in the ELISpot assay after the VEE-UbAAY srRNA boost (day 28) (Fig. 28B, Table 14).

**Table 14.** Immune monitoring after heterologous prime/boost in CT26 tumor mouse model.

| Day 12 | | | Day 21 | | |
|---|---|---|---|---|---|
| Group | Mouse | SFC/1e6 splenocytes | Group | Mouse | SFC/1e6 splenocytes |
| Control | 1 | 1799 | Control | 9 | 167 |
| | 2 | 1442 | | 10 | 115 |
| | 3 | 1235 | | 11 | 347 |

(continued)

| Day 12 | | | Day 21 | | |
|---|---|---|---|---|---|
| Group | Mouse | SFC/1e6 splenocytes | Group | Mouse | SFC/1e6 splenocytes |
| aPD1 | 1 | 737 | aPD1 | 8 | 511 |
| | 2 | 5230 | | 11 | 758 |
| | 3 | 332 | Vax | 9 | 3133 |
| Vax | 1 | 6287 | | 10 | 2036 |
| | 2 | 4086 | | 11 | 6227 |
| Vax + aPD1 | 1 | 5363 | Vax + aPD1 | 8 | 3844 |
| | 2 | 6500 | | 9 | 2071 |
| | | | | 11 | 4888 |

## XVII. ChAdV/srRNA Combination Tumor Model Evaluation

Various dosing protocols using ChAdV68 and self-replicating RNA (srRNA) were evaluated in murine CT26 tumor models.

## XVII.A ChAdV/srRNA Combination Tumor Model Evaluation Methods and Materials

**Tumor Injection**

Balb/c mice were injected with the CT26 tumor cell line. 7 days after tumor cell injection, mice were randomized to the different study arms (28-40 mice per group) and treatment initiated. Balb/c mice were injected in the lower left abdominal flank with $10^6$ CT26 cells/animal. Tumors were allowed to grow for 7 days prior to immunization. The study arms are described in detail in Table 15.

Table 15 - ChAdV/srRNA Combination Tumor Model Evaluation Study Arms

| Group | N | Treatment | Dose | Volume | Schedule | Route |
|---|---|---|---|---|---|---|
| 1 | 40 | chAd68 control | 1e11 vp | 2x 50 uL | day 0 | IM |
| | | srRNA control | 10 ug | 50 uL | day 14, 28, 42 | IM |
| | | Anti-PD1 | 250 ug | 100 uL | 2x / week (start day 0) | IP |
| 2 | 40 | chAd68 control | 1e11 vp | 2x 50 uL | day 0 | IM |
| | | srRNA control | 10 ug | 50 uL | day 14, 28, 42 | IM |
| | | Anti-IgG | 250 ug | 100 uL | 2x / week (start day 0) | IP |
| 3 | 28 | chAd68 vaccine | 1e11 vp | 2x 50 uL | day 0 | IM |
| | | srRNA vaccine | 10 ug | 50 uL | day 14, 28, 42 | IM |
| | | Anti-PD1 | 250 ug | 100 uL | 2x / week (start day 0) | IP |
| 4 | 28 | chAd68 vaccine | 1e11 vp | 2x 50 uL | day 0 | IM |
| | | srRNA vaccine | 10 ug | 50 uL | day 14, 28, 42 | IM |
| | | Anti-IgG | 250 ug | 100 uL | 2x / week (start day 0) | IP |
| 5 | 28 | srRNA vaccine | 10 ug | 50 uL | day 0, 28, 42 | IM |
| | | chAd68 vaccine | 1e11 vp | 2x 50 uL | day 14 | IM |
| | | Anti-PD1 | 250 ug | 100 uL | 2x / week (start day 0) | IP |

(continued)

| Group | N | Treatment | Dose | Volume | Schedule | Route |
|---|---|---|---|---|---|---|
| 6 | 28 | srRNA vaccine | 10 ug | 50 uL | day 0, 28, 42 | IM |
| | | chAd68 vaccine | 1e11 vp | 2x 50 uL | day 14 | IM |
| | | Anti-IgG | 250 ug | 100 uL | 2x / week (start day 0) | IP |
| 7 | 40 | srRNA vaccine | 10 ug | 50 uL | day 0, 14, 28, 42 | IM |
| | | Anti-PD1 | 250 ug | 100 uL | 2x / week (start day 0) | IP |
| 8 | 40 | srRNA vaccine | 10 ug | 50 uL | day 0, 14, 28, 42 | IM |
| | | Anti-IgG | 250 ug | 100 uL | 2x / week (start day 0) | IP |

**Immunizations**

For srRNA vaccine, mice were injected with 10 ug of VEE-MAG25mer srRNA in 100 uL volume, bilateral intramuscular injection (50 uL per leg). For C68 vaccine, mice were injected with $1 \times 10^{11}$ viral particles (VP) of ChAdV68.5WTnt.MAG25mer in 100 uL volume, bilateral intramuscular injection (50 uL per leg). Animals were injected with anti-PD-1 (clone RMP1-14, BioXcell) or anti-IgG (clone MPC-11, BioXcell), 250 ug dose, 2 times per week, via intraperitoneal injection.

**Splenocyte dissociation**

Spleen and lymph nodes for each mouse were pooled in 3 mL of complete RPMI (RPMI, 10% FBS, penicillin/-streptomycin). Mechanical dissociation was performed using the gentleMACS Dissociator (Miltenyi Biotec), following manufacturer's protocol. Dissociated cells were filtered through a 40 micron filter and red blood cells were lysed with ACK lysis buffer (150mM $NH_4Cl$, 10mM $KHCO_3$, 0.1mM $Na_2EDTA$). Cells were filtered again through a 30 micron filter and then resuspended in complete RPMI. Cells were counted on the Attune NxT flow cytometer (Thermo Fisher) using propidium iodide staining to exclude dead and apoptotic cells. Cell were then adjusted to the appropriate concentration of live cells for subsequent analysis.

**Ex vivo enzyme-linked immunospot (ELISPOT) analysis**

ELISPOT analysis was performed according to ELISPOT harmonization guidelines {DOI: 10.1038/nprot.2015.068} with the mouse IFNg ELISpotPLUS kit (MABTECH). $5 \times 10^4$ splenocytes were incubated with 10uM of the indicated peptides for 16 hours in 96-well IFNg antibody coated plates. Spots were developed using alkaline phosphatase. The reaction was timed for 10 minutes and was terminated by running plate under tap water. Spots were counted using an AID vSpot Reader Spectrum. For ELISPOT analysis, wells with saturation >50% were recorded as "too numerous to count". Samples with deviation of replicate wells > 10% were excluded from analysis. Spot counts were then corrected for well confluency using the formula: spot count + 2 x (spot count x %confluence /[100% - %confluence]). Negative background was corrected by subtraction of spot counts in the negative peptide stimulation wells from the antigen stimulated wells. Finally, wells labeled too numerous to count were set to the highest observed corrected value, rounded up to the nearest hundred.

**XVII.B ChAdV/srRNA Combination Evaluation in a CT26 Tumor Model**

The immunogenicity and efficacy of the ChAdV68.5WTnt.MAG25mer/ VEE-MAG25mer srRNA heterologous prime/boost or VEE-MAG25mer srRNA homologous prime/boost vaccines were evaluated in the CT26 mouse tumor model. Balb/c mice were injected with the CT26 tumor cell line. 7 days after tumor cell injection, mice were randomized to the different study arms and treatment initiated. The study arms are described in detail in Table 15 and more generally in Table 16.

Table 16 - Prime/Boost Study Arms

| Group | Prime | Boost |
|---|---|---|
| 1 | Control | Control |
| 2 | Control + anti-PD-1 | Control + anti-PD-1 |

(continued)

| Group | Prime | Boost |
|---|---|---|
| 3 | ChAdV68.5WTnt.MAG25mer | VEE-MAG25mer srRNA |
| 4 | ChAdV68.5WTnt.MAG25mer + anti-PD-1 | VEE-MAG25mer srRNA + anti-PD-1 |
| 5 | VEE-MAG25mer srRNA | ChAdV68.5WTnt.MAG25mer |
| 6 | VEE-MAG25mer srRNA + anti-PD-1 | ChAdV68.5WTnt.MAG25mer + anti-PD-1 |
| 7 | VEE-MAG25mer srRNA | VEE-MAG25mer srRNA |
| 8 | VEE-MAG25mer srRNA + anti-PD-1 | VEE-MAG25mer srRNA + anti-PD-1 |

Spleens were harvested 14 days after the prime vaccination for immune monitoring. Tumor and body weight measurements were taken twice a week and survival was monitored. Strong immune responses were observed in all active vaccine groups.

Median cellular immune responses of 10,630, 12,976, 3319, or 3745 spot forming cells (SFCs) per $10^6$ splenocytes were observed in ELISpot assays in mice immunized with ChAdV68.5WTnt.MAG25mer (ChAdV/group 3), ChAdV68.5WTnt.MAG25mer + anti-PD-1 (ChAdV + PD-1/group 4), VEE-MAG25mer srRNA (srRNA/median for groups 5 & 7 combined), or VEE-MAG25mer srRNA + anti-PD-1 (srRNA + PD-1/median for groups 6 & 8 combined), respectively, 14 days after the first immunization (Fig. 30 and Table 17). In contrast, the vaccine control (group 1) or vaccine control with anti-PD-1 (group 2) exhibited median cellular immune responses of 296 or 285 SFC per $10^6$ splenocytes, respectively.

Table 17 - Cellular immune responses in a CT26 tumor model

| Treatment | Median SFC/$10^6$ Splenocytes |
|---|---|
| Control | 296 |
| PD1 | 285 |
| ChAdV68.5WTnt.MAG25mer (ChAdV) | 10630 |
| ChAdV68.5WTnt.MAG25mer + PD1 (ChAdV + PD-1) | 12976 |
| VEE-MAG25mer srRNA (srRNA) | 3319 |
| VEE-MAG25mer srRNA + PD-1 (srRNA + PD1) | 3745 |

Consistent with the ELISpot data, 5.6, 7.8, 1.8 or 1.9% of CD8 T cells (median) exhibited antigen-specific responses in intracellular cytokine staining (ICS) analyses for mice immunized with ChAdV68.5WTnt.MAG25mer (ChAdV/group 3), ChAdV68.5WTnt.MAG25mer + anti-PD-1 (ChAdV + PD-1/group 4), VEE-MAG25mer srRNA (srRNA/-median for groups 5 & 7 combined), or VEE-MAG25mer srRNA + anti-PD-1 (srRNA + PD-1/median for groups 6 & 8 combined), respectively, 14 days after the first immunization (Fig. 31 and Table 18 . Mice immunized with the vaccine control or vaccine control combined with anti-PD-1 showed antigen-specific CD8 responses of 0.2 and 0.1%, respectively.

Table 18 - CD8 T-Cell responses in a CT26 tumor model

| Treatment | Median % CD8 IFN-gamma Positive |
|---|---|
| Control | 0.21 |
| PD1 | 0.1 |
| ChAdV68.5WTnt.MAG25mer (ChAdV) | 5.6 |
| ChAdV68.5WTnt.MAG25mer + PD1 (ChAdV + PD-1) | 7.8 |
| VEE-MAG25mer srRNA (srRNA) | 1.8 |
| VEE-MAG25mer srRNA + PD-1 (srRNA + PD1) | 1.9 |

Tumor growth was measured in the CT26 colon tumor model for all groups, and tumor growth up to 21 days after treatment initiation (28 days after injection of CT-26 tumor cells) is presented. Mice were sacrificed 21 days after treatment initiation based on large tumor sizes (>2500 mm³); therefore, only the first 21 days are presented to avoid analytical bias. Mean tumor volumes at 21 days were 1129, 848, 2142, 1418, 2198 and 1606 mm³ for ChAdV68.5WTnt.MAG25mer prime/ VEE-MAG25mer srRNA boost (group 3), ChAdV68.5WTnt.MAG25mer prime/ VEE-MAG25mer srRNA boost + anti-PD-1 (group 4), VEE-MAG25mer srRNA prime/ ChAdV68.5WTnt.MAG25mer boost (group 5), VEE-MAG25mer srRNA prime / ChAdV68.5WTnt.MAG25mer boost + anti-PD-1 (group 6), VEE-MAG25mer srRNA prime/ VEE-MAG25mer srRNA boost (group 7) and VEE-MAG25mer srRNA prime/ VEE-MAG25mer srRNA boost + anti-PD-1 (group 8), respectively (Fig 32 and Table 19). The mean tumor volumes in the vaccine control or vaccine control combined with anti-PD-1 were 2361 or 2067 mm³, respectively. Based on these data, vaccine treatment with ChAdV68.5WTnt.MAG25mer / VEE-MAG25mer srRNA (group 3), ChAdV68.5WTnt.MAG25mer / VEE-MAG25mer srRNA + anti-PD-1 (group 4), VEE-MAG25mer srRNA/ ChAdV68.5WTnt.MAG25mer + anti-PD-1 (group 6) and VEE-MAG25mer srRNA/ VEE-MAG25mer srRNA + anti-PD-1 (group 8) resulted in a reduction of tumor growth at 21 days that was significantly different from the control (group 1).

Table 19 - Tumor size at day 21 measured in the CT26 model

| Treatment | Tumor Size (mm³) | SEM |
|---|---|---|
| Control | 2361 | 235 |
| PD1 | 2067 | 137 |
| chAdV/srRNA | 1129 | 181 |
| chAdV/srRNA + PD1 | 848 | 182 |
| srRNA/chAdV | 2142 | 233 |
| srRNA/chAdV + PD1 | 1418 | 220 |
| srRNA | 2198 | 134 |
| srRNA + PD1 | 1606 | 210 |

Survival was monitored for 35 days after treatment initiation in the CT-26 tumor model (42 days after injection of CT-26 tumor cells). Improved survival was observed after vaccination of mice with 4 of the combinations tested. After vaccination, 64%, 46%, 41% and 36% of mice survived with ChAdV68.5WTnt.MAG25mer prime/ VEE-MAG25mer srRNA boost in combination with anti-PD-1 (group 4; P<0.0001 relative to control group 1), VEE-MAG25mer srRNA prime/ VEE-MAG25mer srRNA boost in combination with anti-PD-1 (group 8; P=0.0006 relative to control group 1), ChAdV68.5WTnt.MAG25mer prime/ VEE-MAG25mer srRNA boost (group 3; P=0.0003 relative to control group 1) and VEE-MAG25mer srRNA prime/ ChAdV68.5WTnt.MAG25mer boost in combination with anti-PD-1 (group 6; P=0.0016 relative to control group 1), respectively (Fig. 33 and Table 20). Survival was not significantly different from the control group 1 (≤14%) for the remaining treatment groups [VEE-MAG25mer srRNAprime/ ChAdV68.5WTnt.MAG25-mer boost (group 5), VEE-MAG25mer srRNA prime/ VEE-MAG25mer srRNA boost (group 7) and anti-PD-1 alone (group 2)].

Table 20 - Survival in the CT26 model

| Timepoint | Control | PD1 | chAdV/ srRNA | chAdV/ srRNA + PD1 | srRNA/ chAdV | srRNA/ chAdV + PD1 | srRNA | srRNA + PD1 |
|---|---|---|---|---|---|---|---|---|
| 0 | 100 | 100 | 100 | 100.00 | 100.00 | 100 | 100 | 100 |
| 21 | 96 | 100 | 100 | 100 | 100 | 95 | 100 | 100 |
| 24 | 54 | 64 | 91 | 100 | 68 | 82 | 68 | 71 |
| 28 | 21 | 32 | 68 | 86 | 45 | 68 | 21 | 64 |
| 31 | 7 | 14 | 41 | 64 | 14 | 36 | 11 | 46 |
| 35 | 7 | 14 | 41 | 64 | 14 | 36 | 11 | 46 |

In conclusion, ChAdV68.5WTnt.MAG25mer and VEE-MAG25mer srRNA elicited strong T-cell responses to mouse tumor antigens encoded by the vaccines. Administration of a ChAdV68.5WTnt.MAG25mer prime and VEE-MAG25mer srRNA boost with or without coadministration of anti-PD-1, VEE-MAG25mer srRNA prime and ChAdV68.5WTnt.MAG25mer boost in combination with anti-PD-1 or administration of VEE-MAG25mer srRNA as a homologous prime boost immunization in combination with anti-PD-1 to tumor bearing mice resulted in improved survival.

## XVIII. Non-Human Primate Study

Various dosing protocols using ChAdV68 and self-replicating RNA (srRNA) were evaluated in non-human primates (NHP).

## XVIII.A. Non-Human Primate Study Materials and Methods Immunizations

A priming vaccine was injected intramuscularly in each NHP to initiate the study (vaccine prime). Mamu A01 Indian rhesus macaques were immunized bilaterally with $1 \times 10^{12}$ viral particles ($5 \times 10^{11}$ viral particles per injection) of ChAdV68.5WTnt.MAG25mer , 30 ug of VEE-MAG25MER srRNA, 100 ug of VEE-MAG25mer srRNA or 300 ug of VEE-MAG25mer srRNAformulated in LNP-1 or LNP-2.30 ug, 100 ug or 300 ug VEE-MAG25mer srRNAvaccine boosts was administered intramuscularly 4 weeks after prime vaccination. In additional study arms, 30 ug, 100 ug or 300 ug VEE-MAG25mer srRNA vaccines are administered as a second boost intramuscularly 8 weeks after the intitial prime vaccination. Anti-CTLA-4 was administered SC proximal to the site of vaccine immunization or delivered IV to specified groups. Bilateral injections per dose are administered according to groups outlined in Table 21 and 23.

## Immune Monitoring

PBMCs were isolated 7, 14, 28 or 35 days after prime vaccination using Lymphocyte Separation Medium (LSM, MP Biomedicals) and LeucoSep separation tubes (Greiner Bio-One) and resuspended in RPMI containing 10% FBS and penicillin/streptomycin. Cells were counted on the Attune NxT flow cytometer (Thermo Fisher) using propidium iodide staining to exclude dead and apoptotic cells. Cell were then adjusted to the appropriate concentration of live cells for subsequent analysis. For each monkey in the studies, T cell responses were measured using ELISpot or flow cytometry methods. T cell responses to 6 different rhesus macaque Mamu-A*01 class I epitopes encoded in the vaccines were monitored from PBMCs by measuring induction of cytokines, such as IFN-gamma, using ex vivo enzyme-linked immunospot (ELISpot) analysis. ELISpot analysis was performed according to ELISPOT harmonization guidelines {DOI: 10.1038/nprot.2015.068} with the monkey IFNg ELISpotPLUS kit (MABTECH). 200,000 PBMCs were incubated with 10uM of the indicated peptides for 16 hours in 96-well IFNg antibody coated plates. Spots were developed using alkaline phosphatase. The reaction was timed for 10 minutes and was terminated by running plate under tap water. Spots were counted using an AID vSpot Reader Spectrum. For ELISPOT analysis, wells with saturation >50% were recorded as "too numerous to count". Samples with deviation of replicate wells > 10% were excluded from analysis. Spot counts were then corrected for well confluency using the formula: spot count + 2 x (spot count x %confluence /[100% - %confluence]). Negative background was corrected by subtraction of spot counts in the negative peptide stimulation wells from the antigen stimulated wells. Finally, wells labeled too numerous to count were set to the highest observed corrected value, rounded up to the nearest hundred.

Specific CD4 and CD8 T cell responses to 6 different rhesus macaque Mamu-A*01 class I epitopes encoded in the vaccines are monitored from PBMCs by measuring induction of intracellular cytokines, such as IFN-gamma, using flow cytometry. The results from both methods indicate that cytokines are induced in an antigen-specific manner to epitopes.

## XVIII.B. Evaluation of Immunogenicity in Non-Human Primates (Low and Midrange srRNA Dosing)

This study was designed to (a) evaluate the immunogenicity and preliminary safety of a ChAdV68.5WTnt.MAG25mer priming immunization followed by a VEE-MAG25mer srRNA 100 $\mu$g dose heterologous prime/boost combination; (b) evaluate the kinetics of T-cell responses to the ChAdV68.5WTnt.MAG25-mer srRNA prime/boost combination. This study arm was conducted in mamu A01 Indian rhesus macaques in order to demonstrate immunogenicity. Select antigens used in this study are only recognized in Rhesus macaques, specifically those with a mamu A*01 MHC class I haplotype. Mamu A01 Indian rhesus macaques were randomized to the different study arms (6 macaques per group) and administered an IM injection with either ChAdV68.5WTnt.MAG25mer or VEE-MAG25mer srRNA vector encoding model antigens that includes multiple mamu A01 restricted epitopes. The study arms are as described in Table 21.

This study is also designed evaluate the immunogenicity, preliminary safety, and T-cell response kinetics of VEE-MAG25mer srRNA 30 $\mu$g and 100 $\mu$g doses as a homologous prime/boost as well as compare the immune responses of VEE-MAG25mer srRNA in lipid nanoparticles using LNP1 versus LNP2. These study arms are conducted in a similar fashion to the ChAdV68/srRNA prime/boost described above. The study arms are as described in Table 21.

Table 21 - Low and midrange srRNA dosing NHP immunogenicity study arms

| Group | Prime | Boost 1 | Boost 2 |
|---|---|---|---|
| 1 | VEE-MAG25mer srRNA - LNP1 (30 µg) | VEE-MAG25mer srRNA -LNP1 (30 µg) | VEE-MAG25mer srRNA - LNP1 (30 µg) |
| 2 | VEE-MAG25mer srRNA - LNP1 (100 µg) | VEE-MAG25mer srRNA -LNP1 (100 µg) | VEE-MAG25mer srRNA - LNP1 (100 µg) |
| 3 | VEE-MAG25mer srRNA - LNP2 (100 µg) | VEE-MAG25mer srRNA -LNP2 (100 µg) | VEE-MAG25mer srRNA - LNP2 (100 µg) |
| 4 | ChAdV68.5WTnt.MAG25mer | VEE-MAG25mer srRNA -LNP1 (100 µg) | VEE-MAG25mer srRNA - LNP1 (100 µg) |

PBMCs were collected prior to immunization and every week after the initial immunization for the first 6 weeks for immune monitoring. In addittion, PBMCs are collected 8 and 10 weeks after the initial immunization, for immune monitoring.

Antigen-specific cellular immune responses in peripheral blood mononuclear cells (PBMCs) were measured to six different mamu A01 restricted epitopes prior to immunization and 7, 14, 21, 28 or 35 days after the initial priming immunization with ChAdV68.5WTnt.MAG25mer. Combined immune responses to all six epitopes were plotted for each immune monitoring timepoint (Fig 34 and Table 22). Combined antigen-specific immune responses were observed at all measurements with 1256, 1823, 1905, 987 SFCs per $10^6$ PBMCs (six epitopes combined) 7, 14, 21 or 28 days after the initial ChAdV68.5WTnt.MAG25mer prime immunization, respectively. The immune response showed the expected profile with peak immune responses measured 7-14 days after the prime immunization followed by a contraction in the immune response after 28 days.

Combined antigen-specific cellular immune responses of 1851 SFCs per $10^6$ PBMCs (six epitopes combined) were also measured 7 days after the first boost with VEE-MAG25mer srRNA (i.e. 35 days after the initial immunization with ChAdV68.5WTnt.MAG25mer). The immune response measured 7 days after the first boost with VEE-MAG25mer srRNA (day 35) was comparable to the peak immune response measured for the ChAdV68.5WTnt.MAG25mer prime immunization (day 14) and ~2-fold higher than that measured 28 days after the ChAdV68.5WTnt.MAG25mer prime immunization.

Table 22 - Cellular immune responses with low and midrange srRNA

| Day | Antigen | | | | | |
|---|---|---|---|---|---|---|
| | Tat TL8 | Gag CM9 | Env TL9 | Env CL9 | Gag LW9 | Pol SV9 |
| 0 | 6.6±4.4 | 5.5±5.1 | 7.9±6.7 | 3.5±2.8 | 10.2±7.0 | 4.8±4.1 |
| 7 | 570.1±178.2 | 226.7±119.3 | 214.4±101.0 | 181.5±67.8 | 25.5±14.2 | 38.1±29.9 |
| 14 | 628.0±224.2 | 350.1±112.7 | 286.7±102.0 | 314.7±165.4 | 56.5±19.0 | 186.5±80.2 |
| 21 | 556.0±117.2 | 473.7±106.3 | 367.5±88.5 | 280.8±100.9 | 5 1.9±13.8 | 174.6±60.2 |
| 28 | 328.8±48.3 | 214.4±43.9 | 167.7±48.6 | 143.5±46.6 | 36.7±13.1 | 95.9±32.4 |
| 35 | 545.0±90.2 | 548.1±140.8 | 414.5±92.5 | 159.1±61.6 | 45.2±14.5 | 139.0±52.8 |

**XVIII.C. Evaluation of Immunogenicity in Non-Human Primates (High srRNA Dosing and anti-CTLA4)**

This study was designed to evaluate the impact of route of anti-CTLA4 administration on vaccine induced immune responses (eg, compare local (SC) delivery of anti-CTLA4 in close proximity of the vaccine draining lymph nodes to systemic (IV) administration). This study arm was conducted in mamu A01 Indian rhesus macaques to demonstrate immunogenicity. Vaccine immunogenicity in nonhuman primate species, such as Rhesus, is the best predictor of vaccine potency in humans. Furthermore, select antigens used in this study are only recognized in Rhesus macaques, specifically those with a mamu A*01 MHC class I haplotype. Mamu A01 Indian rhesus macaques were randomized to the different study arms (6 macaques per group) and administered an IM injection with ChAdV68.5WTnt.MAG25mer encoding model antigens that includes multiple mamu A01 restricted antigens. Anti-CTLA-4 was administered SC proximal to the site of vaccine immunization or delivered IV to specified groups. The study arms are described in Table 23

This study is also designed to (a) evaluate the immunogenicity and preliminary safety of VEE-MAG25mer srRNAat a dose of 300 µg as a homologous prime/boost or heterologous prime/boost in combination with ChAdV68.5WTnt.MAG25mer; (b) compare the immune responses of VEE-MAG25mer srRNA in lipid nanoparticles using LNP1 versus LNP2 at the 300 μg dose; and (c) evaluate the kinetics of T-cell responses to VEE-MAG25mer srRNA and ChAdV68.5WTnt.MAG25mer immunizations. These study arems are conducted in mamu A01 Indian rhesus macaques to demonstrate immunogenicity. Vaccine immunogenicity in nonhuman primate species, such as Rhesus, is the best predictor of vaccine potency in humans. Furthermore, select antigens used in this study are only recognized in Rhesus macaques, specifically those with a mamu A*01 MHC class I haplotype. Mamu A01 Indian rhesus macaques are randomized to the different study arms (6 macaques per group) and administered an IM injection with either ChAdV68.5WTnt.MAG25mer or VEE-MAG25mer srRNAencoding model antigens that includes multiple mamu A01 restricted antigens. Anti-CTLA-4 iss administered SC proximal to the site of vaccine immunization or delivered IV to specified groups. The study arms are described in Table 23.

Table 23 - High range srRNA dosing NHP immunogenicity study arms

| Group | Prime | Boost 1 | Boost 2 |
|---|---|---|---|
| 1 | VEE-MAG25mer srRNA-LNP2 (300 μg) | VEE-MAG25mer srRNA -LNP2 (300 μg) | VEE-MAG25mer srRNA -LNP2 (300 μg) |
| 2 | VEE-MAG25mer srRNA-LNP2 (300 μg) +anti-CTLA-4 (SC) | VEE-MAG25mer srRNA -LNP2 (300 μg) +anti-CTLA-4 (SC) | VEE-MAG25mer srRNA -LNP2 (300 μg) +anti-CTLA-4 (SC) |
| 3 | VEE-MAG25mer srRNA-LNP1 (300 μg) | VEE-MAG25mer srRNA -LNP1 (300 μg) | VEE-MAG25mer srRNA -LNP1 (300 μg) |
| 4 | ChAdV68.5WTnt.MAG 25mer | VEE-MAG25mer srRNA -LNP2 (300 μg) | VEE-MAG25mer srRNA -LNP2 (300 μg) |
| 5 | ChAdV68.5WTnt.MAG 25mer +anti-CTLA-4 (SC) | VEE-MAG25mer srRNA -LNP2 (300 μg) +anti-CTLA-4 (SC) | VEE-MAG25mer srRNA -LNP2 (300 μg) +anti-CTLA-4 (SC) |
| 6 | ChAdV68.5WTnt.MAG 25mer +anti-CTLA-4 (IV) | VEE-MAG25mer srRNA -LNP2 (300 μg) +anti-CTLA-4 (IV) | VEE-MAG25mer srRNA -LNP2 (300 μg) +anti-CTLA-4 (IV) |

Mamu A01 Indian rhesus macaques were immunized with ChAdV68.5WTnt.MAG25mer with or without anti-CTLA-4 adminsitered IV or SC. Antigen-specific cellular immune responses in peripheral blood mononuclear cells (PBMCs) were measured to six different mamu A01 restricted epitopes 14 days after the initial immunization and combined immune responses to all six epitopes were plotted (Fig 35 and Table 24). Combined antigen-specific immune responses of 2257, 5887 or 3984 SFCs per $10^6$ PBMCs (six epitopes combined) were observed after a single immunization with ChAdV68.5WTnt.MAG25mer, ChAdV68.5WTnt.MAG25mer with anti-CTLA-4 (IV) or ChAdV68.5WTnt.MAG25mer (SC), respectively.

Table 24 - Cellular immune responses with ChAdV68 and anti-CTLA-4

| | Antigen | | | | | |
|---|---|---|---|---|---|---|
| Group | Tat TL8 | Gag CM9 | Env TL9 | Env CL9 | Gag LW9 | Pol SV9 |
| chAdV | 608.6± 132.6 | 556.7± 136.4 | 478.7± 147.6 | 297.2± 98.3 | 79.8±2 9.9 | 236.4± 66.5 |
| chAdV + anti-CTLA4 IV | 899.8± 287.6 | 1081± 178.7 | 1234± 166.2 | 1360±1 39.8 | 567.6± 265.5 | 744.1± 235.6 |
| chAdV + anti-CTLA4 SC | 995.5± 236.8 | 1149± 158.7 | 629.8± 205.6 | 595.2± 192.6 | 236.1± 71.7 | 378.8± 91.3 |

## Certain Sequences

Sequences for vectors, cassettes, and antibodies are shown below.

| | |
|---|---|
| | Tremelimumab VL (SEQ ID NO:16) |

| | |
|---|---|
| PSSLSASVGDRVTITCRASQSINSYLDWYQQKPGKAPKLLIYAASSLQSGVPSRFSGSGSGTDFTLTI SSLQPEDFATYYCQQYYSTPFTFGPGTKVEIKRTVAAPSVFIFPPSDEQLKSGTASVVCLLNNFYPREAKV | |
| | Tremelimumab VH (SEQ ID NO:17) |
| GVVQPGRSLRLSCAASGFTFSSYGMHWVRQAPGKGLEWVAVIWYDGSNKYYADSVKGRFTISRDNSKN TLYLQMNSLRAEDTAVYYCARDPRGATLYYYYYGMDVWGQGTTVTVSSASTKGPSVFPLAPCSRSTSESTAALG CLVKDYFPEPVTVSWNSGALTSGVH | |
| | Tremelimumab VH CDR1 (SEQ ID NO:18) |
| | GFTFSSYGMH |
| | Tremelimumab VH CDR2 (SEQ ID NO:19) |
| | VIWYDGSNKYYADSV |
| | Tremelimumab VH CDR3 (SEQ ID NO:20) |
| | DPRGATLYYYYYGMDV |
| | Tremelimumab VL CDR1 (SEQ ID NO:21) |
| | RASQSINSYLD |
| | Tremelimumab VL CDR2 (SEQ ID NO:22) |
| | AASSLQS |
| | Tremelimumab VL CDR3 (SEQ ID NO:23) |
| | QQYYSTPFT |
| | |
| Durvalumab (MEDI4736) VL (SEQ ID NO:24) | |
| EIVLTQSPGTLSLSPGERATLSCRASQRVSSSYLAWYQQKPGQAPRLLIYDASSRATGIPDRFSGSGSGTDFTL TISRLEPEDFAVYYCQQYGSLPWTFGQGTKVEIK | |
| MEDI4736 VH (SEQ ID NO:25) | |
| EVQLVESGGGLVQPGGSLRLSCAASGFTFSRYWMSWVRQAPGKGLEWVANIKQDGSEKYYVDSVKGRFTISRDN AKNSLYLQMNSLRAEDTAVYYCAREGGWFGELAFDYWGQGTLVTVSS | |
| MEDI4736 VH CDR1 (SEQ ID NO:26) | |
| RYWMS | |
| MEDI4736 VH CDR2 (SEQ ID NO:27) | |
| NIKQDGSEKYYVDSVKG | |
| MEDI4736 VH CDR3 (SEQ ID NO:28) | |
| | EGGWFGELAFDY |
| | MEDI4736 VL CDR1 (SEQ ID NO:29) |
| | RASQRVSSSYLA |
| | MEDI4736 VL CDR2 (SEQ ID NO:30) |
| | DASSRAT |
| | MEDI4736 VL CDR3 (SEQ ID NO:31) |
| | QQYGSLPWT |

| UbA76-25merPDTT nucleotide (SEQ ID NO:32) |
|---|
| GCCCGGGCATTTAAATGCGATCGCATCGATtacgactctagaatagtctagtccgcaggccaccatgC AGATCTTCGTGAAGACCCTGACCGGCAAGACCATCACCCTAGAGGTGGAGCCCAGTGACACCATCGAGAACGTG AAGGCCAAGATCCAGGATAAAGAGGGCATCCCCCCTGACCAGCAGAGGCTGATCTTTGCCGGCAAGCAGCTGGA AGATGGCCGCACCCTCTCTGATTACAACATCCAGAAGGAGTCAACCCTGCACCTGGTCCTTCGCCTGAGAGGTG cCatgtttcaggcgctgagcgaaggctgcaccccgtatgatattaaccagatgctgaacgtgctgggcgatcat caggtctcaggccttgagcagcttgagagtataatcacttttgaaaaactgactgaatggaccagttctaatgt tatgCCTATCCTGTCTCCTCTGACAAAGGGCATCCTGGGCTTCGTGTTTACCCTGACCGTGCCTTCTGAGAGAG GACTTagctgcattagcgaagcggatgcgaccacccggaaagcgcgaacctgggcgaagaaattctgagccag ctgtatctttggccaaggtgacctaccattcccctagttatgcttaccaccaatttgaaagacgagccaaata taaaagaCACTTCCCCGGCTTTGGCCAGAGCCTGCTGTTTGGCTACCCTGTGTACGTGTTCGGCGATTGCGTGC AGGGCGATtgggatgcgattcgctttcgctattgcgcgccgccgggctatgcgctgctgcgctgcaacgatacc aactatagcgctctgctggctgtgggggccctagaaggacccaggaatcaggactggcttggtgtcccaagaca acttgtaactCGGATGCAGGCTATTCAGAATGCCGGCCTGTGTACCCTGGTGGCCATGCTGGAAGAGACAATCT TCTGGCTGCAAgcgtttctgatggcgctgaccgatagcggcccgaaaaccaacattattgtggatagccagtat gtgatgggcattagcaaaccgagctttcaggaatttgtggattgggaaaacgtgagcccggaactgaacagcac cgatcagccgtttTGGCAAGCCGGAATCCTGGCCAGAAATCTGGTGCCTATGGTGGCCACAGTGCAGGGCCAGA |
| ACCTGAAGTACCAGggtcagtcactagtcatctctgcttctatcattgtcttcaacctgCtggaactggaaggt gattatcgagatgatggcaacgtgtgggtgcataccccgctgagcccgcgcaccctgaacgcgtgggtgaaagc ggtggaagaaaaaaaaggtattccagttcacctagagctggccagtatgaccaacaTggagctcatgagcagta ttgtgcatcagcaggtcAGAACATACGGCCCCGTGTTCATGTGTCTCGGCGGACTGCTTACAATGGTGGCTGGT GCTGTGTGGCTGACAGTGCgagtgctcgagctgttccgggccgcgcagctggccaacgacgtggtcctccagat catggagctttgtggtgcagcgtttcgccaggtgtgccataccaccgtgccgtggccgaacgcgagcctgaccc cgaaatggaacaacgaaaccacccagccccagatcgccaactgcagcgtgtatgactttttttgtgtggctccat tattattctgttcgagacacactttggccaaggtgacctaccatatgaacaaatatgcgtatcatatgctgga aagacgagccaaatataaaagaGGACCAGGACCTGGCGCTAAATTTGTGGCCGCCTGGACACTGAAAGCCGCTG CTGGTCCTGGACCTGGCCAGTACATCAAGGCCAACAGCAAGTTCATCGGCATCACCGAACTCGGACCCGGACCA GGCTGATGATTTCGAAATTTAAATAAGCTTGCGGCCGCTAGGGATAACAGGGTAATtatcacgcccaaacattt acagccgcggtgtcaaaaaccgcgtgg |

| | UbA76-25merPDTT polypeptide (SEQ ID NO:33) |
|---|---|

| MQIFVKTLTGKTITLEVEPSDTIENVKAKIQDKEGIPPDQQRLIFAGKQLEDGRTLSDYNIQKESTLH LVLRLRGAMFQALSEGCTPYDINQMLNVLGDHQVSGLEQLESIINFEKLTEWTSSNVMPILSPLTKGILGFVFT LTVPSERGLSCISEADATTPESANLGEEILSQLYLWPRVTYHSPSYAYHQFERRAKYKRHFPGFGQSLLFGYPV YVFGDCVQGDWDAIRFRYCAPPGYALLRCNDTNYSALLAVGALEGPRNQDWLGVPRQLVTRMQAIQNAGLCTLV AMLEETIFWLQAFLMALTDSGPKTNIIVDSQYVMGISKPSFQEFVDWENVSPELNSTDQPFWQAGILARNLVPM VATVQGQNLKYQGQSLVISASIIVFNLLELEGDYRDDGNVWVHTPLSPRTLNAWVKAVEEKKGIPVHLELASMT NMELMSSIVHQQVRTYGPVFMCLGGLLTMVAGAVWLTVRVLELFRAAQLANDVVLQIMELCGAAFRQVCHTTVP WPNASLTPKWNNETTQPQIANCSVYDFFVWLHYYSVRDTLWPRVTYHMNKYAYHMLERRAKYKRGPGPGAKFVA AWTLKAAAGPGPGQYIKANSKFIGITELGPGPG |
|---|

| | |
|---|---|
| | MAG-25merPDTT nucleotide (SEQ ID NO:34) |

```
ATGGCCGGGATGTTCCAGGCACTGTCCGAAGGCTGCACACCCTATGATATTAACCAGATGCTGAATGTCCTGGG
AGACCACCAGGTCTCTGGCCTGGAGCAGCTGGAGAGCATCATCAACTTCGAGAAGCTGACCGAGTGGACAAGCT
CCAATGTGATGCCTATCCTGTCCCCACTGACCAAGGGCATCCTGGGCTTCGTGTTTACCCTGACAGTGCCTTCT
GAGCGGGGCCTGTCTTGCATCAGCGAGGCAGACGCAACCACACCAGAGTCCGCCAATCTGGGCGAGGAGATCCT
GTCTCAGCTGTACCTGTGGCCCCGGGTGACATATCACTCCCCTTCTTACGCCTATCACCAGTTCGAGCGGAGAG
CCAAGTACAAGAGACACTTCCCAGGCTTTGGCCAGTCTCTGCTGTTCGGCTACCCCGTGTACGTGTTCGGCGAT
TGCGTGCAGGGCGACTGGGATGCCATCCGGTTTAGATACTGCGCACCACCTGGATATGCACTGCTGAGGTGTAA
CGACACCAATTATTCCGCCCTGCTGGCAGTGGGCGCCCTGGAGGGCCCTCGCAATCAGGATTGGCTGGGCGTGC
CAAGGCAGCTGGTGACACGCATGCAGGCCATCCAGAACGCAGGCCTGTGCACCCTGGTGGCAATGCTGGAGGAG
ACAATCTTCTGGCTGCAGGCCTTTCTGATGGCCCTGACCGACAGCGGCCCCAAGACAAACATCATCGTGGATTC
CCAGTACGTGATGGGCATCTCCAAGCCTTCTTTCCAGGAGTTTGTGGACTGGGAGAACGTGAGCCCAGAGCTGA
ATTCCACCGATCAGCCATTCTGGCAGGCAGGAATCCTGGCAAGGAACCTGGTGCCTATGGTGGCCACAGTGCAG
GGCCAGAATCTGAAGTACCAGGGCCAGAGCCTGGTCATCAGCGCCTCCATCATCGTGTTTAACCTGCTGGAGCT
GGAGGGCGACTATCGGGACGATGGCAACGTGTGGGTGCACACCCCACTGAGCCCCAGAACACTGAACGCCTGGG
TGAAGGCCGTGGAGGAGAAGAAGGGCATCCCAGTGCACCTGGAGCTGGCCTCCATGACCAATATGGAGCTGATG
TCTAGCATCGTGCACCAGCAGGTGAGGACATACGGACCCGTGTTCATGTGCCTGGGAGGCCTGCTGACCATGGT
GGCAGGAGCCGTGTGGCTGACAGTGCGGGTGCTGGAGCTGTTCAGAGCCGCCCAGCTGGCCAACGATGTGGTGC
TGCAGATCATGGAGCTGTGCGGAGCAGCCTTTCGCCAGGTGTGCCACACCACAGTGCCATGGCCCAATGCCTCC
CTGACCCCCAAGTGGAACAATGAGACAACACAGCCTCAGATCGCCAACTGTAGCGTGTACGACTTCTTCGTGTG
GCTGCACTACTATAGCGTGAGGGATACCCTGTGGCCCCGCGTGACATACCACATGAATAAGTACGCCTATCACA
TGCTGGAGAGGCGCGCCAAGTATAAGAGAGGCCCTGGCCCAGGCGCAAAGTTTGTGGCAGCATGGACCCTGAAG
GCCGCCGCCGGCCCCGGCCCCGGCCAGTATATCAAGGCTAACAGTAAGTTCATTGGAATCACAGAGCTGGGACC
CGGACCTGGA
```

| | MAG-25merPDTT polypeptide (SEQ ID NO:35) |
| --- | --- |

```
        MAGMFQALSEGCTPYDINQMLNVLGDHQVSGLEQLESIINFEKLTEWTSSNVMPILSPLTKGILGFVF
TLTVPSERGLSCISEADATTPESANLGEEILSQLYLWPRVTYHSPSYAYHQFERRAKYKRHFPGFGQSLLFGYP
VYVFGDCVQGDWDAIRFRYCAPPGYALLRCNDTNYSALLAVGALEGPRNQDWLGVPRQLVTRMQAIQNAGLCTL
VAMLEETIFWLQAFLMALTDSGPKTNIIVDSQYVMGISKPSFQEFVDWENVSPELNSTDQPFWQAGILARNLVP
MVATVQGQNLKYQGQSLVISASIIVFNLLELEGDYRDDGNVWVHTPLSPRTLNAWVKAVEEKKGIPVHLELASM
TNMELMSSIVHQQVRTYGPVFMCLGGLLTMVAGAVWLTVRVLELFRAAQLANDVVLQIMELCGAAFRQVCHTTV
PWPNASLTPKWNNETTQPQIANCSVYDFFVWLHYYSVRDTLWPRVTYHMNKYAYHMLERRAKYKRGPGPGAKFV
AAWTLKAAAGPGPGQYIKANSKFIGITELGPGPG
```

| | |
| --- | --- |
| | Ub7625merPDTT_NoSFL nucleotide (SEQ ID NO:36) |

```
        GCCCGGGCATTTAAATGCGATCGCATCGATtacgactctagaatagtctagtccgcaggccaccatgC
AGATCTTCGTGAAGACCCTGACCGGCAAGACCATCACCCTAGAGGTGGAGCCCAGTGACACCATCGAGAACGTG
AAGGCCAAGATCCAGGATAAAGAGGGCATCCCCCCTGACCAGCAGAGGCTGATCTTTGCCGGCAAGCAGCTGGA
```

(continued)

AGATGGCCGCACCCTCTCTGATTACAACATCCAGAAGGAGTCAACCCTGCACCTGGTCCTTCGCCTGAGAGGTG
cCatgtttcaggcgctgagcgaaggctgcaccccgtatgatattaaccagatgctgaacgtgctgggcgatcat
cagtttaagcacatcaaagcctttgaccggacatttgctaacaacccaggtcccatggttgtgtttgccacacc
tgggCCTATCCTGTCTCCTCTGACAAAGGGCATCCTGGGCTTCGTGTTTACCCTGACCGTGCCTTCTGAGAGAG
GACTTagctgcattagcgaagcggatgcgaccaccccggaaagcgcgaacctgggcgaagaaattctgagccag
ctgtatctttggccaaggggtgacctaccattcccctagttatgcttaccaccaatttgaaagacgagccaaata
taaaagaCACTTCCCCGGCTTTGGCCAGAGCCTGCTGTTTGGCTACCCTGTGTACGTGTTCGGCGATTGCGTGC
AGGGCGATtgggatgcgattcgctttcgctattgcgcgccgccgggctatgcgctgctgcgctgcaacgatacc
aactatagcgctctgctggctgtggggggccctagaaggacccaggaatcaggactggcttggtgtcccaagaca
acttgtaactCGGATGCAGGCTATTCAGAATGCCGGCCTGTGTACCCTGGTGGCCATGCTGGAAGAGACAATCT
TCTGGCTGCAAgcgtttctgatggcgctgaccgatagcggcccgaaaaccaacattattgtggatagccagtat
gtgatgggcattagcaaaccgagctttcaggaatttgtggattgggaaaacgtgagcccggaactgaacagcac
cgatcagccgtttTGGCAAGCCGGAATCCTGGCCAGAAATCTGGTGCCTATGGTGGCCACAGTGCAGGGCCAGA
ACCTGAAGTACCAGggtcagtcactagtcatctctgcttctatcattgtcttcaacctgCtggaactggaaggt
gattatcgagatgatggcaacgtgtgggtgcatacccccgctgagcccgcgcaccctgaacgcgtgggtgaaagc
ggtggaagaaaaaaaaggtattccagttcacctagagctggccagtatgaccaacaTggagctcatgagcagta
ttgtgcatcagcaggtcAGAACATACGGCCCCGTGTTCATGTGTCTCGGCGGACTGCTTACAATGGTGGCTGGT
GCTGTGTGGCTGACAGTGcgagtgctcgagctgttccgggccgcgcagctggccaacgacgtggtcctccagat
catggagctttgtggtgcagcgtttcgccaggtgtgccataccaccgtgccgtggccgaacgcgagcctgaccc
cgaaatggaacaacgaaaccacccagccccagatcgccaactgcagcgtgtatgactttttttgtgtggctccat
tattattctgttcgagacacactttggccaaggggtgacctaccatatgaacaaatatgcgtatcatatgctgga
aagacgagccaaatataaaagaGGACCAGGACCTGGCGCTAAATTTGTGGCCGCCTGGACACTGAAAGCCGCTG
CTGGTCCTGGACCTGGCCAGTACATCAAGGCCAACAGCAAGTTCATCGGCATCACCGAACTCGGACCCGGACCA
GGCTGATGATTTCGAAATTTAAATAAGCTTGCGGCCGCTAGGGATAACAGGGTAATtatcacgcccaaacattt
acagccgcggtgtcaaaaaccgcgtgg

|  |  |
|---|---|
|  | Ub7625merPDTT_NoSFL poly-peptide (SEQ ID NO:37) |

MQIFVKTLTGKTITLEVEPSDTIENVKAKIQDKEGIPPDQQRLIFAGKQLEDGRTLSDYNIQKESTLH
LVLRLRGAMFQALSEGCTPYDINQMLNVLGDHQFKHIKAFDRTFANNPGPMVVFATPGPILSPLTKGILGFVFT
LTVPSERGLSCISEADATTPESANLGEEILSQLYLWPRVTYHSPSYAYHQFERRAKYKRHFPGFGQSLLFGYPV
YVFGDCVQGDWDAIRFRYCAPPGYALLRCNDTNYSALLAVGALEGPRNQDWLGVPRQLVTRMQAIQNAGLCTLV
AMLEETIFWLQAFLMALTDSGPKTNIIVDSQYVMGISKPSFQEFVDWENVSPELNSTDQPFWQAGILARNLVPM
VATVQGQNLKYQGQSLVISASIIVFNLLELEGDYRDDGNVWVHTPLSPRTLNAWVKAVEEKKGIPVHLELASMT
NMELMSSIVHQQVRTYGPVFMCLGGLLTMVAGAVWLTVRVLELFRAAQLANDVVLQIMELCGAAFRQVCHTTVP
WPNASLTPKWNNETTQPQIANCSVYDFFVWLHYYSVRDTLWPRVTYHMNKYAYHMLERRAKYKRGPGPGAKFVA
AWTLKAAAGPGPGQYIKANSKFIGITELGPGPG

ChAdV68.5WTnt.MAG25mer (SEQ ID NO:2); AC_000011.1 with E1 (nt 577 to 3403) and E3 (nt 27,125- 31,825) sequences deleted; corresponding ATCC VR-594 nucleotides substituted at five positions; model neoantigen cassette under the control of the CMV promoter/enhancer inserted in place of deleted E1; SV40 polyA 3' of cassette

```
          CCATCTTCAATAATATACCTCAAACTTTTTGTGCGCGTTAATATGCAAATGAGGCGTTTGAATTTGGG
GAGGAAGGGCGGTGATTGGTCGAGGGATGAGCGACCGTTAGGGGCGGGGCGAGTGACGTTTTGATGACGTGGTT
GCGAGGAGGAGCCAGTTTGCAAGTTCTCGTGGGAAAAGTGACGTCAAACGAGGTGTGGTTTGAACACGGAAATA
CTCAATTTTCCCGCGCTCTCTGACAGGAAATGAGGTGTTTCTGGGCGGATGCAAGTGAAAACGGGCCATTTTCG
CGCGAAAACTGAATGAGGAAGTGAAAATCTGAGTAATTTCGCGTTTATGGCAGGGAGGAGTATTTGCCGAGGGC
CGAGTAGACTTTGACCGATTACGTGGGGGGTTTCGATTACCGTGTTTTTCACCTAAATTTCCGCGTACGGTGTCA
AAGTCCGGTGTTTTTACGTAGGTGTCAGCTGATCGCCAGGGTATTTAAACCTGCGCTCTCCAGTCAAGAGGCCA
CTCTTGAGTGCCAGCGAGAAGAGTTTTCTCCTCCGCGCCGCGAGTCAGATCTACACTTTGAAAGTAGGGATAAC
AGGGTAATgacattgattattgactagttGttaaTAGTAATCAATTACGGGGTCATTAGTTCATAGCCCATATA
TGGAGTTCCGCGTTACATAACTTACGGTAAATGGCCCGCCTGGCTGACCGCCCAACGACCCCCGCCCATTGACG
TCAATAATGACGTATGTTCCCATAGTAACGCCAATAGGGACTTTCCATTGACGTCAATGGGTGGAGTATTTACG
GTAAACTGCCCACTTGGCAGTACATCAAGTGTATCATATGCCAAGTCCGCCCCCTATTGACGTCAATGACGGTA
AATGGCCCGCCTGGCATTATGCCCAGTACATGACCTTACGGGACTTTCCTACTTGGCAGTACATCTACGTATTA
GTCATCGCTATTACCATGgTGATGCGGTTTTGGCAGTACACCAATGGGCGTGGATAGCGGTTTGACTCACGGGG
ATTTCCAAGTCTCCACCCCATTGACGTCAATGGGAGTTTGTTTTGGCACCAAAATCAACGGGACTTTCCAAAAT
GTCGTAATAACCCCGCCCCGTTGACGCAAATGGGCGGTAGGCGTGTACGGTGGGAGGTCTATATAAGCAGAgcT
CGTTTAGTGAACCGTCAGATCGCCTGGAACGCCATCCACGCTGTTTTGACCTCCATAGAAGACAGCGATCGCGc
caccATGGCCGGGATGTTCCAGGCACTGTCCGAAGGCTGCACACCCTATGATATTAACCAGATGCTGAATGTCC
TGGGAGACCACCAGGTCTCTGGCCTGGAGCAGCTGGAGAGCATCATCAACTTCGAGAAGCTGACCGAGTGGACA
AGCTCCAATGTGATGCCTATCCTGTCCCCACTGACCAAGGGCATCCTGGGCTTCGTGTTTACCCTGACAGTGCC
TTCTGAGCGGGGCCTGTCTTGCATCAGCGAGGCAGACGCAACCACACCAGAGTCCGCCAATCTGGGCGAGGAGA
```

```
TCCTGTCTCAGCTGTACCTGTGGCCCCGGGTGACATATCACTCCCCTTCTTACGCCTATCACCAGTTCGAGCGG
AGAGCCAAGTACAAGAGACACTTCCCAGGCTTTGGCCAGTCTCTGCTGTTCGGCTACCCCGTGTACGTGTTCGG
CGATTGCGTGCAGGGCGACTGGGATGCCATCCGGTTTAGATACTGCGCACCACCTGGATATGCACTGCTGAGGT
GTAACGACACCAATTATTCCGCCCTGCTGGCAGTGGGCGCCCTGGAGGGCCCTCGCAATCAGGATTGGCTGGGC
GTGCCAAGGCAGCTGGTGACACGCATGCAGGCCATCCAGAACGCAGGCCTGTGCACCCTGGTGGCAATGCTGGA
GGAGACAATCTTCTGGCTGCAGGCCTTTCTGATGGCCCTGACCGACAGCGGCCCCAAGACAAACATCATCGTGG
ATTCCCAGTACGTGATGGGCATCTCCAAGCCTTCTTTCCAGGAGTTTGTGGACTGGGAGAACGTGAGCCCAGAG
CTGAATTCCACCGATCAGCCATTCTGGCAGGCAGGAATCCTGGCAAGGAACCTGGTGCCTATGGTGGCCACAGT
GCAGGGCCAGAATCTGAAGTACCAGGGCCAGAGCCTGGTCATCAGCGCCTCCATCATCGTGTTTAACCTGCTGG
AGCTGGAGGGCGACTATCGGGACGATGGCAACGTGTGGGTGCACACCCCACTGAGCCCCAGAACACTGAACGCC
TGGGTGAAGGCCGTGGAGGAGAAGAAGGGCATCCCAGTGCACCTGGAGCTGGCCTCCATGACCAATATGGAGCT
GATGTCTAGCATCGTGCACCAGCAGGTGAGGACATACGGACCCGTGTTCATGTGCCTGGGAGGCCTGCTGACCA
TGGTGGCAGGAGCCGTGTGGCTGACAGTGCGGGTGCTGGAGCTGTTCAGAGCCGCCCAGCTGGCCAACGATGTG
GTGCTGCAGATCATGGAGCTGTGCGGAGCAGCCTTTCGCCAGGTGTGCCACACCACAGTGCCATGGCCCAATGC
CTCCCTGACCCCCAAGTGGAACAATGAGACAACACAGCCTCAGATCGCCAACTGTAGCGTGTACGACTTCTTCG
TGTGGCTGCACTACTATAGCGTGAGGGATACCCTGTGGCCCCGCGTGACATACCACATGAATAAGTACGCCTAT
CACATGCTGGAGAGGCGCGCCAAGTATAAGAGAGGCCCTGGCCCAGGCGCAAAGTTTGTGGCAGCATGGACCCT
GAAGGCCGCCGCCGGCCCCGGCCCCGGCCAGTATATCAAGGCTAACAGTAAGTTCATTGGAATCACAGAGCTGG
GACCCGGACCTGGATAATGAGTTTAAACTCCCATTTAAATGTGAGGGTTAATGCTTCGAGCAGACATGATAAGA
TACATTGATGAGTTTGGACAAACCACAACTAGAATGCAGTGAAAAAAATGCTTTATTTGTGAAATTTGTGATGC
TATTGCTTTATTTGTAACCATTATAAGCTGCAATAAACAAGTTAACAACAACAATTGCATTCATTTTATGTTTC
AGGTTCAGGGGGAGATGTGGGAGGTTTTTTAAAGCAAGTAAAACCTCTACAAATGTGGTAAAATAACTATAACG
GTCCTAAGGTAGCGAGTGAGTAGTGTTCTGGGGCGGGGGAGGACCTGCATGAGGGCCAGAATAACTGAAATCTG
TGCTTTTCTGTGTGTTGCAGCAGCATGAGCGGAAGCGGCTCCTTTGAGGGGAGGGGTATTCAGCCCTTATCTGAC
GGGGCGTCTCCCCTCCTGGGCGGGAGTGCGTCAGAATGTGATGGGATCCACGGTGGACGGCCGGCCCGTGCAGC
CCGCGAACTCTTCAACCCTGACCTATGCAACCCTGAGCTCTTCGTTCGGTGACGCAGCTGCCGCCGCAGCTGCT
GCATCTGCCGCCAGCGCCGTGCCGCGGAATGGCCATGGGCGCCGCCGGCTACTACGGCACTCTGGTGGCCAACTCGAG
TTCCACCAATAATCCCGCCAGCCTGAACGAGGAGAAGCTGTTGCTGCTGATGGCCCAGCTCGAGGCCTTGACCC
AGCGCCTGGGCGAGCTGACCCAGCAGGTGGCTCAGCTGCAGGAGCAGACGCGGGCCGCGGTTGCCACGGTGAAA
TCCAAATAAAAAATGAATCAATAAATAAACGGAGACGGTTGTTGATTTTAACACAGAGTCTGAATCTTTATTTG
ATTTTTCGCGCGCGGTAGGCCCTGGACCACCGGTCTCGATCATTGAGCACCCGGTGGATCTTTTCCAGGACCCG
GTAGAGGTGGGCTTGGATGTTGAGGTACATGGGCATGAGCCCGTCCCGGGGGTGGAGGTAGCTCCATTGCAGGG
CCTCGTGCTCGGGGGTGGTGTTGTAAATCACCCAGTCATAGCAGGGGCGCAGGGCATGGTGTTGCACAATATCT
TTGAGGAGGAGACTGATGGCCACGGGCAGCCCTTTGGTGTAGGTGTTTACAAATCTGTTGAGCTGGGAGGGATG
CATGCGGGGGGAGATGAGGTGCATCTTGGCCTGGATCTTGAGATTGGCGATGTTACCGCCCAGATCCCGCCTGG
GGTTCATGTTGTGCAGGACCACCAGCACGGTGTATCCGGTGCACTTGGGGAATTTATCATGCAACTTGGAAGGG
AAGGCGTGAAAGAATTTGGCGACGCCTTTGTGCCCGCCCAGGTTTTCCATGCACTCATCCATGATGATGGCGAT
GGGCCCGTGGGCGGCGGCCTGGGCAAAGACGTTTCGGGGGTCGGACACATCATAGTTGTGGTCCTGGGTGAGGT
CATCATAGGCCATTTTAATGAATTTGGGGCGGAGGGTGCCGGACTGGGGGACAAAGGTACCCTCGATCCCGGGG
GCGTAGTTCCCCTCACAGATCTGCATCTCCCAGGCTTTGAGCTCGGAGGGGGGGATCATGTCCACCTGCGGGGC
GATAAAGAACACGGTTTCCGGGGCGGGGGAGATGAGCTGGGCCGAAAGCAAGTTCCGGAGCAGCTGGGACTTGC
CGCAGCCGGTGGGGCCGTAGATGACCCCGATGACCGGCTGCAGGTGGTAGTTGAGGGAGAGACAGCTGCCGTCC
TCCCGGAGGAGGGGGGCCACCTCGTTCATCATCTCGCGCACGTGCATGTTCTCGCGCACCAGTTCCGCCAGGAG
GCGCTCTCCCCCCAGGGATAGGAGCTCCTGGAGCGAGGCGAAGTTTTTCAGCGGCTTGAGTCCGTCGGCCATGG
GCATTTTGGAGAGGGTTTGTTGCAAGAGTTCCAGGCGGTCCCAGAGCTCGGTGATGTGCTCTACGGCATCTCGA
TCCAGCAGACCTCCTCGTTTCGCGGGTTGGGACGGCTGCGGGAGTAGGGCACCAGACGATGGCGTCCAGCGCA
GCCAGGGTCCGGTCCTTCCAGGGTCGCAGCGTCCGCGTCAGGGTGGTCTCCGTCACGGTGAAGGGGTGCGCGCC
GGGCTGGGCGCTTGCGAGGGTGCGCTTCAGGCTCATCCGGCTGGTCGAAAACCGCTCCCGATCGGCGCCCTGCG
CGTCGGCCAGGTAGCAATTGACCATGAGTTCGTAGTTGAGCGCCTCGGCCCGCGTGGCCTTTGGCGCGGAGCTTA
CCTTTGGAAGTCTGCCCGCAGGCGGGACAGAGGAGGGACTTGAGGGCGTAGAGCTTGGGGGCGGAGGAAGACGGA
CTCGGGGGCGTAGGCGTCCGCGCCGCAGTGGGCGCAGACGGTCTCGCACTCCACGAGCCAGGTGAGGTCGGGCT
GGTCGGGGTCAAAAACCAGTTTCCCGCCGTTCTTTTTGATGCGTTTCTTACCTTTGGTCTCCATGAGCTCGTGT
CCCCGCTGGGTGACAAAGAGGCTGTCCGTGTCCCCGTAGACCGACTTTATGGGCCGGTCCTCGAGCGGTGTGCC
GCGGTCCTCCTCGTAGAGGAACCCCGCCCACTCCGAGACGAAAGCCCGGGTCCAGGCCAGCACGAAGGAGGCCA
CGTGGGACGGGTAGCGGTCGTTGTCCACCAGCGGGTCCACCTTTTCCAGGGTATGCAAACACATGTCCCCCTCG
TCCACATCCAGGAAGGTGATTGGCTTGTAAGTGTAGGCCACGTGACCGGGTGTTCCTGAAGGGGGGCTATAAAA
GGGTGCGGGTCCCTGCTCGTCCTCACTGTCTTCCGGATCGCTGTCCAGGAGCGCCAGCTGTTGGGGTAGGTATT
CCCTCTCGAAGGCGGGCATGACCTCGGCACTCAGGTTGTCAGTTTCTAGAAACGAGGAGGATTTGATATTCACG
GTGCCGGCGGAGATGCCTTTCAAGAGCCCCTCGTCCATCTGGTCAGAAAAGACGATCTTTTTGTTGTCGAGCTT
GGTGGCGAAGGAGCCGTAGAGGGCGTTGGAGAGGAGCTTGGCGATGGAGCGCATGGTCTGGTTTTTTTTCCTTGT
CGGCGCGCTCCTTGGCGGCGATGTTGAGCTGCACGTACTCGCGCGCCACGCACTTCCATTCGGGGAAGACGGTG
GTCAGCTCGTCGGGCACGATTCTGACCTGCCAGCCCCGATTATGCAGGGTGATGAGGTCCACACTGGTGGCCAC
```

```
CTCGCCGCGCAGGGGCTCATTAGTCCAGCAGAGGCGTCCGCCCTTGCGCGAGCAGAAGGGGGGCAGGGGGTCCA
GCATGACCTCGTCGGGGGGGTCGGCATCGATGGTGAAGATGCCGGGCAGGAGGTCGGGGTCAAAGTAGCTGATG
GAAGTGGCCAGATCGTCCAGGGCAGCTTGCCATTCGCGCACGGCCAGCGCGCGCTCGTAGGGACTGAGGGGCGT
GCCCCAGGGCATGGGATGGGTAAGCGCGGAGGCGTACATGCCGCAGATGTCGTAGACGTAGAGGGGCTCCTCGA
GGATGCCGATGTAGGTGGGGTAGCAGCGCCCCCCGCGGATGCTGGCGCGCACGTAGTCATACAGCTCGTGCGAG
GGGGCGAGGAGCCCCGGGCCCAGGTTGGTGCGACTGGGCTTTTCGGCGCGGTAGACGATCTGGCGGAAAATGGC
ATGCGAGTTGGAGGAGATGGTGGGCCTTTGGAAGATGTTGAAGTGGGCGTGGGGCAGTCCGACCGAGTCGCGGA
TGAAGTGGGCGTAGGAGTCTTGCAGCTTGGCGACGAGCTCGGCGGTGACTAGGACGTCCAGAGCGCAGTAGTCG
AGGGTCTCCTGGATGATGTCATACTTGAGCTGTCCCTTTTGTTTCCACAGCTCGCGGTTGAGAAGGAACTCTTC
GCGGTCCTTCCAGTACTCTTCGAGGGGGGAACCCGTCCTGATCTGCACGGTAAGAGCCTAGCATGTAGAACTGGT
TGACGGCCTTGTAGGCGCAGCAGCCCTTCTCCACGGGGAGGGCGTAGGCCTGGGCGGCCTTGCGCAGGGAGGTG
TGCGTGAGGGCGAAAGTGTCCCTGACCATGACCTTGAGGAACTGGTGCTTGAAGTCGATATCGTCGCAGCCCCC
CTGCTCCCAGAGCTGGAAGTCCGTGCGCTTCTTGTAGGCGGGGTTGGGCAAAGCGAAAGTAACATCGTTGAAGA
GGATCTTGCCCGCGCGGGGCATAAAGTTGCGAGTGATGCGGAAAGGTTGGGGCACCTCGGCCCGGTTGTTGATG
ACCTGGGCGGCGAGCACGATCTCGTCGAAGCCGTTGATGTTGTGGCCCACGATGTAGAGTTCCACGAATCGCGG
ACGGCCCTTGACGTGGGGCAGTTTCTTGAGCTCCTCGTAGGTGAGCGTCGTCGGGGTCGCTGAGCCCGTGCTGCT
CGAGCGCCCAGTCGGCGAGATGGGGGTTGGCGCGGAGGAAGGAAGTCCAGAGATCCACGGCCAGGGCGGTTTGC
AGACGGTCCCGGTACTGACGGAACTGCTGCCCGACGGCCATTTTTTCGGGGGTGACGCAGTAGAAGGTGCGGGG
GTCCCCGTGCCAGCGATCCCATTTGAGCTGGAGGGCGAGATCGAGGGCGAGCTCGACGAGCCGGTCGTCCCCGG
AGAGTTTCATGACCAGCATGAAGGGGACGAGCTGCTTGCCGAAGGACCCCATCCAGGTGTAGGTTTCCACATCG
TAGGTGAGGAAGAGCCTTTCGGTGCGAGGATGCGAGCCGATGGGGAAGAACTGGATCTCCTGCCACCAATTGGA
GGAATGGCTGTTGATGTGATGGAAGTAGAAATGCCGACGGCGCGCCGAACACTCGTGCTTGTGTTTATACAAGC
GGCCACAGTGCTCGCAACGCTGCACGGGATGCACGTGCTGCACGAGCTGTACCTGAGTTCCTTTGACGAGGAAT
TTCAGTGGGAAGTGGAGTCGTGGCGCCTGCATCTCGTGCTGTACTACGTCGTGGTGGTCGGCCTGGCCCTCTTC
TGCCTCGATGGTGGTCATGCTGACGAGCCCGCGCGGGAGGCAGGTCCAGACCTCGGCGCGAGCGGGTCGGAGAG
CGAGGACGAGGGCGCGCAGGCCGGAGCTGTCCAGGGTCCTGAGCACGCTGCGGAGTCAGGTCAGTGGGCAGCGGC
GGCGCGCGGTTGACTTGCAGGAGTTTTTCCAGGGCGCGGGGAGGTCCAGATGGTACTTGATCTCCACCGCGCC
ATTGGTGGCGACGTCGATGGCTTGCAGGGTCCCGTGCCCCTGGGGTGTGACCACCGTCCCCGTTTCTTCTTGG
GCGGCTGGGGCGACGGGGGCGGTGCCTCTTCCATGGTTAGAAGCGGCGGCGAGGACGCGCGCCGGGCGGCAGGG
GCGGCTCGGGGCCCGGAGGCAGGGGCGGCAGGGGCACGTCGGCGCCGCGCGCGGGTAGGTTCTGGTACTGCGCC
CGGAGAAGACTGGCGTGAGCGACGACGCGACGGTTGACGTCCTGGATCTGACGCCTCTGGGTGAAGGCCACGGG
ACCCGTGAGTTTGAACCTGAAAGAGAGTTCGACAGAATCAATCTCGGTATCGTTGACGGCGGCCTGCCGCAGGA
TCTCTTGCACGTCGCCCGAGTTGTCCTGGTAGGCGATCTCGGTCATGAACTGCTCGATCTCCTCCTCTTGAAGG
TCTCCGCGGCCGGCGTCCACGGTGGCCGCGAGGTCGTTGGAGATGCGGCCCATGAGCTGCGAGAAGGCGTT
CATGCCCGCCTCGTTCCAGACGCGGCTGTAGACCACGACGCCCTCGGGATCGCgGGCGCGCATGACCACCTGGG
CGAGGTTGAGCTCCACGTGGCGCGTGAAGACCGCGTAGTTGCAGAGGCGCTGGTAGAGGTAGTTGAGCGTGGTG
GCGATGTGCTCGGTGACGAAGAAATACATGATCCAGCGGCGGAGCGGCATCTCGCTGACGTCGCCCAGCGCCTC
CAAACGTTCCATGGCCTCGTAAAAGTCCACGGCGAAGTTGAAAAACTGGGAGTTGCGCGCCGAGACGGTCAACT
CCTCCTCCAGAAGACGGATGAGCTCGGCGATGGTGGCGCGCACCTCGCGCTCGAAGGCCCCCGGGAGTTCCTCC
ACTTCCTCTTCTTCCTCCTCCACTAACATCTCTTCTACTTCCTCCTCAGGCGGCAGTGGTGGCGGGGGAGGGGG
CCTGCGTCGCCGGCGGCGCACGGGCAGACGGTCGATGAAGCGCTCGATGGTCTCGCCGCGCCGGCGTCGCATGG
TCTCGGTGACGGCGCGCCCGTCCTCGCGGGGCCGCAGCGTGAAGACGCCGCCGCGCATCTCCAGGTGGCCGGGG
GGGTCCCCGTTGGGCAGGGAGAGGGCGCTGACGATGCATCTTATCAATTGCCCCGTAGGGACTCCGCGCAAGGA
CCTGAGCGTCTCGAGATCCACGGGATCTGAAAACCGCTGAACGAAGGCTTCGAGCCAGTCGCAGTCGCAAGGTA
GGCTGAGCACGGTTTCTTCTGGCGGGTCATGTTGGTTGGGAGCGGGGGCGGGCGATGCTGCTGGTGATGAAGTTG
AAATAGGCGGTTCTGAGACGGCGGATGGTGGCGAGGAGCACCAGGTCTTTGGGCCCGGCTTGCTGGATGCGCAG
ACGGTCGGCCATGCCCCAGGCGTGGTCCTGACACCTGGCCAGGTCCTTGTAGTAGTCCTGCATGAGCCGCTCCA
CGGGCACCTCCTCCTCGCCCGCGGGCCGTGCATGCGCGTGAGCCCGAAGCCGCGCTGGGGCTGGACGAGCGCC
AGGTCGGCGACGACGCGCTCGGCGAGGATGGCTTGCTGGATCTGGGTGAGGGTGGTCTGGAAGTCATCAAAGTC
GACGAAGCGGTGGTAGGCTCCGGTGTTGATGGTGTAGGAGCAGTTGGCCATGACGGACCAGTTGACGGTCTGGT
GGCCCGGACGCACGAGCTCGTGGTACTTGAGGCGCGAGTAGGCGCGCGTGTCGAAGATGTAGTCGTTGCAGGTG
CGCACCAGGTACTGGTAGCCGATGAGGAAGTGCGGCGGCGGCTGGCGGTAGAGCGGCCATCGCTCGGTGGCGGG
GGCGCCGGGCGCGAGGTCCTCGAGCATGGTGCGGTGGTAGCCGTAGATGTACCTGGACATCCAGGTGATGCCGG
CGGCGGTGGTGGAGGCGCGCGGGAACTCGCGGACGCGGTTCCAGATGTTGCGCAGCGGCAGGAAGTAGTTCATG
GTGGGCACGGTCTGGCCCGTGAGGCGCGCGCAGTCGTGGATGCTCTATACGGGCAAAAACGAAAGCGGTCAGCG
GCTCGACTCCGTGGCCGTGGAGGCTAAGCGAACGGGTTGGGCTGCGCGTGTACCCCGGTTCGAATCTCGAATCAG
GCTGGAGCCGCAGCTAACGTGGTATTGGCACTCCCGTCTCGACCCAAGCCTGCACCAACCCTCCAGGATACGGA
GGCGGGTCGTTTTGCAACTTTTTTTTGGAGGCCGGATGAGACTAGTAAGCGCGGAAAGCGGCCGACCGCGATGG
CTCGCTGCCGTAGTCTGGAGAAGAATCGCCAGGGTTGCGTTGCGGTGTGCCCGGTTCGAGGCCGGCCGGATTC
CGCGGCTAACGAGGGCGTGGCTGCCCGTCGTTTCCAAGACCCCATAGCCAGCCGACTTCTCCAGTTACGGAGC
GAGCCCCTCTTTTGTTTTGTTTGTTTTTGCCAGATGCATCCCGTACTGCGGCAGATGCGCCCCCACCACCCTCC
ACCGCAACAACAGCCCCCTCCACAGCCGGCGCTTCTGCCCCCGCCCCAGCAGCAACTTCCAGCCACGACCGCCG
```

(continued)

```
CGGCCGCCGTGAGCGGGGCTGGACAGAGTTATGATCACCAGCTGGCCTTGGAAGAGGGCGAGGGGCTGGCGCGC
CTGGGGGCGTCGTCGCCGGAGCGGCACCCGCGCGTGCAGATGAAAAGGGACGCTCGCGAGGCCTACGTGCCCAA
GCAGAACCTGTTCAGAGACAGGAGCGGCGAGGAGCCCGAGGAGATGCGCGCGGCCCGGTTCCACGCGGGGCGGG
AGCTGCGGCGCGGCCTGGACCGAAAGAGGGTGCTGAGGGACGAGGATTTCGAGGCGGACGAGCTGACGGGGATC
AGCCCCGCGCGCGCGCACGTGGCCGCGGCCAACCTGGTCACGGCGTACGAGCAGACCGTGAAGGAGGAGGAGCAA
CTTCCAAAAATCCTTCAACAACCACGTGCGCACCCTGATCGCGCGCGAGGAGGTGACCCTGGGCCTGATGCACC
TGTGGGACCTGCTGGAGGCCATCGTGCAGAACCCCACCAGCAAGCCGCTGACGGCGCAGCTGTTCCTGGTGGTG
CAGCATAGTCGGGACAACGAAGCGTTCAGGGAGGCGCTGCTGAATATCACCGAGCCCGAGGGCCGCTGGCTCCT
GGACCTGGTGAACATTCTGCAGAGCATCGTGGTGCAGGAGCGCGGGCTGCCGCTGTCCGAGAAGCTGGCGGCCA
TCAACTTCTCGGTGCTGAGTTTGGGCAAGTACTACGCTAGGAAGATCTACAAGACCCCGTACGTGCCCATAGAC
AAGGAGGTGAAGATCGACGGGTTTTACATGCGCATGACCCTGAAAGTGCTGACCCTGAGCGACGATCTGGGGGT
GTACCGCAACGACAGGATGCACCGTGCGGTGAGCGCCAGCAGGCGGCGCGAGCTGAGCGACCAGGAGCTGATGC
ATAGTCTGCAGCGGGCCCTGACCGGGGCCGGGACCGAGGGGGAGAGCTACTTTGACATGGGCGCGGACCTGCAC
TGGCAGCCCAGCCGCCGGGCCTTGGAGGCGGCGGCAGGACCCTACGTAGAAGAGGTGGACGATGAGGTGGACGA
GGAGGGCGAGTACCTGGAAGACTGATGGCGCGACCGTATTTTTGCTAGATGCAACAACAACAGCCACCTCCTGA
TCCCGCGATGCGGGCGGCGCTGCAGAGCCAGCCGTCCGGCATTAACTCCTCGGACGATTGGACCCAGGCCATGC
AACGCATCATGGCGCTGACGACCCGCAACCCCGAAGCCTTTAGACAGCAGCCCCAGGCCAACCGGCTCTCGGCC
ATCCTGGAGGCCGTGGTGCCCTCGCGCTCCAACCCCACGCACGAGAAGGTCCTGGCCATCGTGAACGCGCTGGT
GGAGAACAAGGCCATCCGCGGCGACGAGGCCGGCCTGGTGTACAACGCGCTGCTGGAGCGCGTGGCCCGCTACA
ACAGCACCAACGTGCAGACCAACCTGGACCGCATGGTGACCGACGTGCGCGAGGCCGTGGCCCAGCGCGAGCGG
TTCCACCGCGAGTCCAACCTGGGATCCATGGTGGCGCTGAACGCCTTCCTCAGCACCCAGCCCGCCAACGTGCC
CCGGGGCCAGGAGGACTACACCAACTTCATCAGCGCCCTGCGCCTGATGGTGACCGAGGTGCCCCAGAGCGAGG
TGTACCAGTCCGGGCCGGACTACTTCTTCCAGACCAGTCGCCAGGGCTTGCAGACCGTGAACCTGAGCCAGGCT
TTCAAGAACTTGCAGGGCCTGTGGGGCGTGCAGGCCCCGGTCGGGGACCGCGCGACGGTGTCGAGCCTGCTGAC
GCCGAACTCGCGCCTGCTGCTGCTGCTGGTGGCCCCCTTCACGGACAGCGGCAGCATCAACCGCAACTCGTACC
TGGGCTACCTGATTAACCTGTACCGCGAGGCCATCGGCCAGGCGCACGGTGGCAGCGAGCAGACCTACCAGGAGATC
ACCCACGTGAGCCGCGCCCTGGGCCAGGACGACCCGGGCAACCTGGAAGCCACCCTGAACTTTTTGCTGACCAA
CCGGTCGCAGAAGATCCCGCCCCAGTACGCGCTCAGCACCGAGGAGGAGCGCATCCTGCGTTACGTGCAGCAGA
GCGTGGGCCTGTTCCTGATGCAGGAGGGGGCCCACCCCAGCGCCGCGCTCGACATGACCGCGCGCAACATGGAG
CCCAGCATGTACGCCAGCAACCGCCCGTTCATCAATAAACTGATGGACTACTTGCATCGGGCGGCCGCCATGAA
CTCTGACTATTTCACCAACGCCATCCTGAATCCCCACTGGCTCCCGCCGCCGGGGTTCTACACGGGCGAGTACG
ACATGCCCGACCCCAATGACGGGTTCCTGTGGGACGATGTGGACAGCAGCGTGTTCTCCCCCCGACCGGGTGCT
AACGAGCGCCCCTTGTGGAAGAAGGAAGGCAGCGACCGACGCCCGTCCTCGGCGCTGTCCGGCCGCGAGGGTGC
TGCCGCGGCGGTGCCCGAGGCCGCCAGTCCTTTCCCGAGCTTGCCCTTCTCGCTGAACAGTATCCGCAGCAGCG
AGCTGGGCAGGATCACGCGCCCGCGCTTGCTGGGCGAAGAGGAGTACTTGAATGACTCGCTGTTGAGACCCGAG
CGGGAGAAGAACTTCCCCAATAACGGGATAGAAAGCCTGGTGGACAAGATGAGCCGCTGGAAGACGTATGCGCA
GGAGCACAGGGACGATCCCCGGGCGTCGCAGGGGGCCACGAGCCGGGGCAGCGCCGCCCGTAAACGCCGGTGGC
ACGACAGGCAGCGGGGACAGATGTGGGACGATGAGGACTCCGCCGACGACAGCAGCGTGTTGGACTTGGGTGGG
AGTGGTAACCCGTTCGCTCACCTGCGCCCCCGTATCGGGCGCATGATGTAAGAGAAACCGAAAATAAATGATAC
TCACCAAGGCCATGGCGACCAGCGTGCGTTCGTTTCTTCTCTGTTGTTGTTGTATCTAGTATGATGAGGCGTGC
GTACCCGGAGGGTCCTCCTCCCTCGTACGAGAGCGTGATGCAGCAGGCGATGGCGGCGGCGGCGATGCAGCCCC
CGCTGGAGGCTCCTTACGTGCCCCCGCGGTACCTGGCGCCTACGGAGGGGCGGAACAGCATTCGTTACTCGGAG
CTGGCACCCTTGTACGATACCACCCGGTTGTACCTGGTGGACAACAAGTCGGCGGACATCGCCTCGCTGAACTA
CCAGAACGACCACAGCAACTTCCTGACCACCGTGGTGCAGAACAATGACTTCACCCCCACGGAGGCCAGCACCC
AGACCATCAACTTTGACGAGCGCTCGCGGTGGGGCGGCCAGCTGAAAACCATCATGCACACCAACATGCCCAAC
GTGAACGAGTTCATGTACGACAACAAGTTCAAGGCGCGGGTGATGGTCTCCCGCAAGACCCCCAATGGGGTGAC
AGTGACAGAGGATTATGATGGTAGTCAGGATGAGCTGAAGTATGAATGGGTGGAATTTGAGCTGCCCGAAGGCA
ACTTCTCGGTGACCATGACCATCGACCTGATGAACAACGCCATCATCGACAATTACTTGGCGGTGGGGCGGCAG
AACGGGGTGCTGGAGAGCGACATCGGCGTGAAGTTCGACACTAGGAACTTCAGGCTGGGCTGGGACCCCGTGAC
CGAGCTGGTCATGCCCGGGGTGTACACCAACGAGGCTTTCCATCCCGATATTGTCTTGCTGCCCGGCTGCGGGG
TGGACTTCACCGAGAGCCGCCTCAGCAACCTGCTGGGCATTCGCAAGAGGCAGCCCTTCCAGGAAGGCTTCCAG
ATCATGTACGAGGATCTGGAGGGGGGGCAACATCCCCGCGCTCCTGGATGTCGACGCCTATGAGAAAAGCAAGGA
GGATGCAGCAGCTGAAGCAACTGCAGCCGTAGCTACCGCCTCTACCGAGGTCAGGGGCGATAATTTTGCAAGCG
CCGCAGCAGTGGCAGCGGCCGGAGGCGGCTGAAACCGAAAGTAAGATAGTCATTCAGCCGGTGGAGAAGGATAGC
AAGAACAGGAGCTACAACGTACTACCGGACAAGATAAACACCGCCTACCGCAGCTGGTACCTAGCCTACAACTA
TGGCGACCCCGAGAAGGGCGTGCGCGCTCCTGGACGCTGCTCACCACCTCGGACGTCACCTGCGGCGTGGAGCAAG
TCTACTGGTCGCTGCCCGACATGATGCAAGACCCGGTCACCTTCCGCTCCACGCGTCAAGTTAGCAACTACCCG
GTGGTGGGCGCCGAGCTCCTGCCCGTCTACTCCAAGAGCTTCTTCAACGAGCAGGCCGTCTACTCGCAGCAGCT
GCGCGCCTTCACCTCGCTTACGCACGTCTTCAACCGCTTCCCCGAGAACCAGATCCTCGTCCGCCCGCCCGCGC
CCACCATTACCACCGTCAGTGAAAACGTTCCTGCTCTCACAGATCACGGGACCCTGCCGCTGCGCAGCAGTATC
CGGGGAGTCCAGCGCGTGACCGTTACTGACGCCAGACGCCGCACCTGCCCCTACGTCTACAAGGCCCTGGGCAT
AGTCGCGCCGCGCGTCCTCTCGAGCCGCACCTTCTAAATGTCCATTCTCATCTCGCCCAGTAATAACACCGGTT
```

```
GGGGCCTGCGCGCGCCCAGCAAGATGTACGGAGGCGCTCGCCAACGCTCCACGCAACACCCCGTGCGCGTGCGC
GGGCACTTCCGCGCTCCCTGGGGCGCCCTCAAGGGCCGCGTGCGGTCGCGCACCACCGTCGACGACGTGATCGA
CCAGGTGGTGGCCGACGCGCGCAACTACACCCCCGCCGCCGCGCCCGTCTCCACCGTGGACGCCGTCATCGACA
GCGTGGTGGCcGACGCGCGCGCCGGTACGCCCGCGCCAAGAGCCGGCGGCGGCGCATCGCCCGGCGGCACCGGAGC
ACCCCGCCATGCGCGCGGCGCGAGCCTTGCTGCGCGCAGGGCCAGGCGCACGGGACGCAGGGCCATGCTCAGGGC
GGCCAGACGCGCGGCTTCAGGCGCCAGCGCCGGCAGGACCCGGAGACGCGCGGCCACGGCGGCGGCGAGCGGCCA
TCGCCAGCATGTCCCGCCCGCGGCGAGGGAACGTGTACTGGGTGCGCGACGCCGCCACCGGTGTGCGCGTGCCC
GTGCGCACCCGCCCCCCTCGCACTTGAAGATGTTCACTTCGCGATGTTGATGTGTCCCAGCGGCGAGGAGGATG
TCCAAGCGCAAATTCAAGGAAGAGATGCTCCAGGTCATCGCGCCTGAGATCTACGGCCCTGCGGTGGTGAAGGA
GGAAAGAAAGCCCCGCAAAATCAAGCGGGTCAAAAAGGACAAAAAGGAAGAAGAAAGTGATGTGGACGGATTGG
TGGAGTTTGTGCGCGAGTTCGCCCCCCGGCGGCGCGTGCAGTGGCGCGGGCGGAAGGTGCAACCGGTGCTGAGA
CCCGGCACCACCGTGGTCTTCACGCCCGGCGAGCGCTCCGGCACCGCGCTTCCAAGCGCTCCTACGACGAGGTGTA
CGGGGATGATGATATTCTGGAGCAGGCGGCCGAGCGCCTGGGCGAGTTTGCTTACGGCAAGCGCAGCCGTTCCG
CACCGAAGGAAGAGGCGGTGTCCATCCCGCTGGACCACGGCAACCCCACGCCGAGCCTCAAGCCCGTGACCTTG
CAGCAGGTGCTGCCGACCGCGGCGCCGCGCCGGGGGGTTCAAGCGCGAGGGCGAGGATCTGTACCCCACCATGCA
GCTGATGGTGCCCAAGCGCCAGAAGCTGGAAGACGTGCTGGAGACCATGAAGGTGGACCCGGACGTGCAGCCCG
AGGTCAAGGTGCGGCCCATCAAGCAGGTGGCCCCGGGCCTGGGCGTGCAGACCGTGGACATCAAGATTCCCACG
GAGCCCATGGAAACGCAGACCGAGCCCATGATCAAGCCCAGCACCAGCACCATGGAGGTGCAGACGGATCCCTG
GATGCCATCGGCTCCTAGTCGAAGACCCCGGCGCAAGTACGGCGCGGCCAGCCTGCTGATGCCCAACTACGCGC
TGCATCCTTCCATCATCCCCACGCCGGGCTACCGCGGCACGCGCTTCTACCGCGGTCATACCAGCAGCCGCCGC
CGCAAGACCACCACTCGCCGCCGCCGTCGCCGCACCGCCGCTGCAACCACCCCTGCCGCCCTGGTGCGGAGAGT
GTACCGCCGCGGCCGCGCACCTCTGACCCTGCCGCGCGCGCGCTACCACCCGAGCATCGCCATTTAAACTTTCG
CCtGCTTTGCAGATCAATGGCCCTCACATGCCGCCTTCGCGTTCCCATTACGGGCTACCGAGGAAGAAAACCGC
GCCGTAGAAGGCTGGCGGGGAACGGGATGCGTCGCCACCACCACCGGCGGCGGCGCGCCATCAGCAAGCGGTTG
GGGGGAGGCTTCCTGCCCGCGCTGATCCCCATCATCGCCGCGGCGATCGGGGCGATCCCCGGCATTGCTTCCGT
GGCGGTGCAGGCCTCTCAGCGCCACTGAGACACACTTGGAAACATCTTGTAATAAACCaATGGACTCTGACGCT
CCTGGTCCTGTGATGTGTTTTCGTAGACAGATGGAAGACATCAATTTTTCGTCCCTGGCTCCGCGACACGGCAC
GCGGCCGTTCATGGGCACCTGGAGCGACATCGGCACCAGCCAACTGAACGGGGGCGCCTTCAATTGGAGCAGTC
TCTGGAGCGGGCTTAAGAATTTCGGGTCCACGCTTAAAACCTATGGCAGCAAGGCGTGGAACAGCACCACAGGG
CAGGCGCTGAGGGATAAGCTGAAAGAGCAGAACTTCCAGCAGAAGGTGGTCGATGGGCTCGCCTCGGGCATCAA
CGGGGTGGTGGACCTGGCCAACCAGGCCGTGCAGCGGCAGATCAACAGCCGCCTGGACCCGGTGCCGCCCGCCG
GCTCCGTGGAGATGCCGCAGGTGGAGGAGGAGCTGCCTCCCCTGGACAAGCGGGGCGAGAAGCGACCCCGCCCC
GATGCGGAGGAGACGCTGCTGACGCACACGGACGAGCCGCCCCCGTACGAGGAGGCGGTGAAACTGGGTCTGCC
CACCACGCGGCCCATCGCGCCCCTGGCCACCGGGGTGCTGAAACCCGAAAAGCCCGCGACCCTGGACTTGCCTC
CTCCCCAGCCTTCCCGCCCCTCTACAGTGGCTAAGCCCCTGCCGCCGGTGGCCGTGGCCCGCGCGCGACCCGGG
GGCACCGCCCGCCCTCATGCGAACTGGCAGAGCACTCTGAACAGCATCGTGGGTCTGGGAGTGCAGAGTGTGAA
GCGCCGCCGCTGCTATTAAACCTACCGTAGCGCTTAACTTGCTTGTCTGTGTGTGTATGTATTATGTCGCCGCC
GCCGCTGTCCACCAGAAGGAGGAGTGAAGAGGCGCGTCGCCGAGTTGCAAGATGGCCACCCCATCGATGCTGCC
CCAGTGGGCGTACATGCACATCGCCGGACAGGACGCTTCGGAGTACCTGAGTCCGGGTCTGGTGCAGTTTGCCC
GCGCCACAGACACCTACTTCAGTCTGGGGAACAAGTTTAGGAACCCCACGGTGGCGCCCACGCACGATGTGACC
ACCGACCGCAGCCAGCGGCTGACGCTGCGCTTCGTGCCCGTGGACCGCGAGGACAACACCTACTCGTACAAAGT
GCGCTACACGCTGGCCGTGGGCGACAACCGCGTGCTGGACATGGCCAGCACCTACTTTGACATCCGCGGCGTGC
TGGATCGGGGCCCTAGCTTCAAACCCTACTCCGGCACCGCCTACAACAGTCTGGCCCCCAAGGGAGCACCCAAC
ACTTGTCAGTGGACATATAAAGCCGATGGTGAAACTGCCACAGAAAAAACCTATACATATGGAAATGCACCCGT
GCAGGGCATTAACATCACAAAAGATGGTATTCAACTTGGAACTGACACCGATGATCAGCCAATCTACGCAGATA
AAACCTATCAGCCTGAACCTCAAGTGGGTGATGCTGAATGGCATGACATCACTGGTACTGATGAAAAGTATGGA
GGCAGAGCTCTTAAGCCTGATACCAAAATGAAGCCTTGTTATGGTTCTTTTGCCAAGCCTACTAATAAAGAAGG
AGGTCAGGCAAATGTGAAAACAGGAACAGGCACTACTAAAGAATATGACATAGACATGGCTTTCTTTGACAACA
GAAGTGCGGCTGCTGCCTGGCCTAGCTCCAGAAATTGTTTTGTATACTGAAAATGTGGATTTGGAAACTCCAGAT
ACCCATATTGTATACAAAGCAGGCACAGATGACAGCAGCTCTTCTATTAATTTGGGTCAGCAAGCCATGCCCAA
CAGACCTAACTACATTGGTTTCAGAGACAACTTTATCGGGCTCATGTACTACAACAGCACTGGCAATATGGGGG
TGCTGGCCGGTCAGGCTTCTCAGCTGAATGCTGTGGTTGACTTGCAAGACAGAAACACCGAGCTGTCCTACCAG
CTCTTGCTTGACTCTCTGGGTGACAGAACCCGGTATTTCAGTATGTGGAATCAGGCGGTGGACAGCTATGATCC
TGATGTGCGCATTATTGAAAATCATGGTGTGGAGGATGAACTTCCCAACTATTGTTTCCCTCTGGATGCTGTTG
GCAGAACAGATACTTATCAGGGAATTAAGGCTAATGGAACTGATCAAACCACATGGACCAAAGATGACAGTGTC
AATGATGCTAATGAGATAGGCAAGGGTAATCCATTCGCCATGGAAATCAACATCCAAGCCAACCTGTGGAGGAA
CTTCCTCTACGCCAACGTGGCCCTGTACCTGCCCGACTCTTACAAGTACACGCCGGCCAATGTTACCCTGCCCA
CCAACACCAACACCTACGATTACATGAACGGCCGGGTGGTGGCGCCCTCGCTGGTGGACTCCTACATCAACATC
GGGGCGCGCTGGTCGCTGGATCCCATGGACAACGTGAACCCCTTCAACCACCACCGCAATGCGGGGCTGCGCTA
CCGCTCCATGCTCCTGGGCAACGGGCGCTACGTGCCCTTCCACATCCAGGTGCCCCAGAAATTTTTCGCCATCA
AGAGCCTCCTGCTCCTGCCCGGGTCCTACACCTACGAGTGGAACTTCCGCAAGGACGTCAACATGATCCTGCAG
AGCTCCCTCGGCAACGACCTGCGCACGGACGGGGCCTCCATCTCCTTCACCAGCATCAACCTCTACGCCACCTT
```

```
CTTCCCCATGGCGCACAACACGGCCTCCACGCTCGAGGCCATGCTGCGCAACGACACCAACGACCAGTCCTTCA
ACGACTACCTCTCGGCGGCCAACATGCTCTACCCCATCCCGGCCAACGCCACCAACGTGCCCATCTCCATCCCC
TCGCGCAACTGGGCCGCCTTCCGCGGCTGGTCCTTCACGCGTCTCAAGACCAAGGAGACGCCCTCGCTGGGCTC
CGGGTTCGACCCCTACTTCGTCTACTCGGGCTCCATCCCCTACCTCGACGGCACCTTCTACCTCAACCACACCT
TCAAGAAGGTCTCCATCACCTTCGACTCCTCCGTCAGCTGGCCCGGCAACGACCCGGCTCCTGACGCCCAACGAG
TTCGAAATCAAGCGCACCGTCGACGGCGAGGGCTACAACGTGGCCCAGTGCAACATGACCAAGGACTGGTTCCT
GGTCCAGATGCTGGCCCACTACAACATCGGCTACCAGGGCTTCTACGTGCCCGAGGGCTACAAGGACCGCATGT
ACTCCTTCTTCCGCAACTTCCAGCCCATGAGCCGCCAGGTGGTGGACGAGGTCAACTACAAGGACTACCAGGCC
GTCACCCTGGCCTACCAGCACAACAACTCGGGCTTCGTCGGCTACCTCGCGCCCACCATGCGCCAGGGCCAGCC
CTACCCCGCCAACTACCCCTACCCGCTCATCGGCAAGAGCGCCGTCACCAGCGTCACCCAGAAAAAGTTCCTCT
GCGACAGGGTCATGTGGCGCATCCCCTTCTCCAGCAACTTCATGTCCATGGGCGCGCTCACCGACCTCGGCCAG
AACATGCTCTATGCCAACTCCGCCCACGCGCTAGACATGAATTTCGAAGTCGACCCCATGGATGAGTCCACCCT
TCTCTATGTTGTCTTCGAAGTCTTCGACGTCGTCCGAGTGCACCAGCCCCACCGCGGCGTCATCGAGGCCGTCT
ACCTGCGCACCCCCTTCTCGGCCGGTAACGCCACCACCTAAGCTCTTGCTTCTTGCAAGCCATGGCCGCGGGCT
CCGGCGAGCAGGAGCTCAGGGCCATCATCCGCGACCTGGGCTGCGGGCCCTACTTCCTGGGCACCTTCGATAAG
CGCTTCCCGGGATTCATGGCCCCGCACAAGCTGGCCTGCGCCATCGTCAACACGGCCGGCCGCGAGACCGGGGG
CGAGCACTGGCTGGCCTTCGCCTGGAACCCGCGCTCGAACACCTGCTACCTCTTCGACCCCTTCGGGTTCTCGG
ACGAGCGCCTCAAGCAGATCTACCAGTTCGAGTACGAGGGCCTGCTGCGCCGCAGCGCCCTGGCCACCGAGGAC
CGCTGCGTCACCCTGGAAAAGTCCACCCAGACCGTGCAGGGTCCGCGCTCGGCCGCCTGCGGGCTCTTCTGCTG
CATGTTCCTGCACGCCTTCGTGCACTGGCCCGACCGCCCCATGGACAAGAACCCCACCATGAACTTGCTGACGG
GGGTGCCCAACGGCATGCTCCAGTCGCCCCAGGTGGAACCCACCCTGCGCCGCAACCAGGAGGCGCTCTACCGC
TTCCTCAACTCCCACTCCGCCTACTTTCGCTCCCACCGCGCGCGCATCGAGAAGGCCACCGCCTTCGACCGCAT
GAATCAAGACATGTAAACCGTGTGTGTATGTTAAATGTCTTTAATAAACAGCACTTTCATGTTACACATGCATC
TGAGATGATTTATTTAGAAATCGAAAGGGTTCTGCCGGGTCTCGGCATGGCCCGCGGGCAGGGACACGTTGCGG
AACTGGTACTTGGCCAGCCACTTGAACTCGGGGATCAGCAGTTTGGGCAGCGGGGTGTCGGGGAAGGAGTCGGT
CCACAGCTTCCGCGTCAGTTGCAGGGCGCCCAGCAGGTCGGGCGCGGAGATCTTGAAATCGCAGTTGGGACCCG
CGTTCTGCGCGCGGGAGTTGCGGTACACGGGGTTGCAGCACTGGAACACCATCAGGGCCGGGTGCTTCACGCTC
GCCAGCACCGTCGCGTCGGTGATGCTCTCCACGTCGAGGTCCTCGGCGTTGGCCATCCCGAAGGGGGTCATCTT
GCAGGTCTGCCTTCCCATGGTGGGCACGCACCCGGGCTTGTGGTTGCAATCGCAGTGCAGGGGGATCAGCATCA
TCTGGGCCTGGTCGGCGTTCATCCCCGGGTACATGGCCTTCATGAAAGCCTCCAATTGCCTGAACGCCTGCTGG
GCCTTGGCTCCCTCGGTGAAGAAGACCCCGCAGGACTTGCTAGAGAACTGGTTGGTGGCGCACCCGGCGTCGTG
CACGCAGCAGCGCGCGTCGTTGTTGGCCAGCTGCACCACGCTGCGCCCCCAGCGGTTCTGGGTGATCTTGGCCC
GGTCGGGGTTCTCCTTCAGCGCGCGCTGCCCGTTCTCGCTCGCCACATCCATCTCGATCATGTGCTCCTTCTGG
ATCATGGTGGTCCCGTGCAGGCACCGCAGCTTGCCCTCGGCCTCGGTGCACCGTGCAGCCACAGCGCGGCACCC
GGTGCACTCCCAGTTCTTGTGGGCGATCTGGGAATGCGCGTGCACGAAGCCCTGCAGGAAGCGGCCCATCATGG
TGGTCAGGGTCTTGTTGCTAGTGAAGGTCAGCGGAATGCCGCGGTGCTCCTCGTTGATGTACAGGTGGCAGATG
CGGCGGTACACCTCGCCCTGCTCGGGCATCAGCTGGAAGTTGGCTTTCAGGTCGGTCTCCACGCGGTAGCGGTC
CATCAGCATAGTCATGATTTCCATACCCTTCTCCCAGGCCGAGACGATGGGCAGGCTCATAGGGTTCTTCACCA
TCATCTTAGCGCTAGCAGCCGCGGCCAGGGGGTCGCTCTCGTCCAGGGTCTCAAAGCTCCGCTTGCCGTCCTTC
TCGGTGATCCGCACCGGGGGGTAGCTGAAGCCCACGGCCGCCAGCTCCTCCTCGGCCTGTCTTTCGTCCTCGCT
GTCCTGGCTGACGTCCTGCAGGACCACATGCTTGGTCTTGCGGGGTTTCTTCTTGGGCGGCAGCGGCGGCGGAG
ATGTTGGAGATGGCGAGGGGGAGCGCGAGTTCTCGCTCACCACTACTATCTCTTCCTCTTCTTGGTCCGAGGCC
ACGCGGCGGTAGGTATGTCTCTTCGGGGGCAGAGGCGGAGGCGACGGGCTCTCGCCGCCGCGACTTGGCGGATG
GCTGGCAGAGCCCCTTCCGCGTTCGGGGGTGCGCTCCCGGCGGCGCTCTGACTGACTTCCTCCGCGGCCGGCCA
TTGTGTTCTCCTAGGGAGGAACAACAAGCATGGAGACTCAGCCATCGCCAACCTCGCCATCTGCCCCCACCGCC
GACGAGAAGCAGCAGCAGCAGAATGAAAGCTTAACCGCCCCGCCGCCCAGCCCCGCCACCTCCGACGCGGCCGT
CCCAGACATGCAAGAGATGGAGGAATCCATCGAGATTGACCTGGGCTATGTGACGCCCGCGGAGCACGAGGAGG
AGCTGGCAGTGCGCTTTTCACAAGAAGAGATACACCAAGAACAGCCAGAGCAGGAAGCAGAGAATGAGCAGAGT
CAGGCTGGGCTCGAGCATGACGGCGACTACCTCCACCTGAGCGGGGGGAGGACGCGCTCATCAAGCATCTGGC
CCGGCAGGCCACCATCGTCAAGGATGCGCTGCTCGGACCGCACCGAGGTGCCCCTCAGCGTGGAGGAGCTCAGCC
GCGCCTACGAGTTGAACCTCTTCTCGCCGCGCGTGCCCCCCAAGCGCCAGCCCAATGGCACCTGCGAGCCCAAC
CCGCGCCCTCAACTTCTACCCGGTCTTCGCGGTGCCCGAGGCCCTGGCCACCTACCACATCTTTTTCAAGAACCA
AAAGATCCCCGTCTCCTGCCGCGCCAACCGCACCCGCGCCGACGCCCTTTTCAACCTGGGTCCCGGCGCCCGCC
TACCTGATATCGCCTCCTTGGAAGAGGTTCCCAAGATCTTCGAGGGTCTGGGCAGCGACGAGACTCGGGCCGCG
AACGCTCTGCAAGGAGAAGGAGGAGAGCATGAGCACCACAGCGCCCTGGTCGAGTTGGAAGGCGACAACGCGCG
GCTGGCGGTGCTCAAACGCACGGTCGAGCTGACCCATTTCGCCTACCCGGCTCTGAACCTGCCCCCCAAAGTCA
TGAGCGCGGTCATGGACCAGGTGCTCATCAAGCGCGTGCGCCCATCTCCGAGGACGAGGGCATGCAAGACTCC
GAGGAGGGCAAGCCCGTGGTCAGCGACGAGCAGCTGGCCCGGTGGCTGGGTCCTAATGCTAGTCCCCAGAGTTT
GGAAGAGCGGCGCAAACTCATGATGGCCGTGGTCCTGGTGACCGTGGAGCTGGAGTGCCTGCGCCGCTTCTTCG
CCGACGCGGAGACCCTGCGCAAGGTCGAGGAGAACCTGCACTACCTCTTCAGGCACGGGTTCGTGCGCCAGGCC
TGCAAGATCTCCAACGTGGAGCTGACCAACCTGGTCTCCTACATGGGCATCTTGCACGAGAACCGCCTGGGGCA
GAACGTGCTGCACACCACCCCTGCGCGGGGAGGCCCGGCGCGACTACATCCGCGACTGCGTCTACCTCTACCTCT
```

```
GCCACACCTGGCAGACGGGCATGGGCGTGTGGCAGCAGTGTCTGGAGGAGCAGAACCTGAAAGAGCTCTGCAAG
CTCCTGCAGAAGAACCTCAAGGGTCTGTGGACCGGGTTCGACGAGCGCACCACCGCCTCGGACCTGGCCGACCT
CATTTTCCCCGAGCGCCTCAGGCTGACGCTGCGCAACGGCCTGCCCGACTTTATGAGCCAAAGCATGTTGCAAA
ACTTTCGCTCTTTCATCCTCGAACGCTCCGGAATCCTGCCCGCCACCTGCTCCGCGCTGCCCTCGGACTTCGTG
CCGCTGACCTTCCGCGAGTGCCCCCCGCCGCTGTGGAGCCACTGCTACCTGCTGCGCCTGGCCAACTACCTGGC
CTACCACTCGGACGTGATCGAGGACGTCAGCGGCGAGGGCCTGCTCGAGTGCCACTGCCGCTGCAACCTCTGCA
CGCCGCACCGCTCCCTGGCCTGCAACCCCCAGCTGCTGAGCGAGACCCAGATCATCGGCACCTTCGAGTTGCAA
GGGCCCAGCGAAGGCGAGGGTTCAGCCGCCAAGGGGGGTCTGAAACTCACCCCGGGGCTGTGGACCTCGGCCTA
CTTGCGCAAGTTCGTGCCCGAGGACTACCATCCCTTCGAGATCAGGTTCTACGAGGACCAATCCATCCGCCCA
AGGCCGAGCTGTCGGCCTGCGTCATCACCCAGGGGGCGATCCTGGCCCAATTGCAAGCCATCCAGAAATCCCGC
CAAGAATTCTTGCTGAAAAAGGGCCGCGGGGTCTACCTCGACCCCCAGACCGGTGAGGAGCTCAACCCCGGCTT
CCCCCAGGATGCCCCGAGGAAACAAGAAGCTGAAAGTGGAGCTGCCGCCCGTGGAGGATTTGGAGGAAGACTGG
GAGAACAGCAGTCAGGCAGAGGAGGAGGAGATGGAGGAAGACTGGGACAGCACTCAGGCAGAGGAGGACAGCCT
GCAAGACAGTCTGGAGGAAGACGAGGAGGAGGCAGAGGAGGAGGTGGAAGAAGCAGCCGCCGCCAGACCGTCGT
CCTCGGCGGGGGAGAAAGCAAGCAGCACGGATACCATCTCCGCTCCGGGTCGGGGTCCCGCTCGACCACACAGT
AGATGGGACGAGACCGGACGATTCCCGAACCCCACCACCCAGACCGGTAAGAAGGAGCGGCAGGGATACAAGTC
CTGGCGGGGGCACAAAAACGCCATCGTCTCCTGCTTGCAGGCCTGCGGGGGCAACATCTCCTTCACCCGGCGCT
ACCTGCTCTTCCACCGCGGGGTGAACTTTCCCCGCAACATCTTGCATTACTACCGTCACCTCCACAGCCCCTAC
TACTTCCAAGAAGAGGCAGCAGCAGCAGAAAAAGACCAGCAGAAAACCAGCAGCTAGAAAATCCACAGCGGCGG
CAGCAGGTGGACTGAGGATCGCGGCGAACGAGCCGGCGCAAACCCGGGAGCTGAGGAACCGGATCTTTCCCACC
CTCTATGCCATCTTCCAGCAGAGTCGGGGGCAGGAGCAGGAACTGAAAGTCAAGAACCGTTCTCTGCGCTCGCT
CACCCGCAGTTGTCTGTATCACAAGAGCGAAGACCAACTTCAGCGCACTCTCGAGGACGCCGAGGCTCTCTTCA
ACAAGTACTGCGCGCTCACTCTTAAAGAGTAGCCCGCGCCCGCCCAGTCGCAGAAAAAGGCGGGAATTACGTCA
CCTGTGCCCTTCGCCCTAGCCGCCTCCACCCATCATCATGAGCAAAGAGATTCCCACGCCTTACATGTGGAGCT
ACCAGCCCCAGATGGGCCTGGCCGCCGGTGCCGCCCAGGACTACTCCACCCGCATGAATTGGCTCAGCGCCGGG
CCCGCGATGATCTCACGGGTGAATGACATCCGCGCCCACCGAAACCAGATACTCCTAGAACAGTCAGCGCTCAC
CGCCCACGCCCCGCAATCACCTCAATCCGCGTAATTGGCCCGCCGCCCTGGTGTACCAGGAAATTCCCCAGCCCA
CGACCGTACTACTTCCGCGAGACGCCCAGGCCGAAGTCCAGCTGACTAACTCAGGTGTCCAGCTGGCGGGCGGC
GCCACCCTGTGTCGTCACCGCCCCGCTCAGGGTATAAAGCGGCTGGTGATCCGGGGCAGAGGCACACAGCTCAA
CGACGAGGTGGTGAGCTCTTCGCTGGGTCTGCGACCTGACGGAGTCTTCCAACTCGCCGGATCGGGGAGATCTT
CCTTCACGCCTCGTCAGGCCGTCCTGACTTTGGAGAGTTCGTCCTCGCAGCCCCGCTCGGGTGGCATCGGCACT
CTCCAGTTCGTGGAGGAGTTCACTCCCTCGGTCTACTTCAACCCCTTCTCCGGCTCCCCGGCCACTACCCGGA
CGAGTTCATCCCGAACTTCGACGCCATCAGCGAGTCGGTGGACGGCTACGATTGAAACTAATCACCCCCTTATC
CAGTGAAATAAAGATCATATTGATGATGATTTTACAGAAATAAAAAATAATCATTTGATTTGAAATAAAGATAC
AATCATATTGATGATTTGAGTTTAACAAAAAAATAAAGAATCACTTACTTGAAATCTGATACCAGGTCTCTGTC
CATGTTTTCTGCCAACACCACTTCACTCCCCTCTTCCCAGCTCTGGTACTGCAGGCCCCGGCGGGCTGCAAACT
TCCTCCACACGCTGAAGGGGATGTCAAATTCCTCCTGTCCCTCAATCTTCATTTTATCTTCTATCAGATGTCCA
AAAAGCGCGTCCGGGTGGATGATGACTTCGACCCCGTCTACCCCTACGATGCAGACAACGCACCGACCGTGCCC
TTCATCAACCCCCCCTTCGTCTCTTCAGATGGATTCCAAGAGAAGCCCCTGGGGGTGTTGTCCCTGCGACTGGC
CGACCCCGTCACCACCAAGAACGGGGAAATCACCCTCAAGCTGGGAGAGGGGGTGGACCTCGATTCCTCGGGAA
AACTCATCTCCAACACGGCCACCAAGGCCGCCGCCCCTCTCAGTTTTTCCAACAACACCATTTCCCTTAACATG
GATCACCCCTTTTACACTAAAGATGGAAAATTATCCTTACAAGTTTCTCCACCATTAAATATACTGAGAACAAG
CATTCTAAACACACTAGCTTTAGGTTTTGGATCAGGTTTAGGACTCCGTGGCTCTGCCTTGGCAGTACAGTTAG
TCTCTCCACTTACATTTGATACTGATGGAAACATAAAGCTTACCTTAGACAGAGGTTTGCATGTTACAACAGGA
GATGCAATTGAAAGCAACATAAGCTGGGCTAAAGGTTTAAAATTTGAAGATGGAGCCATAGCAACCAACATTGG
AAATGGGTTAGAGTTTGGAAGCAGTAGTACAGAAACAGGTGTTGATGATGCTTACCCAATCCAAGTTAAACTTG
GATCTGGCCTTAGCTTTGACAGTACAGGAGCCATAATGGCTGGTAACAAAGAAGACGATAAACTCACTTTGTGG
ACAACACCTGATCCATCACCAAACTGTCAAATACTCGCAGAAAATGATGCAAAACTAACACTTTGCTTGACTAA
ATGTGGTAGTCAAATACTGGCCACTGTGTCAGTCTTAGTTGTAGGAAGTGGAAACCTAAACCCCATTACTGGCA
CCGTAAGCAGTGCTCAGGTGTTTCTACGTTTTGATGCAAACGGTGTTCTTTTAACAGAACATTCTACACTAAAA
AAATACTGGGGGTATAGGCAGGGAGATAGCATAGATGGCACTCCATATACCAATGCTGTAGGATTCATGCCCAA
TTTAAAAGCTTATCCAAAGTCACAAAGTTCTACTACTAAAAATAATATAGTAGGGCAAGTATACATGAATGGAG
ATGTTTCAAAACCTATGCTTCTCACTATAACCCTCAATGGTACTGATGACAGCAACAGTACATATTCAATGTCA
TTTTCATACACCTGGACTAATGGAAGCTATGTTGTTGAGCAACATTTGGGGCTAACTCTTATACCTTCTCATACAT
CGCCCAAGAATGAACACTGTATCCCACCCTGCATGCCAACCCTTCCCACCCCACTCTGTGGAACAAACTCTGAA
ACACAAAATAAAATAAAGTTCAAGTGTTTTATTGATTCAACAGTTTTACAGGATTCGAGCAGTTATTTTTCCTC
CACCCTCCCAGGACATGGAATACACCACCCTCTCCCCCCGCACAGCCTTGAACATCTGAATGCCATTGGTGATG
GACATGCTTTTGGTCTCCACGTTCCACACAGTTTCAGAGCGAGCCAGTCTCGGGTCGGTCAGGGAGATGAAACC
CTCCGGGCACTCCCGCATCTGCACCTCACAGCTCAACAGCTGAGGATTGTCCTCGGTGGTCGGGATCACGGTTA
TCTGGAAGAAGCAGAAGAGCGGCGGTGGGAATCATAGTCCGCGAACGGGATCGGCCGGTGGTGTCGCATCAGGC
CCCGCAGCAGTCGCTGCCGCCGCCGCTCCGTCAAGCTGCTGCTCAGGGGGTCCGGGTCCAGGGACTCCCTCAGC
ATGATGCCCACGGCCCTCAGCATCAGTCGTCTGGTGCGGCGGGCGCAGCAGCGCATGCGGATCTCGCTCAGGTC
```

```
GCTGCAGTACGTGCAACACAGAACCACCAGGTTGTTCAACAGTCCATAGTTCAACACGCTCCAGCCGAAACTCA
TCGCGGGAAGGATGCTACCCACGTGGCCGTCGTACCAGATCCTCAGGTAAATCAAGTGGTGCCCCCTCCAGAAC
ACGCTGCCCACGTACATGATCTCCTTGGGCATGTGGCGGTTCACCACCTCCCGGTACCACATCACCCTCTGGTT
GAACATGCAGCCCCGGATGATCCTGCGGAACCACAGGGCCAGCACCGCCCCGCCCGCCATGCAGCGAAGAGACC
CCGGGTCCCGGCAATGGCAATGGAGGACCCACCGCTCGTACCCGTGGATCATCTGGGAGCTGAACAAGTCTATG
TTGGCACAGCACAGGCATATGCTCATGCATCTCTTCAGCACTCTCAACTCCTCGGGGGTCAAAACCATATCCCA
GGGCACGGGGAACTCTTGCAGGACAGCGAACCCCGCAGAACAGGGCAATCCTCGCACAGAACTTACATTGTGCA
TGGACAGGGTATCGCAATCAGGCAGCACCGGGTGATCCTCCACCAGAGAAGCGCGGGTCTCGGTCTCCTCACAG
CGTGGTAAGGGGGCCGGCCGATACGGGTGATGGCGGGACGCGGCTGATCGTGTTCGCGACCGTGTCATGATGCA
GTTGCTTTCGGACATTTTCGTACTTGCTGTAGCAGAACCTGGTCCGGGCGCTGCACACCGATCGCCGGCGGCGG
TCTCGGCGCTTGGAACGCTCGGTGTTGAAATTGTAAAACAGCCACTCTCTCAGACCGTGGCAGCAGATCTAGGGC
CTCAGGAGTGATGAAGATCCCATCATGCCTGATGGCTCTGATCACATCGACCACCGTGGAATGGGCCAGACCCA
GCCAGATGATGCAATTTTGTTGGGTTTCGGTGACGGCGGGGGAGGGAAGAACAGGAAGAACCATGATTAACTTT
TAATCCAAACGGTCTCGGAGTACTTCAAAATGAAGATCGCGGAGATGGCCACCTCTCGCCCCCGCTGTGTTGGTG
GAAAATAACAGCCAGGTCAAAGGTGATACGGTTCTCGAGATGTTCCACGGTGGCTTCCAGCAAAGCCTCCACGC
GCACATCCAGAAACAAGACAATAGCGAAAGCGGGAGGGTTCTCTAATTCCTCAATCATCATGTTACACTCCTGC
ACCATCCCCAGATAATTTTCATTTTTCCAGCCTTGAATGATTCGAACTAGTTCcTGAGGTAAATCCAAGCCAGC
CATGATAAAGAGCTCGCGCAGAGCGCCCTCCACCGGCATTCTTAAGCACACCCTCATAATTCCAAGATATTCTG
CTCCTGGTTCACCTGCAGCAGATTGACAAGCGGAATATCAAAATCTCTGCCGCGATCCCTGAGCTCCTCCCTCA
GCAATAACTGTAAGTACTCTTTCATATCCTCTCCGAAATTTTTAGCCATAGGACCACCAGGAATAAGATTAGGG
CAAGCCACAGTACAGATAAACCGAAGTCCTCCCCAGTGAGCATTGCCAAATGCAAGACTGCTATAAGCATGCTG
GCTAGACCCGGTGATATCTTCCAGATAACTGGACAGAAAATCGCCCAGGCAATTTTTAAGAAAATCAACAAAAG
AAAAATCCTCCAGGTGGACGTTTAGAGCCTCGGGAACAACGATGAAGTAAATGCAAGCGGTGCGTTCCAGCATG
GTTAGTTAGCTGATCTGTAGAAAAAACAAAAATGAACATTAAACCATGCTAGCCTGGCGAACAGGTGGGTAAAT
CGTTCTCTCCAGCACCAGGCAGGCCACGGGGTCTCCGGCGCGACCCTCGTAAAAATTGTCGCTATGATTGAAAA
CCATCACAGAGAGACGTTCCCGGTGGCCGGCGTGAATGATTCGACAAGATGAATACACCCCCGGAACATTGGCG
TCCGCGAGTGAAAAAAAGCGCCCGAGGAAGCAATAAGGCACTACAATGCTCAGTCTCAAGTCCAGCAAAGCGAT
GCCATGCGGATGAAGCACAAAATTCTCAGGTGCGTACAAAATGTAATTACTCCCCTCCTGCACAGGCAGCAAAG
CCCCGATCCCTCCAGGTACACATACAAAGCCTCAGCGTCCATAGCTTACCGAGCAGCAGCACACAACAGGCGC
AAGAGTCAGAGAAAGGCTGAGCTCTAACCTGTCCACCCGCTCTCTGCTCAATATATAGCCCAGATCTACACTGA
CGTAAAGGCCAAAGTCTAAAAATACCCGCCAAATAATCACACACGCCCAGCACACGCCCAGAAACCGGTGACAC
ACTCAAAAAAATACGCGCACTTCCTCAAACGCCCAAAACTGCCGTCATTTCCGGGTTCCCACGCTACGTCATCA
AAACACGACTTTCAAATTCCGTCGACCGTTAAAAACGTCACCCGCCCCGCCCCTAACGGTCGCCCGTCTCTCAG
CCAATCAGCGCCCGCATCCCCAAATTCAAACACCTCATTTGCATATTAACGCGCACAAAAAGTTTGAGG
```

Venezuelan equine encephalitis virus [VEE] (SEQ ID NO:3) GenBank: L01442.2

```
atgggcggcg catgagagaa gcccagacca attacctacc caaaatggag aaagttcacg
ttgacatcga ggaagacagc ccattcctca gagctttgca gcggagcttc ccgcagtttg
aggtagaagc caagcaggtc actgataatg accatgctaa tgccagagcg ttttcgcatc
tggcttcaaa actgatcgaa acggaggtgg acccatccga cacgatcctt gacattggaa
gtgcgcccgc ccgcagaatg tattctaagc acaagtatca ttgtatctgt ccgatgagat
gtgcggaaga tccggacaga ttgtataagt atgcaactaa gctgaagaaa aactgtaagg
aaataactga taaggaattg gacaagaaaa tgaaggagct cgccgccgtc atgagcgacc
ctgacctgga aactgagact atgtgcctcc acgacgacga gtcgtgtcgc tacgaagggc
aagtcgctgt ttaccaggat gtatacgcgg ttgacggacc gacaagtctc tatcaccaag
ccaataaggg agttagagtc gcctactgga taggctttga caccaccccct tttatgttta
agaacttggc tggagcatat ccatcatact ctaccaactg ggccgacgaa accgtgttaa
cggctcgtaa cataggccta tgcagctctg acgttatgga gcggtcacgt agagggatgt
ccattcttag aaagaagtat ttgaaaccat ccaacaatgt tctattctct gttggctcga
ccatctacca cgagaagagg gacttactga ggagctggca cctgccgtct gtatttcact
tacgtggcaa gcaaaattac acatgtcggt gtgagactat agttagttgc gacgggtacg
tcgttaaaag aatagctatc agtccaggcc tgtatgggaa gccttcaggc tatgctgcta
cgatgcaccg cgagggattc ttgtgctgca aagtgacaga cacattgaac ggggagaggg
tctcttttcc cgtgtgcacg tatgtgccag ctacattgtg tgaccaaatg actggcatac
tggcaacaga tgtcagtgcg gacgacgcgc aaaaactgct ggttgggctc aaccagcgta
tagtcgtcaa cggtcgcacc cagagaaaca ccaataccat gaaaaattac ctttttgcccg
tagtggccca ggcatttgct aggtgggcaa aggaatataa ggaagatcaa gaagatgaaa
ggccactagg actacgagat agacagttag tcatggggtg ttgttgggct tttagaaggc
acaagataac atctatttat aagcgcccgg atacccaaac catcatcaaa gtgaacagcg
atttccactc attcgtgctg cccaggatag gcagtaacac attggagatc gggctgagaa
caagaatcag gaaaatgtta gaggagcaca aggagccgtc acctctcatt accgccgagg
acgtacaaga agctaagtgc gcagccgatg aggctaagga ggtgcgtgaa gccgaggagt
```

```
tgcgcgcagc tctaccacct ttggcagctg atgttgagga gcccactctg gaagccgatg
tcgacttgat gttacaagag gctgggccg gctcagtgga gacacctcgt ggcttgataa
aggttaccag ctacgctggc gaggacaaga tcggctctta cgctgtgctt tctccgcagg
ctgtactcaa gagtgaaaaa ttatcttgca tccaccctct cgctgaacaa gtcatagtga
taacacactc tggccgaaaa gggcgttatg ccgtggaacc ataccatggt aaagtagtgg
tgccagaggg acatgcaata cccgtccagg actttcaagc tctgagtgaa agtgccacca
ttgtgtacaa cgaacgtgag ttcgtaaaca ggtacctgca ccatattgcc acacatggag
gagcgctgaa cactgatgaa gaatattaca aaactgtcaa gcccagcgag cacgacggcg
aatacctgta cgacatcgac aggaaacagt gcgtcaagaa agaactagtc actgggctag
ggctcacagg cgagctggtg gatcctccct tccatgaatt cgcctacgag agtctgagaa
cacgaccagc cgctccttac caagtaccaa ccataggggt gtatggcgtg ccaggatcag
gcaagtctgg catcattaaa agcgcagtca ccaaaaaaga tctagtggtg agcgccaaga
aagaaaactg tgcagaaatt ataagggacg tcaagaaaat gaaagggctg gacgtcaatg
ccagaactgt ggactcagtg ctcttgaatg gatgcaaaca ccccgtagag accctgtata
ttgacgaagc ttttgcttgt catgcaggta ctctcagagc gctcatagcc attataagac
ctaaaaaggc agtgctctgc ggggatccca aacagtgcgg ttttttttaac atgatgtgcc
tgaaagtgca ttttaaccac gagatttgca cacaagtctt ccacaaaagc atctctcgcc
gttgcactaa atctgtgact tcggtcgtct caaccttgtt ttacgacaaa aaaatgagaa
cgacgaatcc gaaagagact aagattgtga ttgacactac cggcagtacc aaacctaagc
aggacgatct cattctcact tgtttcagag ggtgggtgaa gcagttgcaa atagattaca
aaggcaacga aataatgacg gcagctgcct ctcaagggct gacccgtaaa ggtgtgtatg
ccgttcggta caaggtgaat gaaaatcctc tgtacgcacc cacctcagaa catgtgaacg
tcctactgac ccgcacggag gaccgcatcg tgtggaaaac actagccggc gacccatgga
taaaaacact gactgccaag taccctggga atttcactgc cacgatagag gagtggcaag
cagagcatga tgccatcatg aggcacatct ggagagacc ggaccctacc gacgtcttcc
agaataaggc aaacgtgtgt tgggccaagg ctttagtgcc ggtgctgaag accgctggca
tagacatgac cactgaacaa tggaacactg tggattattt tgaaacggac aaagctcact
cagcagagat agtattgaac caactatgcg tgaggttctt tggactcgat ctggactccg
gtctattttc tgcacccact gttccgttat ccattaggaa taatcactgg gataactccc
cgtcgcctaa catgtacggg ctgaataaag aagtggtccg tcagctctct cgcaggtacc
cacaactgcc tcgggcagtt gccactggaa gagtctatga catgaacact ggtacactgc
gcaattatga tccgcgcata aacctagtac ctgtaaacag aagactgcct catgctttag
tcctccacca taatgaacac ccacagagtg acttttcttc attcgtcagc aaattgaagg
gcagaactgt cctggtggtc ggggaaaagt tgtccgtccc aggcaaaatg gttgactggt
tgtcagaccg gcctgaggct accttcagag ctcggctgga tttaggcatc ccaggtgatg
tgcccaaata tgacataata tttgttaatg tgaggacccc atataaatac catcactatc
agcagtgtga agaccatgcc attaagctta gcatgttgac caagaaagct tgtctgcatc
tgaatcccgg cggaacctgt gtcagcatag gttatggtta cgctgacagg gccagcgaaa
gcatcattgg tgctatagcg cggcagttca agttttcccg ggtatgcaaa ccgaaatcct
cacttgaaga gacggaagtt ctgtttgtat tcattgggta cgatcgcaag gcccgtacgc
acaatcctta caagcttttca tcaaccttga ccaacatta tacaggttcc agactccacg
aagccggatg tgcaccctca tatcatgtgg tgcgagggga tattgccacg gccaccgaag
gagtgattat aaatgctgct aacagcaaag gacaacctgg cggagggtg tgcggagcgc
tgtataagaa attcccggaa agcttcgatt tacagccgat cgaagtagga aaagcgcgac
tggtcaaagg tgcagctaaa catatcattc atgccgtagg accaaacttc aacaaagttt
cggaggttga aggtgacaaa cagttggcag aggcttatga gtccatcgct aagattgtca
acgataacaa ttacaagtca gtagcgattc cactgttgtc caccggcatc ttttccggga
acaaagatcg actaacccaa tcattgaacc atttgctgac agctttagac accactgatg
cagatgtagc catatactgc agggacaaga aatgggaaat gactctcaag gaagcagtgg
ctaggagaga agcagtggag gagatatgca tatccgacga ctcttcagtg acagaacctg
atgcagagct ggtgagggtg catccgaaga gttctttggc tggaaggaag ggctacagca
caagcgatgg caaaactttc tcatatttgg aagggaccaa gtttcaccag gcggccaagg
atatagcaga aattaatgcc atgtggcccg ttgcaacgga ggccaatgag caggtatgca
tgtatatcct cggagaaagc atgagcagta ttaggtcgaa atgccccgtc gaagagtcgg
aagcctccac accacctagc acgctgcctt gcttgtgcat ccatgccatg actccagaaa
gagtacagcg cctaaaagcc tcacgtccag aacaaattac tgtgtgctca tcctttccat
tgccgaagta tagaatcact ggtgtgcaga agatccaatg ctcccagcct atattgttct
caccgaaagt gcctgcgtat attcatccaa ggaagtatct cgtggaaaca ccaccggtag
acgagactcc ggagccatcg gcagagaacc aatccacaga ggggacacct gaacaaccac
cacttataac cgaggatgag accaggacta gaacgcctga gccgatcatc atcgaagagg
aagaagagga tagcataagt ttgctgtcag atggcccgac ccaccaggtg ctgcaagtcg
aggcagacat tcacgggccg ccctctgtat ctagctcatc ctggtccatt cctcatgcat
```

```
ccgactttga tgtggacagt ttatccatac ttgacaccct ggagggagct agcgtgacca
gcggggcaac gtcagccgag actaactctt acttcgcaaa gagtatggag tttctggcgc
gaccggtgcc tgcgcctcga acagtattca ggaaccctcc acatcccgct ccgcgcacaa
gaacaccgtc acttgcaccc agcagggcct gctcgagaac cagcctagtt tccaccccgc
caggcgtgaa tagggtgatc actagagagg agctcgaggc gcttacccncg tcacgcactc
ctagcaggtc ggtctcgaga accagcctgg tctccaaccc gccaggcgta aatagggtga
ttacaagaga ggagtttgag gcgttcgtag cacaacaaca atgacggttt gatgcgggtg
catacatctt ttcctccgac accggtcaag ggcatttaca acaaaaatca gtaaggcaaa
cggtgctatc cgaagtggtg ttggagagga ccgaattgga gatttcgtat gccccgcgcc
tcgaccaaga aaaagaagaa ttactacgca agaaattaca gttaaatccc acacctgcta
acagaagcag ataccagtcc aggaaggtgg agaacatgaa agccataaca gctagacgta
ttctgcaagg cctagggcat tatttgaagg cagaaggaaa agtggagtgc taccgaaccc
tgcatcctgt tcctttgtat tcatctagtg tgaaccgtgc cttttcaagc cccaaggtcg
cagtggaagc ctgtaacgcc atgttgaaag agaactttcc gactgtggct tcttactgta
ttattccaga gtacgatgcc tatttggaca tggttgacgg agcttcatgc tgcttagaca
ctgccagttt ttgccctgca aagctgcgca gctttccaaa gaaacactcc tatttggaac
ccacaatacg atcggcagtg ccttcagcga tccagaacac gctccagaac gtcctggcag
ctgccacaaa aagaaattgc aatgtcacgc aaatgagaga attgcccgta ttggattcgg
cggcctttaa tgtggaatgc ttcaagaaat atgcgtgtaa taatgaatat tgggaaacgt
ttaaagaaaa ccccatcagg cttactgaag aaaacgtggt aaattacatt accaaattaa
aaggaccaaa agctgctgct ctttttgcga agacacataa tttgaatatg ttgcaggaca
taccaatgga caggtttgta atggacttaa agagagacgt gaaagtgact ccaggaacaa
aacatactga agaacggccc aagtacagg tgatccaggc tgccgatccg ctagcaacag
cgtatctgtg cggaatccac cgagagctgg ttaggagatt aaatgcggtc ctgcttccga
acattcatac actgtttgat atgtcggctg aagactttga cgctattata gccgagcact
tccagcctgg ggattgtgtt ctggaaactg acatcgcgtc gtttgataaa agtgaggacg
acgccatggc tctgaccgcg ttaatgattc tggaagactt aggtgtggac gcagagctgt
tgacgctgat tgaggcggct ttcggcgaaa tttcatcaat acatttgccc actaaaacta
aatttaaatt cggagccatg atgaaatctg gaatgttcct cacactgttt gtgaacacag
tcattaacat tgtaatcgca agcagagtgt tgagagaacg gctaaccgga tcaccatgtg
cagcattcat tggagatgac aatatcgtga aaggagtcaa atcggacaaa ttaatggcag
acaggtgcgc cacctggttg aatatggaag tcaagattat agatgctgtg gtgggcgaga
aagcgcctta tttctgtgga gggtttattt tgtgtgactc cgtgaccggc acagcgtgcc
gtgtggcaga ccccctaaaa aggctgttta agcttggcaa acctctggca gcagacgatg
aacatgatga tgacaggaga agggcattgc atgaagagtc aacacgctgg aaccgagtgg
gtattctttc agagctgtgc aaggcagtag aatcaaggta tgaaaccgta ggaacttcca
tcatagttat ggccatgact actctagcta gcagtgttaa atcattcagc tacctgagag
gggcccctat aactctctac ggctaacctg aatggactac gacatagtct agtccgccaa
gatgttcccg ttccagccaa tgtatccgat gcagccaatg ccctatcgca acccgttcgc
ggccccgcgc aggccctggt tccccagaac cgaccttttt ctggcgatgc aggtgcagga
attaacccgc tcgatggcta acctgacgtt caagcaacgc cgggacgcgc cacctgaggg
gccatccgct aagaaaccga agaaggaggc ctcgcaaaaa cagaaagggg gaggccaagg
gaagaagaag aagaaccaag ggaagaagaa ggctaagaca gggccgccta atccgaaggc
acagaatgga aacaagaaga agaccaacaa gaaaccaggc aagagacagc gcatggtcat
gaaattggaa tctgacaaga cgttcccaat catgttggaa gggaagataa acggctacgc
ttgtgtggtc ggagggaagt tattcaggcc gatgcatgtg gaaggcaaga tcgacaacga
cgttctggcc gcgcttaaga cgaagaaagc atccaaatac gatcttgagt atgcagatgt
gccacagaac atgcgggccg atacattcaa atacacccat gagaaacccc aaggctatta
cagctggcat catggagcag tccaatatga aaatgggcgt ttcacggtgc cgaaaggagt
tggggccaag ggagacagcg gacgacccat tctggataac cagggacggg tggtcgctat
tgtgctggga ggtgtgaatg aaggatctag gacagccctt tcagtcgtca tgtggaacga
gaagggagtt accgtgaagt atactccgga gaactgcgag caatggtcac tagtgaccac
catgtgtctg ctcgccaatg tgacgttccc atgtgctcaa ccaccaattt gctacgacag
aaaaccagca gagactttgg ccatgctcag cgttaacgtt gacaacccgg ctacgatga
gctgctggaa gcagctgtta agtgccccgg aaggaaaagg agatccaccg aggagctgtt
taaggagtat aagctaacgc gcccttacat ggccagatgc atcagatgtg cagttgggag
ctgccatagt ccaatagcaa tcgaggcagt aaagagcgac gggcacgacg gttatgttag
acttcagact tcctcgcagt atggcctgga ttcctccggc aacttaaagg gcaggaccat
gcggtatgac atgcacggga ccattaaaga gataccacta catcaagtgt cactccatac
atctcgcccg tgtcacattg tggatgggca cggttatttc ctgcttgcca ggtgcccggc
agggggactcc atcaccatgg aatttaagaa agattccgtc acacactcct gctcggtgcc
gtatgaagtg aaatttaatc ctgtaggcag agaactctat actcatcccc cagaacacgg
```

```
agtagagcaa gcgtgccaag tctacgcaca tgatgcacag aacagaggag cttatgtcga
gatgcacctc ccgggctcag aagtggacag cagtttggtt tccttgagcg gcagttcagt
caccgtgaca cctcctgttg ggactagcgc cctggtggaa tgcgagtgtg gcggcacaaa
gatctccgag accatcaaca agacaaaaca gttcagccag tgcacaaaga aggagcagtg
cagagcatat cggctgcaga acgataagtg ggtgtataat tctgacaaac tgcccaaagc
agcgggagcc accttaaaag gaaaactgca tgtcccattc ttgctggcag acggcaaatg
caccgtgcct ctagcaccag aacctatgat aacctttggt ttcagatcag tgtcactgaa
actgcaccct aagaatccca catatctaac cacccgccaa cttgctgatg agcctcacta
cacgcacgag ctcatatctg aaccagctgt taggaatttt accgtcaccg aaaaagggtg
ggagtttgta tggggaaacc acccgccgaa aaggttttgg gcacaggaaa cagcacccgg
aaatccacat gggctaccgc acgaggtgat aactcattat taccacagat accctatgtc
caccatcctg ggtttgtcaa tttgtgccgc cattgcaacc gtttccgttg cagcgtctac
ctggctgttt tgcagatcta gagttgcgtg cctaactcct taccggctaa cacctaacgc
taggatacca ttttgtctgg ctgtgctttg ctgcgcccgc actgcccggg ccgagaccac
ctgggagtcc ttggatcacc tatggaacaa taaccaacag atgttctgga ttcaattgct
gatccctctg gccgccttga tcgtagtgac tcgcctgctc aggtgcgtgt gctgtgtcgt
gccttttta gtcatggccg gcgccgcagg cgccggcgcc tacgagcacg cgaccacgat
gccgagccaa gcgggaatct cgtataacac tatagtcaac agagcaggct acgcaccact
ccctatcagc ataacaccaa caaagatcaa gctgatacct acagtgaact tggagtacgt
cacctgccac tacaaaacag gaatggattc accagccatc aaatgctgcg gatctcagga
atgcactcca acttacaggc ctgatgaaca gtgcaaagtc ttcacagggg tttacccgtt
catgtggggt ggtgcatatt gcttttgcga cactgagaac acccaagtca gcaaggccta
cgtaatgaaa tctgacgact gccttgcgga tcatgctgaa gcatataaag cgcacacagc
ctcagtgcag gcgttcctca acatcacagt gggagaacac tctattgtga ctaccgtgta
tgtgaatgga gaaactcctg tgaatttcaa tgggtcaaa ttaactgcag gtccgctttc
cacagcttgg acacccttg atcgcaaaat cgtgcagtat gccgggggaga tctataatta
tgattttcct gagtatgggg caggacaacc aggagcattt ggagatatac aatccagaac
agtctcaagc tcagatctgt atgccaatac caacctagtg ctgcagagac ccaaagcagg
agcgatccac gtgccataca ctcaggcacc ttcgggtttt gagcaatgga agaaagataa
agctccatca ttgaaattta ccgcccctt cggatgcgaa atatatacaa accccattcg
cgccgaaaac tgtgctgtag ggtcaattcc attagccttt gacattcccg acgccttgtt
caccagggtg tcagaaacac cgacactttc agcggccgaa tgcactctta acgagtgcgt
gtattcttcc gactttggtg ggatcgccac ggtcaagtac tcggccagca agtcaggcaa
gtgcgcagtc catgtgccat cagggactgc taccctaaaa gaagcagcag tcgagctaac
cgagcaaggg tcggcgacta tccatttctc gaccgcaaat atccacccgg agttcaggct
ccaaatatgc acatcatatg ttacgtgcaa aggtgattgt caccccccga aagaccatat
tgtgacacac cctcagtatc acgcccaaac atttacagcc gcggtgtcaa aaaccgcgtg
gacgtggtta acatccctgc tgggaggatc agccgtaatt attataattg gcttggtgct
ggctactatt gtggccatgt acgtgctgac caaccagaaa cataattgaa tacagcagca
attggcaagc tgcttacata gaactcgcgg cgattggcat gccgccttaa aattttatt
ttatttttc ttttcttttc cgaatcggat tttgttttta atatttc
```

VEE-MAG25mer (SEQ ID NO:4); contains MAG-25merPDTT nucleotide (bases 30-1755)

```
        atgggcggcgcatgagagaagcccagaccaattacctacccaaaatggagaaagttcacgttgacatc
gaggaagacagcccattcctcagagctttgcagcggagcttcccgcagtttgaggtagaagccaagcaggtcac
tgataatgaccatgctaatgccagagcgtttttcgcatctggcttcaaaactgatcgaaacggaggtggacccat
ccgacacgatccttgacattggaagtgcgcccgcccgcagaatgtattctaagcacaagtatcattgtatctgt
ccgatgagatgtgcggaagatccggacagattgtataagtatgcaactaagctgaagaaaaactgtaaggaaat
aactgataaggaattggacaagaaaatgaaggagctcgccgccgtcatgagcgaccctgacctggaaactgaga
ctatgtgcctccacgacgacgagtcgtgtcgctacgaaggcaagtcgctgttttaccaggatgtatacgcggtt
gacggaccgacaagtctctatcaccaagccaataagggagttagagtcgcctactggataggctttgacaccac
cccttttatgtttaagaacttggctggagcatatccatcatactctaccaactgggccgacgaaaccgtgttaa
cggctcgtaacataggcctatgcagctctgacgttatggagcggtcacgtagagggatgtccattcttagaaag
aagtatttgaaaccatccaacaatgttctattctctgttggctcgaccatctaccacgagaagagggacttact
gaggagctggacacctgccgtctgtatttcacttacgtggcaagcaaaattacacatgtcggtgtgagactatag
ttagttgcgacgggtacgtcgttaaaagaatagctatcagtccaggcctgtatgggaagccttcaggctatgct
gctacgatgcaccgcgagggattcttgtgctgcaaagtgacagacacattgaacgggagagggtctcttttcc
cgtgtgcacgtatgtgccagctacattgtgtgaccaaatgactggcatactggcaacagatgtcagtgcggacg
acgcgcaaaaactgctggttgggctcaaccagcgtatagtcgtcaacggtcgcacccagagaaacaccaatacc
atgaaaaattaccttttgcccgtagtggcccaggcatttgctaggtgggcaaaggaatataaggaagatcaaga
agatgaaaggccactaggactacgagatagacagttagtcatggggtgttgttgggcttttagaaggcacaaga
```

(continued)

```
taacatctatttataagcgcccggatacccaaaccatcatcaaagtgaacagcgatttccactcattcgtgctg
cccaggataggcagtaacacattggagatcgggctgagaacaagaatcaggaaaatgttagaggagcacaagga
gccgtcacctctcattaccgccgaggacgtacaagaagctaagtgcgcagccgatgaggctaaggaggtgcgtg
aagccgaggagttgcgcgcagctctaccacctttggcagctgatgttgaggagcccactctggaagccgatgtc
gacttgatgttacaagaggctggggccggctcagtggagacacctcgtggcttgataaaggttaccagctacgc
tggcgaggacaagatcggctcttacgctgtgctttcctccgcaggctgtactcaagagtgaaaaattatcttgca
tccaccctctcgctgaacaagtcatagtgataacacactctggccgaaaagggcgttatgccgtggaaccatac
catggtaaagtagtggtgccagagggacatgcaataccccgtccaggactttcaagctctgagtgaaagtgccac
cattgtgtacaacgaacgtgagttcgtaaacaggtacctgcaccatattgccacacatggaggagcgctgaaca
ctgatgaagaatattacaaaactgtcaagcccagcgagcacgacggcgaatacctgtacgacatcgacaggaaa
cagtgcgtcaagaaagaactagtcactgggctagggctcacaggcgagctggtggatcctcccttccatgaatt
cgcctacgagagtctgagaacacgaccagccgcgctccttaccaagtaccaaccataggggtgtatggcgtgccag
gatcaggcaagtctggcatcattaaaagcgcagtcaccaaaaaagatctagtggtgagcgccaagaaagaaaac
tgtgcagaaattataagggacgtcaagaaaatgaaagggctggacgtcaatgccagaactgtggactcagtgct
cttgaatggatgcaaacaccccgtagagaccctgtatattgacgaagcttttgcttgtcatgcaggtactctca
gagcgctcatagccattataagacctaaaaaggcagtgctctgcggggatcccaaacagtgcggttttttaac
atgatgtgcctgaaagtgcattttaaccacgagatttgcacacaagtcttccacaaaagcatctctcgccgttg
cactaaatctgtgacttcggtcgtctcaaccttgtttacgacaaaaaatgagaacgacgaatccgaaagaga
ctaagattgtgattgacactaccggcagtaccaaacctaagcaggacgatctcattcttcacttgtttcagaggg
tgggtgaagcagttgcaaatagattacaaaggcaacgaaataatgacggcagctgcctctcaagggctgacccg
taaaggtgtgtatgccgttcggtacaaggtgaatgaaaatcctctgtacgcacccacctcagaacatgtgaacg
tcctactgacccgcacggaggaccgcatcgtgtggaaaacactagccggcgacccatggataaaaacactgact
gccaagtaccctgggaatttcactgccacgatagaggagtggcaagcagagcatgatgccatcatgaggcacat
cttggagagaccggaccctaccgacgtcttccagaataaggcaaacgtgtgttgggccaaggctttagtgccgg
tgctgaagaccgctggcatagacatgaccactgaacaatggaacactgtggattattttgaaacggacaaagct
cactcagcagagatagtattgaaccaactatgcgtgaggttctttggactcgatctggactccggtctattttc
tgcacccactgttccgttatccattaggaataatcactgggataactcccgtcgcctaacatgtacgggctga
ataaagaagtggtccgtcagctctctcgcaggtacccacaactgcctcgggcagttgccactggaagagtctat
gacatgaacactggtacactgcgcaattatgatccgcgcataaacctagtacctgtaaacagaagactgcctca
tgctttagtcctccaccataatgaacacccacagagtgacttttcttcattcgtcagcaaattgaagggcagaa
ctgtcctggtggtcggggaaaaagttgtccgtcccaggcaaaatggttgactggttgtcagaccggcctgaggct
accttcagagctcggctggatttaggcatcccaggtgatgtgcccaaatatgacataatatttgttaatgtgag
gaccccatataaataccatcactatcagcagtgtgaagaccatgccattaagcttagcatgttgaccaagaaag
cttgtctgcatctgaatcccggcggaacctgtgtcagcataggttatggttacgctgacagggccagcgaaagc
atcattggtgctatagcgcggcagttcaagttttcccgggtatgcaaaccgaaatcctcacttgaagagacgga
agttctgtttgtattcattgggtacgatcgcaaggcccgtacgcacaatccttacaagctttcatcaaccttga
ccaacatttatacaggttccagactccacgaagccggatgtgcaccctcatatcatgtggtgcgagggggatatt
gccacggccaccgaaggagtgattataaatgctgctaacagcaaaggacaacctggcggagggggtgtgcggagc
gctgtataagaaattcccggaaagcttcgatttacagccgatcgaagtaggaaaagcgcgactggtcaaaggtg
cagctaaacatatcattcatgccgtaggaccaaacttcaacaaagtttcggaggttgaaggtgacaaacagttg
gcagaggcttatgagtccatcgctaagattgtcaacgataacaattacaagtcagtagtacagggatacgccctaaaag
cctcacgtccagaacaaattactgtgtgctcatcctttccattgccgaagtatagaatcactggtgtgcagaag
atccaatgctcccagcctatattgttctcaccgaaagtgcctgcgtatattcatccaaggaagtatctcgtgga
aacaccaccggtagacgagactccggagccatcggcagagaaccaatccacagaggggacacctgaacaaccac
cacttataaccgaggatgagaccaggactagaacgcctgagccgatcatcatcgaagaggaagaagaggatagc
ataagtttgctgtcagatggcccgacccaccaggtgctgcaagtcgaggcagacattcacgggccgccctctgt
atctagctcatcctggtccattcctcatgcatccgactttgatgtggacagtttatccatacttgacaccctgg
aggggagctagcgtgaccagcggggcaacgtcagccgagactaactcttacttcgcaaagagtatgtggagtttctg
gcgcgaccggtgcctgcgcctcgaacagtattcaggaaccctccacatcccgctcccgcgcacaagaacaccgtc
acttgcacccagcaggggcctgctcgagaaccagcctagtttccacccccgccaggcgtgaataggggtgatcacta
gagaggagctcgaggcgcttacccccgtcacgcactcctagcaggtcggtctcgagaaccagcctggtctccaac
ccgccaggcgtaaatagggtgattacaagagaggagtttgaggcgttcgtagcacaacaacaatgacggtttga
tgcgggtgcatacatcttttcctccgacaccggtcaagggcatttacaacaaaaatcagtaaggcaaacggtgc
tatccgaagtggtgttggagaggaccgaattggagatttcgtatgccccgcgcctcgaccaagaaaaagaagaa
ttactacgcaagaaattacagttaaatcccacacctgctaacagaagcagataccagtccaggaaggtggagaa
```

```
catgaaagccataacagctagacgtattctgcaaggcctagggcattatttgaaggcagaaggaaaagtggagt
gctaccgaaccctgcatcctgttcctttgtattcatctagtgtgaaccgtgccttttcaagccccaaggtcgca
gtggaagcctgtaacgccatgttgaaagagaactttccgactgtggcttcttactgtattattccagagtacga
tgcctatttggacatggttgacggagcttcatgctgcttagacactgccagttttttgccctgcaaagctgcgca
gctttccaaagaaacactcctatttggaacccacaatacgatcggcagtgccttcagcgatccagaacacgctc
cagaacgtcctggcagctgccacaaaaagaaattgcaatgtcacgcaaatgagagaattgcccgtattggattc
ggcggcctttaatgtggaatgcttcaagaaatatgcgtgtaataatgaatattgggaaacgtttaaagaaaacc
ccatcaggcttactgaagaaaacgtggtaaattacattaccaaattaaaaggaccaaaagctgctgctctttt
gcgaagacacataatttgaatatgttgcaggacataccaatggacaggtttgtaatggacttaaagagagacgt
gaaagtgactccaggaacaaaacatactgaagaacggcccaaggtacaggtgatccaggctgccgatccgctag
caacagcgtatctgtgcggaatccaccgagagctggttaggagattaaatgcggtcctgcttccgaacattcat
acactgtttgatatgtcggctgaagactttgacgctattatagccgagcacttccagcctggggattgtgttct
ggaaactgacatcgcgtcgtttgataaaagtgaggacgacgccatggctctgaccgcgttaatgattctggaag
acttaggtgtggacgcagagctgttgacgctgattgaggcggctttcggcgaaatttcatcaatacatttgccc
actaaaactaaatttaaattcggagccatgatgaaatctggaatgttcctcacactgtttgtgaacacagtcat
taacattgtaatcgcaagcagagtgttgagagaacggctaaccggatcaccatgtgcagcattcattggagatg
acaatatcgtgaaaggagtcaaatcggacaaattaatggcagacaggtgcgccacctggttgaatatggaagtc
aagattatagatgctgtggtgggcgagaaagcgcctttatttctgtggaggggtttattttgtgtgactccgtgac
cggcacagcgtgccgtgtggcagacccctaaaaaggctgtttaagcttggcaaacctctggcagcagacgatg
aacatgatgatgacaggagaagggcattgcatgaagagtcaacacgctggaaccgagtgggtattctttcagag
ctgtgcaaggcagtagaatcaaggtatgaaaccgtaggaacttccatcatagttatggccatgactactctagc
tagcagtgttaaatcattcagctacctgagagggcccctataactctctacggctaacctgaatggactacga
ctctagaatagtctttaatTAAGCCACCATGGCAGGCATGTTTCAGGCGCTGAGCGAAGGCTGCACCCCGTATG
ATATTAACCAGATGCTGAACGTGCTGGGCGATCATCAGGTCTCAGGCCTTGAGCAGCTTGAGAGTATAATCAAC
TTTGAAAAACTGACTGAATGGACCAGTTCTAATGTTATGCCTATCCTGTCTCCTCTGACAAAGGGCATCCTGGG
CTTCGTGTTTACCCTGACCGTGCCTTCTGAGAGAGGACTTAGCTGCATTAGCGAAGCGGATGCGACCACCCCGG
AAAGCGCGAACCTGGGCGAAGAAATTCTGAGCCAGCTGTATCTTTGGCCAAGGGTGACCTACCATTCCCCTAGT
TATGCTTACCACCAATTTGAAAGACGAGCCAAATATAAAAGACACTTCCCGGCTTTGGCCAGAGCCTGCTGTT
TGGCTACCCTGTGTACGTGTTCGGCGATTGCGTGCAGGGCGATTGGGATGCGATTCGCTTTCGCTATTGCGCGC
CGCCGGGCTATGCGCTGCTGCGCTGCAACGATACCAACTATAGCGCTCTGCTGGCTGTGGGGGCCCTAGAAGGA
CCCAGGAATCAGGACTGGCTTGGTGTCCCAAGACAACTTGTAACTCGGATGCAGGCTATTCAGAATGCCGGCCT
GTGTACCCTGGTGGCCATGCTGGAAGAGACAATCTTCTGGCTGCAAGCGTTTCTGATGGCGCTGACCGATAGCG
GCCCGAAAACCAACATTATTGTGGATAGCCAGTATGTGATGGCCATTAGCAAACCGAGCTTTCAGGAATTTGTG
GATTGGGAAAACGTGAGCCCGGAACTGAACAGCACCGATCAGCCGTTTTGGCAAGCCGGAATCCTGGCCAGAAA
TCTGGTGCCTATGGTGGCCACAGTGCAGGGCCAGAACCTGAAGTACCAGGGTCAGTCACTAGTCATCTCTGCTT
CTATCATTGTCTTCAACCTGCTGGAACTGGAAGGTGATTATCGAGATGATGGCAACGTGTGGGTGCATACCCCG
CTGAGCCCGCGCACCCTGAACGCGTGGGTGAAAGCGGTGGAAGAAAAAAAAGGTATTCCAGTTCACCTAGAGCT
GGCCAGTATGACCAACATGGAGCTCATGAGCAGTATTGTGCATCAGCAGGTCAGAACATACGGCCCCGTGTTCA
TGTGTCTCGGCGGACTGCTTACAATGGTGGCTGGTGCTGTGTGGCTGACAGTGCGAGTGCTCGAGCTGTTCCGG
GCCGCGCAGCTGGCCAACGACGTGGTCCTCCAGATCATGGAGCTTTGTGGTGCAGCGTTTCGCCAGGTGTGCCA
TACCACCGTGCCGTGGCCGAACGCGAGCCTGACCCCGAAATGGAACAACGAAACCACCCAGCCCCAGATCGCCA
ACTGCAGCGTGTATGACTTTTTTGTGTGGCTCCATTATTATTCTGTTCGAGACACACTTTGGCCAAGGGTGACC
TACCATATGAACAAATATGCGTATCATATGCTGGAAAGACGAGCCAAATATAAAAGAGGACCAGGACCTGGCGC
TAAATTTGTGGCCGCCTGGACACTGAAAGCCGCTGCTGGTCCTGGACCTGGCCAGTACATCAAGGCCAACAGCA
AGTTCATCGGCATCACCGAACTCGGACCCGGACCAGGCTGATGATTcgaacggccgtatcacgcccaaacattt
acagccgcggtgtcaaaaaccgcgtggacgtggttaacatccctgctgggaggatcagccgtaattattataat
tggcttggtgctggctactattgtggccatgtacgtgctgaccaaccagaaacataattgaatacagcagcaat
tggcaagctgcttacatagaactcgcggcgattggcatgccgccttaaaattttttatttttattttttctttct
tttccgaatcggatttttgttttaatatttcaaaaaaaaaaaaaaaaaaaaaaaaaaaaaaaaaaaaaaaaaaa
aaaaaaaaaaaaaaaaaaaaaaaaaaaaaaaaaaaaa
```

Venezuelan equine encephalitis virus strain TC-83 [TC-83](SEQ ID NO:5) GenBank: L01443.1

```
atgggcggcg catgagagaa gcccagacca attacctacc caaaatggag aaagttcacg
ttgacatcga ggaagacagc ccattcctca gagctttgca gcggagcttc ccgcagtttg
aggtagaagc caagcaggtc actgataatg accatgctaa tgccagagcg ttttcgcatc
tggcttcaaa actgatcgaa acggaggtgg acccatccga cacgatcctt gacattggaa
gtgcgcccgc ccgcagaatg tattctaagc acaagtatca ttgtatctgt ccgatgagat
gtgcggaaga tccggacaga ttgtataagt atgcaactaa gctgaagaaa aactgtaagg
aaataactga taggaattg gacaagaaaa tgaaggagct cgccgccgtc atgagcgacc
ctgacctgga aactgagact atgtgcctcc acgacgacga tcgtgtcgc tacgaaggc
aagtcgctgt ttaccaggat gtatacgcgg ttgacggacc gacaagtctc tatcaccaag
```

```
ccaataaggg agttagagtc gcctactgga taggctttga caccacccct tttatgttta
agaacttggc tggagcatat ccatcatact ctaccaactg ggccgacgaa accgtgttaa
cggctcgtaa cataggccta tgcagctctg acgttatgga gcggtcacgt agagggatgt
ccattcttag aaagaagtat ttgaaaccat ccaacaatgt tctattctct gttggctcga
ccatctacca cgagaagagg gacttactga ggagctggca cctgccgtct gtatttcact
tacgtggcaa gcaaaattac acatgtcggt gtgagactat agttagttgc gacgggtacg
tcgttaaaag aatagctatc agtccaggcc tgtatgggaa gccttcaggc tatgctgcta
cgatgcaccg cgagggattc ttgtgctgca aagtgacaga cacattgaac ggggagaggg
tctcttttcc cgtgtgcacg tatgtgccag ctacattgtg tgaccaaatg actggcatac
tggcaacaga tgtcagtgcg gacgacgcgc aaaaactgct ggttgggctc aaccagcgta
tagtcgtcaa cggtcgcacc cagagaaaca ccaataccat gaaaaattac cttttgcccg
tagtggccca ggcatttgct aggtgggcaa aggaatataa ggaagatcaa gaagatgaaa
ggccactagg actacgagat agacagttag tcatggggtg ttgttgggct tttagaaggc
acaagataac atctatttat aagcgcccgg atacccaaac catcatcaaa gtgaacagcg
atttccactc attcgtgctg cccaggatag gcagtaacac attggagatc gggctgagaa
caagaatcag gaaaatgtta gaggagcaca aggagccgtc acctctcatt accgccgagg
acgtacaaga agctaagtgc gcagccgatg aggctaagga ggtgcgtgaa gccgaggagt
tgcgcgcagc tctaccacct ttggcagctg atgttgagga gcccactctg gaagccgatg
tcgacttgat gttacaagag gctggggccg gctcagtgga gacacctcgt ggcttgataa
aggttaccag ctacgctggc gaggacaaga tcggctctta cgctgtgctt tctccgcagg
ctgtactcaa gagtgaaaaa ttatcttgca tccaccctct cgctgaacaa gtcatagtga
taacacactc tggccgaaaa gggcgttatg ccgtggaacc ataccatggt aaagtagtgg
tgccagaggg acatgcaata cccgtccagg actttcaagc tctgagtgaa agtgccacca
ttgtgtacaa cgaacgtgag ttcgtaaaca ggtacctgca ccatattgcc acacatggag
gagcgctgaa cactgatgaa gaatattaca aaactgtcaa gcccagcgag cacgacggcg
aatacctgta cgacatcgac aggaaacagt gcgtcaagaa agaactagtc actgggctag
ggctcacagg cgagctggtg gatcctccct tccatgaatt cgcctacgag agtctgagaa
cacgaccagc cgctccttac caagtaccaa ccataggggt gtatggcgtg ccaggatcag
gcaagtctgg catcattaaa agcgcagtca ccaaaaaaga tctagtggtg agcgccaaga
aagaaaactg tgcagaaatt ataagggacg tcaagaaaat gaaagggctg gacgtcaatg
ccagaactgt ggactcagtg ctcttgaatg gatgcaaaca ccccgtagag accctgtata
ttgacgaagc ttttgcttgt catgcaggta ctctcagagc gctcatagcc attataagac
ctaaaaaggc agtgctctgc ggggatccca aacagtgcgg tttttttaac atgatgtgcc
tgaaagtgca ttttaaccac gagatttgca cacaagtctt ccacaaaagc atctctcgcc
gttgcactaa atctgtgact tcggtcgtct caaccttgtt ttacgacaaa aaaatgagaa
cgacgaatcc gaaagagact aagattgtga ttgacactac cggcagtacc aaacctaagc
aggacgatct cattctcact tgtttcagag ggtgggtgaa gcagttgcaa atagattaca
aaggcaacga aataatgacg gcagctgcct ctcaagggct gacccgtaaa ggtgtgtatg
ccgttcggta caaggtgaat gaaaatcctc tgtacgcacc cacctcagaa catgtgaacg
tcctactgac ccgcacggag gaccgcatcg tgtggaaaac actagccggc gacccatgga
taaaaacact gactgccaag taccctggga atttcactgc cacgatagag gagtggcaag
cagagcatga tgccatcatg aggcacatct tggagagacc ggaccctacc gacgtcttcc
agaataaggc aaacgtgtgt tgggccaagg cttttagtgc cggtgctgaag accgctggca
tagacatgac cactgaacaa tggaacactg tggattattt tgaaacggac aaagctcact
cagcagagat agtattgaac caactatgcg tgaggttctt tggactcgat ctggactccg
gtctattttc tgcacccact gttccgttat ccattaggaa taatcactgg gataactccc
cgtcgcctaa catgtacggg ctgaataaag aagtggtccg tcagctctct cgcaggtacc
cacaactgcc tcgggcagtt gccactggaa gagtctatga catgaacact ggtacactgc
gcaattatga tccgcgcata aacctagtac ctgtaaacag aagactgcct catgctttag
tcctccacca taatgaacac ccacagagtg acttttcttc attcgtcagc aaattgaagg
gcagaactgt cctggtggtc ggggaaaagt tgtccgtccc aggcaaaatg gttgactggt
tgtcagaccg gcctgaggct accttcagag ctcggctgga tttaggcatc ccaggtgatg
tgcccaaata tgacataata tttgttaatg tgaggacccc atataaatac catcactatc
agcagtgtga agaccatgcc attaagctta gcatgttgac caagaaagct tgtctgcatc
tgaatcccgg cggaacctgt gtcagcatag gttatggtta cgctgacagg gccagcgaaa
gcatcattgg tgctatagcg cggcagttca agttttcccg ggtatgcaaa ccgaaatcct
cacttgaaga gacggaagtt ctgtttgtat tcattgggta cgatcgcaag gcccgtacgc
acaatcctta caagctttca tcaaccttga ccaacatttta tacaggttcc agactccacg
aagccggatg tgcaccctca tatcatgtgg tgcgagggga tattgccacg gccaccgaag
gagtgattat aaatgctgct aacagcaaag gacaacctgg cggaggggtg tgcggagcgc
tgtataagaa attcccggaa agcttcgatt tacagccgat cgaagtagga aaagcgcgac
tggtcaaagg tgcagctaaa catatcattc atgccgtagg accaaacttc aacaaagttt
```

```
cggaggttga aggtgacaaa cagttggcag aggcttatga gtccatcgct aagattgtca
acgataacaa ttacaagtca gtagcgattc cactgttgtc caccggcatc ttttccggga
acaaagatcg actaacccaa tcattgaacc atttgctgac agctttagac accactgatg
cagatgtagc catatactgc agggacaaga aatgggaaat gactctcaag gaagcagtgg
ctaggagaga agcagtggag gagatatgca tatccgacga ctcttcagtg acagaacctg
atgcagagct ggtgagggtg catccgaaga gttctttggc tggaaggaag ggctacagca
caagcgatgg caaaactttc tcatatttgg aagggaccaa gtttcaccag gcggccaagg
atatagcaga aattaatgcc atgtggcccg ttgcaacgga ggccaatgag caggtatgca
tgtatatcct cggagaaagc atgagcagta ttaggtcgaa atgccccgtc gaagagtcgg
aagcctccac accacctagc acgctgcctt gcttgtgcat ccatgccatg actccagaaa
gagtacagcg cctaaaagcc tcacgtccag aacaaattac tgtgtgctca tcctttccat
tgccgaagta tagaatcact ggtgtgcaga agatccaatg ctcccagcct atattgttct
caccgaaagt gcctgcgtat attcatccaa ggaagtatct cgtggaaaca ccaccggtag
acgagactcc ggagccatcg gcagagaacc aatccacaga ggggacacct gaacaaccac
cacttataac cgaggatgag accaggacta gaacgcctga gccgatcatc atcgaagagg
aagaagagga tagcataagt ttgctgtcag atggcccgac ccaccaggtg ctgcaagtcg
aggcagacat tcacgggccg ccctctgtat ctagctcatc ctggtccatt cctcatgcat
ccgactttga tgtggacagt ttatccatac ttgacaccct ggagggagct agcgtgacca
gcggggcaac gtcagccgag actaactctt acttcgcaaa gagtatggag tttctggcgc
gaccggtgcc tgcgcctcga acagtattca ggaaccctcc acatcccgct ccgcgcacaa
gaacaccgtc acttgcaccc agcagggcct gctcgagaac cagcctagtt tccaccccgc
caggcgtgaa tagggtgatc actagagagg agctcgaggc gcttaccccg tcacgcactc
ctagcaggtc ggtctcgaga accagcctgg tctccaaccc gccaggcgta aatagggtga
ttacaagaga ggagtttgag gcgttcgtag cacaacaaca atgacggttt gatgcgggtg
catacatctt ttcctccgac accggtcaag ggcatttaca acaaaaatca gtaaggcaaa
cggtgctatc cgaagtggtg ttggagagga ccgaattgga gatttcgtat gccccgcgcc
tcgaccaaga aaaagaagaa ttactacgca agaaattaca gttaaatccc acacctgcta
acagaagcag ataccagtcc aggaaggtgg agaacatgaa agccataaca gctagacgta
ttctgcaagg cctagggcat tatttgaagg cagaaggaaa agtggagtgc taccgaaccc
tgcatcctgt tcctttgtat tcatctagtg tgaaccgtgc cttttcaagc cccaaggtcg
cagtggaagc ctgtaacgcc atgttgaaag agaactttcc gactgtggct tcttactgta
ttattccaga gtacgatgcc tatttggaca tggttgacgg agcttcatgc tgcttagaca
ctgccagttt ttgccctgca aagctgcgca gctttccaaa gaaacactcc tatttggaac
ccacaatacg atcggcagtg ccttcagcga tccagaacac gctccagaac gtcctggcag
ctgccacaaa aagaaattgc aatgtcacgc aaatgagaga attgcccgta ttggattcgg
cggcctttaa tgtggaatgc ttcaagaaat atgcgtgtaa taatgaatat tgggaaacgt
ttaaagaaaa ccccatcagg cttactgaag aaaacgtggt aaattacatt accaaattaa
aaggaccaaa agctgctgct ctttttgcga agacacataa tttgaatatg ttgcaggaca
taccaatgga caggtttgta atggacttaa agagagacgt gaaagtgact ccaggaacaa
aacatactga agaacggccc aaggtacagg tgatccaggc tgccgatccg ctagcaacag
cgtatctgtg cggaatccac cgagagctgg ttaggagatt aaatgcggtc ctgcttccga
acattcatac actgtttgat atgtcggctg aagactttga cgctattata gccgagcact
tccagcctgg ggattgtgtt ctggaaactg acatcgcgtc gtttgataaa agtgaggacg
acgccatggc tctgaccgcg ttaatgattc tggaagactt aggtgtggac gcagagctgt
tgacgctgat tgaggcggct ttcggcgaaa tttcatcaat acatttgccc actaaaacta
aatttaaatt cggagccatg atgaaatctg gaatgttcct cacactgttt gtgaacacag
tcattaacat tgtaatcgca agcagagtgt tgagagaacg gctaaccgga tcaccatgtg
cagcattcat tggagatgac aatatcgtga aaggagtcaa atcggacaaa ttaatggcag
acaggtgcgc cacctggttg aatatggaag tcaagattat agatgctgtg gtgggcgaga
aagcgcctta tttctgtgga gggtttattt tgtgtgactc cgtgaccggc acagcgtgcc
gtgtggcaga ccccctaaaa aggctgtta agcttggcaa acctctggca gcagacgatg
aacatgatga tgacaggaga agggcattgc atgaagagtc aacacgctgg aaccgagtgg
gtattctttc agagctgtgc aaggcagtag aatcaaggta tgaaaccgta ggaacttcca
tcatagttat ggccatgact actctagcta gcagtgttaa atcattcagc tacctgagag
gggcccctat aactctctac ggctaacctg aatggactac gacatagtct agtccgccaa
gatgttcccg ttccagccaa tgtatccgat gcagccaatg ccctatcgca acccgttcgc
ggccccgcgc aggccctggt tccccagaac cgaccctttt ctggcgatgc aggtgcagga
attaacccgc tcgatggcta acctgacgtt caagcaacgc cgggacgcgc cacctgaggg
gccatccgct aagaaaccga agaaggaggc ctcgcaaaaa cagaaagggg gaggccaagg
gaagaagaag aagaaccaag ggaagaagaa ggctaagaca gggccgccta atccgaaggc
acagaatgga aacaagaaga agaccaacaa gaaaccaggc aagagacagc gcatggtcat
gaaattggaa tctgacaaga cgttcccaat catgttggaa gggaagataa acggctacgc
```

```
ttgtgtggtc ggagggaagt tattcaggcc gatgcatgtg gaaggcaaga tcgacaacga
cgttctggcc gcgcttaaga cgaagaaagc atccaaatac gatcttgagt atgcagatgt
gccacagaac atgcgggccg atacattcaa atacacccat gagaaacccc aaggctatta
cagctggcat catggagcag tccaatatga aaatgggcgt ttcacggtgc cgaaaggagt
tggggccaag ggagacagcg gacgacccat tctggataac cagggacggg tggtcgctat
tgtgctggga ggtgtgaatg aaggatctag gacagccctt tcagtcgtca tgtggaacga
gaagggagtt accgtgaagt atactccgga gaactgcgag caatggtcac tagtgaccac
catgtgtctg ctcgccaatg tgacgttccc atgtgctcaa ccaccaattt gctacgacag
aaaaccagca gagactttgg ccatgctcag cgttaacgtt gacaacccgg ctacgatga
gctgctggaa gcagctgtta agtgccccgg aaggaaaagg agatccaccg aggagctgtt
taaggagtat aagctaacgc gcccttacat ggccagatgc atcagatgtg cagttgggag
ctgccatagt ccaatagcaa tcgaggcagt aaagagcgac gggcacgacg gttatgttag
acttcagact tcctcgcagt atggcctgga ttcctccggc aacttaaagg gcaggaccat
gcggtatgac atgcacggga ccattaaaga gataccacta catcaagtgt cactccatac
atctcgcccg tgtcacattg tggatgggca cggttatttc ctgcttgcca ggtgcccggc
aggggactcc atcaccatgg aatttaagaa agattccgtc acacactcct gctcggtgcc
gtatgaagtg aaatttaatc ctgtaggcag agaactctat actcatcccc cagaacacgg
agtagagcaa gcgtgccaag tctacgcaca tgatgcacag aacagaggag cttatgtcga
gatgcacctc ccgggctcag aagtggacag cagtttggtt ccttgagcg gcagttcagt
caccgtgaca cctcctgttg ggactagcgc cctggtggaa tgcgagtgtg gcggcacaaa
gatctccgag accatcaaca agacaaaaca gttcagccag tgcacaaaga aggagcagtg
cagagcatat cggctgcaga acgataagtg ggtgtataat tctgacaaac tgcccaaagc
agcgggagcc accttaaaag gaaaactgca tgtcccattc ttgctggcag acggcaaatg
caccgtgcct ctagcaccag aacctatgat aacctttggt ttcagatcag tgtcactgaa
actgcaccct aagaatccca catatctaac cacccgccaa cttgctgatg agcctcacta
cacgcacgag ctcatatctg aaccagctgt taggaatttt accgtcaccg aaaaagggtg
ggagtttgta tggggaaacc acccgccgaa aaggttttgg gcacaggaaa cagcacccgg
aaatccacat gggctaccgc acgaggtgat aactcattat taccacagat accctatgtc
caccatcctg ggtttgtcaa tttgtgccgc cattgcaacc gtttccgttg cagcgtctac
ctggctgttt tgcagatcta gagttgcgtg cctaactcct taccggctaa cacctaacgc
taggatacca tttttgtctgg ctgtgctttg ctgcgcccgc actgcccggg ccgagaccac
ctgggagtcc ttggatcacc tatggaacaa taaccaacag atgttctgga ttcaattgct
gatccctctg gccgccttga tcgtagtgac tcgcctgctc aggtgcgtgt gctgtgtcgt
gccttttttta gtcatggccg gcgccgcagg cgccggcgcc tacgagcacg cgaccacgat
gccgagccaa gcgggaatct cgtataacac tatagtcaac agagcaggct acgcaccact
ccctatcagc ataacaccaa caaagatcaa gctgatacct acagtgaact tggagtacgt
cacctgccac tacaaaacag gaatggattc accagccatc aaatgctgcg gatctcagga
atgcactcca acttacaggc ctgatgaaca gtgcaaagtc ttcacagggg tttacccgtt
catgtgggggt ggtgcatatt gcttttgcga cactgagaac acccaagtca gcaaggccta
cgtaatgaaa tctgacgact gccttgcgga tcatgctgaa gcatataaag cgcacacagc
ctcagtgcag gcgttcctca acatcacagt gggagaacac tctattgtga ctaccgtgta
tgtgaatgga gaaactcctg tgaatttcaa tgggggtcaaa ttaactgcag gtccgctttc
cacagcttgg acacccttttg atcgcaaaat cgtgcagtat gccgggggaga tctataatta
tgattttcct gagtatgggg caggacaacc aggagcattt ggagatatac aatccagaac
agtctcaagc tcagatctgt atgccaatac caacctagtg ctgcagagac ccaaagcagg
agcgatccac gtgccataca ctcaggcacc ttcgggtttt gagcaatgga gaaagataa
agctccatca ttgaaattta ccgccccttt cggatgcgaa atatatacaa accccattcg
cgccgaaaac tgtgctgtag ggtcaattcc attagccttt gacattcccg acgccttgtt
caccaggggtg tcagaaacac cgacactttc agcggccgaa tgcactctta acgagtgcgt
gtattcttcc gactttggtg ggatcgccac ggtcaagtac tcggccagca agtcaggcaa
gtgcgcagtc catgtgccat cagggactgc taccctaaaa gaagcagcag tcgagctaac
cgagcaaggg tcggcgacta ccatttctc gaccgcaaat atccacccgg agttcaggct
ccaaatatgc acatcatatg ttacgtgcaa aggtgattgt caccccccga agaccatat
tgtgacacac cctcagtatc acgcccaaac atttacagcc gcggtgtcaa aaaccgcgtg
gacgtggtta acatccctgc tgggaggatc agccgtaatt attataattg cttggtgct
ggctactatt gtggccatgt acgtgctgac caaccagaaa cataattgaa tacagcagca
attggcaagc tgcttacata gaactcgcgg cgattggcat gccgccttaa aatttttatt
ttattttttc ttttcttttc cgaatcggat tttgttttta atatttc
```

| VEE Delivery Vector (SEQ ID NO:6); VEE genome with nucleotides 7544-11175 deleted [alphavirus structural proteins removed] |
| --- |

```
ATGgcggcgcatgagagagaagcccagaccaattacctacccaaaATGGagaaagttcacgttgacatcgaggaa
gacagcccattcctcagagctttgcagcggagcttcccgcagtttgaggtagaagccaagcaggtcactgataa
```

(continued)

```
tgaccatgctaatgccagagcgtttttcgcatctggcttcaaaaactgatcgaaacggaggtggacccatccgaca
cgatccttgacattggaagtgcgcccgcccgcagaatgtattctaagcacaagtatcattgtatctgtccgatg
agatgtgcggaagatccggacagattgtataagtatgcaactaagctgaagaaaaactgtaaggaaataactga
taaggaattggacaagaaaatgaaggagctcgccgccgtcatgagcgaccctgacctggaaactgagactatgt
gcctccacgacgacgagtcgtgtcgctacgaagggcaagtcgctgtttaccaggatgtatacgcggttgacgga
ccgacaagtctctatcaccaagccaataaggagttagagtcgcctactggataggctttgacaccacccctt
tatgtttaagaacttggctggagcatatccatcatactctaccaactgggccgacgaaaccgtgttaacggctc
gtaacataggcctatgcagctctgacgttatggagcggtcacgtagagggatgtccattcttagaaagaagtat
ttgaaaccatccaacaatgttctattctctgttggctcgaccatctaccacgagaagagggacttactgaggag
ctggcacctgccgtctgtatttcacttacgtggcaagcaaaattacacatgtcggtgtgagactatagttagtt
gcgacgggtacgtcgttaaaagaatagctatcagtccaggcctgtatgggaagccttcaggctatgctgctacg
atgcaccgcgagggattcttgtgctgcaaagtgacagacacattgaacggggagaggggtctcttttttcccgtgtg
cacgtatgtgccagctacattgtgtgaccaaatgactggcatactggcaacagatgtcagtgcggacgacgcgc
aaaaactgctggttgggctcaaccagcgtatagtcgtcaacggtcgcacccagagaaacaccaataccatgaaa
aattaccttttgcccgtagtggcccaggcatttgctaggtgggcaaaggaatataaggaagatcaagaagatga
aaggccactaggactacgagatagacagttagtcatggggtgttgttgggcttttagaaggcacaagataacat
ctatttataagcgcccggatacccaaaccatcatcaaagtgaacagcgatttccactcattcgtgctgcccagg
ataggcagtaacacattggagatcgggctgagaacaagaatcaggaaaatgttagaggagcacaaggagccgtc
acctctcattaccgccgaggacgtacaagaagctaagtgcgcagccgatgaggctaaggaggtgcgtgaagcccg
aggagttgcgcgcagctctaccacctttggcagctgatgttgaggagcccactctggaagccgatgtcgacttg
atgttacaagaggctggggccggctcagtggagacacctcgtggcttgataaaggttaccagctacgctggcga
ggacaagatcggctcttacgctgtgctttctccgcaggctgtactcaagagtgaaaaattatcttgcatccacc
ctctcgctgaacaagtcatagtgataacacactctggccgaaaagggcgttatgccgtggaaccataccatggt
aaagtagtggtgccagagggacatgcaataccgtccaggacttttcaagctctgagtgaaagtgccaccattgt
gtacaacgaacgtgagttcgtaaacaggtacctgcaccatattgccacacatggaggagcgctgaacactgatg
aagaatattacaaaactgtcaagcccagcgagcacgacggcgaataccgtgacacctgtcgacaggaaacagtgc
gtcaagaaagaactagtcactgggctagggctcacaggcgagctggtggatcctcccttccatgaattcgccta
cgagagtctgagaacacgaccagccgctccttaccaagtaccaaccataggggtgtatggcgtgccaggatcag
gcaagtctggcatcattaaaagcgcagtcaccaaaaaagatctagtggtgagcgccaagaaagaaaactgtgca
gaaattataagggacgtcaagaaaatgaaagggctggacgtcaatgccagaactgtggactcagtgctcttgaa
tggatgcaaacaccccgtagagaccctgtatattgacgaagcttttgcttgtcatgcaggtactctcagagcgc
tcatagccattataagacctaaaaaggcagtgctctgcggggatcccaaacagtgcggtttttttttaacatgatg
tgcctgaaagtgcattttaaccacgagatttgcacacaagtcttccacaaaagcatctctcgccgttgcactaa
atctgtgacttcggtcgtctcaaccttgtttttacgacaaaaaaatgagaacgacgaatccgaaagagactaaga
ttgtgattgacactaccggcagtaccaaacctaagcaggacgatctcattctcacttgtttcagagggtgggtg
aagcagttgcaaatagattacaaaggcaacgaaataatgacggcagctgcctctcaagggctgacccgtaaagg
tgtgtatgccgttcggtacaaggtgaatgaaaatcctctgtacgcaccccacctcagaacatgtgaacgtcctac
tgacccgcacggaggaccgcatcgtgtggaaaacactagccggcgacccatggataaaaacactgactgccaag
taccctgggaatttcactgccacgatagaggagtggcaagcagagcatgatgccatcatgaggcacatcttgga
gagaccggaccctaccgacgtcttccagaataaggcaaacgtgtgttgggccaaggctttagtgccggtgctga
agaccgctggcatagacatgaccactgaacaatggcaacatgggattattttgaaacggacaaagctcactca
gcagagatagtattgaaccaactatgcgtgaggttctttggactcgatctggactccggtctattttctgcacc
cactgttccgttatccattaggaataatcactgggataactccccgtcgcctaacatgtacgggctgaataaag
aagtggtccgtcagctctctcgcaggtacccacaactgcctcgggcagttgccactggaagagtctatgacatg
aacactggtacactgcgcaattatgatccgcgcataaacctagtacctgtaaacagaagactgcctcatgctttt
agtcctccaccataatgaacacccacagagtgacttttcttcattcgtcagcaaattgaagggcagaactgtcc
tggtggtcgggaaaagttgtccgtcccaggcaaaatggttgactggttgtcagaccggcctgaggctaccttc
agagctcggctggatttaggcatcccaggtgatgtgcccaaatatgacataatattttgttaatgtgaggacccc
atataaataccatcactatcagcagtgtgaagaccatgccattaagcttagcatgtttgaccaagaaagcttgtc
tgcatctgaatcccggcggaacctgtgtcagcataggttatggttacgctgacagggccagcgaaagcatcatt
ggtgctatagcgcggcagttcaagttttcccgggtatgcaaaccgaaatcctcacttgaagagacggaagttct
gtttgtattcattgggtacgatcgcaaggcccgtacgcacaatccttacaagcttttcatcaaccttgaccaaca
tttatacaggttccagactccacgaagccggatgtgcaccctcatatcatgtggtgcgaggggatattgccacg
gccaccgaaggagtgattataaatgctgctaacagcaaaggacaacctggcggagggggtgtgcggagcgctgta
taagaaattcccggaaagcttcgatttacagccgatcgaagtaggaaaagcgcgactggtcaaaggtgcagcta
aacatatcattcatgccgtaggacccaaacttcaacaaagtttcggaggttgaaggtgacaaacagttggcagag
gcttatgagtccatcgctaagattgtcaacgataacaattacaagtcagtagcgattccactgttgtccaccgg
catcttttccgggaacaaagatcgactaacccaatcattgaaccatttgctgacagctttagacaccactgatg
cagatgtagccatatactgcagggacaagaaatgggaaatgactctcaaggaagcagtggctaggagagaagca
gtggaggagatatgcatatccgacgactcttcagtgacagaacctgatgcagagctggtgagggtgcatccgaa
gagttctttggctggaaggaagggctacagcacaagcgatggcaaaactttctcatatttggaagggaccaagt
ttcaccaggcggccaaggatatagcagaaattaatgccatgtggccgttgcaacggaggccaatgagcaggta
```

**145**

```
tgcatgtatatcctcggagaaagcatgagcagtattaggtcgaaatgccccgtcgaagagtcggaagcctccac
accacctagcacgctgccttgcttgtgcatccatgccatgactccagaaagagtacagcgcctaaaagcctcac
gtccagaacaaattactgtgtgctcatcctttccattgccgaagtatagaatcactggtgtgcagaagatccaa
tgctcccagcctatattgttctcaccgaaagtgcctgcgtatattcatccaaggaagtatctcgtggaaacacc
accggtagacgagactccggagccatcggcagagaaccaatccacagaggggacacctgaacaaccaccactta
taaccgaggatgagaccaggactagaacgcctgagccgatcatcatcgaagaggaagaagaggatagcataagt
ttgctgtcagatggcccgacccaccaggtgctgcaagtcgaggcagacattcacgggccgccctctgtatctag
ctcatcctggtccattcctcatgcatccgactttgatgtggacagtttatccatacttgacaccctggagggag
ctagcgtgaccagcggggcaacgtcagccgagactaactcttacttcgcaaagagtatggagtttctggcgcga
ccggtgcctgcgcctcgaacagtattcaggaaccctccacatcccgctccgcgcacaagaacaccgtcacttgc
acccagcagggcctgctcgagaaccagcctagtttccaccccgccaggcgtgaataggggtgatcactagagagg
agctcgaggcgcttaccccgtcacgcactcctagcaggtcggtctcgagaaccagcctggtctccaacccgcca
ggcgtaaataggggtgattacaagagaggagtttgaggcgttcgtagcacaacaacaatgacggtttgatgcggg
tgcatacatcttttcctccgacaccggtcaagggcatttacaacaaaaatcagtaaggcaaacggtgctatccg
aagtggtgttggagaggaccgaattggagatttcgtatgccccgcgcctcgaccaagaaaaagaagaattacta
cgcaagaaattacagttaaatcccacacctgctaacagaagcagataccagtccaggaaggtggagaacatgaa
agccataacagctagacgtattctgcaaggcctagggcattatttgaaggcagaaggaaaagtggagtgctacc
gaaccctgcatcctgttcctttgtattcatctagtgtgaaccgtgccttttcaagccccaaggtcgcagtggaa
gcctgtaacgccatgttgaaagagaactttccgactgtggcttcttactgtattattccagagtacgatgccta
tttggacatggttgacggagcttcatgctgcttagacactgccagttttttgccctgcaaagctgcgcagctttc
caaagaaacactcctatttggaacccacaatacgatcggcagtgccttcagcgatccagaacacgctccagaac
gtcctggcagctgccacaaaaagaaattgcaatgtcacgcaaatgagagaattgcccgtattggattcggcggc
ctttaatgtggaatgcttcaagaaatatgcgtgtaataatgaatattgggaaacgtttaaagaaaaccccatca
ggcttactgaagaaaacgtggtaaattacattaccaaattaaaaggaccaaaagctgctgctcttttttgcgaag
acacataatttgaatatgttgcaggacataccaatggacaggtttgtaatggacttaaagagagacgtgaaagt
gactccaggaacaaaacatactgaagaacggcccaaggtacaggtgatccaggctgccgatccgctagcaacag
cgtatctgtgcggaatccaccgagagctggttaggagattaaatgcggtcctgcttccgaacattcatacactg
tttgatatgtcggctgaagactttgacgctattatagccgagcacttccagcctgggggattgtgttctggaaac
tgacatcgcgtcgtttgataaaagtgaggacgacgccatggctctgaccgcgttaatgattctggaagacttag
gtgtggacgcagagctgttgacgctgattgaggcggctttcggcgaaatttcatcaatacatttgcccactaaa
actaaatttaaattcggagccatgatgaaatctggaatgttcctcacactgtttgtgaacacagtcattaacat
tgtaatcgcaagcagagtgttgagagaacggctaaccggatcaccatgtgcagcattcattggagatgacaata
tcgtgaaaggagtcaaatcggacaaattaatggcagacaggtgcgccacctggttgaatatggaagtcaagatt
atagatgctgtggtgggcgagaaagcgccttttatttctgtgtgaggggtttttattttgtgtgactccgtgaccggcac
agcgtgccgtgtggcagacccccctaaaaaggctgtttaagcttggcaaacctctgcagcagacgatgaacatg
atgatgacaggagaagggcattgcatgaagagtcaacacgctggaaccgagtgggtattctttcagagctgtgc
aaggcagtagaatcaaggtatgaaaccgtaggaacttccatcatagttatggccatgactactctagctagcag
tgttaaatcattcagctacctgagaggggcccctataactctctacggcTAAcctgaatggggactacgactatca
cgcccaaacatttacagccgcggtgtcaaaaaccgcgtggacgtggttaacatccctgctgggaggatcagccg
taattattataattggcttggtgctggctactattgtggccatgtacgtgctgaccaaccagaaacataattga
atacagcagcaattggcaagctgcttacatagaactcgcggcgattggcatgccgcccttaaaattttttattttta
ttttttctttctttttccgaatcggattttttgtttttaatatttcAAAAAAAAAAAAAAAAAAAAAAAAAAAAAAAAAAAA
AAAAAAAAAAAAAAAAAAAAAAAAAAAAAAAAAAAAAAAAAAAAAAAAA
```

TC-83 Delivery Vector(SEQ ID NO:7); TC-83 genome with nucleotides 7544-11175 deleted [alphavirus structural proteins removed]

```
ATAGGCGGCGCATGAGAGAAGCCCAGACCAATTACCTACCCAAAATGGAGAAAGTTCACGTTGACATCGAGGAA
GACAGCCCATTCCTCAGAGCTTTGCAGCGGAGCTTCCCGCAGTTTGAGGTAGAAGCCAAGCAGGTCACTGATAA
TGACCATGCTAATGCCAGAGCGTTTTCGCATCTGGCTTCAAAACTGATCGAAACGGAGGTGGACCCATCCGACA
CGATCCTTGACATTGGAAGTGCGCCCGCCCGCAGAATGTATTCTAAGCACAAGTATCATTGTATCTGTCCGATG
AGATGTGCGGAAGATCCGGACAGATTGTATAAGTATGCAACTAAGCTGAAGAAAAACTGTAAGGAAATAACTGA
TAAGGAATTGGACAAGAAAATGAAGGAGCTCGCCGCCGTCATGAGCGACCCTGACCTGGAAACTGAGACTATGT
GCCTCCACGACGACGAGTCGTGTCGCTACGAAGGGCAAGTCGCTGTTTACCGAGGATGTATACGCGGTTGACGGA
CCGACAAGTCTCTATCACCAAGCCAATAAGGGAGTTAGAGTCGCCTACTGGATAGGCTTTGACACCACCCCTTT
TATGTTTAAGAACTTGGCTGGAGCATATCCATCATACTCTACCAACTGGGCCGACGAAACCGTGTTAACGGCTC
GTAACATAGGCCTATGCAGCTCTGACGTTATGGAGCGGTCACGTAGAGGGATGTCCATTCTTAGAAAGAAGTAT
TTGAAACCATCCAACAATGTTCTATTCTCTGTTGGCTCGACCATCTACCACGAGAAGAGGGACTTACTGAGGAG
CTGGCACCTGCCGTCTGTATTTCACTTACGTGGCAAGCAAAATTACACATGTCGGTGTGAGACTATAGTTAGTT
GCGACGGGTACGTCGTTAAAAGAATAGCTATCAGTCCAGGCCTGTATGGGAAGCCTTCAGGCTATGCTGCTACG
ATGCACCGCGAGGGATTCTTGTGCTGCAAAGTGACAGACACATTGAACGGGGAGAGGGTCTCTTTTCCCGTGTG
CACGTATGTGCCAGCTACATTGTGTGACCAAATGACTGGCATACTGGCAACAGATGTCAGTGCGGACGACGCGC
AAAAACTGCTGGTTGGGCTCAACCAGCGTATAGTCGTCAACGGTCGCACCCAGAGAAACACCAATACCATGAAA
```

```
AATTACCTTTTGCCCGTAGTGGCCCAGGCATTTGCTAGGTGGGCAAAGGAATATAAGGAAGATCAAGAAGATGA
AAGGCCACTAGGACTACGAGATAGACAGTTAGTCATGGGGTGTTGTTGGGCTTTTAGAAGGCACAAGATAACAT
CTATTTATAAGCGCCCGGATACCCAAACCATCATCAAAGTGAACAGCGATTTCCACTCATTCGTGCTGCCCAGG
ATAGGCAGTAACACATTGGAGATCGGGCTGAGAACAAGAATCAGGAAAATGTTAGAGGAGCACAAGGAGCCGTC
ACCTCTCATTACCGCCGAGGACGTACAAGAAGCTAAGTGCGCAGCCGATGAGGCTAAGGAGGTGCGTGAAGCCG
AGGAGTTGCGCGCAGCTCTACCACCTTTGGCAGCTGATGTTGAGGAGCCCACTCTGGAAGCCGATGTCGACTTG
ATGTTACAAGAGGCTGGGGCCGGCTCAGTGGAGACACCTCGTGGCTTGATAAAGGTTACCAGCTACGATGGCGA
GGACAAGATCGGCTCTTACGCTGTGCTTTCTCCGCAGGCTGTACTCAAGAGTGAAAAATTATCTTGCATCCACC
CTCTCGCTGAACAAGTCATAGTGATAACACACTCTGGCCGAAAAGGGCGTTATGCCGTGGAACCATACCATGGT
AAAGTAGTGGTGCCAGAGGGACATGCAATACCCGTCCAGGACTTTCAAGCTCTGAGTGAAAGTGCCACCATTGT
GTACAACGAACGTGAGTTCGTAAACAGGTACCTGCACCATATTGCCCACACATGGAGGAGCGCTGAACACTGATG
AAGAATATTACAAAACTGTCAAGCCCAGCGAGCACGACGGCGAATACCTGTACGACATCGACAGGAAACAGTGC
GTCAAGAAAGAACTAGTCACTGGGCTAGGGCTCACAGGCGAGCTGGTGGATCCTCCCTTCCATGAATTCGCCTA
CGAGAGTCTGAGAACACGACCAGCCGCTCCTTACCAAGTACCAACCATAGGGGTGTATGGCGTGCCAGGATCAG
GCAAGTCTGGCATCATTAAAAGCGCAGTCACCAAAAAAGATCTAGTGGTGAGCGCCAAGAAAGAAACTGTGCA
GAAATTATAAGGGACGTCAAGAAAATGAAAGGGCTGGACGTCAATGCCAGAACTGTGGACTCAGTGCTCTTGAA
TGGATGCAAACACCCCGTAGAGACCCTGTATATTGACGAAGCTTTTGCTTGTCATGCAGGTACTCTCAGAGCGC
TCATAGCCATTATAAGACCTAAAAAGGCAGTGCTCTGCGGGGATCCCAAACAGTGCGGTTTTTTTAACATGATG
TGCCTGAAAGTGCATTTTAACCACGAGATTTGCACACAAGTCTTCCACAAAAGCATCTCTCGCCGTTGCACTAA
ATCTGTGACTTCGGTCGTCTCAACCTTGTTTTACGACAAAAAAATGAGAACGACGAATCCGAAAGAGACTAAGA
TTGTGATTGACACTACCGGCAGTACCAAACCTAAGCAGGACGATCTCATTCTCACTTGTTTCAGAGGGTGGGTG
AAGCAGTTGCAAATAGATTACAAAGGCAACGAAATAATGACGGCAGCTGCCTCTCAAGGGCTGACCCGTAAAGG
TGTGTATGCCGTTCGGTACAAGGTGAATGAAAATCCTCTGTACGCACCCACCTCAGAACATGTGAACGTCCTAC
TGACCCGCACGGAGGACCGCATCGTGTGGAAAACACTAGCCGGCGACCCATGGATAAAAACACTGACTGCCAAG
TACCCTGGGAATTTCACTGCCACGATAGAGGAGTGGCAAGCAGAGCATGATGCCATCATGAGGCACATCTTGGA
GAGACCGGACCCTACCGACGTCTTCCAGAATAAGGCAAACGTGTGTTGGGCCAAGGCTTTAGTGCCGGTGCTGA
AGACCGCTGGCATAGACATGACCACTGAACAATGGAACACTGTGGATTATTTTGAAACGGACAAAGCTCACTCA
GCAGAGATAGTATTGAACCAACTATGCGTGAGGTTCTTTGGACTCGATCTGGACTCCGGTCTATTTTCTGCACC
CACTGTTCCGTTATCCATTAGGAATAATCACTGGGATAACTCCCCGTCGCCTAACATGTACGGGCTGAATAAAG
AAGTGGTCCGTCAGCTCTCTCGCAGGTACCCACAACTGCCTCGGGCAGTTGCCACTGGAAGAGTCTATGACATG
AACACTGGTACACTGCGCAATTATGATCCGCGCATAAACCTAGTACCTGTAAACAGAAGACTGCCTCATGCTTT
AGTCCTCCACCATAATGAACACCCACAGAGTGACTTTTCTTCATTCGTCAGCAAATTGAAGGGCAGAACTGTCC
TGGTGGTCGGGGAAAAGTTGTCCGTCCCAGGCAAATGGTTGACTGGTTGTCAGACCGGCCTGAGGCTACCTTC
AGAGCTCGGCTGGATTTAGGCATCCCAGGTGATGTGCCCAAATATGACATAATATTTGTTAATGTGAGGACCCC
ATATAAATACCATCACTATCAGCAGTGTGAAGACCATGCCATTAAGCTTAGCATGTTGACCAAGAAAGCTTGTC
TGCATCTGAATCCCGGCGGAACCTGTGTCAGCATAGGTTATGGTTACGCTGACAGGGCCAGCGAAAGCATCATT
GGTGCTATAGCGCGGCAGTTCAAGTTTTCCCGGGTATGCAAACCGAAATCCTCACTTGAAGAGACGGAAGTTCT
GTTTGTATTCATTGGGTACGATCGCAAGGCCCGTACGCACAATCCTTACAAGCTTTCATCAACCTTGACCAACA
TTTATACAGGTTCCAGACTCCACGAAGCCGGATGTGCACCCTCATATCATGTGGTGCGAGGGGATATTGCCACG
GCCACCGAAGGAGTGATTATAAATGCTGCTAACAGCAAAGGACAACCTGGCGGAGGGTGTCGGAGCGCTGTA
TAAGAAATTCCCGGAAAGCTTCGATTTACAGCCGATCGAAGTAGGAAAAGCGCGACTGGTCAAAGGTGCAGCTA
AACATATCATTCATGCCGTAGGACCCAAACTTCAACAAAGTTTCGGAGGTTGAAGGTGACAAACAGTTGGCAGAG
GCTTATGAGTCCATCGCTAAGATTGTCAACGATAACAATTACAAGTCAGTAGCGATTCCACTGTTGTCCACCGG
CATCTTTTCCGGGAACAAAGATCGACTAACCCAATCATTGAACCATTTGCTGACAGCTTTAGACACCACTGATG
CAGATGTAGCCATATACTGCAGGGACAAGAAATGGGAAATGACTCTCAAGGAAGCAGTGGCTAGGAGAGAAGCA
GTGGAGGAGATATGCATATCCGACGACTCTTCAGTGACAGAACCTGATGCAGAGCTGGTGAGGGTGCATCCGAA
GAGTTCTTTGGCTGGAAGGAAGGGCTACAGCACAAGCAAGTCAGTAGCGATTCCACTGTTGTCCACCGG
TTCACCAGGCGGCCAAGGATATAGCAGAAATTAATGCCATGTGGCCCGTTGCAACGGAGGCCAATGAGCAGGTA
TGCATGTATATCCTCGGAGAAAGCATGAGCAGTATTAGGTCGAAATGCCCCGTCGAAGAGTCGGAAGCCTCCAC
ACCACCTAGCACGCTGCCTTGCTTGTGCATCCATGCCATGACTCCAGAAAGAGTACAGCGCCTAAAAGCCTCAC
GTCCAGAACAAATTACTGTGTGCTCATCCTTTCCATTGCCGAAGTATAGAATCACTGGTGTGCAGAAGATCCAA
TGCTCCCAGCCTATATTGTTCTCACCGAAAGTGCCTGCGTATATTCATCCAAGGAAGTATCTCGTGGAAACACC
ACCGGTAGACGAGACTCCGGAGCCATCGGCAGAGAACCAATCCACAGAGGGGACACCTGAACAACCACCACTTA
TAACCGAGGATGAGACCAGGACTAGAACGCCTGAGCCGATCATCATCGAAGAGGAAGAAGAGGATAGCATAAGT
TTGCTGTCAGATGGCCCGACCCACCAGGTGCTGCAAGTCGAGGCAGACATTCACGGGCCGCCCTCTGTATCTAG
CTCATCCTGGTCCATTCCTCATGCATCCGACTTTGATGTGGACAGTTTATCCATACTTGACACCCTGGAGGGAG
CTAGCGTGACCAGCGGGGCAACGTCAGCCGAGACTAACTCTTACTTCGCAAAGAGTATGGAGTTTCTGGCGCGA
CCGGTGCCTGCGCCTCGAACAGTATTCAGGAACCCTCCACATCCCGCTCCGCGCACAAGAACACCGTCACTTGC
ACCCAGCAGGGCCTGCTCGAGAACCAGCCTAGTTTCCACCCCGCCAGGCGTGAATAGGGTGATCACTAGAGAGG
AGCTCGAGGCGCTTACCCCGTCACGCACTCCTAGCAGGTCGGTCTCGAGAACCAGCCTGGTCTCCAACCCGCCA
GGCGTAAATAGGGTGATTACAAGAGAGGAGTTTGAGGCGTTCGTAGCACAACAACAATGACGGTTTGATGCGGG
TGCATACATCTTTTCCTCCGACACCGGTCAAGGGCATTTACAACAAAAATCAGTAAGGCAAACGGTGCTATCCG
```

(continued)

| |
|---|
| AAGTGGTGTTGGAGAGGACCGAATTGGAGATTTCGTATGCCCCGCGCCTCGACCAAGAAAAAGAAGAATTACTA CGCAAGAAATTACAGTTAAATCCCACACCTGCTAACAGAAGCAGATACCAGTCCAGGAAGGTGGAGAACATGAA AGCCATAACAGCTAGACGTATTCTGCAAGGCCTAGGGCATTATTTGAAGGCAGAAGGAAAAGTGGAGTGCTACC GAACCCTGCATCCTGTTCCTTTGTATTCATCTAGTGTGAACCGTGCCTTTTCAAGCCCCAAGGTCGCAGTGGAA GCCTGTAACGCCATGTTGAAAGAGAACTTTCCGACTGTGGCTTCTTACTGTATTATTCCAGAGTACGATGCCTA TTTGGACATGGTTGACGGAGCTTCATGCTGCTTAGACACTGCCAGTTTTTGCCCTGCAAAGCTGCGCAGCTTTC CAAAGAAACACTCCTATTTGGAACCCACAATACGATCGGCAGTGCCTTCAGCGATCCAGAACACGCTCCAGAAC GTCCTGGCAGCTGCCACAAAAAGAAATTGCAATGTCACGCAAATGAGAGAATTGCCCGTATTGGATTCGGCGGC CTTTAATGTGGAATGCTTCAAGAAATATGCGTGTAATAATGAATATTGGGAAACGTTTAAAGAAAACCCCATCA GGCTTACTGAAGAAACGTGGTAAATTACATTACCAAATTAAAAGGACCAAAAGCTGCTGCTCTTTTTGCGAAG ACACATAATTTGAATATGTTGCAGGACATACCAATGGACAGGTTTGTAATGGACTTAAAGAGAGACGTGAAAGT GACTCCAGGAACAAAACATACTGAAGAACGGCCCAAGGTACAGGTGATCCAGGCTGCCGATCCGCTAGCAACAG CGTATCTGTGCGGAATCCACCGAGAGCTGGTTAGGAGATTAAATGCGGTCCTGCTTCCGAACATTCATACACTG TTTGATATGTCGGCTGAAGACTTTGACGCTATTATAGCCGAGCACTTCCAGCCTGGGGATTGTGTTCTGGAAAC TGACATCGCCGTCGTTTGATAAAAGTGAGGACGACGCCATGGCTCTGACCGCGTTAATGATTCTGGAAGACTTAG GTGTGGACGCAGAGCTGTTGACGCTGATTGAGGCGGCTTTCGGCGAAATTTCATCAATACATTTGCCCACTAAA ACTAAATTTAAATTCGGAGCCATGATGAAATCTGGAATGTTCCTCACACTGTTTGTGAACACAGTCATTAACAT TGTAATCGCAAGCAGAGTGTTGAGAGAACGGCTAACCGGATCACCATGTGCAGCATTCATTGGAGATGACAATA TCGTGAAAGGAGTCAAATCGGACAAATTAATGGCAGACAGGTGCGCCACCTGGTTGAATATGGAAGTCAAGATT ATAGATGCTGTGGTGGGCGAGAAAGCGCCTTATTTCTGTGGAGGGTTTATTTTGTGTGACTCCGTGACCGGCAC AGCGTGCCGTGTGGCAGACCCCCTAAAAAGGCTGTTTAAGCTTGGCAAACCTCTGGCAGCAGACGATGAACATG ATGATGACAGGAGAAGGGCATTGCATGAAGAGTCAACACGCTGGAACCGAGTGGGTATTCTTTCAGAGCTGTGC AAGGCAGTAGAATCAAGGTATGAAACCGTAGGAACTTCCATCATAGTTATGGCCATGACTACTCTAGCTAGCAG TGTTAAATCATTCAGCTACCTGAGAGGGGCCCCTATAACTCTCTACGGCTAACCTGAATGGACTACGACTATCA CGCCCAAACATTTACAGCCGCGGTGTCAAAAACCGCGTGGACGTGGTTAACATCCCTGCTGGGAGGATCAGCCG TAATTATTATAATTGGCTTGGTGCTGGCTACTATTGTGGCCATGTACGTGCTGACCAACCAGAAACATAATTGA ATACAGCAGCAATTGGCAAGCTGCTTACATAGAACTCGCGGCGATTGGCATGCCGCCTTAAAATTTTTATTTTA TTTTTCTTTTCTTTTCCGAATCGGATTTTGTTTTTAATATTTCAAAAAAAAAAAAAAAAAAAAAAAAAAAAAAA AAAAAAAAAAAAAAAAAAAAAAAAAAAAAAAAAAAAAAAAAAAAA |
| VEE Production Vector (SEQ ID NO:8); VEE genome with nucleotides 7544-11175 deleted, plus 5' T7-promoter, plus 3' restriction sites |
| TAATACGACTCACTATAGGATGggcggcgcatgagagaagcccagaccaattacctacccaaaATGGagaaagt tcacgttgacatcgaggaagacagcccattcctcagagctttgcagcggagcttcccgcagtttgaggtagaag ccaagcaggtcactgataatgaccatgctaatgccagagcgttttcgcatctggcttcaaaactgatcgaaacg gaggtggacccatccgacacgatccttgacattggaagtgcgcccgcccgcagaatgtattctaagcacaagta tcattgtatctgtccgatgagatgtgcggaagatccggacagattgtataagtatgcaactaagctgaagaaaa actgtaaggaaataactgataaggaattggacaagaaaatgaaggagctcgccgccgtcatgagcgaccctgac ctggaaactgagactatgtgcctccacgacgacgagtcgtgtcgctacgaagggcaagtcgctgtttaccagga tgtatacgcggttgacggaccgacaagtctctatcaccaagtccaataagggagttagagtcgcctactggatag gctttgacaccaccccttttatgtttaagaacttggctggagcatatccatcatactctaccaactgggccgac gaaaccgtgttaacggctcgtaacataggcctatgcagctctgacgttatggagcggtcacgtagagggatgtc cattcttagaaagaagtatttgaaaccatccaacaatgttctattctctgttggctcgaccatctaccacgaga agagggacttactgaggagctggcacctgccgtctgtatttcacttacgtggcaagcaaaattacacatgtcgg tgtgagactatagttagttgcgacgggtacgtcgttaaaagaatagctatcagtccaggcctgtatgggaagcc ttcaggctatgctgctacgatgcaccgcgagggattcttgtgctgcaaagtgacagacacattgaacggggaga gggtctcttttcccgtgtgcacgtatgtgccagctacattgtgtgaccaaaatgactggcatactggcaacagat gtcagtgcggacgacgcgcaaaaactgctggttgggctcaaccagcgtatagtcgtcaacggtcgcacccagag aaacaccaataccatgaaaaattacctttttgcccgtagtggcccaggcatttgctaggtgggcaaaggaatata aggaagatcaagaagatgaaaggccactaggactacgagatagacagttagtcatggggtgttgttgggctttt agaaggcacaagataacatctatttataagcgcccggatacccaaaccatcatcaaagtgaacagcgatttcca ctcattcgtgctgcccaggataggcagtaacacattggagatcgggctgagaacaagaatcaggaaaatgttag aggagcacaaggagccgtcacctctcattaccgccgaggacgtacaagaagctaagtgcgcagccgatgaggct aaggaggtgcgtgaagccgaggagttgcgcgcagctctaccaccctttggcagctgatgttgaggagcccactct ggaagccgatgtcgacttgatgttacaagaggctggggccggctcagtggagacacctcgtggcttgataaagg ttaccagctacgctggcgaggacaagatcggctcttacgctgtgctttctccgcaggctgtactcaagagtgaa aaattatcttgcatccaccctctcgctgaacaagtcatagtgataacacactctggccgaaaagggcgttatgc cgtggaaccataccatggtaaagtagtggtgccagagggacatgcaatacccgtccaggactttcaagtctctga gtgaaagtgccaccattgtgtacaacgaacgtgagttcgtaaacaggtacctgcaccatattgccacacatgga ggagcgctgaacactgatgaagaatattacaaaactgtcaagcccagcgagcacgacggcgaatacctgtacga catcgacaggaaacagtgcgtcaagaaagaactagtcactgggctagggctcacaggcgagctggtggatcctc ccttccatgaattcgcctacgagagtctgagaacacgaccagccgctccttaccaagtaccaaccatagggg tg |

(continued)

```
tatggcgtgccaggatcaggcaagtctggcatcattaaaagcgcagtcaccaaaaaagatctagtggtgagcgc
caagaaagaaaactgtgcagaaattataagggacgtcaagaaaatgaaagggctggacgtcaatgccagaactg
tggactcagtgctcttgaatggatgcaaacaccccgtagagaccctgtatattgacgaagcttttgcttgtcat
gcaggtactctcagagcgctcatagccattataagacctaaaaaggcagtgctctgcggggatcccaaacagtg
cggtttttttaacatgatgtgcctgaaagtgcattttaaccacgagatttgcacacaagtcttccacaaaaagca
tctctcgccgttgcactaaatctgtgacttcggtcgtctcaaccttgtttttacgacaaaaaaatgagaacgacg
aatccgaaagagactaagattgtgattgacactaccggcagtaccaaacctaagcaggacgatctcattctcac
ttgtttcagagggtgggtgaagcagttgcaaatagattacaaaggcaacgaaataatgacggcagctgcctctc
aagggctgacccgtaaaggtgtgtatgccgttcggtacaaggtgaatgaaaatcctctgtacgcacccacctca
gaacatgtgaacgtcctactgacccgcacggaggaccgcatcgtgtggaaaacactagccggcgacccatggat
aaaaacactgactgccaagtaccctgggaatttcactgccacgatagaggagtggcaagcagagcatgatgcca
tcatggaggcacatcttggagagaccggaccctaccgacgtcttccagaataaggcaaacgtgtgttgggccaag
gctttagtgccggtgctgaagaccgctggcatagacatgaccactgaacaatggaacactgtggattattttga
aacggacaaagctcactcagcagagatagtattgaaccaactatgcgtgaggttctttggactcgatctggact
ccggtctattttctgcacccactgttccgttatccattaggaataatcactgggataactccccgtcgcctaac
atgtacgggctgaataaagaagtggtccgtcagctctctcgcaggtacccacaactgcctcgggcagttgccac
tggaagagtctatgacatgaacactggtacactgcgcaattatgatccgcgcataaacctagtacctgtaaaca
gaagactgcctcatgctttagtcctccaccataatgaacacccacagagtgacttttcttcattcgtcagcaaa
ttgaagggcagaactgtcctggtggtcggggaaaagttgtccgtcccaggcaaaatggttgactggttgtcaga
ccggcctgaggctaccttcagagctcggctggatttaggcatcccaggtgatgtgcccaaatatgacataatat
ttgttaatgtgaggaccccatataaataccatcactatcagcagtgtgaagaccatgccattaagcttagcatg
ttgaccaagaaagcttgtctgcatctgaatcccggcggaacctgtgtcagcataggttatggttacgctgacag
ggccagcgaaagcatcattggtgctatagcgcggcagttcaagttttcccgggtatgcaaaccgaaatcctcac
ttgaagagacggaagttctgtttgtattcattgggtacgatcgcaaggcccgtacgcacaatccttacaagctt
tcatcaaccttgaccaacatttatacaggttccagactccacgaagccggatgtgcaccctcatatcatgtggt
gcgagggggatattgccacggccaccgaaggagtgattataaatgctgctaacagcaaaggacaacctggcggag
gggtgtgcggagcgctgtataagaaattcccggaaagcttcgatttacagccgatcgaagtaggaaaagcgcga
ctggtcaaaggtgcagctaaacatatcattcatgccgtaggaccaaacttcaacaaagtttcggaggttgaagg
tgacaaacagttggcagaggcttatgagtccatcgctaagattgtcaacgataacaattacaagtcagtagcga
ttccactgttgtccaccggcatctttttccgggaacaaagatcgactaacccaatcattgaaccatttgctgaca
gctttagacaccactgatgcagatgtagccatatactgcagggacaagaaatgggaaatgactctcaaggaagc
agtggctaggagagaagcagtggaggagatatgcatatccgacgactcttcagtgacagaacctgatgcagagc
tggtgagggtgcatccgaagagttctttggctggaaggaagggctacagcacaagcgatggcaaaactttctca
tatttggaagggaccaagtttcaccaggcggccaaggatatagcagaaattaatgccatgtggcccgtttgcaac
ggaggccaatgagcaggtatgcatgtatatcctcggagaaagcatgagcagtattaggtcgaaatgccccgtcg
aagagtcggaagcctccacaccacctagcacgctgccttgcttgtgcatccatgccatgactccagaaagagta
cagcgcctaaaagcctcacgtccagaacaaattactgtgtgctcatcctttccattgccgaagtatagaatcac
tggtgtgcagaagatccaatgctcccagcctatattgttctcaccgaaagtgcctgcgtatattcatccaagga
agtatctcgtggaaacaccaccggtagacgagactccggagccatcggcagagaaccaatccacagaggggaca
cctgaacaaccaccacttataaccgaggatgagaccaggactagaacgcctgagccgatcatcatcgaagagga
agaagaggatagcataagtttgctgtcagatggcccgacccaccaggtgctgcaagtcgaggcagacattcacg
ggccgccctctgtatcagtctcatcctggtccattcctcatgcatccgactttgatgtggacagtttatccata
cttgacaccctggagggagctagcgtgaccagcggggcaacgtcagccgagactaactcttacttcgcaaagag
tatggagtttctggcgcgaccggtgcctgcgcctcgaacagtattcaggaaccctccacatcccgctccgcgca
caagaacaccgtcacttgcacccagcagggcctgctcgagaaccagcctagtttccacccccgccaggcgtgaat
aggtgtgatcactagagaggagctcgaggcgcttacccgtcacgcactcctagcaggtcggtctcgagaaccag
cctggtctccaacccgccaggcgtaaataggqtgattacaagagaggagtttgagqcgttcgtagcacaacaac
aatgacggtttgatgcgggtgcatacatctttttcctccgacaccggtcaaggqcatttacaacaaaaatcagta
aggcaaacggtgctatccgaagtggtgttggagaggaccgaattggagatttcgtatgccccgcgcctcgacca
agaaaaagaagaattactacgcaagaaattacagttaaatcccacacctgctaacagaagcagataccagtcca
ggaaggtggagaacatgaaagccataacagctagacgtattctgcaaggcctagggcattatttgaaggcagaa
ggaaaagtggagtgctaccgaaccctgcatcctgttcctttgtattcatctagtgtgaaccgtgccttttcaag
ccccaaggtcgcagtggaagcctgtaacgccatgttgaaagagaactttccgactgtggcttcttactgtatta
ttccagagtacgatgcctatttggacatggttgacggagcttcatgctgcttagacactgccagttttttgccct
gcaaagctgcgcagctttccaaagaaacactcctatttggaacccacaatacgatcggcagtgccttcagcgat
ccagaacacgctccagaacgtcctggcagctgccacaaaaagaaattgcaatgtcacgcaaatgagagaattgc
ccgtattggattcggcggcccttaatgtggaatgcttcaagaaatatgcgtgtaataatgaatattgggaaacg
tttaaagaaaaccccatcaggcttactgaagaaaacgtggtaaattacattaccaaattaaaaaggaccaaaagc
tgctgctcttttttgcgaagacacataatttgaatatgttgcaggacataccaatggacaggtttgtaatggact
taaagagagacgtgaaagtgactccaggaacaaaacatactgaagaacggcccaaggtacaggtgatccaggct
gccgatccgctagcaacagcgtatctgtgcggaatccaccgagagctggttaggagattaaatgcggtcctgct
tccgaacattcatacactgtttgatatgtcggctgaagactttgacgctattatagccgagcacttccagcctg
```

(continued)

```
gggattgtgttctggaaactgacatcgcgtcgtttgataaaagtgaggacgacgccatggctctgaccgcgtta
atgattctggaagacttaggtgtggacgcagagctgttgacgctgattgaggcggctttcggcgaaatttcatc
aatacatttgcccactaaaactaaatttaaattcggagccatgatgaaatctggaatgttcctcacactgtttg
tgaacacagtcattaacattgtaatcgcaagcagagtgttgagagaacggctaaccggatcaccatgtgcagca
ttcattggagatgacaatatcgtgaaaggagtcaaatcggacaaattaatggcagacaggtgcgccacctggtt
gaatatggaagtcaagattatagatgctgtggtgggcgagaaagcgccttatttctgtggagggtttattttgt
gtgactccgtgaccggcacagcgtgccgtgtggcagaccccctaaaaaggctgtttaagcttggcaaacctctg
gcagcagacgatgaacatgatgatgacaggagaagggcattgcatgaagagtcaacacgctggaaccgagtggg
tattctttcagagctgtgcaaggcagtagaatcaaggtatgaaaccgtaggaacttccatcatagttatggcca
tgactactctagctagcagtgttaaatcattcagctacctgagagggggcccctataactctctacggcTAAcct
gaatggactacgactatcacgcccaaacatttacagccgcggtgtcaaaaaccgcgtggacgtggttaacatcc
ctgctgggaggatcagccgtaattattataattggcttggtgctggctactattgtggccatgtacgtgctgac
caaccagaaacataattgaatacagcagcaattggcaagctgcttacatagaactcgcggcgattggcatgccg
ccttaaaattttttatttttatttttcttttcttttccgaatcggattttgtttttaatatttcAAAAAAAAAA
AAAAAAAAAAAAAAAAAAAAAAAAAAAAAAAAAAAAAAAAAAAAAAAAAAAAAAAAAAAAAAAAAAAAAAAAAA
tacgtagtttaaac
```

TC-83 Production Vector(SEQ ID NO:9); TC-83 genome with nucleotides 7544-11175 deleted, plus 5' T7-promoter, plus 3' restriction sites

```
TAATACGACTCACTATAGGATAGGCGGCGCATGAGAGAAGCCCAGACCAATTACCTACCCAAAATGGAGAAAGT
TCACGTTGACATCGAGGAAGACAGCCCATTCCTCAGAGCTTTGCAGCGGAGCTTCCCGCAGTTTGAGGTAGAAG
CCAAGCAGGTCACTGATAATGACCATGCTAATGCCAGAGCGTTTTCGCATCTGGCTTCAAAACTGATCGAAACG
GAGGTGGACCCATCCGACACGATCCTTGACATTGGAAGTGCGCCCGCCCGCAGAATGTATTCTAAGCACAAGTA
TCATTGTATCTGTCCGATGAGATGTGCGGAAGATCCGGACAGATTGTATAAGTATGCAACTAAGCTGAAGAAAA
ACTGTAAGGAAATAACTGATAAGGAATTGGACAAGAAAATGAAGGAGCTCGCCGCCGTCATGAGCGACCCTGAC
CTGGAAACTGAGACTATGTGCCTCCACGACGACGAGTCGTGTCGCTACGAAGGGCAAGTCGCTGTTTACCAGGA
TGTATACGCGGTTGACGGACCGACAAGTCTCTATCACCAAGCCAATAAGGGAGTTAGAGTCGCCTACTGGATAG
GCTTTGACACCACCCCTTTTATGTTTAAGAACTTGGCTGGAGCATATCCATCATACTCTACCAACTGGGCCGAC
GAAACCGTGTTAACGGCTCGTAACATAGGCCTATGCAGCTCTGACGTTATGGAGCGGTCACGTAGAGGGATGTC
CATTCTTAGAAAGAAGTATTTGAAACCATCCAACAATGTTCTATTCTCTGTTGGCTCGACCATCTACCACGAGA
AGAGGGACTTACTGAGGAGCTGGCACCTGCCGTCTGTATTTCACTTACGTGGCAAGCAAAATTACACATGTCGG
TGTGAGACTATAGTTAGTTGCGACGGGTACGTCGTTAAAAGAATAGCTATCAGTCCAGGCCTGTATGGGAAGCC
TTCAGCTATGCTGCTACGATGCACCGCGAGGGATTCTTGTGCTGCAAAGTGACAGACACATTGAACGGGGAGA
GGGTCTCTTTTCCCGTGTGCACGTATGTGCCAGCTACATTGTGTGACCAAATGACTGGCATACTGGCAACAGAT
GTCAGTGCGGACGACGCGCAAAAACTGCTGGTTGGGCTCAACCAGCGTATAGTCGTCAACGGTCGCACCCAGAG
AAACACCAATACCATGAAAAATTACCTTTTGCCCGTAGTGGCCCAGGCATTTGCTAGGTGGGCAAAGGAATATA
AGGAAGATCAAGAAGATGAAAGGCCACTAGGACTACGAGATAGACAGTTAGTCATGGGGTGTTGTTGGGCTTTT
AGAAGGCACAAGATAACATCTATTTATAAGCGCCCGGATACCCAAACCATCATCAAAGTGAACAGCGATTTCCA
CTCATTCGTGCTGCCCAGGATAGGCAGTAACACATTGGAGATCGGGCTGAGAACAAGAATCAGGAAAATGTTAG
AGGAGCACAAGGAGCCGTCACCTCTCATTACCGCCGAGGACGTACAAGAAGCTAAGTGCGCAGCCGATGAGGCT
AAGGAGGTGCGTGAAGCCGAGGAGTTGCGCGCAGCTCTACCACCTTTGGCAGCTGATGTTGAGGAGCCCACTCT
GGAAGCCGATGTCGACTTGATGTTACAAGAGGCTGGGGCCGGCTCAGTGGAGACACCTCGTGGCTTGATAAAGG
TTACCAGCTACGATGGCGAGGACAAGATCGGCTCTTACGCTGTGCTTTCTCCGCAGGCTGTACTCAAGAGTGAA
AAATTATCTTGCATCCACCCTCTCGCTGAACAAGTCATAGTGATAACACACTCTGGCCGAAAAGGGCGTTATGC
CGTGGAACCATACCATGGTAAAGTAGTGGTGCCAGAGGGACATGCAATACCCGTCCAGGACTTTCAAGCTCTGA
GTGAAAGTGCCACCATTGTGTACAACGAACGTGAGTTCGTAAACAGGTACCTGCACCATATTGCCACACATGGA
GGAGCGCTGAACACTGATGAAGAATATTACAAAACTGTCAAGCCCAGCGAGCACGACGGCGAATACCTGTACGA
CATCGACAGGAAACAGTGCGTCAAGAAAGAACTAGTCACTGGGCTAGGGCTCACAGGCGAGCTGGTGGATCCTC
CCTTCCATGAATTCGCCTACGAGAGTCTGAGAACACGACCAGCCGCTCCTTACCAAGTACCAACCATAGGGGTG
TATGGCGTGCCCAGGATCAGGCAAGTCTGGCATCATTAAAAGCGCAGTCACCAAAAAAGATCTAGTGGTGAGCGC
CAAGAAAGAAACTGTCAGAAATTATAAGGGACGTCAAGAAAATGAAAGGGCTGGACGTCAATGCCAGAACTG
TGGACTCAGTGCTCTTGAATGGATGCAAACACCCCGTAGAGACCCTGTATATTGACGAAGCTTTTGCTTGTCAT
GCAGGTACTCTCAGAGCGCTCATAGCCATTATAAGACCTAAAAAGGCAGTGCTCTGCGGGGATCCCAAACAGTG
CGGTTTTTTTAACATGATGTGCCTGAAAGTGCATTTTAACCACGAGATTTGCACACAAGTCTTCCACAAAAGCA
TCTCTCGCCGTTGCACTAAATCTGTGACTTCGGTCGTCTCAACCTTGTTTTACGACAAAAAAATGAGAACGACG
AATCCGAAAGAGACTAAGATTGTGATTGACACTACCGGCAGTACCAAACCTAAGCAGGACGATCTCATTCTCAC
TTGTTTCAGAGGGTGGGTGAAGCAGTTGCAAATAGATTACAAAGGCAACGAAATAATGACGGCAGCTGCCTCTC
AAGGGCTGACCCGTAAAGGTGTGTATGCCGTTCGGTACAAGGTGAATGAAAATCCTCTGTACGCACCCACCTCA
GAACATGTGAACGTCCTACTGACCCGCACGGAGGACCGCATCGTGTGGAAAACACTAGCCGGCGACCCATGGAT
AAAAACACTGACTGCCAAGTACCCTGGGAATTTCACTGCCACGATAGAGGAGTGGCAAGCAGAGCATGATGCCA
TCATGAGGCACATCTTGGAGAGACCGGACCCTACCGACGTCTTCCAGAATAAGGCAAACGTGTGTTGGGCCAAG
GCTTTAGTGCCGGTGCTGAAGACCGCTGGCATAGACATGACCACTGAACAATGGAACACTGTGGATTATTTTGA
```

```
AACGGACAAAGCTCACTCAGCAGAGATAGTATTGAACCAACTATGCGTGAGGTTCTTTGGACTCGATCTGGACT
CCGGTCTATTTTCTGCACCCACTGTTCCGTTATCCATTAGGAATAATCACTGGGATAACTCCCCGTCGCCTAAC
ATGTACGGGCTGAATAAAGAAGTGGTCCGTCAGCTCTCTCGCAGGTACCCACAACTGCCTCGGGCAGTTGCCAC
TGGAAGAGTCTATGACATGAACACTGGTACACTGCGCAATTATGATCCGCGCATAAACCTAGTACCTGTAAACA
GAAGACTGCCTCATGCTTTAGTCCTCCACCATAATGAACACCCACAGAGTGACTTTTCTTCATTCGTCAGCAAA
TTGAAGGGCAGAACTGTCCTGGTGGTCGGGGAAAAGTTGTCCGTCCCAGGCAAAATGGTTGACTGGTTGTCAGA
CCGGCCTGAGGCTACCTTCAGAGCTCGGCTGGATTTAGGCATCCCAGGTGATGTGCCCAAATATGACATAATAT
TTGTTAATGTGAGGACCCCATATAAATACCATCACTATCAGCAGTGTGAAGACCATGCCATTAAGCTTAGCATG
TTGACCAAGAAAGCTTGTCTGCATCTGAATCCCGGCGGAACCTGTGTCAGCATAGGTTATGGTTACGCTGACAG
GGCCAGCGAAAGCATCATTGGTGCTATAGCGCGGCAGTTCAAGTTTTCCCGGGTATGCAAACCGAAATCCTCAC
TTGAAGAGACGGAAGTTCTGTTTGTATTCATTGGGTACGATCGCAAGGCCCGTACGCACAATCCTTACAAGCTT
TCATCAACCTTGACCAACATTTATACAGGTTCCAGACTCCACGAAGCCGGATGTGCACCCTCATATCATGTGGT
GCGAGGGGATATTGCCACGGCCACCGAAGGAGTGATTATAAATGCTGCTAACAGCAAAGGACAACCTGGCGGAG
GGGTGTGCGGAGCGCTGTATAAGAAATTCCCGGAAAGCTTCGATTTACAGCCGATCGAAGTAGGAAAAGCGCGA
CTGGTCAAAGGTGCAGCTAAACATATCATTCATGCCGTAGGACCAAACTTCAACAAAGTTTCGGAGGTTGAAGG
TGACAAACAGTTGGCAGAGGCTTATGAGTCCATCGCTAAGATTGTCAACGATAACAATTACAAGTCAGTAGCGA
TTCCACTGTTGTCCACCGGCATCTTTTCCGGGAACAAAGATCGACTAACCCAATCATTGAACCATTTGCTGACA
GCTTTAGACACCACTGATGCAGATGTAGCCCATATACTGCAGGGACAAGAAATGGGAAATGACTCTCAAGGAAGC
AGTGGCTAGGAGAGAAGCAGTGGAGGAGATATGCATATCCGACGACTCTTCAGTGACAGAACCTGATGCGAGAGC
TGGTGAGGGTGCATCCGAAGAGTTCTTTGGCTGGAAGGAAGGGCTACAGCACAAGCGATGGCAAAACTTTCTCA
TATTTGGAAGGGACCAAGTTTCACCAGGCGGCCAAGGATATAGCAGAAATTAATGCCATGTGGCCCGTTGCAAC
GGAGGCCAATGAGCAGGTATGCATGTATATCCTCGGAGAAAGCATGAGCAGTATTAGGTCGAAATGCCCCGTCG
AAGAGTCGGAAGCCTCCACACCACCTAGCACGCTGCCTTGCTTGTGCATCCATGCCATGACTCCAGAAAGAGTA
CAGCGCCTAAAAGCCTCACGTCCAGAACAAATTACTGTGTGCTCATCCTTTCCATTGCCGAAGTATAGAATCAC
TGGTGTGCAGAAGATCCAATGCTCCCAGCCTATATTGTTCTCACCGAAAGTGCCTGCGTATATTCATCCAAGGA
AGTATCTCGTGGAAACACCACCGGTAGACGAGACTCCGGAGCCATCGGCAGAGAACCAATCCACAGAGGGGACA
CCTGAACAACCACCACTTATAACCGAGGATGAGACCAGGACTAGAACGCCTGAGCCGATCATCATCGAAGAGGA
AGAAGAGGATAGCATAAGTTTGCTGTCAGATGGCCCGACCCACCAGGTGCTGCAAGTCGAGGCAGACATTCACG
GGCCGCCCTCTGTATCTAGCTCATCCTGGTCCATTCCTCATGCATCCGACTTTGATGTGGACAGTTTATCCATA
CTTGACACCCTGGAGGGAGCTAGCGTGACCAGCGGGGCAACGTCAGCCGAGACTAACTCTTACTTCGCAAAGAG
TATGGAGTTTCTGGCGCGACCGGTGCCTGCGCCTCGAACAGTATTCAGGAACCCTCCACATCCCGCTCCGCGCA
CAAGAACACCGTCACTTGCACCCAGCAGGGCCTGCTCGAGAACCAGCCTAGTTTCCACCCCGCCAGGCGTGAAT
AGGGTGATCACTAGAGAGGAGCCTGAGGCGCTTACCCCGTCACGTCTCCTTAGCAGGTCGGTCTCGAGAACCAG
CCTGGTCTCCAACCCGCCAGGCGTAAATAGGGTGATTACAAGAGAGGAGTTTGAGGCGTTCGTAGCACAACAAC
AATGACGGTTTGATGCGGGTGCATACATCTTTTCCTCCGACACCGGTCAAGGGCATTTACAACAAAAATCAGTA
AGGCAAACGGTGCTATCCGAAGTGGTGTTGGAGAGGACCGAATTGGAGATTTCGTATGCCCCGCGCCTCGACCA
AGAAAAAGAAGAATTACTACGCAAGAAATTACAGTTAAATCCCACACCTGCTAACAGAAGCAGATACCAGTCCA
GGAAGGTGGAGAACATGAAAGCCATAACAGCTAGACGTATTCTGCAAGGCCTAGGGCATTATTTGAAGGCAGAA
GGAAAAGTGGAGTGCTACCGAACCCTGCATCCTGTTCCTTTGTATTCATCTAGTGTGAACCGTGCCTTTTCAAG
CCCCAAGGTCGCAGTGGAAGCCTGTAACGCCATGTTGAAAGAGAACTTTCCGACTGTGGCTTCTTACTGTATTA
TTCCAGAGTACGATGCCTATTTGGACATGGTTGACGGAGCTTCATGCTGCTTAGACACTGCCAGTTTTTGCCCT
GCAAAGCTGCGCAGCTTTCCAAAGAAACACTCCTATTTGGAACCCACAATACGATCGGCAGTGCCTTCAGCGAT
CCAGAACACGCTCCAGAACGTCCTGGCAGCTGCCACAAAAAGAAATTGCAATGTCACGCAAATGAGAGAATTGC
CCGTATTGGATTCGGCGGCCTTTAATGTGGAATGCTTCAAGAAATATGCGTGTAATAATGAATATTGGGAAACG
TTTAAAGAAAACCCCATCAGGCTTACTGAAGAAAACGTGGTAAATTACATTACCAAATTAAAAGGACCAAAAGC
TGCTGCTCTTTTTGCGAAGACACATAATTTGAATATGTTGCAGGACATACCAATGGACAGGTTTGTAATGGACT
TAAAGAGAGACGTGAAAGTGACTCCAGGAACAAAACATACTGAAGAACGGCCCCAAGGTACAGGTGATCCAGGCT
GCCGATCCGCTAGCAACAGCGTATCTGTGCGGAATCCACCGAGAGCTGGTTAGGAGATTAAATGCGGTCCTGCT
TCCGAACATTCATACACTGTTTGATATGTCGGCTGAAGACTTTGACGCTATTATAGCCGAGCACTTCCAGCCTG
GGGATTGTGTTCTGGAAACTGACATCGCGTCGTTTGATAAAAGTGAGGACGACGCCATGGCTCTGACCGCGTTA
ATGATTCTGGAAGACTTAGGTGTGGACGCAGAGCTGTTGACGCTGATTGAGGCGGCTTTCGGCGAAATTTCATC
AATACATTTGCCCACTAAAACTAAATTTAAATTCGGAGCCATGATGAAATCTGGAATGTTCCTCACACTGTTTG
TGAACACAGTCATTAACATTGTAATCGCAAGCAGAGTGTTGAGAGAACGGCTAACCGGATCACCATGTGCAGCA
TTCATTGGAGATGACAATATCGTGAAAGGAGTCAAATCGGACAAATTAATGGCAGACAGGTGCGCCACCTGGTT
GAATATGGAAGTCAAGATTATAGATGCTGTGGTGGGCGAGAAAGCGCCTTATTTCTGTGGAGGGTTTATTTTGT
GTGACTCCGTGACCGGCACAGCGTGCCGTGTGGCAGACCCCCTAAAAAGGCTGTTTAAGCTTGGCAAACCTCTG
GCAGCAGACGATGAACATGATGATGACAGGAGAAGGGCATTGCATGAAGAGTCAACACGCTGGAACCGAGTGGG
TATTCTTTCAGAGCTGTGCAAGGCAGTAGAATCAAGGTATGAAACCGTAGGAACTTCCATCATAGTTATGGCCA
TGACTACTCTAGCTAGCAGTGTTAAATCATTCAGCTACCTGAGAGGGGCCCCTATAACTCTCTACGGCTAACCT
GAATGGACTACGACTATCACGCCCAAACATTTACAGCCGCGGTGTCAAAAACCGCGTGGACGTGGTTAACATCC
CTGCTGGAGGATCAGCCGTAATTATTATAATTGGCTTGGTGCTGGCTACTATTGTGGCCATGTACGTGCTGAC
CAACCAGAACAATAATTGAATACAGCAGCAATTGGCAAGCTGCTTACATAGAACTCGCGGCGATTGGCATGCCG
CCTTAAAATTTTTTATTTTATTTTTCTTTTCTTTTCCGAATCGGATTTTGTTTTTAATATTTCAAAAAAAAAA
AAAAAAAAAAAAAAAAAAAAAAAAAAAAAAAAAAAAAAAAAAAAAAAAAAAAAAAAAAAAAAAAAAAAtacgta
gtttaaac
```

VEE-UbAAY (SEQ ID NO:14); VEE delivery vector with MHC class I mouse tumor epitopes SIINFEKL (SEQ ID NO: 57) and AH1-A5 inserted

(continued)

```
      ATGggcggcgcatgagagaagcccagaccaattacctacccaaaatggagaaagttcacgttgacatc
gaggaagacagcccattcctcagagctttgcagcggagcttcccgcagtttgaggtagaagccaagcaggtcac
tgataatgaccatgctaatgccagagcgttttcgcatctggcttcaaaactgatcgaaacggaggtggacccat
ccgacacgatccttgacattggaagtgcgcccgcccgcagaatgtattctaagcacaagtatcattgtatctgt
ccgatgagatgtgcggaagatccggacagattgtataagtatgcaactaagctgaagaaaaactgtaaggaaat
aactgataaggaattggacaagaaaatgaaggagctcgccgccgtcatgagcgaccctgacctggaaactgaga
ctatgtgcctccacgacgacgagtcgtgtcgctacgaagggcaagtcgctgtttaccaggatgtatacgcggtt
gacggaccgacaagtctctatcaccaagccaataagggagttagagtcgcctactggataggctttgacaccac
cccttttatgtttaagaacttggctggagcatatccatcatactctaccaactgggccgacgaaaccgtgttaa
cggctcgtaacataggcctatgcagctctgacgttatggagcggtcacgtagagggatgtccattcttagaaag
aagtatttgaaaccatccaacaatgttctattctctgttggctcgaccatctaccacgagaagagggacttact
gaggagctggcacctgccgtctgtatttcacttacgtggcaagcaaaattacacatgtcggtgtgagactatag
ttagttgcgacgggtacgtcgttaaaagaatagctatcagtccaggcctgtatggaagccttcaggctatgct
gctacgatgcaccgcgagggattcttgtgctgcaaagtgacagacacattgaacggggagagggtctcttttcc
cgtgtgcacgtatgtgccagctacattgtgtgaccaaatgactggcatactggcaacagatgtcagtgcggacg
acgcgcaaaaactgctggttgggctcaaccagcgtatagtcgtcaacggtcgcacccagagaaacaccaatacc
atgaaaaattacctttttgcccgtagtggcccaggcatttgctaggtgggcaaaggaatataaggaagatcaaga
agatgaaaggccactaggactacgagatagacagttagtcatggggtgttgttgggcttttagaaggcacaaga
taacatctatttataagcgcccggatacccaaaccatcatcaaagtgaacagcgatttccactcattcgtgctg
cccaggataggcagtaacacattggagatcgggctgagaacaagaatcaggaaaatgttagaggagcacaagga
gccgtcacctctcattaccgccgaggacgtacaagaagctaagtgcgcagccgatgaggctaaggaggtgcgtg
aagccgaggagttgcgcgcagctctaccaccttttggcagctgatgttgaggagcccactctggaagccgatgtc
gacttgatgttacaagaggctggggccggctcagtggagacacctcgtggcttgataaaggttaccagctacgc
tggcgaggacaagatcggctcttacgctgtgctttctccgcaggctgtactcaagagtgaaaaattatcttgca
tccaccctctcgctgaacaagtcatagtgataacacactctggccgaaaagggcgttatgccgtggaaccatac
catggtaaagtagtggtgccagagggacatgcaataccgtccaggacttcaagctctgagtgaaagtgccac
cattgtgtacaacgaacgtgagttcgtaaacaggtacctgcaccatattgccacacatggaggagcgctgaaca
ctgatgaagaatattacaaaactgtcaagcccagcgagcacgacggcgaatacctgtacgacatcgacaggaaa
cagtgcgtcaagaaagaactagtcactgggctagggctcacaggcgagctggtggatcctcccttccatgaatt
cgcctacgagagtctgagaacacgaccagccgctccttaccaagtaccaaccataggggtgtatggcgtgccag
gatcaggcaagtctggcatcattaaaagcgcagtcaccaaaaaagatctagtggtgagcgccaagaagaaaac
tgtgcagaaattataagggacgtcaagaaaatgaaagggctggacgtcaatgccagaactgtggactcagtgct
cttgaatggatgcaaacaccccgtagagaccctgtatattgacgaagcttttgcttgtcatgcaggtactctca
gagcgctcatagccattataagacctaaaaaggcagtgctctgcggggatcccaaacagtgcggtttttttaac
atgatgtgcctgaaagtgcattttaaccacgagatttgcacacaagtcttccacaaaagcatctctcgccgttg
cactaaatctgtgacttcggtcgtctcaaccttgttttacgacaaaaaaatgagaacgacgaatccgaaagaga
ctaagattgtgattgacactaccggcagtaccaaacctaagcaggacgatctcattctcacttgtttcagaggg
tgggtgaagcagttgcaaatagattacaaaggcaacgaaataatgacggcagctgcctctcaagggctgacccg
taaaggtgtgtatgccgttcggtacaaggtgaatgaaaatcctctgtacgcacccacctcagaacatgtgaacg
tcctactgacccgcacggaggaccgcatcgtgtggaaaacactagccggcgacccatggataaaaacactgact
gccaagtaccctgggaatttcactgccacgataagaggagtggcaagcagagcatgatgccatcatgaggcacat
cttggagagaccggaccctaccgacgtcttccagaataaggcaaacgtgtgttgggccaaggctttagtgccgg
tgctgaagaccgctggcatagacatgaccactgaacaatggaacactgtggattattttgaaacggacaaagct
cactcagcagagatagtattgaaccaactatgcgtgaggttctttggactcgatctggactccggtctattttc
tgcacccactgttccgttatccattaggaataatcactgggataactccccgtcgcctaacatgtacgggctga
ataaagaagtggtccgtcagctctctcgcaggtacccacaactgcctcgggcagttgccactggaagagtctat
gacatgaacactggtacactgcgcaattatgatccgcgcataaacctagtacctgtaaacagaagactgcctca
tgctttagtcctccaccataatgaacaccacagagtgacttttcttcattcgtcagcaaattgaagggcagaa
ctgtcctggtggtcgggaaaagttgtccgtcccaggcaaaatggttgactggttgtcagaccggcctgaggct
accttcagagctcggctggatttaggcatcccaggtgatgtgcccaaatatgacataatatttgttaatgtgag
gaccccatataaataccatcactatcagcagtgtgaagaccatgccattaagcttagcatgttgaccaagaaag
cttgtctgcatctgaatcccggcggaacctgtgtcagcataggttatggttacgctgacagggccagcgaaagc
atcattggtgctatagcgcggcagttcaagtttttcccgggtatgcaaaccgaaatcctcacttgaagagacgga
agttctgtttgtattcattgggtacgatcgcaaggcccgtacgcacaatccttacaagctttcatcaaccttga
ccaacatttatacaggttccagactccacgaagccggatgtgcaccctcatatcatgtggtgcgaggggatatt
gccacggccaccgaaggagtgattataaatgctgctaacagcaaaggacaacctggcggagggggtgtgcggagc
gctgtataagaaattcccggaaagcttcgatttacagccgatcgaagtaggaaaagcgcgactggtcaaaggtg
```

(continued)

```
cagctaaacatatcattcatgccgtaggaccaaacttcaacaaagtttcggaggttgaaggtgacaaacagttg
gcagaggcttatgagtccatcgctaagattgtcaacgataacaattacaagtcagtagcgattccactgttgtc
caccggcatctttccgggaacaaagatcgactaacccaatcattgaaccatttgctgacagctttagacacca
ctgatgcagatgtagccatatactgcagggacaagaaatgggaaatgactctcaaggaagcagtggctaggaga
gaagcagtggaggagatatgcagcactccgacgactcttcagtgacagaacctgatgcagagctggtgagggtgca
tccgaagagttctttggctggaaggaaggagctacagcacaagcgatggcaaaactttctcatatttggaaggga
ccaagtttcaccaggcggccaaggatatagcagaaattaatgccatgtggcccgttgcaacggaggccaatgag
caggtatgcatgtatatcctcggagaaagcatgagcagtattaggtcgaaatgccccgtcgaagagtcggaagc
ctccacaccacctagcacgctgccttgcttgtgcatccatgccatgactccagaaagagtacagcgcctaaaag
cctcacgtccagaacaaattactgtgtgctcatcctttccattgccgaagtatagaatcactggtgtgcagaag
atccaatgctcccagcctatattgttctcaccgaaagtgcctgcgtatattcatccaaggaagtatctcgtgga
aacaccaccggtagacgagactccggagccatcggcagagaaccaatccacagaggggacacctgaacaaccac
cacttataaccgaggatgagaccaggactagaacgcctgagccgatcatcatcgaagaggaagaagaggatagc
ataagtttgctgtcagatggcccgacccaccaggtgctgcaagtcgaggcagacattcacgggccgccctctgt
atctagctcatcctggtccattcctcatgcatccgactttgatgtggacagtttatccatacttgacaccctgg
agggagctagcgtgaccagcggggcaacgtcagccgagactaactcttacttcgcaaagagtatggagtttctg
gcgcgaccggtgcctgcgcctcgaacagtattcaggaaccctccacatcccgctccgcgcacaagaacaccgtc
acttgcacccagcagggcctgctcgagaaccagcctagtttccaccccgccaggcgtgaataggggtgatcacta
gagaggagctcgaggcgcttaccccgtcacgcactcctagcaggtcggtctcgagaaccagcctggtctccaac
ccgccaggcgtaaataggggttgattacaagagaggagtttgaggcgttcgtagcacaacaacaatgacggtttga
tgcgggtgcatacatctttcctccgacaccggtcaagggcatttacaacaaaaatcagtaaggcaaacggtgc
tatccgaagtggtgttggagaggaccgaattggagatttcgtatgccccgcgcctcgaccaagaaaaagaagaa
ttactacgcaagaaattacagttaaatcccacacctgctaacagaagcagataccagtccaggaaggtggagaa
catgaaagccataacagctagacgtattctgcaaggcctagggcattatttgaaggcagaaggaaaagtggagt
gctaccgaaccctgcatcctgttcctttgtattcatctagtgtgaaccgtgccttttcaagcccccaaggtcgca
gtggaagcctgtaacgccatgttgaaagagaacttttccgactgtggcttcttactgtattattccagagtacga
tgcctatttggacatggttgacggagcttcatgctgcttagcactgccagttttttgccctgcaaagctgcgca
gctttccaaagaaacactcctatttggaacccacaatacgatcggcagtgccttcagcgatccagaacacgctc
cagaacgtcctggcagctgccacaaaagaaattgcaatgtcacgcaaatgagagaattgcccgtattggattc
ggcggcctttaatgtggaatgcttcaagaaatatgcgtgtaataatgaatattgggaaacgtttaaagaaaacc
ccatcaggcttactgaagaaaacgtggtaaattacattaccaaattaaaaggaccaaaagctgctgctctttttt
gcgaagacacataatttgaatatgttgcaggacataccaatggacaggtttgtaatggacttaaagagagacgt
gaaagtgactccaggaacaaaacatactgaagaacggcccaaggtacaggtgatccaggctgccgatccgctag
caacagcgtatctgtgcggaatccaccgagagctggttggtgagattaaatgcggtcctgcttccgaacattcat
acactgtttgatatgtcggctgaagactttgacgctattatagccgagcacttccagcctgggggattgtgttct
ggaaactgacatcgcgtcgtttgataaaagtgaggacgacgccatggctctgaccgcgttaatgattctggaag
acttaggtgtggacgcagagctgttgacgctgattgaggcggctttcggcgaaatttcatcaatacatttgccc
actaaaactaaatttaaattcggagccatgatgaaatctggaatgttcctcacactgtttgtgaacacagtcat
taacattgtaatcgcaagcagagtgttgagagaacggctaaccggatcaccatgtgcagcattcattggagatg
acaatatcgtgaaaggagtcaaatcggacaaattaatggcagacaggtgcgccacctggttgaatatggaagtc
aagattatagatgctgtggtgggcgagaaagcgccttatttctgtggagggtttattttgtgtgactccgtgac
cggcacagcgtgccgtgtggcagacccccctaaaaaggctgtttaagctggcaaacctctggcagcagacgatg
aacatgatgatgacaggagaagggcattgcatgaagagtcaacacgctggaaccgagtgggtattctttcagag
ctgtgcaaggcagtagaatcaaggtatgaaaccgtaggaacttccatcatagttatggccatgactactctagc
tagcagtgttaaatcattcagctacctgagagggggcccctataactctctacggctaacctgaatggactacga
ctctagaatagtctttaattaaagtccgccatatgaggccaccatgCAGATCTTCGTGAAGACCCTGACCGGCA
AGACCATCACCCTAGAGGTGGAGCCCAGTGACACCATCGAGAACGTGAAGGCCAAGATCCAGGATAAAGAGGGC
ATCCCCCCTGACCAGCAGAGGCTGATCTTTGCCGGCAAGCAGCTGGAAGATGGCCGCACCCTCTCTGATTACAA
CATCCAGAAGGAGTCAACCCTGCACCTGGTCCTTCGCCTGAGAGGTGGCGCTGCTTACAGTATAATCAACTTTG
AAAAACTGGCTGCTTACGGCATCCTGGGCTTTGTGTTTACACTGGCTGCCTACCTGCTGTTTGGCTATCCTGTG
TACGTGGCCGCTTATGGACTGTGTACCCTGGTGGCCATGCTGGCTGCTTACAATCTGGTGCCTATGGTGGCCAC
AGTGGCCGCCTATTGTCTTGGCGGACTGCTGACAATGGTGGCAGCCTACAgcccgagctatgcgtatcatcagt
ttGCAGCCTACGGCCCAGGACCAGGCgCTAAATTTGTGGCTGCCTGGACACTGAAAGCCGCCGCTGGACCAGGT
CCTGGACAGTACATCAAGGCCAACAGCAAGTTCATCGGCATCACCGAACTCGGCCCAGGACCAGGCTATCCCTA
CGATGTGCCTGATTACGCCTGATagTGATGATTCGAACGGCCGtatcacgcccaaacatttacagccgcggtgt
caaaaaccgcgtggacgtggttaacatccctgtcggggaggatcagccgtaattattataattggcttggtgctg
gctactattgtggccatgtacgtgctgaccaaccagaaacataattgaatacagcagcaattggcaagctgctt
acatagaactcgcggcgattggcatgccgccttaaaattttattttattttttcttttcttttccgaatcggg
ttttgttttaatatttcAAAAAAAAAAAAAAAAAAAAAAAAAAAAAAAAAAAAAAAAAAAAAAAAAAAAAAAAAA
AAAAAAAAAAAAAAAAAAAAAAAA
```

VEE-Luciferase (SEQ ID NO:15); VEE delivery vector with luciferase gene inserted at 7545

EP 3 544 607 B1

(continued)

```
ATGggcggcgcatgagagaagcccagaccaattacctacccaaaATGGagaaagttcacgttgacatcgaggaa
gacagcccattcctcagagctttgcagcggagcttcccgcagtttgaggtagaagccaagcaggtcactgataa
tgaccatgctaatgccagagcgttttcgcatctggcttcaaaactgatcgaaacggaggtggacccatccgaca
cgatccttgacattggaagtgcgcccgcccgcagaatgtattctaagcacaagtatcattgtatctgtccgatg
agatgtgcggaagatccggacagattgtataagtatgcaactaagctgaagaaaaactgtaaggaaataactga
taaggaattggacaagaaatgaaggagctcgccgccgtcatgagcgaccctgacctggaaactgagactatgt
gcctccacgacgacgagtcgtgtcgctacgaagggcaagtcgctgtttaccaggatgtatacgcggttgacgga
ccgacaagtctctatcaccaagccaataagggagttagagtcgcctactggataggctttgacaccaccccttt
tatgtttaagaacttggctggagcatatccatcatactctaccaactgggccgacgaaaccgtgttaacggctc
gtaacataggcctatgcagctctgacgttatggagcggtcacgtagagggatgtccattcttagaaagaagtat
ttgaaaccatccaacaatgttctattctctgttggctcgaccatctaccacgagaagagggacttactgaggag
ctggcacctgccgtctgtatttcacttacgtggcaagcaaaattacacatgtcggtgtgagactatagttagtt
gcgacgggtacgtcgttaaaagaatagctatcagtccaggcctgtatgggaagccttcaggctatgctgctacg
atgcaccgcgagggattcttgtgctgcaaagtgacagacacattgaacggggagagggtctcttttccgtgtg
cacgtatgtgccagctacattgtgtgaccaaatgactggcatactggcaacagatgtcagtgcggacgacgcgc
aaaaactgctggttgggctcaaccagcgtatagtcgtcaacggtcgcacccagagaaacaccaataccatgaaa
aattacctttgcccgtagtggcccaggcatttgctaggtgggcaaaggaatataaggaagatcaagaagatga
aaggccactaggactacgagatagacagttagtcatggggtgttgttgggcttttagaaggcacaagataacat
ctatttataagcgcccggatacccaaaccatcatcaaagtgaacagcgatttccactcattcgtgctgcccagg
ataggcagtaacacattggagatcgggctgagaacaagaatcaggaaaatgttagaggagcacaaggagccgtc
acctctcattaccgccgaggacgtacaagaagctaagtgcgcagccgatgaggctaaggaggtgcgtgaagccg
aggagttgcgcgcagctctaccacctttggcagctgatgttgaggagcccactctggaagccgatgtcgacttg
atgttacaagaggctggggccggctcagtggagacacctcgtggcttgataaaggttaccagctacgctggcga
ggacaagatcggctcttacgctgtgctttctccgcaggctgtactcaagagtgaaaaattatcttgcatccacc
ctctcgctgaacaagtcatagtgataacacactctggccgaaaagggcgttatgccgtggaaccataccatggt
aaagtagtggtgccagagggacatgcaatacccgtccaggactttcaagctctgagtgaaagtgccaccattgt
gtacaacgaacgtgagttcgtaaacaggtacctgccaccatattgccacacatgtgaggagcgctgaacactgatg
aagaatattacaaaactgtcaagcccagcgagcacgacggcgaatacctgtacgacatcgacaggaaacagtgc
gtcaagaaagaactagtcactgggctagggctcacaggcgagctggtggatcctcccttccatgaattcgccta
cgagagtctgagaacacgaccagccgctcccttaccaagtaccaaccatagggtgtatggcgtgccaggatcag
gcaagtctggcatcattaaaagcgcagtcaccaaaaaagatctagtggtgagcgccaagaaagaaactgtgca
gaaattataagggacgtcaagaaaatgaaagggctggacgtcaatgccagaactgtggactcagtgctcttgaa
tggatgcaaacacccgtagagaccctgtatattgacgaagcttttgcttgtcatgcaggtactctcagagcgc
tcatagccattataagacctaaaaaggcagtgctctgcggggatcccaaacagtcggtttttttaacatgatg
tgcctgaaagtgcattttaaccacgagatttgcacacaagtcttccacaaaagcatctctcgccgttgcactaa
atctgtgacttcggtcgtctcaaccttgttttacgacaaaaaaatgagaacgacgaatccgaaagagactaaga
ttgtgattgacactaccggcagtaccaaacctaagcaggacgatctcattctcacttgtttcagagggtgggtg
aagcagttgcaaatagattacaaaggcaacgaaataatgacggcagctgcctctcaagggctgacccgtaaagg
tgtgtatgccgttcggtacaaggtgaatgaaaatcctctgtacgcacccacctcagaacatgtgaacgtcctac
tgacccgcacggaggaccgcatcgtgtggaaaacactagccggcgacccatggataaaaacactgactgccaag
taccctgggaatttcactgccacgatagaggagtggcaagcagagcatgatgccatcatgaggcacatctttgga
gagaccggaccctaccgacgtcttccagaataaggcaaacgtgtgttgggcccaaggctttagtgccggtgctga
agaccgctggcatagacatgaccactgaacaatgaacactgtggattattttgaaacggacaaagctcactca
gcagagatagtattgaaccaactatgcgtgaggttctttggactcgatctggactccggtctattttctgcacc
cactgttccgttatccattaggaataatcactgggataactccccgtcgcctaacatgtacgggctgaataaag
aagtggtccgtcagctctctcgcaggtacccacaactgcctcgggcagttgccactggaagagtctatgacatg
aacactggtacactgcgcaattatgatccgcgcataaaacctagtacctgtaaacagaagactgcctcatgctttt
agtcctccaccataatgaacacccacagagtgacttttcttcattcgtcagcaaattgaaggagcagaactgtcc
tggtggtcggggaaaagttgtccgtcccaggcaaaatggttgactggttgtcagaccggcctgaggctaccttc
agagctcggctggatttaggcatcccaggtgatgtgcccaaatatgacataaatatttgttaatgtgaggacccc
atataaataccatcactatcagcagtgtgaagaccatgccattaagcttagcatgttgaccaagaaagcttgtc
tgcatctgaatcccggcggaacctgtgtcagcataggttatggttacgctgacagggccagcgaaagcatcatt
ggtgctatagcgcggcagttcaagttttcccgggtatgcaaaccgaaatcctcacttgaagagacggaagttct
gtttgtattcattgggtacgatcgcaaggcccgtacgcacaatccttacaagcttttcatcaacccttgaccaaca
tttatacaggttccagactccacgaagccggatgtgcaccctcatatcatgtggtgcgaggggatattgccacg
gccaccgaaggagtgattataaatgctgctaacagcaaaggacaacctggcgaggggttgtgcggagcgctgta
taagaaattcccggaaagcttcgatttacagccgatcgaagtaggaaaagcgcgactggtcaaaggtgcagcta
aacatatcattcatgccgtaggaccaaacttcaacaaagtttcggaggttgaaggtgacaaacagttggcagag
gcttatgagtccatcgctaagattgtcaacgataacaattacaagtcagtagcgattccactgttgtccaccgg
catcttttccgggaacaaagatcgactaacccaatcattgaaccatttgctgacagctttagacaccactgatg
```

154

```
cagatgtagccatatactgcagggacaagaaatgggaaatgactctcaaggaagcagtggctaggagagaagca
gtggaggagatatgcatatccgacgactcttcagtgacagaacctgatgcagagctggtgagggtgcatccgaa
gagttctttggctggaaggaagggctacagcacaagcgatggcaaaactttctcatatttggaagggaccaagt
ttcaccaggcggccaaggatatagcagaaattaatgccatgtggcccgttgcaacgtgaggccaatgagcaggta
tgcatgtatatcctcggagaaagcatgagcagtattaggtcgaaatgccccgtcgaagagtcggaagcctccac
accacctagcacgctgccttgcttgtgcatccatgccatgactccagaaagagtacagcgcctaaaagcctcac
gtccagaacaaattactgtgtgctcatcctttccattgccgaagtatagaatcactggtgtgcagaagatccaa
tgctcccagcctatattgttctcaccgaaagtgcctgcgtatattcatccaaggaagtatctcgtggaaacacc
accggtagacgagactccggagccatcggcagagaaccaatccacagaggggacacctgaacaaccaccactta
taaccgaggatgagaccaggactagaacgcctgagccgatcatcatcgaagaggaagaagaggatagcataagt
ttgctgtcagatggcccgacccaccaggtgctgcaagtcgaggcagacattcacgggccgccctctgtatctag
ctcatcctggtccattcctcatgcatccgactttgatgtggacagtttatccatacttgacaccctggagggag
ctagctgaccagcggggcaacgtcagccgagactaactcttacttcgcaaagagtatggagtttctggcgcga
ccggtgcctgcgcctcgaacagtattcaggaaccctccacatcccgctccgcgcacaagaacaccgtcacttgc
acccagcagggcctgctcgagaaccagcctagtttccacccccgccaggcgtgaatagggtgatcactagagagg
agctcgaggcgcttacccccgtcacgcactcctagcaggtcggtctcgagaaccagcctggtctccaacccgcca
ggcgtaaataggtgattacaagagaggagtttgaggcgttcgtagcacaacaacaatgacggtttgatgcgggg
tgcatacatcttttcctccgacaccggtcaagggcatttacaacaaaaatcagtaaggcaaacggtgctatccg
aagtggtgttggagaggaccgaattggagatttcgtatgccccgcgcctcgaccaagaaaaagaagaattacta
cgcaagaaattacagttaaatcccacacctgctaacagaagcagataccagtccaggaaggtgggagaacatgaa
agccataacagctagacgtattctgcaaggcctcagggcattatttgaaggcagaaggaaaagtggagtgctacc
gaaccctgcatcctgttcctttgtattcatctagtgtgaaccgtgccttttcaagccccaaggtcgcagtggaa
gcctgtaacgccatgttgaaagagaactttccgactgtggcttcttactgtattattccagagtacgatgccta
tttggacatggttgacggagcttcatgctgcttagacactgccagttttttgccctgcaaagctgcgcagctttc
caaagaaacactcctatttggaacccacaatacgatcggcagtgccttcagcgatccagaacacgctccagaac
gtcctggcagctgccacaaaaagaaattgcaatgtcacgcaaatgagagaattgcccgtattggattcggcggc
ctttaatgtggaatgcttcaagaaatatgcgtgtaataatgaatattgggaaacgtttaaagaaaaccccatca
ggcttactgaagaaaacgtggtaaattacattaccaaattaaaaggaccaaaagctgctgctcttttttgcgaag
acacataaatttgaatatgttgcaggacataccaatggacaggtttgtaatggacttaaagagagacgtgaaagt
gactccaggaacaaaacatactgaagaacggcccaaggtacaggtgatccaggctgccgatccgctagcaacag
cgtatctgtgcggaatccaccgagagctggttaggagattaaatgcggtcctgcttccgaacattcatacactg
tttgatatgtcggctgaagactttgacgctattatagccgagcacttccagcctgggggattgtgttctggaaac
tgacatcgcgtcgtttgataaaagtgaggacgacgccatggctctgaccgcgttaatgattctggaagacttag
gtgtggacgcagagctgttgacgctgattgaggcggcttcgtgcgaaatttcatcaatacatttgcccactaaa
actaaatttaaattcggagccatgatgaaatctggaatgttcctcacactgtttgtgaacacagtcattaacat
tgtaatcgcaagcagagtgttgagagaacggctaaccggatcaccatgtgcagcattcattggagatgacaata
tcgtgaaaggagtcaaatcggacaaattaatggcagacaggtgcgccacctggttgaatatggaagtcaagatt
atagatgctgtggtgggcgagaaagcgcctatttctgtggaggtttatttttgtgtgactccgtgaccggcac
agcgtgccgtgtggcagacccctaaaaaggctgtttaagcttggcaaacctctggcagcagacgatgaacatg
atgatgacaggagaagggcattgcatgaagagtcaacacgctggaaccgagtgggtattctttcagagctgtgc
aaggcagtagaatcaaggtatgaaaccgtaggaacttccatcatagttatggccatgactactctagctagcag
tgttaaatcattcagctacctgagagggggcccctataactctctacggcTAAcctgaatggactacgactctag
aatagtctttaattaaagtccgccatatgagatggaagatgccaaaaacattaagaagggcccagcgccattct
acccactcgaagacgggaccgccggcgagcagctgcacaaagccatgaagcgctacgccctggtgcccggcacc
atcgcctttaccgacgcacatatcgaggtggacattacctacgccgagtacttcgagatgagcgttcggctggc
agaagctatgaagcgctatgggctgaatacaaaccatcggatcgtggtgtgcagcgagaatagcttgcagttct
tcatgcccgtgttgggtgccctgttcatcggtgtggctgtggccccagctaacgacatctacaacgagcgcgag
ctgctgaacagcatgggcatcagccagtccaccgtcgtattcgtgacggcaagaaaggcgtgcaaaagatcctcaa
cgtgcaaaagaagctaccgatcatacaaaagatcatcatcatggatagcaagaccgactaccagggcttccaaa
gcatgtacaccttcgtgacttcccatttgccacccggcttcaacgagtacgacttcgtgcccgagagcttcgac
cgggacaaaaccatcgccctgatcatgaacagtagtggcagtaccggattgcccaagggcgtagccctaccgca
ccgcaccgcttgtgtccgattcagtcatgcccgcgaccccatcttcggcaaccagatcatccccgacaccgcta
tcctcagcgtggtgccatttcaccacggcttcggcatgttcaccacgctgggctacttgatctgcggctttcgg
gtcgtgctcatgtaccgcttcgaggaggagctattcttgcgcagcttgcaagactataagattcaatctgccct
gctggtgcccacactatttagcttcttcgctaagagcactctcatcgacaagtacgacctaagcaacttgcacg
agatcgccagcggcggggcgccgctcagcaaggagagtaggtgaggccgtggccaaacgcttccacctaccaggc
atccgccagggctacggcctgacagaaacaaccagcgccattctgatcaccccccgaaggggacgacaagcctgg
cgcagtaggcaaggtggtgcccttcttcgaggctaaggtggtggacttggacaccggtaagacactgggtgtga
accagcgcggcgagctgtgcgtccgtggccccatgatcatgagcggctacgttaacaaccccgaggctacaaac
gctctcatcgacaaggacggctggctgcacagcggcgacatcgcctactgggacgaggacgagcacttcttcat
cgtggaccggctgaagagcctgatcaaatacaagggctaccaggtagccccagccgaactggagagcatcctgc
tgcaacaccccaacatcttcgacgccggggtcgccggctgccccgacgacgatgccggcgagctgccccgccgca
gtcgtcgtgctggaacacggtaaaaccatgaccgagaaggagatcggtgactatgtggccagccaggttacaac
cgccaagaagctgcgcggtggtgttgtgttcgtggacgaggtgcctaaaggactgaccggcaagtggacgcccc
gcaagatccgcgagattctcattaaggccaagaagggcggcaagatcgccgtgtaaTTCGAACGGCCGtatcac
gcccaaacatttacagccgcggtgtcaaaaaccgcgtggacgtggttaacatccctgctgggaggatcagccgt
aattattataattggcttggtgctggctactattgtggccatgtacgtgctgaccaaccagaaacataattgaa
tacagcagcaattggcaagctgcttacatagaactcgcggcgattggcatgccgccttaaaattttttattttat
ttttctttctttctttccgaatcggattttgttttttaatatttcAAAAAAAAAAAAAAAAAAAAAAAAAAAAAAAA
AAAAAAAAAAAAAAAAAAAAAAAAAAAAAAAAAAAAAAAAAAAAAAAAA
```

ubiquitin (SEQ ID NO:38)

>UbG76 0-228

```
      ATGCAGATCTTCGTGAAGACCCTGACCGGCAAGACCATCACCCTAGAGGTGGAGCCCAGTGACACCATCG
AGAACGTGAAGGCCAAGATCCAGGATAAAGAGGGCATCCCCCCTGACCAGCAGAGGCTGATCTTTGCCGGCAAGCA
GCTGGAAGATGGCCGCACCCTCTCTGATTACAACATCCAGAAGGAGTCAACCCTGCACCTGGTCCTTCGCCTGAGA
GGTGGC
```

Ubiquitin A76 (SEQ ID NO:39)
>UbA76 0-228

```
      ATGCAGATCTTCGTGAAGACCCTGACCGGCAAGACCATCACCCTAGAGGTGGAGCCCAGTGACACCATCG
AGAACGTGAAGGCCAAGATCCAGGATAAAGAGGGCATCCCCCCTGACCAGCAGAGGCTGATCTTTGCCGGCAAGCA
GCTGGAAGATGGCCGCACCCTCTCTGATTACAACATCCAGAAGGAGTCAACCCTGCACCTGGTCCTTCGCCTGAGA
GGTGCC
```

HLA-A2 (MHC class I) signal peptide (SEQ ID NO:40)
>MHC SignalPep 0-78

```
      atggccgtcatggcgccccgaaccctcgtcctgctactctcgggggctctggccctgacccagacctggg
cgggctct
```

HLA-A2 (MHC class I) Trans Membrane domain (SEQ ID NO:41)
>HLA A2 TM Domain 0-201

```
      CCGtcttcccagcccaccatccCCATCGTGGGCAtcattgctggcctggttctctcttggagctgtgatca
ctggagctgtggtcgctgctgtgatgtggaggaggaagagctcagatagaaaaggagggagctactctcaggctgc
aagcagtgacagtgcccagggctctgatgtgtctctcacagcttgtaaagtgtga
```

IgK Leader Seq (SEQ ID NO:42)
>IgK Leader Seq 0-60
atggagaccgatacactgctgctgtgggtgctgctcctgtgggtgccaggaagcacaggc

Human DC-Lamp (SEQ ID NO:43)
>HumanDCLAMP 0-3178

```
      ggcaccgattcggggcctgcccggacttcgccgcacgctgcagaacctcgcccagcgcccaccatgcccc
ggcagctcagcgcggcggccgcgctcttcgcgtccctggccgtaattttgcacgatggcagtcaaatgagagcaaa
agcatttccagaaaccagagattattctcaacctactgcagcagcaacagtacaggacataaaaaaacctgtccag
caaccagctaagcaagcacctcaccaaactttagcagcaagattcatggatggtcatatcacctttcaaacagcgg
ccacagtaaaaaattccaacaactaccccagcaactacaaaaaacactgcaaccaccagcccaattacctacaccct
ggtcacaacccaggccacacccaacaactcacacacagctcctccagttactgaagttacagtcggccctagctta
gccccttattcactgccacccaccatcacccccaccagctcatacagctggaaccagttcatcaaccgtcagccaca
caactgggaacaccactcaacccagtaaccagaccacccttccagcaactttatcgatagcactgcacaaaagcac
aaccggtcagaagcctgatcaacccacccatgccccaggaacaacggcagctgcccacaataccacccgcacagct
gcacctgcctccacggttcctgggcccacccttgcacctcagccatcgtcagtcaagactggaatttatcaggttc
taaacggaagcagactctgtataaaagcagagatggggatacagctgattgttcaagacaaggagtcggttttttc
acctcggagatacttcaacatcgaccccaacgcaacgcaagcctctgggaactgtggcacccgaaaatccaacctt
ctgttgaattttcagggcggatttgtgaatctcacatttaccaaggatgaagaatcatattatatcagtgaagtgg
gagcctatttgaccgtctcagatccagagacagtttaccaaggaatcaaacatgcggtggtgatgttccagacagc
agtcgggcattccttcaagtgcgtgagtgaacagagcctccagttgtcagcccacctgcaggtgaaaacaaccgat
gtccaacttcaagcctttgattttgaagatgaccactttggaaatgtggatgagtgctcgtctgactacacaattg
tgcttcctgtgattggggccatcgtggttggtctctgccttatgggtatgggtgtctataaaatccgcctaaggtg
tcaatcatctggataccagagaatctaattgttgcccggggggggaatgaaaataatggaatttagagaactctttca
tcccttccaggatggatgttgggaaattccctcagagtgtgggtccttcaaacaatgtaaaccaccatcttctatt
caaatgaagtgagtcatgtgtgatttaagttcaggcagcacatcaatttctaaatacttttttgtttatttttatgaa
agatatagtgagctgttttattttctagtttcctttagaatattttagccactcaaagtcaacatttgagatatgtt
gaattaacataatatatgtaaagtagaataagccttcaaattataaaccaagggtcaattgtaactaatactactg
```

```
tgtgtgcattgaagattttattttacccttgatcttaacaaagcctttgctttgttatcaaatggactttcagtgc
ttttactatctgtgtttttatggtttcatgtaacatacatattcctggtgtagcacttaactccttttccactttaa
atttgttttttgtttttttgagacggagtttcactcttgtcacccaggctggagtacagtggcacgatctcggcttat
ggcaacctccgcctcccgggttcaagtgattctcctgcttcagcttcccgagtagctgggattacaggcacacact
accacgcctggctaattttttgtattttttattatagacgggtttcaccatgttggccagactggtcttgaactcttg
acctcaggtgatccacccacctcagcctcccaaagtgctgggattacaggcatgagccattgcgcccggccttaaa
tgtttttttttaatcatcaaaaagaacaacatatctcaggttgtctaagtgttttttatgtaaaaccaacaaaaagaa
caaatcagcttatattttttatcttgatgactcctgctccagaattgctagactaagaattaggtggctacagatg
gtagaactaaacaataagcaagagacaataataatggcccttaattattaacaaagtgccagagtctaggctaagc
actttatctatatctcatttcattctcacaacttataagtgaatgagtaaactgagacttaagggaactgaatcac
ttaaatgtcacctggctaactgatggcagagccagagcttgaattcatgttggtctgacatcaaggtctttggtct
tctccctacaccaagttacctacaagaacaatgacaccacactctgcctgaaggctcacacctcataccagcatac
gctcaccttacagggaaatgggtttatccaggatcatgagacattagggtagatgaaaggagagctttgcagataa
caaaatagcctatccttaataaatcctccactctctggaaggagactgagggggctttgtaaaacattagtcagttg
ctcattttttatgggattgcttagctgggctgtaaagatgaaggcatcaaataaactcaaagtattttttaaattttt
ttgataatagagaaacttcgctaaccaactgttctttcttgagtgtatagccccatcttgtggtaacttgctgctt
ctgcacttcatatccatatttcctattgttcactttattctgtagagcagcctgccaagaattttatttctgctgt
tttttttgctgctaaagaaaggaactaagtcaggatgttaacagaaaagtccacataaccctagaattcttagtca
aggaataattcaagtcagcctagagaccatgttgactttcctcatgtgtttcctttatgactcagtaagttggcaag
gtcctgactttagtcttaataaaacattgaattgtagtaaaggttttttgcaataaaaacttactttgg
```

Mouse LAMP1 (SEQ ID NO:44)
>MouseLamp1 0-1858

```
                attccggaggtgaaaaacaatggcacaacgtgtataatggccagcttctctgcctcctttctgaccacct
acgagactgcgaatggttctcagatcgtgaacatttccctgccagcctctgcagaagtactgaaaaatggcagttc
ttgtggtaaagaaaatgtttctgaccccagcctcacaattacttttggaagaggatatttactgacactcaacttc
acaaaaaatacaacacgttacagtgtccagcatatgtattttacatataacttgtcagatacagaacattttccca
atgccatcagcaaagagatctacaccatggattccacaactgacatcaaggcagacatcaacaaagcataccggtg
tgtcagtgatatccgggtctacatgaagaatgtgaccgttgtgctccgggatgccactatccaggcctacctgtcg
agtggcaacttcagcaaggaagagacacactgcacacaggatggaccttccccaaccactgggccacccagcccct
caccaccacttgtgcccacaaaccccactgtatccaagtacaatgttactggtaacaacggaacctgcctgctggc
ctctatggcactgcaactgaatatcacctacctgaaaaaggacaacaagacggtgaccagagcgttcaacatcagc
ccaaatgacacatctagtgggagttgcggtatcaacttggtgaccctgaaagtggagaacaagaacagagccctgg
aattgcagtttgggatgaatgccagctctagcctgttttttcttgcaaggagtgcgcttgaatatgactcttcctga
tgccctagtgcccacattcagcatctccaaccattcactgaaagctcttcaggccactgtgggaaactcatacaag
tgcaacactgaggaacacatctttgtcagcaagatgctctccctcaatgtcttcagtgtgcaggtccaggcttttca
aggtggacagtgacaggtttgggtctgtggaagagtgtgttcaggatggtaacaacatgttgatccccattgctgt
gggcggtgccctggcagggctgatcctcatcgtcctcattgcctacctcattggcaggaagaggagtcacgccggc
tatcagaccatctagcctggtgggcaggtgcaccagagatgcacaggggcctgttctcacatccccaagcttagat
aggtgtggaagggaggcacactttctggcaaactgttttaaaatctgctttatcaaatgtgaagttcatcttgcaa
catttactatgcacaaaggaataactattgaaatgacggtgttaattttgctaactgggttaaatattgatgagaa
ggctccactgatttgacttttaagacttggtgtttggttcttcattcttttactcagatttaagcctatcaaaggg
atactctggtccagaccttggcctggcaaggtggctgatggttaggctgcacacacttaagaagcaacgggagca
gggaaggcttgcacacaggcacgcacagggtcaacctctggacacttggcttgggctacctggccttggggggggct
gaactctggcatctggctgggtacacacccccccaatttctgtgctctgcacccgtgagctgccactttcctaaa
tagaaaatggcattattttttatttacttttttgtaaagtgatttccagtcttgtgttggcgttcagggtggccctg
tctctgcactgtgtacaataatagattcacactgctgacgtgtcttgcagcgtaggtgggttgtacactgggcatc
agctcacgtaatgcattgcctgtaacgatgctaataaaaa
```

Human Lamp1 cDNA (SEQ ID NO:45)
>Human Lamp1 0-2339

```
        ggcccaaccgccgcccgcgcccccgctctccgcaccgtacccggccgcctcgcgccatggcggcccccgg
cagcgcccggcgacccctgctgctgctactgctgttgctgctgctcggcctcatgcattgtgcgtcagcagcaatg
tttatggtgaaaaatggcaacgggaccgcgtgcataatggccaacttctctgctgccttctcagtgaactacgaca
ccaagagtggcccctaagaacatgacctttgacctgccatcagatgccacagtggtgctcaaccgcagctcctgtgg
aaaagagaacacttctgaccccagtctcgtgattgcttttggaagaggacatacactcactctcaatttcacgaga
aatgcaacacgttacagcgtccagctcatgagtttttgtttataacttgtcagacacacacctttttccccaatgcga
gctccaaagaaatcaagactgtggaatctataactgacatcagggcagatatagataaaaaatacagatgtgttag
tggcacccaggtccacatgaacaacgtgaccgtaacgctccatgatgccaccatccaggcgtacctttccaacagc
agcttcagcaggggagagacacgctgtgaacaagacaggccttccccaaccacagcgcccccgcgccacccagcc
cctcgccctcacccgtgcccaagagcccctctgtggacaagtacaacgtgagcggcaccaacgggacctgcctgct
ggccagcatggggctgcagctgaacctcacctatgagaggaaggacaacacgacggtgacaaggcttctcaacatc

aaccccaacaagacctcggccagcgggagctgcggcgcccacctggtgactctggagctgcacagcgagggcacca
ccgtcctgctcttccagttcgggatgaatgcaagttctagccggttttttcctacaaggaatccagttgaatacaat
tcttcctgacgccagagaccctgcctttaaagctgccaacggctccctgcgagcgctgcaggccacagtcggcaat
tcctacaagtgcaacgcggaggagcacgtccgtgtcacgaaggcgttttcagtcaatatattcaaagtgtgggtcc
aggctttcaaggtggaaggtggccagtttggctctgtggaggagtgtctgctggacgagaacagcatgctgatccc
catcgctgtgggtggtgccctggcggggctggtcctcatcgtcctcatcgcctacctcgtcggcaggaagaggagt
cacgcaggctaccagactatctagcctggtgcacgcaggcacagcagctgcaggggcctctgttcctttctctggg
cttagggtcctgtcgaaggggaggcacactttctggcaaacgtttctcaaatctgcttcatccaatgtgaagttca
tcttgcagcatttactatgcacaacagagtaactatcgaaatgacggtgttaattttgctaactgggttaaatatt
ttgctaactggttaaacattaatatttaccaaagtaggattttgagggtgggggtgctctctctgagggggtgggg
gtgccgctgtctctgagggggtgggggtgccgctgtctctgagggggtgggggtgccgctctctctgagggggtgggg
gtgccgctttctctgagggggtgggggtgccgctctctctgagggggtgggggtgctgctctctccgagggggtgga
atgccgctgtctctgagggggtgggggtgccgctctaaattggctccatatcatttgagtttaggttctggtgttt
ggtttcttcattctttactgcactcagatttaagccttacaaagggaaagcctctggccgtcacacgtaggacgca
tgaaggtcactcgtggtgaggctgacatgctcacacattacaacagtagagagggaaaatcctaagacagaggaac
tccagagatgagtgtctggagcgcttcagttcagctttaaaggccaggacgggccacacgtggctggcggcctcgt
tccagtggcggcacgtccttgggcgtctctaatgtctgcagctcaagggctggcacttttttaaatataaaaatgg
gtgttattttttattttttttttgtaaagtgattttttggtcttctgttgacattcggggtgatcctgttctgcgctgt
gtacaatgtgagatcggtgcgttctcctgatgttttgccgtggcttggggattgtacacgggaccagctcacgtaa
tgcattgcctgtaacaatgtaataaaaagcctctttcttttaaaaaaaaaaaaaaaaaaaaaaaa
```

Tetanus toxoid nulceic acid sequence (SEQ ID NO:46)
CAGTACATCAAGGCCAACAGCAAGTTCATCGGCATCACCGAACTC

Tetanus toxoid amino acid sequence (SEQ ID NO:47)
QYIKANSKFIGITEL

PADRE nulceotide sequence (SEQ ID NO:48)
GCTAAATTTGTGGCTGCCTGGACACTGAAAGCCGCCGCT

PADRE amino acid sequence (SEQ ID NO:49)
AKFVAAWTLKAAA

WPRE (SEQ ID NO:50)
>WPRE 0-593

```
        aatcaacctctggattacaaaatttgtgaaagattgactggtattcttaactatgttgctccttttacgc
tatgtggatacgctgctttaatgcctttgtatcatgctattgcttcccgtatggctttcattttctcctccttgta
taaatcctggttgctgtctctttatgaggagttgtggcccgttgtcaggcaacgtggcgtggtgtgcactgtgttt
gctgacgcaacccccactggttggggcattgccaccacctgtcagctcctttccgggactttcgctttccccctcc
ctattgccacggcggaactcatcgccgcctgccttgcccgctgctggacaggggctcggctgttgggcactgacaa
ttccgtggtgttgtcggggaagctgacgtcctttccatggctgctcgcctgtgttgccacctggattctgcgcggg
acgtccttctgctacgtcccttcggccctcaatccagcggaccttccttcccgcggcctgctgccggctctgcggc
ctcttccgcgtcttcgccttcgccctcagacgagtcggatctccctttgggccgcctccccgcctgt
```

IRES (SEQ ID NO:51)
>eGFP_IRES_SEAP_Insert 1746-2335

```
tctccccccccccctctccctcccccccccccctaacgttactggccgaagccgcttggaataaggccggt
gtgcgtttgtctatatgttattttccaccatattgccgtcttttggcaatgtgagggcccggaaacctggccctgt
cttcttgacgagcattcctaggggtctttcccctctcgccaaaggaatgcaaggtctgttgaatgtcgtgaaggaa
gcagttcctctggaagcttcttgaagacaaacaacgtctgtagcgacccctttgcaggcagcggaacccccccacctg
gcgacaggtgcctctgcggccaaaagccacgtgtataagatacacctgcaaaggcggcacaacccccagtgccacgt
tgtgagttggatagttgtggaaagagtcaaatggctctcctcaagcgtattcaacaaggggctgaaggatgcccag
aaggtaccccattgtatgggatctgatctggggcctcggtgcacatgctttacatgtgttttagtcgaggttaaaaa
aacgtctaggcccccgaaccacggggacgtggttttcctttgaaaaacacgatgataatatg
```

GFP (SEQ ID NO:52)

```
atggtgagcaagggcgaggagctgttcaccggggtggtgcccatcctggtcgagctggacggcgacgtaa
acggccacaagttcagcgtgtccggcgagggcgagggcgatgccacctacggcaagctgaccctgaagttcatctg
caccaccggcaagctgcccgtgccctggcccaccctcgtgaccaccctgacctacggcgtgcagtgcttcagccgc
taccccgaccacatgaagcagcacgacttcttcaagtccgccatgcccgaaggctacgtccaggagcgcaccatct
tcttcaaggacgacggcaactacaagacccgcgccgaggtgaagttcgagggcgacaccctggtgaaccgcatcga
gctgaagggcatcgacttcaaggaggacggcaacatcctggggcacaagctggagtacaactacaacagccacaac

gtctatatcatggccgacaagcagaagaacggcatcaaggtgaacttcaagatccgccacaacatcgaggacggca
gcgtgcagctcgccgaccactaccagcagaacacccccatcggcgacggccccgtgctgctgcccgacaaccacta
cctgagcacccagtccgccctgagcaaagacccaacgagaagcgcgatcacatggtcctgctggagttcgtgacc
gccgccgggatcactctcggcatggacgagctgtacaagtag
```

SEAP (SEQ ID NO:53)

```
atgctgctgctgctgctgctgctgggcctgaggctacagctctccctgggcatcatcccagttgaggagg
agaacccggacttctggaaccgcgaggcagccgaggccctgggtgccgccaagaagctgcagcctgcacagacagc
cgccaagaacctcatcatcttcctgggcgatgggatgggggtgtctacggtgacagctgccaggatcctaaaaggg
cagaagaaggacaaactggggcctgagatacccctggccatggaccgcttcccatatgtggctctgtccaagacat
acaatgtagacaaacatgtgccagacagtggagccacagccacggcctacctgtgcgggggtcaagggcaacttcca
gaccattggcttgagtgcagccgccgctttaaccagtgcaacacgacacgcggcaacgaggtcatctccgtgatg
aatcgggccaagaaagcagggaagtcagtggggagtggtaaccaccacacgagtgcagcacgcctcgccagccggca
cctacgcccacacggtgaaccgcaactggtactcggacgccgacgtgcctgcctcggcccgccaggaggggtgcca
ggacatcgctacgcagctcatctccaacatggacattgacgtgatcctaggtggaggccgaaagtacatgtttcgc
atgggaaccccagaccctgagtacccagatgactacagccaaggtgggaccaggctggacgggaagaatctggtgc
aggaatggctggcgaagcgccagggtgcccggtatgtgtggaaccgcactgagctcatgcaggcttccctggaccc
gtctgtgacccatctcatggtctcttttgagcctggagacatgaaatacgagatccaccgagactccacactggac
ccctccctgatggagatgacagaggctgccctgcgcctgctgagcaggaacccccgcggcttcttcctcttcgtgg
agggtggtcgcatcgaccatggtcatcatgaaagcagggcttaccgggcactgactgagacgatcatgttcgacga
cgccattgagagggcgggccagctcaccagcgaggaggacacgctgagcctcgtcactgccgaccactcccacgtc
ttctccttcggaggctaccccctgcgagggagctccatcttcgggctggcccctggcaaggcccgggacaggaagg
cctacacggtcctcctatacgaaacggtccaggctatgtgctcaaggacggcgcccggccggatgttaccgagag
cgagagcgggagccccgagtatcggcagcagtcagcagtgcccctggacgaagagacccacgcaggcgaggacgtg
gcggtgttcgcgcgcggcccgcaggcgcacctggttcacggcgtgcaggagcagaccttcatagcgcacgtcatgg
ccttcgccgcctgcctggagccctacaccgcctgcgacctggcgcccccgccggcaccaccgacgccgcgcaccc
gggttactctagagtcggggcggccggccgcttcgagcagacatgataa
```

Firefly Luciferase (SEQ ID NO:54)

```
atggaagatgccaaaaacattaagaagggcccagcgccattctacccactcgaagacgggaccgccggcg
agcagctgcacaaagccatgaagcgctacgccctggtgcccggcaccatcgcctttaccgacgcacatatcgaggt
ggacattacctacgccgagtacttcgagatgagcgttcggctggcagaagctatgaagcgctatgggctgaataca
aaccatcggatcgtggtgtgcagcgagaatagcttgcagttcttcatgcccgtgttgggtgccctgttcatcggtg
tggctgtggccccagctaacgacatctacaacgagcgcgagctgctgaacagcatgggcatcagccagcccaccgt
cgtattcgtgagcaagaaagggctgcaaaagatcctcaacgtgcaaaagaagctaccgatcatacaaaagatcatc
atcatggatagcaagaccgactaccagggcttccaaagcatgtacaccttcgtgacttcccatttgccacccggct
tcaacgagtacgacttcgtgcccgagagcttcgaccgggacaaaaccatcgccctgatcatgaacagtagtggcag
taccggattgcccaagggcgtagccctaccgcaccgcaccgcttgtgtccgattcagtcatgcccgcgaccccatc
ttcggcaaccagatcatccccgacaccgctatcctcagcgtggtgccatttcaccacggcttcggcatgttcacca
cgctgggctacttgatctgcggctttcgggtcgtgctcatgtaccgcttcgaggaggagctattcttgcgcagctt
gcaagactataagattcaatctgccctgctggtgcccacactatttagcttcttcgctaagagcactctcatcgac
aagtacgacctaagcaacttgcacgagatcgccagcggcggggcgccgctcagcaaggaggtaggtgaggccgtgg
ccaaacgcttccacctaccaggcatccgccagggctacggcctgacagaaacaaccagcgccattctgatcacccc
cgaaggggacgacaagcctggcgcagtaggcaaggtggtgcccttcttcgaggctaaggtggtggacttggacacc
ggtaagacactgggtgtgaaccagcgcggcgagctgtgcgtccgtggcccccatgatcatgagcggctacgttaaca
accccgaggctacaaacgctctcatcgacaaggacggctggctgcacagcggcgacatcgcctactgggacgagga
cgagcacttcttcatcgtggaccggctgaagagcctgatcaaatacaagggctaccaggtagccccagccgaactg
gagagcatcctgctgcaacaccccaacatcttcgacgccggggtcgccggcctgcccgacgacgatgccggcgagc
tgcccgccgcagtcgtcgtgctggaacacggtaaaaccatgaccgagaaggagatcgtggactatgtggccagcca
ggttacaaccgccaagaagctgcgcggtggtgttgtgttcgtggacgaggtgcctaaaggactgaccggcaagttg
gacgcccgcaagatccgcgagattctcattaaggccaagaagggcggcaagatcgccgtgtaa
```

FMDV 2A (SEQ ID NO:55)
GTAAAGCAAACACTGAACTTTGACCTTCTCAAGTTGGCTGGAGACGTTGAGTCCAATCCTGGGCCC

## References

1. Desrichard, A., Snyder, A. & Chan, T. A. Cancer Neoantigens and Applications for Immunotherapy. Clin. Cancer Res. Off. J. Am. Assoc. Cancer Res. (2015). doi:10.1158/1078-0432.CCR-14-3175

2. Schumacher, T. N. & Schreiber, R. D. Neoantigens in cancer immunotherapy. Science 348, 69-74 (2015).

3. Gubin, M. M., Artyomov, M. N., Mardis, E. R. & Schreiber, R. D. Tumor neoantigens: building a framework for personalized cancer immunotherapy. J. Clin. Invest. 125, 3413-3421 (2015).

4. Rizvi, N. A. et al. Cancer immunology. Mutational landscape determines sensitivity to PD-1 blockade in non-small cell lung cancer. Science 348, 124-128 (2015).

5. Snyder, A. et al. Genetic basis for clinical response to CTLA-4 blockade in melanoma. N. Engl. J. Med. 371, 2189-2199 (2014).

6. Carreno, B. M. et al. Cancer immunotherapy. A dendritic cell vaccine increases the breadth and diversity of melanoma neoantigen-specific T cells. Science 348, 803-808 (2015).

7. Tran, E. et al. Cancer immunotherapy based on mutation-specific CD4+ T cells in a patient with epithelial cancer. Science 344, 641-645 (2014).

8. Hacohen, N. & Wu, C. J.-Y. United States Patent Application: 0110293637 - COMPOSITIONS AND METHODS OF IDENTIFYING TUMOR SPECIFIC NEOANTIGENS. (A1). at <http://appft1.uspto.gov/netacgi/nph-Parser?Sect1=PTO1&Sect2=HITOFF&d=PG01&p=1&u=/netahtml/PTO/srchnum.html&r=1&f=G&l=50&s1=20110293637.PGNR.>

9. Lundegaard, C., Hoof, I., Lund, O. & Nielsen, M. State of the art and challenges in sequence based T-cell epitope prediction. Immunome Res. 6 Suppl 2, S3 (2010).

10. Yadav, M. et al. Predicting immunogenic tumour mutations by combining mass spectrometry and exome sequencing. Nature 515, 572-576 (2014).

11. Bassani-Stemberg, M., Pletscher-Frankild, S., Jensen, L. J. & Mann, M. Mass spectrometry of human leukocyte antigen class I peptidomes reveals strong effects of protein abundance and turnover on antigen presentation. Mol. Cell. Proteomics MCP 14, 658-673 (2015).

12. Van Allen, E. M. et al. Genomic correlates of response to CTLA-4 blockade in metastatic melanoma. Science 350, 207-211 (2015).

13. Yoshida, K. & Ogawa, S. Splicing factor mutations and cancer. Wiley Interdiscip. Rev. RNA 5, 445-459 (2014).

14. Cancer Genome Atlas Research Network. Comprehensive molecular profiling of lung adenocarcinoma. Nature 511, 543-550 (2014).

15. Rajasagi, M. et al. Systematic identification of personal tumor-specific neoantigens in chronic lymphocytic leukemia. Blood 124, 453-462 (2014).

16. Downing, S. R. et al. United States Patent Application: 0120208706 - OPTIMIZATION OF MULTIGENE ANALYSIS OF TUMOR SAMPLES. (A1). at <http://appft1.uspto.gov/netacgi/nph-Parser?Sect1=PTO1&Sect2=HIT OFF&d=PG01&p=1&u=/netahtml/PTO/srchnum.html&r=1& f=G&l=50&s1=20120208706.PGNR.>

17. Target Capture for NextGen Sequencing - IDT. at <http://www.idtdna.com/pages/products/nextgen/target-capture>

18. Shukla, S. A. et al. Comprehensive analysis of cancer-associated somatic mutations in class I HLA genes. Nat. Biotechnol. 33, 1152-1158 (2015).

19. Cieslik, M. et al. The use of exome capture RNA-seq for highly degraded RNA with application to clinical cancer sequencing. Genome Res. 25, 1372-1381 (2015).

20. Bodini, M. et al. The hidden genomic landscape of acute myeloid leukemia: subclonal structure revealed by undetected mutations. Blood 125, 600-605 (2015).

21. Saunders, C. T. et al. Strelka: accurate somatic small-variant calling from sequenced tumor-normal sample pairs. Bioinforma. Oxf. Engl. 28, 1811-1817 (2012).

22. Cibulskis, K. et al. Sensitive detection of somatic point mutations in impure and heterogeneous cancer samples. Nat. Biotechnol. 31, 213-219 (2013).

23. Wilkerson, M. D. et al. Integrated RNA and DNA sequencing improves mutation detection in low purity tumors. Nucleic Acids Res. 42, e107 (2014).

24. Mose, L. E., Wilkerson, M. D., Hayes, D. N., Perou, C. M. & Parker, J. S. ABRA: improved coding indel detection via assembly-based realignment. Bioinforma. Oxf. Engl. 30, 2813-2815 (2014).

25. Ye, K., Schulz, M. H., Long, Q., Apweiler, R. & Ning, Z. Pindel: a pattern growth approach to detect break points of large deletions and medium sized insertions from paired-end short reads. Bioinforma. Oxf. Engl. 25, 2865-2871 (2009).

26. Lam, H. Y. K. et al. Nucleotide-resolution analysis of structural variants using BreakSeq and a breakpoint library. Nat. Biotechnol. 28, 47-55 (2010).

27. Frampton, G. M. et al. Development and validation of a clinical cancer genomic profiling test based on massively parallel DNA sequencing. Nat. Biotechnol. 31, 1023-1031 (2013).

28. Boegel, S. et al. HLA typing from RNA-Seq sequence reads. Genome Med. 4, 102 (2012).

29. Liu, C. et al. ATHLATES: accurate typing of human leukocyte antigen through exome sequencing. Nucleic Acids Res. 41, e142 (2013).

30. Mayor, N. P. et al. HLA Typing for the Next Generation. PloS One 10, e0127153 (2015).

31. Roy, C. K., Olson, S., Graveley, B. R., Zamore, P. D. & Moore, M. J. Assessing long-distance RNA sequence connectivity via RNA-templated DNA-DNA ligation. eLife 4, (2015).

32. Song, L. & Florea, L. CLASS: constrained transcript assembly of RNA-seq reads. BMC Bioinformatics 14 Suppl 5, S14 (2013).

33. Maretty, L., Sibbesen, J. A. & Krogh, A. Bayesian transcriptome assembly. Genome Biol. 15, 501 (2014).

34. Pertea, M. et al. StringTie enables improved reconstruction of a transcriptome from RNA-seq reads. Nat. Biotechnol. 33, 290-295 (2015).

35. Roberts, A., Pimentel, H., Trapnell, C. & Pachter, L. Identification of novel transcripts in annotated genomes using RNA-Seq. Bioinforma. Oxf. Engl. (2011). doi:10.1093/bioinformatics/btr355

36. Vitting-Seerup, K., Porse, B. T., Sandelin, A. & Waage, J. spliceR: an R package for classification of alternative splicing and prediction of coding potential from RNA-seq data. BMC Bioinformatics 15, 81 (2014).

37. Rivas, M. A. et al. Human genomics. Effect of predicted protein-truncating genetic variants on the human transcriptome. Science 348, 666-669 (2015).

38. Skelly, D. A., Johansson, M., Madeoy, J., Wakefield, J. & Akey, J. M. A powerful and flexible statistical framework for testing hypotheses of allele-specific gene expression from RNA-seq data. Genome Res. 21, 1728-1737 (2011).

39. Anders, S., Pyl, P. T. & Huber, W. HTSeq--a Python framework to work with high-throughput sequencing data. Bioinforma. Oxf. Engl. 31, 166-169 (2015).

40. Furney, S. J. et al. SF3B1 mutations are associated with alternative splicing in uveal melanoma. Cancer Discov. (2013). doi:10.1158/2159-8290.CD-13-0330

41. Zhou, Q. et al. A chemical genetics approach for the functional assessment of novel cancer genes. Cancer Res. (2015). doi:10.1158/0008-5472.CAN-14-2930

42. Maguire, S. L. et al. SF3B1 mutations constitute a novel therapeutic target in breast cancer. J. Pathol. 235, 571-580 (2015).

43. Carithers, L. J. et al. A Novel Approach to High-Quality Postmortem Tissue Procurement: The GTEx Project. Biopreservation Biobanking 13, 311-319 (2015).

44. Xu, G. et al. RNA CoMPASS: a dual approach for pathogen and host transcriptome analysis of RNA-seq datasets. PloS One 9, e89445 (2014).

45. Andreatta, M. & Nielsen, M. Gapped sequence alignment using artificial neural networks: application to the MHC class I system. Bioinforma. Oxf. Engl. (2015). doi:10.1093/bioinformatics/btv639

46. Jorgensen, K. W., Rasmussen, M., Buus, S. & Nielsen, M. NetMHCstab - predicting stability of peptide-MHC-I complexes; impacts for cytotoxic T lymphocyte epitope discovery. Immunology 141, 18-26 (2014).

47. Larsen, M. V. et al. An integrative approach to CTL epitope prediction: a combined algorithm integrating MHC class I binding, TAP transport efficiency, and proteasomal cleavage predictions. Eur. J. Immunol. 35, 2295-2303 (2005).

48. Nielsen, M., Lundegaard, C., Lund, O. & Keşmir, C. The role of the proteasome in generating cytotoxic T-cell epitopes: insights obtained from improved predictions of proteasomal cleavage. *Immunogenetics* **57,** 33-41 (2005).

49. Boisvert, F.-M. et al. A Quantitative Spatial Proteomics Analysis of Proteome Turnover in Human Cells. Mol. Cell. Proteomics 11, M111.011429-M111.011429 (2012).

50. Duan, F. et al. Genomic and bioinformatic profiling of mutational neoepitopes reveals new rules to predict anticancer immunogenicity. J. Exp. Med. 211, 2231-2248 (2014).

51. Janeway's Immunobiology: 9780815345312: Medicine & Health Science Books @ Amazon.com. at <http://www.amazon.com/Janeways-Immunobiology-Kenneth-Murphy/dp/0815345313>

52. Calis, J. J. A. et al. Properties of MHC Class I Presented Peptides That Enhance Immunogenicity. PLoS Comput. Biol. 9, e1003266 (2013).

53. Zhang, J. et al. Intratumor heterogeneity in localized lung adenocarcinomas delineated by multiregion sequencing. Science 346, 256-259 (2014)

54. Walter, M. J. et al. Clonal architecture of secondary acute myeloid leukemia. N. Engl. J. Med. 366, 1090-1098 (2012).

55. Hunt DF, Henderson RA, Shabanowitz J, Sakaguchi K, Michel H, Sevilir N, Cox AL, Appella E, Engelhard VH. Characterization of peptides bound to the class I MHC molecule HLA-A2.1 by mass spectrometry. Science 1992. 255: 1261-1263.

56. Zarling AL, Polefrone JM, Evans AM, Mikesh LM, Shabanowitz J, Lewis ST, Engelhard VH, Hunt DF. Identification of class I MHC-associated phosphopeptides as targets for cancer immunotherapy. Proc Natl Acad Sci U S A. 2006 Oct 3;103(40):14889-94.

57. Bassani-Sternberg M, Pletscher-Frankild S, Jensen LJ, Mann M. Mass spectrometry of human leukocyte antigen class I peptidomes reveals strong effects of protein abundance and turnover on antigen presentation. Mol Cell Proteomics. 2015 Mar;14(3):658-73. doi: 10.1074/mcp.M114.042812.

58. Abelin JG, Trantham PD, Penny SA, Patterson AM, Ward ST, Hildebrand WH, Cobbold M, Bai DL, Shabanowitz J, Hunt DF. Complementary IMAC enrichment methods for HLA-associated phosphopeptide identification by mass spectrometry. Nat Protoc. 2015 Sep;10(9):1308-18. doi: 10.1038/nprot.2015.086. Epub 2015 Aug 6

59. Barnstable CJ, Bodmer WF, Brown G, Galfre G, Milstein C, Williams AF, Ziegler A. Production of monoclonal antibodies to group A erythrocytes, HLA and other human cell surface antigens-new tools for genetic analysis. Cell. 1978 May;14(1):9-20.

60. Goldman JM, Hibbin J, Kearney L, Orchard K, Th'ng KH. HLA-DR monoclonal antibodies inhibit the proliferation of normal and chronic granulocytic leukaemia myeloid progenitor cells. Br J Haematol. 1982 Nov;52(3):411-20.

61. Eng JK, Jahan TA, Hoopmann MR. Comet: an open-source MS/MS sequence database search tool. Proteomics. 2013 Jan;13(1):22-4. doi: 10.1002/pmic.201200439. Epub 2012 Dec 4.

62. Eng JK, Hoopmann MR, Jahan TA, Egertson JD, Noble WS, MacCoss MJ. A deeper look into Comet-- implementation and features. J Am Soc Mass Spectrom. 2015 Nov;26(11):1865-74. doi: 10.1007/s13361-015-1179-x. Epub 2015 Jun 27.

63. Lukas Käll, Jesse Canterbury, Jason Weston, William Stafford Noble and Michael J. MacCoss. Semi-supervised learning for peptide identification from shotgun proteomics datasets. Nature Methods 4:923 - 925, November 2007

64. Lukas Käll, John D. Storey, Michael J. MacCoss and William Stafford Noble. Assigning confidence measures to peptides identified by tandem mass spectrometry. Journal of Proteome Research, 7(1):29-34, January 2008

65. Lukas Käll, John D. Storey and William Stafford Noble. Nonparametric estimation of posterior error probabilities associated with peptides identified by tandem mass spectrometry. Bioinformatics, 24(16):142-148, August 2008

66. Kinney RM, BJ Johnson , VL Brown , DW Trent. Nucleotide Sequence of the 26 S mRNA of the Virulent Trinidad Donkey Strain of Venezuelan Equine Encephalitis Virus and Deduced Sequence of the Encoded Structural Proteins. Virology 152 (2), 400-413. 1986 Jul 30.

67. Jill E Slansky, Frédérique M Rattis, Lisa F Boyd, Tarek Fahmy, Elizabeth M Jaffee, Jonathan P Schneck, David H Margulies, Drew M Pardoll. Enhanced Antigen-Specific Antitumor Immunity with Altered Peptide Ligands that Stabilize the MHC-Peptide-TCR Complex. Immunity, Volume 13, Issue 4, 1 October 2000, Pages 529-538.

68. A Y Huang, P H Gulden, A S Woods, M C Thomas, C D Tong, W Wang, V H Engelhard, G Pasternack, R Cotter, D Hunt, D M Pardoll, and E M Jaffee. The immunodominant major histocompatibility complex class I-restricted antigen of a murine colon tumor derives from an endogenous retroviral gene product. Proc Natl Acad Sci U S A.; 93(18): 9730-9735, 1996 Sep 3.

**EP 3 544 607 B1**

69. JOHNSON, BARBARA J. B., RICHARD M. KINNEY, CRYSTLE L. KOST AND DENNIS W. TRENT. Molecular Determinants of Alphavirus Neurovirulence: Nucleotide and Deduced Protein Sequence Changes during Attenuation of Venezuelan Equine Encephalitis Virus. J Gen Virol 67:1951-1960, 1986.

70. Aamoudse, C.A., Krüse, M., Konopitzky, R., Brouwenstijn, N., and Schrier, P.I. (2002). TCR reconstitution in Jurkat reporter cells facilitates the identification of novel tumor antigens by cDNA expression cloning. Int J Cancer 99, 7-13.

71. Alexander, J., Sidney, J., Southwood, S., Ruppert, J., Oseroff, C., Maewal, A., Snoke, K., Serra, H.M., Kubo, R.T., and Sette, A. (1994). Development of high potency universal DR-restricted helper epitopes by modification of high affinity DR-blocking peptides. Immunity 1, 751-761.

72. Banu, N., Chia, A., Ho, Z.Z., Garcia, A.T., Paravasivam, K., Grotenbreg, G.M., Bertoletti, A., and Gehring, A.J. (2014). Building and optimizing a virus-specific T cell receptor library for targeted immunotherapy in viral infections. Scientific Reports 4, 4166.

73. Cornet, S., Miconnet, I., Menez, J., Lemonnier, F., and Kosmatopoulos, K. (2006). Optimal organization of a polypeptide-based candidate cancer vaccine composed of cryptic tumor peptides with enhanced immunogenicity. Vaccine 24, 2102-2109.

74. Depla, E., van der Aa, A., Livingston, B.D., Crimi, C., Allosery, K., de Brabandere, V., Krakover, J., Murthy, S., Huang, M., Power, S., et al. (2008). Rational design of a multiepitope vaccine encoding T-lymphocyte epitopes for treatment of chronic hepatitis B virus infections. Journal of Virology 82, 435-450.

75. Ishioka, G.Y., Fikes, J., Hermanson, G., Livingston, B., Crimi, C., Qin, M., del Guercio, M.F., Oseroff, C., Dahlberg, C., Alexander, J., et al. (1999). Utilization of MHC class I transgenic mice for development of minigene DNA vaccines encoding multiple HLA-restricted CTL epitopes. J Immunol 162, 3915-3925.

76. Janetzki, S., Price, L., Schroeder, H., Britten, C.M., Welters, M.J.P., and Hoos, A. (2015). Guidelines for the automated evaluation of Elispot assays. Nat Protoc 10, 1098-1115.

77. Lyons, G.E., Moore, T., Brasic, N., Li, M., Roszkowski, J.J., and Nishimura, M.I. (2006). Influence of human CD8 on antigen recognition by T-cell receptor-transduced cells. Cancer Res 66, 11455-11461.

78. Nagai, K., Ochi, T., Fujiwara, H., An, J., Shirakata, T., Mineno, J., Kuzushima, K., Shiku, H., Melenhorst, J.J., Gostick, E., et al. (2012). Aurora kinase A-specific T-cell receptor gene transfer redirects T lymphocytes to display effective antileukemia reactivity. Blood 119, 368-376.

79. Panina-Bordignon, P., Tan, A., Termijtelen, A., Demotz, S., Corradin, G., and Lanzavecchia, A. (1989). Universally immunogenic T cell epitopes: promiscuous binding to human MHC class II and promiscuous recognition by T cells. Eur J Immunol 19, 2237-2242.

80. Vitiello, A., Marchesini, D., Furze, J., Sherman, L.A., and Chesnut, R.W. (1991). Analysis of the HLA-restricted influenza-specific cytotoxic T lymphocyte response in transgenic mice carrying a chimeric human-mouse class I major histocompatibility complex. J Exp Med 173, 1007-1015.

81. Yachi, P.P., Ampudia, J., Zal, T., and Gascoigne, N.R.J. (2006). Altered peptide ligands induce delayed CD8-T cell receptor interaction--a role for CD8 in distinguishing antigen quality. Immunity 25, 203-211.

82. Pushko P, Parker M, Ludwig GV, Davis NL, Johnston RE, Smith JF. Replicon-helper systems from attenuated Venezuelan equine encephalitis virus: expression of heterologous genes in vitro and immunization against heterologous pathogens in vivo. Virology. 1997 Dec 22;239(2):389-401.

83. Strauss, JH and E G Strauss. The alphaviruses: gene expression, replication, and evolution. Microbiol Rev. 1994 Sep; 58(3): 491-562.

84. Rhême C, Ehrengruber MU, Grandgirard D. Alphaviral cytotoxicity and its implication in vector development. Exp Physiol. 2005 Jan;90(1):45-52. Epub 2004 Nov 12.

85. Riley, Michael K. II, and Wilfred Vermerris. Recent Advances in Nanomaterials for Gene Delivery-A Review. Nanomaterials 2017, 7(5), 94.

86. Frolov I, Hardy R, Rice CM. Cis-acting RNA elements at the 5' end of Sindbis virus genome RNA regulate minus- and plus-strand RNA synthesis. RNA. 2001 Nov;7(11):1638-51.

87. Jose J, Snyder JE, Kuhn RJ. A structural and functional perspective of alphavirus replication and assembly. Future Microbiol. 2009 Sep;4(7):837-56.

**Claims**

1. A chimpanzee adenovirus vector comprising a neoantigen cassette, the neoantigen cassette comprising:

(1) a plurality of neoantigen-encoding nucleic acid sequences derived from a tumor present within a subject, the plurality comprising:

at least two tumor-specific and subject-specific MHC class I neoantigen-encoding nucleic acid sequences each comprising:

a. a MHC class I epitope encoding nucleic acid sequence with at least one alteration that makes the encoded peptide sequence distinct from the corresponding peptide sequence encoded by a wild-type nucleic acid sequence,

b. a 5' linker sequence, wherein the 5' linker sequence is a native 5' nucleic acid sequence of the MHC I epitope, and wherein the 5' linker sequence encodes a peptide that is at least 2 amino acids in length and

c. a 3' linker sequence, wherein the 3' linker sequence is a native 3' nucleic acid sequence of the MHC I epitope, and wherein the 3' linker sequence encodes a peptide that is at least 2 amino acids in length,

wherein at least one neoantigen-encoding nucleic acid sequence in the plurality is linked to a distinct neoantigen-encoding nucleic acid sequence in the plurality by its linker sequence;

(2) at least one promoter sequence operably linked to at least one sequence of the plurality,

(3) at least one MHC class II antigen-encoding nucleic acid sequence.

2. The vector of claim 1, comprising at least one GPGPG-encoding linker sequence (SEQ ID NO:56), optionally wherein the at least one GPGPG-encoding linker sequence (SEQ ID NO:56) links at least one MHC class II neoantigen-encoding nucleic acid sequence to one of the MHC class I neoantigen-encoding nucleic acid sequences.

3. The vector of claim 1, wherein an ordered sequence of each element of the neoantigen cassette is described in a formula, from 5' to 3', comprising:

$$P_a\text{-}(L5_b\text{-}N_c\text{-}L3_d)_X\text{-}(G5_e\text{-}U_f)_Y\text{-}G3_g\text{-}A_h$$

wherein P comprises the at least one promoter sequence operably linked to at least one sequence of the plurality, where a = 1,

N comprises one of the MHC class I epitope encoding nucleic acid sequence with at least one alteration that makes the encoded peptide sequence distinct from the corresponding peptide sequence encoded by the wild-type nucleic acid sequence, where c = 1,

L5 comprises the 5' linker sequence, where b = 1,

L3 comprises the 3' linker sequence, where d = 1,

G5 comprises a GPGPG-encoding linker sequence (SEQ ID NO:56), where e = 0 or 1,

G3 comprises a GPGPG-encoding linker sequence (SEQ ID NO:56), where g = 0 or 1,

U comprises one of the at least one MHC class II antigen-encoding nucleic acid sequence, where f = 1,

A comprises at least one polyadenylation sequence, where h = 0 or 1,

X = 2 to 400, where for each X the corresponding $N_c$ is a distinct MHC class I epitope encoding nucleic acid sequence, and

Y = 1-2, where for each Y the corresponding $U_f$ is a distinct MHC class II antigen-encoding nucleic acid sequence.

4. The vector of claim 3, wherein

b = 1, d = 1, e = 1, g = 1, h = 1, X = 20, Y = 2,

P is a CMV promoter sequence,

each N encodes a MHC class I epitope 7-15 amino acids in length,

L5 is the native 5' nucleic acid sequence of the MHC I epitope wherein the 5' linker sequence encodes a peptide that is at least 5 amino acids in length,

L3 is the native 3' nucleic acid sequence of the MHC I epitope wherein the 3' linker sequence encodes a peptide that is at least 5 amino acids in length,

U is each of a PADRE MHC class II sequence and a Tetanus toxoid MHC class II sequence,

the chimpanzee adenovirus vector comprises a modified ChAdV68 sequence comprising the sequence of SEQ ID NO: 1 having an E1 deletion from nucleotide 577 to nucleotide 3403 and an E3 deletion from nucleotide 27,125 to nucleotide 31,825 and the neoantigen cassette is inserted within the E1 deletion, and

each of the MHC class I neoantigen-encoding nucleic acid sequences encodes a polypeptide that is 25 amino acids in length.

5. The vector of claim 1, wherein at least one of the neoantigen-encoding nucleic acid sequences in the plurality encodes a polypeptide sequence or portion thereof that is presented by MHC class I on the tumor cell surface.

6. The vector of claim 5, wherein the plurality comprises at least 2-400 nucleic acid sequences and (1) wherein at least two of the neoantigen-encoding nucleic acid sequences in the plurality encode polypeptide sequences or portions thereof that are presented by MHC class I on the tumor cell surface, or (2) when administered to the subject and translated, at least one of the neoantigens are presented on antigen presenting cells resulting in an immune response targeting at least one of the neoantigens on the tumor cell surface.

7. The vector of claim 6, wherein the expression of each of the at least 2-400 neoantigen-encoding nucleic acid sequences is driven by the at least one promoter.

8. The vector of claim 1, wherein the 5' and 3' linkers are 2-20 amino acid residues in length; optionally wherein the 5' and 3' linkers are 5, 7 or 8 amino acid residues in length.

9. The vector of any of the above claims, wherein the at least one alteration comprises a point mutation, a frameshift mutation, a non-frameshift mutation, a deletion mutation, an insertion mutation, a splice variant, a genomic rearrangement, or a proteasome-generated spliced antigen.

10. The vector of claim 1, wherein the plurality comprises at least 2, 3, 4, 5, 6, 7, 8, 9, 10, 11, 12, 13, 14, 15, 16, 17, 18, 19, 20 or up to 400 nucleic acid sequences.

11. The vector of claim 1, wherein each MHC class I neoantigen-encoding nucleic acid sequence encodes a polypeptide sequence between 8 and 35 amino acids in length, optionally 9-17, 9-25, 8, 9, 10, 11, 12, 13, 14, 15, 16, 17, 18, 19, 20, 21, 22, 23, 24, 25, 26, 27, 28, 29, 30, 31, 32, 33, 34 or 35 amino acids in length.

12. The vector of claim 1, wherein the at least one MHC class II antigen-encoding nucleic acid sequence comprises at least one universal MHC class II antigen-encoding nucleic acid sequence, optionally wherein the at least one universal sequence comprises at least one sequence from at least one of Tetanus toxoid and PADRE.

13. The vector of claim 1, wherein the at least one promoter sequence is inducible.

14. The vector of any of the above claims, wherein the neoantigen cassette further comprises at least one poly-adenylation (poly A) sequence operably linked to at least one of the sequences in the plurality, optionally wherein the polyA sequence is located 3' of the at least one sequence in the plurality.

15. The vector of claim 14, wherein the poly A sequence comprises an SV40 polyA sequence.

16. The vector of claim 1, wherein the vector is a chimpanzee adenovirus (ChAdV) 68 vector.

17. The vector of claim 1, wherein the vector comprises one or more genes or regulatory sequences obtained from the sequence of SEQ ID NO: 1, optionally wherein the one or more genes is selected from the group consisting of the chimpanzee adenovirus inverted terminal repeats (ITR), E1A, E1B, E2A, E2B, E3, E4, L1, L2, L3, L4, and L5 genes of the sequence set forth in SEQ ID NO: 1, optionally wherein the one or more genes comprises each of the chimpanzee adenovirus ITRs, E2A, E2B, L1, L2, L3, L4, and L5 genes of the sequence set forth in SEQ ID NO: 1.

18. The vector of claim 1, wherein the neoantigen cassette is inserted in the vector at a deleted chimpanzee adenovirus region that allows incorporation of the neoantigen cassette, optionally wherein the deleted chimpanzee adenovirus region is an E1 region or a E3 region.

19. The vector of claim 1, wherein the vector comprises one or more deletions between base pair number 577 and 3403 of the sequence shown in SEQ ID NO:1 or between base pair 456 and 3014 of the sequence shown in SEQ ID NO: 1, and optionally wherein the vector further comprises one or more deletions between base pair 27,125 and 31,825 of the sequence shown in SEQ ID NO:1 or between base pair 27,816 and 31,333 of the sequence set forth in SEQ ID NO:1.

20. The vector of claim 1, wherein the at least two MHC class I neoantigen-encoding nucleic acid sequences are selected by performing the steps of:

(1) obtaining at least one of exome, transcriptome, or whole genome tumor nucleotide sequencing data from the tumor, wherein the tumor nucleotide sequencing data is used to obtain data representing peptide sequences of each of a set of neoantigens;
(2) inputting the peptide sequence of each neoantigen into a presentation model to generate a set of numerical likelihoods that each of the neoantigens is presented by one or more of the MHC alleles on the tumor cell surface of the tumor, the set of numerical likelihoods having been identified at least based on received mass spectrometry data, optionally wherein the presentation model represents dependence between: presence of a pair of a particular one of the MHC alleles and a particular amino acid at a particular position of a peptide sequence; and likelihood of presentation on the tumor cell surface, by the particular one of the MHC alleles of the pair, of such a peptide sequence comprising the particular amino acid at the particular position; and
(3) selecting a subset of the set of neoantigens based on the set of numerical likelihoods to generate a set of selected neoantigens which are used to generate the at least two MHC class I neoantigen-encoding nucleic acid sequences, optionally wherein a number of the set of selected neoantigens is 2-20; and

optionally wherein selecting the set of selected neoantigens comprises selecting neoantigens selected from the group consisting of: (a) neoantigens that have an increased likelihood of being presented on the tumor cell surface relative to unselected neoantigens based on the presentation model, (b) neoantigens that have an increased likelihood of being capable of inducing a tumor-specific immune response in the subject relative to unselected neoantigens based on the presentation model, (c) neoantigens that have an increased likelihood of being capable of being presented to naïve T cells by professional antigen presenting cells (APCs) relative to unselected neoantigens based on the presentation model, optionally wherein the APC is a dendritic cell (DC), (d) neoantigens that have a decreased likelihood of being subject to inhibition via central or peripheral tolerance relative to unselected neoantigens based on the presentation model, and (e) neoantigens that have a decreased likelihood of being capable of inducing an autoimmune response to normal tissue in the subject relative to unselected neoantigens based on the presentation model.

21. The vector of claim 1, wherein the neoantigen cassette comprises junctional epitope sequences formed by adjacent sequences in the neoantigen cassette, wherein at least one or each junctional epitope sequence has an affinity of greater than 500 nM for MHC from the subject, optionally wherein each junctional epitope sequence is non-self.

22. A pharmaceutical composition comprising the vector of claim 1 and a pharmaceutically acceptable carrier.

23. The vector of claim 1 or the pharmaceutical composition of claim 22, for use in a method for treating cancer in a subject, the method comprising administering the vector or pharmaceutical composition to the subject.

24. The vector or pharmaceutical composition for use of claim 23, wherein the method further comprises administering to the subject an immune modulator, optionally wherein the immune modulator is administered before, concurrently with, or after administration of the vector or pharmaceutical composition.

25. The vector or pharmaceutical composition for use of claim 24, wherein the immune modulator is an anti-CTLA4 antibody or an antigen-binding fragment thereof, an anti-PD-1 antibody or an antigen-binding fragment thereof, an anti-PD-L1 antibody or an antigen-binding fragment thereof, an anti-4-1BB antibody or an antigen-binding fragment thereof, or an anti-OX-40 antibody or an antigen-binding fragment thereof.

26. The vector or pharmaceutical composition for use of any of claims 23 to 25, wherein the vector, composition, and/or immune modulator is administered intramuscularly (IM), intradermally (ID), subcutaneously (SC), or intravenously (IV).

27. A method of manufacturing the chimpanzee adenovirus vector of claim 1, the method comprising:

obtaining a plasmid sequence comprising the neoantigen cassette;
transfecting the plasmid sequence into one or more host cells; and
isolating the chimpanzee adenovirus vector from the one or more host cells.

28. The method of manufacturing of claim 27, wherein isolating comprises:

lysing the host cell to obtain a cell lysate comprising the chimpanzee adenovirus vector; and
purifying the chimpanzee adenovirus vector from the cell lysate and optionally also from media used to culture the host cell.

**Patentansprüche**

1. Schimpansen-Adenovirus-Vektor, der eine Neoantigen-Kassette umfasst, die Neoantigen-Kassette umfassend:

(1) eine Vielzahl von Neoantigen-kodierenden Nukleinsäuresequenzen, die von einem in einem Subjekt vorhandenen Tumor stammen, die Vielzahl umfassend:

mindestens zwei tumorspezifische und subjektspezifische MHC-Klasse-I-Neoantigen-kodierende Nukleinsäuresequenzen, die jeweils umfassen:

a. eine MHC-Klasse-I-Epitop-kodierende Nukleinsäuresequenz mit mindestens einer Veränderung, die die kodierte Peptidsequenz von der entsprechenden Peptidsequenz unterscheidet, die von einer Wildtyp-Nukleinsäuresequenz kodiert wird,
b. eine 5'-Linkersequenz, wobei die 5'-Linkersequenz eine native 5'-Nukleinsäuresequenz des MHC-I-Epitops ist, und wobei die 5'-Linkersequenz ein Peptid kodiert, das mindestens 2 Aminosäuren lang ist, und
c. eine 3'-Linkersequenz, wobei die 3'-Linkersequenz eine native 3'-Nukleinsäuresequenz des MHC-I-Epitops ist, und wobei die 3'-Linkersequenz ein Peptid kodiert, das mindestens 2 Aminosäuren lang ist,

wobei mindestens eine Neoantigen-kodierende Nukleinsäuresequenz in der Vielzahl durch ihre Linkersequenz mit einer anderen Neoantigen-kodierenden Nukleinsäuresequenz in der Vielzahl verbunden ist;

(2) mindestens eine Promotorsequenz, die operativ mit mindestens einer Sequenz der Vielzahl verbunden ist,
(3) mindestens eine MHC-Klasse-II-Antigen-kodierende Nukleinsäuresequenz.

2. Vektor nach Anspruch 1, umfassend mindestens eine GPGPG-kodierende Linkersequenz (SEQ ID NO:56), wobei optional die mindestens eine GPGPG-kodierende Linkersequenz (SEQ ID NO:56) mindestens eine MHC-Klasse-II-Neoantigen-kodierende Nukleinsäuresequenz mit einer der MHC-Klasse-I-Neoantigen-kodierenden Nukleinsäuresequenzen verbindet.

3. Vektor nach Anspruch 1, wobei eine geordnete Sequenz jedes Elements der Neoantigen-Kassette in einer Formel von 5' bis 3' beschrieben ist, umfassend:

$$P_a\text{-}(L5_b\text{-}N_c\text{-}L3_d)_X\text{-}(G5_e\text{-}U_f)_Y\text{-}G3_g\text{-}A_h$$

wobei P die mindestens eine Promotorsequenz umfasst, die operativ mit mindestens einer Sequenz der Vielzahl verbunden ist, wobei a = 1 ist,
N eine der MHC-Klasse-I-Epitop-kodierenden Nukleinsäuresequenzen mit mindestens einer Veränderung umfasst, die die kodierte Peptidsequenz von der entsprechenden Peptidsequenz unterscheidet, die von der Wildtyp-Nukleinsäuresequenz kodiert wird, wobei c = 1 ist,
L5 die 5'-Linkersequenz umfasst, wobei b = 1 ist,
L3 die 3'-Linkersequenz umfasst, wobei d = 1 ist,
G5 eine GPGPG-kodierende Linkersequenz (SEQ ID NO:56) umfasst, wobei e = 0 oder 1 ist,
G3 eine GPGPG-kodierende Linkersequenz (SEQ ID NO:56) umfasst, wobei g = 0 oder 1 ist,
U eine der mindestens einen MHC-Klasse-II-Antigenkodierenden Nukleinsäuresequenzen umfasst, wobei f = 1 ist,
A mindestens eine Polyadenylierungssequenz umfasst, wobei h = 0 oder 1 ist,
X = 2 bis 400 ist, wobei für jedes X das entsprechende $N_c$ eine unterschiedliche MHC-Klasse-I-Epitop-kodierende Nukleinsäuresequenz ist, und
Y = 1 bis 2 ist, wobei für jedes Y das entsprechende $U_f$ eine unterschiedliche MHC-Klasse-II-Antigen-kodierende Nukleinsäuresequenz ist.

**4.** Vektor nach Anspruch 3, wobei

b = 1, d = 1, e = 1, g = 1, h = 1, X = 20, Y = 2,
P eine CMV-Promotorsequenz ist,
jedes N ein MHC-Klasse-I-Epitop mit einer Länge von 7 bis 15 Aminosäuren kodiert,
L5 die native 5'-Nukleinsäuresequenz des MHC-I-Epitops ist, wobei die 5'-Linkersequenz ein Peptid kodiert, das mindestens 5 Aminosäuren lang ist,
L3 die native 3'-Nukleinsäuresequenz des MHC-I-Epitops ist, wobei die 3'-Linkersequenz ein Peptid kodiert, das mindestens 5 Aminosäuren lang ist,
U jeweils eine PADRE-MHC-Klasse-II-Sequenz und eine Tetanustoxoid-MHC-Klasse-II-Sequenz ist,
der Schimpansen-Adenovirus-Vektor eine modifizierte ChAdV68-Sequenz umfasst, die die Sequenz von SEQ ID NO:1 mit einer E1-Deletion von Nukleotid 577 bis Nukleotid 3403 und einer E3-Deletion von Nukleotid 27.125 bis Nukleotid 31.825 umfasst, und die Neoantigen-Kassette innerhalb der E1-Deletion inseriert wird, und jede der MHC-Klasse-I-Neoantigen-kodierenden Nukleinsäuresequenzen ein Polypeptid mit einer Länge von 25 Aminosäuren kodiert.

**5.** Vektor nach Anspruch 1, wobei mindestens eine der Neoantigen-kodierenden Nukleinsäuresequenzen in der Vielzahl eine Polypeptidsequenz oder einen Teil davon kodiert, die bzw. der von MHC-Klasse I auf der Tumorzelloberfläche präsentiert wird.

**6.** Vektor nach Anspruch 5, wobei die Vielzahl mindestens 2 bis 400 Nukleinsäuresequenzen umfasst und (1) wobei mindestens zwei der Neoantigen-kodierenden Nukleinsäuresequenzen in der Vielzahl Polypeptidsequenzen oder Teile davon kodieren, die von MHC Klasse I auf der Tumorzelloberfläche präsentiert werden, oder (2) wenn sie dem Subjekt verabreicht und translatiert werden, mindestens eines der Neoantigene auf antigenpräsentierenden Zellen präsentiert wird, was zu einer Immunantwort führt, die auf mindestens eines der Neoantigene auf der Tumorzelloberfläche abzielt.

**7.** Vektor nach Anspruch 6, wobei die Expression jeder der mindestens 2 bis 400 Neoantigen-kodierenden Nukleinsäuresequenzen durch den mindestens einen Promotor gesteuert wird.

**8.** Vektor nach Anspruch 1, wobei die 5'- und 3'-Linker eine Länge von 2 bis 20 Aminosäureresten aufweisen; wobei optional die 5'- und 3'-Linker eine Länge von 5, 7 oder 8 Aminosäureresten aufweisen.

**9.** Vektor nach einem der vorstehenden Ansprüche, wobei die mindestens eine Veränderung eine Punktmutation, eine Frameshift-Mutation, eine Nicht-Frameshift-Mutation, eine Deletionsmutation, eine Insertionsmutation, eine Spleißvariante, eine genomisches Rearrangement oder ein durch Proteasom erzeugtes gespleißtes Antigen umfasst.

**10.** Vektor nach Anspruch 1, wobei die Vielzahl mindestens 2, 3, 4, 5, 6, 7, 8, 9, 10, 11, 12, 13, 14, 15, 16, 17, 18, 19, 20 oder bis zu 400 Nukleinsäuresequenzen umfasst.

**11.** Vektor nach Anspruch 1, wobei jede MHC-Klasse-I-Neoantigen-kodierende Nukleinsäuresequenz eine Polypeptidsequenz mit einer Länge zwischen 8 und 35 Aminosäuren, optional einer Länge von 9 bis 17, 9 bis 25, 8, 9, 10, 11, 12, 13, 14, 15, 16, 17, 18, 19, 20, 21, 22, 23, 24, 25, 26, 27, 28, 29, 30, 31, 32, 33, 34 oder 35 Aminosäuren kodiert.

**12.** Vektor nach Anspruch 1, wobei die mindestens eine MHC-Klasse-II-Antigen-kodierende Nukleinsäuresequenz mindestens eine universelle MHC-Klasse-II-Antigen-kodierende Nukleinsäuresequenz umfasst, wobei optional die mindestens eine universelle Sequenz mindestens eine Sequenz aus mindestens einem von Tetanustoxoid und PADRE umfasst.

**13.** Vektor nach Anspruch 1, wobei die mindestens eine Promotorsequenz induzierbar ist.

**14.** Vektor nach einem der vorstehenden Ansprüche, wobei die Neoantigen-Kassette ferner mindestens eine Polyadenylierungssequenz (polyA-Sequenz) umfasst, die operativ mit mindestens einer der Sequenzen in der Vielzahl verbunden ist, wobei die polyA-Sequenz optional 3' der mindestens einen Sequenz in der Vielzahl angeordnet ist.

**15.** Vektor nach Anspruch 14, wobei die polyA-Sequenz eine SV40-polyA-Sequenz umfasst.

**16.** Vektor nach Anspruch 1, wobei der Vektor ein Schimpansen-Adenovirus-68-Vektor (ChAdV-68-Vektor) ist.

**EP 3 544 607 B1**

**17.** Vektor nach Anspruch 1, wobei der Vektor ein oder mehrere Gene oder regulatorische Sequenzen umfasst, die aus der Sequenz SEQ ID NO: 1 erhalten wurden, wobei optional das eine oder die mehreren Gene aus der Gruppe ausgewählt sind, die aus den invertierten terminalen Wiederholungen (Inverted Terminal Repeats, ITR) des Schimpansen-Adenovirus, E1A-, E1B-, E2A-, E2B-, E3-, E4-, L1-, L2-, L3-, L4- und L5-Genen des in SEQ ID NO: 1 aufgeführten Sequenzsatzes ausgewählt sind, wobei optional das eine oder die mehreren Gene jedes der Schimpansen-Adenovirus-ITRs, E2A-, E2B-, L1-, L2-, L3-, L4- und L5-Gene des in SEQ ID NO: 1 aufgeführten Sequenzsatzes umfassen.

**18.** Vektor nach Anspruch 1, wobei die Neoantigen-Kassette in den Vektor an einer deletierten Schimpansen-Adenovirus-Region inseriert ist, die die Einbindung der Neoantigen-Kassette ermöglicht, wobei optional die deletierte Schimpansen-Adenovirus-Region eine E1-Region oder eine E3-Region ist.

**19.** Vektor nach Anspruch 1, wobei der Vektor eine oder mehrere Deletionen zwischen Basenpaar Nummer 577 und 3403 der in SEQ ID NO:1 gezeigten Sequenz oder zwischen Basenpaar 456 und 3014 der in SEQ ID NO:1 gezeigten Sequenz umfasst, und wobei der Vektor optional ferner eine oder mehrere Deletionen zwischen den Basenpaaren 27.125 und 31.825 der in SEQ ID NO:1 gezeigten Sequenz oder zwischen den Basenpaaren 27.816 und 31.333 der in SEQ ID NO:1 aufgeführten Sequenz umfasst.

**20.** Vektor nach Anspruch 1, wobei die mindestens zwei MHC-Klasse-I-Neoantigen-kodierenden

Nukleinsäuresequenzen ausgewählt werden, indem die folgenden Schritte durchgeführt werden:

(1) Erhalten mindestens eines von Exom-, Transkriptom- oder Ganzgenom-Tumor-Nukleotidsequenzierungsdaten aus dem Tumor, wobei die Tumor-Nukleotidsequenzierungsdaten verwendet werden, um Daten zu gewinnen, die die Peptidsequenzen von jedem eines Satzes von Neoantigenen repräsentieren;
(2) Eingeben der Peptidsequenz jedes Neoantigens in ein Präsentationsmodell, um einen Satz numerischer Wahrscheinlichkeiten zu erzeugen, dass jedes der Neoantigene durch eines oder mehrere der MHC-Allele auf der Tumorzelloberfläche des Tumors präsentiert wird, wobei der Satz numerischer Wahrscheinlichkeiten mindestens auf der Grundlage empfangener Massenspektrometriedaten identifiziert wurde, wobei das Präsentationsmodell optional die Abhängigkeit zwischen folgenden Faktoren darstellt: dem Vorhandensein eines Paares eines bestimmten MHC-Allels und einer bestimmten Aminosäure an einer bestimmten Position einer Peptidsequenz; und der Wahrscheinlichkeit der Präsentation einer solchen Peptidsequenz, die die bestimmte Aminosäure an der bestimmten Position umfasst, auf der Tumorzelloberfläche durch das bestimmte MHC-Allel des Paares; und
(3) Auswählen eines Teilsatzes des Satzes von Neoantigenen auf der Grundlage des Satzes numerischer Wahrscheinlichkeiten, um einen Satz ausgewählter Neoantigene zu erzeugen, die verwendet werden, um die mindestens zwei MHC-Klasse-I-Neoantigen-kodierenden Nukleinsäuresequenzen zu erzeugen, wobei optional die Anzahl des Satzes ausgewählter Neoantigene 2 bis 20 beträgt; und

wobei optional das Auswählen des Satzes ausgewählter Neoantigene das Auswählen von Neoantigenen umfasst, die aus der Gruppe ausgewählt sind, die besteht aus: (a) Neoantigenen, die auf der Grundlage des Präsentationsmodells im Vergleich zu nicht ausgewählten Neoantigenen eine erhöhte Wahrscheinlichkeit aufweisen, auf der Tumorzelloberfläche präsentiert zu werden, (b) Neoantigene, die auf der Grundlage des Präsentationsmodells im Vergleich zu nicht ausgewählten Neoantigenen eine erhöhte Wahrscheinlichkeit aufweisen, eine tumorspezifische Immunantwort in dem Subjekten auszulösen, (c) Neoantigene, die auf der Grundlage des Präsentationsmodells im Vergleich zu nicht ausgewählten Neoantigenen eine erhöhte Wahrscheinlichkeit aufweisen, von professionellen antigenpräsentierenden Zellen (APCs) naiven T-Zellen präsentiert werden zu können, wobei die APC optional eine dendritische Zelle (DC) ist, (d) Neoantigene, die auf der Grundlage des Präsentationsmodells im Vergleich zu nicht ausgewählten Neoantigenen eine geringere Wahrscheinlichkeit aufweisen, einer Hemmung durch zentrale oder periphere Toleranz zu unterliegen, und (e) Neoantigene, die auf der Grundlage des Präsentationsmodells im Vergleich zu nicht ausgewählten Neoantigenen eine geringere Wahrscheinlichkeit aufweisen, eine Autoimmunreaktion auf normales Gewebe in dem Subjekt auszulösen.

**21.** Vektor nach Anspruch 1, wobei die Neoantigen-Kassette junktionale Epitopsequenzen umfasst, die durch benachbarte Sequenzen in der Neoantigen-Kassette gebildet werden, wobei mindestens eine oder jede junktionale Epitopsequenz eine Affinität von mehr als 500 nM für MHC aus dem Subjekt aufweist, wobei optional jede junktionale Epitopsequenz Nicht-Selbst ist.

22. Pharmazeutische Zusammensetzung, umfassend den Vektor nach Anspruch 1 und einen pharmazeutisch akzeptablen Träger.

23. Vektor nach Anspruch 1 oder die pharmazeutische Zusammensetzung nach Anspruch 22 zur Verwendung in einem Verfahren zur Behandlung von Krebs bei einem Subjekt, wobei das Verfahren die Verabreichung des Vektors oder der pharmazeutischen Zusammensetzung an das Subjekt umfasst.

24. Vektor oder die pharmazeutische Zusammensetzung zur Verwendung nach Anspruch 23, wobei das Verfahren ferner das Verabreichen eines Immunmodulators an das Subjekt umfasst, wobei der Immunmodulator optional vor, gleichzeitig mit oder nach der Verabreichung des Vektors oder der pharmazeutischen Zusammensetzung verabreicht wird.

25. Vektor oder die pharmazeutische Zusammensetzung zur Verwendung nach Anspruch 24, wobei der Immunmodulator ein Anti-CTLA4-Antikörper oder ein Antigen-bindendes Fragment davon, ein Anti-PD-1-Antikörper oder ein Antigen-bindendes Fragment davon, ein Anti-PD-L1-Antikörper oder ein Antigen-bindendes Fragment davon, ein Anti-4-1BB-Antikörper oder ein Antigen-bindendes Fragment davon oder ein Anti-OX-40-Antikörper oder ein Antigen-bindendes Fragment davon ist.

26. Vektor oder die pharmazeutische Zusammensetzung zur Verwendung nach einem der Ansprüche 23 bis 25, wobei der Vektor, die Zusammensetzung und/oder der Immunmodulator intramuskulär (IM), intradermal (ID), subkutan (SC) oder intravenös (IV) verabreicht wird.

27. Verfahren zum Herstellen des Schimpansen-Adenovirus-Vektors nach Anspruch 1, das Verfahren umfassend:

Erhalten einer Plasmidsequenz, die die Neoantigen-Kassette umfasst;
Transfizieren der Plasmidsequenz in eine oder mehrere Wirtszellen; und
Isolieren des Schimpansen-Adenovirus-Vektors aus der einen oder den mehreren Wirtszellen.

28. Verfahren zum Herstellen nach Anspruch 27, das Isolieren umfassend:

Lysieren der Wirtszelle, um ein Zelllysat zu erhalten, das den Schimpansen-Adenovirus-Vektor umfasst; und
Reinigen des Schimpansen-Adenovirus-Vektors aus dem Zelllysat und optional auch aus den zur Kultivierung der Wirtszelle verwendeten Medien.

## Revendications

1. Vecteur d'adénovirus de chimpanzé comprenant une cassette de néo-antigènes, la cassette de néo-antigènes comprenant :

(1) une pluralité de séquences d'acide nucléique codant pour un néo-antigène dérivées d'une tumeur présente chez un sujet, la pluralité comprenant :

au moins deux séquences d'acide nucléique codant pour un néo-antigène du CMH de classe I spécifique à la tumeur et spécifique au sujet, chacune comprenant :

a. une séquence d'acide nucléique codant pour un épitope du CMH de classe I avec au moins une altération qui rend la séquence peptidique codée distincte de la séquence peptidique correspondante codée par une séquence d'acide nucléique de type sauvage,
b. une séquence de liaison 5', dans lequel la séquence de liaison 5' est une séquence d'acide nucléique 5' native de l'épitope du CMH I, et dans lequel la séquence de liaison 5' code pour un peptide d'au moins 2 acides aminés de longueur et
c. une séquence de liaison 3', dans lequel la séquence de liaison 3' est une séquence d'acide nucléique 3' native de l'épitope du CMH I, et dans lequel la séquence de liaison 3' code pour un peptide d'au moins 2 acides aminés de longueur,

dans lequel au moins une séquence d'acide nucléique codant pour un néo-antigène dans la pluralité est liée à une séquence d'acide nucléique codant pour un néo-antigène distincte dans la pluralité par sa séquence de liaison ;

(2) au moins une séquence promotrice liée en fonctionnement à au moins une séquence de la pluralité,
(3) au moins une séquence d'acide nucléique codant pour un antigène du CMH de classe II.

**2.** Vecteur selon la revendication 1, comprenant au moins une séquence de liaison codant pour GPGPG (SEQ ID NO : 56), éventuellement dans lequel l'au moins une séquence de liaison codant pour GPGPG (SEQ ID NO : 56) lie au moins une séquence d'acide nucléique codant pour un néo-antigène du CMH de classe II à l'une des séquences d'acide nucléique codant pour un néo-antigène du CMH de classe I.

**3.** Vecteur selon la revendication 1, dans lequel une séquence ordonnée de chaque élément de la cassette de néo-antigènes est décrite dans une formule, de 5' à 3', comprenant :

$$P_a\text{-}(L5_b\text{-}N_c\text{-}L3_d)_X\text{-}(G5_e\text{-}U_f)_Y\text{-}G3_g\text{-}A_h$$

dans lequel P comprend l'au moins une séquence promotrice liée en fonctionnement à au moins une séquence de la pluralité, où $a = 1$,
N comprend l'une des séquences d'acide nucléique codant pour l'épitope I du CMH avec au moins une altération qui rend la séquence peptidique codée distincte de la séquence peptidique correspondante codée par la séquence d'acide nucléique de type sauvage, où $c = 1$,
L5 comprend la séquence de liaison 5', où $b = 1$,
L3 comprend la séquence de liaison 3', où $d = 1$,
G5 comprend une séquence de liaison codant pour GPGPG (SEQ ID NO :56), où $e = 0$ ou 1,
G3 comprend une séquence de liaison codant pour GPGPG (SEQ ID NO :56), où $g = 0$ ou 1,
U comprend l'une de l'au moins une séquence d'acide nucléique codant pour un antigène du CMH de classe II, où $f = 1$,
A comprend au moins une séquence de polyadénylation, où $h = 0$ ou 1,
$X = 2$ à 400, où pour chaque X le $N_c$ correspondant est une séquence d'acide nucléique codant pour un épitope distinct du CMH de classe I, et
$Y = 1$ à 2, où pour chaque Y le $U_f$ correspondant est une séquence d'acide nucléique codant pour un antigène CMH de classe II distinct.

**4.** Vecteur selon la revendication 3, dans lequel

$b = 1$, $d = 1$, $e = 1$, $g = 1$, $h = 1$, $X = 20$, $Y = 2$,
P est une séquence promotrice du CMV,
chaque N code un épitope de classe I du CMH d'une longueur de 7 à 15 acides aminés,
L5 est la séquence d'acide nucléique 5' native de l'épitope du CMH I dans lequel la séquence de liaison 5' code pour un peptide d'au moins 5 acides aminés de longueur,
L3 est la séquence d'acide nucléique 3' native de l'épitope du CMH I dans lequel la séquence de liaison 3' code pour un peptide d'au moins 5 acides aminés de longueur,
U représente une séquence du CMH de classe II du PADRE et une séquence du CMH de classe II de l'anatoxine tétanique,
le vecteur adénovirus de chimpanzé comprend une séquence ChAdV68 modifiée comprenant la séquence de SEQ ID NO : 1 ayant une délétion E1 du nucléotide 577 au nucléotide 3403 et une délétion E3 du nucléotide 27 125 au nucléotide 31 825 et la cassette de néo-antigènes est insérée dans la délétion E1, et
chacune des séquences d'acide nucléique codant pour le néo-antigène du CMH de classe I code pour un polypeptide d'une longueur de 25 acides aminés.

**5.** Vecteur selon la revendication 1, dans lequel au moins l'une des séquences d'acide nucléique codant pour un néo-antigène dans la pluralité code pour une séquence polypeptidique ou une partie de celle-ci qui est présentée par le CMH de classe I sur la surface de la cellule tumorale.

**6.** Vecteur selon la revendication 5, dans lequel la pluralité comprend au moins 2 à 400 séquences d'acide nucléique et (1) dans lequel au moins deux des séquences d'acide nucléique codant pour un néo-antigène dans la pluralité codent pour des séquences polypeptidiques ou des parties de celles-ci qui sont présentées par le CMH de classe I sur la surface de la cellule tumorale, ou (2) lorsqu'il est administré au sujet et traduit, au moins l'un des néo-antigènes est présenté sur des cellules présentant des antigènes, ce qui entraîne une réponse immunitaire ciblant au moins l'un des néo-antigènes sur la surface de la cellule tumorale.

**7.** Vecteur selon la revendication 6, dans lequel l'expression de chacune de l'au moins 2 à 400 séquences d'acide nucléique codant pour un néo-antigène est pilotée par l'au moins un promoteur.

**8.** Vecteur selon la revendication 1, dans lequel les liaisons 5' et 3' ont une longueur de 2 à 20 résidus d'acides aminés ; éventuellement dans lequel les liaisons 5' et 3' ont une longueur de 5, 7 ou 8 résidus d'acides aminés.

**9.** Vecteur selon l'une quelconque des revendications précédentes, dans lequel l'au moins une altération comprend une mutation ponctuelle, une mutation par décalage de cadre, une mutation sans décalage de cadre, une mutation par délétion, une mutation par insertion, une variante d'épissage, un réarrangement génomique, ou un antigène épissé généré par le protéasome.

**10.** Vecteur selon la revendication 1, dans lequel la pluralité comprend au moins 2, 3, 4, 5, 6, 7, 8, 9, 10, 11, 12, 13, 14, 15, 16, 17, 18, 19, 20 ou jusqu'à 400 séquences d'acide nucléique.

**11.** Vecteur selon la revendication 1, dans lequel chaque séquence d'acide nucléique codant pour un néo-antigène du CMH de classe I code pour une séquence polypeptidique d'une longueur comprise entre 8 et 35 acides aminés, éventuellement de 9 à 17, 9 à 25, 8, 9, 10, 11, 12, 13, 14, 15, 16, 17, 18, 19, 20, 21, 22, 23, 24, 25, 26, 27, 28, 29, 30, 31, 32, 33, 34 ou 35 acides aminés.

**12.** Vecteur selon la revendication 1, dans lequel l'au moins une séquence d'acide nucléique codant pour l'antigène de classe II du CMH comprend au moins une séquence d'acide nucléique codant pour l'antigène de classe II du CMH universelle, éventuellement dans lequel l'au moins une séquence universelle comprend au moins une séquence provenant d'au moins l'un des anatoxines tétaniques et PADRE.

**13.** Vecteur selon la revendication 1, dans lequel l'au moins une séquence promotrice est inductible.

**14.** Vecteur selon l'une quelconque des revendications précédentes, dans lequel la cassette de néo-antigènes comprend également au moins une séquence de polyadénylation (polyA) liée de manière fonctionnelle à au moins l'une des séquences de la pluralité, éventuellement dans lequel la séquence polyA est située en 3' de l'au moins une séquence de la pluralité.

**15.** Vecteur selon la revendication 14, dans lequel la séquence polyA comprend une séquence polyA SV40.

**16.** Vecteur selon la revendication 1, dans lequel le vecteur est un vecteur d'adénovirus de chimpanzé (ChAdV) 68.

**17.** Vecteur selon la revendication 1, dans lequel le vecteur comprend un ou plusieurs gènes ou séquences régulatrices obtenus à partir de la séquence SEQ ID NO : 1, éventuellement dans lequel les un ou plusieurs gènes sont sélectionnés dans le groupe constitué des gènes de répétitions terminales inversées (ITR) de l'adénovirus du chimpanzé, E1A, E1B, E2A, E2B, E3, E4, L1, L2, L3, L4 et L5 de la séquence énoncée dans SEQ ID NO : 1, éventuellement dans lequel les un ou plusieurs gènes comprennent chacun des gènes ITR de l'adénovirus de chimpanzé, E2A, E2B, L1, L2, L3, L4 et L5 de la séquence énoncée dans SEQ ID NO : 1.

**18.** Vecteur selon la revendication 1, dans lequel la cassette de néo-antigènes est insérée dans le vecteur au niveau d'une région d'adénovirus de chimpanzé supprimée qui permet l'incorporation de la cassette de néo-antigènes, éventuellement dans lequel la région d'adénovirus de chimpanzé supprimée est une région E1 ou une région E3.

**19.** Vecteur selon la revendication 1, dans lequel le vecteur comprend une ou plusieurs délétions entre la paire de bases numéro 577 et 3403 de la séquence représentée dans SEQ ID NO : 1 ou entre la paire de bases 456 et 3014 de la séquence représentée dans SEQ ID NO : 1, et éventuellement dans lequel le vecteur comprend également une ou plusieurs délétions entre la paire de bases 27 125 et 31 825 de la séquence représentée dans SEQ ID NO : 1 ou entre la paire de bases 27 816 et 31 333 de la séquence énoncée dans SEQ ID NO : 1.

**20.** Vecteur selon la revendication 1, dans lequel les au moins deux séquences d'acide nucléique codant pour un néo-antigène du CMH de classe I sont sélectionnées en réalisant les étapes :

(1) d'obtention d'au moins une des données de séquençage des nucléotides tumoraux de l'exome, du transcriptome, ou du génome entier de la tumeur, dans lequel les données de séquençage des nucléotides tumoraux sont utilisées pour obtenir des données représentant les séquences peptidiques de chacun d'un ensemble de néo-antigènes ;

(2) d'introduction de la séquence peptidique de chaque néo-antigène dans un modèle de présentation afin de générer un ensemble de probabilités numériques que chacun des néo-antigènes soit présenté par un ou plusieurs des allèles du CMH à la surface de la cellule tumorale, l'ensemble de probabilités numériques ayant été identifié au moins sur la base de données de spectrométrie de masse reçues, éventuellement dans lequel le modèle de présentation représente une dépendance entre : la présence d'une paire d'un allèle particulier des allèles du CMH et d'un acide aminé particulier à une position particulière d'une séquence peptidique ; et la probabilité de présentation à la surface de la cellule tumorale, par l'allèle particulier des allèles du CMH de la paire, d'une telle séquence peptidique comprenant l'acide aminé particulier à la position particulière ; et

(3) de sélection d'un sous-ensemble de l'ensemble de néo-antigènes sur la base de l'ensemble de probabilités numériques pour générer un ensemble de néo-antigènes sélectionnés qui sont utilisés pour générer les au moins deux séquences d'acide nucléique codant pour les néo-antigènes de classe I du CMH, éventuellement dans lequel un nombre de l'ensemble de néo-antigènes sélectionnés est compris entre 2 et 20 ; et

éventuellement dans lequel la sélection de l'ensemble de néo-antigènes sélectionnés comprend la sélection de néo-antigènes sélectionnés dans le groupe constitué : (a) des néo-antigènes qui ont une probabilité accrue d'être présentés à la surface des cellules tumorales par rapport aux néo-antigènes non sélectionnés sur la base du modèle de présentation, (b) des néo-antigènes qui ont une probabilité accrue d'être capables d'induire une réponse immunitaire spécifique à la tumeur chez le sujet par rapport aux néo-antigènes non sélectionnés sur la base du modèle de présentation, (c) des néo-antigènes qui ont une probabilité accrue d'être capables d'être présentés aux lymphocytes T naïfs par des cellules présentant des antigènes professionnelles (APC) par rapport aux néo-antigènes non sélectionnés sur la base du modèle de présentation, éventuellement dans lequel l'APC est une cellule dendritique (DC), (d) des néo-antigènes qui ont une probabilité réduite d'être sujets à une inhibition par le biais d'une tolérance centrale ou périphérique par rapport aux néo-antigènes non sélectionnés sur la base du modèle de présentation, et (e) des néo-antigènes qui ont une probabilité réduite d'être capables d'induire une réponse auto-immune à un tissu normal chez le sujet par rapport aux néo-antigènes non sélectionnés sur la base du modèle de présentation.

21. Vecteur selon la revendication 1, dans lequel la cassette de néo-antigènes comprend des séquences d'épitopes jonctionnelles formées par des séquences adjacentes dans la cassette de néo-antigènes, dans lequel au moins une ou chaque séquence d'épitopes jonctionnelle a une affinité supérieure à 500 nM pour le CMH du sujet, éventuellement dans lequel chaque séquence d'épitopes jonctionnelle est non-soi.

22. Composition pharmaceutique comprenant le vecteur selon la revendication 1 et un support pharmaceutiquement acceptable.

23. Vecteur selon la revendication 1 ou composition pharmaceutique selon la revendication 22, à utiliser dans un procédé de traitement du cancer chez un sujet, le procédé comprenant l'administration du vecteur ou de la composition pharmaceutique au sujet.

24. Vecteur ou composition pharmaceutique à utiliser selon la revendication 23, dans lequel le procédé comprend également l'administration au sujet d'un modulateur immunitaire, éventuellement dans lequel le modulateur immunitaire est administré avant, simultanément, ou après l'administration du vecteur ou de la composition pharmaceutique.

25. Vecteur ou composition pharmaceutique à utiliser selon la revendication 24, dans lequel le modulateur immunitaire est un anticorps anti-CTLA4 ou un fragment de liaison à l'antigène de celui-ci, un anticorps anti-PD-1 ou un fragment de liaison à l'antigène de celui-ci, un anticorps anti-PD-L1 ou un fragment de liaison à l'antigène de celui-ci, un anticorps anti-4-1BB ou un fragment de liaison à l'antigène de celui-ci, ou un anticorps anti-OX-40 ou un fragment de liaison à l'antigène de celui-ci.

26. Vecteur ou composition pharmaceutique à utiliser selon l'une quelconque des revendications 23 à 25, dans lequel le vecteur, la composition, et/ou le modulateur immunitaire sont administrés par voie intramusculaire (IM), intradermique (ID), sous-cutanée (SC), ou intraveineuse (IV).

27. Procédé de fabrication du vecteur d'adénovirus de chimpanzé selon la revendication 1, le procédé comprenant :

l'obtention d'une séquence plasmidique comprenant la cassette de néo-antigènes ;
la transfection de la séquence plasmidique dans une ou plusieurs cellules hôtes ; et
l'isolement du vecteur d'adénovirus de chimpanzé à partir des une ou plusieurs cellules hôtes.

28. Procédé de fabrication selon la revendication 27, dans lequel l'isolement comprend :

la lyse de la cellule hôte pour obtenir un lysat cellulaire comprenant le vecteur adénovirus de chimpanzé ; et
la purification du vecteur d'adénovirus de chimpanzé à partir du lysat cellulaire et éventuellement également à partir du milieu utilisé pour cultiver la cellule hôte.

# Current clinical approaches to neoantigen identification

**Epitope generation process**

▓▓▓ Typically addressed in neoantigen studies ▓▓▓ Not currently addressed

**Common prediction pipeline steps**

- Epitope generation process is typically modeled incompletely
- Binding generally considered most selective step and is included, but aggregate impact of other steps may be large
- Using expression and binding filters alone has potential for high false positive rate

**FIG 1A**

# Most recent literature suggests <5% of predicted bound peptides can be found presented on tumor cells

| NetMHCcon IC50≤500nM | MS-based identification | |
| --- | --- | --- |
| | HLAp | Not detected |
| Binders | 1,579 | 47,777 |
| Non-binders | 153 | 1,140,967 |

3%

| NetMHCcon IC50≤50nM | MS-based identification | |
| --- | --- | --- |
| | HLAp | Not detected |
| Binders | 1,133 | 14,059 |
| Non-binders | 599 | 1,174,685 |

- **Only 7/170 (4%)** predicted neo-epitopes were eluted and detected by mass-spec
- Possibly due to sensitivity limits of particular mass-spec detection approach

Yadav, Nature Vol 515 17 November 2014

Bassani-Sternberg M, Mol Cell Proteomics. 2015;14(3):658-73

**FIG 1B**

FIG 1C

# Binding prediction necessary but not sufficient

Binding affinity prediction vs. mass-spec peptide detection in JY (EBV)-immortalized LCL

Max of affinity predictions for HLA-A-0201 and HLA-B-0702, restricted to genes in whole proteome

Data from Bassani-Sternberg M, Mol Cell Proteomics. 2015; Gritstone analysis

**FIG 1D**

**FIG 1E**

FIG 1F

Positive Predictive Value (at Stringent Thresholds)

FIG 1G

**FIG 2A**

**FIG 2B**

**FIG 2C**

Single-Allele Cell Lines

Peptide Isolation

HLA-A*01:01

YEMFNDKSF

Multiple-Allele Cell Lines

Peptide Isolation

HLA-A*01:01
HLA-A*02:01
HLA-B*07:02
HLA-B*08:01
HLA-C*01:03
HLA-C*01:04

YEMFNDKSF
HROEIFSHDFJ
FJIEJFOESS
NEIOREIREI
JFKSIFEMMSJDSSU
KNFLENFIESOFI

EP 3 544 607 B1

**FIG 3**

Presentation Identification System
160

Data Management Module
312

Encoding Module
314

Training Module
316

Prediction Module
320

Cassette Design Module
324

Training Data Store
170

Presentation Models
175

Training Data
170A

Allele-Dependent ($x^i$)  |  Allele-Independent ($w^i$)

| Peptide Sequence ($p^i$) | Affinity ($b^i$-nM) | Stability ($s^i$-h) | Allele ($a^i$) | C-Flanking Sequence ($c^i$) | mRNA Q. ($m^i$-FPKM) | Label ($y^i$) |
|---|---|---|---|---|---|---|
| QCEIOWARE | 1000 | 1 | HLA-C*01:03 | FJELFISBOSJFIE | $10^2$ | Not Presented |
| FIEUHFWI | 1500 | 15 | HLA-C*01:03 | FEGRKUOOI | $10^{-3}$ | Presented |
| FEWRHRJTRUJR | 650 | 20 | HLA-C*01:03 | PJFIOEJOIJGEIO | $10^1$ | Presented |
| QIEJOEIJE | 500 | 1 | HLA-B*07:02 | PJFIOEJOIJGEIO | 1 | Presented |
|  | 600 | 14 | HLA-C*01:03 |  |  |  |
|  | 1200 | 7 | HLA-A*01:01 |  |  |  |

**FIG 4**

EP 3 544 607 B1

FIG 5

$$x_3{}^k \longrightarrow \boxed{\begin{array}{c} \textit{Shared Network} \\ (NN_H) \end{array}} \longrightarrow$$

$$NN_1(x_3{}^k)$$

$$NN_2(x_3{}^k)$$

$$NN_3(x_3{}^k)$$

$$NN_m(x_3{}^k)$$

**FIG 6A**

**FIG 6B**

EP 3 544 607 B1

EP 3 544 607 B1

$$x_3{}^k \longrightarrow \boxed{\begin{array}{c} \textit{HLA-A*04:01 Network} \\ \textit{(NN}_3\textit{)} \end{array}} \xrightarrow{NN_3(\textbf{\textit{x}}_3{}^k)} \boxed{f} \longrightarrow u^k$$

**FIG 7**

**FIG 8**

**FIG 9**

**FIG 10**

FIG 11

**FIG 12**

FIG 13A

FIG 13B

| Model | AUC | Log Loss | PPV at 10% Recall |
|---|---|---|---|
| Sigmoid-of-Sums | 0.9278 | $12.2 \cdot 10^{-4}$ | 0.114 |
| Sum-of-Sigmoids | 0.9723 | $5.78 \cdot 10^{-4}$ | 0.152 |
| Hyperbolic Tangent | **0.9734** | $\mathbf{5.72 \cdot 10^{-4}}$ | 0.156 |
| Second Order | 0.9727 | $5.74 \cdot 10^{-4}$ | **0.160** |

## FIG 13C

| Model | AUC | Log Loss | PPV at 10% Recall |
|---|---|---|---|
| With A2/B7 Single-Allele Data | **0.9818** | $5.40 \cdot 10^{-4}$ | **0.215** |
| Without A2/B7 Single-Allele Data | 0.9803 | $\mathbf{5.31 \cdot 10^{-4}}$ | 0.211 |

## FIG 13D

| Setup | Correlation |
|---|---|
| A2 model predicting B7 | 0.004 |
| A2 model predicting A2 | 0.294 |
| B7 model predicting B7 | 0.366 |
| B7 model predicting A2 | 0.002 |

**FIG 13E**

| Allele | P2 | P9 |
|---|---|---|
| A2 | L 89% | V 58% |
| | M 5% | L 32% |
| B7 | P 98% | L 76 % |
| | | V 8% |

**FIG 13F**

| Model | AUC | Log Loss | PPV at 10% Recall |
|---|---|---|---|
| Allele-interacting | 0.9723 | $5.78 \cdot 10^{-4}$ | 0.152 |
| Allele-noninteracting | **0.9732** | **$5.53 \cdot 10^{-4}$** | **0.188** |

**FIG 13G**

FIG 13H

**FIG 13I**

FIG 13J

**FIG 14**

FIG 15

## FIG 16A

☒ linker  ▨ class I MHC epitope

▨ class II MHC epitope

## FIG 16B

| # | HLA | Sequence | Origin |
|---|-----|----------|--------|
| 1 | A*0201 | NLVPMVATV | HCMV pp65 (495– 503) |
| 2 | A*0201 | CLGGLLTMV | EBV LMP2A(426-434) |
| 3 | A*0201 | GLCTLVAML | EBV BMLF1 (280–288) |
| 4 | A*0201 | LLFGYPVYV | HTLV-1 Tax (11-19) |
| 5 | A*0201 | GILGFVFTL | Influenza A Matrix 1 (58– 66) |
| | MHC-II | AKFVAAWTLKAAA | PADRE (artificial seq) |
| | MHC-II | QYIKANSKFIGITE | Tetanus toxoid (830–844) |

EP 3 544 607 B1

FIG 17

FIG 18

FIG 19A

| # | HLA | Sequence | Origin |
|---|-----|----------|--------|
| 1 | A*02:01 | NLVPMVATV | HCMV pp65 495-504 |
| 2 | A*02:01 | CLGGLLTMV | EBV LMP-2 426-434 |
| 3 | A*02:01 | GLCTLVAML | EBV BMLF-1 259-267 |
| 4 | A*02:01 | LLFGYPVYV | HTLV1 Tax 11-19 |
| 5 | A*02:01 | GILGFVFTL | Influenza A MP 58-66 |
| 6 | A*02:01 | DLMGYIPAV | HCV core 132-140 |
| 7 | A*02:01 | FLPSDFFPSV | HBV core antigen 18-27 |
| 8 | A*02:01 | FLLTRILTI | HBV envelope 183-191 |
| 9 | A*02:01 | WLSLLVPFV | HBV surface antigen 172-181 |
| 10 | A*02:01 | FLLSLGIHL | HBV polymerase 573-581 |
| 11 | A*02:01 | ILKEPVHGV | HIV-1 RT 476-484 |
| 12 | A*02:01 | YMLDLQPETT | HPV 16 E7 11-20 |
| 13 | A*02:01 | CINGVCWTV | HCV NS3 1073-1081 |
| 14 | A*02:01 | YLLPRRGPRL | HCV core 35-44 |
| 15 | A*02:01 | FLYALALLL | EBV LMP-2 356-364 |
| 16 | A*02:01 | AAGIGILTV | MELAN-A/MART-1 (27-35) |
| 17 | A*02:01 | SLLMWITQV | NY-ESO-1(157-165) C9V |
| 18 | A*03:01 | KLGGALQAK | CVM-IE1 |
| 19 | A*03:01 | RLRAEAQVK | EBV-EBNA-3a |
| 20 | B*44:05 | EENLLDFVRF | EBV EBNASC (281-290) |
| 21 | B*44:05 | EEYLQAFTY | Self ABCD3 protein |

# FIG 19B

FIG 20A

EP 3 544 607 B1

**NHP Epitopes**

|  | MHC | Sequence |
|---|---|---|
| 1 | Mamu*01 | CTPYDINQM |
| 4 | Mamu*01 | TTPESANL |
| 7 | Mamu*01 | CAPPGYALL |
| 10 | Mamu*01 | SGPKTNIIV |
| 14 | Mamu*01 | LSPRTLNAW |
| 18 | Mamu*01 | TVPWPNASL |

**Human Epitopes**

|  | HLA | Sequence |
|---|---|---|
| 3 | A*02:01 | GILGFVFTL |
| 6 | A*02:01 | LLFGYPVYV |
| 9 | A*02:01 | GLCTLVAML |
| 12 | A*02:01 | NLVPMVATV |
| 16 | A*02:01 | CLGGLLTMV |

**Murine MHC-I Epitopes**

|  | MHC | Sequence |
|---|---|---|
| 2 | H-2Kb | SIINFEKL |
| 5 | H-2Ld | SPSYAYHQF |
| 8 | H-2Db | EGPRNQDWL |
| 11 | H-2Kb | DWENVSPEL |
| 13 | H-2Kb | SIIVFNLL |
| 15 | H-2Db | ASMTNMELM |
| 17 | H-2Db | AQLANDVVL |
| 19 | H-2Kb | SVYDFFVWL |
| 20 | H-2Ld | MNKYAYHML |

**Universal MHC-II Epitopes**

|  | HLA | Sequence |
|---|---|---|
| 1 | MHC-II | AKFVAAWTLKAAA |
| 2 | MHC-II | QYIKANSKFIGITEL |

## FIG 20B

FIG 21A    FIG 21B    FIG 21C

FIG 22C

FIG 22B

FIG 22A

pAd68 Transfection of 293A cells in 6 well plate 1e$^6$ cells/well

—10d→ Harvest primary

—1d→ Expand to 2° infect T25 2e$^6$ cell/flask

—3d→ Expand to 3° 2e$^7$ cells /150 cm$^2$

—3d→ Production run 400 mL 293F 1e6 cells/mL

3d ⇢ Harvest ⇢ CsCl purification 2 gradients

Dialysis into ARM buffer or Sucrose based buffer

Titer by IU & VP assays

**FIG 23**

nsP1-4    26S    Antigen or Reporter

**FIG 24**

FIG 25

216

FIG 26A

FIG 26B

FIG 27A

**FIG 27B**

FIG 27C

**FIG 27D**

FIG 28A

Day 21

FIG 28B

**FIG 29**

FIG 30

FIG 31

**FIG 32**

FIG 33

Day post treatment

Percent survival

Control
PD1
chAdV/srRNA
chAdV/srRNA + PD1
srRNA/chAdV
srRNA/chAdV + PD1
srRNA
srRNA + PD1

**FIG 34**

**FIG 35**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9402888 B2
- US 4656127 A
- FR 2650840, Cohen, D.
- WO 9102087 A
- FR 9215712 W, Goelet, P.
- US 20060252077 A
- US 7283337 B
- US 8093021 B
- US 6083716 A
- WO 9513392 A
- US 5240846 A
- WO 9613597 A
- US 4235871 A

- US 4501728 A
- US 4837028 A
- US 5019369 A
- US 5580859 A
- US 5589466 A
- US 5204253 A
- US 5279833 A
- US 9106309 B
- WO 2017106638 A
- US 0110293637 B, Hacohen, N. & Wu, C. J.-Y.
- US 0120208706 A, Downing, S. R.


**Non-patent literature cited in the description**

- **LIEPE et al.** A large fraction of HLA class I ligands are proteasome-generated spliced peptides. *Science*, 21 October 2016, vol. 354 (6310), 354-358
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat'l. Acad. Sci. USA*, 1988, vol. 85, 2444
- **ALTSCHUL et al.** *J. Mol. Biol.*, 1990, vol. 215, 403-410
- **T.E. CREIGHTON**. Proteins: Structures and Molecular Properties. W.H. Freeman and Company, 1993
- **A.L. LEHNINGER**. Biochemistry. Worth Publishers, Inc.
- **SAMBROOK et al.** Molecular Cloning: A Laboratory Manual. 1989
- Methods In Enzymology. Academic Press, Inc.
- Remington's Pharmaceutical Sciences. Mack Publishing Company, 1990
- **CAREY** ; **SUNDBERG**. Advanced Organic Chemistry. Plenum Press, 1992, vol. A,B
- **KOMHER, J. S. et al.** *Nucl. Acids. Res.*, 1989, vol. 17, 7779-7784
- **SOKOLOV, B. P.** *Nucl. Acids Res.*, 1990, vol. 18, 3671
- **SYVANEN, A.-C. et al.** *Genomics*, 1990, vol. 8, 684-692
- **KUPPUSWAMY, M. N. et al.** *Proc. Natl. Acad. Sci. (U.S.A.)*, 1991, vol. 88, 1143-1147

- **PREZANT, T. R. et al.** *Hum. Mutat.*, 1992, vol. 1, 159-164
- **UGOZZOLI, L. et al.** *GATA*, 1992, vol. 9, 107-112
- **NYREN, P. et al.** *Anal. Biochem.*, 1993, vol. 208, 171-175
- **SYVANEN, A.-C. et al.** *Amer. J. Hum. Genet.*, 1993, vol. 52, 46-59
- **MERRIFIELD**. *Science*, 1986, vol. 232, 341-347
- **BARANY** ; **MERRIFIELD**. The Peptides. Academic Press, 1979, 1-284
- **STEWART** ; **YOUNG**. Solid Phase Peptide Synthesis. 1984
- **BOSHART et al.** *Cell*, 1985, vol. 41, 521-530
- **T. A. KOST et al.** *Nucl. Acids Res.*, 1983, vol. 11 (23), 8287
- **SAMBROOK et al.** Molecular Cloning. A Laboratory Manual.. Cold Spring Harbor Laboratory, 1989
- **FANG et al.** Stable antibody expression at therapeutic levels using the 2A peptide. *Nat Biotechnol.*, 17 April 2005, vol. 23 (5), 584-90
- **STRAUSS**. *Microbrial Review*, 1994
- *Frolov RNA*, 2001
- **JOSE**. *Future Microbiol*, 2009
- **WU et al.** *J. Biol. Chem.*, 1989, vol. 264, 16985-16987
- **K. J. FISHER** ; **J. M. WILSON**. *Biochem. J.*, 01 April 1994, vol. 299, 49
- **SZOKA et al.** *Ann. Rev. Biophys. Bioeng.*, 1980, vol. 9, 467

- **WOLFF et al.** *Science*, 1990, vol. 247, 1465-1468
- **MANNINO** ; **GOULD-FOGERITE**. *BioTechniques*, 1988, vol. 6 (7), 682-691
- **FELGNER et al.** *Proc. Natl. Acad. Sci. USA*, 1987, vol. 84, 7413-7414
- **BO LI** ; **COLIN N. DEWEY**. RSEM: accurate transcript quantification from RNA-Seq data with or without a reference genome. *BMC Bioinformatics*, August 2011, vol. 12, 323
- **RIVAS et al.** *Science*, 2015
- **RIVAS**. *Science*, 2015
- **DICKS**. *PloS ONE*, 2012, vol. 7, e40385
- **SLANSKY et al.** *Immunity*, 2000, vol. 13, 529-538
- **KINNEY**. *Virology*, 1986, vol. 152, 400-413
- **SLANSKY et al.** *Immunity*, 2000
- **HUANG et al.** *Proc Natl Acad Sci USA*, 1996, vol. 93, 9730-9735
- **DESRICHARD, A.** ; **SNYDER, A.** ; **CHAN, T. A.** Cancer Neoantigens and Applications for Immunotherapy. *Clin. Cancer Res. Off. J. Am. Assoc. Cancer Res.*, 2015
- **SCHUMACHER, T. N.** ; **SCHREIBER, R. D.** Neoantigens in cancer immunotherapy. *Science*, 2015, vol. 348, 69-74
- **GUBIN, M. M.** ; **ARTYOMOV, M. N.** ; **MARDIS, E. R.** ; **SCHREIBER, R. D.** Tumor neoantigens: building a framework for personalized cancer immunotherapy. *J. Clin. Invest.*, 2015, vol. 125, 3413-3421
- **RIZVI, N. A. et al.** Cancer immunology. Mutational landscape determines sensitivity to PD-1 blockade in non-small cell lung cancer.. *Science*, 2015, vol. 348, 124-128
- **SNYDER, A. et al.** Genetic basis for clinical response to CTLA-4 blockade in melanoma.. *N. Engl. J. Med.*, 2014, vol. 371, 2189-2199
- **CARRENO, B. M. et al.** Cancer immunotherapy. A dendritic cell vaccine increases the breadth and diversity of melanoma neoantigen-specific T cells.. *Science*, 2015, vol. 348, 803-808
- **TRAN, E. et al.** Cancer immunotherapy based on mutation-specific CD4+ T cells in a patient with epithelial cancer.. *Science*, 2014, vol. 344, 641-645
- **LUNDEGAARD, C.** ; **HOOF, I.** ; **LUND, O.** ; **NIELSEN, M.** State of the art and challenges in sequence based T-cell epitope prediction. *Immunome Res.*, 2010, vol. 6 (2), S3
- **YADAV, M. et al.** Predicting immunogenic tumour mutations by combining mass spectrometry and exome sequencing.. *Nature*, 2014, vol. 515, 572-576
- **BASSANI-STEMBERG, M.** ; **PLETSCHER-FRAN-KILD, S.** ; **JENSEN, L. J.** ; **MANN, M.** Mass spectrometry of human leukocyte antigen class I peptidomes reveals strong effects of protein abundance and turnover on antigen presentation.. *Mol. Cell. Proteomics MCP*, 2015, vol. 14, 658-673
- **VAN ALLEN, E. M. et al.** Genomic correlates of response to CTLA-4 blockade in metastatic melanoma.. *Science*, 2015, vol. 350, 207-211
- **YOSHIDA, K.** ; **OGAWA, S.** Splicing factor mutations and cancer.. *Wiley Interdiscip. Rev. RNA*, 2014, vol. 5, 445-459
- Comprehensive molecular profiling of lung adenocarcinoma.. *Nature*, 2014, vol. 511, 543-550
- **RAJASAGI, M. et al.** Systematic identification of personal tumor-specific neoantigens in chronic lymphocytic leukemia.. *Blood*, 2014, vol. 124, 453-462
- *Target Capture for NextGen Sequencing - IDT*, http://www.idtdna.com/pages/products/nextgen/target-capture
- **SHUKLA, S. A. et al.** Comprehensive analysis of cancer-associated somatic mutations in class I HLA genes.. *Nat. Biotechnol.*, 2015, vol. 33, 1152-1158
- **CIESLIK, M. et al.** The use of exome capture RNA-seq for highly degraded RNA with application to clinical cancer sequencing.. *Genome Res.*, 2015, vol. 25, 1372-1381
- **BODINI, M. et al.** The hidden genomic landscape of acute myeloid leukemia: subclonal structure revealed by undetected mutations.. *Blood*, 2015, vol. 125, 600-605
- **SAUNDERS, C. T. et al.** Strelka: accurate somatic small-variant calling from sequenced tumor-normal sample pairs.. *Bioinforma. Oxf. Engl.*, 2012, vol. 28, 1811-1817
- **CIBULSKIS, K. et al.** Sensitive detection of somatic point mutations in impure and heterogeneous cancer samples.. *Nat. Biotechnol.*, 2013, vol. 31, 213-219
- **WILKERSON, M. D. et al.** Integrated RNA and DNA sequencing improves mutation detection in low purity tumors.. *Nucleic Acids Res.*, 2014, vol. 42, e107
- **MOSE, L. E.** ; **WILKERSON, M. D.** ; **HAYES, D. N.** ; **PEROU, C. M.** ; **PARKER, J. S.** ABRA: improved coding indel detection via assembly-based realignment.. *Bioinforma. Oxf. Engl.*, 2014, vol. 30, 2813-2815
- **YE, K.** ; **SCHULZ, M. H.** ; **LONG, Q.** ; **APWEILER, R.** ; **NING, Z.** Pindel: a pattern growth approach to detect break points of large deletions and medium sized insertions from paired-end short reads.. *Bioinforma. Oxf. Engl.*, 2009, vol. 25, 2865-2871
- **LAM, H. Y. K. et al.** Nucleotide-resolution analysis of structural variants using BreakSeq and a breakpoint library.. *Nat. Biotechnol.*, 2010, vol. 28, 47-55

- **FRAMPTON, G. M. et al.** Development and validation of a clinical cancer genomic profiling test based on massively parallel DNA sequencing.. *Nat. Biotechnol.*, 2013, vol. 31, 1023-1031
- **BOEGEL, S. et al.** HLA typing from RNA-Seq sequence reads.. *Genome Med.*, 2012, vol. 4, 102
- **LIU, C. et al.** ATHLATES: accurate typing of human leukocyte antigen through exome sequencing.. *Nucleic Acids Res.*, 2013, vol. 41, e142
- **MAYOR, N. P. et al.** HLA Typing for the Next Generation.. *PloS One*, 2015, vol. 10, e0127153
- **ROY, C. K.** ; **OLSON, S.** ; **GRAVELEY, B. R.** ; **ZAMORE, P. D.** ; **MOORE, M. J.** Assessing long-distance RNA sequence connectivity via RNA-templated DNA-DNA ligation.. *eLife*, 2015, vol. 4
- **SONG, L.** ; **FLOREA, L.** CLASS: constrained transcript assembly of RNA-seq reads. *BMC Bioinformatics*, 2013, vol. 14 (5), S14
- **MARETTY, L.** ; **SIBBESEN, J. A.** ; **KROGH, A.** Bayesian transcriptome assembly.. *Genome Biol.*, 2014, vol. 15, 501
- **PERTEA, M. et al.** StringTie enables improved reconstruction of a transcriptome from RNA-seq reads.. *Nat. Biotechnol.*, 2015, vol. 33, 290-295
- **ROBERTS, A.** ; **PIMENTEL, H.** ; **TRAPNELL, C.** ; **PACHTER, L.** Identification of novel transcripts in annotated genomes using RNA-Seq. *Bioinforma. Oxf. Engl.*, 2011
- **VITTING-SEERUP, K.** ; **PORSE, B. T.** ; **SANDELIN, A.** ; **WAAGE, J.** spliceR: an R package for classification of alternative splicing and prediction of coding potential from RNA-seq data.. *BMC Bioinformatics*, 2014, vol. 15, 81
- **RIVAS, M. A. et al.** Human genomics. Effect of predicted protein-truncating genetic variants on the human transcriptome.. *Science*, 2015, vol. 348, 666-669
- **SKELLY, D. A.** ; **JOHANSSON, M.** ; **MADEOY, J.** ; **WAKEFIELD, J.** ; **AKEY, J. M.** A powerful and flexible statistical framework for testing hypotheses of allele-specific gene expression from RNA-seq data.. *Genome Res.*, 2011, vol. 21, 1728-1737
- **ANDERS, S.** ; **PYL, P. T.** ; **HUBER, W.** HTSeq--a Python framework to work with high-throughput sequencing data. *Bioinforma. Oxf. Engl.*, 2015, vol. 31, 166-169
- **FURNEY, S. J. et al.** SF3B1 mutations are associated with alternative splicing in uveal melanoma.. *Cancer Discov.*, 2013
- **ZHOU, Q. et al.** A chemical genetics approach for the functional assessment of novel cancer genes.. *Cancer Res.*, 2015
- **MAGUIRE, S. L. et al.** SF3B1 mutations constitute a novel therapeutic target in breast cancer.. *J. Pathol.*, 2015, vol. 235, 571-580
- **CARITHERS, L. J. et al.** A Novel Approach to High-Quality Postmortem Tissue Procurement: The GTEx Project.. *Biopreservation Biobanking*, 2015, vol. 13, 311-319
- **XU, G. et al.** RNA CoMPASS: a dual approach for pathogen and host transcriptome analysis of RNA-seq datasets.. *PloS One*, 2014, vol. 9, e89445
- **ANDREATTA, M.** ; **NIELSEN, M.** Gapped sequence alignment using artificial neural networks: application to the MHC class I system.. *Bioinforma. Oxf. Engl.*, 2015
- **JORGENSEN, K. W.** ; **RASMUSSEN, M.** ; **BUUS, S.** ; **NIELSEN, M.** NetMHCstab - predicting stability of peptide-MHC-I complexes; impacts for cytotoxic T lymphocyte epitope discovery.. *Immunology*, 2014, vol. 141, 18-26
- **LARSEN, M. V. et al.** An integrative approach to CTL epitope prediction: a combined algorithm integrating MHC class I binding, TAP transport efficiency, and proteasomal cleavage predictions.. *Eur. J. Immunol.*, 2005, vol. 35, 2295-2303
- **BOISVERT, F.-M. et al.** A Quantitative Spatial Proteomics Analysis of Proteome Turnover in Human Cells.. *Mol. Cell. Proteomics*, 2012, vol. 11, M111.011429-M111.011429
- **DUAN, F. et al.** Genomic and bioinformatic profiling of mutational neoepitopes reveals new rules to predict anticancer immunogenicity.. *J. Exp. Med.*, 2014, vol. 211, 2231-2248
- **CALIS, J. J. A. et al.** Properties of MHC Class I Presented Peptides That Enhance Immunogenicity.. *PLoS Comput. Biol.*, 2013, vol. 9, e1003266
- **ZHANG, J. et al.** Intratumor heterogeneity in localized lung adenocarcinomas delineated by multi-region sequencing.. *Science*, 2014, vol. 346, 256-259
- **WALTER, M. J. et al.** Clonal architecture of secondary acute myeloid leukemia.. *N. Engl. J. Med.*, 2012, vol. 366, 1090-1098
- **HUNT DF** ; **HENDERSON RA** ; **SHABANOWITZ J** ; **SAKAGUCHI K** ; **MICHEL H** ; **SEVILIR N** ; **COX AL** ; **APPELLA E** ; **ENGELHARD VH**. Characterization of peptides bound to the class I MHC molecule HLA-A2.1 by mass spectrometry.. *Science*, 1992, vol. 255, 1261-1263
- **ZARLING AL** ; **POLEFRONE JM** ; **EVANS AM** ; **MIKESH LM** ; **SHABANOWITZ J** ; **LEWIS ST** ; **ENGELHARD VH** ; **HUNT DF**. Identification of class I MHC-associated phosphopeptides as targets for cancer immunotherapy.. *Proc Natl Acad Sci U S A.*, 03 October 2006, vol. 103 (40), 14889-94

- **BASSANI-STERNBERG M** ; **PLETSCHER-FRAN-KILD S** ; **JENSEN LJ** ; **MANN M**. Mass spectrometry of human leukocyte antigen class I peptidomes reveals strong effects of protein abundance and turnover on antigen presentation.. *Mol Cell Proteomics*, March 2015, vol. 14 (3), 658-73
- **ABELIN JG** ; **TRANTHAM PD** ; **PENNY SA** ; **PATTERSON AM** ; **WARD ST** ; **HILDEBRAND WH** ; **COBBOLD M** ; **BAI DL** ; **SHABANOWITZ J** ; **HUNT DF**. Complementary IMAC enrichment methods for HLA-associated phosphopeptide identification by mass spectrometry.. *Nat Protoc.*, 06 August 2015, vol. 10 (9), 1308-18
- **BARNSTABLE CJ** ; **BODMER WF** ; **BROWN G** ; **GALFRE G** ; **MILSTEIN C** ; **WILLIAMS AF** ; **ZIEGLER A**. Production of monoclonal antibodies to group A erythrocytes, HLA and other human cell surface antigens-new tools for genetic analysis.. *Cell*, May 1978, vol. 14 (1), 9-20
- **GOLDMAN JM** ; **HIBBIN J** ; **KEARNEY L** ; **ORCHARD K** ; **TH'NG KH**. HLA-DR monoclonal antibodies inhibit the proliferation of normal and chronic granulocytic leukaemia myeloid progenitor cells.. *Br J Haematol.*, November 1982, vol. 52 (3), 411-20
- **ENG JK** ; **JAHAN TA** ; **HOOPMANN MR**. Comet: an open-source MS/MS sequence database search tool.. *Proteomics.*, 04 December 2012, vol. 13 (1), 22-4
- **ENG JK** ; **HOOPMANN MR** ; **JAHAN TA** ; **EGERTSON JD** ; **NOBLE WS** ; **MACCOSS MJ**. A deeper look into Comet--implementation and features.. *J Am Soc Mass Spectrom.*, 27 June 2015, vol. 26 (11), 1865-74
- **LUKAS KÄLL** ; **JESSE CANTERBURY** ; **JASON WESTON** ; **WILLIAM STAFFORD NOBLE** ; **MICHAEL J. MACCOSS**. Semi-supervised learning for peptide identification from shotgun proteomics datasets.. *Nature Methods*, November 2007, vol. 4, 923-925
- **LUKAS KÄLL** ; **JOHN D. STOREY** ; **MICHAEL J. MACCOSS** ; **WILLIAM STAFFORD NOBLE**. Assigning confidence measures to peptides identified by tandem mass spectrometry. *Journal of Proteome Research*, January 2008, vol. 7 (1), 29-34
- **LUKAS KÄLL** ; **JOHN D. STOREY** ; **WILLIAM STAFFORD NOBLE**. Nonparametric estimation of posterior error probabilities associated with peptides identified by tandem mass spectrometry. *Bioinformatics*, August 2008, vol. 24 (16), 142-148
- **KINNEY RM** ; **BJ JOHNSON** ; **VL BROWN** ; **DW TRENT**. Nucleotide Sequence of the 26 S mRNA of the Virulent Trinidad Donkey Strain of Venezuelan Equine Encephalitis Virus and Deduced Sequence of the Encoded Structural Proteins.. *Virology*, 30 July 1986, vol. 152 (2), 400-413
- **JILL E SLANSKY** ; **FRÉDÉRIQUE M RATTIS** ; **LISA F BOYD** ; **TAREK FAHMY** ; **ELIZABETH M JAFFEE** ; **JONATHAN P SCHNECK** ; **DAVID H MARGULIES** ; **DREW M PARDOLL**. Enhanced Antigen-Specific Antitumor Immunity with Altered Peptide Ligands that Stabilize the MHC-Peptide-TCR Complex. *Immunity*, 01 October 2000, vol. 13 (4), 529-538
- **A Y HUANG** ; **P H GULDEN** ; **A S WOODS** ; **M C THOMAS** ; **C D TONG** ; **W WANG** ; **V H ENGELHARD** ; **G PASTERNACK** ; **R COTTER** ; **D HUNT**. The immunodominant major histocompatibility complex class I-restricted antigen of a murine colon tumor derives from an endogenous retroviral gene product.. *Proc Natl Acad Sci U S A.*, 03 September 1996, vol. 93 (18), 9730-9735
- **JOHNSON, BARBARA J. B.** ; **RICHARD M. KINNEY** ; **CRYSTLE L. KOST** ; **DENNIS W. TRENT**. Molecular Determinants of Alphavirus Neurovirulence: Nucleotide and Deduced Protein Sequence Changes during Attenuation of Venezuelan Equine Encephalitis Virus.. *J Gen Virol*, 1986, vol. 67, 1951-1960
- **AAMOUDSE, C.A.** ; **KRÜSE, M.** ; **KONOPITZKY, R.** ; **BROUWENSTIJN, N.** ; **SCHRIER, P.I.** TCR reconstitution in Jurkat reporter cells facilitates the identification of novel tumor antigens by cDNA expression cloning.. *Int J Cancer*, 2002, vol. 99, 7-13
- **ALEXANDER, J.** ; **SIDNEY, J.** ; **SOUTHWOOD, S.** ; **RUPPERT, J.** ; **OSEROFF, C.** ; **MAEWAL, A.** ; **SNOKE, K.** ; **SERRA, H.M.** ; **KUBO, R.T.** ; **SETTE, A.** Development of high potency universal DR-restricted helper epitopes by modification of high affinity DR-blocking peptides. *Immunity*, 1994, vol. 1, 751-761
- **BANU, N.** ; **CHIA, A.** ; **HO, Z.Z.** ; **GARCIA, A.T.** ; **PARAVASIVAM, K.** ; **GROTENBREG, G.M.** ; **BERTOLETTI, A.** ; **GEHRING, A.J.** Building and optimizing a virus-specific T cell receptor library for targeted immunotherapy in viral infections. *Scientific Reports*, 2014, vol. 4, 4166
- **CORNET, S.** ; **MICONNET, I.** ; **MENEZ, J.** ; **LEMONNIER, F.** ; **KOSMATOPOULOS, K.** Optimal organization of a polypeptide-based candidate cancer vaccine composed of cryptic tumor peptides with enhanced immunogenicity.. *Vaccine*, 2006, vol. 24, 2102-2109
- **DEPLA, E.** ; **VAN DER AA, A.** ; **LIVINGSTON, B.D.** ; **CRIMI, C.** ; **ALLOSERY, K.** ; **DE BRABANDERE, V.** ; **KRAKOVER, J.** ; **MURTHY, S.** ; **HUANG, M.** ; **POWER, S. et al.** Rational design of a multiepitope vaccine encoding T-lymphocyte epitopes for treatment of chronic hepatitis B virus infections.. *Journal of Virology*, 2008, vol. 82, 435-450

- **ISHIOKA, G.Y.** ; **FIKES, J.** ; **HERMANSON, G.** ; **LIVINGSTON, B.** ; **CRIMI, C.** ; **QIN, M.** ; **DEL GUERCIO, M.F.** ; **OSEROFF, C.** ; **DAHLBERG, C.** ; **ALEXANDER, J. et al.** Utilization of MHC class I transgenic mice for development of minigene DNA vaccines encoding multiple HLA-restricted CTL epitopes.. *J Immunol*, 1999, vol. 162, 3915-3925
- **JANETZKI, S.** ; **PRICE, L.** ; **SCHROEDER, H.** ; **BRITTEN, C.M.** ; **WELTERS, M.J.P.** ; **HOOS, A.** Guidelines for the automated evaluation of Elispot assays.. *Nat Protoc*, 2015, vol. 10, 1098-1115
- **LYONS, G.E.** ; **MOORE, T.** ; **BRASIC, N.** ; **LI, M.** ; **ROSZKOWSKI, J.J.** ; **NISHIMURA, M.I.** Influence of human CD8 on antigen recognition by T-cell receptor-transduced cells.. *Cancer Res*, 2006, vol. 66, 11455-11461
- **NAGAI, K.** ; **OCHI, T.** ; **FUJIWARA, H.** ; **AN, J.** ; **SHIRAKATA, T.** ; **MINENO, J.** ; **KUZUSHIMA, K.** ; **SHIKU, H.** ; **MELENHORST, J.J.** ; **GOSTICK, E. et al.** Aurora kinase A-specific T-cell receptor gene transfer redirects T lymphocytes to display effective antileukemia reactivity.. *Blood*, 2012, vol. 119, 368-376
- **PANINA-BORDIGNON, P.** ; **TAN, A.** ; **TERMIJTELEN, A.** ; **DEMOTZ, S.** ; **CORRADIN, G.** ; **LANZAVECCHIA, A.** Universally immunogenic T cell epitopes: promiscuous binding to human MHC class II and promiscuous recognition by T cells.. *Eur J Immunol*, 1989, vol. 19, 2237-2242
- **VITIELLO, A.** ; **MARCHESINI, D.** ; **FURZE, J.** ; **SHERMAN, L.A.** ; **CHESNUT, R.W.** Analysis of the HLA-restricted influenza-specific cytotoxic T lymphocyte response in transgenic mice carrying a chimeric human-mouse class I major histocompatibility complex.. *J Exp Med*, 1991, vol. 173, 1007-1015

- **YACHI, P.P.** ; **AMPUDIA, J.** ; **ZAL, T.** ; **GASCOIGNE, N.R.J.** Altered peptide ligands induce delayed CD8-T cell receptor interaction--a role for CD8 in distinguishing antigen quality.. *Immunity*, 2006, vol. 25, 203-211
- **PUSHKO P** ; **PARKER M** ; **LUDWIG GV** ; **DAVIS NL** ; **JOHNSTON RE** ; **SMITH JF**. Replicon-helper systems from attenuated Venezuelan equine encephalitis virus: expression of heterologous genes in vitro and immunization against heterologous pathogens in vivo.. *Virology.*, 22 December 1997, vol. 239 (2), 389-401
- **STRAUSS, JH** ; **E G STRAUSS**. The alphaviruses: gene expression, replication, and evolution.. *Microbiol Rev.*, September 1994, vol. 58 (3), 491-562
- **RHÊME C** ; **EHRENGRUBER MU** ; **GRANDGIRARD D**. Alphaviral cytotoxicity and its implication in vector development.. *Exp Physiol.*, 12 November 2004, vol. 90 (1), 45-52
- **RILEY, MICHAEL K. II** ; **WILFRED VERMERRIS**. Recent Advances in Nanomaterials for Gene Delivery-A Review. *Nanomaterials*, 2017, vol. 7 (5), 94
- **FROLOV I** ; **HARDY R** ; **RICE CM**. Cis-acting RNA elements at the 5' end of Sindbis virus genome RNA regulate minus- and plus-strand RNA synthesis.. *RNA.*, November 2001, vol. 7 (11), 1638-51
- **JOSE J** ; **SNYDER JE** ; **KUHN RJ**. A structural and functional perspective of alphavirus replication and assembly.. *Future Microbiol.*, September 2009, vol. 4 (7), 837-56